(19) Europäisches Patentamt
European Patent Office
Office européen des brevets

(11) EP 1 975 252 A1

(12) **EUROPEAN PATENT APPLICATION**

(43) Date of publication:
**01.10.2008 Bulletin 2008/40**

(51) Int Cl.:
***C12Q 1/68*** *(2006.01)*

(21) Application number: **07290373.5**

(22) Date of filing: **29.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **INSERM (Institut National de la Santé et de la Recherche Medicale)**
**75013 Paris (FR)**

(72) Inventor: **The designation of the inventor has not yet been filed**

(74) Representative: **Catherine, Alain et al**
**Cabinet Harlé & Phélip**
**7, rue de Madrid**
**75008 Paris (FR)**

(54) **Methods for the prognosis or for the diagnosis of a thyroid disease**

(57) The present invention relates to an integrated *in vitro* method for predicting or diagnosing a specific thyroid disease in an individual, which method is based on the quantification of the expression level of disease-specific marker genes, selected from the group consisting of marker genes that are described in the specification.

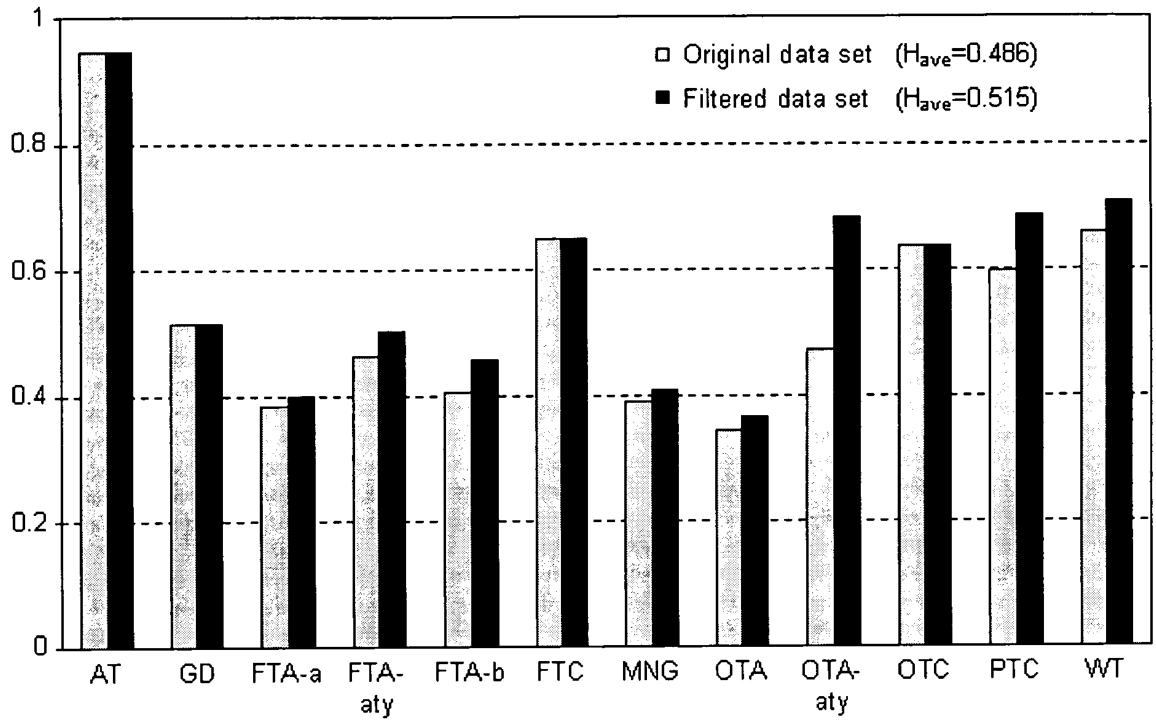


**Figure 2**



Printed by Jouve, 75001 PARIS (FR)

## Description

### FIELD OF THE INVENTION

The present invention relates to methods for predicting or diagnosing a specific thyroid disease in an individual, which methods are based on the quantification of the level of expression of thyroid disease-specific marker genes.

### BACKGROUND OF THE INVENTION

Thyroid is a small endocrine gland that mainly produces two thyroid hormones, respectively tri-iodothyronine (T3) and thyroxine (T4). Thyroid is the subject of various dysfunctions that affect a large number of individuals worldwide, with diverse health consequences, including death. Six classes of differentiated thyroid diseases originating from follicular cells have, to date, been determined which are, respectively (i) follicular adenoma and carcinoma, (ii) papillary carcinoma, (iii) oncocytic adenoma and carcinoma, (iv) Grave's disease, (v) autoimmune thyroiditis and (vi) atypical adenomas.

To date, clinical classification of thyroid diseases, as established by the World Heath Organization (WHO), is mainly based, if not exclusively based, on conventional parameters such as hormone dosage and histology methods. Regarding more particularly thyroid tumors, the one skilled in the art may refer to the WHO classification (DeLellis RA, Lloyd RV, Heitz PU, Eng C (eds) (2004) World Health Organization classification of tumours: Tumours of endocrine organs. IARC, Lyons).

However, this conventional classification of thyroid tumors does not allow a systematic and clear distinction between different thyroid tumors. Illustratively, some thyroid tumors, such as adenomas, are histologically highly heterogeneous, presenting a trabecular, micro- or macrofollicular appearance. The classical features distinguishing carcinomas from adenomas, such as vascular or capsular invasion, are sometimes difficult to evaluate and can lead to interobserver variations (Hirokawa et al., 2002, Am J Surg Pathol, Vol. 26 : 1508-1514; Franc et al., 2003, Hum Pathol, Vol. 34 : 1092-1100; Lloyd et al., 2004, Am J Surg Pathol, Vol. 28 : 1336-13340). Occasionally, adenomas without vascular or capsular invasion show pronounced cellular proliferation, irregular cytological characteristics or unusual histological patterns leading to their classification as atypical adenomas. According to the WHO classification, this term does not correspond to any specific biological behaviour (DeLellis et al., 2004, Worl Health Organization Classification of Tumours : Pathology and Genetics of Tumours of Endocrine Organs. In : 320).

The annual incidence of thyroid cancer varies considerably in different registries, ranging from 1.2 to 2.6 per 100,000 individuals in men and from 2.0 to 3.8 per 100,000 individuals in women. It is particularly elevated in Iceland and Hawaii, being nearly two times higher than in North European countries, Canada and the USA. Recorded incidence of thyroid cancer increased by 2.4 fold between 1973 and 2002. However, this increase has been attributed to increased diagnosis of tumors measuring less than 2 cm in diameter, and has not been associated with an increase in mortality.

The above clinical data illustrate that there is a need in the art for an improved prognosis or diagnosis of thyroid diseases, including thyroid tumors, in view of starting a suitable medical treatment as early as possible, so as to improve the clinical outcome.

Attempts have been made in the art for identifying new markers for improving diagnosis of several thyroid cancers, namely papillary thyroid carcinoma, follicular thyroid carcinomas and thyroid oncocytic tumors, through gene or protein profiling techniques.

Immunohistochemical analysis does not accurately differentiate between benign and malignant lesions. This is particularly true in the case of follicular lesions. Indeed, many of the markers proposed have failed to produce convincing results. Galectin-3 seemed to be the best marker to distinguish between follicular benign and malignant lesions (Hermann M, 2002, Arch Pathol Lab Med, Vol. 126(n°6) : 710-716). However, this marker is less informative when dealing with controversial classes of tumor, such as oncocytic adenoma or carcinoma (Nascimento M, 2001, Endocr Pathol, Vol. 12 (n°3) : 275-279;; Oestreicher-Kedem Y, 2004 , Head neck, Vol. 26(n°11) : 960-966).

In this context, marker genes that are either under-expressed or over-expressed in papillary thyroid carcinomas have already been identified (Huang et al., 2001, Proc. Natl. Acad Sci, Vol. 98(n°26) : 15044-15049; Yano et al., 2004, Clinical Cancer Research, Vol. 10 : 2035-2043; Baris et al., 2005, Oncogene, Vol. 24 : 4155-4161; Jarzab et al., 2005, Cancer Research, Vol. 65(n°4) : 1587-1597).

However, most gene profiling studies known in the art, including those cited above, have compared carcinomas and benign lesions in only a few types of tumors.

There have been two studies on a larger number of different tumors, one distinguishing between benign and malignant tumors (Finlay et al., 2004, The Journal of Clinical Endocrinology & Metabolism, Vol. 89(n°7) : 3214-3223), and the other focussing on follicular thyroid carcinomas and the expression of the PAX8-PPARγ fusion protein (Giordano et al., 2006, Clinical Cancer Research, Vol. 12 : 1983-1993). However, since none of these studies had analyzed a wide range of types of thyroid tumors, including atypical cases, the candidate gene markers proposed cannot be considered specific.

Two microarray studies have proposed combination of genes to distinguish between FTC and benign lesions or PTC (Weber et al., 2005, J Clin Endocrinol Metab, Vol. 90 : 2512-2521). While these small sets of genes may help predict the most frequent lesions of the thyroid, they give no information on, notably, atypical nodules.

Although some attempts have been made in the art for defining new markers of thyroid cancers, it appears that further studies are needed for validating their specificity.

Beyond the strict requirement for a high statistical relevance and a high specificity of any novel marker for a thyroid disease, including a thyroid cancer, there is also a need in the art for a method that would allow an exhaustive prognosis or diagnosis of any one of the known thyroid disease, and not solely a diagnosis of a single thyroid pathology or few thyroid pathologies.

## SUMMARY OF THE INVENTION

The present invention relates to an integrated *in vitro* method for predicting or diagnosing a specific thyroid disease in an individual, which method is based on the quantification of the expression level of disease-specific marker genes, selected from the group consisting of marker genes specific for (i) autoimmune thyroiditis (AT), (ii) marker genes specific for Grave's disease (GD), (iii) marker genes specific for macrofollicular adenoma (FTA-a), (iv) marker genes specific for atypical follicular adenoma (FTA-aty), (v) marker genes specific for microfollicular adenoma (FTA-b), (vi) marker genes specific for follicular carcinoma (FTC), (vii) marker genes specific for multinodular goitres (MNG), (viii) marker genes specific for oncocytic adenoma (OTA), (ix) marker genes specific for atypical oncocytic adenoma (OTA-aty), (x) marker genes specific for oncocytic carcinoma (OTC) and (xi) marker genes specific for papillary carcinoma (PTC).

## BRIEF DESCRIPTION OF THE FIGURES

**Figure 1 : Gene selection by the average separation parameter.**

This graph shows all possible sets of best genes on the X-axis, i.e. with the lowest P-values, and the corresponding average separation parameters on the Y-axis. Low values of separation indicate well-separated classes and high values poorly-separated classes. The parameter has to be minimized while the gene selection size has to be maximized to improve the power of analysis. A point of discontinuity represents the optimal average separation equal to 0.1732 for the 1197 best ranked genes.

**Figure 2 : Effect of sample filtering on data set homogeneity.**

This figure shows homogeneity values for each tissue from the original data set (grey bars) and from the filtered data set (black bars). The average homogeneity ($H_{ave}$) of the original data set is equal to 0.486. The average homogeneity of the filtered data set is equal to 0.515.

## DETAILED DESCRIPTION OF THE INVENTION

The present inventors have simultaneously analyzed the gene-expression signatures of more than 90% of the medically-recognized thyroid lesions in order to select disease-specific marker genes for each class of thyroid disease, and especially of each class of thyroid tumor. All differentiated follicular tumors or pathologies, as well as the wild type tissue (WT), were represented in the analysis: macrofollicular (FTA-a) and microfollicular (FTA-b) adenomas, multinodular goitres (MNG), follicular (FTC) and papillary (PTC) carcinomas, autoimmune thyroiditis (AT) and Graves' disease (GD). The analysis performed by the inventors also includes atypical adenomas and oncocytic tumors associated to follicular or papillary tumors: atypical follicular adenomas (FTA-aty), oncocytic adenomas (OTA), atypical oncocytic adenomas (OTA-aty) and oncocytic carcinomas (OTC). The molecular classification of the tumors has then been compared to the pathologic diagnostic groups.

The experimental work performed by the inventors has allowed the identification of disease-specific marker genes, which expression is deregulated, specifically in patients suffering from a given thyroid disease selected from the group of marker genes consisting of (i) marker genes specific for autoimmune thyroiditis (AT), (ii) marker genes specific for Grave's disease (GD), (iii) marker genes specific for macrofollicular adenoma (FTA-a), (iv) marker genes specific for atypical follicular adenoma (FTA-aty), (v) marker genes specific for microfollicular adenoma (FTA-b), (vi) marker genes specific for follicular carcinoma (FTC), (vii) marker genes specific for multinodular goitres (MNG), (viii) marker genes specific for oncocytic adenoma (OTA), (ix) marker genes specific for atypical oncocytic adenoma (OTA-aty), (x) marker genes specific for oncocytic carcinoma (OTC) and (xi) marker genes specific for papillary carcinoma (PTC).

Thus, the present invention provides for an *in vitro* method allowing (1) detecting a deregulation of the expression of one or more disease-specific marker genes, each marker gene being comprised in a single group of marker genes

among groups (i) to (xi) listed above, and then (2) predicting or diagnosing the occurrence of a specific kind of thyroid disease, the said specific kind of thyroid disease being determined by determining which group (i) to (xi) comprises the marker gene(s) for which a deregulation has been detected.

An object of the present invention consists of an integrated *in vitro* method for the prediction or for the diagnosis of a specific thyroid disease in a patient comprising the steps of:

a) providing a thyroid tissue sample previously collected from the said patient;

b) quantifying, in the said thyroid tissue sample, the expression level of two or more disease-specific marker genes that are indicative of the risk of occurrence of, or of the occurrence of, a specific thyroid disease, wherein the said two or more disease-specific marker genes are selected from two or more of the groups of marker genes consisting of:

(i) marker genes specific for autoimmune thyroiditis (AT) that are contained as inserted DNA in the recombinant vectors selected from the group of vectors having the following IMAGE clone ID N° :

(i-1) 669510, 725096, 366100, 325155, 154172, 787938, 744055, 592276, 740941, 73778, 667174, 739193, 295412, 23827, 136686, 744385, 471641, 724276, 28584, 268972, 739097, 744943, 724609, 2074228, 365913, 687782, 23872, 668452, 681959, 687379, 23271, 731745, 738332, 726760, 666911, 724150, 682555, 587186, 813260, 136646, 309645, 809828, 487152, 564994, 667348, 730149, 731115, 738945, 985457, 179288, 742862, 78353, 503189, 1087348, 753215, 471574, 471696, 549054, 744657, 731357, 713422, 486708, 666794, 726830, 730829, 300268, 725364, 665376, 375746, 324079, 365271, 172982, 724562, 839101, 265558, 52226, 713922, 136919, 682119, 745106, 1877668, 1594414, 471829, 739956, 23945, 173841, 31120, 342181, 742580, 135941, 32339, 687912, 666154, 743774, 738970, 22711, 501971, 364846, 175864, 666792, 724416, 174683, 667892, 744087, 68950, 2467442, 136807, 744439, 1716286, 121551, 744797, 742061, 666778, 342181, 740158, 724596, 143759, 666315, 50202, 667514, 364959, 258666, 148796, 364324, 72744, 669263, 667533, 365177, 2055272, 731298, 512910, 470408, 137720, 2237263, 712295, 284828, 81911, 251452, 135705, 153667, 666007, 2266583, 136863, 724119, 136223, 727251, 485872, 137238, 713653, 364412, 471702, 668684, 727056, 665674, 251250, 364706, 136260, 136560, 738938, 382423, 430263, 2139164, 309697, 773192, 726272, 682088, 666367, 21716, 135689, 383945, 727289, 724554, 364729, 665649, 668152, 811582, 666551, 472111, 489945, 486304, 416744, 258865, 744050, 743016, 1846326, 509606, 666110, 51232, 3659591, 667355, 364974, 666860, 179266, 727092, 24176, 725076, 137353, 307094, 23765, 666168, 725836, 172996, 364926, 525221, 472160, 667110, 730012, 135980, 136014, 2046361, 726763, 731726, 135887, 365288, 44881, 724508, 146976, 744616, 366159, 724895, 724892, 668912, 743987, 23817, 665538, 687625, 136606, 713879, 26164, 667587, 731410, 743422, 687532, 726335, 669318, 214611, 669181, 713647, 178792, 1417901, 135766, 364469, 565317, 382738, 669188, 666361, 667117, 591907, 173288, 257555, 214008, 730938, 868169, 731404, 667112, 135773, 34007, 1521706, 687679, 730534, 33621, 664233, 135125, 155345, 208991, 177827, 667353, 745003, 731685, 79729, 25664, 139242, 666140, 270549, 173200, 364777, 740381, 795309, 731423, 49888, 741891, 232714, 743290, 133531, 665379, 669319, 258127, 1238492, 682555, 726821, 1420676, 364510, 668186, 44193, 365045, 32917, 1174287, 725371, 119772, 730834, 252382, 179214, 1288183, 628418, 382791, 2384812, 364822, 726513, 282587, 731047, 25883, 134858, 207920, 668476, 251147, 667259, 38403, 365311, 178908, 1911930, 135379, 681875, 149809, 971276, 687667, 738558, 136952, 687287, 744606, 743118, 364352, 667375, 726647, 134978, 726675, 743394, 738424, 729929, 782688, 726782, 135303, 742695, 363955, 731348, 366889, 194350, 436554, 668536, 725340, 174861, 1859532, 261500, 714414, 667361, 742739, 364741, 366032, 729510, 745490, 731021, 342551, 740457, 726596, 2032639, 136121, 745072, 665086, 738966, 731073, 135407, 471664, 301122, 738506, 136747, 714437, 485104, 179733, 713913, 742542, 743760, 26651, 743519, 196992, 665392, 364717, 731277, 667067, 136868, 564878, 743245, 668089, 136399, 383718, 741954, 742590, 544683, 134976, 365589, 730300, 381854, 731305, 2348237, 669564, 714049, 366067, 487148, 668239, 1837488, 359202, 266361, 172785, 25895, 545475, 501527, 726981, 359051, 230126, 724642, 668815, 687852, 26455, 286138, 682507, 135539, 469549, 687953, 366085, 744395, 726353, 324666, 135352, 25865, 175772, 134671, 730699, 22908, 343987, 366842, 323438, 26519, 366042, 727067, 365121 and 359461,
(i-2) 43541, 687990, 365990, 562904, 725548, 365877, 742763, 180259, 2449149, 1089025, 713623, 382760, 258747, 743961, 174396, 724306, 1505308, 726454, 665452, 665833, 1352408, 731060, 73703, 207926, 489326, 1743279, 139073, 1626951, 1578721, 188393, 156720, 781738, 1645668, 714493, 1896337, 195340, 203166, 310519, 503874, 743722, 324066, 738944, 201467, 1870594, 122874, 140405, 204686, 2497617, 2306096, 1707527, 609631, 356960, 2345206, 740931, 342256, 725473, 682207, 2242054, 868577, 727154, 279470, 813174, 252489, 2341829, 729924, 263251, 250883, 738896, 1326169, 448163, 731648, 2113771, 365349, 731023, 744980, 668584, 727496, 132933, 592598, 46991, 128065, 730037, 356841, 2238108, 823680, 292042, 504539, 485803, 340657, 590264, 1084386, 742853, 210587, 884365, 134615, 740927, 1088781, 2091591, 667239, 727430, 486401, 1286238, 742577, 1916307, 3054031, 366289, 366233, 80688, 665632, 379200, 162246, 743246, 486935, 287843, 365755, 811024, 489249, 840753, 731689, 50491, 1422194, 1251197, 1942634, 725268, 666410, 743415, 364448, 743367, 1287638, 668442, 28511, 1422791, 743224, 124701, 725612, 471568, 156183, 840687, 731751, 1705397, 26061, 666061, 725345, 740347, 346610, 131839, 123950, 2336358, 135801, 666425, 809959, 1550616, 2148946, 2728204, 366585, 667727, 116906, 288807, 345034, 768241, 667440, 1089513, 1131054, 1256485, 245426, 427786, 742696 and 744911

(ii) marker genes specific for Grave's disease (GD that are contained as inserted DNA in the recombinant vectors selected from the group of vectors having the following IMAGE clone ID N° :

(ii-1) 25664, 868169, 731380, 1049033, 730942, 731616, 726578, 366388, 485872, 1662274, 2074228, 730926, 745106, 731021, 207920, 258865, 503189, 745490, 1103402, 1911930, 731305, 173296, 669181, 727491, 713879, 359461, 265558, 687667, 668239, 232366, 730814, 484535, 743774, 667587, 665086, 366889, 723950, 256177, 1859532, 665433, 985457, 564878, 376356, 383868, 471664, 724642, 23817, 724596, 487152, 669549, 155345, 347362, 666425, 24541, 290265, 667252, 725231, 343987, 705147, 1238492, 745072, 544683, 3054031, 744604, 731073, 2237263, 136919, 486447, 740620, 1420676, 151247, 738558, 731645, 179288, 667683, 135125, 723914, 666110, 381854, 365056, 738970, 724554, 301122, 382521, 341763, 136747, 364717, 484577, 668510, 145696, 594279, 725340, 712118, 82687, 252291, 666551, 713859, 359051, 786662, 741497, 731298, 729957, 743903, 682555, 30981, 666778, 135407, 742919, 378458, 743579, 258127, 365613, 357807, 383528, 486189, 738938, 279172, 665649, 172828, 738332, 549054, 742695, 213529, 667048, 307094, 436554, 324079, 365526, 471574, 742580, 666277, 2348237, 485652, 744606, 52226, 236119, 730924, 173200, 342551, 744087, 345034, 731590, 743118, 261500, 136223, 668536, 666928 and 730149,
(ii-2) 687990, 743961, 486493, 379279 and 667313,

(iii) marker genes specific for macrofollicular adenoma (FTA-a) that are contained as inserted DNA in the recombinant vectors selected from the group of vectors having the following IMAGE clone ID N° :

(iii-1) 2348237, 135379, 743851, 1712992, 376699, 1251197, 1174287, 173841, 486221, 265345, 666564, 252382, 173371, 731073, 253386, 1285305, 342700, 179214, 3054031, 359797, 486189, 258865, 731742, 666061, 135705, 745072, 731044, 731616, 2148946, 258966, 504539, 725188, 213529, 742919, 682585, 666554, 825224, 1049033, 365045, 742837, 127507, 725198, 116906, 135875, 135773, 486282, 343987, 232366, 80162, 566862, 724544, 731115, 725454, 813174, 340657, 838611, 730534, 742763, 178255, 687336, 742542, 667683, 725473, 139073, 731202, 594279, 727289, 382423, 724556, 740457, 682276, 666361, 713879, 1016390, 667110, 359461, 25664, 743519, 486540, 1645668, 744899, 562904, 1174342, 258127, 1916307, 665693, 714109, 665649, 687270, 1942634, 1352408, 364686, 357442, 729957, 667598, 739247, 382738, 145696, 1578721, 544683, 745512, 249311, 705147, 727482, 365526, 724884, 1575056, 739432, 743224, 730779, 137238, 284828, 667048, 739237, 731047, 1326169, 364570, 174396, 665086, 235938, 666860, 743394, 758037, 740090, 594655, 760065, 731076, 1188588, 137478, ,868169, 1012990, 382069, 666425, 135203, 341763, 669549, 726894, 731277, 485104, 668239, 682207, 741406, 201467, 1088781, 382002, 236155, 731758, 364934, 235882, 178792, 365056, 668152, 665452, 136646, 666110, 668146, 347503, 744616, 177857, 258747, 665156, 1308954, 471664, 485872, 587186, 136919, 504596, 265558, 135407, 156183, 484577, 725707, 173200, 137454, 743078, 155345, 666794, 667375, 135303, 134976, 301122, 741497, 725585, 743579, 263251, 744074, 42280, 742695, 731598, 666426, 743603, 665403, 188232, 1859532, 290265, 884365, 512910, 687953, 731357, 723950, 136747, 31924, 279470, 741790, 781738, 204686, 666334, 119939, 1085884, 489326, 356841, 743982, 1707527, 35628, 730814, 738358, 667252, 740927, 342581, 137131, 2150502, 2032639, 2306096, 767779, 743182, 309697, 744374, 2238108, 136223, 666279, 726821, 383945, 726618, 23817, 188393, 741474, 25895, 135980, 136014, 741066, 382521, 731275, 136598, 2345206, 382791, 724238, 485803, 725970, 682119, 276727, 668476, 713647, 742061, 1075949, 241481, 347362, 725548, 1492440, 1117183, 726600, 489249, 2345206, 327182, 133531, 2214396, 71428, 665342, 742743, 666235, 743259, 2030501, 666703, 179534, 744983, 356960, 1561035, 726647, 1741589, 137719, 2048801, 487148, 33664, 43541, 744895, 687667, 132752, 122874 and 2032639,
(iii-2) 745003, 1844968, 364324, 357201, 24392, 1420676, 687532, 724562, 729510, 725371, 24083, 738938, 730938, 148796, 137205, 174683, 21808, 3659591, 325155, 666792, 154172, 295412, 176543, 24532, 359202, 68950, 2046361, 725364, 741178, 668360, 300194, 23872, 364352, 364469, 726353, 742100, 23271, 667112, 25949, 22711, 744055, 430263, 738506, 738945, 743245, 812967, 744087, 250883, 741954, 667892, 364974, 744505, 187482, 52226, 23945, 179288, 738507, 1526058, 782688, 174861, 669295, 713859, 136868, 24300, 731726, 682251, 21716, 38789, 25883, 172693, 725340, 669141, 712295, 23641, 738944, 1716286, 723867, 726272, 23827, 357807, 136801, 135887, 2254555, 53333, 472111, 214008, 744106, 136462, 725533, 366042, 173820, 136114, 364575, 68972, 687679, 366436, 2237263, 32339, 26486, 1698236, 279150, 1101773, 666986, 669470, 257259, 667313, 128694, 178750, 2341829, 472160 and 727123,

(iv) marker genes specific for atypical follicular adenoma (FTA-aty) that are contained as inserted DNA in the recombinant vectors selected from the group of vectors having the following IMAGE clone ID N° :

(iv-1) 503874, 666128, 838611, 208991, 739097, 1089025, 725548, 739193, 155839, 28584, 202897, 743331, 743619, 725345, 345034, 235938, 729924, 681957, 30219, 773330, 665632, 2048801, 760065, 119939, 50491, 195340, 740941, 201467, 1581686, 743603, 207920, 731616, 71428, 30471, 504646, 487848, 342581, 1942634, 2238108, 740158, 42280, 196338, 742739, 743615, 1074708, 366484, 30981, 743246, 592598, 485104, 174396, 364777, 379540, 3054031, 123950, 1536968, 366100, 489326, 24587, 665086, 342256, 448163, 35628, 544818, 179534, 665156, 309645, 726454, 738896, 31924, 665452, 564878, 1286238, 379517, 177857, 744410, 730926, 809828, 288807, 740105, 366842, 146049, 713831, 140347, 666279, 341763, 665784, 24532, 33133, 365045, 1288183, 133531, 744926, 742763, 472111, 1012990, 743259, 1707527, 365121, 743270, 666315, 2484270, 471696, 364706, 364741, 80384, 590264, 285693, 173296, 1561035, 469549, 1422194, 731689, 667527, 1860115, 1087348, 204686, 1075949, 378813, 28511, 731422, 813174, 740931, 25883, 310519, 340657, 727289, 743722, 743367, 1662274, 364083, 136399, 731363, 743579, 1088781, 131839, 839101, 2449149, 1846326, 741406, 823590, 724014, 178750, 713623, 767779, 366067, 743078, 383966, 295412, 323438, 364729, 122874, 724642, 795309, 178255, 382760, 135941, 1102600, 69166, 231903, 682276, 1716286, 667533, 40304, 32917, 668452, 731060, 665820, 1624260, 1352408, 471664, 486401, 731722, 135689, 740604, 2139164, 739109, 135671, 666703, 433155, 687800, 502244, 73703, 484577, 1251197, 731202, 381032, 811024, 347503, 238840, 128065, 382791, 1870594, 258114, 731742, 727056, 781738, 487152, 731305, 731726, 342181, 745512, 356960, 365271, 78353, 730924, 742853, 2091591, 731023, 173200, 504596, 666792, 383718, 382002, 741474, 366032, 135766, 383945, 840687, 364436, 729957, 501527 and 364686,
(iv-2) 50202, 738970, 128694, 741891, 666671, 366388, 146976, 687667, 587186, 667727, 669136, 744001, 666361, 668300, 713647, 2074228, 282587, 179288, 687270, 726830, 666860, 687896, 364510, 324666, 300194, 268972, 135096, 565754, 136361, 300268, 713422, 758037, 713653, 366042, 723914, 172693, 208097, 549054, 730300, 175864, 178908, 687468, 134978, 729956, 503189 and 681875,

(v) marker genes specific for microfollicular adenoma (FTA-b) that are contained as inserted DNA in the recombinant vectors selected from the group of vectors having the following IMAGE clone ID N° :

(v-1) 740931 ; 731751 ; 840687; 666128; 379540; 667259; 725585; 503874 ; 23612 ; : 3054031 ; 208097 ; 265558 ; 136976, 838611, 725707, 594279, 343987, 487474, 731128, 279172, 773330, 1089025, 725502, 2091591, 141216, 235882, 665452, 1251197, 236399, 50491, 376699, 727496, 725877, 682207, 687667, 729924, 179534, 724366, 30471, 743246, 1088781, 345034, 195340, 868577, 2238108, 2148946, 258114, 136919, 1870594, 1241974, 172828, 731348, 740158, 592598, 1326169, 809959, 741790, 725268, 119939, 135980, , 136014, 740604, 730037, 448163, 668239, 512420, 258865, 365526, 667365, 742577, 116906, 258747, 738896, 342256, 486221, 288807, 743259, 136223, 725970, 342700, 327182, 264166, 1049033, 365801, 156720, 725473, 1074708, 290265, 687379, 71428, 1624260, 486401, 669564, 133531, 232388, 726454, 1705397, 487848, 809719, 251427, 287843, 1870594, 744926, 682119, 1641901, 742901, 1707527, 742853, 33133, 664233, 504596, 364204, 285693, 25664, 174396, 743722, 209137, 310519, 42280, 249311, 1505308, 727178, 667067, 731726, 46991, 137454, 740347, 666334, 666671, 359461, 487302, 1352408, 504646, 743118, 365919, 687800, 26061, 301122, 122874, 129438, 666235, 471664, 741497, 1117183, 364526, 175772, 179214, 471568, 744606, 1103402, 1942634, 727263, 484577, 745490, 31924, 682555, 665693, 823680, 1626951, 562904, 682311, 132752, 359797, 485803, 725548, 705147, 730012, 504539, 491004, 667440, 1917430, 743774, 666110, 309697, 724416, 135303, 731019, 1287638, 742743, 740718, 1645668, 378813, 1016390, 124701, 162246, 731738, 681890, 309645, 665376, 238840, 489326, 665632, 201467, 188232, 486189, 485085, 203166, 744374, 813174, 1743279, 742763, 156183, 669549, 376356, 665086, 2449149, 137719, 139073, 357442, 745083, 724544, 132933, 665433, 2497617, 22085, 364570, 713879, 731023, 713922, 252291, 1911930, 436554, 743415, 145696, 178348, 667239, 236155, 196992, 2484742, 781738 and 210587,
(v-2) 724609, 365913, 666154, 267358, 665379, 667892, 251250, 713653, 365589, 1087348, 366042, 743146, 174654, 744657, 214008, 666007, 137005, 364436, 738558, 146976, 682175, 725533, 365121, 667112, 770858, 3659591, 469345, 154172, 731404, 730036, 135671, 739097, 739193, 743205, 811582, 364352, 725364, 668684, 725076, 666946, 136260, 971276, 2139164, 365271, 307025, 366254, 125552, 669263, 744439, 26519, 564994, 668815, 725335, 592276, 176543, 296702, 668152, 295412, 149809, 24176, 153667, 726675, 486510, 1526058, 366243, 148796, 194350, 143759, 33621, 668089 and 669319,

(vi) marker genes specific for follicular carcinoma (FTC) that are contained as inserted DNA in the recombinant vectors selected from the group of vectors having the following IMAGE clone ID N° :

(vi-1) 501476, 309009, 739193, 251452, 681957, 739097, 364448, 469549, 202897, 195340, 731380, 2345206, 23765, 471641, 502244, 713653, 486189, 740604, 682207, 740941, 469647, 743519, 743367, 966894, 727056, 2336358, 52897, 1326169, 773330, 139073, 725548, 137238, 742853, 838611, 22908, 207920, 375746, 738896, 152802, 343987, 743246, 1089025, 342256, 729924, 727496, 667514, 50491, 726454, 486401, 2238108, 730123, 342551, 1186334, 365589, 1837488, 1942634, 276727, 666986, 324066, 724014, 364083, 2449149, 744943, 258747, 276658, 265558, 731745, 726763, 28511, 235882, 1707527, 1896337, 448163, 301122, 724306, 687800, 669318, 2214396, 175268, 740347, 365271, 366074, 594279, 1942634, 366289, 665086, 1641901, 724366, 509606, 26061, 162246, 489249, 24541, 723923, 731751, 744410, 1870594, 743259, 731277, 381854, 25949, 489326, 491004, 2728204, 666703, 251147, 1870594, 484535, 258114, 26519, 487148, 726684, 149809, 731616, 252291, 71428, 364324, 156183, 25865, 1705397, 251250, 79729, 365562, 811024, 730926, 1085884, 366159, 682276, 364469, 668442, 741905, 356841, 730814, 177827, 42280, 724151, 743270, 136260,485085, 742837, 666128, 666279, 727092, 364777, 179334, 757248, 2484270, 287843, 469345, 667061, 485803, 727491, 213113, 364846, 743619, 743987, 180640, 364706, 365177, 592598, 971276, 1581686, 713671, 129438, 1117183, 345034, 741066, 1286238, 178792, 740931, 744657, 740381, 2237263, 666154, 665538, 135671, 172996, 1698236, 768241 and 188393,
(vi-2) 782688, 136014, 135980, 725335, 724150, 307094, 723752, 510318, 726578, 324079, 2139164, 143759, 739237, 152655, 136646, 309697, 384087, 365408, 376699, 134671, 609631, 238840, 1074708, 366254, 742919, 257259, 840753, 668924, 136801, 743245, 731428, 731410, 738507, 430263, 744374, 1492440, 135887, 726782, 379517 and 726849,

(vii) marker genes specific for multinodular goitres (MNG) that are contained as inserted DNA in the recombinant vectors selected from the group of vectors having the following IMAGE clone ID N° :

(vii-1) 284828, 173841, 667527, 202897, 469549, 207920, 469647, 363955, 323438, 276658, 263251, 731277, 35628, 741474, 743394, 212366, 258865, 724014, 742837, 1117183, 276727, 811582, 1743279, 666279, 666703, 725585, 714498, 525221, 739109, 731380, 382738, 1012990, 188232, 1012903, 726675, 356960, 235882, 208439, 178348, 727056, 124781, 300268, 868577, 327182, 249311, 266361, 241481, 376153, 745490, 31924, 235938, 687667, 44881, 725548, 383868, 342551, 382521, 667727, 365562, 359051, 136863, 773286, 236155, 744899, 366042, 383945, 723923, 340657, 668442, 382791, 666794, 121551, 346610, 232366, 110481, 128065, 379937, 731076, 682207, 50202, 725198, 208991, 173371, 724895, 742763, 666426, 743182, 381854, 347503, 743903, 838611, 155345, 2030501, 811024, 489326, 731645, 365521, 135671, 136747, 665433, 1705397, 731689, 724609, 740105, 731389, 1288183, 80162, 127507, 382002, 665086, 213113, 141216, 713913, 813174, 665495, 667110, 744395, 668300, 594279, 668510, 177857, 745512, 382069, 110101, 2242054, 310519, 38403, 3054031, 1188588, 665814, 667756, 173818, 731758, 2345206, 471568, 301122, 730814, 743270, 1859532, 382423, 725677, 744410, 723914, 1846326, 129438, 667514, 823680, 162246, 731115, 125552, 666367, 484535, 2113771, 667252, 744050, 562904, 666554, 667355, 137131, 760065, 727289, 731616, 230126, 725231, 135801, 512420, 594510, 40304, 1641901, 180259, 296702, 485872, 136919, 364741, 2148946, 364436, 136976, 1917430, 1049033, 264166, 741406, 196338, 1088980, 768241, 742061, 69166, 739237, 512910, 42280, 134416, 724238, 357442, 731598, 740347, 730834, 2384812, 724554, 201467, 1308954, 731305, 256177, 157847, 687336, 134615, 73778, 739851, 258966, 136801, 383718, 666334, 486221, 177775, 740457, 713608, 485085, 119772, 31120, 544683, 145696, 383528, 743619, 341763, 471664, 342581, 726647, 743982, 743290, 356841, 178255, 724812, 156183, 152847, 324066, 731742, 724884, 265558, 124701, 376983, 725188, 743016, 140405, 253386, 713879, 238840, 30471, 485803, 179266, 364885, 592276, 486189, 725473, 723950, 724537, 1645668, 137719, 1174287, 743517, 257555, 188393, 745123, 71428, 741497, 544818, 251427, 366085, 25865, 236119, 485104, 1570420, 174654, 745072,

(vii-2) 731202, 179534, 116906 and 1942634, 687625, 726782, 364324, 148796, 744505, 26164, 665668, 713837, 172828, 731726, 151247, 486356, 342181, 471641, 72744, 135689, 740941, 366032, 154172, 743987, 713422, 279172, 131839, 666551, 665392, 2055272, 729957, 258666, 743391, 53333, 109142, 743615, 133531, 309645, 68972, 682311, 666946, 194302, 26455, 744439, 739451, 137010, 669295, 325155, 731685, 172982, 738506, 51232, 78353, 742695, 1690788, 52226, 136114, 153667, 134858 and 667587,

(viii) marker genes specific for oncocytic adenoma (OTA) that are contained as inserted DNA in the recombinant vectors selected from the group of vectors having the following IMAGE clone ID N° :

(viii-1) 80688, 1870594, 731738, 1870594, 365348, 681875, 731751, 136114, 365161, 491004, 724366, 744983, 740718, 884365, 687381, 236399, 731726, 809719, 757248, 172982, 743774, 666410, 743961, 667239, 725502, 265345, 366436, 668584, 725076, 365349, 148796, 23612, 725726, 667361, 53333, 279470, 729510, 725877, 729956, 345034, 665784, 730300, 382612, 137454, 135689, 667310, 1860115, 731714, 365097, 729971, 731128, 682311, 549054, 726092, 609631, 366042, 714437, 68972, 741954, 725791, 730012, 669564, 666455, 238840, 173820, 743485, 726782, 235882, 743760, 364324, 823680, 179334, 729542, 135930, 738506, 155839, 416959, 682109, 175268, 26486, 725707, 725970, 667348, 669263, 28705, 725612, 162246, 666140, 738332, 365877, 740941, 727056, 838611, 731275, 310519, 384087, 174879, 744087, 727496, 379279, 25226, 731428, 365990, 364568, 2266583, 180082, 590264, 725836, 731648, 253386, 151247, 724562, 713837, 379517, 726454, 135887, 682088, 725340, 730606, 840753, 1422791, 723752, 669149, 741891, 669295, 382760, 208097, 665632, 731665, 486708, 285693, 1932168, 726578, 1896337, 250883, 713671, 730037, 740620, 682149, 116906, 669470, 1526058, 365090, 740245, 24541, 1326169, 486935, 365000, 727123, 1877668, 666066, 687306, 503874, 687541, 738944, 726849, 365609, 782688, 38789, 365271, 739901, 145112, 2381770, 742853, 484535, 356841, 743391, 365613, 738970, 364204, 740347, 742806, 152655, 2341829, 112819, 592598, 668089, 739956, 714049, 668684, 293916, 666128, 139242, 51232, 743118, 2728204, 565754, 727229, 2254555, 742543, 364448, 135096, 486401, 364575, 713831, 687990, 668924, 738896, 131839, 207926, 342551, 210587, 324079, 366388, 251216, 137005, 741894, 22085, 44193, 109142, 666361, 148212, 140405, 726272, 448163, 135875, 24392, 725401, 739247, 366289, 868577, 134858, 140347, 261656, 665376, 743246, 1420676, 726596, 357201, 668283, 172828, 487474, 665833, 309645, 376597, 731685, 2484742, 366089, 286138, 687912, 1536968, 666007, 667313, 1641901, 136361, 282587, 665156, 365311, 730123, 173119, 366842, 178750, 1707527, 364510, 364412, 366159, 668707, 279150, 739451, 365408, 745490, 667365, 510318, 2238108, 68950, 743043, 2306096, 740130, 669141, 743722, 22384, 682555, 682251, 669181, 743259, 208991, 342256, 744106, 666425, 738358, 726684, 365562, 745106, 665392, 731060, 687625, 23945, 1239535, 682528, 725268, 486493, 292042, 2467442, 134671, 486356, 665437, 713623, 666029, 379540, 1942634, 252489, 295412, 365121, 594279, 23827, 666517, 682479, 726779, 730829, 21808, 135450, 742100, 34007, 288807, 1016390, 114008, 366254, 713422, 730149, 469549, 135539, 665452, 725394, 665254, 759865, 667174, 744945, 726634, 172785, 2011082, 714013, 33621, 359461, 137131, 134978, 301122, 23271, 366115, 366074, 687270, 687667, 503189, 357807, 743415, 128694, 731398, 180259, 666235, 667353, 682555, 136868, 135991, 687287, 1741589, 52897, 687852, 781738, 26164, 152802, 1089025, 154172, 43541, 24300, 25949, 725677, 214611, 744439, 23872, 2032639, 730938, 743125, 2214396, 727251, 723923, 738938, 134785, 173288, 725364, 26455, 213113, 730036, 687896, 667587, 22908, 731404, 325155, 731423, 46991, 727457, 300194, 669136, 268972, 726513, 667598, 133531, 713653, 30219, 188232, 727263, 53081, 203166, 2237263, 740604, 743987, 738945, 669510, 123950, 26651, 665342, 724892, 687782, 194302, 307025, 727154, 1911930, 469647, 471641, 366032, 29541, 740224, 724150, 136598, 153667, 212366, 666778, 364526, 738966, 3659591, 22711, 1088781, 744505, 744606, 135203, 667061, 365177, 758037, 25883, 713647, 740580, 726353, 178908, 156720, 1844968, 137010, 136801, 724276, 24176, 740381, 137720, 725096, 179288 and 665668,

(viii-2) 1662274, 145696, 730926, 731115, 155345, 376356, 666794, 739109, 179534, 628418, 741406, 485872, 232388, 667110, 724895, 33133, 730834, 743270, 731380, 713913, 2048801, 485104, 731422, 124781, 731616, 724014, 668186, 731598, 1012990, 382738, 714498, 745072, 1942634, 342581, 347503, 731742, 724537, 365045, 738424, 724556, 730814, 666703, 341763, 739851, 744841, 744895, 726894, 512910, 384404, 382621, 744074, 258127, 364741, 136747, 1174342, 177967, 760065, 177857, 687468, 723950, 2384812, 504646, 740105, 365056, 137478, 378458, 753215, 724642, 731021, 3054031, 178255, 433155, 731076, 744410, 743603, 731722, 742046, 727289, 714493, 178792, 1581686, 1705397, 1308954, 366243, 667252, 731590, 724238, 730924, 504596, 544818, 35628, 486221, 381032, 501527, 258865, 741474, 724884, 276658, 1186334, 731227, 665649, 1859532, 564878, 71428, 666279, 668146, 731241, 257555, 381118, 665814, 726821, 364686, 208439, 82687, 1085884, 727296, 740457, 667527, 1671903, 201467, 135379, 795736, 1075949, 739432, 363955, 213529, 743331, 137238, 743619, 1241974, 632026, 730699, 743451, 665820, 725188, 731202, 379937, 487148, 1288183, 172996, 382069, 731277, 230126, 743982, 743579, 40304, 376983, 179266, 40134, 235938, 731689, 136863, 665433, 727067, 743078, 809959, 381854, 813174, 1846326, 382002, 2030501, 666334, 767779, 731305, 471829, 383868, 566862, 731758, 1624260, 1917430, 364777, 743519, 667355, 364729, 591907, 25865, 241481, 525221, 127507, 307094, 135352, 236119, 743811, 382791, 742061, 323438, 251147, 364706, 487152, 364717, 136686, 364885, 178348, 731363, 666085, 1174287, 667657, 347362, 743268, 196338, 173200, 486189, 665403, 487848, 745512, 731044, 472160, 713624, 177775, 173628, 594655, 744616, 364932, 157847, 744899, 681890, 745133, 713608, 740090, 727178, 743394, 471574, 471664, 486304, 731745, 366585, 1012903, 24587, 742837, 236155, 726600, 152847, 486540, 731019, 985457, 383718, 731645, 724554, 714414, 1578721, 786662, 383966, 261500, 119772, 743182, 366233, 364822, 132752, 364356, 31924, 366085, 38403, 668510, 1743279, 743290, 263251, 30471, ,486447, 729929, 2242054, 382521, 296702, 173371, 383945, 121551, 179133, 136919, 666367, 740158, 682585, 1285305, 730942, 284828, 668329, 42280, 136121, 25664, 1561035, 379200, 669549, 668452, 726618, 256177, 724596, 743016, 135671, 177827, 364469, 124701, 485652, ,667259, 712118, 346610,743220, 1049033 and 430471,

(ix) marker genes specific for atypical oncocytic adenoma (OTA-aty) that are contained as inserted DNA in the recombinant vectors selected from the group of vectors having the following IMAGE clone ID N° :

(ix-1) 745003, 740130, 471641, 682088, 731404, 2254555, 2381770, 214008, 300268, 2467442, 125552, 666517, 366159, 740927, 665784, 24392, 727457, 148796, 590264, 665632, 345034, 840753, 309645, 743043, 731275, 51232, 667048, 838611, 773330, 884365, 667514, 364412, 366115, 757248, 146049, 140347, 342181, 727123, 30219, 155839, 382612, 723923, 682276, 682479, 743118, 740941, 356841, 53081, 745490, 687381, 324079, 173818, 731060, 743367, 365000, 669149, 285693, 731398, 110481, 726578, 687990, 23612, 723752, 713831, 1536968, 725076, 471702, 119939, 448163, 365271, 738506, 665674, 666128, 29541, 204686, 731665, 682251, 724126, 22908, 152802, 356960, 173119, 366289, 669141, 1420676, 682109, 301122, 357442, 174879, 868577, 25883, 288807, 366089, 471852, 162246, 742100, 743722, 666455, 265558, 744943, 379279, 668924, 203166, 194302, 729971, 724366, 682311, 667348, 365877, 731423, 713859, 730037, 23641, 713837, 376597, 135125, 669263, 123950, 172982, 1911930, 152655, 122874, 1084386, 839101, 251427, 666168, 112819, 565317, 1188588, 366889, 743485, 471568, 744505, 669510, 667361, 135689, 666564, 738332, 23765, 668584, 669319, 744087, 188393, 667587, 795309, 292042, 812967, 135450, 327182, 666946, 366032, 1707527, 667310, 52897, 137131, 366042, 738938, 725726, 667313, 665392, 1846326, 730149, 744606, 489249, 739237, 731047, 687541, 365161, 687800, 68950, 136260, 109142, 69166, 357807, 207926, 2091591, 1550616, 740381, 731714, 364575, 1286238, 364526 and 151247, [Under-expressed]

(ix-2) 724556, 668329, 173200, 174654, 731227, 1624260, 743078, 739576, 3054031 and 119772, [Over-expressed]

(x) marker genes specific for oncocytic carcinoma (OTC) that are contained as inserted DNA in the recombinant vectors selected from the group of vectors having the following IMAGE clone ID N° :

(x-1) 727123, 724366, 366289, 471641, 501476, 744943, 2237263, 155839, 140347, 743774, 1716286, 365271, 236399, 731423, 252291, 812967, 739097, 471702, 1420676, 739193, 740347, 669564, 609631, 731751, 666671, 745490, 285693, 235882, 725791, 23612, 345034, 503874, 838611, 81911, 469647, 667514, 730012, 730300, 740941, 738358, 486493, 809719, 726684, 687667, 162246, 740620, 366436, 726454, 469549, 146976, 665784, 731738, 365349, 238840, 491004, 122874, 46991, 726763, 78353, 738896, 178908, 416959, 486401, 665632, 742590, 180259, 666455, 22711, 665674, 148796, 489326, 25883, 724306, 745003, 594279, 342256, 26455, 143759, 687852, 592598, 713422, 173820, 136560, 209137, 136361, 743205, 366042, 665538, 727056, 1837488, 782688, 744087, 725877, 2238108, 257259, 725836, 666154, 743246, 971276, 448163, 666410, 137720, 1844968, 73703, 770858, 2055272, 487474, 731023, 667348, 50491, 740130, 342551, 713623, 2032639, 723923, 2728204, 176543, 781738, 839101, 32339, 731357, 738970, 364204, 1089025, 364575, 34007, 135096, 364412, 743259, 1870594, 1101773, 366233, 342290, 366067, 565317, 742853, 29541, 682528, 80688, 731398, 713831, 309697, 744983, 187482, 665452, 665376, 359051, 139242, 1707527, 731726, 145112, 744055, 282587, 136114, 1526058, 366115, 123950, 743043, 416744, 1536968, 309645, 738332, 471696, 365090, 667310, 1088781, 149809, 173119, 724416, 743146, 109142, 1239535, 668815, 364510, 738938, 741954, 668089, 151247, 729542, 665833, 549054, 116906, 666946, 135941, 472111, 809828, 135450, 26651, 2115808, 713671, 743615, 731714, 666128, 366842, 724892, 730938, 22908, 324079, 365613, 231903, 743422, 687541, 668360, 287843, 489249, 342181, 207920, 364324, 682109, 743367, 2381770, 22085, 730123, 135875, 250883, 667061, 724126, 1521706, 687679, 208097, 745106, 38789, 682479, 744606, 668684, 726272, 743451, 25949, 744001, 1870594, 179334, 364568, 357807, 590264, 137353, 207926, 731298, 173288, 565754, 69166, 668584, 364926, 154172, 725726, 725340, 23765, 687625, 743391, 292042, 2032639, 128694, 258666, 382612, 725394, 687381, 469345, 730036, 180640, 188393, 52897, 295412, 51232, 884365, 744945, 174683, 195340, 279470, 731648, 665392, 730606, 379540, 43541, 725364, 731404, 729924, 137454, 665437, 253386, 359202, 731685, 364974, 135689, 727229, 2467442, 1417901, 725707, 204686, 741891, 53081, 134785, 682555, 22384, 134858, 379279, 135539, 300194, 2214396, 687270, 731060, 174861, 366388, 738944, 365288, 251216, 545475, 726779, 136868, 743118, 135352, 180082, 2113771, 178750, 731128, 179733, 137205, 740927, 376153, 365097 and 30219, [Under-expressed]

(x-2) 731019, 366085, 258127, 382002, 714414, 486189, 725188, 1241974, 666703, 487152, 40304, 811024, 25865, 666279, 745133, 347362, 258966, 471664, 82687, 740931, 739109, 628418, 731742, 42280, 724014, 1012903, 1288183, 382423, 512910, 667527, 730942, 731021, 3054031, 724596, 179266, 742061, 667683, 743603, 71428 and 713608, [Over-expressed]

(xi) marker genes specific for papillary carcinoma (PTC) that are contained as inserted DNA in the recombinant vectors selected from the group of vectors having the following IMAGE clone ID N° :

(xi-1) 726763, 739193, 28584, 739097, 25883, 1716286, 365045, 666367, 379200, 365271, 731227, 726760, 384404, 208991, 744943, 1846326, 364729, 730926, 795736, 136686, 667117, 743517, 724151, 296702, 742837, 364777, 364706, 731616, 666986, 80384, 666007, 1837488, 383868, 40304, 366100, 744055, 136919, 230126, 1288183, 813174, 469647, 787938, 740158, 713608, 669318, 795309, 812967, 325155, 382791, 724596, 743703, 724238, 154172, 366484, 667657, 469549, 124781, 668452, 713831, 713913, 383718, 382621, 744616, 364686, 667533, 744074, 364822, 49888, 24587, 363955, 114008, 201467, 295412, 375746, 666315, 744385, 544818, 364846, 145696, 738424, 1624260, 485652, 1285305, 2484270, 194302, 770858, 366074, 823590, 724014, 379937, 194350, 727321, 366243, 68950, 364741, 731305, 1174342, 744895, 342181, 985457, 2384812, 727289, 731277, 1085884, 727296, 383966, 729924, 382738, 471574, 682585, 525221, 1102600, 665379, 753215, 359202, 1581686, 744797, 726513, 743982, 731722, 486304, 323438, 743422, 727491, 364436, 1844968, 666128, 30981, 382069, 3659591, 730829, 433155, 687953, 667176, 1671903, 364324, 726779, 668146, 743579, 26164, 740457, 307025, 667527, 135941, 744657, 665433, 731590, 378458, 723923, 136747, 136399, 744941, 365913, 730924, 376983, 669149, 682251, 2046361, 30471, 665403, 743043, 712118, 153667, 342290, 731422, 666703, 667067, 665814, 742743, 82687, 809828, 173628, 2345206, 471702, 179534, 730814, 172996, 668329, 471641, 665820, 731598, 136121, 148796, 743331, 1942634, 731758, 1352408, 309697, 731202, 472111, 501527, 78353, 741474, 738945, 178750, 743603, 724642, 33621, 364083, 381032, 745003, 366032, 971276, 177967, 609631, 725345, 745512, 71428, 485085, 743903, 744439, 743270, 738966, 196338, 731363, 743394, 366067, 727067, 745106, 31924, 724895, 34007, 744841, 52226, 731021, 682276, 687679, 135352, 1662274, 285693, 307094, 42280, 1308954, 668815, 665668, 232714, 1594414, 682119, 26455, 667110, 134785, 665674, 731380, 180640, 838611, 742046, 30219, 207920, 743016, 667259, 628418, 667252, 504646, 667361, 742061, 726675, 231903, 667892, 136863, 725533, 1917430, 137353, 744106, 714493, 32917, 714013, 342181, 501476, 730834, 79729, 666794, 2467442, 1698236, 665632, 743268, 741066, 726618, 743290, 665495, 73778, 744410, 669149, 381118, 809959, 276658, 632026, 122874, 744050, 731745, 1174287, 745123, 342581, 681890, 136462, 26651, 668186, 740090, 196992, 1578721, 745072, 669263, 729929, 726821, 740941, 1422194, 714414, 173820, 251250, 365056, 724609, 1075949, 512910, 132752, 740105, 2048801, 178255, 364717, 687379, 232388, 342181, 178908, 744604, 714498, 665342, 731241, 487152, 504158, 668476, 743619, 687800, 667355, 724812, 33133, 739432, 730938, 590264, 726634, 687532, 146049, 213529, 687896, 1626951, 284828, 1049033, 341763, 727056, 3054031, 726600, 156183, 731115, 471696, 143759, 366115, 383528, 731742, 31120, 174861, 668584, 743451, 112819, 2030501, 724556, 1101773, 137010, 2345206, 731076, 365000, 1707527, 366233, 366436, 1087348, 668360, 241481, 731404, 21716, 738938, 53333, 383945, 2055272, 682507, 1012990, 180259, 759865 and 723950, [Under-expressed]

(xi-2) 758037, 1932168, 489945, 738970, 667756, 265558, 2113771, 365562, 1712992, 739237, 135539, 159809, 268972, 135303, 1916307, 356841, 682088, 665437, 744945, 2728204, 324079, 727540, 470408, 740347, 364526, 343987, 730300, 594279, 162246, 549054, 743774, 510318, 136976, 725677, 667440, 757248, 140405, 2214396, 436554, 357807, 2306096, 668239, 365919, 665693, 125552, 725585, 823680, 359051, 1846326, 309009, 252291, 1877668, 1505308, 145105, 1741589, 327182, 188393, 743125, 132933, 52897, 731751, 359461, 173841, 212366, 768241, 324066, 730037, 235882, 668300, 2266583, 738358, 723914, 116906, 731738, 209137, 745490, 208097, 44193, 1743279, 110481, 24541, 727251, 745083, 1103402, 681957, 135689, 132935, 731128, 376153, 282587, 666361, 135875, 726636, 1641901, 1690788, 2011082, 366159, 32784, 22711, 713671, 44881, 1492440, 364448, 365526, 725707, 725454, 365348, 1896337, 251427, 725096, 137454, 727092, 357442, 725502, 152655, 668283, 136801, 43541, 667727, 725970, 266361, 135991, 365801, 1238492, 1417901, 731047, 665156, 175772, 141216, 365521, 742919, 743367, 731299, 730012, 489249, 744980, 301122, 713647, 416959, 23612, 128065, 236399, 726335, 366388, 731298, 384087, 300268, 134978, 279470, 740718, 665452, 208439, 738332, 726272, 136014, 135980, 731060, 742100, 364570, 487474, 868577, 1084386, 364412 and 249311,

whereby an expression value is provided for each quantified marker gene expression level;

c) comparing (i) the expression value obtained at step b) for each disease-specific marker gene with (ii) a control expression value for the said marker gene, whereby a deregulated expression level of each disease-specific marker gene may be determined;

d) predicting or diagnosing the occurrence of a thyroid disease in the said patient if one or more disease-specific marker genes comprised in a group of marker genes, among the groups (i) to (xi) defined at step b), has a deregulated expression level; and

e) identifying, among the groups of disease-specific marker genes (i) to (xi) defined at step b), the group comprising the said one or more marker genes having a deregulated expression level, whereby the kind of specific thyroid disease that is predicted or diagnosed in the said patient is determined.

As used herein, the expression "disease-specific" means a relationship with a single specific thyroid disease. Thus, according to the invention, a "disease-specific" marker gene denotes a gene whose expression is deregulated only in patients that are affected by a specific thyroid disease. Preferably, thyroid diseases are selected from the group consisting of (i) autoimmune thyroiditis (AT), (ii) Grave’s disease (GD), macrofollicular adenoma (FTA-a), (iv) atypical follicular adenoma (FTA-aty), (v) microfollicular adenoma (FTA-b), (vi) follicular carcinoma (FTC), (vii) multinodular goitres (MNG), (viii) oncocytic adenoma (OTA), (ix) atypical oncocytic adenoma (OTA-aty), (x) oncocytic carcinoma (OTC) and (xi) papillary carcinoma (PTC).

The disease-specific marker genes that are listed in groups (i-1), (ii-1), (iii-1), (iv-1), (v-1), (vi-1), (vii-1), (viii-1), (ix-1), (x-1) and (xi-1) are "under-expressed" in patients affected with the corresponding thyroid disease. Thus, in patients affected with the specified thyroid disease of a given group selected from (i-1) to (xi-1), the expression level of the said disease-specific marker genes is lower than the expression level of the same genes that is determined in patients which are not affected with the said given thyroid disease, including patients which are not affected with a thyroid disease.

The disease-specific marker genes that are listed in groups (i-2), (ii-2), (iii-2), (iv-2), (v-2), (vi-2), (vii-2), (viii-2), (ix-2), (x-2) and (xi-2) are "over-expressed" in patients affected with the corresponding thyroid disease. Thus, in patients affected with the specified thyroid disease of a given group selected from (i-2) to (xi-2), the expression level of the said disease-specific marker genes is higher than the expression level of the same genes that is determined in patients which are not affected with the said given thyroid disease, including patients which are not affected with a thyroid disease.

The *in vitro* prediction or diagnostic method above is termed an "integrated" method, since the said method allows the simultaneous testing of at least two disease-specific marker genes that are, when taken together, indicative of more than one thyroid disease. The integrated prediction or diagnosis method according to the invention thus allows to simultaneously test for the risk of occurrence of, or for the occurrence of, a thyroid disease, among all, or almost all, the known thyroid diseases. In some embodiments, the integrated method according to the invention allows the testing for the risk of occurrence of, or for the occurrence of, any one, or at least almost any one, thyroid disease among those that are already clinically classified.

Thus, according to the prediction or diagnosis method according to the invention, it is now possible to predict or diagnose the occurrence of any kind of thyroid disease, and especially any kind of follicular thyroid tumor, by performing a single test procedure using a unique biological sample from a patient.

The integrated *in vitro* method according to the invention consists of a prediction method, since the marker gene expression deregulation, that is determined after having performed the comparison step c), generally occurs very early during the course of the thyroid disease progression, and in most cases, if not in all cases, far before that any other detectable phenotypic change is available to the clinician’s analysis who implements conventional clinical methods for diagnosing thyroid diseases, like those established by the World Health Organization (WHO), namely the WHO International Classification of diseases, including the WHO Classification of pituitary tumors.

The integrated *in vitro* method also consists of a diagnosis method, since it is shown herein a specific relationship between (i) a deregulation of the expression of each marker gene disclosed in the present specification and (ii) a particular kind of thyroid disease, as conventionally classified, e.g. according to the clinical analysis methods established by the WHO International Classification of diseases.

The "thyroid tissue sample" that is provided at step a) of the method consists of a sample from the thyroid tissue originating from the patient to be tested, which tissue sample comprises at least the minimum number of cells allowing the production of an amount of nucleic acid expression products (e.g. mRNA or cDNA) for performing step b) in optimal conditions. Generally, the thyroid tissue sample comprises at least $10^3$ cells, and preferably at least $10^6$ cells from the thyroid organ. Typically, the thyroid tissue sample that is provided at step a) of the method may be any of the specimens such as those taken by fine needle aspiration from a subject, or those prepared by excision and extirpation of a part of the thyroid.

At step b) of the method, quantifying the expression level of disease-specific marker genes encompasses determining an absolute or relative quantification value that illustrates the said marker genes expression activity. Quantification encompasses determining a quantification value for the mRNA synthesized by each of the disease-specific marker genes tested, or of the cDNA that may be obtained from the corresponding mRNA. As it will be specified further in the present specification, the quantification value that is determined at step b) of the method may consist of an absolute quantification value that reflects the amount of mRNA produced from each disease-specific marker gene tested that is present in the patient's thyroid tissue sample. In other embodiments of the method, the said quantification value may be expressed as a relative value, e.g.. the ratio between (i) the amount of mRNA produced by the disease-specific marker gene tested and (ii) the amount of mRNA produced by a gene that is constitutively expressed, e.g. a housekeeping gene like actin.

As used herein, a "gene" encompasses, or alternatively consists of, a nucleic acid that is contained in the human genome and which is expressible, i.e. which is able to give rise to a corresponding mRNA. Thus, a "gene", as used in the present specification, consists of a human genomic nucleic acid encoding a mRNA, whether or not the encoded mRNA codes for a polypeptide.

The identity of every disease-specific marker gene of interest that is described in the present specification is unequivocally defined as a gene, whose expression gives rise to a mRNA, and wherein the said mRNA (or its corresponding double-stranded cDNA) comprises the heterologous nucleic acid insert included in a recombinant vector that is unequivocally referenced by a "Clone ID N°" in the I.M.A.G.E. collection of human cDNA clones (Integrated Molecular Analysis of Genomes and their Expression), which are mainly available under the form of arrayed cDNA libraries. Thus, a disease-specific marker gene is identified herein as a IMAGE clone ID number, which consists of the reference number of a specific clone that is publicly available upon request to the IMAGE Consortium or to agreed distributors, including (i) the American Type Culture Collection (ATCC, Manassas, VA, USA), (ii) Open Biosystems (Huntsville, AL, USA) or (iii) Research Genetics/Invitrogen (Carlsbad, CA, USA).

The one skilled in the art, on the sole basis of the IMAGE clone ID N° reference for a disease-specific marker gene described herein, has thus a full access to the corresponding biological material, i.e. the recombinant vector that comprises, as an inserted cDNA, the nucleic acid sequence originating form the mRNA that is encoded by the corresponding disease-specific marker gene.

The disease-specific marker genes described herein are all available as IMAGE human cDNA clones, wherein the human cDNA is contained in the pT3T7 cloning vector, which consists of a well known pUC19-based phagemid vector comprising both the phage T3 and T7 promoters, and which is functional in *E. coli.*

The nucleic acid sequence of the human cDNA of interest that is inserted in each recombinant vector identified as an IMAGE clone ID N° may be easily determined by the one skilled in the art, by using conventional techniques of DNA amplification and sequencing. Illustratively, for obtaining the nucleic acid sequence of the disease-specific marker genes described herein that are inserted in the pT3T7 cloning vector, the one skilled in the art may amplify the DNA insert using primers that specifically hybridize with the T3 promoter sequence (5'-AATTAATTAACCCTCACTAAAGGGT-3' - **SEQ ID N°1**) or with the T7 promoter sequence (5'-AGCTGTAATACGACTCACTATAGGG-3' - **SEQ ID N° 2**), before sequencing.

Alternatively, the nucleic acid sequence of the human cDNA of interest that is inserted in each recombinant vector identified as an IMAGE clone ID N° is directly available upon a query based on the IMAGE clone ID number disclosed herein at the IMAGE Consortium database, e.g. at the following Web address : http://image.llnl.gov/image/html/query tools.shtml.

As a further alternative of access to the nucleic acid sequences of the disease-specific marker genes disclosed herein, the one skilled in the art may refer to the gene names corresponding to the IMAGE clone ID numbers that are listed in **Tables 3-24**, which gene names consist of the unequivocal name of each human gene that is attributed by the HUGO Gene Nomenclature Committee (HGNC). Nucleic acid sequences of the disease-specific marker genes disclosed herein are thus also available upon query at the HGCN database on the basis of the internationally recognized gene name, e.g. at the following Web address : http://www.gene.ucl.ac.uk/cgi-bin/nomenclature/searchgenes.pl.

Tables 3-24 list the disease-specific marker genes of interest whose expression level may be quantified, when performing the *in vitro* prediction or diagnosis method according to the invention.

Table 3 lists the disease-specific marker genes that are indicative of the occurrence of AT, if under-expressed.
Table 4 lists the disease-specific marker genes that are indicative of the occurrence of AT, if over-expressed.
Table 5 lists the disease-specific marker genes that are indicative of the occurrence of GD, if under-expressed.
Table 6 lists the disease-specific marker genes that are indicative of the occurrence of GD, if over-expressed.
Table 7 lists the disease-specific marker genes that are indicative of the occurrence of FTA-a, if under-expressed.
Table 8 lists the disease-specific marker genes that are indicative of the occurrence of FTA-a, if over-expressed.
Table 9 lists the disease-specific marker genes that are indicative of the occurrence of FTA-atypical, if under-expressed.
Table 10 lists the disease-specific marker genes that are indicative of the occurrence of FTA-atypical, if over-expressed.
Table 11 lists the disease-specific marker genes that are indicative of the occurrence of FTA-b, if under-expressed.
Table 12 lists the disease-specific marker genes that are indicative of the occurrence of FTA-b, if over-expressed.
Table 13 lists the disease-specific marker genes that are indicative of the occurrence of FTC, if under-expressed.
Table 14 lists the disease-specific marker genes that are indicative of the occurrence of FTC, if over-expressed.
Table 15 lists the disease-specific marker genes that are indicative of the occurrence of MNG, if under-expressed.
Table 16 lists the disease-specific marker genes that are indicative of the occurrence of MNG, if over-expressed.
Table 17 lists the disease-specific marker genes that are indicative of the occurrence of OTA, if under-expressed.
Table 18 lists the disease-specific marker genes that are indicative of the occurrence of OTA, if over-expressed.
Table 19 lists the disease-specific marker genes that are indicative of the occurrence of OTA atypical, if under-expressed.
Table 20 lists the disease-specific marker genes that are indicative of the occurrence of AT, if over-expressed.
Table 21 lists the disease-specific marker genes that are indicative of the occurrence of OTC, if under-expressed.
Table 22 lists the disease-specific marker genes that are indicative of the occurrence of OTC, if over-expressed.
Table 23 lists the disease-specific marker genes that are indicative of the occurrence of PTC, if under-expressed.
Table 24 lists the disease-specific marker genes that are indicative of the occurrence of PTC, if over-expressed.

In each of Tables 3-24, the left column ("name") indicates, when available, the disease-specific marker gene name, according to the HUGO international nomenclature. When the international nomenclature gene name was not available, the IMAGE clone ID n° is indicated. The second column ("cloid") indicates the IMAGE clone ID n° of the recombinant vector comprising, inserted therein, the cDNA encoding the disease-specific marker gene that is publicly available upon request near the IMAGE Consortium or near an agreed distributor Company. The third column ("p value") indicates the statistical relevance of the relationship between (i) a deregulated expression of the disease-specific marker gene and (ii) the occurrence of the thyroid disease. The right column ("fold") indicates the mean fold over-expression or under-expression of the disease-specific marker gene that has been experimentally determined, as compared to the "control" expression level value that has been determined from thyroid tissue samples originating from patients which are not affected with the specific thyroid disease, which include patients which are affected with a distinct thyroid disease, as well as patients which are not affected with any thyroid disease..

In all cases, the one skilled in the art may design detection and/or quantification means, including nucleic acid primers or probes, specific for every one of the marker genes of interest described herein, on the basis of the IMAGE clone cDNA material and also on the basis of their sequences that are available from the various sequence databases that are referred to above.

Illustratively pair of primers that specifically hybridise with the target nucleic acid gene marker of interest may be designed by any one of the numerous methods known in the art, based on the known partial or complete sequence of the said marker gene.

In certain embodiments, for each of the marker genes of the invention, at least one pair of specific primers, as well as the corresponding detection nucleic acid probe, is already referenced and entirely described in the public "Quantitative PCR primer database", notably at the following Internet address : "http://Ipgws.nci.nih.gov/cgi-bin/PrimerViewer".

In all cases, the one skilled in the art may design nucleic acid primers or probes that specifically hybridise with each of the marker genes described herein, starting from their known 3'-end and/or 5'-end nucleic acid sequences.

In other embodiments, a specific pair of primers may be designed using the method disclosed in the US Patent n° US 6,892,141 to Nakae et al., the entire disclosure of which is herein incorporated by reference.

Examples of polynucleotides that are directly usable as primers or probes, or alternatively are usable for designing primers or probes, for the purpose of the invention consist of the polynucleotides having the nucleic acid sequences SEQ ID N° 3 to 420, each polynucleotide being specific for a given disease-specific marker gene described herein, as it will be detailed elsewhere in the present specification.

As disclosed in the examples herein, the inventors have performed a wide range differential expression analysis of more than 8000 candidate genes on thyroid tissue samples previously collected from patients affected with a thyroid disease. The collection of thyroid tissue samples that has been used for performing the differential expression analysis comprised various sets of thyroid tissue samples, each set of thyroid tissue samples comprising three or more tissue samples originating from the same number of patients for which a specific thyroid disease had been clinically diagnosed, and the whole sets of thyroid tissue samples covering all, or at least almost all, thyroid diseases that are presently known in the art.

The wide range differential expression analysis of more than 8000 candidate genes was performed by :

(i) manufacturing DNA microarrays by immobilization on a solid support of labeled candidate gene-specific probes, each labeled probe being previously prepared by PCR amplification of each of the more than 8000 IMAGE clone vectors, each IMAGE clone vector containing a candidate gene nucleic acid. Each labeled probe has the ability to specifically hybridize with an expression product of the corresponding candidate gene.;
(ii) (ii-a) extracting the total RNA material from each thyroid tissue sample previously collected from patients affected with a thyroid disease, and (ii-b) obtaining labeled cDNAs from the total population of mRNAs extracted at step (ii-a). Total RNA extracted from individuals that are not affected with a thyroid disease is also used, as further controls.
(iii) hybridizing the labeled cDNAs obtained at step (ii) with the DNA microarrays manufactured at step (i);
(iv) measuring the signal intensities that are generated after the formation of hybrid complexes between (i) a plurality of probes immobilized on the DNA microarrays and (ii) a plurality of labeled cDNAs, whereby an expression value for each candidate gene is obtained, and
(v) determining :

(v-1) for each of the candidate gene tested, if there exists a relevant relationship between the expression level value of the said candidate gene, as measured at step (iv), and the occurrence of the thyroid disease, or
(v-2) for sets of two or more of the candidate genes tested, if there exists a relevant relationship between the expression level value of the said candidate gene, as measured at step (iv), and the occurrence of the thyroid disease

The determination of a relevant relationship between the expression level value of the said candidate gene, at step (v-2) above, and the occurrence of the thyroid disease, may be performed by any one of the methods of the suitable statistical analysis that are well known from the one skilled in the art.

The determination of a relevant relationship between the expression level values of a set of two or more candidate genes, as measured at step (iv) above, and the occurrence of the thyroid disease was performed as it follows :

(1)

(1-a) performing a non parametric one-way analysis of variance (ANOVA) for detecting differences between the 12 class means (i.e. individuals affected with a thyroid disease among the 11 thyroid disease classes (i) to (xi) previously described, and individuals not affected with a thyroid disease).
(1-b) lowering the incidence of multiple testing effects, using the false discovery rate (FDR) controlling procedure.
(1-c) determining gene expression specificities with t-tests, by comparing mean class expression with gene profiles. (1-d) removing divergent measurements (artefacts).

(2) assessing both (i) homogeneity of each of the 12 thyroid tissue classes and (ii) homogeneity across all the 12 classes.
(3) scoring each thyroid tissue sample by its similarity to the corresponding class centroids.
Steps (1) to (3) allow correcting the raw expression level measurements that were performed at step (iv) of the method above.
(4) evaluating the thyroid disease prediction power of the candidate genes tested, by calculating, for sets of two or more candidate genes, a predictive score which is assigned after comparing the statistical relevance values of the said sets of candidate genes obtained by one or more statistical method, such as one or more statistical method selected from the group consisting of (i) the Linear Diagonal Discriminant Analysis (LDDA), the K-nearest neighbors for (ii) K=1 and (iii) K=3, and (iv) the Nearest Centroid (NC). The predictive score that is finally calculated for each set of candidate genes may consist of a p value, in which case the statistical relevancy of a given candidate gene for predicting a given thyroid disease increases with decreasing values of the p value predictive score.
(5) selecting a candidate gene as a disease-specific marker gene if the assigned score value indicates a statistical relevancy which is higher than a threshold statistical relevancy (predictive score threshold value).

According to the invention a candidate gene consists of a disease-specific marker gene if its predictive score value, calculated as a p value, is lower than 0.05, when using the method generally disclosed above and detailed in the examples herein.

According to the invention a set of candidate genes consists of a set of disease-specific marker gene if its predictive score value, calculated as a p value, is lower than 0.05, when using the method generally disclosed above and detailed in the examples herein.

A complete list of the candidate genes that have been finally selected as disease-specific genes according to the invention is disclosed in Tables 3-24. All the disease-specific marker genes that are listed in Tables 3-24 have a p value that is lower than 0.05, when the p value is assessed by the method described in the examples herein.

Thus, in all embodiments of the *in vitro* prediction or diagnosis method according to the invention, the two or more disease-specific marker genes that are quantified at step b) consist of marker genes having a p value predictive score lower than 0.05, as shown in Tables 3-24.

The accuracy of the prediction or the diagnosis results that are obtained at steps d) and e) of the *in vitro* method increases with an increasing number of disease-specific marker genes belonging to a single thyroid disease class (i) to (xi) that are quantified at step b).

Depending on the accuracy of the prediction or of the diagnosis results which is sought, when performing the *in vitro* prediction or diagnosis method according to the invention, the one skilled in the art will adapt step b), so as to use a suitable combination of (1) the number of disease-specific marker genes to be quantified for a specific thyroid disease class among classes (i) to (xi) defined previously and (2) the predictive score (e.g. the p value) of the disease-specific marker genes to be quantified.

As it is shown in Tables 3-24, for a given class of thyroid disease among the classes (i) to (xi) previously defined herein, (a) a part of the disease-specific marker genes may be over-expressed, and (b) the remaining part of the disease-specific marker genes may be under-expressed, as compared to its expression level value found for all other thyroid disease classes as well as for healthy individuals.

At step b) of the *in vitro* prediction or diagnosis method according to the invention, the number of disease-specific marker genes that are quantified in each group (i) to (xi) is of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40,41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133,134,135,136,137,138,139,140,141,142,143,144,145,146,147,148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199; 200, 201, 202, 203, 204, 205, 206, 207, 208, 209, 210, 211, 212, 213,214,215,216,217,218,219,220,221,222,223,224,225,226,227,228, 229,230,231,232,233,234,235,236,237,238,239,240,241,242,243,244, 245,246,247,248,249,250,251,252,253,254,255,256,257,258,259,260, 261,262,263,264,265,266,267,268,269,270,271,272,273,274,275,276, 277,278,279,280,281,282,283,284,285,286,287,288,289,290,291,292, 293,294,295,296,297,298,299,300,301,302,303,304,305,306,307,308, 309,310,311,312,313,314,315,316,317,318,319,320,321,322,323,324, 325,326,327,328,329,330,331,332,333,334,335,336,337,338,339,340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, 700, 701, 702, 703, 704, 705, 706, 707, 708, 709, 710, 711, 712, 713, 714, 715, 716, 717, 718, 719, 720, 721, 722, 723, 724, 725, 726, 727, 728, 729, 730, 731, 732, 733, 734, 735, 736, 737, 738, 739, 740, 741, 742, 743, 744, 745, 746 or 747, it being understood that the maximum number of disease-specific marker genes is limited by the number of disease-specific market genes that are described herein in each of the groups (i) to (xi).

As it will be detailed further in the specification, the comparison step c) is performed using a "control" expression value for each disease-specific marker gene that is tested. The said control expression value consists of the mean expression value for the said disease-specific marker gene that is found in individuals that are not affected with the thyroid disease to which a deregulation of the said marker gene is associated.

In some preferred embodiments, the comparison step c) consists of comparing the expression value of a plurality of marker genes that are known to be deregulated in patients affected with one of the thyroid diseases, selected from the thyroid diseases (i) to (xi), with the reference expression values for each of the said marker genes that are previously determined in patients that are not affected with the same thyroid disease, including healthy individuals that are not affected with any thyroid disease. The collection of expression values for the tested marker genes may also be termed an "expression profile" of these genes. In these preferred embodiments step c) encompasses comparing (i) the expression profile of the gene markers of interest that has been determined from the biological sample of the patient under testing with (ii) at least one reference expression profile of the same marker genes that has been previously determined from patients that are not affected with the said thyroid disease selected from the thyroid disease (i) to (xi). In certain of these preferred embodiments step c) consists of comparing (i) the expression profile of the gene markers of interest that has been determined from the biological sample of the patient under testing with (ii) at every reference expression profile of the same marker genes that has been previously determined from patients that are not affected with the said thyroid disease selected from the thyroid disease (i) to (xi), which may also include patients that are not effected with any thyroid disease. Then, at step d), a thyroid disease is predicted if the "experimental" expression profile that is determined for the patient tested is similar with one reference expression profile corresponding to patients affected with a specific thyroid disease selected from the thyroid diseases (i) to (xi). Then, at step e), the kind of specific thyroid disease is predicted, which consists of the thyroid disease for which the corresponding reference expression profile is the most close to the experimental expression profile that has been determined for the patient tested.

In certain embodiments of the method, the expression level value of a disease-specific marker gene can be provided as a relative expression level value. To determine a relative expression level value of a disease-specific marker gene, illustratively a disease-specific marker gene belonging to group (i) described herein, the level of expression of the said marker gene is previously determined for 10 or more samples of thyroid tissue originating from patients who are not affected with autoimmune thyroiditis (AT), prior to the determination of the expression level for the sample in question. The median expression level of the said disease-specific marker gene belonging to group (i), which has been determined in the larger number of samples, is determined and this median expression value is used as a baseline expression level, that may also be termed "control" value for the said disease-specific marker gene. The expression level of the said disease-specific marker gene determined for the test sample (absolute level of expression) is then divided by the median expression value obtained for that marker. This provides a relative expression level.

For a given disease-specific marker gene whose expression level is quantified at step b), a deregulated expression level is determined at step c) if there exists a relevant difference between (i) the expression value obtained at step b) and (ii) the reference control value. The said relevant difference may be any expression level difference that is found statistically significant, irrespective of the statistical method that is used. Generally, a relevant difference is found if, either :

(i) the deregulated expression of the disease-specific marker gene consists of an under-expression, as compared to the control expression value, the deregulated expression value being 0.98 fold or less the control expression value; or

(ii) the deregulated expression of the disease-specific marker gene consists of an over-expression, as compared to the control expression value with a $p$ value $< 0.05$.

At step d) of the *in vitro* prediction or diagnosis method according to the invention, the occurrence of a thyroid disease is predicted or diagnosed if a deregulated expression level of one or more disease-specific marker genes belonging to one group of marker genes selected from groups (i) to (xi) has been determined at step c).

Generally, if a deregulated expression level for one or more disease-specific marker genes is determined at step c), the said one or more disease-specific marker genes whose expression is deregulated all belong to the same group of marker genes, among groups (i) to (xi) described herein. Incidentally, errors may be introduced when performing steps b) or c) of the method, leading to the determination of a deregulated expression level of marker genes belonging to more than one group of marker genes, among groups (i) to (xi) described herein. The incidence of such artifacts may be easily avoided if, for each group (i) to (xi) for which marker genes expression level is quantified at step b), more than one disease-specific marker gene is included.

Thus, at step b), for each of the groups (i) to (xi) that is tested, preferably the expression level of 2 or more disease-specific marker genes, more preferably the expression level of 5 or more disease-specific marker genes, and most preferably the expression level of 12 or more disease-specific marker genes, is quantified, so as to lower the incidence of eventual technical artifacts which may occur when performing the *in vitro* prediction or diagnosis method according to the invention.

As already mentioned above, the *in vitro* prediction or diagnosis method according to the invention allows the prediction or the diagnosis of a thyroid disease selected from the groups (i) to (xi) of thyroid diseases described herein. In some embodiments, the said *in vitro* method allows the prediction or the diagnosis of all, or almost all, known thyroid diseases, if a simultaneous testing of marker genes included in a plurality of groups of disease-specific marker genes, among groups (i) to (xi), is performed, at step b) of the method.

Thus, in certain embodiments of the *in vitro* prediction or diagnosis method according to the invention, step b) consists of quantifying the expression level of marker genes included in more than two groups of disease-specific marker genes, selected from groups (i) to (xi) described herein. In certain embodiments of the method, step b) comprises quantifying the expression level of marker genes included in 3, 4, 5, 6, 7, 8, 9, 10 or 11 groups of disease-specific marker genes, selected from groups (i) to (xi) described herein.

The occurrence of every kind of thyroid disease, or at least almost every kind of thyroid disease, may be predicted or diagnosed when using the *in vitro* prediction or diagnosis method according to the invention, if, at step b), the expression level of one or more disease-specific marker genes in each of the groups (i) to (xi) of marker genes is quantified.

Thus, in certain preferred embodiments of the *in vitro* prediction or diagnosis method according to the invention, step b) comprises quantifying the expression level of disease-specific marker genes selected from each of groups (i) to (xi) described herein.

At step e) of the *in vitro* prediction or diagnosis method according to the invention, the kind of thyroid disease that is affecting the tested patient is determined by simply assessing to which group of disease-specific marker genes, among the groups (i) to (xi) described herein, belong the marker gene(s) for which a deregulated expression level has been determined at step c). Illustratively, if a deregulated expression level has been determined at step c) for one more disease-specific marker genes belonging to group (vi), then the occurrence of a follicular carcinoma (FTC) is predicted or diagnosed for the patient tested.

In the embodiments wherein, at step b) of the method, it is quantified the expression level of less than every one of the disease-specific marker genes belonging to a specific group, among groups (i) to (xi) described herein, then it is quantified preferably highly significant marker genes of the said specific group, so as to counterbalance the use of an eventual low number of disease-specific marker genes, for the said specific group.

In certain embodiments, if step b) of the method is performed by quantifying the expression level of less than 20 disease-specific marker genes in each group of disease-specific marker genes, among groups (i) to (xi), then the said disease-specific marker genes may be selected; in each of groups (i) to (xi), from the groups consisting of :

(i) marker genes specific for autoimmune thyroiditis (AT) that are contained as inserted DNA in the recombinant vectors selected from the group of vectors having the following IMAGE clone ID N° :

(i-1) 669510, 725096, 366100, 325155, 154172, 787938, 744055, 592276, 740941, 73778, 667174, 739193, 295412, 23827, 136686, 744385, 471641, 724276, 28584 and 268972,
(i-2) 43541, 687990, 365990, 562904, 725548, 365877, 742763, 180259, 2449149, 1089025, 713623, 382760, 258747, 743961, 174396, 724306, 1505308, 726454, 665452 and 665833,

(ii) marker genes specific for Grave's disease (GD that are contained as inserted DNA in the recombinant vectors selected from the group of vectors having the following IMAGE clone ID N° :

(ii-1) 25664, 868169, 731380, 1049033, 730942, 731616, 726578, 366388, 485872, 1662274, 2074228, 730926, 745106, 731021, 207920, 258865, 503189, 745490, 1103402 and 1911930,
(ii-2) 687990, 743961, 486493, 379279 and 667313,

(iii) marker genes specific for macrofollicular adenoma (FTA-a) that are contained as inserted DNA in the recombinant vectors selected from the group of vectors having the following IMAGE clone ID N° :

(iii-1) 2348237, 135379, 743851, 1712992, 376699, 1251197, 1174287, 173841, 486221, 265345, 666564, 252382, 173371, 731073, 253386, 1285305, 342700, 179214, 3054031 and 359797,
(iii-2) 745003, 1844968, 364324, 357201, 24392, 1420676, 687532, 724562, 729510, 725371, 24083, 738938, 730938, 148796, 137205, 174683, 21808, 3659591, 325155 and 666792,

(iv) marker genes specific for atypical follicular adenoma (FTA-aty) that are contained as inserted DNA in the recombinant vectors selected from the group of vectors having the following IMAGE clone ID N° :

(iv-1) 503874, 666128, 838611, 208991, 739097, 1089025, 725548, 739193, 155839, 28584, 202897, 743331, 743619, 725345, 345034, 235938, 729924, 681957, 30219 and 773330,
(iv-2) 50202, 738970, 128694, 741891, 666671, 366388, 146976, 687667, 587186, 667727, 669136, 744001, 666361, 668300, 713647, 2074228, 282587, 179288, 687270 and 726830,

(v) marker genes specific for microfollicular adenoma (FTA-b) that are contained as inserted DNA in the recombinant vectors selected from the group of vectors having the following IMAGE clone ID N° :

(v-1) 740931 ; 731751 ; 840687; 666128; 379540; 667259; 725585; 503874 ; 23612 ; : 3054031 ; 208097 ; 265558 ; 136976, 838611, 725707, 594279, 343987, 487474, 731128, 279172 and 773330,
(v-2) 724609, 365913, 666154, 267358, 665379, 667892, 251250, 713653, 365589, 1087348, 366042, 743146, 174654, 744657, 214008, 666007, 137005, 364436, 738558 and 146976,

(vi) marker genes specific for follicular carcinoma (FTC) that are contained as inserted DNA in the recombinant vectors selected from the group of vectors having the following IMAGE clone ID N° :

(vi-1) 501476, 309009, 739193, 251452, 681957, 739097, 364448, 469549, 202897, 195340, 731380, 2345206, 23765, 471641, 502244, 713653, 486189, 740604, 682207 and 740941,
(vi-2) 782688, 136014, 135980, 725335, 724150, 307094, 723752, 510318, 726578, 324079, 2139164, 143759, 739237, 152655, 136646, 309697, 384087, 365408, 376699 and 134671

(vii) marker genes specific for multinodular goitres (MNG) that are contained as inserted DNA in the recombinant vectors selected from the group of vectors having the following IMAGE clone ID N° :

(vii-1) 284828, 173841, 667527, 202897, 469549, 207920, 469647, 363955, 323438, 276658, 263251, 731277, 35628, 741474, 743394, 212366, 258865, 724014, 742837 and 1117183
(vii-2) 687625, 726782, 364324, 148796, 744505, 26164, 665668, 713837, 172828, 731726, 151247, 486356, 342181, 471641, 72744, 135689, 740941, 366032 and 154172,

(viii) marker genes specific for oncocytic adenoma (OTA) that are contained as inserted DNA in the recombinant vectors selected from the group of vectors having the following IMAGE clone ID N° :

(viii-1) 80688, 1870594, 731738, 1870594, 365348, 681875, 731751, 136114, 365161, 491004, 724366, 744983, 740718, 884365, 687381, 236399, 731726, 809719, 757248 and 172982,
(viii-2) 1662274, 145696, 730926, 731115, 155345, 376356, 666794, 739109, 179534, 628418, 741406, 485872, 232388, 667110, 724895, 33133, 730834, 743270, 731380, and 713913,

(ix) marker genes specific for atypical oncocytic adenoma (OTA-aty) that are contained as inserted DNA in the recombinant vectors selected from the group of vectors having the following IMAGE clone ID N° :

(ix-1) 745003, 740130, 471641, 682088, 731404, 2254555, 2381770, 214008, 300268, 2467442, 125552, 666517, 366159, 740927, 665784, 24392, 727457, 148796, 590264 and 665632,
(ix-2) 724556, 668329, 173200, 174654, 731227, 1624260, 743078, 739576, 3054031 and 119772,

(x) marker genes specific for oncocytic varcinoma (OTC) that are contained as inserted DNA in the recombinant vectors selected from the group of vectors having the following IMAGE clone ID N° :

(x-1) 727123, 724366, 366289, 471641, 501476, 744943, 2237263, 155839, 140347, 743774, 1716286, 365271, 236399, 731423, 252291, 812967, 739097, 471702, 1420676 and 739193,
(x-2) 731019, 366085, 258127, 382002, 714414, 486189, 725188, 1241974, 666703, 487152, 40304, 811024, 25865, 666279, 745133, 347362, 258966, 471664, 82687 and 740931,

(xi) marker genes specific for papillary carcinoma (PTC) that are contained as inserted DNA in the recombinant vectors selected from the group of vectors having the following IMAGE clone ID N° :

(xi-1) 726763, 739193, 28584, 739097, 25883, 1716286, 365045, 666367, 379200, 365271, 731227, 726760, 384404, 208991, 744943, 1846326, 364729, 730926, 795736 and 136686,
(xi-2) 758037, 1932168, 489945, 738970, 667756, 265558, 2113771, 365562, 1712992, 739237, 135539, 159809, 268972, 135303, 1916307, 356841, 682088, 665437, 744945 and 2728204,

In most preferred embodiments, if step b) of the method is performed by quantifying the expression level of highly significant disease-specific marker genes which are selected, in each of groups (i) to (xi), from the groups consisting of :

(i) marker genes specific for autoimmune thyroiditis (AT) that are contained as inserted DNA in the recombinant vectors selected from the group of vectors having the following IMAGE clone ID N° :

(i-2) 1088781 (SEQ ID N°3), 1089513 (SEQ ID N° 4), 1131054 (SEQ ID N° 5), 116906 (SEQ ID N° 6-7), 122874 (SEQ ID N° 8-9), 123950 (SEQ ID N° 10-11), 1251197 (SEQ ID N° 12), 1256485 (SEQ ID N° 13), 1286238 (SEQ ID N° 14), 1326169 (SEQ ID N° 15), 1352408 (SEQ ID N° 16), 140405 (SEQ ID N° 17-18), 1578721 (SEQ ID N° 19), 162246 (SEQ ID N° 20-21), 1705397 (SEQ ID N° 22), 1707527 (SEQ ID N° 23), 174396 (SEQ ID N° 24-25), 180259 (SEQ ID N° 26-27), 1870594 (SEQ ID N° 28), 188393 (SEQ ID N° 29-30), 1942634 (SEQ ID N° 31), 201467 (SEQ ID N° 32-33), 204686 (SEQ ID N° 34-35), 210587 (SEQ ID N° 36-37), 2238108 (SEQ ID N° 38), 245426 (SEQ ID N° 39-40), 250883 (SEQ ID N° 41-42), 2728204 (SEQ ID N° 43), 279470 (SEQ ID N° 44), 287843 SEQ ID N° 45), 3054031 (SEQ ID N° 46-47), 310519 (SEQ ID N° 48-49), 342256 (SEQ ID N° 50-51), 345034 (SEQ ID N° 52-53), 356841 (SEQ ID N° 54-55), 356960 (SEQ ID N° 56-57), 366289 (SEQ ID N° 58), 379200 (SEQ ID N° 59), 382760 (SEQ ID N° 60-61), 427786 (SEQ ID N° 62-63), 448163 (SEQ ID N° 64), 46991 (SEQ ID N° 65-66), 485803 (SEQ ID N° 67-68), 486401 (SEQ ID N° 69-70), 489326 SEQ ID N° 71-72), 503874 (SEQ ID N°73-74), 50491 (SEQ ID N° 75), 590264 (SEQ ID N° 76-77), 592598 (SEQ ID N° 78-79), 609631 (SEQ ID N° 80-81), 665452 (SEQ ID N° 82-83), 666410 (SEQ ID N° 84), 667239 (SEQ ID N° 85), 668584 (SEQ ID N° 86), 682207 (SEQ ID N° 87-88), 687990 (SEQ ID N° 89-90), 713623 (SEQ ID N° 91-92), 725473 (SEQ ID N° 93-94), 725548 (SEQ ID N° 95-96), 726454 (SEQ ID N° 97-98), 727496 (SEQ ID N° 99-100), 730037 (SEQ ID N° 101-102), 731751 (SEQ ID 107, 738944 (SEQ ID N° 108), 740347 (SEQ ID N° 109-110), 740931 (SEQ ID N° 111-112), 742696 (SEQ ID N° 113-114), 742853 (SEQ ID N° 115-116), 743246 (SEQ ID N° 117-118), 743367 (SEQ ID N° 119-120), 744911 (SEQ ID N° 121), 80688 (SEQ ID N° 122), 811024 (SEQ ID N° 123-124), 813174 (SEQ ID N° 125-126), 823680 (SEQ ID N° 127-128) and 884365 (SEQ ID N° 129),
(i-1) 1174287 (SEQ ID N° 130), 119772 (SEQ ID N° 131-132), 133531 (SEQ ID N° 133-134), 135689 (SEQ ID N° 135-136), 136919 (SEQ ID N° 137-138), 139242 (SEQ ID N° 139-140), 1420676 (SEQ ID N° 141), 148796 (SEQ ID N° 142), 154172 (SEQ ID N° 143), 155345 (SEQ ID N° 144-145), 1716286 (SEQ ID N° 146), 172982 (SEQ ID N° 147), 173841 (SEQ ID N° 148-149), 1846326 (SEQ ID N° 150), 1877668 (SEQ ID N° 151), 208991 (SEQ ID N° 152-153), 214008 (SEQ ID N° 154), 2266583 (SEQ ID N° 155), 22711 (SEQ ID N° 156), 2384812 (SEQ ID N° 157), 258127 (SEQ ID N° 158), 25883 (SEQ ID N° 159-160), 258865 (SEQ ID N° 161), 265558 (SEQ ID N° 162-163), 28584 (164-165), 295412 (166-167), 301122 (SEQ ID N° 168), 309645 (SEQ ID N° 169-170), 324079 (SEQ ID N° 171-172), 325155 (SEQ ID N° 173-174), 343987 (SEQ ID N° 175), 359051 (SEQ ID N° 176-177), 359461 (SEQ ID N° 178-179), 364324 (SEQ ID N° 180-181), 364412 (SEQ ID N° 182-183), 365045 (SEQ ID N° 184-185), 365121 (SEQ ID N° 186-187), 365271 (SEQ ID N° 188-189), 365589 (SEQ ID N° 190-191), 365913 (SEQ ID N° 192), 366032 (SEQ ID N° 193-194), 366042 (SEQ ID N° 195-196), 366100 (SEQ ID N° 197-198), 38403 (SEQ ID N° 199), 471641 (SEQ ID N° 200-201), 471664 (SEQ ID N° 202-203), 487152 (SEQ ID N° 204-205), 512910 (SEQ ID N° 206-207), 545475 (SEQ ID N° 208-209), 549054 (SEQ ID N° 210-211), 587186 (SEQ ID N° 212-213), 592276 (SEQ ID N° 214-215), 628418 (SEQ ID N° 216-217), 665086 (SEQ ID N° 218-219), 665376 SEQ ID N° 220-221), 666154 (SEQ ID N° 222-223), 666361 (SEQ ID N° 224-225), 667174 (SEQ ID N° 226), 667348 (SEQ ID N° 227-228), 667892 (SEQ ID N° 229), 668684 (SEQ ID N° 230), 681875 (SEQ ID N° 231-232), 687667 (SEQ ID N° 233), 714437 (SEQ ID N° 234-235), 724562 (SEQ ID N° 236), 724892 (SEQ ID N° 237-238), 725076 (SEQ ID N° 239-240), 726596 (SEQ ID N° 241-242), 727056 (SEQ ID N° 243-244), 727251 (SEQ ID N° 245), 727289 (SEQ ID N° 246-247), 72744 (SEQ ID N° 248-249), 729510 (SEQ ID N° 250-251), 730012 (SEQ ID N° 252-253), 730300 (SEQ ID N° 254), 731060 (SEQ ID N° 255-256), 731115 (SEQ ID N° 257-258), 731726 (SEQ ID N° 259-260), 738332 (SEQ ID N° 261-262), 738970 (SEQ ID N° 263), 739097 (SEQ ID N° 264-265), 739193 (SEQ ID N° 266-267), 740457 (SEQ ID N° 268-269), 740941 (SEQ ID N° 270), 743774 (SEQ ID N° 271), 744087 (SEQ ID N° 272), 744439 (SEQ ID N° 273), 745003 (SEQ ID N° 274), 745072 (SEQ ID N° 275), 745490 (SEQ ID N° 276), 782688 (SEQ ID N° 277-278) and 78353 (SEQ ID N° 279-280), 787938 (SEQ ID N° 281),

(ii) marker genes specific for Grave's disease (GD that are contained as inserted DNA in the recombinant vectors selected from the group of vectors having the following IMAGE clone ID N° :

(ii-1) 145696 (SEQ ID N° 282), 151247 (SEQ ID N° 283-284), 1662274 (SEQ ID N° 285), 173296 (SEQ ID N° 286-287), 341763 (SEQ ID N° 288-289), 486189 (SEQ ID N° 290-291), 594279 (SEQ ID N° 292), 730926 (SEQ ID N° 293-294), 731616 (SEQ ID N° 295) and 740620 (SEQ ID N° 296-297),

(iii) marker genes specific for macrofollicular adenoma (FTA-a) that are contained as inserted DNA in the recombinant vectors selected from the group of vectors having the following IMAGE clone ID N° :

(iii-2) 136114 (SEQ ID N° 298-299), 364575 (SEQ ID N° 300-301) and 366436 (SEQ ID N° 302-303), [Over-expressed]

(iii-1) 1188588 (SEQ ID N° 304-305), 137131 (SEQ ID N° 306-307), 137454 (SEQ ID N° 308), 173371 (SEQ ID N° 309-310), 235882 (SEQ ID N° 311-312), 235938 (SEQ ID N° 313-314), 253386 (SEQ ID N° 315-316), 265345 (SEQ ID N° 317-318), 276727 (SEQ ID N° 319), 42280 (SEQ ID N° 320-321), 486221 (SEQ ID N° 322-323), 666703 (SEQ ID N° 324-325), 682276 (SEQ ID N° 326), 725707 (SEQ ID N° 327-328), 726600 (SEQ ID N° 329-330), 741406 (SEQ ID N° 331-332), 743259 (SEQ ID N° 333-334), 744983 (SEQ ID N° 335) and 838611 (SEQ ID N° 336-337),

(iv) marker genes specific for atypical follicular adenoma (FTA-aty) that are contained as inserted DNA in the recombinant vectors selected from the group of vectors having the following IMAGE clone ID N° :

(iv-2) 208097 (SEQ ID N° 338-339), 669136 (SEQ ID N° 340-341) and 687468 (SEQ ID N° 342-343),
(iv-1) 1089025 (SEQ ID N° 344), 1536698 (SEQ ID N° 345), 155839 (SEQ ID N° 346-347), 238840 (SEQ ID N° 348-349), 30219 (SEQ ID N° 350-351), 33133 (SEQ ID N° 352-353), 378813 (SEQ ID N° 354), 379517 (SEQ ID N° 355), 379540 (SEQ ID N° 356), 40304 (SEQ ID N° 357), 665784 (SEQ ID N°358-359), 666128 (SEQ ID N° 360-361), 667527 (SEQ ID N° 362), 724014 SEQ ID N° 363-364), 739109 (SEQ ID N° 365), 740105 (SEQ ID N° 366), 740604 (SEQ ID N° 367-368) and 743270 (SEQ ID N° 369-370),

(v) marker genes specific for microfollicular adenoma (FTA-b) that are contained as inserted DNA in the recombinant vectors selected from the group of vectors having the following IMAGE clone ID N° :

(v-1) 1641901 (SEQ ID N° 371), 23612 (SEQ ID N° 372-373), 236399 (SEQ ID N° 374-375), 491004 (SEQ ID N° 376-377), 667365 (SEQ ID N° 378-379), 724366 (SEQ ID N° 380-381), 725877 (SEQ ID N° 382-383), 731128 (SEQ ID N° 384-385), 731738 (SEQ ID N° 386-387) and 809719 (SEQ ID N° 388-389),

(vi) marker genes specific for follicular carcinoma (FTC) that are contained as inserted DNA in the recombinant vectors selected from the group of vectors having the following IMAGE clone ID N° :

(vi-1) 179334 (SEQ ID N° 390-391), 713671 (SEQ ID N° 392-393), 726684 (SEQ ID N° 394-395) and 757248 (SEQ ID N° 396)

(vii) marker genes specific for multinodular goitres (MNG) that are contained as inserted DNA in the recombinant vectors selected from the group of vectors having the following IMAGE clone ID N° :

(vii-2) 194302 (SEQ ID N° 397-398),
(vii-1) 1012990 (SEQ ID N° 399), 208439 (SEQ ID N° 400-401) and 36521 (SEQ ID N° 402-403)

(viii) marker genes specific for oncocytic adenoma (OTA) that are contained as inserted DNA in the recombinant vectors selected from the group of vectors having the following IMAGE clone ID N° :

(viii-2) 430471 (SEQ ID N° 404)
(viii-1) 112819 (SEQ ID N° 405), 180082 (SEQ ID N° 406-407), 365348 (SEQ ID N° 408-409), 366115 (SEQ ID N° 410-411), 667310 (SEQ ID N° 412-413), 713831 (SEQ ID N° 414-415), 726092 (SEQ ID N° 416-417), 730606 (SEQ ID N° 418-419) and 743125 (SEQ ID N° 420)

The sequence references (SEQ IDs) consist of nucleotide sequences that are comprised in the corresponding disease-specific marker genes. For each disease-specific marker gene that is listed above, a reference to (i) the 3'-end sequence, (ii) the 5'-end sequence or (iii) both the 3'-end sequence and the 5'-end sequence is given in parentheses.
Indeed, the sequences that are referred to above may be used as such as nucleic acid primers or probes specific for a given disease-specific marker gene. These sequences may also be used to design other nucleic acid probes or primers specific for a given disease-specific marker gene.
These sequences may also be used to amplify the nucleic acid insert of the corresponding IMAGE clone vector, so as to generate a suitable amount of a given disease-specific marker gene nucleic acid.

### *General methods for quantifying the expression level* **of** *disease-specific marker genes*

Any one of the methods known by the one skilled in the art for quantifying a nucleic acid biological marker encompassed herein may be used for performing the *in vitro* prediction or diagnosis method of the invention. Thus any one of the standard and non-standard (emerging) techniques well known in the art for detecting and quantifying a protein or a nucleic acid in a sample can readily be applied.

Such techniques include detection and quantification of nucleic acid biological markers with nucleic probes or primers.

The expression level of a disease-specific marker gene described herein may be quantified by any one of a wide variety of well known methods for detecting expression of a transcribed nucleic acid. Non-limiting examples of such methods include nucleic acid hybridisation methods, nucleic acid reverse transcription methods, and nucleic acid amplification methods.

In certain embodiments, the expression level of a disease-specific marker gene is assessed by preparing mRNA/cDNA (i.e. a transcribed polynucleotide) from cells originating from a thyroid tissue sample of a patient to be tested, and by hybridising the mRNA/cDNA with a reference polynucleotide which is a complement of a marker nucleic acid, or a fragment thereof, the said marker nucleic acid being comprised in the expression product of a disease-specific marker gene included in one of groups (i) to (xi) described herein. cDNA can, optionally, be amplified using any of a variety of polymerase chain reaction methods prior to hybridisation with the reference polynucleotide.

In some preferred embodiments of the *in vitro* prediction or diagnosis method according to the invention, step b) of expression level quantification of two or more disease-specific marker genes is performed using DNA microarrays. Illustratively, according to this preferred embodiment, a mixture of transcribed polynucleotides obtained from the thyroid tissue sample, or alternatively a mixture of the corresponding cDNAs, is contacted with a substrate having fixed thereto a plurality of polynucleotides, each of these polynucleotides consisting of a polynucleotide complementary to, or homologous with, at least a portion (e.g. at least 7, 10, 15, 20, 25, 30, 40, 50, 100, 500, or more consecutive nucleotide residues) of a disease-specific marker gene. If polynucleotides complementary to or homologous with are differentially detectable on the substrate (e.g. detectable using different chromophores or fluorophores, or fixed to different selected positions), then the levels of expression of a plurality of disease-specific marker genes can be quantified simultaneously using a single substrate (e.g. a "gene chip" microarray of polynucleotides fixed at selected positions). When a method of assessing marker expression is used which involves hybridisation of one nucleic acid with another, it is preferred that the hybridisation be performed under stringent hybridisation conditions.

In certain embodiments of the *in vitro* prediction or diagnosis method according to the invention, step b) comprises the steps of :

b1) providing two or more sets of nucleic acids, each nucleic acid contained in a set hybridizing specifically with a nucleic acid expression product of a disease-specific marker gene comprised in one of groups (i) to (xi);
b2) reacting the sets of nucleic acids provided at step b1) with nucleic acid expression products that are previously extracted from the thyroid tissue sample provided at step a);
b3) detecting and quantifying the nucleic acid complexes formed between (i) the sets of nucleic acids provided at step b1) and (ii) the nucleic acid expression products that are extracted from the thyroid tissue sample provided at step a);

The nucleic acids that are provided at step b1) may also be conventionally termed nucleic acid probes, each nucleic acid probe having the ability to specifically hybridize with an expression product (mRNA or cDNA) from a disease-specific marker gene selected from the group consisting of the disease-specific marker genes comprised in groups (i) to (xi) described in the present specification.

A "set" of nucleic acids that is provided at step b1) consists of one or more nucleic acids (e.g. nucleic acid probes) that all hybridize with an expression product from the same disease-specific marker gene. In the embodiments wherein a set of nucleic acids comprises two or more nucleic acids, the said nucleic acids may be identical or distinct. In the embodiments wherein a set of nucleic acids comprises two or more distinct nucleic acids, the said distinct nucleic acids preferably hybridize with distinct nucleic acid portions, most preferably non-overlapping portions, of the expression product of the same disease-specific marker gene.

As it will be described in detail below, preferred embodiments of steps b1) to b3) are performed with DNA microarrays.

Thus, in most preferred embodiments of step b) of the *in vitro* prediction or diagnosis method according to the invention, the expression level quantification of the disease-specific marker genes is performed by using suitable DNA microarrays. In such a marker detection/quantification format, the mRNA is immobilised on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the markers of the present invention. Specific hybridization technology which may be practiced to generate the expression profiles employed in the subject methods includes the technology described in U.S. Pat. Nos. 5,143,854; 5,288,644; 5,324,633; 5,432,049; 5,470,710; 5,492,806; 5,503,980; 5,510,270; 5,525,464; 5,547,839; 5,580,732; 5,661,028; 5,800,992; the disclosures of which are herein incorporated by reference; as well as WO 95/21265; WO 96/31622; WO 97/10365; WO 97/27317; EP 373 203; and EP 785 280. In these methods, an array of "probe" nucleic acids that includes a probe for each of the phenotype determinative genes whose expression is being assayed is contacted with target nucleic acids as described above. Contact is carried out under hybridization conditions, e.g., stringent hybridization conditions as described above, and unbound nucleic acid is then removed. The resultant pattern of hybridized nucleic acid provides information regarding expression for each of the genes that have been probed, where the expression information is in terms of whether or not the gene is expressed and, typically, at what level, where the expression data, i.e., expression profile, is both qualitative and quantitative.

Suitable carriers or solid phase supports for such assays include any material capable of binding the class of molecule to which the marker or probe belongs. Well-known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above mentioned approaches, the non-immobilised component is added to the solid phase upon which the second component is anchored. After the reaction is complete, uncomplexed components may be removed (e.g., by washing) under conditions such that any complexes formed will remain immobilised upon the solid phase. The detection of marker/probe complexes anchored to the solid phase can be accomplished in a number of methods outlined herein.

In a preferred embodiment, the probe, when it is the unanchored assay component, can be labelled for the purpose of detection and readout of the assay, either directly or indirectly, with detectable labels discussed herein and which are well-known to one skilled in the art.

In another embodiment, determination of the ability of a probe to recognise a marker can be accomplished without labelling either assay component (probe or marker) by utilising a technology such as real-time Biomolecular Interaction Analysis (BIA) (see, e.g., Sjolander, S. and Urbaniczky, C., 1991, Anal. Chem. 63:2338-2345 and Szabo et al., 1995, Curr. Opin. Struct. Biol. 5:699-705). As used herein, "BIA" or "surface plasmon resonance" is a technology for studying biospecific interactions in real time, without labelling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, in another embodiment, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas, G., and Minton, A. P., 1993, Trends Biochem Sci. 18(8):284-7). Standard chromatographic techniques may also be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, e.g., Heegaard, N. H., 1998, J. Mol. Recognit. Winter 11(1-6):141-8; Hage, D. S., and Tweed, S. A. J Chromatogr B Biomed Sci Appl 1997 Oct. 10;699(1-2):499-525). Gel electrophoresis may also be employed to separate complexed assay components from unbound components (see, e.g., Ausubel et al., ed., Current Protocols in Molecular Biology, John Wiley & Sons, New York, 1987-1999). In this technique, protein or nucleic acid complexes are separated based on size or charge, for example. In order to maintain the binding interaction during the electrophoretic process, non-denaturing gel matrix materials and conditions in the absence of reducing agent are typically preferred. SELDI-TOF technique may also be employed on matrix or beads coupled with active surface, or not, or antibody coated surface, or beads.

Appropriate conditions to the particular assay and components thereof will be well known to one skilled in the art.

As already mentioned above, preferred expression quantification methods use isolated RNA. For *in vitro* methods, any RNA isolation technique that does not select against the isolation of mRNA can be utilised for the purification of RNA from thyroid tissue sample (see, e.g., Ausubel et al., ed., Current Protocols in Molecular Biology, John Wiley & Sons, New York 1987-1999). Additionally, large numbers of tissue samples can readily be processed using techniques well known to those of skill in the art, such as, for example, the single-step RNA isolation process of Chomczynski (1989, U.S. Pat. No. 4,843,155).

An alternative method for determining the level of mRNA marker in a sample involves the process of nucleic acid amplification, e.g., by rtPCR (the experimental embodiment set forth in Mullis, 1987, U.S. Pat. No. 4,683,202), ligase chain reaction (Barany, 1991, Proc. Natl. Acad. Sci. USA, 88:189-193), self sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technology 6:1197), rolling circle replication (Lizardi et al., U.S. Pat. No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

As an alternative to making determinations based on the absolute expression level of the marker, determinations may be based on the normalised expression level of the marker. Expression levels are normalised by correcting the absolute expression level of a marker by comparing its expression to the expression of a gene that is not a marker, e.g., a housekeeping gene that is constitutively expressed. Suitable genes for normalisation include housekeeping genes such as the actin gene and the ribosomal 18S gene. This normalisation allows the comparison of the expression level of one or more tissue-specific biological marker of interest in one sample.

The most preferred methods for quantifying a biological marker for the purpose of carrying out the metastasis prediction method of the invention are described hereunder.

***Quantifying the expression level of disease-specific marker genes by cDNA microarrays***

According to this embodiment, a microarray may be constructed based on the disease-specific marker genes that are disclosed throughout the present specification. Preferably, oligonucleotide probes that specifically hybridize with the expression products (mRNA or cDNA) from each of the disease-specific marker genes tested are immobilized on a solid support, most preferably on an ordered arrangement, so as to manufacture the DNA microarray. These marker gene-specific detection probes should be designed and used in conditions such that only nucleic acids having a disease-specific marker gene sequence may hybridize and give a positive result.

Most existing microarrays, such as those provided by Affymetrix (California), may be used with the present invention.

One of skill in the art will appreciate that an enormous number of array designs are suitable. The high density array will typically include a number of probes that specifically hybridize to the sequences of interest. See WO 99/32660 for methods of producing probes for a given gene or genes. In a preferred embodiment, the array will include one or more control probes.

*Nucleic acid probes immobilized on the microarray devices*

High density array chips include « test probes » that specifically hybridize with mRNAs or cDNAs consisting of the products of expression of the meatstasis-specific biological markers that are described herein.

Test probes may be oligonucleotides that range from about 5 to about 500 or about 5 to about 50 nucleotides, more preferably from about 10 to about 40 nucleotides and most preferably from about 15 to about 40 nucleotides in length. In other particularly preferred embodiments, the probes are about 20 or 25 nucleotides in length. In another preferred embodiment, test probes are double or single strand DNA sequences. DNA sequences may be isolated or cloned from natural sources or amplified from natural sources using natural nucleic acid as templates. These probes have sequences complementary to particular subsequences of the metastasis-specific markers whose expression they are designed to detect.

In addition to test probes that bind the target nucleic acid(s) of interest, the high density array can contain a number of control probes. The control probes fall into three categories referred to herein as normalization controls; expression level controls; and mismatch controls. Normalization controls are oligonucleotide or other nucleic acid probes that are complementary to labeled reference oligonucleotides or other nucleic acid sequences that are added to the nucleic acid sample. The signals obtained from the normalization controls after hybridization provide a control for variations in hybridization conditions, label intensity, "reading" efficiency and other factors that may cause the signal of a perfect hybridization to vary between arrays. In a preferred embodiment, signals (e.g. fluorescence intensity) read from all other probes in the array are divided by the signal (, fluorescence intensity) from the control probes thereby normalizing the measurements. Virtually any probe may serve as a normalization control. However, it is recognized that hybridization efficiency varies with base composition and probe length. Preferred normalization probes are selected to reflect the average length of the other probes present in the array; however, they can be selected to cover a range of lengths. The normalization control(s) can also be selected to reflect the (average) base composition of the other probes in the array, however in a preferred embodiment, only one or a few probes are used and they are selected such that they hybridize well (i.e., no secondary structure) and do not match any target-specific probes. Expression level controls are probes that hybridize specifically with constitutively expressed genes in the biological sample. Virtually any constitutively expressed gene provides a suitable target for expression level controls. Typical expression level control probes have sequences complementary to subsequences of constitutively expressed "housekeeping genes" including the .beta.-actin gene, the transferrin receptor gene, and the GAPDH gene. Mismatch controls may also be provided for the probes to the target genes, for expression level controls or for normalization controls. Mismatch controls are oligonucleotide probes or other nucleic acid probes identical to their corresponding test or control probes except for the presence of one or more mismatched bases. A mismatched base is a base selected so that it is not complementary to the corresponding base in the target sequence to which the probe would otherwise specifically hybridize. One or more mismatches are selected such that under appropriate hybridization conditions (e.g., stringent conditions) the test or control probe would be expected to hybridize with its target sequence, but the mismatch probe would not hybridize (or would hybridize to a significantly lesser extent). Preferred mismatch probes contain a central mismatch. Thus, for example, where a probe is a twenty-mer, a corresponding mismatch probe may have the identical sequence except for a single base mismatch (e.g., substituting a G, a C or a T for an A) at any of positions 6 through 14 (the central mismatch). Mismatch probes thus provide a control for non-specific binding or cross hybridization to a nucleic acid in the sample other than the target to which the probe is directed. Mismatch probes also indicate whether hybridization is specific or not.

*Solid Supports for DNA microarrays*

Solid supports containing oligonucleotide probes for differentially expressed genes can be any solid or semisolid support material known to those skilled in the art. Suitable examples include, but are not limited to, membranes, filters, tissue culture dishes, polyvinyl chloride dishes, beads, test strips, silicon or glass based chips and the like. Suitable glass wafers and hybridization methods are widely available. Any solid surface to which oligonucleotides can be bound, either directly or indirectly, either covalently or non-covalently, can be used. In some embodiments, it may be desirable to attach some oligonucleotides covalently and others non-covalently to the same solid support. A preferred solid support is a high density array or DNA chip. These contain a particular oligonucleotide probe in a predetermined location on the array. Each predetermined location may contain more than one molecule of the probe, but each molecule within the predetermined location has an identical sequence. Such predetermined locations are termed features. There may be, for example, from 2, 10, 100, 1000 to 10,000, 100,000 or 400,000 of such features on a single solid support. The solid support or the area within which the probes are attached may be on the order of a square centimeter. Methods of forming high density arrays of oligonucleotides with a minimal number of synthetic steps are known. The oligonucleotide analogue array can be synthesized on a solid substrate by a variety of methods, including, but not limited to, light-directed chemical coupling, and mechanically directed coupling (see U.S. Pat. No. 5,143,854 to Pirrung et al.; U.S. Pat. No. 5,800,992 to Fodor et al.; U.S. Pat. No. 5,837,832 to Chee et al.

In brief, the light-directed combinatorial synthesis of oligonucleotide arrays on a glass surface proceeds using automated phosphoramidite chemistry and chip masking techniques. In one specific implementation, a glass surface is derivatized with a silane reagent containing a functional group, e.g., a hydroxyl or amine group blocked by a photolabile protecting group. Photolysis through a photolithographic mask is used selectively to expose functional groups which are then ready to react with incoming 5' photoprotected nucleoside phosphoramidites. The phosphoramidites react only with those sites which are illuminated (and thus exposed by removal of the photolabile blocking group). Thus, the phosphoramidites only add to those areas selectively exposed from the preceding step. These steps are repeated until the desired array of sequences has been synthesized on the solid surface. Combinatorial synthesis of different oligonucleotide analogues at different locations on the array is determined by the pattern of illumination during synthesis and the order of addition of coupling reagents.

In addition to the foregoing, methods which can be used to generate an array of oligonucleotides on a single substrate are described in WO 93/09668 to Fodor et al. High density nucleic acid arrays can also be fabricated by depositing premade or natural nucleic acids in predetermined positions. Synthesized or natural nucleic acids are deposited on specific locations of a substrate by light directed targeting and oligonucleotide directed targeting. Another embodiment uses a dispenser that moves from region to region to deposit nucleic acids in specific spots.

Oligonucleotide probe arrays for expression monitoring can be made and used according to any techniques known in the art (see for example, Lockhart et al., Nat. Biotechnol. 14, 1675-1680 (1996); McGall et al., Proc. Nat. Acad. Sci. USA 93, 13555-13460 (1996). Such probe arrays may contain at least two or more oligonucleotides that are complementary to or hybridize to two or more of the genes described herein. Such arrays may also contain oligonucleotides that are complementary to or hybridize to at least 3, 4, 5, 6, 7, 8, 9, 10, 20, 30, 50, 70, 80, 90, 100, 110, 120, 130, 140, 150, 200, 210, 220, 230, 240, 250, 260, 270, 280, 290, 300, 350, 400, 450, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000 or more of the disease-specific marker genes described therein.

*Hybridization*

Nucleic acid hybridization simply involves contacting a probe and target nucleic acid under conditions where the probe and its complementary target can form stable hybrid duplexes through complementary base pairing (see WO 99/32660 to Lockhart). The nucleic acids that do not form hybrid duplexes are then washed away leaving the hybridized nucleic acids to be detected, typically through detection of an attached detectable label. It is generally recognized that nucleic acids are denatured by increasing the temperature or decreasing the salt concentration of the buffer containing the nucleic acids. Under low stringency conditions (e.g., low temperature and/or high salt) hybrid duplexes (e.g., DNA-DNA, RNA-RNA or RNA-DNA) will form even where the annealed sequences are not perfectly complementary. Thus, specificity of hybridization is reduced at lower stringency. Conversely, at higher stringency (e.g., higher temperature or lower salt) successful hybridization requires fewer mismatches. One of skill in the art will appreciate that hybridization conditions may be selected to provide any degree of stringency. In a preferred embodiment, hybridization is performed at low stringency, in this case in 6.times.SSPE-T at 37.degree. C. (0.005% Triton x-100) to ensure hybridization and then subsequent washes are performed at higher stringency (e.g., 1.times.SSPE-T at 37.degree. C.) to eliminate mismatched hybrid duplexes. Successive washes may be performed at increasingly higher stringency (e.g. down to as low as 0.25.times.SSPE-T at 37.degree. C. to 50.degree. C. until a desired level of hybridization specificity is obtained. Stringency can also be increased by addition of agents such as formamide. Hybridization specificity may be evaluated by comparison of hybridization to the test probes with hybridization to the various controls that can be present (e.g., expression level controls, normalization controls, mismatch controls, etc.).

In general, there is a tradeoff between hybridization specificity (stringency) and signal intensity. Thus, in a preferred embodiment, the wash is performed at the highest stringency that produces consistent results and that provides a signal intensity greater than approximately 10% of the background intensity. The hybridized array may be washed at successively higher stringency solutions and read between each wash. Analysis of the data sets thus produced will reveal a wash stringency above which the hybridization pattern is not appreciably altered and which provides adequate signal for the particular oligonucleotide probes of interest.

*Signal Detection*

The hybridized nucleic acids are typically detected by detecting one or more labels attached to the sample nucleic acids. The labels may be incorporated by any of a number of means well known to those of skill in the art (see WO 99/32660 to Lockhart). Any suitable methods can be used to detect one or more of the markers described herein. For example, gas phase ion spectrometry can be used. This technique includes, e.g., laser desorption/ionization mass spectrometry. In some embodiments, the sample can be prepared prior to gas phase ion spectrometry, e.g., pre-fractionation, two-dimensional gel chromatography, high performance liquid chromatography, etc. to assist detection of markers.

***Quantifying the expression level of disease-specific marker genes by nucleic acid amplification***

In certain embodiments, the expression level of a disease-specific marker gene, or of a set of disease-specific marker genes, may be quantified with any one of the nucleic acid amplification methods known in the art.

The polymerase chain reaction (PCR) is a highly sensitive-and powerful method for such biological markers quantification

For performing any one of the nucleic acid amplification method that is appropriate for quantifying a biological marker when performing the *in vitro* prediction or diagnosis method of the invention, a pair of primers that specifically hybridise with the target mRNA or with the target cDNA is required.

A pair of primers that specifically hybridise with the target nucleic acid biological marker of interest may be designed by any one of the numerous methods known in the art. Illustratively, primers that specifically hybridize with a disease-specific marker gene described herein may be easily designed by the one skilled in the art, on the basis of the nucleic acid sequence of the said disease-specific marker gene, like it is found for example in the IMAGE Consortium database, or alternatively in the HGCN database, or also after simply sequencing the DNA insert contained in the corresponding IMAGE phagemid vector.

In certain embodiments, for each of the biological markers of the invention, at least one pair of specific primers, as well as the corresponding detection nucleic acid probe, is already referenced and entirely described in the public "Quantitative PCR primer database", notably at the following Internet address : http://lpgws.nci.nih.gov/cgi-bin/PrimerViewer.

In other embodiments, a specific pair of primers may be designed using the method disclosed in the US Patent n° US 6,892,141 to Nakae et al., the entire disclosure of which is herein incorporated by reference.

Many specific adaptations of the PCR technique are known in the art for both qualitative and quantitative detection purposes. In particular, methods are known to utilise fluorescent dyes for detecting and quantifying amplified PCR products. In situ amplification and detection, also known as homogenous PCR, have also been previously described. See e.g. Higuchi et al., (Kinetics PCR Analysis: Real-time Monitoring of DNA Amplification Reactions, Bio/Technology, Vol 11, pp 1026-1030 (1993)), Ishiguro et al., (Homogeneous quantitative Assay of Hepatitis C Virus RNA by Polymerase Chain Reaction in the Presence of a Fluorescent Intercalater, Anal. Biochemistry 229, pp 20-213 (1995)), and Wittwer et al., (Continuous Fluorescence Monitoring of Rapid cycle DNA Amplification, Biotechniques, vol.22, pp 130-138 (1997.))

A number of other methods have also been developed to quantify nucleic acids (Southern, E. M., J. Mol. Biol., 98:503-517, 1975; Sharp, P. A., et al., Methods Enzymol. 65:750-768, 1980; Thomas, P. S., Proc. Nat. Acad. Sci., 77: 5201-5205, 1980). More recently, PCR and RT-PCR methods have been developed which are capable of measuring the amount of a nucleic acid in a sample. One approach, for example, measures PCR product quantity in the log phase of the reaction before the formation of reaction products plateaus (Kellogg, D. E., et al., Anal. Biochem. 189:202-208 (1990); and Pang, S., et al., Nature 343:85-89 (1990)). A gene sequence contained in all samples at relatively constant quantity is typically utilised for sample amplification efficiency normalisation. This approach, however, suffers from several drawbacks. The method requires that each sample have equal input amounts of the nucleic acid and that the amplification efficiency between samples be identical until the time of analysis. Furthermore, it is difficult using the conventional methods of PCR quantitation such as gel electrophoresis or plate capture hybridisation to determine that all samples are in fact analysed during the log phase of the reaction as required by the method.

Another method called quantitative competitive (QC)-PCR, as the name implies, relies on the inclusion of an internal control competitor in each reaction (Becker-Andre, M., Meth. Mol. Cell Biol. 2:189-201 (1991); Piatak, M. J., et al., BioTechniques 14:70-81 (1993); and Piatak, M. J., et al., Science 259:1749-1754 (1993)). The efficiency of each reaction is normalised to the internal competitor. A known amount of internal competitor is typically added to each sample. The unknown target PCR product is compared with the known competitor PCR product to obtain relative quantitation. A difficulty with this general approach lies in developing an internal control that amplifies with the same efficiency of the target molecule.

For instance, the nucleic acid amplification method that is used may consist of Real-Time quantitative PCR analysis.

Real-time or quantitative PCR (QPCR) allows quantification of starting amounts of DNA, cDNA, or RNA templates. QPCR is based on the detection of a fluorescent reporter molecule that increases as PCR product accumulates with each cycle of amplification. Fluorescent reporter molecules include dyes that bind double-stranded DNA (i.e. SYBR Green I) or sequence-specific probes (i.e. Molecular Beacons or TaqMan® Probes).

Preferred nucleic acid amplification methods are quantitative PCR amplification methods, including multiplex quantitative PCR method such as the technique disclosed in the published US patent Application n° US 2005/0089862, to Therianos et al., the entire disclosure of which is herein incorporated by reference.

Illustratively, for quantifying biological markers of the invention, tumor tissue samples are snap-frozen shortly after biopsy collection. Then, total RNA from a "thyroid tissue sample" is isolated and quantified. Then, each sample of the extracted and quantified RNA is reverse-transcribed and the resulting cDNA is amplified by PCR, using a pair of specific primers for each biological marker that is quantified. Control pair of primers are simultaneously used as controls, such as pair of primers that specifically hybridise with TBP cDNA, 18S cDNA and GADPH cDNA, or any other well known "housekeeping" gene.

Illustrative embodiments of quantification of the expression level of the disease-specific marker genes described herein are disclosed in the examples herein.

### *Kits for predicting or diagnosing the occurrence* of a *thyroid disease*

The invention also relates to a kit for the *in vitro* prediction or diagnosis of the occurrence of a thyroid disease in a patient (e.g. in a thyroid tissue sample previously collected from a patient to be tested). The kit comprises a plurality of reagents, each of which is capable of binding specifically with a nucleic acid that is comprised in an expression product (mRNA or cDNA) from a disease-specific marker gene selected from the disease-specific marker genes included in groups (i) to (xi) described herein.

Suitable reagents for binding with a marker nucleic acid (e.g. a mRNA, a spliced mRNA, a cDNA, or the like) include complementary nucleic acids. For example, the nucleic acid reagents may include oligonucleotides (labelled or non-labelled) fixed to a substrate, labelled oligonucleotides not bound with a substrate, pairs of PCR primers, molecular beacon probes, and the like.

Another object of the present invention consists of a kit for the *in vitro* prediction or diagnosis of the occurrence of a thyroid disease in a patient, which kit comprises means for quantifying the expression level of two or more disease-specific marker genes that are indicative of the risk of occurrence of, or of the occurrence of, a specific thyroid disease, wherein the said two or more disease-specific marker genes are selected from two or more of the groups of marker genes consisting of :

(i) marker genes specific for autoimmune thyroiditis (AT) that are contained as inserted DNA in the recombinant vectors selected from the group of vectors having the following IMAGE clone ID N° already specified previously in the present specification;
(ii) marker genes specific for Grave's disease (GD that are contained as inserted DNA in the recombinant vectors selected from the group of vectors having the following IMAGE clone ID N° already specified previously in the present specification;
(iii) marker genes specific for macrofollicular adenoma (FTA-a) that are contained as inserted DNA in the recombinant vectors selected from the group of vectors having the following IMAGE clone ID N° already specified previously in the present specification;
(iv) marker genes specific for atypical follicular adenoma (FTA-aty) that are contained as inserted DNA in the recombinant vectors selected from the group of vectors having the following IMAGE clone ID N° already specified previously in the present specification;
(v) marker genes specific for microfollicular adenoma (FTA-b) that are contained as inserted DNA in the recombinant vectors selected from the group of vectors having the following IMAGE clone ID N° already specified previously in the present specification;
(vi) marker genes specific for follicular carcinoma (FTC) that are contained as inserted DNA in the recombinant vectors selected from the group of vectors having the following IMAGE clone ID N° already specified previously in the present specification;
(vii) marker genes specific for multinodular goitres (MNG) that are contained as inserted DNA in the recombinant vectors selected from the group of vectors having the following IMAGE clone ID N° already specified previously in the present specification;
(viii) marker genes specific for oncocytic adenoma (OTA) that are contained as inserted DNA in the recombinant vectors selected from the group of vectors having the following IMAGE clone ID N° already specified previously in the present specification;
(ix) marker genes specific for atypical oncocytic adenoma (OTA-aty) that are contained as inserted DNA in the recombinant vectors selected from the group of vectors having the following IMAGE clone ID N° already specified previously in the present specification;
(x) marker genes specific for oncocytic varcinoma (OTC) that are contained as inserted DNA in the recombinant vectors selected from the group of vectors having the following IMAGE clone ID N° already specified previoulsy in the present specification; and
(xi) marker genes specific for papillary carcinoma (PTC) that are contained as inserted DNA in the recombinant vectors selected from the group of vectors having the following IMAGE clone ID N° already specified previously in the present specification.

The present invention also encompasses various alternative embodiments of the said prediction or diagnosis kit, wherein the said kit comprises combination of marker quantification means, for quantifying the expression level of various combinations of the disease-specific marker genes that are described in the present specification.

Most preferably, a prediction or diagnosis kit according to the invention consists of a DNA microarray comprising probes hybridizing to the nucleic acid expression products (mRNAs or cDNAs) of the disease-specific gene markers described herein.

This invention also pertains to a collection of nucleic acids that is useful for predicting or diagnosing the occurrence of a thyroid disease in a patient, wherein the said collection of nucleic acids comprises a combination of at least two distinct nucleic acids, each distinct nucleic acid hybridizing specifically with a disease-specific marker gene described herein, belonging to a group of disease-specific marker genes selected from groups (i) to (xi).

In certain embodiments of the said collection of nucleic acids, each of the said nucleic acids consists of an IMAGE vector selected among the IMAGE vectors described herein, each IMAGE vector being used as a nucleic acid probe that specifically hybridises with an expression product of a given disease-specific gene.

In certain other embodiments of the said collection of nucleic acids, each of the said nucleic acid is selected from the group of nucleic acids consisting of SEQ ID N°3 to SEQ ID N° 420 that are described elsewhere in the present specification.

Another object of the present invention consists of a kit for monitoring the effectiveness of a therapeutic treatment of a patient affected with a thyroid disease, and especially a thyroid tumor, with a pharmaceutical agent, which kit comprises means for quantifying one or more disease-specific marker genes that are indicative of the occurrence of a specific thyroid disease selected in the suitable specific group of disease-specific marker genes, among groups (i) to (xi) described herein. Illustratively, if the patient to be monitored has been diagnosed as being affected with autoimmune thyroiditis, then the monitoring kit comprises means (e.g. specific nucleic acid probes) for quantifying the expression level of two or more disease-specific marker genes belonging to group (i) of marker genes that is described herein.

The present invention also encompasses various alternative embodiments of the said monitoring kit, wherein the said monitoring kit comprises combination of marker detection and/or marker quantification means, for detecting and/or quantifying various combinations of the disease-specific marker genes described in the present specification.

In preferred embodiments, a kit according to the invention comprises (i) a combination or a set of specific nucleic acid probes or (ii) a combination or a set of nucleic acid primers, each kind of probes of primers hybridising specifically with the expression product (mRNA or cDNA) of a disease-specific marker gene selected from the disease-specific marker genes described herein.

In certain embodiments wherein nucleic acid probes are used, including when these probes are immobilised in an arrayed ordering on a solid support, the said marker gene-specific probes may consists of the whole corresponding IMAGE vectors that comprise a marker gene-specific nucleic acid inserted therein.

In other embodiments wherein nucleic acid probes are used, specifically dedicated DNA microarrays may be manufactured by immobilising on a solid support the suitable set of gene-specific probes, the said gene-specific probes being either (i) the cDNA inserts that have been previously excised from the IMAGE vectors of interest, or (ii) nucleic acid fragments thereof comprising 12 or more consecutive nucleotides thereof.

In other embodiments, the said kit comprises a combination or a set of pair of primers comprising at least two kind of pair of primers, each kind of pair of primers being selected from the group consisting of pair of primers hybridising with each of the selected disease-specific marker genes among those disclosed in the present specification.

A primer kit according to the invention may comprise 2 to 20 kinds of pair or primers, each kind of pair of primers hybridising specifically with one biological marker of the invention. For instance, a primer kit according to the invention may comprise 2, 3, 4, 5, 6, 7, 8, 9, 10, 11, 12, 13, 14, 15, 16, 17, 18, 19 or 20 kinds of pairs of primers, each kind of pair of primers hybridising specifically with a single disease-specific marker gene as disclosed herein.

Notably, at least one pair of specific primers, as well as the corresponding detection nucleic acid probe, that hybridise specifically with one disease-specific marker gene of interest, is already referenced and entirely described in the public "Quantitative PCR primer database", notably at the following Internet address : http://Ipgws.nci.nih.gov/cgi-bin/PrimerViewer.

Illustrative nucleic acids that are directly usable as primers, or alternatively that are usable for designing primers, consist of the nucleic acids of SEQ ID N° 3 to SEQ ID N° 420 that are disclosed herein.

### *Monitoring anti-cancer treatments*

Monitoring the influence of agents (e.g., drug compounds) on the level of expression of one or more disease-specific marker genes identified according to the invention can be applied for monitoring the progression of the already diagnosed thyroid disease of the patient with time, which includes the development or the regression of the said thyroid disease. For example, the effectiveness of an agent to affect disease-specific marker gene expression can be monitored during treatments of subjects receiving treatments against the diagnosed specific thyroid disease, and especially treatments against the diagnosed specific thyroid tumor.

In certain embodiments, the present invention provides a method for monitoring the effectiveness of treatment of a subject with an agent (e.g., an agonist, antagonist, peptidomimetic, protein, peptide, nucleic acid, small molecule, or other drug candidate) comprising the steps of (i) obtaining a pre-administration thyroid tissue sample from a subject prior to administration of the agent; (ii) quantifying the level of expression of one or more selected disease-specific marker genes of the invention in the pre-administration thyroid tissues sample; (iii) obtaining one or more post-administration thyroid tissue samples from the subject; (iv) quantifying the level of expression of the disease-specific marker genes in the post-administration samples; (v) comparing the level of expression of the disease-specific marker genes in the pre-administration sample with the level of expression of the disease-specific marker genes in the post-administration sample or samples; and (vi) altering the administration of the agent to the subject accordingly.

Because repeated collection of biological samples from the patient affected with a thyroid disease are needed for performing the monitoring method described above, then preferred biological samples consist of thyroid tissue samples collected by conventional needle aspiration form the subject patient.

Accordingly, the present invention also relates to a method for adapting a pharmaceutical treatment in a patient affected with a thyroid disease, wherein said method comprises the steps of :

a) performing, on at least one thyroid tissue sample collected from the said patient, the *in vitro* prediction or diagnosis method that is disclosed herein;

b) adapting the pharmaceutical treatment of the said patient by (i) increasing or decreasing the amounts of the pharmaceutical agent, (ii) combining the said pharmaceutical agent with one or more distinct pharmaceutical agent (s) or (iii) replacing the said pharmaceutical agent by one or more distinct pharmaceutical agent(s).

***Methods for selecting biological markers indicative of metastasis***

This invention also pertains to methods for selecting one or more disease-specific marker genes that are indicative of the occurrence of, or of the risk of occurrence of, a thyroid disease in a patient.

The said disease-specific marker gene selection method according to the invention preferably comprises the steps of :

a) providing means for quantifying one or more candidate marker genes in a thyroid tissue sample;

b) providing a plurality of collections of thyroid tissue samples originating from patients affected with a thyroid disease, wherein each of the said collections consists of a plurality of thyroid tissue samples originating from patients that are affected with a single specific thyroid disease, preferably selected from the group of thyroid diseases consisting of (i) autoimmune thyroiditis (AT), (ii) Grave's disease (GD), macrofollicular adenoma (FTA-a), (iv) atypical follicular adenoma (FTA-aty), (v) microfollicular adenoma (FTA-b), (vi) follicular carcinoma (FTC), (vii) multinodular goitres (MNG), (viii) oncocytic adenoma (OTA), (ix) atypical oncocytic adenoma (OTA-aty), (x) oncocytic carcinoma (OTC) and (xi) papillary carcinoma (PTC);

c) quantifying the expression level of each of the one or more candidate marker genes, separately in every thyroid tissue sample contained in each collection of thyroid tissue samples;

d) selecting, in the group of candidate marker genes whose expression level is quantified at step c), those marker genes that are under-expressed or over-expressed exclusively in only one collection of thyroid tissue samples that is comprised in the plurality of collections of tissue samples provided at step b), whereby a set of marker genes is selected for each collection of tissue samples, the said set of marker genes comprising disease-specific marker genes, the under-expression of which, or the over-expression of which, is indicative of the occurrence of, or of the risk of occurrence of, a thyroid disease in the said patient.

Illustrative embodiments of the selection method above are fully described in the examples herein.

For performing step a) of the selection method above, the marker quantification means encompass means for quantifying marker gene-specific nucleic acids, such as oligonucleotide primers or probes. Illustratively, DNA microarrays may be used at step a) of the selection method above.

Means for specifically quantifying any one of the known potential marker gene, e.g. any gene-specific nucleic acid, may be provided at step a) of the selection method.

Each collection of thyroid tissue samples that is provided at step b) of the selection method above comprises a number of thyroid tissue samples originating from individuals that are all affected with the same thyroid disease, the said disease being preferably selected form the group consisting of (i) autoimmune thyroiditis (AT), (ii) Grave's disease (GD), macrofollicular adenoma (FTA-a), (iv) atypical follicular adenoma (FTA-aty), (v) microfollicular adenoma (FTA-b), (vi) follicular carcinoma (FTC), (vii) multinodular goitres (MNG), (viii) oncocytic adenoma (OTA), (ix) atypical oncocytic adenoma (OTA-aty), (x) oncocytic carcinoma (OTC) and (xi) papillary carcinoma (PTC).

Preferably, each collection of thyroid tissue samples comprises samples originating from at least 5 distinct individuals affected with the same thyroid disease, and most preferably at least 20, 25 or 30 distinct individuals affected with the same thyroid disease. The statistical relevance of the disease-specific marker genes that are finally selected at the end of the selection method generally increases with the number of distinct individuals tested, and thus with the number of thyroid tissue samples comprised in each collection that is provided at step b).

At step c), quantification of the candidate marker genes on the thyroid tissue samples provided at step b), using the quantification means provided at step a), may be performed according to any one of the quantification methods that are described elsewhere in the present specification.

At step d), each candidate marker gene quantified at step c) in a first specific collection of thyroid tissue samples, (e.g. thyroid tissue samples previously collected in patients affected with autoimmune thyroiditis (AT)) is compared to the quantification results found for the same marker in all of the other collections of thyroid tissue samples, e.g. collections of thyroid tissue samples from individuals affected with GD, FTA-a, FTA-aty, FTA-b, FTC, MNG, OTA, OTA-aty, OTC and PTC, as well as a collection of thyroid tissue samples originating form individuals not affected with a thyroid disease. Then, only those candidate marker genes that are differentially expressed (i.e. (i) under-expressed, (ii) not expressed, (iii) over-expressed in the said first collection of thyroid tissue samples, as compared to the other collections of thyroid tissue samples, are positively selected as disease-specific marker genes indicative of the occurrence of a specific thyroid disease (e.g. autoimmune thyroiditis (AT)). At step d), the selection of statistically relevant disease-specific marker genes, by comparing the expression level of a candidate marker gene in one collection of thyroid tissue samples with the expression level of the said candidate marker gene in every other collection of thyroid tissue samples, is termed a "One Versus All" ("OVA") pairwise comparison, as it is fully described in the examples herein.

The statistical relevance of each candidate marker gene tested, at step d), may be performed by calculating the p value for the said marker, for example using a univariate t-test, as disclosed in the examples herein. Generally, a marker is selected at step d) of the selection method above, when its p value is lower than 0.05.

The statistical relevance of the marker selection, at step d) of the method, may be further increased by using other statistical methods, wherein the said other statistical methods mayconsist of performing a multivariate permutation test, so as to provide 90% confidence that a false marker selection rate is less than 10%.

For further increasing the statistical relevance of the markers initially selected, at step d) of the selection method above, those markers that were initially selected as described above may be submitted to a further cycle of selection, for example by assaying the initially selected markers on further collections of thyroid tissue samples. This further cycle of selection may consist of, for example, performing a further expression analysis of the initially selected markers, for example by technique of quantitative RT-PCR expression analysis or by using DNA microarrays.

According to such a quantitative expression analysis, the quantification measure of expression of each initially selected marker may be normalised against a control value, e.g. the quantification measure of expression of a control gene such as TBP. The results may be expressed as N-fold difference of each marker relative to the value in normal thyroid tissues or to the value in all other thyroid tissues (normal and disease). Statistical relevance of each initially selected marker is then confirmed, for example at confidence levels of more than 95% (P of less than 0.05) using the Mann-Whitney U Test.

The present invention is further illustrated by, without in any way being limited to, the examples below.

## EXAMPLES

## A. Materials and Methods of the Examples

### *A.1. Tissue samples*

Samples were obtained from 132 human thyroid tumors and 34 controls. We studied follicular adenomas exclusively: 26 macrofollicular, 17 microfollicular and 10 atypical adenomas. Atypical adenomas were defined by nuclear features and vascular or cellular modification without capsular and/or vascular invasion. We included 24 adenomas with the largest nodule originating from multinodular goiters, with macrofollicular (23 cases) or microfollicular features (1 case). We examined 30 oncocytic follicular adenomas and 5 atypical oncocytic adenomas defined using the same criteria as for non-oncocytic adenomas. Mitochondrial quantity was appreciated by immunohistochemistry, using monoclonal anti-cytochrome c oxydase antibody (Clone 113-1, Biogenex Laboratories, Inc., San Ramon, CA, USA). We examined three types of carcinoma: follicular (3 cases), oncocytic (4 cases), and papillary carcinoma (13 cases). Control samples consisted of 24 wild type tissues, 5 samples of autoimmune thyroiditis, and 5 samples of Graves' disease. Table 1 summarizes the characteristics of the samples.

The diagnoses were made according to the WHO classification of tumors [23]. Seventy five anonymous samples were obtained from the Ambroise Paré Hospital (APHP, Boulogne S/Seine, France) and 67 anonymous samples from the University Hospital (Angers, France).

### *A.2. cDNA arrays*

*Extraction of total RNA*

Total RNA was extracted from tissue samples using a standard guanidium isothiocyanate protocol (TRIzol Reagent, Life Technologies, Inc., Gaithersburg, MD, USA) and quantified on a nanodrop apparatus. RNA integrity was checked using a Bio-Analyzer 2100 (Agilent Technologies, Waldbronn, Germany).

*Microarrays*

Gene expression was analyzed by hybridization of nylon cDNA arrays with radioactive probes. The arrays contained spotted PCR products from 8,862 selected IMAGE clones (MRC Rosalind Franklin Centre for Genomics Research, GeneService, Cambridge, UK) human cDNA clones and control clones. Clones were selected on the basis of the following criteria: the 3' location of the corresponding mRNA sequences, the same cloning vector (pT3T7), the same host bacteria, and approximately the same insert size. The majority of clones were selected such that they contained genes with a proven or putative implication in cancer or immune reactions. The IMAGE clones consisted of approximately 84% genes and 16% established sequence tags (ESTs). The control clones consisted of three differently sized polyadenylated sequences and pT7T3D cloning vectors (negative controls). PCR amplification and robotic spotting of PCR products on Hybond $N^+$ membranes (Amersham Pharmacia Biotech, Little Chalfont, UK) were performed according to protocols described elsewhere [Bertucci F, Van Hulst S, Bernard K, Loriod B, Granjeaud S, Tagett R, Starkey M, Nguyen C, Jordan B, Birnbaum D: Expression scanning of an array of growth control genes in human tumor cell lines. Oncogene 1999, Vol. 18:3905-3912].

*Hybridization of cDNA arrays*

cDNA arrays were first hybridized with a labeled oligonucleotide vector to determine the precise amount of cDNA accessible for hybridization in each spot. After stripping, each array was hybridized with a complex target prepared from 5 μg total RNA by simultaneous RT and [$\alpha$-$^{33}$P]deoxy-CTP labeling as described elsewhere [Microarray protocol of the TAGC laboratory [http://taac.univ-mrs.fr/pub/cancer/]. Each sample was hybridized on an individual array. After washing, hybridization images were obtained by scanning with an imaging plate device (Fuji BAS 5000, Raytest, Paris, France). Signal intensities were quantified using ArrayGauge software (Fujifilm Medical Systems, Stanford, CT, USA).

*Normalization*

All data were subjected to print-tip Lowess normalization [Yang YH, Dudoit S, Luu P, Lin DM, Peng V, Ngai J, Speed TP: Normalization for cDNA microarray data: a robust composite method addressing single and multiple slide systematic variation. Nucleic Acids Res 2002, 30:e15.]. Complex target measurements were corrected for the amount of spotted DNA measured by the oligovector hybridization. Spots exhibiting weak oligovector hybridization (low amounts of spotted DNA) were considered as missing values. Genes with an expression similar to the background and genes with missing values over an entire sample class were withdrawn from the analysis. Between-array normalization was obtained by dividing each measurement by the median value of the array.

***A.3. Data analysis and statistical methods***

*Statistical tests and correction for multiplicity*

One-way analysis of variance (ANOVA) was used to detect differences between the 12 class means. Multiple testing effects were taken into account using the false discovery rate (FDR) controlling procedure [Benjamini Y, Yekutieli, D.: The control of the false discovery rate in multiple testing under dependency. ANN STAT 2001, 29:1165-1188]. Gene expression specificities were determined with t-tests (two-tailed 5% risk) by comparing mean class expressions with gene profiles. Outlier values were filtered for protein-expression data by removing measurements which diverged from the class mean by more than 1.5 standard deviations.

*Clustering and ontology analysis*

Hierarchical clustering of the genes was computed on median gene-centered and log-transformed data using average linkage and uncentered correlation distances. Expression levels of marker genes were normalized before computation. Computation and visualization were done with the Cluster and TreeView software [Eisen MB, Spellman PT, Brown PO, Botstein D: Cluster analysis and display of genome-wide expression patterns. Proc Natl Acad Sci USA 1998, 95:14863-14868.]. Gene ontology enrichments into gene clusters were computed by GoMiner software [Zeeberg BR, Feng W, Wang G, Wang MD, Fojo AT, Sunshine M, Narasimhan S, Kane DW, Reinhold WC, Lababidi S, et al: GoMiner: a resource for biological interpretation of genomic and proteomic data. Genome Biol 2003, 4:R28.].

*Similarity, homogeneity and separation parameters*

For the set of twelve classes $C_i$, $C = \{C_1, C_2, \dots, C_{12}\}$, we used average homogeneity and separation parameters, defined elsewhere in clustering algorithms [Sharan R, Shamir R: CLICK: a clustering algorithm with applications to gene expression analysis. Proc Int Conf Intell Syst Mol Biol 2000, 8:307-316.], to evaluate the data set quality. Each class $C_i$ was composed of $O_j$ samples and we defined its centroid $\overline{O_i}$ as its mean gene-expression signature. We defined the similarity S as the correlation coefficient of two gene-expression signatures from samples or centroids. The homogeneity of a given class $C_i$ was defined as:

$$H(C_i) = \frac{1}{\|C_i\|} \sum_{O_j \in C_i} S(O_j, \overline{O}_i) \quad (1)$$

The average homogeneity across all the classes was defined as:

$$H_{ave} = \frac{1}{N} \sum_{C_i \in C} \|C_i\| \cdot H(C_i) \quad (2)$$

Finally, the average separation parameter was defined as:

$$S_{ave} = \frac{\sum_{C_i \neq C_j} \left(\|C_i\| \cdot \|C_j\|\right) \cdot s\left(C_i, C_j\right)}{\sum_{C_i \neq C_j} \|C_i\| \cdot \|C_j\|} \quad (3)$$

Significance of similarity was assessed at a one-tailed 5 % risk from 10,000 bootstrapped data sets. Pairwise similarity parameters were normalized by their expected value from bootstrapped distributions, clustered by Cluster software with the Spearman rank-correlation and the average linkage method, and visualized by TreeView Software.

*Sample filtering*

Each sample was scored by its similarity S to the class centroids. Samples were removed from the analysis if their maximal score was not coherent with histological results and class similarities. For instance, atypical oncocytic adenomas were not filtered if their maximal score related to classical oncocytic adenomas, whereas some were filtered if they related to thyroiditis.

*Automated classifiers*

We used automated classifiers to evaluate the predictive power of the selected genes. These analyses were performed using BRB ArrayTools developed by Dr. Richard Simon and Amy Peng [30]. Algorithms were the Linear diagonal discriminant analysis (LDDA), the K-nearest neighbors for K=1 and K=3, and the nearest centroid (NC). We computed the prediction for several gene subsets selected by cutoffs to univariate tests (from 1 E-4 to 1 E-17). To evaluate classifier performances we used the values of population size (*POP*), the number of positive (POS) cases in the population, the size of the detection set (S*ET*), and the number of true positives (*TP*) and true negatives (*TN*) in the detection set. The sensitivity (*SEN*) was given by *SEN=TP/POS,* and the positive predictive value (*PPV*) by *PPV=TP/SET.* Performance was determined by the classical accuracy coefficient (ACC) defined as:

$$ACC = \frac{(TP + TN)}{POP} \quad (4)$$

Finally, we compared the classical accuracy coefficient with the geometric mean (G) defined as:

$$G = \sqrt{SEN \cdot PPV} \quad (5)$$

For each subset of genes, we compared the 4 algorithms by assigning to them a score. This score is the sum of G values over the 12 tissue classes.

## Example 1 : Selection and validation of disease-specific marker genes

### *1.1. Experimental data*

The total RNA from 132 samples of human thyroid tumors and 34 control tissues, detailed in table 1, was isolated and used to generate a radio-labeled cDNA, which was hybridized to microarrays containing 8,862 human cDNA clones. We analyzed the expression profiles of differential genes to identify the main signatures and compared the 12 types of thyroid tumor to set up a classification.

*Gene expression signatures of thyroid samples*

We used one-way ANOVA statistical tests to detect variations in the gene expressions of the 12 classes of thyroid lesions defined by histopathological criteria. Since the classes were of different sizes, statistical bias tending to increase the number of false discoveries could be expected. We limited this bias by optimizing the average separation parameter used in clustering algorithms [Sharan R, Shamir R: CLICK: a clustering algorithm with applications to gene expression analysis. Proc Int Conf Intell Syst Mol Biol 2000, 8:307-316] on all possible best sets of genes (Figure 1). The average separation of the classes depended on the size of the sets of genes. This parameter needed to be minimized to improve between-class separation, whereas the size of the gene set needed to be maximized to improve the power of the selection method. A discontinuity appeared at the optimal separation value of 0.17 and a gene set size of 1,197 genes, allowing for 1.5% false discoveries.

Hierarchical clustering of the optimal gene set showed the fit of gene-expression signatures on thyroid lesions, revealing strong and subtle gene expression profiles. Within-class heterogeneity was observed in all classes, but it was less in AT or FTC. Nine main clusters of correlated genes were identified, including some class-specific genes among which we searched for enriched gene ontology terms (right side of the figure). Oncocytomas had consistently large signatures which mainly divided the gene set into two parts in which the genes were either over- or under-expressed. The main cluster of over-expressed genes contained the malic enzyme 3 gene (ME3), which is involved in oxidoreductase activity, the ATPase type 11 B gene (ATP11 B), and the neurotrophic tyrosine kinase receptor type 2 gene (NTRK2), which is related to ATP binding. The main PTC cluster of over-expressed genes contained the Cbp/p300-interacting transactivator gene (CITED1), a known papillary marker [ Prasad ML, Pellegata NS, Kloos RT, Barbacioru C, Huang Y, de la Chapelle A: CITED1 protein expression suggests Papillary Thyroid Carcinoma in high throughput tissue microarray-based study. Thyroid 2004, 14:169-175]. Some of these genes also showed over-expression in FTA-aty samples. The AT signature was homogeneous and showed a large cluster of over-expressed genes, which were also over-expressed in some other samples, notably in the GD, PTC and WT classes. They included the major histocompatibility complex DP beta 1 gene (HLA-DPB1) and the chemokine ligand 19 gene (CCL19), respectively involved in immune and inflammatory responses suggestive of lymphocyte invasion. Follicular adenoma profiles (FTA-a, -b, MNG) were weakly expressed and not clearly distinguishable from each other. Despite these two drawbacks, we were able to identify an adenoma-specific cluster that included death-associated protein gene (DAP) involved in apoptosis. Over-expressed WT genes were clustered together (box 6), and included the aquaporin 11 (AQP11) gene and the solute carrier family 17 member 2 gene (SLC17A2), involved respectively in cellular volume and cellular metabolism. The other gene clusters were multi-specific.

*Comparison and classification of thyroid tissues*

In order to define the 12 histopathological follicular thyroid lesions, we examined each of the 66 possible pairwise comparisons of the categories. We took into account only the parameters significant at a one-tailed 5% risk based on 10,000 bootstrapped data sets. This allowed a global classification of the thyroid lesions (dendogram on the left of the figure) and the visualization of all the pairwise comparisons (cells of the matrix).

The dendogram revealed three well-defined clusters of thyroid lesions grouping the benign/normal, malign and oncocytic classes by within-group similarity and inter-group dissimilarity. The benign/normal cluster grouped AT, GD, FTA-a, WT and MNG. The GD class was significantly similar to the whole cluster. Significant similarities were also found between AT and WT, and between MNG and FTA-a. In the malignant cluster, a significant similarity was observed between PTC and FTA-aty. In the oncocytic cluster, no significant similarity was found between OTA and OTC. Atypical oncocytic adenomas were similar to both OTA and OTC. The FTA-b class, which showed dissimilarities with all the other classes, was therefore considered as a singleton in this classification.

We detailed two significant similarities and dissimilarities involving either AT or OTC with FTC, OTA-aty or WT. The correlation coefficients of all sample signatures with respect to class centroids were computed and plotted on 2D graphs. Samples from similar classes were expected to be poorly separated whereas samples from dissimilar classes were expected to be sharply separated in the 2D plane. We first examined the AT and FTC classes that had been found significantly dissimilar. The graph (panel B top) showed the perfect separation of AT and FTC samples. We also examined the AT and WT classes (panel B bottom) that had been found significantly similar. The graph showed good correlations of all AT samples with WT (R>0.44; P-value<0.096), with 2 samples at a 5% risk. Five WT samples out of 22 had high correlations with AT (R > 0.45; P-value<0.079), with 3 samples at a 5% risk. Two WT samples were clearly clustered with the AT samples. Then, we examined the dissimilar OTC and WT classes and observed the perfect separation (panel C top). Finally, we examined the significantly similar OTC and OTA-aty classes (panel C bottom). Three out of 4 OTC samples were well correlated with OTA-aty (R>0.367; P-value<0.051), with 2 samples at a 5% risk. One OTA-aty sample out of 5 correlated significantly with OTC (R = 0.42; P-value<0.027).

*Filtering of divergent samples*

Since within-class heterogeneity was observed in expression signatures, we reduced heterogeneity to obtain core class signatures by filtering the most divergent samples. The filtering consisted in removing samples which were maximally scored by the similarity parameter in an irrelevant class, with respect to histological features (Table 1), or the results of significant dissimilarities (Figure 3A). Twenty samples were thus removed from the analysis: 12 samples excluded for a high signal of thyroiditis (7 FTA, 3 OTA and 2 WT); 2 follicular variants of PTC had been misclassified in FTA; and 4 FTA and 2 OTA samples were excluded because of high heterogeneity, presenting either oxyphilic or papillary foci in their nodules. Since 3 of the 5 GD samples were divergent, they were not excluded from the analysis.

We used homogeneity parameters to compare the quality of the data sets before and after filtering of the divergent samples (Figure 2). The average homogeneity was improved after filtering (from 0.476 to 0.515). The homogeneity of the AT, GD, FTC and OTC classes remained unchanged since no samples were excluded, while the homogeneity of all the other classes was improved. The greatest improvement (from 0.47 to 0.68) was found in the OTA-aty class, in which 2 samples out of 5 were excluded. The lowest improvements were observed in the FTA-a class (from 0.38 to 0.40), the MNG class (from 0.39 to 0.41) and the OTA class (from 0.34 to 0.37).

*Candidate marker genes*

We used a t-statistics procedure to determine lists of candidate marker genes for each class of thyroid tumor from the filtered data set. Two lists of under- or over-expressed genes were defined at a 5% risk for each class. The gene expression profiles of the top 5 genes of each list are shown in Figure 5 for all the samples.

The genes had heterogeneous profiles but specific expression levels in target samples. Anti-correlations of signatures were observed between classes such as FTA-a genes in OTC, FTA-aty genes in WT, FTA-b genes in AT, MNG genes in oncocytomas, OTA genes in AT or WT, PTC genes in FTA-b or WT, and WT genes in oncocytomas or PTC. Correlated signatures were also observed such as those of GD genes in WT, FTA-a genes in MNG, FTA-aty genes in PTC and vice-versa, oncocytomas with each other, and PTC genes in FTC. Oncocytic classes had similar signatures but they were distinguishable by the amplitude of expression.

*Automated sample prediction*

We evaluated the capability of the gene-expression data to predict the class of each sample. As it was shown [Yukinawa N, Oba S, Kato K, Taniguchi K, Iwao-Koizumi K, Tamaki Y, Noguchi S, Ishii S: A multi-class predictor based on a probabilistic model: application to gene expression profiling-based diagnosis of thyroid tumors. BMC Genomics 2006, 7:190.], performances of a classifier can be unstable when varying the cutoff for gene selection. We searched for the best subset of genes by observing a scoring function for 4 algorithms when varying the gene selection.

The 1-Nearest Neighbor (1-NN) classifier and the Linear Diagonal Discriminant Analysis (LDDA) showed globally the best performances. The Nearest Centroid (NC) classifier was mainly ranked the third except with poorly (>1E-7) or very stringent cutoffs (<1E-15). The 3-Nearest Neighbors classifier (3-NN) showed the worst performances. For all the classifiers, very stringent cutoffs (<1E-13) deserved the performances. For the 2 best ones, 1-NN and LDDA, the maximum score was associated to a cutoff of 1 E-9 which represented 258 genes.

For this particular cutoff we ranked the classifiers relatively to each others for each tissue class (Table 2). The rank 1 was given to the best algorithm and the rank 4 to the worst (except for equality). The sum over each class indicated that LDDA had the lowest (best) ranks (Sum=21). The 1-NN classifier was very close (Sum=22). NC was the third (Sum=25) and 3-NN was the worst (Sum=43).

Finally, we detailed the performances of the LDDA and 1-NN classifiers with a gene selection at P<1E-9. We examined two intuitive parameters as the sensitivity (SEN) and the positive predictive value (PPV). We also examined two parameters which summarized the performances as the accuracy (ACC) and the geometric mean (G). When observing SEN and PPV, performances depended dramatically of the class from quite perfect detections (AT and WT) to the worst case (OTA-aty). Though cases were very different, the ACC did not reflect it and was always higher than 0.79.

For GD, the LDDA classifier had low performances (SEN=0.2, PPV=0.167, G=0.18) but a high ACC value (0.94) which was better than the good OTA case (SEN=0.74; PPV=0.87; G=0.80; ACC=0.93). For OTA-aty, no true positives were predicted by the 2 algorithms (SEN=0; PPV=0) but ACC was over 0.96 whereas G equaled 0. The ACC led to over-estimations and the geometric mean G, used in machine-learning techniques [Kubat M: Machine Learning for the Detection of Oil Spills in Satellite Radar Images. Machine Learning 1998, 30:195-215], gave results concordant with SEN and PPV (according to its definition) and proved more intuitive. So we used it to interpret the data.

The 2 algorithms showed relatively similar results. The AT and WT classes were the best cases (from G=0.89 to 0.96). OTA and PTC were well detected by 1-NN (respectively G=0.77 and G=0.73) and even more by LDDA (respectively G=0.80 and G=0.85). FTA-aty and FTA-b were also well detected and better by 1-NN (from 0.58 to 0.67). Macrofollicular adenomas (FTA-a and MNG) were detected with medium performances (from 0.38 to 0.50). GD was either badly detected by LDDA (G=0.18) or detected with medium performances by 1-NN (0.45). OTA-aty was the worst case with no detection whatever the algorithm used (G=0).

### *1.2. Detailed discussion of the results*

Gene expression profiling is a powerful technique for finding new molecular markers for tumor growth as well as for providing insights into the molecular mechanisms involved in tumorigenesis. However, current microarray studies on thyroid tumors fail to propose truly specific markers because they are based on very incomplete sets of thyroid tumor classes. We have conducted the first microarray study including more than 90% of the types of thyroid tumor known.

*Extracting information from data concerning heterogeneous and rare thyroid tumors*

We measured 8,862 gene expression levels for 166 samples representing 12 different classes of thyroid lesions. However, thyroid tumors are heterogeneous and subject to diagnostic misclassifications [Hirokawa M, Carney JA, Goellner JR, DeLellis RA, Heffess CS, Katoh R, Tsujimoto M, Kakudo K: Observer variation of encapsulated follicular lesions of the thyroid gland. Am J Surg Pathol 2002, 26:1508-1514; Franc B, de la Salmoniere P, Lange F, Hoang C, Louvel A, de Roquancourt A, Vilde F, Hejblum G, Chevret S, Chastang C: Interobserver and intraobserver reproducibility in the histopathology of follicular thyroid carcinoma. Hum Pathol 2003, 34:1092-1100.; Lloyd RV, Erickson LA, Casey MB, Lam KY, Lohse CM, Asa SL, Chan JK, DeLellis RA, Harach HR, Kakudo K, et al: Observer variation in the diagnosis of follicular variant of papillary thyroid carcinoma. Am J Surg Pathol 2004, 28:1336-1340]. The classical methods of analysis are affected by weak intra-class homogeneity and by unbalanced classes since very few samples were available for the rarer pathologies. Because of the presence of rare samples, we used an optimal gene selection method (Figure 1) to limit false discoveries due to statistical bias caused by unbalanced class sizes. This method allows the detection of bias and limits the effect by optimizing the power of the analysis. It proved useful with our data set and is worth comparing with other gene selection methods.

The hierarchical clustering of the data can be a complement to supervised methods, it shows the strongest molecular signals but it fails to reveal the specificity of some of the classes of tumor, such as GD, FTA-a, FTA-b and MNG. However, useful information can be extracted from these classes with other methods such as that of global classification and the definition of several candidate markers. As the hierarchical clustering algorithm assumes that genes behave similarly in all the samples and that they are each associated with a single biological function or process [Tanay A, Sharan R, Shamir R: Discovering statistically significant biclusters in gene expression data. Bioinformatics 2002, 18 Suppl 1:S136-144], it shows limitations when many samples are involved.

Within-class gene expression signatures of thyroid tumors are heterogeneous. Filtering divergent tissues is useful for the definition of the core signatures of tissues but it prevents the analysis of heterogeneity that might otherwise bring subclasses to light. However, it leads to a better choice of class-specific candidate marker genes by improving within-class and overall homogeneity (Figure 2). Large data sets containing both abundant and rare samples can pose a problem for predictive algorithms because of the presence of unbalanced positive and negative samples. In such cases, the interpretation of the classical accuracy coefficient (ACC) is far from intuitive and leads to over-estimation of performance. For instance, if 13 our 166 samples had actually been PTCs, and 10 samples were predicted to be PTCs whereas none were truly PTC, then the accuracy would still work out at as high as 86%. In contrast, the use of the geometric mean G is unbiased and quite intuitive [Kubat M: Machine Learning for the Detection of Oil Spills in Satellite Radar Images. Machine Learning 1998, 30:195-215.]. Thus, in the hypothetical case discussed, G would be equal to zero.

*The classification based on gene expression is complementary to the WHO classification*

The classification based on gene expression divides the thyroid tumors into three distinct groups (benign/normal, malignant and oncocytic) and separates microfollicular adenomas (FTA-b). Pairwise distinctions of benign/normal and malign tumors are consistent with published reports, as in the case of PTC and FTA [Finley DJ, Arora N, Zhu B, Gallagher L, Fahey TJ, 3rd: Molecular profiling distinguishes papillary carcinoma from benign thyroid nodules. J Clin Endocrinol Metab 2004, 89:3214-3223].

Moreover, thanks to the large and almost complete set of thyroid tumors included in our study, the results may be generalized to the whole range of thyroid tumors, from the benign/normal to the malignant and oncocytic tumors. Oncocytomas are the most divergent tumors compared to normal tissue, particularly with respect to the large number of genes involved in mitochondrial activity. Moreover, since they appear unrelated to PTCs or FTCs in the classification of thyroid tumors based on gene expression (Figure 3A), they should be analyzed independently [Giordano TJ, Au AY, Kuick R, Thomas DG, Rhodes DR, Wilhelm KG, Jr., Vinco M, Misek DE, Sanders D, Zhu Z, et al: Delineation, functional validation, and bioinformatic evaluation of gene expression in thyroid follicular carcinomas with the PAX8-PPARG translocation. Clin Cancer Res 2006, 12:1983-1993] and not be considered as PTC- or FTC-related tumors [Barden CB, Shister KW, Zhu B, Guiter G, Greenblatt DY, Zeiger MA, Fahey TJ, 3rd: Classification of follicular thyroid tumors by molecular signature: results of gene profiling. Clin Cancer Res 2003, 9:1792-1800.] in gene expression studies.

We observed that microfollicular adenoma presents a signature different from macrofollicular adenoma and follicular carcinoma. We may consider this subtype as a distinct entity because of the mechanism involved in its pathogenesis, although we do not know the impact of this specific profile on the tumoral outcome. Besides, variations in the gene expression signatures of macrofollicular adenomas (FTA-a and MNG) do not reflect the apparition of multiple nodules in the thyroid and lead automated classifiers to have low performances on these 2 cases. They should be considered as a single class in global expression studies. We think that it is worth investigating differences in macro- and microfollicular adenomas to understand the microfollicular machinery.

Non-oncocytic malignant and atypical tissues are clustered together in the classification. A significant similarity exists between the PTC and FTA-aty classes. No particular similarity or dissimilarity was found for the FTC class. The lack of significant results may be explained by the small size of the FTC sample; larger samples should produce more conclusive results. The close relationship between the PTC and FTA-aty classes added argument to the proposal terminology of a "differentiated tumor of uncertain malignant potential". This designation permitted to clearly distinguish the atypical lesions for which the pending diagnosis is PTC from the others which are either non malignant or candidate to a FTC diagnosis and referred as "follicular tumor of uncertain malignant potential".

*Markers for the classification of main thyroid pathologies*

The identification of pathologic tissue by morphological features is not always precise and exclusive. In thyroid lesions, it has been shown that inter- and intra-observer variations are significant [Aldred MA, Huang Y, Liyanarachchi S, Pellegata NS, Gimm O, Jhiang S, Davuluri RV, de la Chapelle A, Eng C: Papillary and follicular thyroid carcinomas show distinctly different microarray expression profiles and can be distinguished by a minimum of five genes. J Clin Oncol 2004, 22:3531-3539; Finley DJ, Lubitz CC, Wei C, Zhu B, Fahey TJ, 3rd: Advancing the molecular diagnosis of thyroid nodules: defining benign lesions by molecular profiling. Thyroid 2005, 15:562-568].

Mutations and rearrangements can define a class and its evolution more precisely than the morphology, as in the case of papillary and follicular thyroid carcinomas [Giordano TJ, Au AY, Kuick R, Thomas DG, Rhodes DR, Wilhelm KG, Jr., Vinco M, Misek DE, Sanders D, Zhu Z, et al: Delineation, functional validation, and bioinformatic evaluation of gene expression in thyroid follicular carcinomas with the PAX8-PPARG translocation. Clin Cancer Res 2006, 12: 1983-1993; Giordano TJ, Kuick R, Thomas DG, Misek DE, Vinco M, Sanders D, Zhu Z, Ciampi R, Roh M, Shedden K, et al: Molecular classification of papillary thyroid carcinoma: distinct BRAF, RAS, and RET/PTC mutation-specific gene expression profiles discovered by DNA microarray analysis. Oncogene 2005, 24:6646-6656].

However, some histotypes like oncocytic carcinomas escape to this molecular classification [Nikiforova MN, Lynch RA, Biddinger PW, Alexander EK, Dorn GW, 2nd, Tallini G, Kroll TG, Nikiforov YE: RAS point mutations and PAX8-PPAR gamma rearrangement in thyroid tumors: evidence for distinct molecular pathways in thyroid follicular carcinoma. J Clin Endocrinol Metab 2003, 88:2318-2326]. Several groups have evaluated the usefulness of biomarkers combination in the diagnosis of suspicious thyroid lesions [Weber, 2004; Ito, 2005; Prasad, 2005]. But they failed to show use in clinical practice, as an adjunctive test for exploring such nodules. However, development of new antibodies against selected markers has proved to accurate the diagnosis of suspicious lesions [Cerutti JM, Latini FR, Nakabashi C, Delcelo R, Andrade VP, Amadei MJ, Maciel RM, Hojaij FC, Hollis D, Shoemaker J, Riggins GJ: Diagnosis of suspicious thyroid nodules using four protein biomarkers. Clin Cancer Res 2006, 12:3311-3318.]. These antibodies were done after selection by gene expression profiling. That proved the invaluable assistance of this technology in choosing the most appropriate markers to distinguish between the various thyroid lesions. But this study also shows that several new custom antibodies might be produced and tested before using in clinical practice. Currently, we show that a classification of thyroid lesions based on gene expression patterns is a useful complement to the pathologic classification. We postulate that dedicated microarrays containing a subset of 258 selected genes may be used to reclassify some lesions. All of the 12 classes show high predictive accuracy using our method. However, one class, the OTA-Aty class, presents a low G value. It suggests that this class does not really exist at molecular level, on contrary to the FTA-Aty class. This notion is in accordance with previous results where some OTA and OTC gathered on microarray data (Finley, 2004, surgery). Our study offers a sensitive method that may be used on fine needle aspiration for classifying thyroid lesions. Defining all of these subclasses is a close necessary for classifying follicular lesions situated in the grey zone area.

**Table 1 :** Thyroid tissue samples included in the analysis

| *Thyroid tissue* | *N* | *Histology* | *Follicle architecture* |
|---|---|---|---|
| **Controls** | | | |
| Wild type (WT) | 24 | Normal | Normal |
| Graves's disease (GD) | 5 | Diffuse goiter | Normal |
| Autoimmune thyroiditis (AT) | 5 | Diffuse lymphocyte thyroiditis | Normal |
| **Benign tumors** | | | |
| Oncocytic adenoma (OTA) | 30 | Over 95% oncocytic cells and mitochondrial antibody ++ | Micro (17) or macro (13) |
| *Follicular adenomas (FTA): complete fibrous capsule* | | | |
| Macrofollicular (FTA-a) | 26 | Unique nodule | Macro* |
| Microfollicular (FTA-b) | 17 | Unique nodule | Micro** |
| Multinodular goiter (MNG) | 24 | largest nodule of a multinodular goiter | Micro (1) or macro (23) |
| *Atypical adenomas: complete fibrous capsule and moderate vascular invasion* | | | |
| Follicular (FTA-aty) | 10 | Unique nodule and atypical nucleus | Micro (1) or Macro(9) |
| Oncocytic (OTA-aty) | 5 | Unique nodule and atypical nucleus | Micro (2) or Macro (3) |
| **Differentiated malignant tumors: capsular and vascular invasion** | | | |
| Follicular carcinoma (FTC) | 3 | Unique | Micro |
| Oncocytic carcinoma (OTC) | 4 | Over 95% oncocytic cells and mitochondrial antibody ++ | Trabecular |
| Papillary carcinoma (PTC) | 13 | Typical papillary nuclear features | Papillary architecture (5) or follicular variant (8) |

* Micro = microfollicular; ** Macro = macrofollicular

**Table 2** : Relative performance ranks of the classifiers based on the geometric means G

| *Class* | *LDDA** | *1-NN*** | *3-NN**** | *NC***** |
|---|---|---|---|---|
| AT | 1 | 2 | 4 | 3 |
| FTA-a | 2 | 1 | 4 | 3 |
| FTA-aty | 3 | 2 | 4 | 1 |
| FTA-b | 3 | 2 | 4 | 1 |
| FTC | 2 | 1 | 3 | 3 |
| GD | 2 | 1 | 3 | 3 |
| MNG | 1 | 3 | 4 | 1 |
| OTA | 1 | 2 | 4 | 3 |
| OTA-aty | 1 | 1 | 1 | 1 |
| OTC | 1 | 3 | 4 | 2 |
| PTC | 2 | 3 | 4 | 1 |
| WT | 2 | 1 | 4 | 3 |
| *Sum* | *21* | *22* | *43* | *25* |

* LLDA = Linear Diagonal Discriminant Analysis; ** 1-NN = 1-Nearest Neighbor; *** 3-NN = 3-Nearest Test Neighbor; **** NC = Nearest Centroid

| Table 3 | AT | Under | |
|---|---|---|---|
| name | cloid | p-value | fold |
| IQCA | 669510 | 1,04E-34 | 0,44 |
| KRT18 | 725096 | 1,15E-33 | 0,36 |
| MATN2 | 366100 | 1,08E-28 | 0,34 |
| KRTHA4 | 325155 | 1,50E-23 | 0,21 |
| FCGBP | 154172 | 1,42E-21 | 0,08 |
| SLC4A4 | 787938 | 8,75E-21 | 0,31 |
| IMAGE:744055 Transcribed locus | 744055 | 7,13E-20 | 0,22 |
| KRT7 | 592276 | 8,12E-17 | 0,27 |
| C10orf116 | 740941 | 9,03E-16 | 0,40 |
| GSTM1 | 73778 | 1,29E-15 | 0,39 |
| LOC400451 | 667174 | 1,38E-14 | 0,27 |
| CRABP1 | 739193 | 2,75E-14 | 0,12 |
| RAD51L1 | 295412 | 5,98E-14 | 0,28 |
| HSPB1 | 23827 | 1,66E-13 | 0,53 |
| IMAGE:136686 | 136686 | 1,14E-12 | 0,40 |
| IMAGE:744385 Similar to heat shock 10kDa protein 1 (chaperonin 10); heat shock 10kD protein 1 (chaperonin 10) | 744385 | 1,43E-12 | 0,50 |
| RODH | 471641 | 2,05E-12 | 0,18 |
| CHRAC1 | 724276 | 2,06E-12 | 0,43 |
| MATN2 | 28584 | 6,45E-12 | 0,22 |
| CTNNA1 | 268972 | 6,80E-12 | 0,63 |
| MGC99813 | 739097 | 6,83E-12 | 0,19 |
| FLJ14351 | 744943 | 8,09E-12 | 0,17 |
| PLXNC1 | 724609 | 8,10E-12 | 0,44 |
| ICOSLG | 2074228 | 4,39E-11 | 0,40 |
| IMAGE:365913 Transcribed locus | 365913 | 4,41E-11 | 0,48 |
| NFKB1 | 687782 | 6,62E-11 | 0,41 |
| IMAGE:23872 AF034176 Human mRNA (Tripodis and Ragoussis) Homo sapiens cDNA clone ntcon5 contig | 23872 | 9,23E-11 | 0,62 |
| FLJ23577 | 668452 | 1,01E-10 | 0,38 |

| Table 3 | AT | Under | |
|---|---|---|---|
| name | cloid | p-value | fold |
| PON3 | 681959 | 1,62E-10 | 0,39 |
| ANKRD28 | 687379 | 5,78E-10 | 0,41 |
| FLJ12895 | 23271 | 1,31E-09 | 0,53 |
| C6orf118 | 731745 | 1,63E-09 | 0,63 |
| IMAGE:738332 Transcribed locus | 738332 | 1,88E-09 | 0,26 |
| LOC440934 | 726760 | 2,25E-09 | 0,47 |
| IMAGE:666911 Transcribed locus | 666911 | 4,05E-09 | 0,37 |
| HSPB1 | 724150 | 4,15E-09 | 0,50 |
| IGF1R | 682555 | 4,46E-09 | 0,60 |
| LRPAP1 | 587186 | 8,31E-09 | 0,28 |
| GNB4 | 813260 | 9,71E-09 | 0,30 |
| NY-REN-41 | 136646 | 1,10E-08 | 0,52 |
| CAV1 | 309645 | 1,88E-08 | 0,39 |
| E2F5 | 809828 | 2,42E-08 | 0,32 |
| LRRIQ2 | 487152 | 2,49E-08 | 0,67 |
| GRB10 | 564994 | 2,81E-08 | 0,49 |
| ITPR1 | 667348 | 3,94E-08 | 0,69 |
| TCEA2 | 730149 | 5,89E-08 | 0,51 |
| SLC36A3 | 731115 | 6,86E-08 | 0,69 |
| IMAGE:738945 | 738945 | 7,57E-08 | 0,63 |
| PIP | 985457 | 8,60E-08 | 0,52 |
| TPCN1 | 179288 | 1,02E-07 | 0,51 |
| SLC22A18 | 742862 | 1,08E-07 | 0,62 |
| MT1F | 78353 | 1,17E-07 | 0,17 |
| VEGFC | 503189 | 1,53E-07 | 0,48 |
| ID1 | 1087348 | 2,20E-07 | 0,23 |
| GNAI1 | 753215 | 2,34E-07 | 0,45 |
| MRPS11 | 471574 | 2,79E-07 | 0,54 |
| KLHL9 | 471696 | 2,92E-07 | 0,31 |
| APLP2 | 549054 | 3,20E-07 | 0,30 |
| LMLN | 744657 | 3,23E-07 | 0,52 |
| IMAGE:731357 Transcribed locus | 731357 | 3,83E-07 | 0,65 |
| MGC17299 | 713422 | 4,48E-07 | 0,51 |
| FARP1 | 486708 | 4,61E-07 | 0,44 |
| IMAGE:666794 Hypothetical LOC388170 | 666794 | 4,76E-07 | 0,68 |
| FLJ10748 | 726830 | 5,32E-07 | 0,50 |
| DKFZp761P0423 | 730829 | 5,50E-07 | 0,50 |
| CLDN7 | 300268 | 6,15E-07 | 0,39 |
| PVRL2 | 725364 | 7,25E-07 | 0,60 |

| Table 3 | AT | Under | |
|---|---|---|---|
| name | cloid | p-value | fold |
| PDCD4 | 665376 | 7,38E-07 | 0,45 |
| WNT4 | 375746 | 7,99E-07 | 0,67 |
| CCND1 | 324079 | 8,65E-07 | 0,40 |
| GLT8D2 | 365271 | 9,21E-07 | 0,58 |
| EPHB6 | 172982 | 9,40E-07 | 0,48 |
| PPARD | 724562 | 9,65E-07 | 0,55 |
| GJA1 | 839101 | 9,68E-07 | 0,42 |
| CITED1 | 265558 | 1,18E-06 | 0,27 |
| LOC51760 | 52226 | 1,31E-06 | 0,56 |
| GSTM2 | 713922 | 1,40E-06 | 0,50 |
| CCL28 | 136919 | 1,53E-06 | 0,58 |
| HOXD11 | 682119 | 1,61E-06 | 0,40 |
| ANKRD20B | 745106 | 1,61E-06 | 0,46 |
| IMAGE:1877 668 | 1877668 | 1,95E-06 | 0,44 |
| BMP7 | 1594414 | 1,98E-06 | 0,32 |
| RABL5 | 471829 | 2,09E-06 | 0,49 |
| FGFRL1 | 739956 | 2,18E-06 | 0,33 |
| DJ159A19.3 | 23945 | 2,27E-06 | 0,51 |
| CACNB2 | 173841 | 2,39E-06 | 0,66 |
| SASH1 | 31120 | 2,49E-06 | 0,40 |
| BCL2 | 342181 | 2,50E-06 | 0,52 |
| PEMT | 742580 | 2,68E-06 | 0,46 |
| SEMA7A | 135941 | 2,91E-06 | 0,20 |
| SLA | 32339 | 2,98E-06 | 0,36 |
| TYMS | 687912 | 3,29E-06 | 0,52 |
| PDE8A | 666154 | 3,34E-06 | 0,55 |
| IMP-2 | 743774 | 4,07E-06 | 0,40 |
| DJ462O23.2 | 738970 | 4,08E-06 | 0,57 |
| RPS6KA2 | 22711 | 4,25E-06 | 0,51 |
| YES1 | 501971 | 5,09E-06 | 0,58 |
| FLJ12998 | 364846 | 5,14E-06 | 0,56 |
| FLJ20160 | 175864 | 5,37E-06 | 0,55 |
| ARHGAP21 | 666792 | 5,79E-06 | 0,78 |
| IMAGE:7244 16 Transcribed locus | 724416 | 6,99E-06 | 0,32 |
| OTUB1 | 174683 | 7,07E-06 | 0,63 |
| DCI | 667892 | 7,80E-06 | 0,36 |
| PLA2G6 | 744087 | 8,56E-06 | 0,54 |
| CCNE1 | 68950 | 9,61E-06 | 0,45 |
| ZBTB16 | 2467442 | 9,79E-06 | 0,35 |
| NR2F6 | 136807 | 1,10E-05 | 0,49 |
| IMAGE:7444 39 Transcribed locus | 744439 | 1,31E-05 | 0,41 |
| IMAGE:1716 286 | 1716286 | 1,52E-05 | 0,33 |
| LAD1 | 121551 | 1,69E-05 | 0,70 |
| SH3RF2 | 744797 | 1,97E-05 | 0,45 |

| Table 3 | AT | Under | |
|---|---|---|---|
| name | cloid | p-value | fold |
| IMAGE:7420 61 | 742061 | 2,01E-05 | 0,72 |
| LATS2 | 666778 | 2,21E-05 | 0,54 |
| BCL2 | 342181 | 2,28E-05 | 0,55 |
| DUSP24 | 740158 | 2,57E-05 | 0,40 |
| DCXR | 724596 | 2,78E-05 | 0,58 |
| CKLFSF4 | 143759 | 2,91E-05 | 0,41 |
| IMAGE:6663 15 Homo sapiens, clone IMAGE:5312 086, mRNA | 666315 | 2,94E-05 | 0,60 |
| SNCB | 50202 | 3,77E-05 | 0,50 |
| TP53I11 | 667514 | 3,92E-05 | 0,59 |
| GFM2 | 364959 | 4,24E-05 | 0,44 |
| NR6A1 | 258666 | 4,39E-05 | 0,52 |
| GATA3 | 148796 | 4,73E-05 | 0,37 |
| PRUNE | 364324 | 4,73E-05 | 0,61 |
| NR2F2 | 72744 | 5,83E-05 | 0,38 |
| SGSH | 669263 | 6,29E-05 | 0,61 |
| HAS3 | 667533 | 6,62E-05 | 0,35 |
| FLJ32731 | 365177 | 6,76E-05 | 0,63 |
| LRP2 | 2055272 | 6,77E-05 | 0,39 |
| IMAGE:7312 98 CDNA FLJ42482 fis, clone BRACE2032 134 | 731298 | 7,03E-05 | 0,62 |
| COX6A1 | 512910 | 7,09E-05 | 0,60 |
| CTTN | 470408 | 7,09E-05 | 0,52 |
| POLI | 137720 | 7,40E-05 | 0,38 |
| EPHA4 | 2237263 | 7,94E-05 | 0,53 |
| BAD | 712295 | 8,56E-05 | 0,65 |
| ABCA8 | 284828 | 9,00E-05 | 0,64 |
| MT1F | 81911 | 9,32E-05 | 0,22 |
| ARMCX3 | 251452 | 9,47E-05 | 0,67 |
| ZNF550 | 135705 | 9,85E-05 | 0,60 |
| CES2 | 153667 | 1,14E-04 | 0,67 |
| USP13 | 666007 | 1,14E-04 | 0,54 |
| IMAGE:2266 583 | 2266583 | 1,18E-04 | 0,42 |
| SYCP2 | 136863 | 1,26E-04 | 0,53 |
| CRELD1 | 724119 | 1,31E-04 | 0,67 |
| IMAGE:1362 23 | 136223 | 1,45E-04 | 0,71 |
| CD9 | 727251 | 1,46E-04 | 0,47 |
| CHRDL2 | 485872 | 1,46E-04 | 0,73 |
| ANXA11 | 137238 | 1,47E-04 | 0,73 |
| MESDC2 | 713653 | 1,48E-04 | 0,60 |
| DHRS6 | 364412 | 1,48E-04 | 0,43 |
| LYPLA3 | 471702 | 1,54E-04 | 0,62 |

| Table 3 | AT | Under | |
|---|---|---|---|
| name | cloid | p-value | fold |
| IMAGE:668684 Transcribed locus, weakly similar to XP_209041.2 PREDICTED: similar to KIAA1503 protein [Homo sapiens] | 668684 | 1,61E-04 | 0,45 |
| SYN3 | 727056 | 1,63E-04 | 0,54 |
| BDKRB2 | 665674 | 1,73E-04 | 0,38 |
| TMED4 | 251250 | 1,75E-04 | 0,60 |
| APIN | 364706 | 1,77E-04 | 0,49 |
| IMMP2L | 136260 | 1,84E-04 | 0,57 |
| VPS13D | 136560 | 1,93E-04 | 0,67 |
| KIAA1196 | 738938 | 1,96E-04 | 0,59 |
| IMAGE:382423 Transcribed locus | 382423 | 2,13E-04 | 0,61 |
| RTN3 | 430263 | 2,56E-04 | 0,63 |
| BRAF | 2139164 | 2,57E-04 | 0,50 |
| ALDH1A1 | 309697 | 2,57E-04 | 0,48 |
| DDX1 | 773192 | 2,59E-04 | 0,58 |
| SSH3 | 726272 | 3,01E-04 | 0,59 |
| LOC126669 | 682088 | 3,08E-04 | 0,53 |
| PTPN4 | 666367 | 3,12E-04 | 0,58 |
| PTDSS2 | 21716 | 3,15E-04 | 0,60 |
| PDLIM1 | 135689 | 3,19E-04 | 0,51 |
| PSMD11 | 383945 | 3,44E-04 | 0,76 |
| IMAGE:727289 Transcribed locus | 727289 | 3,46E-04 | 0,68 |
| PML | 724554 | 3,85E-04 | 0,60 |
| ZNF319 | 364729 | 4,16E-04 | 0,34 |
| IMAGE:665649 Transcribed locus | 665649 | 4,42E-04 | 0,64 |
| RGS20 | 668152 | 4,63E-04 | 0,57 |
| GOLPH2 | 811582 | 4,75E-04 | 0,44 |
| LOC80298 | 666551 | 4,76E-04 | 0,54 |
| IMAGE:472111 Transcribed locus | 472111 | 4,88E-04 | 0,43 |
| CTTN | 489945 | 5,35E-04 | 0,62 |
| RASAL2 | 486304 | 5,40E-04 | 0,67 |
| CTBP2 | 416744 | 5,45E-04 | 0,67 |

| Table 3 | AT | Under | |
|---|---|---|---|
| name | cloid | p-value | fold |
| CLECSF12 | 258865 | 5,47E-04 | 0,39 |
| PSCD3 | 744050 | 5,48E-04 | 0,79 |
| KCNK4 | 743016 | 5,49E-04 | 0,65 |
| BMP5 | 1846326 | 5,51E-04 | 0,54 |
| COX8A | 509606 | 5,64E-04 | 0,57 |
| CENTD1 | 666110 | 5,81E-04 | 0,75 |
| ESRRBL1 | 51232 | 6,12E-04 | 0,53 |
| S100A2 | 3659591 | 6,31E-04 | 0,60 |
| FSHPRH1 | 667355 | 6,32E-04 | 0,59 |
| PP1201 | 364974 | 6,47E-04 | 0,58 |
| PDGFD | 666860 | 6,59E-04 | 0,74 |
| CTXN1 | 179266 | 6,77E-04 | 0,63 |
| RYK | 727092 | 7,01E-04 | 0,78 |
| RGS6 | 24176 | 7,34E-04 | 0,55 |
| NT5C2 | 725076 | 7,70E-04 | 0,73 |
| PEF | 137353 | 7,85E-04 | 0,58 |
| GCAT | 307094 | 7,89E-04 | 0,81 |
| IMAGE:23765 | 23765 | 8,14E-04 | 0,60 |
| FBXO21 | 666168 | 8,25E-04 | 0,49 |
| LRP5 | 725836 | 8,38E-04 | 0,60 |
| CREBBP | 172996 | 8,59E-04 | 0,73 |
| LOC92154 | 364926 | 8,87E-04 | 0,69 |
| ALDH3A1 | 525221 | 9,17E-04 | 0,67 |
| NIFIE14 | 472160 | 9,48E-04 | 0,51 |
| SIAT8F | 667110 | 9,49E-04 | 0,61 |
| INHBB | 730012 | 9,53E-04 | 0,52 |
| EGFR | 135980 | 9,54E-04 | 0,55 |
| IMAGE:136014 | 136014 | 9,57E-04 | 0,55 |
| SGCG | 2046361 | 1,01E-03 | 0,76 |
| CDH16 | 726763 | 1,04E-03 | 0,47 |
| IMAGE:731726 Transcribed locus | 731726 | 1,10E-03 | 0,66 |
| AXIN2 | 135887 | 1,13E-03 | 0,60 |
| MDC1 | 365288 | 1,15E-03 | 0,78 |
| TU3A | 44881 | 1,16E-03 | 0,62 |
| SLC12A8 | 724508 | 1,16E-03 | 0,59 |
| ETF1 | 146976 | 1,20E-03 | 0,69 |
| IMAGE:744616 DNA damage repair and recombination protein RAD52 pseudogene | 744616 | 1,24E-03 | 0,58 |
| RNF123 | 366159 | 1,28E-03 | 0,58 |
| DC12 | 724895 | 1,38E-03 | 0,80 |
| PTK2 | 724892 | 1,43E-03 | 0,59 |

| Table 3 | AT | Under | |
|---|---|---|---|
| name | cloid | p-value | fold |
| EVC2 | 668912 | 1,45E-03 | 0,84 |
| ARL6IP2 | 743987 | 1,49E-03 | 0,57 |
| IMAGE:23817 CDNA FLJ12202 fis, clone MAMMA1000908 | 23817 | 1,50E-03 | 0,55 |
| ARID1B | 665538 | 1,55E-03 | 0,72 |
| NEXN | 687625 | 1,60E-03 | 0,72 |
| HNRPF | 136606 | 1,61E-03 | 0,54 |
| MX1 | 713879 | 1,65E-03 | 0,68 |
| IMAGE:26164 | 26164 | 1,67E-03 | 0,68 |
| PRKG1 | 667587 | 1,70E-03 | 0,66 |
| DKFZp547K054 | 731410 | 1,71E-03 | 0,64 |
| IMAGE:743422 | 743422 | 1,74E-03 | 0,65 |
| FBXW8 | 687532 | 1,83E-03 | 0,62 |
| CLDN7 | 726335 | 1,85E-03 | 0,50 |
| DEPDC6 | 669318 | 1,85E-03 | 0,49 |
| SMAD4 | 214611 | 1,90E-03 | 0,55 |
| ZNF651 | 669181 | 1,90E-03 | 0,57 |
| TM4SF8 | 713647 | 1,91E-03 | 0,58 |
| SYNPO | 178792 | 1,92E-03 | 0,68 |
| ABCC8 | 1417901 | 2,05E-03 | 0,61 |
| LOC285500 | 135766 | 2,15E-03 | 0,63 |
| NDUFA7 | 364469 | 2,23E-03 | 0,60 |
| TSHR | 565317 | 2,25E-03 | 0,36 |
| ANP32E | 382738 | 2,31E-03 | 0,61 |
| IMAGE:669188 | 669188 | 2,37E-03 | 0,51 |
| PGR1 | 666361 | 2,37E-03 | 0,77 |
| FLJ13197 | 667117 | 2,38E-03 | 0,58 |
| RHOD | 591907 | 2,39E-03 | 0,56 |
| BTBD14B | 173288 | 2,48E-03 | 0,61 |
| IMAGE:257555 | 257555 | 2,54E-03 | 0,63 |
| CDH1 | 214008 | 2,70E-03 | 0,34 |
| PH-4 | 730938 | 2,75E-03 | 0,58 |
| LPL | 868169 | 2,78E-03 | 0,58 |
| LOC340073 | 731404 | 2,79E-03 | 0,55 |
| C9orf119 | 667112 | 2,84E-03 | 0,57 |
| TERF1 | 135773 | 2,86E-03 | 0,67 |
| LOC388886 | 34007 | 3,02E-03 | 0,53 |
| JAG2 | 1521706 | 3,08E-03 | 0,72 |
| LPXN | 687679 | 3,15E-03 | 0,66 |
| TMEM23 | 730534 | 3,19E-03 | 0,77 |
| KIAA0789 | 33621 | 3,23E-03 | 0,43 |
| GSTM2 | 664233 | 3,33E-03 | 0,49 |
| IMAGE:1351 | 135125 | 3,33E-03 | 0,62 |

| Table 3 | AT | Under | |
|---|---|---|---|
| name | cloid | p-value | fold |
| 25 | | | |
| IGKV1-5 | 155345 | 3,35E-03 | 0,62 |
| KLF1 | 208991 | 3,37E-03 | 0,43 |
| SYT7 | 177827 | 3,38E-03 | 0,65 |
| IMAGE:667353 Transcribed locus | 667353 | 3,59E-03 | 0,76 |
| ZNF76 | 745003 | 3,60E-03 | 0,69 |
| IMAGE:731685 Transcribed locus | 731685 | 3,66E-03 | 0,72 |
| MAP7 | 79729 | 3,98E-03 | 0,49 |
| DOK5 | 25664 | 4,00E-03 | 0,65 |
| PDGFRL | 139242 | 4,04E-03 | 0,58 |
| IMAGE:666140 Transcribed locus, weakly similar to XP_498467.1 PREDICTED: hypothetical protein XP_498467 [Homo sapiens] | 666140 | 4,06E-03 | 0,65 |
| ETV5 | 270549 | 4,26E-03 | 0,63 |
| C6orf176 | 173200 | 4,34E-03 | 0,55 |
| PPIL4 | 364777 | 4,41E-03 | 0,41 |
| TUSC1 | 740381 | 4,50E-03 | 0,61 |
| SOD3 | 795309 | 4,50E-03 | 0,74 |
| C14orf140 | 731423 | 4,52E-03 | 0,52 |
| ARF4L | 49888 | 4,53E-03 | 0,55 |
| RGL2 | 741891 | 4,57E-03 | 0,81 |
| BCL2 | 232714 | 4,61E-03 | 0,68 |
| IMAGE:743290 Transcribed locus | 743290 | 4,66E-03 | 0,64 |
| CAV1 | 133531 | 4,69E-03 | 0,67 |
| C6orf85 | 665379 | 4,74E-03 | 0,71 |
| PIGV | 669319 | 4,80E-03 | 0,59 |
| TIMP2 | 258127 | 5,06E-03 | 0,66 |
| NR2F6 | 1238492 | 5,07E-03 | 0,70 |
| IGF1R | 682555 | 5,09E-03 | 0,65 |
| MFAP3L | 726821 | 5,09E-03 | 0,67 |
| PLXNB2 | 1420676 | 5,10E-03 | 0,58 |
| SATB1 | 364510 | 5,10E-03 | 0,82 |
| DEXI | 668186 | 5,31E-03 | 0,59 |
| VCL | 44193 | 5,31E-03 | 0,63 |

| Table 3 | AT | Under | |
|---|---|---|---|
| name | cloid | p-value | fold |
| HSPC159 | 365045 | 5,58E-03 | 0,60 |
| FLJ22386 | 32917 | 6,06E-03 | 0,63 |
| PLCG1 | 1174287 | 6,11E-03 | 0,74 |
| DAP | 725371 | 6,20E-03 | 0,67 |
| DUSP3 | 119772 | 6,35E-03 | 0,62 |
| EIF2C4 | 730834 | 6,43E-03 | 0,75 |
| IMAGE:252382 | 252382 | 6,53E-03 | 0,59 |
| C10orf6 | 179214 | 6,56E-03 | 0,80 |
| BAK1 | 1288183 | 6,60E-03 | 0,64 |
| GPD1 | 628418 | 6,66E-03 | 0,67 |
| GNAS | 382791 | 6,71E-03 | 0,67 |
| BIN1 | 2384812 | 6,83E-03 | 0,59 |
| KIAA1277 | 364822 | 6,86E-03 | 0,68 |
| IMAGE:726513 Similar to 40S ribosomal protein S3 | 726513 | 6,95E-03 | 0,70 |
| CA11 | 282587 | 7,06E-03 | 0,68 |
| LEMD1 | 731047 | 7,18E-03 | 0,65 |
| ASGR1 | 25883 | 7,35E-03 | 0,56 |
| LOC286170 | 134858 | 7,42E-03 | 0,64 |
| SLC17A2 | 207920 | 7,47E-03 | 0,49 |
| FLJ37587 | 668476 | 7,51E-03 | 0,69 |
| FLJ21159 | 251147 | 7,60E-03 | 0,78 |
| MLPH | 667259 | 7,71E-03 | 0,49 |
| SLIT1 | 38403 | 7,80E-03 | 0,70 |
| ZNF398 | 365311 | 8,35E-03 | 0,60 |
| FBXL16 | 178908 | 8,83E-03 | 0,74 |
| CAV1 | 1911930 | 8,91E-03 | 0,61 |
| TTC7B | 135379 | 9,25E-03 | 0,78 |
| IMAGE:681875 | 681875 | 9,30E-03 | 0,66 |
| GATA2 | 149809 | 9,39E-03 | 0,79 |
| RAP1GA1 | 971276 | 9,50E-03 | 0,66 |
| IMAGE:687667 CDNA clone IMAGE:4811759, partial cds | 687667 | 9,52E-03 | 0,59 |
| TEX13A | 738558 | 9,75E-03 | 0,67 |
| DKFZp566O084 | 136952 | 9,79E-03 | 0,60 |
| KIAA1958 | 687287 | 9,87E-03 | 0,65 |
| ZFOC1 | 744606 | 9,99E-03 | 0,57 |
| DKFZp434M202 | 743118 | 1,02E-02 | 0,63 |
| FLJ13491 | 364352 | 1,02E-02 | 0,72 |
| PPP2CB | 667375 | 1,02E-02 | 0,56 |
| IMAGE:726647 | 726647 | 1,04E-02 | 0,74 |

| Table 3 | AT | Under | |
|---|---|---|---|
| name | cloid | p-value | fold |
| Transcribed locus | | | |
| KCNK5 | 134978 | 1,04E-02 | 0,70 |
| FLJ44005 | 726675 | 1,06E-02 | 0,66 |
| DMD | 743394 | 1,07E-02 | 0,61 |
| IMAGE:738424 | 738424 | 1,12E-02 | 0,72 |
| PINK1 | 729929 | 1,14E-02 | 0,66 |
| DNALI1 | 782688 | 1,16E-02 | 0,53 |
| IMAGE:726782 Transcribed locus | 726782 | 1,20E-02 | 0,64 |
| HT007 | 135303 | 1,22E-02 | 0,79 |
| C9orf10OS | 742695 | 1,30E-02 | 0,60 |
| IMAGE:363955 Transcribed locus | 363955 | 1,31E-02 | 0,62 |
| FLJ37034 | 731348 | 1,32E-02 | 0,70 |
| KRT17 | 366889 | 1,33E-02 | 0,63 |
| TSG101 | 194350 | 1,34E-02 | 0,66 |
| MPO | 436554 | 1,38E-02 | 0,80 |
| IMAGE:668536 CDNA FLJ41685 fis, clone HCASM2006338 | 668536 | 1,39E-02 | 0,47 |
| TETRAN | 725340 | 1,41E-02 | 0,72 |
| IMAGE:174861 Transcribed locus | 174861 | 1,46E-02 | 0,69 |
| IMAGE:1859532 | 1859532 | 1,47E-02 | 0,82 |
| CIRH1A | 261500 | 1,48E-02 | 0,64 |
| UQCRC1 | 714414 | 1,53E-02 | 0,69 |
| LOC286272 | 667361 | 1,54E-02 | 0,70 |
| IMAGE:742739 CDNA FLJ12931 fis, clone NT2RP2004861 | 742739 | 1,55E-02 | 0,65 |
| IMAGE:364741 | 364741 | 1,58E-02 | 0,68 |
| C20orf19 | 366032 | 1,60E-02 | 0,66 |
| LOC147650 | 729510 | 1,61E-02 | 0,70 |
| TSC | 745490 | 1,62E-02 | 0,66 |
| AURKC | 731021 | 1,63E-02 | 0,77 |
| HIS1 | 342551 | 1,65E-02 | 0,65 |
| PCGF4 | 740457 | 1,67E-02 | 0,68 |
| ZNF512 | 726596 | 1,69E-02 | 0,77 |

| Table 3 | AT | Under | |
|---|---|---|---|
| name | cloid | p-value | fold |
| LILRA1 | 2032639 | 1,71E-02 | 0,59 |
| IMAGE:136121 CDNA FLJ10247 fis, clone HEMBB1000705 | 136121 | 1,73E-02 | 0,73 |
| IMAGE:745072 Transcribed locus | 745072 | 1,78E-02 | 0,72 |
| DEFB1 | 665086 | 1,80E-02 | 0,52 |
| IMAGE:738966 Transcribed locus | 738966 | 1,82E-02 | 0,52 |
| NAT5 | 731073 | 1,84E-02 | 0,65 |
| LARS | 135407 | 1,87E-02 | 0,65 |
| FBXO46 | 471664 | 1,96E-02 | 0,60 |
| ECM1 | 301122 | 1,96E-02 | 0,39 |
| HDAC9 | 738506 | 1,97E-02 | 0,65 |
| PSG3 | 136747 | 1,99E-02 | 0,59 |
| SEMA4B | 714437 | 1,99E-02 | 0,73 |
| IMAGE:485104 Transcribed locus | 485104 | 2,05E-02 | 0,63 |
| ITIH5 | 179733 | 2,06E-02 | 0,60 |
| LEF1 | 713913 | 2,07E-02 | 0,68 |
| LOC285961 | 742542 | 2,12E-02 | 0,65 |
| MED19 | 743760 | 2,15E-02 | 0,81 |
| CDC42EP2 | 26651 | 2,15E-02 | 0,68 |
| DPF2 | 743519 | 2,18E-02 | 0,79 |
| AKR1C1 | 196992 | 2,21E-02 | 0,38 |
| IMAGE:665392 Hypothetical LOC388790 | 665392 | 2,23E-02 | 0,81 |
| MRPL45 | 364717 | 2,25E-02 | 0,64 |
| HMG20A | 731277 | 2,28E-02 | 0,72 |
| PKNOX1 | 667067 | 2,32E-02 | 0,72 |
| ACTR1B | 136868 | 2,41E-02 | 0,69 |
| DLX5 | 564878 | 2,45E-02 | 0,57 |
| GGN | 743245 | 2,47E-02 | 0,80 |
| ZNF580 | 668089 | 2,48E-02 | 0,76 |
| CREB3L2 | 136399 | 2,49E-02 | 0,52 |
| IMAGE:383718 | 383718 | 2,52E-02 | 0,71 |
| SLC25A23 | 741954 | 2,58E-02 | 0,52 |
| RP4-622L5 | 742590 | 2,62E-02 | 0,85 |
| LCN2 | 544683 | 2,69E-02 | 0,68 |
| LOC56930 | 134976 | 2,70E-02 | 0,79 |
| FLJ45459 | 365589 | 2,71E-02 | 0,76 |

| Table 3 | AT | Under | |
|---|---|---|---|
| name | cloid | p-value | fold |
| SPATA12 | 730300 | 2,78E-02 | 0,67 |
| GALNT7 | 381854 | 2,83E-02 | 0,73 |
| MCC | 731305 | 2,84E-02 | 0,69 |
| BRMS1 | 2348237 | 2,86E-02 | 0,70 |
| FRMD4B | 669564 | 2,89E-02 | 0,63 |
| ERK8 | 714049 | 2,91E-02 | 0,67 |
| CDR2 | 366067 | 2,94E-02 | 0,76 |
| WIPI49 | 487148 | 3,02E-02 | 0,83 |
| SCEL | 668239 | 3,06E-02 | 0,72 |
| ABCB1 | 1837488 | 3,12E-02 | 0,62 |
| COL18A1 | 359202 | 3,12E-02 | 0,70 |
| MLANA | 266361 | 3,13E-02 | 0,70 |
| LOC283070 | 172785 | 3,14E-02 | 0,71 |
| FGF1 | 25895 | 3,23E-02 | 0,73 |
| PAX8 | 545475 | 3,24E-02 | 0,41 |
| KLHL9 | 501527 | 3,25E-02 | 0,72 |
| IMAGE:726981 Transcribed locus, strongly similar to XP_526325.1 PREDICTED: similar to retinol binding protein 2, cellular; retinol-binding protein 2, cellular [Pan troglodytes] | 726981 | 3,28E-02 | 0,73 |
| CDH3 | 359051 | 3,33E-02 | 0,77 |
| VTN | 230126 | 3,38E-02 | 0,74 |
| PARD3 | 724642 | 3,53E-02 | 0,72 |
| FLT4 | 668815 | 3,53E-02 | 0,68 |
| KIAA0515 | 687852 | 3,55E-02 | 0,71 |
| SIN3A | 26455 | 3,56E-02 | 0,68 |
| CITED2 | 286138 | 3,58E-02 | 0,74 |
| EBAG9 | 682507 | 3,58E-02 | 0,65 |
| FLJ31438 | 135539 | 3,61E-02 | 0,66 |
| HBB | 469549 | 3,63E-02 | 0,50 |
| ACADSB | 687953 | 3,76E-02 | 0,68 |
| KIAA1280 | 366085 | 3,80E-02 | 0,68 |
| MTUS1 | 744395 | 3,88E-02 | 0,60 |
| LOC146909 | 726353 | 3,91E-02 | 0,79 |
| BCAR1 | 324666 | 3,94E-02 | 0,77 |
| LOC339903 | 135352 | 3,97E-02 | 0,64 |
| MESP1 | 25865 | 4,05E-02 | 0,73 |
| S100A1 | 175772 | 4,11E-02 | 0,60 |

| Table 3 | AT | Under | |
|---|---|---|---|
| name | cloid | p-value | fold |
| DNCLI2 | 134671 | 4,18E-02 | 0,71 |
| S100A16 | 730699 | 4,40E-02 | 0,75 |
| IMAGE:22908 Transcribed locus, weakly similar to NP_775735.1 l(3)mbt-like 4 [Homo sapiens] | 22908 | 4,56E-02 | 0,76 |
| DPP4 | 343987 | 4,56E-02 | 0,72 |
| NCAM1 | 366842 | 4,62E-02 | 0,80 |
| MAPK3 | 323438 | 4,64E-02 | 0,74 |
| LRRC28 | 26519 | 4,68E-02 | 0,57 |
| PBXIP1 | 366042 | 4,76E-02 | 0,76 |
| IMAGE:727067 Similar to hypothetical protein SB153 isoform 1 | 727067 | 4,76E-02 | 0,77 |
| EMX2 | 365121 | 4,96E-02 | 0,74 |
| NOTCH1 | 359461 | 4,99E-02 | 0,65 |

| Table 4 | AT | Over | |
|---|---|---|---|
| name | cloid | p-value | fold |
| PTK2B | 43541 | 2,72E-05 | 2,23 |
| ARHGEF6 | 687990 | 4,43E-05 | 2,00 |
| IMAGE:365990 | 365990 | 1,34E-04 | 1,32 |
| STK17A | 562904 | 2,09E-04 | 3,60 |
| HLA-DPB1 | 725548 | 4,45E-04 | 4,47 |
| SIAT4B | 365877 | 4,46E-04 | 1,32 |
| FCHO1 | 742763 | 5,60E-04 | 2,71 |
| GIMAP5 | 180259 | 5,64E-04 | 3,22 |
| CCL13 | 2449149 | 6,35E-04 | 3,15 |
| IMAGE:1089025 | 1089025 | 7,30E-04 | 12,77 |
| IFITM3 | 713623 | 7,87E-04 | 2,21 |
| BTG1 | 382760 | 9,03E-04 | 2,34 |
| IL10RA | 258747 | 9,17E-04 | 4,77 |
| FLJ10858 | 743961 | 9,41E-04 | 1,86 |
| STX7 | 174396 | 1,03E-03 | 7,61 |
| GNAI2 | 724306 | 1,06E-03 | 1,36 |
| IL13 | 1505308 | 1,09E-03 | 1,66 |
| SALL4 | 726454 | 1,11E-03 | 11,25 |
| HCLS1 | 665452 | 1,19E-03 | 5,103473 |
| IMAGE:665833 | 665833 | 1,28E-03 | 1,86 |
| CD38 | 1352408 | 1,34E-03 | 3,46 |
| MGAT3 | 731060 | 1,48E-03 | 2,02 |
| TNFRSF1B | 73703 | 1,49E-03 | 2,56 |
| SULT1E1 | 207926 | 1,52E-03 | 1,23 |
| CERKL | 489326 | 1,70E-03 | 5,60 |
| CD8B1 | 1743279 | 2,38E-03 | 2,01 |
| IL2RB | 139073 | 2,50E-03 | 3,459251 |
| SLAMF1 | 1626951 | 2,54E-03 | 2,51 |
| CBL | 1578721 | 2,62E-03 | 3,37 |
| POU2F2 | 188393 | 2,66E-03 | 2,90 |
| RNASE4 | 156720 | 2,74E-03 | 1,84 |
| GATA4 | 781738 | 3,03E-03 | 3,80 |
| PDK1 | 1645668 | 3,07E-03 | 2,03 |
| SRGAP2 | 714493 | 3,13E-03 | 1,25 |
| HDAC1 | 1896337 | 3,50E-03 | 2,16 |
| GC | 195340 | 3,69E-03 | 4,97 |
| ACTB | 203166 | 3,97E-03 | 1,91 |
| F10 | 310519 | 4,05E-03 | 1,580546 |
| NBL1 | 503874 | 5,07E-03 | 6,38 |
| IMAGE:743722 | 743722 | 5,15E-03 | 3,83 |
| YWHAH | 324066 | 5,22E-03 | 1,45 |
| PDCD1LG2 | 738944 | 5,28E-03 | 2,78 |
| PLCG2 | 201467 | 5,28E-03 | 2,986551 |
| APOE | 1870594 | 5,58E-03 | 5,323048 |
| RUNX3 | 122874 | 5,87E-03 | 4,16 |
| GSS | 140405 | 6,10E-03 | 2,46 |
| FXYD1 | 204686 | 6,16E-03 | 1,62 |

| Table 4 | AT | Over | |
|---|---|---|---|
| name | cloid | p-value | fold |
| IMAGE:2497617 | 2497617 | 6,40E-03 | 1,77 |
| STAT2 | 2306096 | 6,71E-03 | 1,74 |
| CCL19 | 1707527 | 7,61E-03 | 34,27 |
| BAZ2B | 609631 | 7,62E-03 | 2,15 |
| CASP4 | 356960 | 8,00E-03 | 3,00 |
| CCR7 | 2345206 | 8,18E-03 | 2,62 |
| IFI30 | 740931 | 8,38E-03 | 4,70 |
| IMAGE:342256 | 342256 | 8,64E-03 | 21,04 |
| KLRK1 | 725473 | 9,04E-03 | 3,94 |
| IRF4 | 682207 | 9,24E-03 | 4,80 |
| TYMS | 2242054 | 9,47E-03 | 1,85 |
| S100A4 | 868577 | 9,52E-03 | 2,49 |
| IMAGE:727154 Transcribed locus | 727154 | 9,72E-03 | 2,26 |
| CASP7 | 279470 | 9,89E-03 | 1,99 |
| TOSO | 813174 | 9,90E-03 | 3,28 |
| FLJ12592 | 252489 | 1,06E-02 | 1,94 |
| LILRB4 | 2341829 | 1,06E-02 | 1,47 |
| CPXM2 | 729924 | 1,12E-02 | 2,72 |
| RUNX1 | 263251 | 1,12E-02 | 1,56 |
| UBE1L | 250883 | 1,13E-02 | 1,73 |
| IMAGE:738896 | 738896 | 1,14E-02 | 18,07 |
| NCK1 | 1326169 | 1,14E-02 | 5,22 |
| SEPT-05 | 448163 | 1,15E-02 | 18,97 |
| NFYA | 731648 | 1,17E-02 | 2,21 |
| IMAGE:2113771 | 2113771 | 1,21E-02 | 2,22 |
| HNRPA3 | 365349 | 1,21E-02 | 1,22 |
| WDR5 | 731023 | 1,24E-02 | 2,80 |
| MLL4 | 744980 | 1,29E-02 | 1,81 |
| NYREN18 | 668584 | 1,31E-02 | 2,21 |
| IMAGE:727496 Transcribed locus | 727496 | 1,32E-02 | 6,10 |
| IMAGE:132933 | 132933 | 1,33E-02 | 1,21 |
| LASP1 | 592598 | 1,39E-02 | 11,76 |
| IMAGE:46991 Similar to breakpoint cluster region isoform 1 | 46991 | 1,40E-02 | 4,89 |
| BID | 128065 | 1,41E-02 | 2,16 |
| SMARCA5 | 730037 | 1,48E-02 | 2,82 |
| CD5 | 356841 | 1,49E-02 | 2,31 |
| PIK3CA | 2238108 | 1,51E-02 | 11,79 |
| CASP3 | 823680 | 1,55E-02 | 2,51 |

| Table 4 | AT | Over | |
|---|---|---|---|
| name | cloid | p-value | fold |
| DAXX | 292042 | 1,56E-02 | 1,91 |
| IMAGE:504539 | 504539 | 1,56E-02 | 1,91 |
| RGS19 | 485803 | 1,61E-02 | 3,13 |
| LEFTY2 | 340657 | 1,62E-02 | 3,621832 |
| MGP | 590264 | 1,76E-02 | 2,58 |
| GADD45GIP1 | 1084386 | 1,79E-02 | 2,82 |
| IMAGE:742853 | 742853 | 1,81E-02 | 9,42 |
| SEMA4D | 210587 | 1,81E-02 | 3,62 |
| HMGB2 | 884365 | 1,83E-02 | 1,87 |
| NOMO1 | 134615 | 1,84E-02 | 1,70 |
| IMAGE:740927 | 740927 | 1,91E-02 | 1,46 |
| FABP5 | 1088781 | 2,02E-02 | 14,00 |
| KLRB1 | 2091591 | 2,03E-02 | 4,00 |
| G3BP | 667239 | 2,08E-02 | 4,05 |
| HPS4 | 727430 | 2,09E-02 | 2,04 |
| ZNF251 | 486401 | 2,15E-02 | 14,82 |
| TRAF5 | 1286238 | 2,15E-02 | 2,90 |
| MAPKAPK2 | 742577 | 2,17E-02 | 1,56 |
| STAT6 | 1916307 | 2,19E-02 | 1,79 |
| IL2RA | 3054031 | 2,20E-02 | 1,81 |
| APOL3 | 366289 | 2,26E-02 | 3,52 |
| DGKQ | 366233 | 2,31E-02 | 1,84 |
| MLL | 80688 | 2,32E-02 | 1,78 |
| APOL1 | 665632 | 2,35E-02 | 4,01 |
| IRAK1 | 379200 | 2,41E-02 | 1,64 |
| TIMP1 | 162246 | 2,47E-02 | 4,54 |
| FLJ27523 | 743246 | 2,48E-02 | 12,16 |
| CRYZ | 486935 | 2,48E-02 | 1,46 |
| GRM3 | 287843 | 2,56E-02 | 4,69 |
| MYO5A | 365755 | 2,62E-02 | 1,44 |
| BST2 | 811024 | 2,65E-02 | 2,64 |
| CKLFSF3 | 489249 | 2,70E-02 | 1,54651 |
| CCL5 | 840753 | 2,74E-02 | 1,63 |
| IMAGE:731689 Transcribed locus | 731689 | 2,74E-02 | 1,25 |
| BAI3 | 50491 | 2,77E-02 | 4,54 |
| LILRB3 | 1422194 | 2,78E-02 | 2,77 |
| ATR | 1251197 | 2,79E-02 | 2,31 |
| PDCD1LG1 | 1942634 | 2,81E-02 | 2,44 |
| LOC440712 | 725268 | 2,85E-02 | 2,10 |
| DCN | 666410 | 2,87E-02 | 1,70 |
| IMAGE:743415 Transcribed locus | 743415 | 2,88E-02 | 2,30 |
| IKBKE | 364448 | 2,91E-02 | 1,49 |

| Table 4 | AT | Over | |
|---|---|---|---|
| name | cloid | p-value | fold |
| NCB5OR | 743367 | 3,01E-02 | 1,99 |
| MAP2 | 1287638 | 3,19E-02 | 1,44 |
| DDR2 | 668442 | 3,20E-02 | 1,17 |
| SYT1 | 28511 | 3,26E-02 | 1,61 |
| PPP1CA | 1422791 | 3,29E-02 | 1,43 |
| IMAGE:743224 Transcribed locus, strongly similar to XP_518162.1 PREDICTED: similar to Zinc finger protein 62 homolog (Zfp-62) (ZT3) [Pan troglodytes] | 743224 | 3,34E-02 | 1,76 |
| VIL2 | 124701 | 3,35E-02 | 1,87 |
| CECR1 | 725612 | 3,36E-02 | 1,33 |
| HN1 | 471568 | 3,55E-02 | 2,25 |
| ICAM3 | 156183 | 3,60E-02 | 3,73 |
| MUC1 | 840687 | 3,66E-02 | 2,25 |
| IMAGE:731751 Transcribed locus | 731751 | 3,66E-02 | 1,33 |
| S100A12 | 1705397 | 3,67E-02 | 2,13 |
| PTPNS1 | 26061 | 3,87E-02 | 1,894355 |
| NRP1 | 666061 | 3,89E-02 | 1,38 |
| NEK6 | 725345 | 3,92E-02 | 2,27 |
| PREB | 740347 | 3,94E-02 | 4,022672 |
| SFN | 346610 | 4,09E-02 | 1,65 |
| FOLR1 | 131839 | 4,11E-02 | 1,47 |
| IFNAR2 | 123950 | 4,16E-02 | 3,29 |
| BCAP29 | 2336358 | 4,24E-02 | 2,02 |
| TNIP1 | 135801 | 4,27E-02 | 1,59 |
| TOP1 | 666425 | 4,28E-02 | 1,24 |
| ETV4 | 809959 | 4,42E-02 | 1,23 |
| FGF4 | 1550616 | 4,53E-02 | 1,40 |
| WAS | 2148946 | 4,57E-02 | 3,27 |
| KLRD1 | 2728204 | 4,58E-02 | 1,80 |
| SNX8 | 366585 | 4,61E-02 | 1,57 |
| KPNA2 | 667727 | 4,62E-02 | 1,44 |
| PMS2 | 116906 | 4,68E-02 | 4,11 |
| SLAMF8 | 288807 | 4,80E-02 | 3,22 |
| CXCL14 | 345034 | 4,82E-02 | 4,25 |
| DNMT1 | 768241 | 4,91E-02 | 1,71 |
| PARP9 | 667440 | 4,91E-02 | 1,75 |

| Table 5 | GD | Under | |
|---|---|---|---|
| name | cloid | p-value | fold |
| DOK5 | 25664 | 2,9346E-09 | 0,57 |
| LPL | 868169 | 1,1605E-06 | 0,55 |
| ARHGAP10 | 731380 | 4,7117E-06 | 0,65 |
| CLGN | 1049033 | 9,1105E-06 | 0,61 |
| DECR2 | 730942 | 1,5383E-05 | 0,61 |
| IMAGE:731616 MRNA; cDNA DKFZp686A102 (from clone DKFZp686A102) | 731616 | 1,852E-05 | 0,59 |
| IQCF3 | 726578 | 2,3435E-05 | 0,71 |
| PSAT1 | 366388 | 3,1255E-05 | 0,79 |
| CHRDL2 | 485872 | 3,6361E-05 | 0,71 |
| SERPINB5 | 1662274 | 0,00015833 | 0,66 |
| ICOSLG | 2074228 | 0,00018032 | 0,72 |
| OBSCN | 730926 | 0,00018977 | 0,81 |
| ANKRD20B | 745106 | 0,00019641 | 0,57 |
| AURKC | 731021 | 0,00020316 | 0,57 |
| SLC17A2 | 207920 | 0,00021513 | 0,47 |
| CLECSF12 | 258865 | 0,00041555 | 0,34 |
| VEGFC | 503189 | 0,00048933 | 0,59 |
| TSC | 745490 | 0,00057708 | 0,51 |
| LAMB3 | 1103402 | 0,00060694 | 0,61 |
| CAV1 | 1911930 | 0,00067972 | 0,61 |
| MCC | 731305 | 0,00108212 | 0,55 |
| NOLA2 | 173296 | 0,00116175 | 0,69 |
| ZNF651 | 669181 | 0,00116222 | 0,76 |
| IMAGE:727491 | 727491 | 0,00121836 | 0,45 |
| MX1 | 713879 | 0,00133899 | 0,64 |
| NOTCH1 | 359461 | 0,00137335 | 0,72 |
| CITED1 | 265558 | 0,00146133 | 0,52 |
| IMAGE:687667 CDNA clone IMAGE:4811759, partial cds | 687667 | 0,00149013 | 0,5 |
| SCEL | 668239 | 0,00152752 | 0,65 |
| IMAGE:232366 CDNA FLJ37672 fis, clone BRHIP2012059 | 232366 | 0,00210682 | 0,61 |
| C21orf84 | 730814 | 0,0021117 | 0,67 |
| AOC3 | 484535 | 0,00216625 | 0,7 |
| IMP-2 | 743774 | 0,00217549 | 0,52 |
| PRKG1 | 667587 | 0,00219736 | 0,67 |

| Table 5 | GD | Under | |
|---|---|---|---|
| name | cloid | p-value | fold |
| DEFB1 | 665086 | 0,00251628 | 0,42 |
| KRT17 | 366889 | 0,00252804 | 0,71 |
| SAP30L | 723950 | 0,00263794 | 0,78 |
| PANK1 | 256177 | 0,00274492 | 0,75 |
| IMAGE:1859532 | 1859532 | 0,00278248 | 0,77 |
| C20orf18 | 665433 | 0,00297457 | 0,62 |
| PIP | 985457 | 0,00350912 | 0,8 |
| DLX5 | 564878 | 0,00369818 | 0,66 |
| ST5 | 376356 | 0,00372025 | 0,79 |
| MAPRE2 | 383868 | 0,00390703 | 0,64 |
| FBXO46 | 471664 | 0,00493537 | 0,6 |
| PARD3 | 724642 | 0,00552617 | 0,73 |
| IMAGE:23817 CDNA FLJ12202 fis, clone MAMMA1000908 | 23817 | 0,00606222 | 0,5 |
| DCXR | 724596 | 0,00613681 | 0,58 |
| LRRIQ2 | 487152 | 0,00618225 | 0,83 |
| EPRS | 669549 | 0,00624669 | 0,71 |
| IGKV1-5 | 155345 | 0,00626237 | 0,63 |
| CXCL13 | 347362 | 0,00699423 | 0,83 |
| TOP1 | 666425 | 0,00725755 | 0,74 |
| ETV1 | 24541 | 0,00727553 | 0,68 |
| RFPL1 | 290265 | 0,00745468 | 0,69 |
| FLJ25067 | 667252 | 0,0075477 | 0,72 |
| MGC45840 | 725231 | 0,00862765 | 0,73 |
| DPP4 | 343987 | 0,00891505 | 0,55 |
| RBBP4 | 705147 | 0,00896277 | 0,77 |
| NR2F6 | 1238492 | 0,00901903 | 0,7 |
| IMAGE:745072 Transcribed locus | 745072 | 0,00950104 | 0,59 |
| LCN2 | 544683 | 0,01012252 | 0,63 |
| IL2RA | 3054031 | 0,01017643 | 0,84 |
| IMAGE:744604 | 744604 | 0,01062584 | 0,59 |
| NAT5 | 731073 | 0,0108843 | 0,67 |
| EPHA4 | 2237263 | 0,01141372 | 0,58 |
| CCL28 | 136919 | 0,01174159 | 0,83 |
| PPAP2C | 486447 | 0,01184402 | 0,73 |
| TPM2 | 740620 | 0,01184508 | 0,68 |
| PLXNB2 | 1420676 | 0,0119905 | 0,72 |
| FOXO1A | 151247 | 0,01279281 | 0,8 |
| TEX13A | 738558 | 0,01337572 | 0,74 |
| AD023 | 731645 | 0,01337901 | 0,68 |
| TPCN1 | 179288 | 0,01386141 | 0,67 |
| IMAGE:667683 Full- | 667683 | 0,01420475 | 0,8 |

| Table 5 | GD | Under | |
|---|---|---|---|
| name | cloid | p-value | fold |
| length cDNA clone CS0DK008YI09 of HeLa cells Cot 25-normalized of Homo sapiens (human) | | | |
| IMAGE:135125 | 135125 | 0,01424832 | 0,65 |
| SPINT1 | 723914 | 0,0145432 | 0,83 |
| CENTD1 | 666110 | 0,01458746 | 0,66 |
| GALNT7 | 381854 | 0,01458803 | 0,65 |
| KIAA1754L | 365056 | 0,015234 | 0,76 |
| DJ462O23.2 | 738970 | 0,01526675 | 0,82 |
| PML | 724554 | 0,01579712 | 0,84 |
| ECM1 | 301122 | 0,01646653 | 0,37 |
| IMAGE:382521 | 382521 | 0,01686383 | 0,67 |
| CASP5 | 341763 | 0,01711585 | 0,67 |
| PSG3 | 136747 | 0,01796368 | 0,64 |
| MRPL45 | 364717 | 0,01798173 | 0,59 |
| IMAGE:484577 Transcribed locus, weakly similar to NP_000991.1 ribosomal protein L39; 60S ribosomal protein L39 [Homo sapiens] | 484577 | 0,01809095 | 0,58 |
| FLJ22471 | 668510 | 0,0182197 | 0,6 |
| KLRD1 | 145696 | 0,01870053 | 0,72 |
| CLDN1 | 594279 | 0,01895465 | 0,39 |
| TETRAN | 725340 | 0,01932623 | 0,72 |
| RAB8A | 712118 | 0,01937338 | 0,88 |
| HP | 82687 | 0,01968016 | 0,79 |
| LOC120224 | 252291 | 0,01972775 | 0,61 |
| LOC80298 | 666551 | 0,01993545 | 0,7 |
| C7orf27 | 713859 | 0,02018391 | 0,92 |
| CDH3 | 359051 | 0,02040993 | 0,64 |
| CGI-128 | 786662 | 0,02095299 | 0,73 |
| LCN2 | 741497 | 0,02142784 | 0,4 |
| IMAGE:731298 CDNA FLJ42482 fis, clone BRACE2032 | 731298 | 0,02244117 | 0,6 |

| Table 5 | GD | Under | |
|---|---|---|---|
| name | cloid | p-value | fold |
| 134 | | | |
| MKKS | 729957 | 0,02271256 | 0,64 |
| IMAGE:743903 Homo sapiens, clone IMAGE:5266541, mRNA | 743903 | 0,02274312 | 0,59 |
| IGF1R | 682555 | 0,02318362 | 0,79 |
| GCK | 30981 | 0,02333357 | 0,76 |
| LATS2 | 666778 | 0,02359943 | 0,75 |
| LARS | 135407 | 0,02385825 | 0,68 |
| IMAGE:742919 Transcribed locus | 742919 | 0,02420455 | 0,84 |
| IMAGE:378458 | 378458 | 0,02425923 | 0,78 |
| IMAGE:743579 | 743579 | 0,02441628 | 0,71 |
| TIMP2 | 258127 | 0,02458546 | 0,71 |
| GLG1 | 365613 | 0,02464356 | 0,83 |
| CCNE1 | 357807 | 0,02635602 | 0,75 |
| IMAGE:383528 Transcribed locus, weakly similar to XP_375099.1 PREDICTED: similar to hypothetical protein FLJ25224 [Homo sapiens] | 383528 | 0,02656958 | 0,36 |
| ALDH1A3 | 486189 | 0,0273157 | 0,69 |
| KIAA1196 | 738938 | 0,02776484 | 0,76 |
| AQP4 | 279172 | 0,02819313 | 0,69 |
| IMAGE:665649 Transcribed locus | 665649 | 0,02902427 | 0,72 |
| ALCAM | 172828 | 0,02935177 | 0,72 |
| IMAGE:738332 Transcribed locus | 738332 | 0,02979191 | 0,53 |
| APLP2 | 549054 | 0,03108305 | 0,8 |
| C9orf10OS | 742695 | 0,03165276 | 0,77 |
| IMAGE:213529 | 213529 | 0,03175448 | 0,88 |

| Table 5 | GD | Under | |
|---|---|---|---|
| name | cloid | p-value | fold |
| Transcribed locus | | | |
| SOSTDC1 | 667048 | 0,03195182 | 0,8 |
| GCAT | 307094 | 0,03222091 | 0,87 |
| MPO | 436554 | 0,03392843 | 0,73 |
| CCND1 | 324079 | 0,03432325 | 0,65 |
| CD47 | 365526 | 0,03494508 | 0,84 |
| MRPS11 | 471574 | 0,03589496 | 0,68 |
| PEMT | 742580 | 0,03618273 | 0,71 |
| PKIG | 666277 | 0,03628293 | 0,64 |
| BRMS1 | 2348237 | 0,0374802 | 0,7 |
| FLJ35630 | 485652 | 0,03841929 | 0,65 |
| ZFOC1 | 744606 | 0,03860826 | 0,74 |
| LOC51760 | 52226 | 0,03910408 | 0,76 |
| C22orf16 | 236119 | 0,03972491 | 0,75 |
| IMAGE:730 924 Transcribed locus | 730924 | 0,04130682 | 0,86 |
| C6orf176 | 173200 | 0,04189653 | 0,75 |
| HIS1 | 342551 | 0,04212231 | 0,63 |
| PLA2G6 | 744087 | 0,04281272 | 0,8 |
| CXCL14 | 345034 | 0,04431193 | 0,53 |
| PPP2R4 | 731590 | 0,04445623 | 0,79 |
| DKFZp434M 202 | 743118 | 0,04619395 | 0,77 |
| CIRH1A | 261500 | 0,04708019 | 0,76 |
| IMAGE:136 223 | 136223 | 0,04869167 | 0,77 |
| IMAGE:668 536 CDNA FLJ41685 fis, clone HCASM200 6338 | 668536 | 0,04890577 | 0,58 |
| TM7SF3 | 666928 | 0,04909454 | 0,79 |
| TCEA2 | 730149 | 0,04988725 | 0,66 |

| Table 6 | GD | Over | |
|---|---|---|---|
| name | cloid | p-value | fold |
| ARHGEF6 | 687990 | 1,40E-02 | 1,46 |
| FLJ10858 | 743961 | 2,05E-02 | 1,28 |
| GPR124 | 486493 | 2,48E-02 | 1,47 |
| IMAGE:3792 79 | 379279 | 2,61 E-02 | 1,18 |

(continued)

| Table 6 | GD | Over | |
|---|---|---|---|
| name | cloid | p-value | fold |
| RIPK3 | 667313 | 4,78E-02 | 1,35 |

| Table 7 | FTA-a | Under | |
|---|---|---|---|
| name | cloid | p-value | fold |
| BRMS1 | 2348237 | 1,2E-07 | 0,66 |
| TTC7B | 135379 | 2,4E-07 | 0,63 |
| FBXO10 | 743851 | 3,4E-07 | 0,68 |
| ZNF217 | 1712992 | 3,8E-07 | 0,71 |
| LOC90110 | 376699 | 5,4E-07 | 0,71 |
| ATR | 1251197 | 5,9E-07 | 0,75 |
| PLCG1 | 1174287 | 6,4E-07 | 0,58 |
| CACNB2 | 173841 | 9,4E-07 | 0,58 |
| VDAC1 | 486221 | 1,2E-06 | 0,60 |
| CDKN1B | 265345 | 1,8E-06 | 0,71 |
| LOC91526 | 666564 | 2E-06 | 0,68 |
| IMAGE:252382 | 252382 | 3,6E-06 | 0,80 |
| C1QBP | 173371 | 5,4E-06 | 0,68 |
| NAT5 | 731073 | 5,6E-06 | 0,68 |
| NR2F1 | 253386 | 5,9E-06 | 0,67 |
| SPI1 | 1285305 | 7,4E-06 | 0,70 |
| BNIP3 | 342700 | 1E-05 | 0,69 |
| C10orf6 | 179214 | 1,1E-05 | 0,75 |
| IL2RA | 3054031 | 1,3E-05 | 0,70 |
| TFRC | 359797 | 1,6E-05 | 0,66 |
| ALDH1A3 | 486189 | 1,9E-05 | 0,62 |
| CLECSF12 | 258865 | 1,9E-05 | 0,43 |
| C6orf216 | 731742 | 2,1E-05 | 0,71 |
| NRP1 | 666061 | 2,2E-05 | 0,68 |
| ZNF550 | 135705 | 2,3E-05 | 0,66 |
| IMAGE:745072 Transcribed locus | 745072 | 2,4E-05 | 0,62 |
| GLRX2 | 731044 | 3,1E-05 | 0,74 |
| IMAGE:731616 MRNA; cDNA DKFZp686A102 (from clone DKFZp686A102) | 731616 | 3,5E-05 | 0,56 |
| WAS | 2148946 | 4,2E-05 | 0,61 |
| RPS6KA5 | 258966 | 5,3E-05 | 0,72 |
| IMAGE:504539 | 504539 | 5,4E-05 | 0,70 |
| MDH1 | 725188 | 5,4E-05 | 0,73 |
| IMAGE:213529 Transcrib | 213529 | 5,5E-05 | 0,71 |

| Table 7 | FTA-a | Under | |
|---|---|---|---|
| name | cloid | p-value | fold |
| ed locus | | | |
| IMAGE:742919 Transcribed locus | 742919 | 8,2E-05 | 0,82 |
| IMAGE:682585 | 682585 | 9,5E-05 | 0,72 |
| HSPH1 | 666554 | 0,00011 | 0,71 |
| RAE1 | 825224 | 0,00011 | 0,79 |
| CLGN | 1049033 | 0,00012 | 0,67 |
| HSPC159 | 365045 | 0,00013 | 0,73 |
| IMAGE:742837 Transcribed locus | 742837 | 0,00014 | 0,67 |
| KIAA0427 | 127507 | 0,00014 | 0,78 |
| ANKRD32 | 725198 | 0,00015 | 0,71 |
| PMS2 | 116906 | 0,00016 | 0,61 |
| MORF4L1 | 135875 | 0,00018 | 0,78 |
| TERF1 | 135773 | 0,00019 | 0,74 |
| CDKL1 | 486282 | 0,00021 | 0,74 |
| DPP4 | 343987 | 0,00021 | 0,52 |
| IMAGE:232366 CDNA FLJ37672 fis, clone BRHIP2012059 | 232366 | 0,00022 | 0,71 |
| RAD51C | 80162 | 0,00023 | 0,75 |
| COX7B | 566862 | 0,00026 | 0,64 |
| IMAGE:724544 Similar to 40S ribosomal protein S25 | 724544 | 0,00027 | 0,74 |
| SLC36A3 | 731115 | 0,00027 | 0,77 |
| CKS2 | 725454 | 0,00028 | 0,68 |
| TOSO | 813174 | 0,00032 | 0,69 |
| LEFTY2 | 340657 | 0,00033 | 0,68 |
| APOD | 838611 | 0,00034 | 0,37 |
| TMEM23 | 730534 | 0,00037 | 0,82 |
| FCHO1 | 742763 | 0,00038 | 0,74 |
| RPL27A | 178255 | 0,00039 | 0,57 |
| VPS26 | 687336 | 0,0004 | 0,78 |
| LOC285961 | 742542 | 0,00042 | 0,80 |

| Table 7 | FTA-a | Under | |
|---|---|---|---|
| name | cloid | p-value | fold |
| IMAGE:6 67683 Full-length cDNA clone CS0DK0 08YI09 of HeLa cells Cot 25-normalized of Homo sapiens (human) | 667683 | 0,00046 | 0,78 |
| KLRK1 | 725473 | 0,00046 | 0,72 |
| IL2RB | 139073 | 0,0005 | 0,67 |
| MUTED | 731202 | 0,0006 | 0,72 |
| CLDN1 | 594279 | 0,00067 | 0,34 |
| IMAGE:7 27289 Transcribed locus | 727289 | 0,00068 | 0,61 |
| IMAGE:3 82423 Transcribed locus | 382423 | 0,0007 | 0,65 |
| IMAGE:7 24556 Transcribed locus | 724556 | 0,00071 | 0,78 |
| PCGF4 | 740457 | 0,00071 | 0,69 |
| IGSF10 | 682276 | 0,00072 | 0,76 |
| PGR1 | 666361 | 0,00075 | 0,81 |
| MX1 | 713879 | 0,0008 | 0,73 |
| DEK | 1016390 | 0,00086 | 0,77 |
| SIAT8F | 667110 | 0,00092 | 0,70 |
| NOTCH1 | 359461 | 0,00095 | 0,76 |
| DOK5 | 25664 | 0,00096 | 0,71 |
| DPF2 | 743519 | 0,00099 | 0,77 |
| OR7E38P | 486540 | 0,00099 | 0,82 |
| PDK1 | 1645668 | 0,00101 | 0,78 |
| IMAGE:7 44899 Homo sapiens, clone IMAGE:4 837072, mRNA | 744899 | 0,00102 | 0,75 |
| STK17A | 562904 | 0,00103 | 0,70 |
| PRKCABP | 1174342 | 0,00108 | 0,82 |
| TIMP2 | 258127 | 0,00111 | 0,78 |
| STAT6 | 1916307 | 0,00118 | 0,82 |
| ATIC | 665693 | 0,00119 | 0,80 |
| LOC157697 | 714109 | 0,00119 | 0,79 |
| IMAGE:6 65649 Transcribed locus | 665649 | 0,00119 | 0,75 |
| MEF2C | 687270 | 0,0012 | 0,80 |
| PDCD1LG1 | 1942634 | 0,0012 | 0,74 |
| CD38 | 1352408 | 0,00123 | 0,80 |
| OSR1 | 364686 | 0,00127 | 0,76 |
| CD47 | 357442 | 0,0013 | 0,80 |
| MKKS | 729957 | 0,00136 | 0,80 |
| PSIP1 | 667598 | 0,00142 | 0,81 |
| SF3B1 | 739247 | 0,00146 | 0,79 |
| ANP32E | 382738 | 0,00146 | 0,69 |
| KLRD1 | 145696 | 0,00147 | 0,80 |
| CBL | 1578721 | 0,00147 | 0,73 |
| LCN2 | 544683 | 0,00153 | 0,54 |
| IMAGE:7 45512 | 745512 | 0,00168 | 0,79 |
| PTPN4 | 249311 | 0,0017 | 0,77 |
| RBBP4 | 705147 | 0,00175 | 0,77 |
| TIGD7 | 727482 | 0,0018 | 0,82 |
| CD47 | 365526 | 0,00182 | 0,80 |
| SPATA5L1 | 724884 | 0,00184 | 0,71 |
| UBE2E3 | 1575056 | 0,00188 | 0,77 |
| H17 | 739432 | 0,00193 | 0,77 |
| IMAGE:7 43224 Transcribed locus, strongly similar to XP_5181 62.1 PREDICTED: similar to Zinc finger protein 62 homolog (Zfp-62) (ZT3) [Pan troglodytes] | 743224 | 0,00197 | 0,72 |
| DNPEP | 730779 | 0,00205 | 0,77 |

| Table 7 | FTA-a | Under | |
|---|---|---|---|
| name | cloid | p-value | fold |
| ANXA11 | 137238 | 0,00209 | 0,86 |
| ABCA8 | 284828 | 0,00213 | 0,70 |
| SOSTDC 1 | 667048 | 0,00216 | 0,77 |
| SENP6 | 739237 | 0,00216 | 0,75 |
| LEMD1 | 731047 | 0,00224 | 0,67 |
| NCK1 | 1326169 | 0,00225 | 0,69 |
| NSUN4 | 364570 | 0,00231 | 0,82 |
| STX7 | 174396 | 0,00244 | 0,63 |
| DEFB1 | 665086 | 0,00244 | 0,40 |
| BAK1 | 235938 | 0,00245 | 0,74 |
| PDGFD | 666860 | 0,00245 | 0,80 |
| DMD | 743394 | 0,00246 | 0,70 |
| HIVEP1 | 758037 | 0,00248 | 0,79 |
| ATP5G3 | 740090 | 0,00255 | 0,82 |
| UBE2D2 | 594655 | 0,00275 | 0,81 |
| MEOX1 | 760065 | 0,00296 | 0,75 |
| FOXN4 | 731076 | 0,00297 | 0,78 |
| SPDEF | 1188588 | 0,00302 | 0,77 |
| LOC5625 1 | 137478 | 0,00312 | 0,77 |
| LPL | 868169 | 0,0032 | 0,72 |
| BAP1 | 1012990 | 0,00336 | 0,73 |
| SIAT8C | 382069 | 0,00336 | 0,77 |
| TOP1 | 666425 | 0,00339 | 0,85 |
| IMAGE:1 35203 Transcrib ed locus | 135203 | 0,00347 | 0,79 |
| CASP5 | 341763 | 0,00364 | 0,67 |
| EPRS | 669549 | 0,00368 | 0,83 |
| FAM44C | 726894 | 0,0038 | 0,84 |
| HMG20A | 731277 | 0,00417 | 0,72 |
| IMAGE:4 85104 Transcrib ed locus | 485104 | 0,00423 | 0,69 |
| SCEL | 668239 | 0,00425 | 0,82 |
| IRF4 | 682207 | 0,00432 | 0,74 |
| RIN1 | 741406 | 0,0044 | 0,78 |
| PLCG2 | 201467 | 0,00461 | 0,78 |
| FABP5 | 1088781 | 0,00488 | 0,47 |
| MAK | 382002 | 0,00498 | 0,74 |
| ELK4 | 236155 | 0,00507 | 0,76 |
| IMAGE:7 31758 Transcrib ed locus, weakly similar to NP_0810 70.1 kiaa-iso | 731758 | 0,00521 | 0,77 |

| Table 7 | FTA-a | Under | |
|---|---|---|---|
| name | cloid | p-value | fold |
| protein homolog; Band 47B [Mus musculus ] | | | |
| DAPK1 | 364934 | 0,00547 | 0,82 |
| MRC2 | 235882 | 0,00557 | 0,58 |
| SYNPO | 178792 | 0,00562 | 0,81 |
| KIAA175 4L | 365056 | 0,00563 | 0,76 |
| RGS20 | 668152 | 0,00594 | 0,78 |
| HCLS1 | 665452 | 0,00599 | 0,70 |
| NY-REN-41 | 136646 | 0,00606 | 0,79 |
| CENTD1 | 666110 | 0,0061 | 0,81 |
| PHC2 | 668146 | 0,00637 | 0,79 |
| MAP2 | 347503 | 0,00639 | 0,74 |
| IMAGE:7 44616 DNA damage repair and recombin ation protein RAD52 pseudog ene | 744616 | 0,00648 | 0,78 |
| IMAGE:1 77857 Transcrib ed locus | 177857 | 0,00656 | 0,79 |
| IL10RA | 258747 | 0,00664 | 0,70 |
| WDR20 | 665156 | 0,00676 | 0,81 |
| CTRL | 1308954 | 0,00707 | 0,82 |
| FBXO46 | 471664 | 0,00716 | 0,72 |
| CHRDL2 | 485872 | 0,0075 | 0,80 |
| LRPAP1 | 587186 | 0,00762 | 0,60 |
| CCL28 | 136919 | 0,00767 | 0,84 |
| SLC39A2 | 504596 | 0,00779 | 0,83 |
| CITED1 | 265558 | 0,0078 | 0,49 |
| LARS | 135407 | 0,00805 | 0,79 |
| ICAM3 | 156183 | 0,00823 | 0,79 |
| IMAGE:4 84577 Transcrib ed locus, weakly similar to NP_0009 91.1 ribosomal | 484577 | 0,00852 | 0,68 |

| Table 7 | FTA-a | Under | |
|---|---|---|---|
| name | cloid | p-value | fold |
| protein L39; 60S ribosomal protein L39 [Homo sapiens] | | | |
| RASGRP1 | 725707 | 0,00864 | 0,68 |
| C6orf176 | 173200 | 0,00866 | 0,83 |
| FLJ22794 | 137454 | 0,00895 | 0,82 |
| IMAGE:743078 | 743078 | 0,00904 | 0,83 |
| IGKV1-5 | 155345 | 0,00922 | 0,83 |
| IMAGE:666794 Hypothetical LOC388170 | 666794 | 0,00944 | 0,74 |
| PPP2CB | 667375 | 0,00968 | 0,84 |
| HT007 | 135303 | 0,00976 | 0,84 |
| LOC56930 | 134976 | 0,00977 | 0,86 |
| ECM1 | 301122 | 0,0098 | 0,30 |
| LCN2 | 741497 | 0,01015 | 0,35 |
| PITPNA | 725585 | 0,01039 | 0,70 |
| IMAGE:743579 | 743579 | 0,01044 | 0,74 |
| RUNX1 | 263251 | 0,01059 | 0,79 |
| IMAGE:744074 Transcribed locus | 744074 | 0,01063 | 0,75 |
| SAFB | 42280 | 0,01086 | 0,69 |
| C9orf10OS | 742695 | 0,01194 | 0,84 |
| MGC23909 | 731598 | 0,012 | 0,77 |
| CLNS1A | 666426 | 0,01248 | 0,82 |
| IMAGE:743603 | 743603 | 0,01255 | 0,78 |
| IMAGE:665403 Transcribed locus | 665403 | 0,01258 | 0,83 |
| KLF4 | 188232 | 0,01265 | 0,63 |
| IMAGE:1859532 | 1859532 | 0,01275 | 0,83 |
| RFPL1 | 290265 | 0,01296 | 0,81 |
| HMGB2 | 884365 | 0,01308 | 0,84 |
| COX6A1 | 512910 | 0,01322 | 0,84 |
| ACADSB | 687953 | 0,01324 | 0,77 |

| Table 7 | FTA-a | Under | |
|---|---|---|---|
| name | cloid | p-value | fold |
| IMAGE:731357 Transcribed locus | 731357 | 0,01343 | 0,87 |
| SAP30L | 723950 | 0,0137 | 0,84 |
| PSG3 | 136747 | 0,0142 | 0,79 |
| PCSK6 | 31924 | 0,01471 | 0,71 |
| CASP7 | 279470 | 0,01475 | 0,86 |
| AFTIPHILIN | 741790 | 0,01511 | 0,79 |
| GATA4 | 781738 | 0,01514 | 0,80 |
| FXYD1 | 204686 | 0,01535 | 0,83 |
| ATP11B | 666334 | 0,01546 | 0,73 |
| S100A10 | 119939 | 0,01567 | 0,65 |
| COX5A | 1085884 | 0,01572 | 0,80 |
| CERKL | 489326 | 0,01615 | 0,74 |
| CD5 | 356841 | 0,01627 | 0,75 |
| NME7 | 743982 | 0,01628 | 0,76 |
| CCL19 | 1707527 | 0,01688 | 0,32 |
| JUP | 35628 | 0,01697 | 0,70 |
| C21orf84 | 730814 | 0,01699 | 0,77 |
| HOXB6 | 738358 | 0,01764 | 0,84 |
| FLJ25067 | 667252 | 0,01864 | 0,69 |
| IMAGE:740927 | 740927 | 0,01893 | 0,76 |
| MRC2 | 342581 | 0,01961 | 0,72 |
| GPC3 | 137131 | 0,01963 | 0,82 |
| CCL22 | 2150502 | 0,02001 | 0,85 |
| LILRA1 | 2032639 | 0,02014 | 0,82 |
| STAT2 | 2306096 | 0,02037 | 0,85 |
| HRK | 767779 | 0,02045 | 0,69 |
| PDXP | 743182 | 0,02046 | 0,84 |
| ALDH1A1 | 309697 | 0,02059 | 0,78 |
| DKFZP564D166 | 744374 | 0,02064 | 0,81 |
| PIK3CA | 2238108 | 0,02077 | 0,54 |
| IMAGE:136223 | 136223 | 0,02096 | 0,85 |
| IMAGE:666279 CDNA FLJ30779 fis, clone FEBRA2000815 | 666279 | 0,02123 | 0,72 |
| MFAP3L | 726821 | 0,02166 | 0,81 |
| PSMD11 | 383945 | 0,02171 | 0,77 |
| HTATIP2 | 726618 | 0,02245 | 0,83 |
| IMAGE:23817 | 23817 | 0,02275 | 0,80 |

| Table 7 | FTA-a | Under | |
|---|---|---|---|
| name | cloid | p-value | fold |
| CDNA FLJ12202 fis, clone MAMMA1000908 | | | |
| POU2F2 | 188393 | 0,02319 | 0,72 |
| GPI | 741474 | 0,02319 | 0,80 |
| FGF1 | 25895 | 0,0233 | 0,82 |
| EGFR | 135980 | 0,02368 | 0,80 |
| IMAGE:136014 | 136014 | 0,02388 | 0,80 |
| ZNF250 | 741066 | 0,02392 | 0,53 |
| IMAGE:382521 | 382521 | 0,02438 | 0,70 |
| LOC286076 | 731275 | 0,02463 | 0,83 |
| IMAGE:136598 MRNA; cDNA DKFZp686D22106 (from clone DKFZp686D22106 ) | 136598 | 0,02485 | 0,88 |
| CCR7 | 2345206 | 0,02512 | 0,78 |
| GNAS | 382791 | 0,02579 | 0,70 |
| ME3 | 724238 | 0,02586 | 0,74 |
| RGS19 | 485803 | 0,02627 | 0,85 |
| BCAP29 | 725970 | 0,02682 | 0,79 |
| HOXD11 | 682119 | 0,02693 | 0,80 |
| CD69 | 276727 | 0,02719 | 0,82 |
| FLJ37587 | 668476 | 0,02773 | 0,87 |
| TM4SF8 | 713647 | 0,02822 | 0,83 |
| IMAGE:742061 | 742061 | 0,02872 | 0,78 |
| TFF1 | 1075949 | 0,02928 | 0,74 |
| CASP10 | 241481 | 0,02963 | 0,75 |
| CXCL13 | 347362 | 0,03057 | 0,87 |
| HLA-DPB1 | 725548 | 0,03155 | 0,74 |
| IL13RA1 | 1492440 | 0,03217 | 0,88 |
| IMAGE:1117183 | 1117183 | 0,03277 | 0,79 |
| NME1 | 726600 | 0,03337 | 0,82 |
| CKLFSF3 | 489249 | 0,03492 | 0,88 |
| CCR7 | 2345206 | 0,03508 | 0,86 |
| CCND3 | 327182 | 0,03567 | 0,85 |

| Table 7 | FTA-a | Under | |
|---|---|---|---|
| name | cloid | p-value | fold |
| CAV1 | 133531 | 0,03597 | 0,80 |
| PTHLH | 2214396 | 0,03608 | 0,74 |
| LILRB5 | 71428 | 0,03622 | 0,75 |
| IMAGE:665342 Transcribed locus, strongly similar to NP_065847.1 semaphorin 6A1; semaphorin 6A-1 [Homo sapiens] | 665342 | 0,03709 | 0,90 |
| LOC253981 | 742743 | 0,0371 | 0,82 |
| CUGBP2 | 666235 | 0,03791 | 0,88 |
| TPD52 | 743259 | 0,03834 | 0,46 |
| JAK1 | 2030501 | 0,03845 | 0,80 |
| OAS1 | 666703 | 0,0394 | 0,74 |
| KCNQ2 | 179534 | 0,03951 | 0,83 |
| ZNF395 | 744983 | 0,04028 | 0,85 |
| CASP4 | 356960 | 0,04144 | 0,80 |
| TGFB3 | 1561035 | 0,04297 | 0,84 |
| IMAGE:726647 Transcribed locus | 726647 | 0,04331 | 0,83 |
| MAPK14 | 1741589 | 0,04332 | 0,89 |
| MAN1A1 | 137719 | 0,04366 | 0,68 |
| NTRK2 | 2048801 | 0,04379 | 0,78 |
| WIPI49 | 487148 | 0,04534 | 0,88 |
| SEMA3A | 33664 | 0,046 | 0,83 |
| PTK2B | 43541 | 0,0464 | 0,88 |
| ULK4 | 744895 | 0,04646 | 0,85 |
| IMAGE:687667 CDNA clone IMAGE:4811759, partial cds | 687667 | 0,04704 | 0,58 |
| AMD1 | 132752 | 0,04762 | 0,90 |
| RUNX3 | 122874 | 0,04914 | 0,80 |
| LILRA1 | 2032639 | 0,04959 | 0,85 |

| Table 8 | FTA-a | Over | |
|---|---|---|---|
| name | cloid | p-value | fold |
| ZNF76 | 745003 | 3,83E-05 | 1,61 |
| CABIN1 | 1844968 | 0,00023 | 1,64 |
| PRUNE | 364324 | 2,36E-04 | 1,36 |
| CST3 | 357201 | 5,87E-04 | 1,26 |
| BAT3 | 24392 | 0,00067 | 1,36 |
| PLXNB2 | 1420676 | 6,94E-04 | 1,80 |
| FBXW8 | 687532 | 7,31E-04 | 1,21 |
| PPARD | 724562 | 0,00077 | 1,27 |
| LOC147650 | 729510 | 8,26E-04 | 1,30 |
| DAP | 725371 | 9,98E-04 | 1,55 |
| EIF4G1 | 24083 | 1,02E-03 | 1,64 |
| KIAA1196 | 738938 | 1,09E-03 | 1,62 |
| PH-4 | 730938 | 1,39E-03 | 1,43 |
| GATA3 | 148796 | 1,42E-03 | 1,43 |
| CORO1B | 137205 | 1,47E-03 | 1,37 |
| OTUB1 | 174683 | 1,49E-03 | 1,37 |
| IMAGE:21808 Transcribed locus | 21808 | 1,94E-03 | 1,34 |
| S100A2 | 3659591 | 2,01E-03 | 1,32 |
| KRTHA4 | 325155 | 2,19E-03 | 1,60 |
| ARHGAP21 | 666792 | 2,57E-03 | 1,19 |
| FCGBP | 154172 | 2,63E-03 | 1,35 |
| RAD51L1 | 295412 | 2,70E-03 | 1,57 |
| IMAGE:176543 | 176543 | 2,87E-03 | 1,31 |
| FLJ14981 | 24532 | 3,45E-03 | 1,41 |
| COL18A1 | 359202 | 0,00366 | 1,64 |
| CCNE1 | 68950 | 4,27E-03 | 1,43 |
| SGCG | 2046361 | 4,36E-03 | 1,22 |
| PVRL2 | 725364 | 4,46E-03 | 1,27 |
| IMAGE:741178 CDNA FLJ38849 fis, clone MESAN2008936 | 741178 | 4,49E-03 | 1,30 |
| RBM25 | 668360 | 4,64E-03 | 1,35 |
| BCL2L11 | 300194 | 4,68E-03 | 1,56 |
| IMAGE:23872 AF034176 Human mRNA (Tripodis and Ragoussis) Homo sapiens cDNA clone ntcon5 contig | 23872 | 4,96E-03 | 1,17 |
| FLJ13491 | 364352 | 0,00525 | 1,35 |
| NDUFA7 | 364469 | 0,00528 | 1,24 |
| LOC146909 | 726353 | 0,00531 | 1,32 |
| LGALS3BP | 742100 | 5,46E-03 | 1,41 |
| FLJ12895 | 23271 | 5,96E-03 | 1,33 |
| C9orf119 | 667112 | 0,00607 | 1,26 |

| Table 8 | FTA-a | Over | |
|---|---|---|---|
| name | cloid | p-value | fold |
| GPR108 | 25949 | 6,22E-03 | 1,42 |
| RPS6KA2 | 22711 | 6,63E-03 | 1,67 |
| IMAGE:744055 Transcribed locus | 744055 | 7,01E-03 | 1,57 |
| RTN3 | 430263 | 7,06E-03 | 1,22 |
| HDAC9 | 738506 | 7,91E-03 | 1,22 |
| IMAGE:738945 | 738945 | 7,97E-03 | 1,23 |
| GGN | 743245 | 8,24E-03 | 1,21 |
| TM4SF9 | 812967 | 8,54E-03 | 1,40 |
| PLA2G6 | 744087 | 8,74E-03 | 1,40 |
| UBE1L | 250883 | 9,66E-03 | 1,37 |
| SLC25A23 | 741954 | 9,88E-03 | 1,49 |
| DCI | 667892 | 0,00991 | 1,28 |
| PP1201 | 364974 | 0,00997 | 1,20 |
| IMAGE:744505 | 744505 | 0,01063 | 1,44 |
| TLN1 | 187482 | 1,11E-02 | 1,38 |
| LOC51760 | 52226 | 1,15E-02 | 1,42 |
| DJ159A19.3 | 23945 | 1,15E-02 | 1,47 |
| TPCN1 | 179288 | 1,28E-02 | 1,29 |
| AGPAT3 | 738507 | 1,29E-02 | 1,18 |
| GCLC | 1526058 | 1,38E-02 | 1,20 |
| DNALI1 | 782688 | 1,43E-02 | 1,44 |
| IMAGE:174861 Transcribed locus | 174861 | 1,53E-02 | 1,31 |
| HDAC6 | 669295 | 1,57E-02 | 1,18 |
| C7orf27 | 713859 | 1,63E-02 | 1,23 |
| ACTR1B | 136868 | 1,73E-02 | 1,24 |
| GNB1 | 24300 | 1,74E-02 | 1,16 |
| IMAGE:731726 Transcribed locus | 731726 | 1,80E-02 | 1,42 |
| RANBP10 | 682251 | 1,83E-02 | 1,21 |
| PTDSS2 | 21716 | 1,83E-02 | 1,19 |
| PIAS1 | 38789 | 1,85E-02 | 1,20 |
| ASGR1 | 25883 | 1,87E-02 | 1,91 |
| PDAP1 | 172693 | 1,88E-02 | 1,20 |
| TETRAN | 725340 | 1,96E-02 | 1,36 |
| AMPD2 | 669141 | 2,00E-02 | 1,23 |
| BAD | 712295 | 2,09E-02 | 1,21 |
| FOXM1 | 23641 | 2,22E-02 | 1,19 |
| PDCD1LG2 | 738944 | 2,27E-02 | 1,21 |
| IMAGE:1716286 | 1716286 | 2,27E-02 | 1,44 |
| PNKP | 723867 | 2,27E-02 | 1,21 |
| SSH3 | 726272 | 2,46E-02 | 1,17 |
| HSPB1 | 23827 | 2,54E-02 | 1,37 |
| CCNE1 | 357807 | 2,67E-02 | 1,24 |
| IMAGE:136801 | 136801 | 2,67E-02 | 1,19 |
| AXIN2 | 135887 | 0,02753 | 1,16 |
| IMAGE:225455 | 2254555 | 2,77E-02 | 1,20 |

| Table 8 | FTA-a | Over | |
|---|---|---|---|
| name | cloid | p-value | fold |
| 5 | | | |
| KCNC3 | 53333 | 2,81E-02 | 1,42 |
| IMAGE:472111 Transcribed locus | 472111 | 2,83E-02 | 1,49 |
| CDH1 | 214008 | 2,94E-02 | 1,24 |
| KIAA1838 | 744106 | 2,96E-02 | 1,15 |
| ANAPC2 | 136462 | 2,97E-02 | 1,20 |
| MGC5178 | 725533 | 0,03005 | 1,20 |
| PBXIP1 | 366042 | 3,08E-02 | 1,25 |
| CaMKIINalpha | 173820 | 3,09E-02 | 1,33 |
| MBNL1 | 136114 | 3,38E-02 | 1,30 |
| FLJ10707 | 364575 | 3,41E-02 | 1,15 |
| GALT | 68972 | 3,47E-02 | 1,19 |
| LPXN | 687679 | 3,47E-02 | 1,16 |
| SENP6 | 366436 | 3,51E-02 | 1,18 |
| EPHA4 | 2237263 | 3,56E-02 | 1,37 |
| SLA | 32339 | 3,61E-02 | 1,41 |
| IMAGE:26486 CDNA FLJ43345 fis, clone NT2RI3008228 | 26486 | 3,67E-02 | 1,23 |
| NF2 | 1698236 | 3,78E-02 | 1,44 |
| CRLF3 | 279150 | 3,84E-02 | 1,17 |
| IL11RA | 1101773 | 3,84E-02 | 1,33 |
| FLJ30707 | 666986 | 3,89E-02 | 1,16 |
| IMAGE:669470 CDNA clone IMAGE:5206119, partial cds | 669470 | 4,03E-02 | 1,18 |
| PPP1CA | 257259 | 4,05E-02 | 1,16 |
| RIPK3 | 667313 | 4,06E-02 | 1,31 |
| OAZ2 | 128694 | 4,33E-02 | 1,15 |
| GALNT9 | 178750 | 4,35E-02 | 1,31 |
| LILRB4 | 2341829 | 4,45E-02 | 1,15 |
| NIFIE14 | 472160 | 4,53E-02 | 1,16 |
| IMAGE:727123 Transcribed locus, strongly similar to NP_004491.1 heterogeneous nuclear ribonucleoprotein C isoform b; nuclear ribonucleoprotein particle C2 protein; nuclear ribonucleoprotein particle C1 protein [Homo sapiens] | 727123 | 4,64E-02 | 1,14 |

| Table 9 | | FTA-atypical | Under |
|---|---|---|---|
| name | cloid | p-value | fold |
| NBL1 | 503874 | 5,13E-11 | 0,22 |
| DF | 666128 | 2,64E-09 | 0,34 |
| APOD | 838611 | 2,03E-07 | 0,19 |
| KLF1 | 208991 | 4,17E-07 | 0,37 |
| MGC99813 | 739097 | 6,06E-07 | 0,3 |
| IMAGE:1089025 | 1089025 | 6,77E-07 | 0,25 |
| HLA-DPB1 | 725548 | 1,15E-06 | 0,5 |
| CRABP1 | 739193 | 1,71E-06 | 0,27 |
| MMP2 | 155839 | 4,59E-06 | 0,52 |
| MATN2 | 28584 | 5,28E-06 | 0,45 |
| LILRB2 | 202897 | 7,11E-06 | 0,52 |
| SIGLEC7 | 743331 | 7,86E-06 | 0,64 |
| IMAGE:743619 | 743619 | 9,02E-06 | 0,61 |
| NEK6 | 725345 | 9,81E-06 | 0,6 |
| CXCL14 | 345034 | 1,16E-05 | 0,2 |
| BAK1 | 235938 | 1,44E-05 | 0,65 |
| CPXM2 | 729924 | 1,73E-05 | 0,56 |
| ETHE1 | 681957 | 1,95E-05 | 0,73 |
| LRP1 | 30219 | 1,98E-05 | 0,55 |
| GPNMB | 773330 | 2,86E-05 | 0,25 |
| APOL1 | 665632 | 3,32E-05 | 0,4 |
| NTRK2 | 2048801 | 3,48E-05 | 0,49 |
| MEOX1 | 760065 | 3,88E-05 | 0,6 |
| S100A10 | 119939 | 3,89E-05 | 0,59 |
| BAI3 | 50491 | 3,90E-05 | 0,58 |
| GC | 195340 | 4,63E-05 | 0,61 |
| C10orf116 | 740941 | 5,60E-05 | 0,5 |
| PLCG2 | 201467 | 6,66E-05 | 0,65 |
| VAV2 | 1581686 | 6,79E-05 | 0,69 |
| IMAGE:743603 | 743603 | 7,55E-05 | 0,66 |
| SLC17A2 | 207920 | 9,06E-05 | 0,43 |
| IMAGE:731616 MRNA; cDNA DKFZp686A102 (from clone DKFZp686A102) | 731616 | 1,16E-04 | 0,46 |
| LILRB5 | 71428 | 1,50E-04 | 0,64 |
| SYP | 30471 | 1,78E-04 | 0,65 |
| PTGES | 504646 | 1,83E-04 | 0,77 |
| CCDC6 | 487848 | 1,97E-04 | 0,67 |
| MRC2 | 342581 | 2,34E-04 | 0,51 |
| PDCD1LG1 | 1942634 | 2,77E-04 | 0,72 |
| PIK3CA | 2238108 | 2,98E-04 | 0,37 |
| DUSP24 | 740158 | 3,21E-04 | 0,4 |
| SAFB | 42280 | 3,29E-04 | 0,53 |

| Table 9 | | FTA-atypical | Under |
|---|---|---|---|
| name | cloid | p-value | fold |
| GJA5 | 196338 | 3,41E-04 | 0,45 |
| IMAGE:742739 CDNA FLJ12931 fis, clone NT2RP2004861 | 742739 | 3,99E-04 | 0,57 |
| IMAGE:743615 Full-length cDNA clone CS0DK001YE19 of HeLa cells Cot 25-normalized of Homo sapiens (human) | 743615 | 4,35E-04 | 0,72 |
| BIK | 1074708 | 4,37E-04 | 0,75 |
| GTPBP1 | 366484 | 4,94E-04 | 0,69 |
| GCK | 30981 | 5,91E-04 | 0,73 |
| FLJ27523 | 743246 | 6,04E-04 | 0,43 |
| LASP1 | 592598 | 6,09E-04 | 0,47 |
| IMAGE:485104 Transcribed locus | 485104 | 6,19E-04 | 0,64 |
| STX7 | 174396 | 6,31E-04 | 0,54 |
| PPIL4 | 364777 | 6,96E-04 | 0,4 |
| C19orf33 | 379540 | 7,02E-04 | 0,58 |
| IL2RA | 3054031 | 7,14E-04 | 0,69 |
| IFNAR2 | 123950 | 7,17E-04 | 0,59 |
| CD3E | 1536968 | 7,38E-04 | 0,61 |
| MATN2 | 366100 | 7,66E-04 | 0,64 |
| CERKL | 489326 | 7,80E-04 | 0,64 |
| IMAGE:24587 | 24587 | 7,81E-04 | 0,6 |
| DEFB1 | 665086 | 7,91E-04 | 0,36 |
| IMAGE:342256 | 342256 | 8,29E-04 | 0,28 |
| SEPT-05 | 448163 | 9,20E-04 | 0,28 |
| JUP | 35628 | 1,01E-03 | 0,56 |
| USP4 | 544818 | 1,06E-03 | 0,64 |
| KCNQ2 | 179534 | 1,10E-03 | 0,62 |
| WDR20 | 665156 | 1,15E-03 | 0,78 |
| CAV1 | 309645 | 1,18E-03 | 0,5 |
| SALL4 | 726454 | 1,18E-03 | 0,48 |
| IMAGE:738896 | 738896 | 1,20E-03 | 0,32 |
| PCSK6 | 31924 | 1,27E-03 | 0,63 |
| HCLS1 | 665452 | 1,27E-03 | 0,66 |
| DLX5 | 564878 | 1,27E-03 | 0,55 |
| TRAF5 | 1286238 | 1,48E-03 | 0,76 |

| Table 9 | | FTA-atypical | Under |
|---|---|---|---|
| name | cloid | p-value | fold |
| COL27A1 | 379517 | 1,59E-03 | 0,66 |
| IMAGE:177857 Transcribed locus | 177857 | 1,59E-03 | 0,63 |
| IMAGE:744410 Transcribed locus | 744410 | 1,61E-03 | 0,71 |
| OBSCN | 730926 | 1,67E-03 | 0,73 |
| E2F5 | 809828 | 1,71E-03 | 0,56 |
| SLAMF8 | 288807 | 1,84E-03 | 0,74 |
| IMAGE:740105 | 740105 | 2,04E-03 | 0,67 |
| NCAM1 | 366842 | 2,06E-03 | 0,72 |
| FRZB | 146049 | 2,23E-03 | 0,62 |
| AQP11 | 713831 | 2,30E-03 | 0,6 |
| COL1A1 | 140347 | 2,30E-03 | 0,6 |
| IMAGE:666279 CDNA FLJ30779 fis, clone FEBRA2000815 | 666279 | 2,46E-03 | 0,62 |
| CASP5 | 341763 | 2,47E-03 | 0,53 |
| CFH | 665784 | 2,51E-03 | 0,53 |
| FLJ14981 | 24532 | 2,52E-03 | 0,65 |
| SIAT8B | 33133 | 2,52E-03 | 0,68 |
| HSPC159 | 365045 | 2,67E-03 | 0,74 |
| BAK1 | 1288183 | 2,71E-03 | 0,58 |
| CAV1 | 133531 | 2,80E-03 | 0,59 |
| IMAGE:744926 Transcribed locus | 744926 | 2,95E-03 | 0,61 |
| FCHO1 | 742763 | 3,01E-03 | 0,78 |
| IMAGE:472111 Transcribed locus | 472111 | 3,05E-03 | 0,59 |
| BAP1 | 1012990 | 3,05E-03 | 0,66 |
| TPD52 | 743259 | 3,11E-03 | 0,29 |
| CCL19 | 1707527 | 3,14E-03 | 0,21 |
| EMX2 | 365121 | 3,16E-03 | 0,69 |
| BIC | 743270 | 3,26E-03 | 0,65 |
| IMAGE:666315 Homo sapiens, clone IMAGE:5312086, mRNA | 666315 | 3,28E-03 | 0,66 |
| IMAGE:2484270 | 2484270 | 3,50E-03 | 0,67 |
| KLHL9 | 471696 | 3,57E-03 | 0,66 |

| Table 9 | | FTA-atypical | Under |
|---|---|---|---|
| name | cloid | p-value | fold |
| APIN | 364706 | 3,70E-03 | 0,61 |
| IMAGE:364741 | 364741 | 4,03E-03 | 0,62 |
| VAV1 | 80384 | 4,05E-03 | 0,53 |
| MGP | 590264 | 4,05E-03 | 0,66 |
| SFRP4 | 285693 | 4,12E-03 | 0,54 |
| NOLA2 | 173296 | 4,30E-03 | 0,8 |
| TGFB3 | 1561035 | 4,48E-03 | 0,66 |
| HBB | 469549 | 4,48E-03 | 0,44 |
| LILRB3 | 1422194 | 4,59E-03 | 0,62 |
| IMAGE:731689 Transcribed locus | 731689 | 4,76E-03 | 0,73 |
| IMAGE:667527 | 667527 | 4,81E-03 | 0,62 |
| TLX3 | 1860115 | 4,86E-03 | 0,63 |
| ID1 | 1087348 | 4,93E-03 | 0,6 |
| FXYD1 | 204686 | 4,96E-03 | 0,65 |
| TFF1 | 1075949 | 5,04E-03 | 0,58 |
| SLPI | 378813 | 5,42E-03 | 0,56 |
| SYT1 | 28511 | 5,47E-03 | 0,73 |
| RNF32 | 731422 | 5,77E-03 | 0,54 |
| TOSO | 813174 | 5,84E-03 | 0,55 |
| IFI30 | 740931 | 5,91E-03 | 0,6 |
| ASGR1 | 25883 | 6,02E-03 | 0,64 |
| F10 | 310519 | 6,20E-03 | 0,73 |
| LEFTY2 | 340657 | 6,42E-03 | 0,69 |
| IMAGE:727289 Transcribed locus | 727289 | 6,73E-03 | 0,65 |
| IMAGE:743722 | 743722 | 6,88E-03 | 0,7 |
| NCB5OR | 743367 | 6,92E-03 | 0,55 |
| SERPINB5 | 1662274 | 7,01E-03 | 0,72 |
| AXL | 364083 | 7,01E-03 | 0,59 |
| CREB3L2 | 136399 | 7,23E-03 | 0,56 |
| GRIK2 | 731363 | 7,95E-03 | 0,74 |
| IMAGE:743579 | 743579 | 7,97E-03 | 0,55 |
| FABP5 | 1088781 | 8,38E-03 | 0,45 |
| FOLR1 | 131839 | 8,85E-03 | 0,65 |
| GJA1 | 839101 | 8,97E-03 | 0,63 |
| CCL13 | 2449149 | 8,99E-03 | 0,77 |
| BMP5 | 1846326 | 9,07E-03 | 0,72 |
| RIN1 | 741406 | 9,10E-03 | 0,67 |
| SIAT7B | 823590 | 9,35E-03 | 0,66 |
| SCMH1 | 724014 | 1,00E-02 | 0,6 |
| GALNT9 | 178750 | 1,01E-02 | 0,69 |
| IFITM3 | 713623 | 1,03E-02 | 0,73 |
| HRK | 767779 | 1,05E-02 | 0,69 |

| Table 9 | | FTA-atypical | Under |
|---|---|---|---|
| name | cloid | p-value | fold |
| CDR2 | 366067 | 1,08E-02 | 0,83 |
| IMAGE:743078 | 743078 | 1,08E-02 | 0,82 |
| IMAGE:383966 | 383966 | 1,09E-02 | 0,67 |
| RAD51L1 | 295412 | 1,09E-02 | 0,68 |
| MAPK3 | 323438 | 1,10E-02 | 0,68 |
| ZNF319 | 364729 | 1,14E-02 | 0,45 |
| RUNX3 | 122874 | 1,17E-02 | 0,73 |
| PARD3 | 724642 | 1,22E-02 | 0,73 |
| SOD3 | 795309 | 1,23E-02 | 0,52 |
| RPL27A | 178255 | 1,28E-02 | 0,58 |
| BTG1 | 382760 | 1,29E-02 | 0,8 |
| SEMA7A | 135941 | 1,30E-02 | 0,55 |
| KLK2 | 1102600 | 1,31E-02 | 0,65 |
| BMP4 | 69166 | 1,32E-02 | 0,72 |
| UNC13D | 231903 | 1,41E-02 | 0,55 |
| IGSF10 | 682276 | 1,44E-02 | 0,8 |
| IMAGE:1716286 | 1716286 | 1,48E-02 | 0,63 |
| HAS3 | 667533 | 1,51E-02 | 0,69 |
| SDHA | 40304 | 1,52E-02 | 0,75 |
| FLJ22386 | 32917 | 1,53E-02 | 0,79 |
| FLJ23577 | 668452 | 1,60E-02 | 0,61 |
| MGAT3 | 731060 | 1,62E-02 | 0,79 |
| TRIP12 | 665820 | 1,62E-02 | 0,62 |
| MATN1 | 1624260 | 1,67E-02 | 0,78 |
| CD38 | 1352408 | 1,82E-02 | 0,74 |
| FBXO46 | 471664 | 1,84E-02 | 0,68 |
| ZNF251 | 486401 | 1,86E-02 | 0,56 |
| LOC388284 | 731722 | 1,88E-02 | 0,75 |
| PDLIM1 | 135689 | 1,91E-02 | 0,7 |
| ISG20 | 740604 | 1,92E-02 | 0,69 |
| BRAF | 2139164 | 2,05E-02 | 0,82 |
| AP2S1 | 739109 | 2,16E-02 | 0,87 |
| ITGA5 | 135671 | 2,31E-02 | 0,74 |
| OAS1 | 666703 | 2,32E-02 | 0,63 |
| F5 | 433155 | 2,33E-02 | 0,7 |
| THAP3 | 687800 | 2,37E-02 | 0,56 |
| TREM2 | 502244 | 2,41E-02 | 0,78 |
| TNFRSF1B | 73703 | 2,43E-02 | 0,82 |
| IMAGE:484577 Transcribed locus, weakly similar to NP_000991.1 ribosomal protein L39; 60S ribosomal protein L39 [Homo | 484577 | 2,43E-02 | 0,69 |

| Table 9 | | FTA-atypical | Under |
|---|---|---|---|
| name | cloid | p-value | fold |
| sapiens] | | | |
| ATR | 1251197 | 2,46E-02 | 0,83 |
| MUTED | 731202 | 2,47E-02 | 0,65 |
| TM4SF9 | 381032 | 2,49E-02 | 0,76 |
| BST2 | 811024 | 2,50E-02 | 0,75 |
| MAP2 | 347503 | 2,50E-02 | 0,71 |
| MGC5395 | 238840 | 2,53E-02 | 0,79 |
| BID | 128065 | 2,60E-02 | 0,82 |
| GNAS | 382791 | 2,65E-02 | 0,66 |
| APOE | 1870594 | 2,66E-02 | 0,69 |
| SPP1 | 258114 | 2,66E-02 | 0,52 |
| C6orf216 | 731742 | 2,70E-02 | 0,8 |
| SYN3 | 727056 | 2,72E-02 | 0,66 |
| GATA4 | 781738 | 2,92E-02 | 0,81 |
| LRRIQ2 | 487152 | 3,01E-02 | 0,75 |
| MCC | 731305 | 3,16E-02 | 0,59 |
| IMAGE:731726 Transcribed locus | 731726 | 3,23E-02 | 0,73 |
| BCL2 | 342181 | 3,33E-02 | 0,82 |
| IMAGE:745512 | 745512 | 3,38E-02 | 0,85 |
| CASP4 | 356960 | 3,45E-02 | 0,75 |
| GLT8D2 | 365271 | 3,51E-02 | 0,64 |
| MT1F | 78353 | 3,57E-02 | 0,64 |
| IMAGE:730924 Transcribed locus | 730924 | 3,74E-02 | 0,74 |
| IMAGE:742853 | 742853 | 4,03E-02 | 0,67 |
| KLRB1 | 2091591 | 4,11E-02 | 0,73 |
| WDR5 | 731023 | 4,14E-02 | 0,8 |
| C6orf176 | 173200 | 4,19E-02 | 0,9 |
| SLC39A2 | 504596 | 4,25E-02 | 0,75 |
| ARHGAP21 | 666792 | 4,27E-02 | 0,86 |
| IMAGE:383718 | 383718 | 4,31E-02 | 0,77 |
| MAK | 382002 | 4,33E-02 | 0,83 |
| GPI | 741474 | 4,37E-02 | 0,78 |
| C20orf19 | 366032 | 4,50E-02 | 0,73 |
| LOC285500 | 135766 | 4,56E-02 | 0,68 |
| PSMD11 | 383945 | 4,57E-02 | 0,77 |
| MUC1 | 840687 | 4,79E-02 | 0,71 |
| PITPNC1 | 364436 | 4,79E-02 | 0,74 |
| MKKS | 729957 | 4,82E-02 | 0,79 |
| KLHL9 | 501527 | 4,82E-02 | 0,83 |
| OSR1 | 364686 | 4,96E-02 | 0,75 |

| Table 10 | FTA-atypical | Over | |
|---|---|---|---|
| name | cloid | p-value | fold |
| SNCB | 50202 | 6,55E-04 | 2,00 |
| DJ462O23.2 | 738970 | 1,64E-03 | 1,68 |
| OAZ2 | 128694 | 2,70E-03 | 1,42 |
| RGL2 | 741891 | 5,30E-03 | 1,49 |
| IMAGE:666671 Transcribed locus | 666671 | 7,53E-03 | 1,33 |
| PSAT1 | 366388 | 7,99E-03 | 1,52 |
| ETF1 | 146976 | 8,36E-03 | 1,68 |
| IMAGE:687667 CDNA clone IMAGE:4811759, partial cds | 687667 | 9,11E-03 | 3,863 |
| LRPAP1 | 587186 | 9,83E-03 | 2,52 |
| KPNA2 | 667727 | 1,44E-02 | 1,394 |
| IMAGE:669136 Transcribed locus, weakly similar to NP_060312.1 hypothetical protein FLJ20489 [Homo sapiens] | 669136 | 1,55E-02 | 1,55 |
| RBM5 | 744001 | 1,68E-02 | 1,18 |
| PGR1 | 666361 | 1,71E-02 | 1,35 |
| IMAGE:668300 Transcribed locus, strongly similar to NP_683708.1 Toll-interleukin 1 receptor domain-containing adaptor protein isoform b; adapter protein wyatt; MyD88 adapter-like protein; TIR domain-containing | 668300 | 1,73E-02 | 1,41 |

| Table 10 | FTA-atypical | Over | |
|---|---|---|---|
| name | cloid | p-value | fold |
| adapter protein; Toll-interleukin 1 receptor (TIR) domain-containing adaptor protein [Homo sapiens] | | | |
| TM4SF8 | 713647 | 1,76E-02 | 1,43 |
| ICOSLG | 2074228 | 1,79E-02 | 1,47 |
| CA11 | 282587 | 1,82E-02 | 1,52 |
| TPCN1 | 179288 | 1,93E-02 | 1,28 |
| MEF2C | 687270 | 2,09E-02 | 1,45 |
| FLJ10748 | 726830 | 2,10E-02 | 1,744 |
| PDGFD | 666860 | 2,15E-02 | 1,46 |
| IMAGE:687896 PREDICTED: Homo sapiens olfactory receptor, family 7, subfamily E, member 31 pseudogene (OR7E31P), mRNA | 687896 | 2,32E-02 | 1,28 |
| SATB1 | 364510 | 2,96E-02 | 1,28 |
| BCAR1 | 324666 | 3,02E-02 | 1,22 |
| BCL2L11 | 300194 | 3,03E-02 | 1,344 |
| CTNNA1 | 268972 | 3,05E-02 | 1,17 |
| FLJ20254 | 135096 | 3,08E-02 | 1,24 |
| HMGN1 | 565754 | 3,10E-02 | 1,28 |
| TNFSF12 | 136361 | 3,12E-02 | 1,32 |
| CLDN7 | 300268 | 3,31E-02 | 1,59 |
| MGC17299 | 713422 | 3,32E-02 | 1,30 |
| HIVEP1 | 758037 | 3,34E-02 | 1,196 |
| MESDC2 | 713653 | 3,52E-02 | 1,419 |
| PBXIP1 | 366042 | 3,66E-02 | 1,67 |
| SPINT1 | 723914 | 3,90E-02 | 1,55 |
| PDAP1 | 172693 | 3,91E-02 | 1,2 |
| ABCC3 | 208097 | 3,94E-02 | 2,35 |
| APLP2 | 549054 | 4,01E-02 | 1,36 |
| SPATA12 | 730300 | 4,04E-02 | 1,39 |
| FLJ20160 | 175864 | 4,15E-02 | 1,43 |
| FBXL16 | 178908 | 4,37E-02 | 1,45 |
| MGC19604 | 687468 | 4,42E-02 | 1,27 |
| KCNK5 | 134978 | 4,54E-02 | 1,62 |
| IMAGE:7299 | 729956 | 4,63E-02 | 1,244 |

| Table 10 | FTA-atypical | Over | |
|---|---|---|---|
| name | cloid | p-value | fold |
| 56 | | | |
| VEGFC | 503189 | 4,91E-02 | 1,20 |
| IMAGE:681875 | 681875 | 4,91E-02 | 1,29 |

| Table 11 | FTA-b | Under | |
|---|---|---|---|
| name | cloid | p-value | fold |
| IFI30 | 740931 | 1,4326E-13 | 0,29 |
| IMAGE:731751 Transcribed locus | 731751 | 2,0423E-10 | 0,47 |
| MUC1 | 840687 | 5,8417E-10 | 0,5 |
| DF | 666128 | 8,9417E-09 | 0,37 |
| C19orf33 | 379540 | 1,0372E-07 | 0,56 |
| MLPH | 667259 | 1,2264E-07 | 0,4 |
| PITPNA | 725585 | 3,9776E-07 | 0,46 |
| NBL1 | 503874 | 8,9123E-07 | 0,39 |
| TIAM1 | 23612 | 1,184E-06 | 0,37 |
| IL2RA | 3054031 | 1,2903E-06 | 0,66 |
| ABCC3 | 208097 | 1,3495E-06 | 0,41 |
| CITED1 | 265558 | 1,4726E-06 | 0,27 |
| IMAGE:136976 Transcribed locus, moderately similar to NP_777603.1 hypothetical protein FLJ25976 [Homo sapiens] | 136976 | 1,7574E-06 | 0,61 |
| APOD | 838611 | 2,6068E-06 | 0,24 |
| RASGRP1 | 725707 | 7,0993E-06 | 0,46 |
| CLDN1 | 594279 | 9,4387E-06 | 0,19 |
| DPP4 | 343987 | 1,0189E-05 | 0,45 |
| NOG | 487474 | 1,3124E-05 | 0,56 |
| FLJ12604 | 731128 | 1,323E-05 | 0,58 |
| AQP4 | 279172 | 1,9121E-05 | 0,59 |
| GPNMB | 773330 | 1,9836E-05 | 0,24 |
| IMAGE:1089025 | 1089025 | 2,3035E-05 | 0,34 |
| GTPBP5 | 725502 | 2,5821E-05 | 0,52 |
| KLRB1 | 2091591 | 2,6472E-05 | 0,63 |
| CD4 | 141216 | 4,1509E-05 | 0,66 |
| MRC2 | 235882 | 4,2346E-05 | 0,52 |
| HCLS1 | 665452 | 4,6839E-05 | 0,61 |
| ATR | 1251197 | 4,6948E-05 | 0,77 |
| SPINL | 236399 | 4,9438E-05 | 0,44 |
| BAI3 | 50491 | 5,2305E-05 | 0,62 |
| LOC90110 | 376699 | 5,4077E-05 | 0,77 |
| IMAGE:727496 Transcribed locus | 727496 | 5,6847E-05 | 0,55 |
| SLC6A8 | 725877 | 6,9226E-05 | 0,32 |

| Table 11 | FTA-b | Under | |
|---|---|---|---|
| name | cloid | p-value | fold |
| IRF4 | 682207 | 9,8647E-05 | 0,67 |
| IMAGE:687667 CDNA clone IMAGE:4811759, partial cds | 687667 | 0,00011938 | 0,4 |
| CPXM2 | 729924 | 0,00012549 | 0,6 |
| KCNQ2 | 179534 | 0,00013834 | 0,72 |
| C4A | 724366 | 0,00014538 | 0,6 |
| SYP | 30471 | 0,00014852 | 0,64 |
| FLJ27523 | 743246 | 0,00015423 | 0,39 |
| FABP5 | 1088781 | 0,00018612 | 0,35 |
| CXCL14 | 345034 | 0,00021831 | 0,24 |
| GC | 195340 | 0,00023132 | 0,65 |
| S100A4 | 868577 | 0,00024012 | 0,44 |
| PIK3CA | 2238108 | 0,00025026 | 0,36 |
| WAS | 2148946 | 0,00025378 | 0,66 |
| SPP1 | 258114 | 0,000267 | 0,35 |
| CCL28 | 136919 | 0,00027959 | 0,71 |
| APOE | 1870594 | 0,00028207 | 0,49 |
| ZNF258 | 1241974 | 0,00028771 | 0,74 |
| ALCAM | 172828 | 0,00031982 | 0,66 |
| FLJ37034 | 731348 | 0,00035286 | 0,75 |
| DUSP24 | 740158 | 0,00035953 | 0,49 |
| LASP1 | 592598 | 0,00037148 | 0,43 |
| NCK1 | 1326169 | 0,00038479 | 0,72 |
| ETV4 | 809959 | 0,00038529 | 0,77 |
| AFTIPHILIN | 741790 | 0,00040278 | 0,74 |
| LOC440712 | 725268 | 0,00040484 | 0,65 |
| S100A10 | 119939 | 0,00041115 | 0,64 |
| EGFR | 135980 | 0,000416 | 0,59 |
| IMAGE:136014 | 136014 | 0,00041903 | 0,59 |
| ISG20 | 740604 | 0,00042284 | 0,6 |
| SMARCA5 | 730037 | 0,00047821 | 0,76 |
| SEPT-05 | 448163 | 0,00048532 | 0,24 |
| SCEL | 668239 | 0,00049646 | 0,75 |
| S100A6 | 512420 | 0,00049777 | 0,63 |
| CLECSF12 | 258865 | 0,00055249 | 0,42 |
| CD47 | 365526 | 0,00055731 | 0,74 |
| EED | 667365 | 0,00057991 | 0,76 |
| MAPKAPK2 | 742577 | 0,00059626 | 0,78 |
| PMS2 | 116906 | 0,00062492 | 0,63 |
| IL10RA | 258747 | 0,00078912 | 0,69 |
| IMAGE:738896 | 738896 | 0,00086466 | 0,32 |
| IMAGE:342256 | 342256 | 0,00088142 | 0,28 |

| Table 11 | FTA-b | Under | |
|---|---|---|---|
| name | cloid | p-value | fold |
| VDAC1 | 486221 | 0,00088583 | 0,71 |
| SLAMF8 | 288807 | 0,00091298 | 0,67 |
| TPD52 | 743259 | 0,00102858 | 0,21 |
| IMAGE:136 223 | 136223 | 0,00102862 | 0,77 |
| BCAP29 | 725970 | 0,00104691 | 0,66 |
| BNIP3 | 342700 | 0,00108019 | 0,72 |
| CCND3 | 327182 | 0,00109024 | 0,79 |
| POPDC3 | 264166 | 0,0012516 | 0,75 |
| CLGN | 1049033 | 0,00129805 | 0,72 |
| SAMD10 | 365801 | 0,00133812 | 0,8 |
| RNASE4 | 156720 | 0,00140105 | 0,74 |
| KLRK1 | 725473 | 0,00158467 | 0,78 |
| BIK | 1074708 | 0,00163982 | 0,77 |
| RFPL1 | 290265 | 0,00164775 | 0,83 |
| ANKRD28 | 687379 | 0,00169361 | 0,73 |
| LILRB5 | 71428 | 0,0016981 | 0,75 |
| MATN1 | 1624260 | 0,00177668 | 0,77 |
| ZNF251 | 486401 | 0,00179489 | 0,42 |
| FRMD4B | 669564 | 0,00181652 | 0,64 |
| CAV1 | 133531 | 0,00185645 | 0,68 |
| DCAMKL1 | 232388 | 0,00191401 | 0,72 |
| SALL4 | 726454 | 0,00194797 | 0,53 |
| S100A12 | 1705397 | 0,00201356 | 0,75 |
| CCDC6 | 487848 | 0,00201872 | 0,73 |
| URB | 809719 | 0,00217689 | 0,64 |
| IMAGE:251 427 | 251427 | 0,00220137 | 0,74 |
| GRM3 | 287843 | 0,00221023 | 0,75 |
| APOE | 1870594 | 0,00230094 | 0,63 |
| IMAGE:744 926 Transcribed locus | 744926 | 0,00237853 | 0,64 |
| HOXD11 | 682119 | 0,00238989 | 0,69 |
| NOTCH2 | 1641901 | 0,00255668 | 0,55 |
| IMAGE:742 901 | 742901 | 0,00256267 | 0,78 |
| CCL19 | 1707527 | 0,00265849 | 0,18 |
| IMAGE:742 853 | 742853 | 0,00269303 | 0,6 |
| SIAT8B | 33133 | 0,00287355 | 0,82 |
| GSTM2 | 664233 | 0,00328285 | 0,76 |
| SLC39A2 | 504596 | 0,00332451 | 0,78 |
| FLJ35794 | 364204 | 0,00343664 | 0,68 |
| SFRP4 | 285693 | 0,00366218 | 0,56 |
| DOK5 | 25664 | 0,0037568 | 0,67 |
| STX7 | 174396 | 0,0037569 | 0,66 |
| IMAGE:743 722 | 743722 | 0,00380385 | 0,78 |
| GABRE | 209137 | 0,00395568 | 0,73 |
| F10 | 310519 | 0,00403123 | 0,74 |

| Table 11 | FTA-b | Under | |
|---|---|---|---|
| name | cloid | p-value | fold |
| SAFB | 42280 | 0,00407958 | 0,68 |
| PTPN4 | 249311 | 0,00432638 | 0,8 |
| IL13 | 1505308 | 0,00447394 | 0,74 |
| MAD2L2 | 727178 | 0,00456201 | 0,74 |
| PKNOX1 | 667067 | 0,0045851 | 0,71 |
| IMAGE:731 726 Transcribed locus | 731726 | 0,00458772 | 0,75 |
| IMAGE:469 91 Similar to breakpoint cluster region isoform 1 | 46991 | 0,00461083 | 0,73 |
| FLJ22794 | 137454 | 0,00464619 | 0,75 |
| PREB | 740347 | 0,00469747 | 0,52 |
| ATP11B | 666334 | 0,00476466 | 0,72 |
| IMAGE:666 671 Transcribed locus | 666671 | 0,00504158 | 0,81 |
| NOTCH1 | 359461 | 0,00506296 | 0,71 |
| HSPB7 | 487302 | 0,0052282 | 0,66 |
| CD38 | 1352408 | 0,00526111 | 0,76 |
| PTGES | 504646 | 0,00534239 | 0,81 |
| DKFZp434 M202 | 743118 | 0,00543931 | 0,72 |
| STAU | 365919 | 0,00562066 | 0,82 |
| THAP3 | 687800 | 0,00576148 | 0,52 |
| PTPNS1 | 26061 | 0,00606792 | 0,74 |
| ECM1 | 301122 | 0,00616842 | 0,28 |
| RUNX3 | 122874 | 0,00623461 | 0,79 |
| MYC | 129438 | 0,0064463 | 0,63 |
| CUGBP2 | 666235 | 0,00725838 | 0,82 |
| FBXO46 | 471664 | 0,00745485 | 0,73 |
| LCN2 | 741497 | 0,0079592 | 0,35 |
| IMAGE:111 7183 | 1117183 | 0,00799012 | 0,79 |
| FMO5 | 364526 | 0,00847507 | 0,79 |
| S100A1 | 175772 | 0,00862472 | 0,65 |
| C10orf6 | 179214 | 0,0086753 | 0,8 |
| HN1 | 471568 | 0,00879178 | 0,74 |
| ZFOC1 | 744606 | 0,00881888 | 0,75 |
| LAMB3 | 1103402 | 0,00904345 | 0,74 |
| PDCD1LG1 | 1942634 | 0,00917818 | 0,79 |
| FAM36A | 727263 | 0,00962448 | 0,77 |
| IMAGE:484 577 Transcribed locus, weakly | 484577 | 0,01003627 | 0,67 |

| Table 11 | FTA-b | Under | |
|---|---|---|---|
| name | cloid | p-value | fold |
| similar to NP_000991 .1 ribosomal protein L39; 60S ribosomal protein L39 [Homo sapiens] | | | |
| TSC | 745490 | 0,01016313 | 0,58 |
| PCSK6 | 31924 | 0,01040107 | 0,7 |
| IGF1R | 682555 | 0,01044883 | 0,78 |
| ATIC | 665693 | 0,01064698 | 0,83 |
| CASP3 | 823680 | 0,01086953 | 0,72 |
| SLAMF1 | 1626951 | 0,01116187 | 0,81 |
| STK17A | 562904 | 0,01118617 | 0,76 |
| IMAGE:682 311 LOC44072 2 | 682311 | 0,01131518 | 0,81 |
| AMD1 | 132752 | 0,01141962 | 0,81 |
| TFRC | 359797 | 0,01168045 | 0,78 |
| RGS19 | 485803 | 0,01210737 | 0,79 |
| HLA-DPB1 | 725548 | 0,01225207 | 0,71 |
| RBBP4 | 705147 | 0,01232497 | 0,78 |
| INHBB | 730012 | 0,01243708 | 0,67 |
| IMAGE:504 539 | 504539 | 0,01255589 | 0,77 |
| C4A | 491004 | 0,01259752 | 0,71 |
| PARP9 | 667440 | 0,0127547 | 0,82 |
| CD33 | 1917430 | 0,01352763 | 0,73 |
| IMP-2 | 743774 | 0,01360949 | 0,51 |
| CENTD1 | 666110 | 0,01509604 | 0,78 |
| ALDH1A1 | 309697 | 0,01556687 | 0,68 |
| IMAGE:724 416 Transcribed locus | 724416 | 0,01642786 | 0,7 |
| HT007 | 135303 | 0,01657774 | 0,83 |
| MGC29784 | 731019 | 0,01673005 | 0,87 |
| MAP2 | 1287638 | 0,01674651 | 0,7 |
| LOC25398 1 | 742743 | 0,0168504 | 0,75 |
| LOC38990 6 | 740718 | 0,01771548 | 0,85 |
| PDK1 | 1645668 | 0,01800445 | 0,84 |
| SLPI | 378813 | 0,01811761 | 0,62 |
| DEK | 1016390 | 0,0200655 | 0,81 |
| VIL2 | 124701 | 0,02020796 | 0,75 |
| TIMP1 | 162246 | 0,02061013 | 0,57 |
| RFX2 | 731738 | 0,0206857 | 0,69 |
| C4orf16 | 681890 | 0,02095375 | 0,82 |
| CAV1 | 309645 | 0,0222001 | 0,64 |
| PDCD4 | 665376 | 0,02257053 | 0,72 |
| MGC5395 | 238840 | 0,02292419 | 0,83 |
| CERKL | 489326 | 0,0238201 | 0,77 |
| APOL1 | 665632 | 0,02452738 | 0,63 |
| PLCG2 | 201467 | 0,02564117 | 0,8 |
| KLF4 | 188232 | 0,02771743 | 0,71 |
| ALDH1A3 | 486189 | 0,02811344 | 0,69 |
| GFPT2 | 485085 | 0,02897134 | 0,82 |
| ACTB | 203166 | 0,02910806 | 0,76 |
| DKFZP564 D166 | 744374 | 0,02968329 | 0,85 |
| TOSO | 813174 | 0,0306376 | 0,79 |
| CD8B1 | 1743279 | 0,03083639 | 0,84 |
| FCHO1 | 742763 | 0,03087022 | 0,83 |
| ICAM3 | 156183 | 0,03162562 | 0,85 |
| EPRS | 669549 | 0,03174483 | 0,85 |
| ST5 | 376356 | 0,03189569 | 0,86 |
| DEFB1 | 665086 | 0,03223041 | 0,57 |
| CCL13 | 2449149 | 0,0324626 | 0,79 |
| MAN1A1 | 137719 | 0,03287328 | 0,66 |
| IL2RB | 139073 | 0,0337379 | 0,84 |
| CD47 | 357442 | 0,03389369 | 0,77 |
| USP18 | 745083 | 0,03455337 | 0,8 |
| IMAGE:724 544 Similar to 40S ribosomal protein S25 | 724544 | 0,03490328 | 0,73 |
| IMAGE:132 933 | 132933 | 0,03499764 | 0,8 |
| C20orf18 | 665433 | 0,03600838 | 0,73 |
| IMAGE:249 7617 | 2497617 | 0,03637846 | 0,81 |
| LIPT1 | 22085 | 0,03746726 | 0,82 |
| NSUN4 | 364570 | 0,03889642 | 0,76 |
| MX1 | 713879 | 0,04036441 | 0,8 |
| WDR5 | 731023 | 0,0411845 | 0,82 |
| GSTM2 | 713922 | 0,04311433 | 0,84 |
| LOC12022 4 | 252291 | 0,04339193 | 0,73 |
| CAV1 | 1911930 | 0,04340567 | 0,79 |
| MPO | 436554 | 0,04449178 | 0,86 |
| IMAGE:743 415 Transcribed locus | 743415 | 0,0451026 | 0,86 |
| KLRD1 | 145696 | 0,04603368 | 0,85 |
| ATF4 | 178348 | 0,04624908 | 0,84 |
| G3BP | 667239 | 0,04690026 | 0,84 |
| ELK4 | 236155 | 0,04765789 | 0,84 |

| Table 11 | FTA-b | Under | |
|---|---|---|---|
| name | cloid | p-value | fold |
| AKR1C1 | 196992 | 0,04840514 | 0,45 |
| IKBKB | 2484742 | 0,04904713 | 0,87 |
| GATA4 | 781738 | 0,04957556 | 0,83 |
| SEMA4D | 210587 | 0,04995642 | 0,79 |

| Table 12 | FTA-b | Over | |
|---|---|---|---|
| **name** | **cloid** | **p-value** | **fold** |
| PLXNC1 | 724609 | 1,13E-03 | 1,92 |
| IMAGE:365913 Transcribed locus | 365913 | 1,44E-03 | 1,54 |
| PDE8A | 666154 | 1,47E-03 | 1,30 |
| SPARC | 267358 | 2,52E-03 | 1,56 |
| C6orf85 | 665379 | 2,79E-03 | 1,25 |
| DCI | 667892 | 3,32E-03 | 1,88 |
| TMED4 | 251250 | 5,09E-03 | 1,46 |
| MESDC2 | 713653 | 6,21E-03 | 1,44 |
| FLJ45459 | 365589 | 6,88E-03 | 1,35 |
| ID1 | 1087348 | 7,35E-03 | 1,71 |
| PBXIP1 | 366042 | 7,53E-03 | 1,41 |
| FAM38B | 743146 | 0,00757 | 1,275 |
| KNS2 | 174654 | 0,00785 | 1,564 |
| LMLN | 744657 | 8,03E-03 | 1,36 |
| CDH1 | 214008 | 9,02E-03 | 1,48 |
| USP13 | 666007 | 9,33E-03 | 1,57 |
| MGC3329 | 137005 | 0,00969 | 1,297 |
| PITPNC1 | 364436 | 1,06E-02 | 1,56 |
| TEX13A | 738558 | 1,12E-02 | 1,33 |
| ETF1 | 146976 | 0,01148 | 1,526 |
| FLJ12681 | 682175 | 1,15E-02 | 1,33 |
| MGC5178 | 725533 | 0,01202 | 1,32 |
| EMX2 | 365121 | 1,22E-02 | 1,46 |
| C9orf119 | 667112 | 1,24E-02 | 1,39 |
| CD34 | 770858 | 1,25E-02 | 1,43 |
| S100A2 | 3659591 | 1,31E-02 | 1,44 |
| KDR | 469345 | 0,01314 | 1,341 |
| FCGBP | 154172 | 1,37E-02 | 1,52 |
| LOC340073 | 731404 | 1,45E-02 | 1,56 |
| MXD4 | 730036 | 1,57E-02 | 1,22 |
| ITGA5 | 135671 | 1,59E-02 | 1,31 |
| MGC99813 | 739097 | 1,63E-02 | 2,20 |
| CRABP1 | 739193 | 1,63E-02 | 2,53 |
| IMAGE:743205 | 743205 | 1,65E-02 | 1,41 |
| GOLPH2 | 811582 | 1,71E-02 | 1,33 |
| FLJ13491 | 364352 | 1,85E-02 | 1,33 |
| PVRL2 | 725364 | 1,86E-02 | 1,50 |
| IMAGE:668684 Transcribed locus, weakly similar to XP_209041.2 PREDICTED: similar to KIAA1503 protein [Homo sapiens] | 668684 | 2,04E-02 | 1,31 |
| NT5C2 | 725076 | 0,02133 | 1,281 |
| IMAGE:666946 | 666946 | 2,15E-02 | 1,28 |
| IMMP2L | 136260 | 2,22E-02 | 1,17 |
| RAP1GA1 | 971276 | 2,24E-02 | 1,54 |
| BRAF | 2139164 | 2,26E-02 | 1,29 |
| GLT8D2 | 365271 | 2,28E-02 | 2,01 |
| RANBP10 | 307025 | 2,29E-02 | 1,20 |
| GLG1 | 366254 | 2,33E-02 | 1,35 |
| CD63 | 125552 | 2,35E-02 | 1,30 |
| SGSH | 669263 | 2,39E-02 | 1,36 |
| IMAGE:744439 Transcribed locus | 744439 | 2,63E-02 | 1,26 |
| LRRC28 | 26519 | 2,69E-02 | 1,38 |
| GRB10 | 564994 | 2,78E-02 | 1,48 |
| FLT4 | 668815 | 2,93E-02 | 1,26 |
| DCTN2 | 725335 | 3,12E-02 | 1,17 |
| KRT7 | 592276 | 3,21E-02 | 1,72 |
| IMAGE:176543 | 176543 | 3,34E-02 | 1,19 |
| DIO1 | 296702 | 3,37E-02 | 1,57 |
| RGS20 | 668152 | 3,44E-02 | 1,27 |
| RAD51L1 | 295412 | 0,0345 | 1,565 |
| GATA2 | 149809 | 3,60E-02 | 1,26 |
| RGS6 | 24176 | 3,62E-02 | 1,44 |
| CES2 | 153667 | 3,67E-02 | 1,32 |
| FLJ44005 | 726675 | 3,73E-02 | 1,42 |
| CDH13 | 486510 | 3,88E-02 | 1,43 |
| GCLC | 1526058 | 4,06E-02 | 1,17 |
| C2orf7 | 366243 | 4,50E-02 | 1,27 |
| GATA3 | 148796 | 4,51E-02 | 1,31 |
| TSG101 | 194350 | 4,52E-02 | 1,16 |
| CKLFSF4 | 143759 | 4,59E-02 | 1,24 |
| KIAA0789 | 33621 | 4,59E-02 | 1,30 |
| ZNF580 | 668089 | 4,90E-02 | 1,28 |
| PIGV | 669319 | 5,00E-02 | 1,16 |

| Table 13 | FTC | Under | |
|---|---|---|---|
| name | cloid | p-value | fold |
| TIMP3 | 501476 | 3,27118E-28 | 0,255 |
| MDK | 309009 | 4,17066E-17 | 0,216 |
| CRABP1 | 739193 | 1,13484E-16 | 0,071 |
| ARMCX3 | 251452 | 3,43628E-15 | 0,346 |
| ETHE1 | 681957 | 3,82022E-15 | 0,593 |
| MGC99813 | 739097 | 2,29648E-14 | 0,14 |
| IKBKE | 364448 | 6,52667E-14 | 0,703 |
| HBB | 469549 | 1,08191E-10 | 0,196 |
| LILRB2 | 202897 | 1,82324E-10 | 0,477 |
| GC | 195340 | 7,63448E-10 | 0,467 |
| ARHGAP10 | 731380 | 1,46351E-09 | 0,6 |
| CCR7 | 2345206 | 4,3297E-09 | 0,569 |
| IMAGE:23765 | 23765 | 5,53388E-09 | 0,55 |
| RODH | 471641 | 2,47866E-08 | 0,243 |
| TREM2 | 502244 | 2,75199E-08 | 0,682 |
| MESDC2 | 713653 | 6,65348E-08 | 0,465 |
| ALDH1A3 | 486189 | 1,02206E-07 | 0,631 |
| ISG20 | 740604 | 1,21406E-07 | 0,623 |
| IRF4 | 682207 | 1,50961E-07 | 0,558 |
| C10orf116 | 740941 | 1,82217E-07 | 0,355 |
| HBA2 | 469647 | 2,06716E-07 | 0,185 |
| DPF2 | 743519 | 2,65982E-07 | 0,768 |
| NCB5OR | 743367 | 4,04597E-07 | 0,441 |
| TRAF2 | 966894 | 9,00279E-07 | 0,652 |
| SYN3 | 727056 | 1,0981E-06 | 0,303 |
| BCAP29 | 2336358 | 2,65105E-06 | 0,523 |
| TOE1 | 52897 | 3,47029E-06 | 0,682 |
| NCK1 | 1326169 | 4,11032E-06 | 0,655 |
| GPNMB | 773330 | 4,11149E-06 | 0,191 |
| IL2RB | 139073 | 6,44261E-06 | 0,742 |
| HLA-DPB1 | 725548 | 6,72755E-06 | 0,448 |
| ANXA11 | 137238 | 9,73001E-06 | 0,794 |
| IMAGE:742853 | 742853 | 1,43601E-05 | 0,464 |
| APOD | 838611 | 1,96792E-05 | 0,267 |
| IMAGE:22908 Transcribed locus, weakly similar to NP_775735.1 l(3)mbt-like 4 [Homo sapiens] | 22908 | 2,94694E-05 | 0,524 |
| SLC17A2 | 207920 | 3,13767E-05 | 0,278 |

| Table 13 | FTC | Under | |
|---|---|---|---|
| name | cloid | p-value | fold |
| WNT4 | 375746 | 5,12808E-05 | 0,783 |
| IMAGE:738896 | 738896 | 5,38726E-05 | 0,17 |
| PLA2G2A | 152802 | 5,92307E-05 | 0,61 |
| DPP4 | 343987 | 6,26833E-05 | 0,495 |
| FLJ27523 | 743246 | 8,22724E-05 | 0,347 |
| IMAGE:1089025 | 1089025 | 8,66321E-05 | 0,405 |
| IMAGE:342256 | 342256 | 9,00293E-05 | 0,171 |
| CPXM2 | 729924 | 9,0502E-05 | 0,379 |
| IMAGE:727496 Transcribed locus | 727496 | 0,000107885 | 0,569 |
| TP53I11 | 667514 | 0,000107922 | 0,46 |
| BAI3 | 50491 | 0,00010801 | 0,563 |
| SALL4 | 726454 | 0,000203015 | 0,336 |
| ZNF251 | 486401 | 0,000204182 | 0,342 |
| PIK3CA | 2238108 | 0,000241673 | 0,316 |
| FLJ12903 | 730123 | 0,000273124 | 0,686 |
| HIS1 | 342551 | 0,000280435 | 0,52 |
| BCL2L12 | 1186334 | 0,000312735 | 0,752 |
| FLJ45459 | 365589 | 0,000371257 | 0,554 |
| ABCB1 | 1837488 | 0,000377025 | 0,682 |
| PDCD1LG1 | 1942634 | 0,000469177 | 0,785 |
| CD69 | 276727 | 0,000475157 | 0,514 |
| FLJ30707 | 666986 | 0,000530185 | 0,889 |
| YWHAH | 324066 | 0,000614917 | 0,701 |
| SCMH1 | 724014 | 0,000705203 | 0,557 |
| AXL | 364083 | 0,000726257 | 0,397 |
| CCL13 | 2449149 | 0,000733565 | 0,665 |
| FLJ14351 | 744943 | 0,00080337 | 0,391 |
| IL10RA | 258747 | 0,000976141 | 0,564 |
| IGHM | 276658 | 0,00121878 | 0,633 |
| CITED1 | 265558 | 0,0012369 | 0,487 |
| C6orf118 | 731745 | 0,001239503 | 0,705 |
| CDH16 | 726763 | 0,001947806 | 0,389 |
| SYT1 | 28511 | 0,002006575 | 0,618 |
| MRC2 | 235882 | 0,00205901 | 0,452 |
| CCL19 | 1707527 | 0,002223263 | 0,182 |
| HDAC1 | 1896337 | 0,002482715 | 0,694 |
| SEPT-05 | 448163 | 0,002647911 | 0,341 |
| ECM1 | 301122 | 0,002741544 | 0,224 |
| GNAI2 | 724306 | 0,002774431 | 0,893 |
| THAP3 | 687800 | 0,002955378 | 0,496 |
| DEPDC6 | 669318 | 0,002997235 | 0,368 |
| PTHLH | 2214396 | 0,003118854 | 0,674 |
| IMAGE:175268 | 175268 | 0,003169415 | 0,477 |
| PREB | 740347 | 0,003182387 | 0,355 |

| Table 13 | FTC | Under | |
|---|---|---|---|
| name | cloid | p-value | fold |
| GLT8D2 | 365271 | 0,003312971 | 0,507 |
| LOH12CR 2 | 366074 | 0,003465031 | 0,706 |
| CLDN1 | 594279 | 0,003571509 | 0,327 |
| PDCD1LG 1 | 1942634 | 0,003641855 | 0,603 |
| APOL3 | 366289 | 0,003733369 | 0,709 |
| DEFB1 | 665086 | 0,003754037 | 0,44 |
| NOTCH2 | 1641901 | 0,00433718 | 0,516 |
| C4A | 724366 | 0,005027025 | 0,419 |
| COX8A | 509606 | 0,006006104 | 0,469 |
| PTPNS1 | 26061 | 0,006559782 | 0,53 |
| TIMP1 | 162246 | 0,006602902 | 0,333 |
| CKLFSF3 | 489249 | 0,006843773 | 0,753 |
| ETV1 | 24541 | 0,007109204 | 0,767 |
| SPON2 | 723923 | 0,00735775 | 0,628 |
| IMAGE:73 1751 Transcribe d locus | 731751 | 0,007589669 | 0,548 |
| IMAGE:74 4410 Transcribe d locus | 744410 | 0,00769332 | 0,779 |
| APOE | 1870594 | 0,007740935 | 0,323 |
| TPD52 | 743259 | 0,007753957 | 0,352 |
| HMG20A | 731277 | 0,007809821 | 0,564 |
| GALNT7 | 381854 | 0,008046311 | 0,836 |
| GPR108 | 25949 | 0,008356628 | 0,56 |
| CERKL | 489326 | 0,008740448 | 0,482 |
| C4A | 491004 | 0,009053823 | 0,445 |
| KLRD1 | 2728204 | 0,009193925 | 0,702 |
| OAS1 | 666703 | 0,009198225 | 0,586 |
| FLJ21159 | 251147 | 0,00940131 | 0,668 |
| APOE | 1870594 | 0,009962428 | 0,454 |
| AOC3 | 484535 | 0,010034148 | 0,634 |
| SPP1 | 258114 | 0,010053336 | 0,457 |
| LRRC28 | 26519 | 0,011941366 | 0,387 |
| WIPI49 | 487148 | 0,012228545 | 0,735 |
| LAPTM4A | 726684 | 0,012475634 | 0,269 |
| GATA2 | 149809 | 0,012854907 | 0,758 |
| IMAGE:73 1616 MRNA; cDNA DKFZp68 6A102 (from clone DKFZp68 6A102) | 731616 | 0,013356612 | 0,415 |
| LOC1202 | 252291 | 0,014999368 | 0,594 |

| Table 13 | FTC | Under | |
|---|---|---|---|
| name | cloid | p-value | fold |
| 24 | | | |
| LILRB5 | 71428 | 0,016005286 | 0,745 |
| PRUNE | 364324 | 0,016566051 | 0,608 |
| ICAM3 | 156183 | 0,016831639 | 0,649 |
| MESP1 | 25865 | 0,017300239 | 0,735 |
| S100A12 | 1705397 | 0,017625347 | 0,606 |
| TMED4 | 251250 | 0,017788466 | 0,307 |
| MAP7 | 79729 | 0,01797944 | 0,572 |
| IMAGE:36 5562 Transcribe d locus, moderatel y similar to XP_52138 9.1 PREDICT ED: similar to UTY [Pan troglodyte s] | 365562 | 0,018131317 | 0,77 |
| BST2 | 811024 | 0,018268156 | 0,73 |
| OBSCN | 730926 | 0,019249071 | 0,661 |
| COX5A | 1085884 | 0,020164977 | 0,667 |
| RNF123 | 366159 | 0,021646555 | 0,853 |
| IGSF10 | 682276 | 0,022377266 | 0,753 |
| NDUFA7 | 364469 | 0,024164639 | 0,48 |
| DDR2 | 668442 | 0,024237373 | 0,566 |
| TACC1 | 741905 | 0,02441973 | 0,496 |
| CD5 | 356841 | 0,024474896 | 0,636 |
| C21orf84 | 730814 | 0,025612809 | 0,747 |
| SYT7 | 177827 | 0,02577182 | 0,587 |
| SAFB | 42280 | 0,025773596 | 0,765 |
| MTAP | 724151 | 0,026004214 | 0,763 |
| BIC | 743270 | 0,026405121 | 0,738 |
| IMMP2L | 136260 | 0,026660518 | 0,536 |
| GFPT2 | 485085 | 0,026990136 | 0,741 |
| IMAGE:74 2837 Transcribe d locus | 742837 | 0,027538211 | 0,83 |
| DF | 666128 | 0,028156903 | 0,452 |
| IMAGE:66 6279 CDNA FLJ30779 fis, clone FEBRA20 00815 | 666279 | 0,029379728 | 0,624 |
| RYK | 727092 | 0,029455425 | 0,728 |
| PPIL4 | 364777 | 0,02990287 | 0,314 |
| TJP1 | 179334 | 0,030457336 | 0,563 |

| Table 13 | FTC | Under | |
|---|---|---|---|
| name | cloid | p-value | fold |
| CAPN3 | 757248 | 0,030652263 | 0,681 |
| IMAGE:2484270 | 2484270 | 0,031332245 | 0,707 |
| GRM3 | 287843 | 0,031389431 | 0,727 |
| KDR | 469345 | 0,031468457 | 0,521 |
| ADCY3 | 667061 | 0,031634804 | 0,526 |
| RGS19 | 485803 | 0,032147875 | 0,497 |
| IMAGE:727491 | 727491 | 0,034722498 | 0,364 |
| CXCL12 | 213113 | 0,035745974 | 0,694 |
| FLJ12998 | 364846 | 0,036806278 | 0,576 |
| IMAGE:743619 | 743619 | 0,036830771 | 0,726 |
| ARL6IP2 | 743987 | 0,037296129 | 0,493 |
| DCTN1 | 180640 | 0,037381682 | 0,554 |
| APIN | 364706 | 0,038549022 | 0,62 |
| FLJ32731 | 365177 | 0,039009106 | 0,77 |
| LASP1 | 592598 | 0,039649505 | 0,594 |
| RAP1GA1 | 971276 | 0,040572673 | 0,687 |
| VAV2 | 1581686 | 0,042133135 | 0,748 |
| FLJ34433 | 713671 | 0,042784525 | 0,821 |
| MYC | 129438 | 0,043192437 | 0,578 |
| IMAGE:1117183 | 1117183 | 0,043871839 | 0,671 |
| CXCL14 | 345034 | 0,044460416 | 0,292 |
| ZNF250 | 741066 | 0,044484132 | 0,419 |
| TRAF5 | 1286238 | 0,044516219 | 0,635 |
| SYNPO | 178792 | 0,044836883 | 0,64 |
| IFI30 | 740931 | 0,045498386 | 0,616 |
| LMLN | 744657 | 0,046068298 | 0,648 |
| TUSC1 | 740381 | 0,046224198 | 0,898 |
| EPHA4 | 2237263 | 0,046590574 | 0,784 |
| PDE8A | 666154 | 0,047026631 | 0,555 |
| ARID1B | 665538 | 0,047648823 | 0,631 |
| ITGA5 | 135671 | 0,048291346 | 0,699 |
| CREBBP | 172996 | 0,048559519 | 0,817 |
| NF2 | 1698236 | 0,048577039 | 0,629 |
| DNMT1 | 768241 | 0,049510935 | 0,69 |
| POU2F2 | 188393 | 0,049542968 | 0,619 |

| Table 14 | FTC | Over | |
|---|---|---|---|
| name | cloid | p-value | fold |
| DNALI1 | 782688 | 5,13E-31 | 1,83 |
| IMAGE:136014 | 136014 | 4,35E-09 | 1,73 |
| EGFR | 135980 | 4,38E-09 | 1,73 |
| DCTN2 | 725335 | 4,32E-08 | 1,39 |
| HSPB1 | 724150 | 9,31E-07 | 1,59 |
| GCAT | 307094 | 3,74E-06 | 1,29 |
| IMAGE:723752 | 723752 | 1,88E-05 | 1,34 |
| GRB7 | 510318 | 2,89E-05 | 1,35 |
| IQCF3 | 726578 | 2,42E-04 | 2,43 |
| CCND1 | 324079 | 2,91E-04 | 2,47 |
| BRAF | 2139164 | 3,09E-04 | 1,28 |
| CKLFSF4 | 143759 | 4,45E-04 | 1,64 |
| SENP6 | 739237 | 7,42E-04 | 1,77 |
| CAPNS1 | 152655 | 7,56E-04 | 1,31 |
| NY-REN-41 | 136646 | 4,34E-03 | 1,43 |
| ALDH1A1 | 309697 | 6,65E-03 | 1,61 |
| IMAGE:384087 | 384087 | 0,01 | 1,39 |
| DGKD | 365408 | 1,14E-02 | 2,21 |
| LOC90110 | 376699 | 1,16E-02 | 1,42 |
| DNCLI2 | 134671 | 1,19E-02 | 2,26 |
| BAZ2B | 609631 | 1,21E-02 | 1,74 |
| MGC5395 | 238840 | 1,46E-02 | 2,41 |
| BIK | 1074708 | 1,53E-02 | 1,12 |
| GLG1 | 366254 | 1,63E-02 | 1,62 |
| IMAGE:742919 Transcribed locus | 742919 | 1,72E-02 | 1,81 |
| PPP1CA | 257259 | 2,09E-02 | 1,41 |
| CCL5 | 840753 | 2,37E-02 | 1,90 |
| IMAGE:668924 | 668924 | 2,58E-02 | 1,43 |
| IMAGE:136801 | 136801 | 2,72E-02 | 2,18 |
| GGN | 743245 | 2,96E-02 | 1,11 |
| IMAGE:731428 Transcribed locus, weakly similar to XP_521389.1 PREDICTED: similar to UTY [Pan troglodytes] | 731428 | 3,12E-02 | 1,54 |
| DKFZp547K054 | 731410 | 3,31E-02 | 1,93 |
| AGPAT3 | 738507 | 3,71E-02 | 1,55 |
| RTN3 | 430263 | 3,77E-02 | 1,80 |
| DKFZP564D166 | 744374 | 3,79E-02 | 1,36 |
| IL13RA1 | 1492440 | 3,81E-02 | 1,32 |
| AXIN2 | 135887 | 3,84E-02 | 1,84 |
| IMAGE:726782 Transcribed locus | 726782 | 4,33E-02 | 2,26 |
| COL27A1 | 379517 | 4,47E-02 | 1,29 |
| C9orf72 | 726849 | 4,95E-02 | 2,25 |

| Table 15 | MNG | Under | |
|---|---|---|---|
| name | cloid | p-value | fold |
| ABCA8 | 284828 | 1,79E-06 | 0,62 |
| CACNB2 | 173841 | 2,60E-06 | 0,59 |
| IMAGE:667527 | 667527 | 9,53E-06 | 0,63 |
| LILRB2 | 202897 | 9,61E-06 | 0,54 |
| HBB | 469549 | 1,12E-05 | 0,38 |
| SLC17A2 | 207920 | 1,30E-05 | 0,45 |
| HBA2 | 469647 | 1,46E-05 | 0,4 |
| IMAGE:363955 Transcribed locus | 363955 | 1,70E-05 | 0,6 |
| MAPK3 | 323438 | 1,81E-05 | 0,65 |
| IGHM | 276658 | 1,96E-05 | 0,77 |
| RUNX1 | 263251 | 3,29E-05 | 0,72 |
| HMG20A | 731277 | 4,64E-05 | 0,68 |
| JUP | 35628 | 5,75E-05 | 0,59 |
| GPI | 741474 | 6,80E-05 | 0,72 |
| DMD | 743394 | 6,85E-05 | 0,67 |
| ABCC5 | 212366 | 7,48E-05 | 0,78 |
| CLECSF12 | 258865 | 7,61E-05 | 0,41 |
| SCMH1 | 724014 | 9,16E-05 | 0,59 |
| IMAGE:742837 Transcribed locus | 742837 | 1,15E-04 | 0,71 |
| IMAGE:1117183 | 1117183 | 1,20E-04 | 0,75 |
| CD69 | 276727 | 1,54E-04 | 0,73 |
| GOLPH2 | 811582 | 1,55E-04 | 0,76 |
| CD8B1 | 1743279 | 1,56E-04 | 0,77 |
| IMAGE:666279 CDNA FLJ30779 fis, clone FEBRA2000815 | 666279 | 1,77E-04 | 0,6 |
| OAS1 | 666703 | 1,85E-04 | 0,65 |
| PITPNA | 725585 | 1,98E-04 | 0,59 |
| TRIM8 | 714498 | 2,67E-04 | 0,78 |
| ALDH3A1 | 525221 | 2,90E-04 | 0,66 |
| AP2S1 | 739109 | 2,94E-04 | 0,79 |
| ARHGAP10 | 731380 | 3,14E-04 | 0,65 |
| ANP32E | 382738 | 3,79E-04 | 0,68 |
| BAP1 | 1012990 | 4,47E-04 | 0,7 |
| KLF4 | 188232 | 5,18E-04 | 0,58 |
| ABR | 1012903 | 5,18E-04 | 0,77 |
| FLJ44005 | 726675 | 5,89E-04 | 0,79 |
| CASP4 | 356960 | 6,18E-04 | 0,76 |
| MRC2 | 235882 | 6,38E-04 | 0,56 |
| PRDX2 | 208439 | 6,58E-04 | 0,76 |
| ATF4 | 178348 | 7,17E-04 | 0,76 |

| Table 15 | MNG | Under | |
|---|---|---|---|
| name | cloid | p-value | fold |
| SYN3 | 727056 | 7,28E-04 | 0,58 |
| SQLE | 124781 | 7,44E-04 | 0,72 |
| CLDN7 | 300268 | 7,48E-04 | 0,76 |
| S100A4 | 868577 | 7,51E-04 | 0,46 |
| CCND3 | 327182 | 7,62E-04 | 0,81 |
| PTPN4 | 249311 | 8,22E-04 | 0,79 |
| MLANA | 266361 | 8,46E-04 | 0,78 |
| CASP10 | 241481 | 8,58E-04 | 0,68 |
| ADAMTS9 | 376153 | 9,08E-04 | 0,71 |
| TSC | 745490 | 1,10E-03 | 0,53 |
| PCSK6 | 31924 | 1,15E-03 | 0,67 |
| BAK1 | 235938 | 1,17E-03 | 0,7 |
| IMAGE:687667 CDNA clone IMAGE:4811759, partial cds | 687667 | 1,19E-03 | 0,48 |
| TU3A | 44881 | 1,21E-03 | 0,72 |
| HLA-DPB1 | 725548 | 1,22E-03 | 0,67 |
| MAPRE2 | 383868 | 1,35E-03 | 0,64 |
| HIS1 | 342551 | 1,38E-03 | 0,75 |
| IMAGE:382521 | 382521 | 1,41E-03 | 0,66 |
| KPNA2 | 667727 | 1,55E-03 | 0,76 |
| IMAGE:365562 Transcribed locus, moderately similar to XP_521389.1 PREDICTED: similar to UTY [Pan troglodytes] | 365562 | 1,75E-03 | 0,8 |
| CDH3 | 359051 | 1,76E-03 | 0,61 |
| SYCP2 | 136863 | 1,80E-03 | 0,71 |
| SLC9A3R1 | 773286 | 1,81E-03 | 0,74 |
| ELK4 | 236155 | 1,81E-03 | 0,74 |
| IMAGE:744899 Homo sapiens, clone IMAGE:4837072, mRNA | 744899 | 1,90E-03 | 0,77 |
| PBXIP1 | 366042 | 1,92E-03 | 0,81 |
| PSMD11 | 383945 | 2,05E-03 | 0,72 |
| SPON2 | 723923 | 2,08E-03 | 0,76 |
| LEFTY2 | 340657 | 2,08E-03 | 0,74 |
| DDR2 | 668442 | 2,10E-03 | 0,78 |
| GNAS | 382791 | 2,15E-03 | 0,64 |

| Table 15 | MNG | Under | |
|---|---|---|---|
| name | cloid | p-value | fold |
| IMAGE:666794 Hypothetical LOC388170 | 666794 | 2,17E-03 | 0,74 |
| LAD1 | 121551 | 2,17E-03 | 0,8 |
| SFN | 346610 | 2,19E-03 | 0,8 |
| IMAGE:232366 CDNA FLJ37672 fis, clone BRHIP2012059 | 232366 | 2,20E-03 | 0,73 |
| PPP3CC | 110481 | 2,27E-03 | 0,79 |
| BID | 128065 | 2,27E-03 | 0,79 |
| IMAGE:379937 | 379937 | 2,31E-03 | 0,72 |
| FOXN4 | 731076 | 2,31E-03 | 0,78 |
| IRF4 | 682207 | 2,41E-03 | 0,69 |
| SNCB | 50202 | 2,45E-03 | 0,7 |
| ANKRD32 | 725198 | 2,56E-03 | 0,79 |
| KLF1 | 208991 | 2,66E-03 | 0,69 |
| C1QBP | 173371 | 2,67E-03 | 0,75 |
| DC12 | 724895 | 2,73E-03 | 0,76 |
| FCHO1 | 742763 | 2,82E-03 | 0,79 |
| CLNS1A | 666426 | 2,83E-03 | 0,8 |
| PDXP | 743182 | 2,87E-03 | 0,81 |
| GALNT7 | 381854 | 2,90E-03 | 0,73 |
| MAP2 | 347503 | 2,96E-03 | 0,75 |
| IMAGE:743903 Homo sapiens, clone IMAGE:5266541, mRNA | 743903 | 3,01E-03 | 0,6 |
| APOD | 838611 | 3,05E-03 | 0,47 |
| IGKV1-5 | 155345 | 3,08E-03 | 0,79 |
| JAK1 | 2030501 | 3,16E-03 | 0,76 |
| BST2 | 811024 | 3,20E-03 | 0,8 |
| CERKL | 489326 | 3,27E-03 | 0,71 |
| AD023 | 731645 | 3,45E-03 | 0,82 |
| EMILIN2 | 365521 | 3,45E-03 | 0,82 |
| ITGA5 | 135671 | 3,46E-03 | 0,76 |
| PSG3 | 136747 | 3,67E-03 | 0,74 |
| C20orf18 | 665433 | 3,69E-03 | 0,7 |
| S100A12 | 1705397 | 3,71E-03 | 0,78 |
| IMAGE:731689 Transcribed locus | 731689 | 3,80E-03 | 0,82 |
| PLXNC1 | 724609 | 4,01E-03 | 0,77 |
| IMAGE:740105 | 740105 | 4,31E-03 | 0,77 |
| CCDC7 | 731389 | 4,33E-03 | 0,74 |

| Table 15 | MNG | Under | |
|---|---|---|---|
| name | cloid | p-value | fold |
| BAK1 | 1288183 | 4,52E-03 | 0,62 |
| RAD51C | 80162 | 4,75E-03 | 0,83 |
| KIAA0427 | 127507 | 4,83E-03 | 0,85 |
| MAK | 382002 | 4,94E-03 | 0,76 |
| DEFB1 | 665086 | 4,98E-03 | 0,44 |
| CXCL12 | 213113 | 5,21E-03 | 0,82 |
| CD4 | 141216 | 5,35E-03 | 0,72 |
| LEF1 | 713913 | 5,36E-03 | 0,76 |
| TOSO | 813174 | 5,39E-03 | 0,73 |
| C21orf7 | 665495 | 5,69E-03 | 0,77 |
| SIAT8F | 667110 | 6,23E-03 | 0,73 |
| MTUS1 | 744395 | 6,27E-03 | 0,61 |
| IMAGE:668300 Transcribed locus, strongly similar to NP_683708.1 Toll-interleukin 1 receptor domain-containing adaptor protein isoform b; adapter protein wyatt; MyD88 adapter-like protein; TIR domain-containing adapter protein; Toll-interleukin 1 receptor (TIR) domain-containing adaptor protein [Homo sapiens] | 668300 | 6,39E-03 | 0,83 |
| CLDN1 | 594279 | 6,42E-03 | 0,46 |
| FLJ22471 | 668510 | 6,75E-03 | 0,56 |
| IMAGE:177857 Transcribed locus | 177857 | 6,78E-03 | 0,74 |
| IMAGE:745512 | 745512 | 6,99E-03 | 0,84 |
| SIAT8C | 382069 | 7,32E-03 | 0,83 |
| STAT1 | 110101 | 7,34E-03 | 0,81 |

| Table 15 | MNG | Under | |
|---|---|---|---|
| name | cloid | p-value | fold |
| TYMS | 2242054 | 7,38E-03 | 0,83 |
| F10 | 310519 | 7,60E-03 | 0,82 |
| SLIT1 | 38403 | 7,79E-03 | 0,78 |
| IL2RA | 3054031 | 7,91E-03 | 0,81 |
| SPDEF | 1188588 | 8,08E-03 | 0,81 |
| ATP2B4 | 665814 | 8,11E-03 | 0,72 |
| TRAF4 | 667756 | 8,19E-03 | 0,82 |
| IMAGE:173818 Transcribed locus | 173818 | 8,27E-03 | 0,81 |
| IMAGE:731758 Transcribed locus, weakly similar to NP_081070.1 kiaa-iso protein homolog; Band 47B [Mus musculus] | 731758 | 8,32E-03 | 0,82 |
| CCR7 | 2345206 | 8,54E-03 | 0,85 |
| HN1 | 471568 | 8,84E-03 | 0,77 |
| ECM1 | 301122 | 8,94E-03 | 0,3 |
| C21orf84 | 730814 | 8,99E-03 | 0,75 |
| BIC | 743270 | 9,01E-03 | 0,79 |
| IMAGE:1859532 | 1859532 | 9,68E-03 | 0,81 |
| IMAGE:382423 Transcribed locus | 382423 | 9,70E-03 | 0,73 |
| PCTK3 | 725677 | 9,81E-03 | 0,8 |
| IMAGE:744410 Transcribed locus | 744410 | 9,87E-03 | 0,82 |
| SPINT1 | 723914 | 9,92E-03 | 0,83 |
| BMP5 | 1846326 | 1,00E-02 | 0,75 |
| MYC | 129438 | 1,02E-02 | 0,7 |
| TP53I11 | 667514 | 1,04E-02 | 0,63 |
| CASP3 | 823680 | 1,08E-02 | 0,75 |
| TIMP1 | 162246 | 1,10E-02 | 0,62 |
| SLC36A3 | 731115 | 1,12E-02 | 0,81 |
| CD63 | 125552 | 1,14E-02 | 0,85 |
| PTPN4 | 666367 | 1,14E-02 | 0,71 |
| AOC3 | 484535 | 1,15E-02 | 0,77 |
| IMAGE:2113771 | 2113771 | 1,20E-02 | 0,83 |
| FLJ25067 | 667252 | 1,21E-02 | 0,68 |
| PSCD3 | 744050 | 1,21E-02 | 0,83 |
| STK17A | 562904 | 1,22E-02 | 0,8 |
| HSPH1 | 666554 | 1,25E-02 | 0,79 |
| FSHPRH1 | 667355 | 1,25E-02 | 0,81 |
| GPC3 | 137131 | 1,26E-02 | 0,78 |
| MEOX1 | 760065 | 1,28E-02 | 0,78 |
| IMAGE:727289 Transcribed locus | 727289 | 1,28E-02 | 0,71 |
| IMAGE:731616 MRNA; cDNA DKFZp686A102 (from clone DKFZp686A102) | 731616 | 1,32E-02 | 0,68 |
| VTN | 230126 | 1,35E-02 | 0,74 |
| MGC45840 | 725231 | 1,43E-02 | 0,79 |
| TNIP1 | 135801 | 1,43E-02 | 0,79 |
| S100A6 | 512420 | 1,47E-02 | 0,8 |
| CALM1 | 594510 | 1,47E-02 | 0,77 |
| SDHA | 40304 | 1,50E-02 | 0,82 |
| NOTCH2 | 1641901 | 1,53E-02 | 0,64 |
| GIMAP5 | 180259 | 1,53E-02 | 0,79 |
| DIO1 | 296702 | 1,54E-02 | 0,74 |
| CHRDL2 | 485872 | 1,57E-02 | 0,85 |
| CCL28 | 136919 | 1,60E-02 | 0,83 |
| IMAGE:364741 | 364741 | 1,61E-02 | 0,74 |
| WAS | 2148946 | 1,63E-02 | 0,75 |
| PITPNC1 | 364436 | 1,66E-02 | 0,8 |
| IMAGE:136976 Transcribed locus, moderately similar to NP_777603.1 hypothetical protein FLJ25976 [Homo sapiens] | 136976 | 1,72E-02 | 0,78 |
| CD33 | 1917430 | 1,74E-02 | 0,74 |
| CLGN | 1049033 | 1,74E-02 | 0,77 |
| POPDC3 | 264166 | 1,76E-02 | 0,8 |
| RIN1 | 741406 | 1,77E-02 | 0,8 |
| GJA5 | 196338 | 1,79E-02 | 0,75 |
| UBE2L6 | 1088980 | 1,80E-02 | 0,84 |
| DNMT1 | 768241 | 1,81E-02 | 0,86 |
| IMAGE:742061 | 742061 | 1,81E-02 | 0,78 |
| BMP4 | 69166 | 1,83E-02 | 0,77 |

| Table 15 | MNG | Under | |
|---|---|---|---|
| name | cloid | p-value | fold |
| SENP6 | 739237 | 1,84E-02 | 0,8 |
| COX6A1 | 512910 | 1,86E-02 | 0,78 |
| SAFB | 42280 | 1,91E-02 | 0,73 |
| PLTP | 134416 | 1,92E-02 | 0,82 |
| ME3 | 724238 | 1,96E-02 | 0,76 |
| CD47 | 357442 | 2,02E-02 | 0,83 |
| MGC23909 | 731598 | 2,05E-02 | 0,72 |
| PREB | 740347 | 2,06E-02 | 0,64 |
| EIF2C4 | 730834 | 2,09E-02 | 0,85 |
| BIN1 | 2384812 | 2,10E-02 | 0,8 |
| PML | 724554 | 2,12E-02 | 0,85 |
| PLCG2 | 201467 | 2,13E-02 | 0,78 |
| CTRL | 1308954 | 2,15E-02 | 0,83 |
| MCC | 731305 | 2,16E-02 | 0,71 |
| PANK1 | 256177 | 2,17E-02 | 0,84 |
| PRKCDBP | 157847 | 2,19E-02 | 0,79 |
| VPS26 | 687336 | 2,22E-02 | 0,85 |
| NOMO1 | 134615 | 2,24E-02 | 0,82 |
| GSTM1 | 73778 | 2,31E-02 | 0,79 |
| S100A16 | 739851 | 2,33E-02 | 0,83 |
| RPS6KA5 | 258966 | 2,33E-02 | 0,87 |
| IMAGE:136801 | 136801 | 2,40E-02 | 0,82 |
| IMAGE:383718 | 383718 | 2,40E-02 | 0,81 |
| ATP11B | 666334 | 2,43E-02 | 0,75 |
| VDAC1 | 486221 | 2,47E-02 | 0,78 |
| C15orf29 | 177775 | 2,49E-02 | 0,77 |
| PCGF4 | 740457 | 2,53E-02 | 0,78 |
| EIF4EBP1 | 713608 | 2,56E-02 | 0,83 |
| GFPT2 | 485085 | 2,60E-02 | 0,76 |
| DUSP3 | 119772 | 2,63E-02 | 0,85 |
| SASH1 | 31120 | 2,66E-02 | 0,82 |
| LCN2 | 544683 | 2,68E-02 | 0,67 |
| KLRD1 | 145696 | 2,76E-02 | 0,88 |
| IMAGE:383528 Transcribed locus, weakly similar to XP_375099.1 PREDICTED: similar to hypothetical protein FLJ25224 [Homo sapiens] | 383528 | 2,79E-02 | 0,36 |
| IMAGE:743619 | 743619 | 2,81E-02 | 0,81 |
| CASP5 | 341763 | 2,82E-02 | 0,71 |
| FBXO46 | 471664 | 2,82E-02 | 0,75 |
| MRC2 | 342581 | 2,84E-02 | 0,73 |
| IMAGE:726647 Transcribed locus | 726647 | 2,87E-02 | 0,84 |
| NME7 | 743982 | 2,87E-02 | 0,7 |
| IMAGE:743290 Transcribed locus | 743290 | 2,95E-02 | 0,76 |
| CD5 | 356841 | 3,06E-02 | 0,8 |
| RPL27A | 178255 | 3,06E-02 | 0,69 |
| ADAM12 | 724812 | 3,07E-02 | 0,74 |
| ICAM3 | 156183 | 3,13E-02 | 0,84 |
| GAPD | 152847 | 3,13E-02 | 0,76 |
| YWHAH | 324066 | 3,15E-02 | 0,86 |
| C6orf216 | 731742 | 3,18E-02 | 0,84 |
| SPATA5L1 | 724884 | 3,19E-02 | 0,74 |
| CITED1 | 265558 | 3,23E-02 | 0,55 |
| VIL2 | 124701 | 3,27E-02 | 0,81 |
| SLC2A4RG | 376983 | 3,35E-02 | 0,86 |
| MDH1 | 725188 | 3,38E-02 | 0,87 |
| KCNK4 | 743016 | 3,38E-02 | 0,83 |
| GSS | 140405 | 3,42E-02 | 0,82 |
| NR2F1 | 253386 | 3,48E-02 | 0,8 |
| MX1 | 713879 | 3,55E-02 | 0,82 |
| MGC5395 | 238840 | 3,57E-02 | 0,84 |
| SYP | 30471 | 3,58E-02 | 0,66 |
| RGS19 | 485803 | 3,61E-02 | 0,82 |
| CTXN1 | 179266 | 3,61E-02 | 0,86 |
| LOC284669 | 364885 | 3,61E-02 | 0,78 |
| KRT7 | 592276 | 3,63E-02 | 0,83 |
| ALDH1A3 | 486189 | 3,67E-02 | 0,76 |
| KLRK1 | 725473 | 3,71E-02 | 0,84 |
| SAP30L | 723950 | 3,81E-02 | 0,85 |
| PSEN1 | 724537 | 3,87E-02 | 0,86 |
| PDK1 | 1645668 | 3,92E-02 | 0,86 |
| MAN1A1 | 137719 | 3,93E-02 | 0,66 |
| PLCG1 | 1174287 | 4,01E-02 | 0,78 |
| IMAGE:743517 Transcribed locus | 743517 | 4,03E-02 | 0,84 |
| IMAGE:257555 | 257555 | 4,03E-02 | 0,76 |
| POU2F2 | 188393 | 4,12E-02 | 0,8 |
| IMAGE:745123 Transcribed locus | 745123 | 4,22E-02 | 0,58 |
| LILRB5 | 71428 | 4,28E-02 | 0,81 |
| LCN2 | 741497 | 4,32E-02 | 0,47 |
| USP4 | 544818 | 4,38E-02 | 0,76 |

| Table 15 | MNG | Under | |
|---|---|---|---|
| name | cloid | p-value | fold |
| IMAGE:251427 | 251427 | 4,44E-02 | 0,83 |
| KIAA1280 | 366085 | 4,52E-02 | 0,85 |
| MESP1 | 25865 | 4,57E-02 | 0,86 |
| C22orf16 | 236119 | 4,63E-02 | 0,81 |
| IMAGE:485104 Transcribed locus | 485104 | 4,72E-02 | 0,72 |
| CCL1 | 1570420 | 4,72E-02 | 0,77 |
| KNS2 | 174654 | 4,79E-02 | 0,85 |
| IMAGE:745072 Transcribed locus | 745072 | 4,82E-02 | 0,78 |
| MUTED | 731202 | 4,88E-02 | 0,77 |
| KCNQ2 | 179534 | 4,90E-02 | 0,83 |
| PMS2 | 116906 | 4,92E-02 | 0,77 |
| PDCD1LG1 | 1942634 | 4,94E-02 | 0,72 |

| Table 16 | MNG | Over | |
|---|---|---|---|
| **name** | **cloid** | **p-value** | **fold** |
| NEXN | 687625 | 7,18E-05 | 1,40 |
| IMAGE:726782 Transcribed locus | 726782 | 2,00E-03 | 1,43 |
| PRUNE | 364324 | 2,56E-03 | 1,30 |
| GATA3 | 148796 | 2,80E-03 | 1,40 |
| IMAGE:744505 | 744505 | 2,84E-03 | 1,35 |
| IMAGE:26164 | 26164 | 3,13E-03 | 1,29 |
| TTC17 | 665668 | 3,15E-03 | 1,29 |
| FLJ20674 | 713837 | 3,84E-03 | 1,20 |
| ALCAM | 172828 | 3,93E-03 | 1,23 |
| IMAGE:731726 Transcribed locus | 731726 | 4,48E-03 | 1,54 |
| FOXO1A | 151247 | 4,60E-03 | 1,30 |
| ZNF83 | 486356 | 4,65E-03 | 1,34 |
| BCL2 | 342181 | 6,17E-03 | 1,36 |
| RODH | 471641 | 6,87E-03 | 2,44 |
| NR2F2 | 72744 | 7,02E-03 | 1,39 |
| PDLIM1 | 135689 | 8,66E-03 | 1,49 |
| C10orf116 | 740941 | 9,15E-03 | 1,67 |
| C20orf19 | 366032 | 1,03E-02 | 1,27 |
| FCGBP | 154172 | 1,26E-02 | 1,34 |
| ARL6IP2 | 743987 | 1,27E-02 | 1,26 |
| MGC17299 | 713422 | 1,28E-02 | 1,40 |
| AQP4 | 279172 | 1,28E-02 | 1,65 |
| FOLR1 | 131839 | 1,31E-02 | 1,53 |
| LOC80298 | 666551 | 1,45E-02 | 1,19 |
| IMAGE:665392 Hypothetical LOC388790 | 665392 | 1,54E-02 | 1,36 |
| LRP2 | 2055272 | 1,76E-02 | 1,44 |
| MKKS | 729957 | 1,78E-02 | 1,23 |
| NR6A1 | 258666 | 1,96E-02 | 1,29 |
| PHC3 | 743391 | 2,05E-02 | 1,12 |
| KCNC3 | 53333 | 2,15E-02 | 1,18 |
| FOXO3A | 109142 | 2,18E-02 | 1,28 |
| IMAGE:743615 Full-length cDNA clone CS0DK001YE19 of HeLa cells Cot 25-normalized of Homo sapiens (human) | 743615 | 2,28E-02 | 1,13 |
| CAV1 | 133531 | 2,29E-02 | 1,39 |
| CAV1 | 309645 | 2,31E-02 | 1,78 |
| GALT | 68972 | 2,49E-02 | 1,18 |
| IMAGE:682311 LOC440722 | 682311 | 2,61E-02 | 1,21 |
| IMAGE:666946 | 666946 | 2,61E-02 | 1,18 |
| FLJ21918 | 194302 | 2,73E-02 | 1,10 |
| SIN3A | 26455 | 2,80E-02 | 1,12 |
| IMAGE:744439 Transcribed locus | 744439 | 2,86E-02 | 1,16 |
| THUMPD1 | 739451 | 2,96E-02 | 1,24 |
| IL6ST | 137010 | 3,31E-02 | 1,18 |
| HDAC6 | 669295 | 3,34E-02 | 1,17 |
| KRTHA4 | 325155 | 3,60E-02 | 1,51 |
| IMAGE:731685 Transcribed locus | 731685 | 3,71E-02 | 1,23 |
| EPHB6 | 172982 | 3,75E-02 | 1,39 |
| HDAC9 | 738506 | 3,79E-02 | 1,24 |
| ESRRBL1 | 51232 | 3,82E-02 | 1,16 |
| MT1F | 78353 | 3,89E-02 | 1,39 |
| C9orf10OS | 742695 | 4,03E-02 | 1,17 |
| ETV4 | 1690788 | 4,11E-02 | 1,30 |
| LOC51760 | 52226 | 4,12E-02 | 1,27 |
| MBNL1 | 136114 | 4,19E-02 | 1,16 |
| CES2 | 153667 | 4,81E-02 | 1,14 |
| LOC286170 | 134858 | 4,81E-02 | 1,21 |
| PRKG1 | 667587 | 4,91E-02 | 1,16 |

| Table 17 | OTA | Under | |
|---|---|---|---|
| name | cloid | p-value | fold |
| MLL | 80688 | 1,6437E-15 | 0,51 |
| APOE | 1870594 | 9,7061E-13 | 0,39 |
| RFX2 | 731738 | 9,0218E-12 | 0,48 |
| APOE | 1870594 | 3,2318E-11 | 0,29 |
| FUS | 365348 | 5,854E-11 | 0,60 |
| IMAGE:681875 | 681875 | 6,0611E-11 | 0,70 |
| IMAGE:731751 Transcribed locus | 731751 | 7,5532E-11 | 0,44 |
| MBNL1 | 136114 | 1,6046E-10 | 0,58 |
| NPFF | 365161 | 1,7737E-10 | 0,55 |
| C4A | 491004 | 1,9841E-10 | 0,35 |
| C4A | 724366 | 3,9317E-10 | 0,33 |
| ZNF395 | 744983 | 5,8686E-10 | 0,62 |
| LOC389906 | 740718 | 6,1317E-10 | 0,72 |
| HMGB2 | 884365 | 1,3605E-09 | 0,64 |
| ANKRD28 | 687381 | 1,6567E-09 | 0,64 |
| SPINL | 236399 | 3,6199E-09 | 0,31 |
| IMAGE:731726 Transcribed locus | 731726 | 8,8574E-09 | 0,53 |
| URB | 809719 | 9,1784E-09 | 0,48 |
| CAPN3 | 757248 | 1,6524E-08 | 0,64 |
| EPHB6 | 172982 | 1,7797E-08 | 0,42 |
| IMP-2 | 743774 | 3,0771E-08 | 0,33 |
| DCN | 666410 | 3,1847E-08 | 0,51 |
| FLJ10858 | 743961 | 3,3637E-08 | 0,63 |
| G3BP | 667239 | 6,2302E-08 | 0,63 |
| GTPBP5 | 725502 | 8,9429E-08 | 0,50 |
| CDKN1B | 265345 | 1,3838E-07 | 0,65 |
| SENP6 | 366436 | 1,4497E-07 | 0,74 |
| NYREN18 | 668584 | 1,4512E-07 | 0,62 |
| NT5C2 | 725076 | 1,6229E-07 | 0,70 |
| HNRPA3 | 365349 | 1,6398E-07 | 0,65 |
| GATA3 | 148796 | 2,4888E-07 | 0,63 |
| TIAM1 | 23612 | 2,8111E-07 | 0,34 |
| IMAGE:725726 Hypothetical gene supported by AK093801 | 725726 | 2,8637E-07 | 0,68 |
| LOC286272 | 667361 | 3,1104E-07 | 0,72 |
| KCNC3 | 53333 | 3,1744E-07 | 0,60 |
| CASP7 | 279470 | 3,4531E-07 | 0,72 |
| LOC147650 | 729510 | 3,7898E-07 | 0,66 |
| SLC6A8 | 725877 | 4,4149E-07 | 0,22 |
| IMAGE:729956 | 729956 | 4,703E-07 | 0,69 |

| Table 17 | OTA | Under | |
|---|---|---|---|
| name | cloid | p-value | fold |
| CXCL14 | 345034 | 5,6941E-07 | 0,11 |
| CFH | 665784 | 1,4226E-06 | 0,47 |
| SPATA12 | 730300 | 1,5763E-06 | 0,65 |
| NUP214 | 382612 | 1,6434E-06 | 0,58 |
| FLJ22794 | 137454 | 1,6888E-06 | 0,63 |
| PDLIM1 | 135689 | 1,7329E-06 | 0,59 |
| SNTB2 | 667310 | 1,8243E-06 | 0,76 |
| TLX3 | 1860115 | 1,9435E-06 | 0,53 |
| IMAGE:731714 CDNA FLJ26188 fis, clone ADG04821 | 731714 | 2,3202E-06 | 0,65 |
| IMAGE:365097 CDNA FLJ25129 fis, clone CBR06594 | 365097 | 2,5097E-06 | 0,75 |
| SNRP70 | 729971 | 2,6207E-06 | 0,57 |
| FLJ12604 | 731128 | 2,8824E-06 | 0,60 |
| IMAGE:682311 LOC440722 | 682311 | 2,9031E-06 | 0,71 |
| APLP2 | 549054 | 2,9384E-06 | 0,61 |
| HP1-BP74 | 726092 | 4,2033E-06 | 0,58 |
| BAZ2B | 609631 | 4,4078E-06 | 0,73 |
| PBXIP1 | 366042 | 4,4151E-06 | 0,58 |
| SEMA4B | 714437 | 4,6922E-06 | 0,70 |
| GALT | 68972 | 5,1029E-06 | 0,70 |
| SLC25A23 | 741954 | 5,2058E-06 | 0,65 |
| CICE | 725791 | 5,2466E-06 | 0,72 |
| INHBB | 730012 | 5,2829E-06 | 0,45 |
| FRMD4B | 669564 | 5,3205E-06 | 0,54 |
| MGC40214 | 666455 | 5,4593E-06 | 0,77 |
| MGC5395 | 238840 | 5,5794E-06 | 0,62 |
| CaMKIINalpha | 173820 | 6,3166E-06 | 0,63 |
| IMAGE:743485 Similar to RIKEN cDNA E130306M17 gene | 743485 | 7,0609E-06 | 0,64 |
| IMAGE:726782 Transcribed locus | 726782 | 7,5601E-06 | 0,71 |
| MRC2 | 235882 | 8,1186E-06 | 0,44 |
| MED19 | 743760 | 8,1229E-06 | 0,73 |
| PRUNE | 364324 | 8,2734E-06 | 0,67 |
| CASP3 | 823680 | 8,3059E-06 | 0,63 |
| TJP1 | 179334 | 8,8933E-06 | 0,69 |
| SEPT-02 | 729542 | 8,9128E-06 | 0,58 |
| CCBP2 | 135930 | 9,9031E-06 | 0,70 |

| Table 17 | OTA | Under | |
|---|---|---|---|
| name | cloid | p-value | fold |
| HDAC9 | 738506 | 9,9758E-06 | 0,74 |
| MMP2 | 155839 | 1,0013E-05 | 0,54 |
| NFIB | 416959 | 1,043E-05 | 0,64 |
| RDH5 | 682109 | 1,0868E-05 | 0,73 |
| IMAGE:175268 | 175268 | 1,108E-05 | 0,64 |
| IMAGE:26486 CDNA FLJ43345 fis, clone NT2RI3008228 | 26486 | 1,129E-05 | 0,78 |
| RASGRP1 | 725707 | 1,1705E-05 | 0,44 |
| BCAP29 | 725970 | 1,2079E-05 | 0,56 |
| ITPR1 | 667348 | 1,2089E-05 | 0,65 |
| SGSH | 669263 | 1,2526E-05 | 0,71 |
| IMAGE:28705 | 28705 | 1,3308E-05 | 0,75 |
| CECR1 | 725612 | 1,4252E-05 | 0,74 |
| TIMP1 | 162246 | 1,4504E-05 | 0,40 |
| IMAGE:666140 Transcribed locus, weakly similar to XP_498467.1 PREDICTED: hypothetical protein XP_498467 [Homo sapiens] | 666140 | 1,5033E-05 | 0,75 |
| IMAGE:738332 Transcribed locus | 738332 | 1,6574E-05 | 0,43 |
| SIAT4B | 365877 | 1,7795E-05 | 0,78 |
| C10orf116 | 740941 | 1,8397E-05 | 0,49 |
| SYN3 | 727056 | 1,8892E-05 | 0,52 |
| APOD | 838611 | 1,9338E-05 | 0,30 |
| LOC286076 | 731275 | 1,9611E-05 | 0,64 |
| F10 | 310519 | 2,0346E-05 | 0,73 |
| IMAGE:384087 | 384087 | 2,1119E-05 | 0,69 |
| STYX | 174879 | 2,2844E-05 | 0,68 |
| PLA2G6 | 744087 | 2,6403E-05 | 0,60 |
| IMAGE:727496 Transcribed locus | 727496 | 2,6792E-05 | 0,53 |
| IMAGE:379279 | 379279 | 2,7991E-05 | 0,76 |
| COG3 | 25226 | 2,8031E-05 | 0,70 |

| Table 17 | OTA | Under | |
|---|---|---|---|
| name | cloid | p-value | fold |
| IMAGE:731428 Transcribed locus, weakly similar to XP_521389.1 PREDICTED: similar to UTY [Pan troglodytes] | 731428 | 3,4732E-05 | 0,75 |
| IMAGE:365990 | 365990 | 3,8574E-05 | 0,69 |
| FMNL2 | 364568 | 4,0777E-05 | 0,76 |
| IMAGE:2266583 | 2266583 | 4,0793E-05 | 0,69 |
| MDGA1 | 180082 | 4,196E-05 | 0,73 |
| MGP | 590264 | 4,9648E-05 | 0,53 |
| LRP5 | 725836 | 5,9288E-05 | 0,72 |
| NFYA | 731648 | 5,9861E-05 | 0,75 |
| NR2F1 | 253386 | 6,0806E-05 | 0,71 |
| FOXO1A | 151247 | 6,436E-05 | 0,65 |
| PPARD | 724562 | 6,7271E-05 | 0,71 |
| FLJ20674 | 713837 | 6,9459E-05 | 0,81 |
| COL27A1 | 379517 | 6,9891E-05 | 0,71 |
| SALL4 | 726454 | 7,059E-05 | 0,37 |
| AXIN2 | 135887 | 7,4334E-05 | 0,70 |
| LOC126669 | 682088 | 7,448E-05 | 0,70 |
| TETRAN | 725340 | 8,5261E-05 | 0,73 |
| CTH | 730606 | 8,5368E-05 | 0,72 |
| CCL5 | 840753 | 8,8355E-05 | 0,68 |
| PPP1CA | 1422791 | 9,3449E-05 | 0,72 |
| IMAGE:723752 | 723752 | 9,349E-05 | 0,72 |
| PRKCQ | 669149 | 9,6294E-05 | 0,74 |
| RGL2 | 741891 | 0,00010227 | 0,75 |
| HDAC6 | 669295 | 0,00010356 | 0,75 |
| BTG1 | 382760 | 0,0001116 | 0,74 |
| ABCC3 | 208097 | 0,00011391 | 0,50 |
| APOL1 | 665632 | 0,00011456 | 0,48 |
| IMAGE:731665 | 731665 | 0,00012147 | 0,70 |
| FARP1 | 486708 | 0,0001285 | 0,68 |
| SFRP4 | 285693 | 0,00013182 | 0,49 |
| IL19 | 1932168 | 0,00013629 | 0,59 |
| IQCF3 | 726578 | 0,00014161 | 0,73 |
| HDAC1 | 1896337 | 0,00014481 | 0,71 |
| UBE1L | 250883 | 0,00014933 | 0,63 |
| FLJ34433 | 713671 | 0,00015152 | 0,77 |
| SMARCA5 | 730037 | 0,00015195 | 0,75 |
| TPM2 | 740620 | 0,0001529 | 0,59 |
| UPF2 | 682149 | 0,0001531 | 0,79 |
| PMS2 | 116906 | 0,00016313 | 0,59 |

| Table 17 | OTA | Under | |
|---|---|---|---|
| name | cloid | p-value | fold |
| IMAGE:669470 CDNA clone IMAGE:5206119, partial cds | 669470 | 0,0001635 | 0,80 |
| GCLC | 1526058 | 0,0001647 | 0,78 |
| CITED2 | 365090 | 0,00016528 | 0,60 |
| GRK5 | 740245 | 0,00016593 | 0,71 |
| ETV1 | 24541 | 0,00016784 | 0,76 |
| NCK1 | 1326169 | 0,00017218 | 0,66 |
| CRYZ | 486935 | 0,00018293 | 0,83 |
| SPTLC2 | 365000 | 0,00019182 | 0,78 |
| IMAGE:727123 Transcribed locus, strongly similar to NP_004491.1 heterogeneous nuclear ribonucleoprotein C isoform b; nuclear ribonucleoprotein particle C2 protein; nuclear ribonucleoprotein particle C1 protein [Homo sapiens] | 727123 | 0,00020299 | 0,77 |
| IMAGE:1877668 | 1877668 | 0,00020813 | 0,74 |
| PTPRS | 666066 | 0,00021386 | 0,74 |
| IRAK1BP1 | 687306 | 0,0002186 | 0,75 |
| NBL1 | 503874 | 0,00022339 | 0,50 |
| PSPC1 | 687541 | 0,00023946 | 0,76 |
| PDCD1LG2 | 738944 | 0,00025058 | 0,71 |
| C9orf72 | 726849 | 0,0002553 | 0,70 |
| CA3 | 365609 | 0,0002601 | 0,74 |
| DNALI1 | 782688 | 0,0002748 | 0,62 |
| PIAS1 | 38789 | 0,00028384 | 0,73 |
| GLT8D2 | 365271 | 0,00028457 | 0,57 |
| CYP51A1 | 739901 | 0,00030959 | 0,73 |
| IMAGE:145112 | 145112 | 0,00031549 | 0,71 |
| CDKN1C | 2381770 | 0,00031705 | 0,69 |
| IMAGE:742853 | 742853 | 0,00032014 | 0,50 |

| Table 17 | OTA | Under | |
|---|---|---|---|
| name | cloid | p-value | fold |
| AOC3 | 484535 | 0,00032283 | 0,69 |
| CD5 | 356841 | 0,00032358 | 0,71 |
| PHC3 | 743391 | 0,00033419 | 0,79 |
| GLG1 | 365613 | 0,00034049 | 0,70 |
| DJ462O23.2 | 738970 | 0,00034686 | 0,74 |
| FLJ35794 | 364204 | 0,00035268 | 0,64 |
| PREB | 740347 | 0,00037117 | 0,45 |
| TFDP2 | 742806 | 0,00038692 | 0,66 |
| CAPNS1 | 152655 | 0,00038965 | 0,78 |
| LILRB4 | 2341829 | 0,00042057 | 0,77 |
| SLC9A5 | 112819 | 0,00042518 | 0,66 |
| LASP1 | 592598 | 0,00043231 | 0,42 |
| ZNF580 | 668089 | 0,00043314 | 0,74 |
| FGFRL1 | 739956 | 0,00043483 | 0,64 |
| ERK8 | 714049 | 0,00043612 | 0,72 |
| IMAGE:668684 Transcribed locus, weakly similar to XP_209041.2 PREDICTED: similar to KIAA1503 protein [Homo sapiens] | 668684 | 0,00044757 | 0,76 |
| GLMN | 293916 | 0,00044948 | 0,84 |
| DF | 666128 | 0,00045614 | 0,49 |
| PDGFRL | 139242 | 0,00045735 | 0,61 |
| ESRRBL1 | 51232 | 0,00046822 | 0,70 |
| DKFZp434M202 | 743118 | 0,00047312 | 0,73 |
| KLRD1 | 2728204 | 0,00051707 | 0,67 |
| HMGN1 | 565754 | 0,00051902 | 0,72 |
| MAP3K4 | 727229 | 0,00053005 | 0,76 |
| IMAGE:2254555 | 2254555 | 0,00056693 | 0,73 |
| THEM2 | 742543 | 0,000571 | 0,68 |
| IKBKE | 364448 | 0,00058383 | 0,78 |
| FLJ20254 | 135096 | 0,00060077 | 0,75 |
| ZNF251 | 486401 | 0,00060459 | 0,34 |
| FLJ10707 | 364575 | 0,00063587 | 0,79 |
| AQP11 | 713831 | 0,00064759 | 0,64 |
| ARHGEF6 | 687990 | 0,00068864 | 0,80 |
| IMAGE:668924 | 668924 | 0,00072815 | 0,79 |
| IMAGE:738896 | 738896 | 0,00075859 | 0,29 |
| FOLR1 | 131839 | 0,00076028 | 0,60 |
| SULT1E1 | 207926 | 0,00078718 | 0,80 |
| HIS1 | 342551 | 0,00080399 | 0,72 |

| Table 17 | OTA | Under | |
|---|---|---|---|
| name | cloid | p-value | fold |
| SEMA4D | 210587 | 0,00080847 | 0,76 |
| CCND1 | 324079 | 0,00085252 | 0,67 |
| PSAT1 | 366388 | 0,00087487 | 0,66 |
| CCNL1 | 251216 | 0,00087862 | 0,68 |
| MGC3329 | 137005 | 0,00088837 | 0,77 |
| C10orf61 | 741894 | 0,00095106 | 0,72 |
| LIPT1 | 22085 | 0,00095361 | 0,71 |
| VCL | 44193 | 0,00103155 | 0,79 |
| FOXO3A | 109142 | 0,0010426 | 0,75 |
| PGR1 | 666361 | 0,00108576 | 0,76 |
| CREB1 | 148212 | 0,00109064 | 0,81 |
| GSS | 140405 | 0,00109778 | 0,74 |
| SSH3 | 726272 | 0,00114183 | 0,75 |
| SEPT-05 | 448163 | 0,00117115 | 0,26 |
| MORF4L1 | 135875 | 0,00122685 | 0,75 |
| BAT3 | 24392 | 0,00123243 | 0,75 |
| IMAGE:7254 01 | 725401 | 0,00124171 | 0,77 |
| SF3B1 | 739247 | 0,0012449 | 0,67 |
| APOL3 | 366289 | 0,00126395 | 0,64 |
| S100A4 | 868577 | 0,00130679 | 0,44 |
| LOC286170 | 134858 | 0,00131926 | 0,69 |
| COL1A1 | 140347 | 0,00137465 | 0,51 |
| DNCLI2 | 261656 | 0,00138781 | 0,74 |
| PDCD4 | 665376 | 0,00144209 | 0,59 |
| FLJ27523 | 743246 | 0,00146851 | 0,45 |
| PLXNB2 | 1420676 | 0,00149466 | 0,69 |
| ZNF512 | 726596 | 0,00156733 | 0,72 |
| CST3 | 357201 | 0,00160531 | 0,75 |
| WHSC2 | 668283 | 0,00162671 | 0,75 |
| ALCAM | 172828 | 0,00163738 | 0,77 |
| NOG | 487474 | 0,00165986 | 0,73 |
| IMAGE:6658 33 | 665833 | 0,00178979 | 0,77 |
| CAV1 | 309645 | 0,00182486 | 0,59 |
| MTUS1 | 376597 | 0,00182533 | 0,52 |
| IMAGE:7316 85 Transcribed locus | 731685 | 0,0018723 | 0,83 |
| IKBKB | 2484742 | 0,00188154 | 0,81 |
| PP784 | 366089 | 0,00188347 | 0,74 |
| CITED2 | 286138 | 0,00188602 | 0,76 |
| TYMS | 687912 | 0,00193104 | 0,73 |
| CD3E | 1536968 | 0,00197216 | 0,70 |
| USP13 | 666007 | 0,00198696 | 0,74 |
| RIPK3 | 667313 | 0,00202685 | 0,68 |
| NOTCH2 | 1641901 | 0,00206595 | 0,54 |
| TNFSF12 | 136361 | 0,00207861 | 0,82 |
| CA11 | 282587 | 0,0020984 | 0,74 |
| WDR20 | 665156 | 0,00234165 | 0,83 |
| ZNF398 | 365311 | 0,00235137 | 0,72 |
| FLJ12903 | 730123 | 0,00243509 | 0,82 |
| EVI5L | 173119 | 0,00249305 | 0,72 |
| NCAM1 | 366842 | 0,00252012 | 0,74 |
| GALNT9 | 178750 | 0,00257206 | 0,77 |
| CCL19 | 1707527 | 0,00264302 | 0,17 |
| SATB1 | 364510 | 0,00277604 | 0,79 |
| DHRS6 | 364412 | 0,00279084 | 0,76 |
| RNF123 | 366159 | 0,00283529 | 0,79 |
| IMAGE:6687 07 TGF-betaIIR beta | 668707 | 0,00284093 | 0,76 |
| CRLF3 | 279150 | 0,00284281 | 0,78 |
| THUMPD1 | 739451 | 0,00284724 | 0,77 |
| DGKD | 365408 | 0,00291163 | 0,78 |
| TSC | 745490 | 0,00296358 | 0,52 |
| EED | 667365 | 0,00305049 | 0,76 |
| GRB7 | 510318 | 0,00305513 | 0,83 |
| PIK3CA | 2238108 | 0,00307726 | 0,44 |
| CCNE1 | 68950 | 0,00312811 | 0,71 |
| TIE1 | 743043 | 0,00338928 | 0,73 |
| STAT2 | 2306096 | 0,00346577 | 0,79 |
| POLR2A | 740130 | 0,00347566 | 0,73 |
| AMPD2 | 669141 | 0,00356607 | 0,78 |
| IMAGE:7437 22 | 743722 | 0,00368853 | 0,76 |
| IMAGE:2238 4 CDNA FLJ34183 fis, clone FCBBF3016 987 | 22384 | 0,00374794 | 0,80 |
| IGF1R | 682555 | 0,00381952 | 0,80 |
| RANBP10 | 682251 | 0,0038256 | 0,80 |
| ZNF651 | 669181 | 0,00385349 | 0,83 |
| TPD52 | 743259 | 0,00385608 | 0,28 |
| KLF1 | 208991 | 0,00386372 | 0,66 |
| IMAGE:3422 56 | 342256 | 0,00392068 | 0,32 |
| KIAA1838 | 744106 | 0,00395933 | 0,79 |
| TOP1 | 666425 | 0,0039984 | 0,83 |
| HOXB6 | 738358 | 0,00426232 | 0,81 |
| LAPTM4A | 726684 | 0,00435163 | 0,66 |
| IMAGE:3655 62 Transcribed locus, moderately similar to XP_521389. 1 PREDICTED : similar to UTY [Pan | 365562 | 0,00436529 | 0,78 |

| Table 17 | OTA | Under | |
|---|---|---|---|
| name | cloid | p-value | fold |
| troglodytes] | | | |
| ANKRD20B | 745106 | 0,00437688 | 0,66 |
| IMAGE:665392 Hypothetical LOC388790 | 665392 | 0,00443685 | 0,79 |
| MGAT3 | 731060 | 0,00445388 | 0,78 |
| NEXN | 687625 | 0,00446474 | 0,81 |
| DJ159A19.3 | 23945 | 0,0044943 | 0,75 |
| SELE | 1239535 | 0,004537 | 0,78 |
| ATXN1 | 682528 | 0,00455958 | 0,75 |
| LOC440712 | 725268 | 0,00473002 | 0,71 |
| GPR124 | 486493 | 0,00477135 | 0,79 |
| DAXX | 292042 | 0,00488144 | 0,83 |
| ZBTB16 | 2467442 | 0,00498586 | 0,67 |
| DNCLI2 | 134671 | 0,00499696 | 0,74 |
| ZNF83 | 486356 | 0,00506848 | 0,76 |
| DELGEF | 665437 | 0,00509526 | 0,78 |
| IFITM3 | 713623 | 0,00515614 | 0,76 |
| IMAGE:666029 CDNA FLJ43311 fis, clone NT2RI2009855 | 666029 | 0,00516625 | 0,71 |
| C19orf33 | 379540 | 0,0053682 | 0,69 |
| PDCD1LG1 | 1942634 | 0,00541203 | 0,79 |
| FLJ12592 | 252489 | 0,0054876 | 0,77 |
| RAD51L1 | 295412 | 0,00558334 | 0,69 |
| EMX2 | 365121 | 0,00561885 | 0,73 |
| CLDN1 | 594279 | 0,00570286 | 0,44 |
| HSPB1 | 23827 | 0,00579753 | 0,66 |
| C21orf55 | 666517 | 0,00584797 | 0,76 |
| TBC1D22A | 682479 | 0,00589263 | 0,81 |
| CNN1 | 726779 | 0,0058977 | 0,76 |
| DKFZp761P0423 | 730829 | 0,00596086 | 0,67 |
| IMAGE:21808 Transcribed locus | 21808 | 0,00612512 | 0,80 |
| YTHDF3 | 135450 | 0,00614088 | 0,76 |
| LGALS3BP | 742100 | 0,00646127 | 0,58 |
| LOC388886 | 34007 | 0,00661606 | 0,76 |
| SLAMF8 | 288807 | 0,00672479 | 0,73 |
| DEK | 1016390 | 0,00680353 | 0,82 |
| PDK2 | 114008 | 0,00684106 | 0,85 |
| GLG1 | 366254 | 0,00690562 | 0,83 |
| MGC17299 | 713422 | 0,00716637 | 0,83 |
| TCEA2 | 730149 | 0,00727116 | 0,83 |
| HBB | 469549 | 0,00736441 | 0,52 |
| FLJ31438 | 135539 | 0,00740367 | 0,81 |
| HCLS1 | 665452 | 0,00742076 | 0,73 |
| CLPX | 725394 | 0,00742676 | 0,81 |
| PLEKHH2 | 665254 | 0,00803369 | 0,73 |
| MMRN1 | 759865 | 0,00838721 | 0,78 |
| LOC400451 | 667174 | 0,0084152 | 0,73 |
| MGC4707 | 744945 | 0,00868037 | 0,83 |
| BBS4 | 726634 | 0,00906801 | 0,83 |
| LOC283070 | 172785 | 0,00927382 | 0,74 |
| MAPK14 | 2011082 | 0,0094865 | 0,79 |
| MXD3 | 714013 | 0,00950378 | 0,82 |
| KIAA0789 | 33621 | 0,00966515 | 0,66 |
| NOTCH1 | 359461 | 0,0098421 | 0,79 |
| GPC3 | 137131 | 0,01032142 | 0,78 |
| KCNK5 | 134978 | 0,01043433 | 0,70 |
| ECM1 | 301122 | 0,01051771 | 0,29 |
| FLJ12895 | 23271 | 0,01069841 | 0,76 |
| PLAC9 | 366115 | 0,01071461 | 0,68 |
| LOH12CR2 | 366074 | 0,01111041 | 0,76 |
| MEF2C | 687270 | 0,01126036 | 0,86 |
| IMAGE:687667 CDNA clone IMAGE:4811759, partial cds | 687667 | 0,0113645 | 0,56 |
| VEGFC | 503189 | 0,01190861 | 0,79 |
| CCNE1 | 357807 | 0,01195592 | 0,78 |
| IMAGE:743415 Transcribed locus | 743415 | 0,01197603 | 0,82 |
| OAZ2 | 128694 | 0,01241057 | 0,84 |
| IMAGE:731398 | 731398 | 0,01252095 | 0,80 |
| GIMAP5 | 180259 | 0,01300622 | 0,76 |
| CUGBP2 | 666235 | 0,01304288 | 0,84 |
| IMAGE:667353 Transcribed locus | 667353 | 0,01314807 | 0,83 |
| IGF1R | 682555 | 0,0132764 | 0,83 |
| ACTR1B | 136868 | 0,0134187 | 0,84 |
| BZRP | 135991 | 0,01347669 | 0,79 |
| KIAA1958 | 687287 | 0,01353299 | 0,79 |
| MAPK14 | 1741589 | 0,01358144 | 0,80 |
| TOE1 | 52897 | 0,01361943 | 0,81 |
| KIAA0515 | 687852 | 0,0136789 | 0,79 |
| GATA4 | 781738 | 0,01434771 | 0,79 |
| IMAGE:26164 | 26164 | 0,01438992 | 0,83 |
| PLA2G2A | 152802 | 0,01468617 | 0,71 |
| IMAGE:1089025 | 1089025 | 0,01469019 | 0,52 |

| Table 17 | OTA | Under | |
|---|---|---|---|
| name | cloid | p-value | fold |
| FCGBP | 154172 | 0,01497258 | 0,69 |
| PTK2B | 43541 | 0,0150726 | 0,86 |
| GNB1 | 24300 | 0,01516968 | 0,78 |
| GPR108 | 25949 | 0,01526513 | 0,83 |
| PCTK3 | 725677 | 0,01530815 | 0,78 |
| SMAD4 | 214611 | 0,01537935 | 0,83 |
| IMAGE:744439 Transcribed locus | 744439 | 0,01590113 | 0,86 |
| IMAGE:23872 AF034176 Human mRNA (Tripodis and Ragoussis) Homo sapiens cDNA clone ntcon5 contig | 23872 | 0,01600529 | 0,82 |
| LILRA1 | 2032639 | 0,01630429 | 0,71 |
| PH-4 | 730938 | 0,01635938 | 0,85 |
| PET112L | 743125 | 0,01665049 | 0,76 |
| PTHLH | 2214396 | 0,01725027 | 0,69 |
| CD9 | 727251 | 0,01759599 | 0,82 |
| SPON2 | 723923 | 0,01769163 | 0,78 |
| KIAA1196 | 738938 | 0,01786927 | 0,80 |
| SMAD4 | 134785 | 0,01807119 | 0,82 |
| BTBD14B | 173288 | 0,01820373 | 0,76 |
| PVRL2 | 725364 | 0,01827273 | 0,82 |
| SIN3A | 26455 | 0,01860378 | 0,84 |
| CXCL12 | 213113 | 0,01879575 | 0,84 |
| MXD4 | 730036 | 0,01889125 | 0,77 |
| IMAGE:687896 PREDICTED: Homo sapiens olfactory receptor, family 7, subfamily E, member 31 pseudogene (OR7E31P), mRNA | 687896 | 0,01910117 | 0,84 |
| PRKG1 | 667587 | 0,01917332 | 0,83 |
| IMAGE:22908 Transcribed locus, weakly similar to NP_775735.1 l(3)mbt-like | 22908 | 0,01931064 | 0,73 |

| Table 17 | OTA | Under | |
|---|---|---|---|
| name | cloid | p-value | fold |
| 4 [Homo sapiens] | | | |
| LOC340073 | 731404 | 0,01951017 | 0,73 |
| KRTHA4 | 325155 | 0,01952696 | 0,67 |
| C14orf140 | 731423 | 0,0201277 | 0,86 |
| IMAGE:46991 Similar to breakpoint cluster region isoform 1 | 46991 | 0,020745 | 0,75 |
| RBBP7 | 727457 | 0,02094568 | 0,85 |
| BCL2L11 | 300194 | 0,02100048 | 0,81 |
| IMAGE:669136 Transcribed locus, weakly similar to NP_060312.1 hypothetical protein FLJ20489 [Homo sapiens] | 669136 | 0,02136336 | 0,87 |
| CTNNA1 | 268972 | 0,02150881 | 0,83 |
| IMAGE:726513 Similar to 40S ribosomal protein S3 | 726513 | 0,02197021 | 0,82 |
| PSIP1 | 667598 | 0,02215201 | 0,78 |
| CAV1 | 133531 | 0,02220907 | 0,76 |
| MESDC2 | 713653 | 0,02242909 | 0,82 |
| LRP1 | 30219 | 0,02320607 | 0,74 |
| KLF4 | 188232 | 0,02332609 | 0,72 |
| FAM36A | 727263 | 0,02337422 | 0,75 |
| IMAGE:53081 | 53081 | 0,02360138 | 0,77 |
| ACTB | 203166 | 0,02394687 | 0,84 |
| EPHA4 | 2237263 | 0,02437397 | 0,77 |
| ISG20 | 740604 | 0,02440903 | 0,77 |
| ARL6IP2 | 743987 | 0,0246722 | 0,82 |
| IMAGE:738945 | 738945 | 0,02503128 | 0,86 |
| IQCA | 669510 | 0,02514619 | 0,78 |
| IFNAR2 | 123950 | 0,0253019 | 0,80 |
| CDC42EP2 | 26651 | 0,02564027 | 0,83 |
| IMAGE:665342 Transcribed locus, strongly similar to NP_065847. | 665342 | 0,02624304 | 0,85 |

| Table 17 | OTA | Under | |
|---|---|---|---|
| name | cloid | p-value | fold |
| 1 semaphorin 6A1; semaphorin 6A-1 [Homo sapiens] | | | |
| PTK2 | 724892 | 0,02711896 | 0,86 |
| NFKB1 | 687782 | 0,02773524 | 0,79 |
| FLJ21918 | 194302 | 0,02783552 | 0,85 |
| RANBP10 | 307025 | 0,02789145 | 0,85 |
| IMAGE:727154 Transcribed locus | 727154 | 0,02978266 | 0,73 |
| CAV1 | 1911930 | 0,03051702 | 0,69 |
| HBA2 | 469647 | 0,0305271 | 0,58 |
| RODH | 471641 | 0,03080742 | 0,59 |
| C20orf19 | 366032 | 0,03094876 | 0,86 |
| NFE2L2 | 29541 | 0,03104685 | 0,81 |
| DALRD3 | 740224 | 0,03140547 | 0,74 |
| HSPB1 | 724150 | 0,03142051 | 0,73 |
| IMAGE:136598 MRNA; cDNA DKFZp686D22106 (from clone DKFZp686D22106) | 136598 | 0,03145463 | 0,84 |
| CES2 | 153667 | 0,03153081 | 0,85 |
| ABCC5 | 212366 | 0,03156946 | 0,83 |
| LATS2 | 666778 | 0,03163588 | 0,78 |
| FMO5 | 364526 | 0,03165625 | 0,78 |
| IMAGE:738966 Transcribed locus | 738966 | 0,03174724 | 0,71 |
| S100A2 | 3659591 | 0,03190236 | 0,83 |
| RPS6KA2 | 22711 | 0,0320225 | 0,79 |
| FABP5 | 1088781 | 0,03220196 | 0,56 |
| IMAGE:744505 | 744505 | 0,03276021 | 0,80 |
| ZFOC1 | 744606 | 0,03369331 | 0,83 |
| IMAGE:135203 Transcribed locus | 135203 | 0,03392561 | 0,86 |
| ADCY3 | 667061 | 0,03422424 | 0,87 |
| FLJ32731 | 365177 | 0,03436124 | 0,81 |
| HIVEP1 | 758037 | 0,03478063 | 0,88 |
| ASGR1 | 25883 | 0,03536376 | 0,73 |
| TM4SF8 | 713647 | 0,03671912 | 0,82 |
| MGC10561 | 740580 | 0,03741305 | 0,84 |
| LOC146909 | 726353 | 0,0379498 | 0,82 |

| Table 17 | OTA | Under | |
|---|---|---|---|
| name | cloid | p-value | fold |
| FBXL16 | 178908 | 0,03911145 | 0,84 |
| RNASE4 | 156720 | 0,03921697 | 0,84 |
| CABIN1 | 1844968 | 0,04247472 | 0,80 |
| IL6ST | 137010 | 0,04386624 | 0,82 |
| IMAGE:136801 | 136801 | 0,04568167 | 0,85 |
| CHRAC1 | 724276 | 0,04626673 | 0,82 |
| RGS6 | 24176 | 0,04643662 | 0,82 |
| TUSC1 | 740381 | 0,04785098 | 0,84 |
| POLI | 137720 | 0,04797886 | 0,78 |
| KRT18 | 725096 | 0,04854209 | 0,80 |
| TPCN1 | 179288 | 0,04857424 | 0,86 |
| TTC17 | 665668 | 0,04953371 | 0,81 |

| Table 18 | OTA | Over | |
|---|---|---|---|
| name | cloid | p-value | fold |
| SERPINB5 | 1662274 | 1,34E-05 | 1,70 |
| KLRD1 | 145696 | 2,28E-05 | 1,61 |
| OBSCN | 730926 | 4,16E-05 | 1,66 |
| SLC36A3 | 731115 | 4,18E-05 | 1,60 |
| IGKV1-5 | 155345 | 4,47E-05 | 1,48 |
| ST5 | 376356 | 1,05E-04 | 1,30 |
| IMAGE:666794 Hypothetical LOC388170 | 666794 | 0,00013 | 1,89 |
| AP2S1 | 739109 | 1,54E-04 | 1,34 |
| KCNQ2 | 179534 | 1,72E-04 | 2,09 |
| GPD1 | 628418 | 1,96E-04 | 1,52 |
| RIN1 | 741406 | 2,48E-04 | 1,86 |
| CHRDL2 | 485872 | 2,57E-04 | 1,73 |
| DCAMKL1 | 232388 | 2,72E-04 | 1,60 |
| SIAT8F | 667110 | 2,77E-04 | 2,03 |
| DC12 | 724895 | 2,88E-04 | 1,92 |
| SIAT8B | 33133 | 3,13E-04 | 1,60 |
| EIF2C4 | 730834 | 3,16E-04 | 1,49 |
| BIC | 743270 | 3,28E-04 | 1,81 |
| ARHGAP10 | 731380 | 3,60E-04 | 1,99 |
| LEF1 | 713913 | 3,70E-04 | 1,71 |
| NTRK2 | 2048801 | 4,50E-04 | 2,29 |
| IMAGE:485104 Transcribed locus | 485104 | 4,70E-04 | 2,48 |
| RNF32 | 731422 | 4,98E-04 | 2,09 |
| SQLE | 124781 | 5,03E-04 | 1,66 |
| IMAGE:731616 MRNA; cDNA DKFZp686A102 (from clone DKFZp686A102) | 731616 | 5,26E-04 | 3,04 |
| SCMH1 | 724014 | 6,07E-04 | 2,30 |
| DEXI | 668186 | 6,32E-04 | 1,36 |
| MGC23909 | 731598 | 6,41E-04 | 2,10 |
| BAP1 | 1012990 | 7,10E-04 | 1,80 |
| ANP32E | 382738 | 7,26E-04 | 2,05 |
| TRIM8 | 714498 | 7,33E-04 | 1,56 |
| IMAGE:745072 Transcribed locus | 745072 | 7,40E-04 | 1,93 |
| PDCD1LG1 | 1942634 | 7,59E-04 | 1,52 |
| MRC2 | 342581 | 7,65E-04 | 2,22 |
| MAP2 | 347503 | 7,82E-04 | 1,76 |
| C6orf216 | 731742 | 7,88E-04 | 1,57 |
| PSEN1 | 724537 | 7,95E-04 | 1,31 |
| HSPC159 | 365045 | 0,00081 | 1,76 |
| IMAGE:738424 | 738424 | 8,19E-04 | 1,34 |
| IMAGE:724556 Transcribed locus | 724556 | 8,66E-04 | 1,50 |
| C21orf84 | 730814 | 8,68E-04 | 2,00 |
| OAS1 | 666703 | 9,26E-04 | 2,23 |
| CASP5 | 341763 | 9,75E-04 | 2,21 |
| S100A16 | 739851 | 1,01E-03 | 1,47 |
| IMAGE:744841 LOC441054 | 744841 | 1,03E-03 | 1,41 |
| ULK4 | 744895 | 1,08E-03 | 1,64 |
| FAM44C | 726894 | 1,12E-03 | 1,33 |
| COX6A1 | 512910 | 1,14E-03 | 1,51 |
| FLJ11184 | 384404 | 1,18E-03 | 1,80 |
| DAB1 | 382621 | 1,21E-03 | 1,54 |
| IMAGE:744074 Transcribed locus | 744074 | 1,27E-03 | 2,29 |
| TIMP2 | 258127 | 1,32E-03 | 1,39 |
| IMAGE:364741 | 364741 | 1,34E-03 | 2,17 |
| PSG3 | 136747 | 1,35E-03 | 1,64 |
| PRKCABP | 1174342 | 1,35E-03 | 1,40 |
| SLC6A1 | 177967 | 1,37E-03 | 1,86 |
| MEOX1 | 760065 | 1,49E-03 | 1,93 |
| IMAGE:177857 Transcribed locus | 177857 | 1,53E-03 | 2,02 |
| MGC19604 | 687468 | 1,55E-03 | 1,36 |
| SAP30L | 723950 | 1,58E-03 | 1,41 |
| BIN1 | 2384812 | 1,64E-03 | 1,53 |
| PTGES | 504646 | 1,64E-03 | 1,43 |
| IMAGE:740105 | 740105 | 1,64E-03 | 1,53 |
| KIAA1754L | 365056 | 0,00167 | 1,75 |
| LOC56251 | 137478 | 1,68E-03 | 1,57 |
| IMAGE:378458 | 378458 | 1,69E-03 | 1,55 |
| GNAI1 | 753215 | 1,73E-03 | 1,44 |
| PARD3 | 724642 | 1,75E-03 | 1,57 |
| AURKC | 731021 | 1,76E-03 | 1,69 |
| IL2RA | 3054031 | 1,76E-03 | 1,69 |
| RPL27A | 178255 | 1,79E-03 | 3,05 |
| F5 | 433155 | 1,87E-03 | 1,65 |
| FOXN4 | 731076 | 1,90E-03 | 1,85 |
| IMAGE:7444 | 744410 | 1,92E-03 | 1,56 |

| Table 18 | OTA | Over | |
|---|---|---|---|
| name | cloid | p-value | fold |
| 10 Transcribed locus | | | |
| IMAGE:743603 | 743603 | 1,93E-03 | 1,66 |
| LOC388284 | 731722 | 1,97E-03 | 1,47 |
| LOC440434 | 742046 | 1,99E-03 | 1,34 |
| IMAGE:727289 Transcribed locus | 727289 | 2,04E-03 | 1,77 |
| SRGAP2 | 714493 | 2,13E-03 | 1,65 |
| SYNPO | 178792 | 2,14E-03 | 1,40 |
| VAV2 | 1581686 | 2,16E-03 | 1,32 |
| S100A12 | 1705397 | 2,17E-03 | 1,46 |
| CTRL | 1308954 | 2,29E-03 | 1,39 |
| C2orf7 | 366243 | 2,30E-03 | 1,28 |
| FLJ25067 | 667252 | 2,36E-03 | 2,56 |
| PPP2R4 | 731590 | 2,38E-03 | 1,43 |
| ME3 | 724238 | 2,43E-03 | 2,15 |
| IMAGE:730924 Transcribed locus | 730924 | 2,50E-03 | 1,36 |
| SLC39A2 | 504596 | 2,62E-03 | 1,38 |
| USP4 | 544818 | 2,64E-03 | 1,80 |
| JUP | 35628 | 2,70E-03 | 1,96 |
| VDAC1 | 486221 | 2,72E-03 | 1,46 |
| TM4SF9 | 381032 | 2,75E-03 | 1,30 |
| KLHL9 | 501527 | 2,76E-03 | 1,31 |
| CLECSF12 | 258865 | 2,77E-03 | 3,51 |
| GPI | 741474 | 2,79E-03 | 1,35 |
| SPATA5L1 | 724884 | 2,81E-03 | 1,72 |
| IGHM | 276658 | 2,82E-03 | 1,56 |
| BCL2L12 | 1186334 | 2,85E-03 | 1,30 |
| MGC20781 | 731227 | 2,96E-03 | 1,49 |
| IMAGE:665649 Transcribed locus | 665649 | 3,08E-03 | 1,66 |
| IMAGE:1859532 | 1859532 | 3,11E-03 | 1,43 |
| DLX5 | 564878 | 0,00313 | 1,98 |
| LILRB5 | 71428 | 3,20E-03 | 1,73 |
| IMAGE:666279 CDNA FLJ30779 fis, clone FEBRA2000815 | 666279 | 3,21E-03 | 2,30 |
| PHC2 | 668146 | 3,23E-03 | 1,82 |
| MPST | 731241 | 3,28E-03 | 1,79 |
| IMAGE:2575 | 257555 | 3,32E-03 | 1,59 |

| Table 18 | OTA | Over | |
|---|---|---|---|
| name | cloid | p-value | fold |
| 55 | | | |
| KIAA1463 | 381118 | 3,39E-03 | 1,44 |
| ATP2B4 | 665814 | 3,43E-03 | 1,68 |
| MFAP3L | 726821 | 3,43E-03 | 1,35 |
| OSR1 | 364686 | 3,58E-03 | 1,84 |
| PRDX2 | 208439 | 3,74E-03 | 1,33 |
| HP | 82687 | 3,76E-03 | 1,46 |
| COX5A | 1085884 | 3,78E-03 | 1,67 |
| QIL1 | 727296 | 3,79E-03 | 1,32 |
| PCGF4 | 740457 | 3,90E-03 | 1,75 |
| IMAGE:667527 | 667527 | 3,94E-03 | 1,96 |
| LHB | 1671903 | 4,01E-03 | 1,60 |
| PLCG2 | 201467 | 4,13E-03 | 1,56 |
| TTC7B | 135379 | 4,23E-03 | 1,58 |
| FKBP6 | 795736 | 4,28E-03 | 1,55 |
| TFF1 | 1075949 | 4,28E-03 | 2,13 |
| H17 | 739432 | 4,31E-03 | 1,32 |
| IMAGE:363955 Transcribed locus | 363955 | 4,31E-03 | 1,67 |
| IMAGE:213529 Transcribed locus | 213529 | 4,47E-03 | 1,62 |
| SIGLEC7 | 743331 | 4,53E-03 | 1,71 |
| ANXA11 | 137238 | 4,53E-03 | 1,26 |
| IMAGE:743619 | 743619 | 4,59E-03 | 1,60 |
| ZNF258 | 1241974 | 4,73E-03 | 1,22 |
| COX6B1 | 632026 | 5,11E-03 | 1,39 |
| S100A16 | 730699 | 5,32E-03 | 1,34 |
| KIAA0759 | 743451 | 5,36E-03 | 2,52 |
| TRIP12 | 665820 | 5,38E-03 | 1,70 |
| MDH1 | 725188 | 5,49E-03 | 1,31 |
| MUTED | 731202 | 5,62E-03 | 1,98 |
| IMAGE:379937 | 379937 | 5,72E-03 | 1,80 |
| WIPI49 | 487148 | 5,74E-03 | 1,56 |
| BAK1 | 1288183 | 5,81E-03 | 2,02 |
| CREBBP | 172996 | 5,83E-03 | 1,75 |
| SIAT8C | 382069 | 5,88E-03 | 1,52 |
| HMG20A | 731277 | 5,90E-03 | 1,94 |
| VTN | 230126 | 5,96E-03 | 1,78 |
| NME7 | 743982 | 6,01E-03 | 2,37 |
| IMAGE:743579 | 743579 | 6,02E-03 | 2,02 |
| SDHA | 40304 | 6,05E-03 | 1,48 |
| SLC2A4RG | 376983 | 6,10E-03 | 1,42 |
| CTXN1 | 179266 | 6,29E-03 | 1,40 |
| LOC113655 | 40134 | 6,31E-03 | 1,36 |

| Table 18 | OTA | Over | |
|---|---|---|---|
| name | cloid | p-value | fold |
| BAK1 | 235938 | 6,36E-03 | 1,77 |
| IMAGE:731689 Transcribed locus | 731689 | 6,44E-03 | 1,30 |
| SYCP2 | 136863 | 6,55E-03 | 2,05 |
| C20orf18 | 665433 | 6,64E-03 | 2,01 |
| IMAGE:727067 Similar to hypothetical protein SB153 isoform 1 | 727067 | 6,69E-03 | 1,57 |
| IMAGE:743078 | 743078 | 6,71E-03 | 1,39 |
| ETV4 | 809959 | 6,71E-03 | 1,50 |
| GALNT7 | 381854 | 6,94E-03 | 1,65 |
| TOSO | 813174 | 7,04E-03 | 1,58 |
| BMP5 | 1846326 | 0,00714 | 1,63 |
| MAK | 382002 | 7,18E-03 | 1,46 |
| JAK1 | 2030501 | 7,23E-03 | 1,71 |
| ATP11B | 666334 | 7,37E-03 | 2,27 |
| HRK | 767779 | 7,39E-03 | 1,99 |
| MCC | 731305 | 7,50E-03 | 2,48 |
| RABL5 | 471829 | 0,00755 | 1,27 |
| MAPRE2 | 383868 | 7,81E-03 | 2,35 |
| COX7B | 566862 | 8,32E-03 | 1,55 |
| IMAGE:731758 Transcribed locus, weakly similar to NP_081070.1 kiaa-iso protein homolog; Band 47B [Mus musculus] | 731758 | 8,39E-03 | 1,72 |
| MATN1 | 1624260 | 8,41E-03 | 1,44 |
| CD33 | 1917430 | 8,43E-03 | 1,72 |
| PPIL4 | 364777 | 8,46E-03 | 2,28 |
| DPF2 | 743519 | 0,00849 | 1,49 |
| FSHPRH1 | 667355 | 8,90E-03 | 1,40 |
| ZNF319 | 364729 | 8,93E-03 | 2,52 |
| RHOD | 591907 | 9,04E-03 | 1,29 |
| MESP1 | 25865 | 0,00912 | 1,20 |
| CASP10 | 241481 | 9,15E-03 | 1,98 |
| ALDH3A1 | 525221 | 9,76E-03 | 1,81 |
| KIAA0427 | 127507 | 9,77E-03 | 1,35 |
| GCAT | 307094 | 9,92E-03 | 1,26 |
| LOC339903 | 135352 | 9,92E-03 | 1,52 |
| C22orf16 | 236119 | 9,93E-03 | 1,46 |

| Table 18 | OTA | Over | |
|---|---|---|---|
| name | cloid | p-value | fold |
| NDUFS3 | 743811 | 9,99E-03 | 1,24 |
| GNAS | 382791 | 1,00E-02 | 1,90 |
| IMAGE:742061 | 742061 | 1,00E-02 | 1,60 |
| MAPK3 | 323438 | 1,00E-02 | 1,62 |
| FLJ21159 | 251147 | 1,02E-02 | 1,27 |
| APIN | 364706 | 1,02E-02 | 1,83 |
| LRRIQ2 | 487152 | 1,07E-02 | 1,54 |
| MRPL45 | 364717 | 1,11E-02 | 1,66 |
| IMAGE:136686 | 136686 | 1,14E-02 | 1,36 |
| LOC284669 | 364885 | 1,18E-02 | 1,56 |
| ATF4 | 178348 | 1,22E-02 | 1,34 |
| GRIK2 | 731363 | 1,24E-02 | 1,32 |
| CLIC4 | 666085 | 1,25E-02 | 1,41 |
| PLCG1 | 1174287 | 1,28E-02 | 1,79 |
| KIF27 | 667657 | 1,29E-02 | 1,35 |
| CXCL13 | 347362 | 1,30E-02 | 1,37 |
| DDX54 | 743268 | 1,34E-02 | 1,35 |
| GJA5 | 196338 | 1,36E-02 | 1,68 |
| C6orf176 | 173200 | 1,37E-02 | 1,39 |
| ALDH1A3 | 486189 | 1,37E-02 | 1,99 |
| IMAGE:665403 Transcribed locus | 665403 | 1,37E-02 | 1,52 |
| CCDC6 | 487848 | 1,38E-02 | 1,40 |
| IMAGE:745512 | 745512 | 1,43E-02 | 1,38 |
| GLRX2 | 731044 | 1,45E-02 | 1,39 |
| NIFIE14 | 472160 | 1,51E-02 | 1,26 |
| C10orf99 | 713624 | 1,53E-02 | 1,22 |
| C15orf29 | 177775 | 1,53E-02 | 1,46 |
| SEPW1 | 173628 | 1,60E-02 | 2,05 |
| UBE2D2 | 594655 | 1,63E-02 | 1,27 |
| IMAGE:744616 DNA damage repair and recombination protein RAD52 pseudogene | 744616 | 1,64E-02 | 1,40 |
| IMAGE:364932 CDNA FLJ35491 fis, clone SMINT2008625, moderately similar to GLYCINE CLEAVAGE SYSTEM H | 364932 | 1,65E-02 | 1,44 |

| Table 18 | OTA | Over | |
|---|---|---|---|
| name | cloid | p-value | fold |
| PROTEIN PRECURSOR | | | |
| PRKCDBP | 157847 | 1,67E-02 | 1,44 |
| IMAGE:744899 Homo sapiens, clone IMAGE:4837072, mRNA | 744899 | 1,67E-02 | 1,56 |
| C4orf16 | 681890 | 1,67E-02 | 1,32 |
| IMAGE:745133 Transcribed locus | 745133 | 1,70E-02 | 1,21 |
| EIF4EBP1 | 713608 | 1,71E-02 | 1,38 |
| ATP5G3 | 740090 | 1,74E-02 | 1,37 |
| MAD2L2 | 727178 | 1,74E-02 | 1,35 |
| DMD | 743394 | 1,75E-02 | 1,74 |
| MRPS11 | 471574 | 1,82E-02 | 1,80 |
| FBXO46 | 471664 | 1,82E-02 | 1,61 |
| RASAL2 | 486304 | 1,86E-02 | 1,95 |
| C6orf118 | 731745 | 1,88E-02 | 1,34 |
| SNX8 | 366585 | 1,96E-02 | 1,29 |
| ABR | 1012903 | 1,97E-02 | 1,35 |
| IMAGE:24587 | 24587 | 2,03E-02 | 1,31 |
| IMAGE:742837 Transcribed locus | 742837 | 2,03E-02 | 1,58 |
| ELK4 | 236155 | 2,07E-02 | 1,27 |
| NME1 | 726600 | 2,13E-02 | 1,48 |
| GAPD | 152847 | 2,13E-02 | 1,54 |
| OR7E38P | 486540 | 2,17E-02 | 1,22 |
| MGC29784 | 731019 | 2,22E-02 | 1,19 |
| PIP | 985457 | 2,28E-02 | 1,26 |
| IMAGE:383718 | 383718 | 2,36E-02 | 1,51 |
| AD023 | 731645 | 2,37E-02 | 1,29 |
| PML | 724554 | 2,45E-02 | 1,17 |
| UQCRC1 | 714414 | 2,46E-02 | 1,42 |
| CBL | 1578721 | 2,49E-02 | 1,30 |
| CGI-128 | 786662 | 2,51E-02 | 1,24 |
| IMAGE:383966 | 383966 | 0,02518 | 1,31 |
| CIRH1A | 261500 | 2,55E-02 | 1,24 |
| DUSP3 | 119772 | 2,65E-02 | 1,28 |
| PDXP | 743182 | 2,81E-02 | 1,28 |
| DGKQ | 366233 | 2,90E-02 | 1,19 |
| KIAA1277 | 364822 | 2,94E-02 | 1,50 |
| AMD1 | 132752 | 2,95E-02 | 1,17 |
| LHFPL2 | 364356 | 2,98E-02 | 1,46 |

| Table 18 | OTA | Over | |
|---|---|---|---|
| name | cloid | p-value | fold |
| PCSK6 | 31924 | 2,99E-02 | 2,16 |
| KIAA1280 | 366085 | 2,99E-02 | 1,23 |
| SLIT1 | 38403 | 3,10E-02 | 1,26 |
| FLJ22471 | 668510 | 3,10E-02 | 2,44 |
| CD8B1 | 1743279 | 3,27E-02 | 1,19 |
| IMAGE:743290 Transcribed locus | 743290 | 3,35E-02 | 2,01 |
| RUNX1 | 263251 | 3,39E-02 | 1,42 |
| SYP | 30471 | 3,40E-02 | 2,11 |
| PPAP2C | 486447 | 3,41E-02 | 1,33 |
| PINK1 | 729929 | 3,44E-02 | 1,30 |
| TYMS | 2242054 | 3,49E-02 | 1,22 |
| IMAGE:382521 | 382521 | 3,52E-02 | 1,54 |
| DIO1 | 296702 | 3,53E-02 | 1,45 |
| C1QBP | 173371 | 3,57E-02 | 1,52 |
| PSMD11 | 383945 | 3,57E-02 | 1,54 |
| LAD1 | 121551 | 3,59E-02 | 1,17 |
| MIF | 179133 | 3,59E-02 | 1,26 |
| CCL28 | 136919 | 3,61E-02 | 1,21 |
| PTPN4 | 666367 | 3,61E-02 | 1,40 |
| DUSP24 | 740158 | 3,70E-02 | 1,57 |
| IMAGE:682585 | 682585 | 3,86E-02 | 1,27 |
| SPI1 | 1285305 | 3,92E-02 | 1,34 |
| DECR2 | 730942 | 3,93E-02 | 1,39 |
| ABCA8 | 284828 | 3,95E-02 | 1,45 |
| HSPC072 | 668329 | 0,04017 | 1,73 |
| SAFB | 42280 | 4,07E-02 | 2,48 |
| IMAGE:136121 CDNA FLJ10247 fis, clone HEMBB1000705 | 136121 | 4,07E-02 | 1,55 |
| DOK5 | 25664 | 4,11E-02 | 1,56 |
| TGFB3 | 1561035 | 4,20E-02 | 1,32 |
| IRAK1 | 379200 | 4,23E-02 | 1,16 |
| EPRS | 669549 | 0,04334 | 1,16 |
| FLJ23577 | 668452 | 4,35E-02 | 2,08 |
| HTATIP2 | 726618 | 4,36E-02 | 1,18 |
| PANK1 | 256177 | 4,38E-02 | 1,20 |
| DCXR | 724596 | 4,46E-02 | 1,22 |
| KCNK4 | 743016 | 4,52E-02 | 1,36 |
| ITGA5 | 135671 | 4,55E-02 | 1,28 |
| SYT7 | 177827 | 0,04549 | 1,53 |
| NDUFA7 | 364469 | 4,59E-02 | 1,21 |
| VIL2 | 124701 | 4,66E-02 | 1,26 |
| FLJ35630 | 485652 | 4,66E-02 | 1,21 |
| MLPH | 667259 | 4,73E-02 | 1,76 |

| Table 18 | OTA | Over | |
|---|---|---|---|
| **name** | **cloid** | **p-value** | **fold** |
| RAB8A | 712118 | 4,81E-02 | 1,31 |
| SFN | 346610 | 4,83E-02 | 1,16 |
| FLJ12517 | 743220 | 4,86E-02 | 1,14 |
| CLGN | 1049033 | 0,04934 | 1,66 |

| Table 19 | | OTA-atypical | Under |
|---|---|---|---|
| name | cloid | p-value | fold |
| ZNF76 | 745003 | 7,8753E-23 | 0,60 |
| POLR2A | 740130 | 3,2874E-20 | 0,58 |
| RODH | 471641 | 9,42E-18 | 0,13 |
| LOC126669 | 682088 | 1,5036E-17 | 0,59 |
| LOC340073 | 731404 | 5,6235E-17 | 0,47 |
| IMAGE:2254555 | 2254555 | 5,0996E-16 | 0,58 |
| CDKN1C | 2381770 | 1,0691E-13 | 0,57 |
| CDH1 | 214008 | 2,0729E-13 | 0,38 |
| CLDN7 | 300268 | 3,277E-13 | 0,64 |
| ZBTB16 | 2467442 | 1,2466E-10 | 0,40 |
| CD63 | 125552 | 3,0515E-09 | 0,72 |
| C21orf55 | 666517 | 8,6603E-09 | 0,65 |
| RNF123 | 366159 | 1,3739E-08 | 0,76 |
| IMAGE:740927 | 740927 | 2,5316E-08 | 0,57 |
| CFH | 665784 | 4,6026E-08 | 0,37 |
| BAT3 | 24392 | 3,2661E-07 | 0,73 |
| RBBP7 | 727457 | 5,0481E-07 | 0,87 |
| GATA3 | 148796 | 5,6774E-07 | 0,55 |
| MGP | 590264 | 5,8207E-07 | 0,33 |
| APOL1 | 665632 | 8,5144E-07 | 0,40 |
| CXCL14 | 345034 | 1,0689E-06 | 0,15 |
| CCL5 | 840753 | 3,0169E-06 | 0,44 |
| CAV1 | 309645 | 3,7112E-06 | 0,25 |
| TIE1 | 743043 | 4,493E-06 | 0,71 |
| LOC286076 | 731275 | 5,0989E-06 | 0,67 |
| ESRRBL1 | 51232 | 9,4953E-06 | 0,69 |
| SOSTDC1 | 667048 | 1,0076E-05 | 0,81 |
| APOD | 838611 | 1,4867E-05 | 0,25 |
| GPNMB | 773330 | 1,5722E-05 | 0,26 |
| HMGB2 | 884365 | 2,4273E-05 | 0,50 |
| TP53I11 | 667514 | 3,3387E-05 | 0,48 |
| DHRS6 | 364412 | 7,2289E-05 | 0,71 |
| PLAC9 | 366115 | 7,6205E-05 | 0,46 |
| CAPN3 | 757248 | 0,00010028 | 0,61 |
| FRZB | 146049 | 0,00010878 | 0,56 |
| COL1A1 | 140347 | 0,00011911 | 0,26 |
| BCL2 | 342181 | 0,00013964 | 0,65 |
| IMAGE:727123 Transcribed locus, strongly similar to NP_004491.1 heterogeneous nuclear ribonucleoprotein C isoform b; nuclear ribonucleoprotein particle C2 protein; nuclear ribonucleoprotein particle C1 protein [Homo sapiens] | 727123 | 0,00016327 | 0,75 |
| LRP1 | 30219 | 0,00018822 | 0,52 |
| MMP2 | 155839 | 0,00020213 | 0,42 |
| NUP214 | 382612 | 0,0002167 | 0,53 |
| SPON2 | 723923 | 0,00025027 | 0,73 |
| IGSF10 | 682276 | 0,00026272 | 0,65 |
| TBC1D22A | 682479 | 0,00027374 | 0,73 |
| DKFZp434M202 | 743118 | 0,00029492 | 0,69 |
| C10orf116 | 740941 | 0,00029723 | 0,40 |
| CD5 | 356841 | 0,00035219 | 0,75 |
| IMAGE:53081 | 53081 | 0,00035776 | 0,78 |
| TSC | 745490 | 0,0003911 | 0,42 |
| ANKRD28 | 687381 | 0,00040418 | 0,55 |
| CCND1 | 324079 | 0,00045244 | 0,69 |
| IMAGE:173818 Transcribed locus | 173818 | 0,00050225 | 0,81 |
| MGAT3 | 731060 | 0,00051388 | 0,57 |
| NCB5OR | 743367 | 0,00053407 | 0,55 |
| SPTLC2 | 365000 | 0,00057248 | 0,79 |
| PRKCQ | 669149 | 0,00057256 | 0,53 |
| SFRP4 | 285693 | 0,00057595 | 0,48 |
| IMAGE:731398 | 731398 | 0,00067333 | 0,67 |
| PPP3CC | 110481 | 0,00069215 | 0,74 |
| IQCF3 | 726578 | 0,00080815 | 0,68 |
| ARHGEF | 687990 | 0,00086388 | 0,66 |

| Table 19 | | OTA-atypical | Under |
|---|---|---|---|
| name | cloid | p-value | fold |
| 6 | | | |
| TIAM1 | 23612 | 0,00089109 | 0,31 |
| IMAGE:723752 | 723752 | 0,00092229 | 0,68 |
| AQP11 | 713831 | 0,00092762 | 0,47 |
| CD3E | 1536968 | 0,00093072 | 0,45 |
| NT5C2 | 725076 | 0,00099636 | 0,57 |
| LYPLA3 | 471702 | 0,00102036 | 0,58 |
| S100A10 | 119939 | 0,00120361 | 0,56 |
| SEPT-05 | 448163 | 0,0012145 | 0,26 |
| GLT8D2 | 365271 | 0,00131886 | 0,39 |
| HDAC9 | 738506 | 0,00143075 | 0,72 |
| BDKRB2 | 665674 | 0,00144781 | 0,51 |
| DF | 666128 | 0,00145035 | 0,26 |
| NFE2L2 | 29541 | 0,00153006 | 0,77 |
| FXYD1 | 204686 | 0,00159585 | 0,67 |
| IMAGE:731665 | 731665 | 0,00167237 | 0,54 |
| RANBP10 | 682251 | 0,00172358 | 0,72 |
| LOC284371 | 724126 | 0,00245517 | 0,71 |
| IMAGE:22908 Transcribed locus, weakly similar to NP_775735.1 l(3)mbt-like 4 [Homo sapiens] | 22908 | 0,00250266 | 0,76 |
| PLA2G2A | 152802 | 0,00253136 | 0,70 |
| CASP4 | 356960 | 0,00285061 | 0,83 |
| EVI5L | 173119 | 0,00338218 | 0,63 |
| APOL3 | 366289 | 0,00370487 | 0,58 |
| AMPD2 | 669141 | 0,00384401 | 0,82 |
| PLXNB2 | 1420676 | 0,00409831 | 0,58 |
| RDH5 | 682109 | 0,00428632 | 0,62 |
| ECM1 | 301122 | 0,0044458 | 0,27 |
| CD47 | 357442 | 0,00479452 | 0,62 |
| STYX | 174879 | 0,00490228 | 0,58 |
| S100A4 | 868577 | 0,00504892 | 0,35 |
| ASGR1 | 25883 | 0,00527851 | 0,44 |
| SLAMF8 | 288807 | 0,00568981 | 0,70 |
| PP784 | 366089 | 0,00582582 | 0,66 |
| IMAGE:471852 CDNA FLJ4006 | 471852 | 0,005834 | 0,73 |

| Table 19 | | OTA-atypical | Under |
|---|---|---|---|
| name | cloid | p-value | fold |
| 1 fis, clone TESOP2000083 | | | |
| TIMP1 | 162246 | 0,00610248 | 0,40 |
| LGALS3BP | 742100 | 0,0066389 | 0,36 |
| IMAGE:743722 | 743722 | 0,00674466 | 0,66 |
| MGC40214 | 666455 | 0,00721803 | 0,78 |
| CITED1 | 265558 | 0,00748256 | 0,43 |
| FLJ14351 | 744943 | 0,00769555 | 0,30 |
| IMAGE:379279 | 379279 | 0,00786189 | 0,64 |
| IMAGE:668924 | 668924 | 0,00797669 | 0,71 |
| ACTB | 203166 | 0,00896929 | 0,86 |
| FLJ21918 | 194302 | 0,00901426 | 0,67 |
| SNRP70 | 729971 | 0,00908157 | 0,56 |
| C4A | 724366 | 0,01003098 | 0,46 |
| IMAGE:682311 LOC440722 | 682311 | 0,01031247 | 0,74 |
| ITPR1 | 667348 | 0,0106412 | 0,59 |
| SIAT4B | 365877 | 0,01066903 | 0,62 |
| C14orf140 | 731423 | 0,01082785 | 0,77 |
| C7orf27 | 713859 | 0,0108452 | 0,76 |
| SMARCA5 | 730037 | 0,01143683 | 0,65 |
| FOXM1 | 23641 | 0,01240416 | 0,57 |
| FLJ20674 | 713837 | 0,01347485 | 0,86 |
| MTUS1 | 376597 | 0,01348587 | 0,64 |
| IMAGE:135125 | 135125 | 0,01360246 | 0,58 |
| SGSH | 669263 | 0,01424674 | 0,53 |
| IFNAR2 | 123950 | 0,01445371 | 0,83 |
| EPHB6 | 172982 | 0,0150956 | 0,32 |
| CAV1 | 1911930 | 0,0153774 | 0,38 |
| CAPNS1 | 152655 | 0,01547068 | 0,73 |
| RUNX3 | 122874 | 0,0160785 | 0,64 |
| GADD45GIP1 | 1084386 | 0,0161436 | 0,73 |
| GJA1 | 839101 | 0,01616925 | 0,53 |
| IMAGE:251427 | 251427 | 0,01673194 | 0,77 |
| FBXO21 | 666168 | 0,01684513 | 0,65 |
| SLC9A5 | 112819 | 0,01735236 | 0,69 |
| TSHR | 565317 | 0,01748004 | 0,48 |

| Table 19 | | OTA-atypical | Under |
|---|---|---|---|
| name | cloid | p-value | fold |
| SPDEF | 1188588 | 0,01788258 | 0,85 |
| KRT17 | 366889 | 0,01859126 | 0,56 |
| IMAGE:743485 Similar to RIKEN cDNA E130306M17 gene | 743485 | 0,0187952 | 0,69 |
| HN1 | 471568 | 0,01882732 | 0,70 |
| IMAGE:744505 | 744505 | 0,02002197 | 0,73 |
| IQCA | 669510 | 0,02005822 | 0,58 |
| LOC286272 | 667361 | 0,02077784 | 0,71 |
| PDLIM1 | 135689 | 0,02118121 | 0,45 |
| LOC91526 | 666564 | 0,0217601 | 0,76 |
| IMAGE:738332 Transcribed locus | 738332 | 0,02220518 | 0,29 |
| IMAGE:23765 | 23765 | 0,02343381 | 0,68 |
| NYREN18 | 668584 | 0,02345468 | 0,80 |
| PIGV | 669319 | 0,02466844 | 0,79 |
| PLA2G6 | 744087 | 0,02496728 | 0,48 |
| POU2F2 | 188393 | 0,02538555 | 0,68 |
| PRKG1 | 667587 | 0,02549526 | 0,75 |
| SOD3 | 795309 | 0,02637889 | 0,38 |
| DAXX | 292042 | 0,02655992 | 0,75 |
| TM4SF9 | 812967 | 0,02671422 | 0,50 |
| YTHDF3 | 135450 | 0,02725943 | 0,66 |
| CCND3 | 327182 | 0,02743722 | 0,87 |
| IMAGE:666946 | 666946 | 0,02807361 | 0,74 |
| C20orf19 | 366032 | 0,02845889 | 0,64 |
| CCL19 | 1707527 | 0,02868686 | 0,38 |
| SNTB2 | 667310 | 0,02953683 | 0,87 |
| TOE1 | 52897 | 0,02974582 | 0,65 |
| GPC3 | 137131 | 0,029841 | 0,65 |
| PBXIP1 | 366042 | 0,0300236 | 0,49 |
| KIAA1196 | 738938 | 0,03031142 | 0,81 |
| IMAGE:725726 Hypothetical gene supported by AK093801 | 725726 | 0,03126401 | 0,58 |

| Table 19 | | OTA-atypical | Under |
|---|---|---|---|
| name | cloid | p-value | fold |
| RIPK3 | 667313 | 0,03128138 | 0,63 |
| IMAGE:665392 Hypothetical LOC388790 | 665392 | 0,03169622 | 0,61 |
| BMP5 | 1846326 | 0,03227381 | 0,72 |
| TCEA2 | 730149 | 0,03292087 | 0,76 |
| ZFOC1 | 744606 | 0,0347902 | 0,67 |
| CKLFSF3 | 489249 | 0,03501322 | 0,71 |
| SENP6 | 739237 | 0,0350721 | 0,62 |
| LEMD1 | 731047 | 0,03610772 | 0,58 |
| PSPC1 | 687541 | 0,03646334 | 0,90 |
| NPFF | 365161 | 0,03913586 | 0,58 |
| THAP3 | 687800 | 0,0399181 | 0,58 |
| CCNE1 | 68950 | 0,04140347 | 0,50 |
| IMMP2L | 136260 | 0,04147879 | 0,86 |
| FOXO3A | 109142 | 0,04212529 | 0,61 |
| BMP4 | 69166 | 0,04398031 | 0,63 |
| CCNE1 | 357807 | 0,04398241 | 0,64 |
| SULT1E1 | 207926 | 0,04421397 | 0,74 |
| KLRB1 | 2091591 | 0,04434553 | 0,72 |
| FGF4 | 1550616 | 0,04512349 | 0,59 |
| TUSC1 | 740381 | 0,04739201 | 0,67 |
| IMAGE:731714 CDNA FLJ26188 fis, clone ADG04821 | 731714 | 0,04824836 | 0,59 |
| FLJ10707 | 364575 | 0,04833655 | 0,83 |
| TRAF5 | 1286238 | 0,04846001 | 0,70 |
| FMO5 | 364526 | 0,04864895 | 0,72 |
| FOXO1A | 151247 | 0,0489662 | 0,57 |

| Table 20 | OTA-atypical | Over | |
|---|---|---|---|
| **name** | **cloid** | **p-value** | **fold** |
| IMAGE:7245 56 Transcribed locus | 724556 | 5,95E-07 | 1,25 |
| HSPC072 | 668329 | 2,07E-04 | 1,40 |
| C6orf176 | 173200 | 1,24E-03 | 1,75 |
| KNS2 | 174654 | 3,56E-03 | 1,13 |
| MGC20781 | 731227 | 4,21 E-03 | 1,22 |
| MATN1 | 1624260 | 1,44E-02 | 1,52 |
| IMAGE:7430 78 | 743078 | 1,47E-02 | 1,17 |
| FBXW5 | 739576 | 2,20E-02 | 1,28 |
| IL2RA | 3054031 | 3,15E-02 | 1,32 |
| DUSP3 | 119772 | 4,06E-02 | 1,66 |

| Table 21 | OTC | Under | |
|---|---|---|---|
| name | cloid | p-value | fold |
| IMAGE:727123 Transcribed locus, strongly similar to NP_004491.1 heterogeneous nuclear ribonucleoprotein C isoform b; nuclear ribonucleoprotein particle C2 protein; nuclear ribonucleoprotein particle C1 protein [Homo sapiens] | 727123 | 2,7617E-23 | 0,68 |
| C4A | 724366 | 6,3678E-20 | 0,11 |
| APOL3 | 366289 | 4,4335E-16 | 0,39 |
| RODH | 471641 | 3,0605E-14 | 0,24 |
| TIMP3 | 501476 | 3,8968E-14 | 0,35 |
| FLJ14351 | 744943 | 1,8705E-13 | 0,12 |
| EPHA4 | 2237263 | 3,9675E-13 | 0,44 |
| MMP2 | 155839 | 5,2372E-13 | 0,32 |
| COL1A1 | 140347 | 1,232E-12 | 0,22 |
| IMP-2 | 743774 | 1,8908E-12 | 0,27 |
| IMAGE:1716286 | 1716286 | 6,4804E-12 | 0,26 |
| GLT8D2 | 365271 | 2,3704E-11 | 0,28 |
| SPINL | 236399 | 3,4967E-11 | 0,33 |
| C14orf140 | 731423 | 4,4437E-11 | 0,78 |
| LOC120224 | 252291 | 4,4649E-11 | 0,60 |
| TM4SF9 | 812967 | 5,8913E-11 | 0,51 |
| MGC99813 | 739097 | 1,1187E-10 | 0,20 |
| LYPLA3 | 471702 | 1,5636E-10 | 0,70 |
| PLXNB2 | 1420676 | 1,9854E-10 | 0,34 |
| CRABP1 | 739193 | 2,1378E-10 | 0,14 |
| PREB | 740347 | 2,692E-10 | 0,23 |
| FRMD4B | 669564 | 8,1194E-10 | 0,51 |
| BAZ2B | 609631 | 8,1218E-10 | 0,63 |
| IMAGE:731751 Transcribed locus | 731751 | 1,6927E-09 | 0,48 |
| IMAGE:666671 Transcribed locus | 666671 | 1,1003E-08 | 0,58 |
| TSC | 745490 | 1,8198E-08 | 0,27 |
| SFRP4 | 285693 | 2,4749E-08 | 0,35 |
| MRC2 | 235882 | 1,2867E-07 | 0,39 |
| CICE | 725791 | 1,6529E-07 | 0,49 |
| TIAM1 | 23612 | 1,8313E-07 | 0,35 |
| CXCL14 | 345034 | 1,8994E-07 | 0,08 |
| NBL1 | 503874 | 1,9736E-07 | 0,24 |
| APOD | 838611 | 3,0261E-07 | 0,18 |
| MT1F | 81911 | 3,9725E-07 | 0,18 |
| HBA2 | 469647 | 4,0621E-07 | 0,22 |
| TP53I11 | 667514 | 4,8536E-07 | 0,46 |
| INHBB | 730012 | 6,3101E-07 | 0,35 |
| SPATA12 | 730300 | 6,4449E-07 | 0,60 |
| C10orf116 | 740941 | 1,0112E-06 | 0,25 |
| HOXB6 | 738358 | 1,8287E-06 | 0,76 |
| GPR124 | 486493 | 2,4789E-06 | 0,50 |
| URB | 809719 | 2,7159E-06 | 0,54 |
| LAPTM4A | 726684 | 3,1145E-06 | 0,47 |
| IMAGE:687667 CDNA clone IMAGE:4811759, partial cds | 687667 | 3,4863E-06 | 0,30 |
| TIMP1 | 162246 | 3,5649E-06 | 0,26 |
| TPM2 | 740620 | 3,6412E-06 | 0,27 |
| SENP6 | 366436 | 5,3738E-06 | 0,55 |
| SALL4 | 726454 | 5,8869E-06 | 0,35 |
| HBB | 469549 | 8,147E-06 | 0,25 |
| ETF1 | 146976 | 9,4951E-06 | 0,44 |
| CFH | 665784 | 1,0597E-05 | 0,40 |
| RFX2 | 731738 | 1,0616E-05 | 0,48 |
| HNRPA3 | 365349 | 1,0734E-05 | 0,65 |
| MGC5395 | 238840 | 1,0969E-05 | 0,40 |
| C4A | 491004 | 1,1538E-05 | 0,33 |
| RUNX3 | 122874 | 1,5173E-05 | 0,60 |
| IMAGE:46991 Similar to breakpoint cluster region isoform 1 | 46991 | 1,5437E-05 | 0,44 |
| CDH16 | 726763 | 1,6824E-05 | 0,33 |
| MT1F | 78353 | 1,9038E-05 | 0,15 |
| IMAGE:738896 | 738896 | 1,9575E-05 | 0,15 |
| FBXL16 | 178908 | 2,0945E-05 | 0,80 |
| NFIB | 416959 | 2,1672E-05 | 0,69 |
| ZNF251 | 486401 | 2,9225E-05 | 0,25 |
| APOL1 | 665632 | 2,923E-05 | 0,45 |
| RP4-622L5 | 742590 | 2,9406E-05 | 0,57 |
| GIMAP5 | 180259 | 3,1108E-05 | 0,45 |
| MGC40214 | 666455 | 3,4186E-05 | 0,61 |

| Table 21 | OTC | Under | |
|---|---|---|---|
| name | cloid | p-value | fold |
| RPS6KA2 | 22711 | 3,7532E-05 | 0,50 |
| BDKRB2 | 665674 | 3,7744E-05 | 0,36 |
| GATA3 | 148796 | 3,7841E-05 | 0,37 |
| CERKL | 489326 | 3,7966E-05 | 0,55 |
| ASGR1 | 25883 | 3,8987E-05 | 0,45 |
| GNAI2 | 724306 | 4,1649E-05 | 0,44 |
| ZNF76 | 745003 | 4,1776E-05 | 0,43 |
| CLDN1 | 594279 | 4,3619E-05 | 0,25 |
| IMAGE:342256 | 342256 | 5,2243E-05 | 0,15 |
| SIN3A | 26455 | 5,6921E-05 | 0,61 |
| CKLFSF4 | 143759 | 5,7456E-05 | 0,79 |
| KIAA0515 | 687852 | 7,835E-05 | 0,52 |
| LASP1 | 592598 | 8,0253E-05 | 0,35 |
| MGC17299 | 713422 | 8,7659E-05 | 0,55 |
| CaMKIINalpha | 173820 | 9,6232E-05 | 0,49 |
| VPS13D | 136560 | 0,00011233 | 0,58 |
| GABRE | 209137 | 0,00011566 | 0,56 |
| TNFSF12 | 136361 | 0,00011734 | 0,64 |
| IMAGE:743205 | 743205 | 0,00011953 | 0,45 |
| PBXIP1 | 366042 | 0,00012575 | 0,41 |
| ARID1B | 665538 | 0,00013937 | 0,88 |
| SYN3 | 727056 | 0,00015868 | 0,41 |
| ABCB1 | 1837488 | 0,00015965 | 0,54 |
| DNALI1 | 782688 | 0,00017057 | 0,23 |
| PLA2G6 | 744087 | 0,00017386 | 0,35 |
| SLC6A8 | 725877 | 0,00018797 | 0,26 |
| PIK3CA | 2238108 | 0,0001957 | 0,37 |
| PPP1CA | 257259 | 0,00019642 | 0,71 |
| LRP5 | 725836 | 0,00020223 | 0,55 |
| PDE8A | 666154 | 0,00021562 | 0,62 |
| FLJ27523 | 743246 | 0,00022705 | 0,42 |
| RAP1GA1 | 971276 | 0,00023382 | 0,61 |
| SEPT-05 | 448163 | 0,000238 | 0,20 |
| DCN | 666410 | 0,00024122 | 0,29 |
| POLI | 137720 | 0,00024263 | 0,46 |
| CABIN1 | 1844968 | 0,0002434 | 0,40 |
| TNFRSF1B | 73703 | 0,00024434 | 0,58 |
| CD34 | 770858 | 0,00024685 | 0,51 |
| LRP2 | 2055272 | 0,00025885 | 0,40 |
| NOG | 487474 | 0,00027732 | 0,72 |
| WDR5 | 731023 | 0,00030289 | 0,75 |
| ITPR1 | 667348 | 0,00033483 | 0,53 |
| BAI3 | 50491 | 0,00034402 | 0,59 |
| POLR2A | 740130 | 0,00034704 | 0,41 |
| HIS1 | 342551 | 0,0003806 | 0,65 |
| IFITM3 | 713623 | 0,00038781 | 0,42 |
| LILRA1 | 2032639 | 0,0004104 | 0,50 |
| SPON2 | 723923 | 0,00044302 | 0,72 |
| KLRD1 | 2728204 | 0,00045592 | 0,57 |
| IMAGE:176543 | 176543 | 0,00046548 | 0,56 |
| GATA4 | 781738 | 0,00048721 | 0,63 |
| GJA1 | 839101 | 0,00054374 | 0,55 |
| SLA | 32339 | 0,00055491 | 0,48 |
| IMAGE:731357 Transcribed locus | 731357 | 0,00055529 | 0,78 |
| DJ462O23.2 | 738970 | 0,00060349 | 0,52 |
| FLJ35794 | 364204 | 0,00060589 | 0,56 |
| IMAGE:1089025 | 1089025 | 0,0007481 | 0,28 |
| FLJ10707 | 364575 | 0,00076696 | 0,45 |
| LOC388886 | 34007 | 0,00076698 | 0,52 |
| FLJ20254 | 135096 | 0,00078261 | 0,56 |
| DHRS6 | 364412 | 0,00080511 | 0,46 |
| TPD52 | 743259 | 0,00081655 | 0,21 |
| APOE | 1870594 | 0,0008476 | 0,47 |
| IL11RA | 1101773 | 0,00099007 | 0,45 |
| DGKQ | 366233 | 0,00099155 | 0,66 |
| CCL15 | 342290 | 0,00110731 | 0,39 |
| CDR2 | 366067 | 0,00112025 | 0,86 |
| TSHR | 565317 | 0,00115444 | 0,46 |
| IMAGE:742853 | 742853 | 0,00116933 | 0,59 |
| NFE2L2 | 29541 | 0,00119931 | 0,55 |
| ATXN1 | 682528 | 0,00121938 | 0,47 |
| MLL | 80688 | 0,00127262 | 0,45 |
| IMAGE:731398 | 731398 | 0,00132903 | 0,54 |
| AQP11 | 713831 | 0,00135026 | 0,55 |
| ALDH1A1 | 309697 | 0,00135541 | 0,46 |
| ZNF395 | 744983 | 0,00139312 | 0,56 |
| TLN1 | 187482 | 0,00151133 | 0,41 |
| HCLS1 | 665452 | 0,00155934 | 0,62 |
| PDCD4 | 665376 | 0,00163269 | 0,51 |
| CDH3 | 359051 | 0,00167179 | 0,53 |
| PDGFRL | 139242 | 0,00172177 | 0,49 |
| CCL19 | 1707527 | 0,00177893 | 0,16 |
| IMAGE:731726 Transcribed locus | 731726 | 0,00179977 | 0,32 |
| IMAGE:145112 | 145112 | 0,0018241 | 0,60 |
| IMAGE:744055 Transcribed locus | 744055 | 0,00184821 | 0,53 |
| CA11 | 282587 | 0,00186777 | 0,56 |
| MBNL1 | 136114 | 0,00190617 | 0,49 |

| Table 21 | OTC | Under | |
|---|---|---|---|
| name | cloid | p-value | fold |
| GCLC | 1526058 | 0,00195642 | 0,55 |
| PLAC9 | 366115 | 0,00200838 | 0,41 |
| IFNAR2 | 123950 | 0,0023353 | 0,66 |
| TIE1 | 743043 | 0,00253709 | 0,65 |
| CTBP2 | 416744 | 0,00258981 | 0,73 |
| CD3E | 1536968 | 0,0026095 | 0,40 |
| CAV1 | 309645 | 0,00270324 | 0,31 |
| IMAGE:738332 Transcribed locus | 738332 | 0,00290812 | 0,48 |
| KLHL9 | 471696 | 0,00300852 | 0,53 |
| CITED2 | 365090 | 0,00301673 | 0,53 |
| SNTB2 | 667310 | 0,00308273 | 0,61 |
| FABP5 | 1088781 | 0,00315076 | 0,38 |
| GATA2 | 149809 | 0,00322922 | 0,74 |
| EVI5L | 173119 | 0,00323442 | 0,59 |
| IMAGE:724416 Transcribed locus | 724416 | 0,00338129 | 0,41 |
| FAM38B | 743146 | 0,00379144 | 0,69 |
| FOXO3A | 109142 | 0,00389287 | 0,46 |
| SELE | 1239535 | 0,00404397 | 0,57 |
| FLT4 | 668815 | 0,00404811 | 0,52 |
| SATB1 | 364510 | 0,00409049 | 0,50 |
| KIAA1196 | 738938 | 0,00430547 | 0,64 |
| SLC25A23 | 741954 | 0,00436505 | 0,53 |
| ZNF580 | 668089 | 0,00454562 | 0,52 |
| FOXO1A | 151247 | 0,00472685 | 0,58 |
| SEPT-02 | 729542 | 0,00479294 | 0,49 |
| IMAGE:665833 | 665833 | 0,00484015 | 0,76 |
| APLP2 | 549054 | 0,00487222 | 0,45 |
| PMS2 | 116906 | 0,00494457 | 0,54 |
| IMAGE:666946 | 666946 | 0,00542678 | 0,76 |
| SEMA7A | 135941 | 0,00543695 | 0,28 |
| IMAGE:472111 Transcribed locus | 472111 | 0,00552796 | 0,48 |
| E2F5 | 809828 | 0,00605492 | 0,36 |
| YTHDF3 | 135450 | 0,00636167 | 0,57 |
| CDC42EP2 | 26651 | 0,00640863 | 0,72 |
| PCSK5 | 2115808 | 0,0064231 | 0,54 |
| FLJ34433 | 713671 | 0,00657683 | 0,61 |
| IMAGE:743615 Full-length cDNA clone CS0DK001YE19 of HeLa | 743615 | 0,00685838 | 0,72 |

| Table 21 | OTC | Under | |
|---|---|---|---|
| name | cloid | p-value | fold |
| cells Cot 25-normalized of Homo sapiens (human) | | | |
| IMAGE:731714 CDNA FLJ26188 fis, clone ADG04821 | 731714 | 0,00686952 | 0,54 |
| DF | 666128 | 0,00697577 | 0,45 |
| NCAM1 | 366842 | 0,00698396 | 0,61 |
| PTK2 | 724892 | 0,00728776 | 0,51 |
| PH-4 | 730938 | 0,00757078 | 0,68 |
| IMAGE:22908 Transcribed locus, weakly similar to NP_775735.1 l(3)mbt-like 4 [Homo sapiens] | 22908 | 0,00791864 | 0,76 |
| CCND1 | 324079 | 0,00834738 | 0,55 |
| GLG1 | 365613 | 0,00835991 | 0,55 |
| UNC13D | 231903 | 0,00842373 | 0,75 |
| IMAGE:743422 | 743422 | 0,00847541 | 0,73 |
| PSPC1 | 687541 | 0,00850591 | 0,57 |
| RBM25 | 668360 | 0,00902449 | 0,65 |
| GRM3 | 287843 | 0,00907766 | 0,63 |
| CKLFSF3 | 489249 | 0,00916924 | 0,59 |
| BCL2 | 342181 | 0,00939327 | 0,59 |
| SLC17A2 | 207920 | 0,00940866 | 0,53 |
| PRUNE | 364324 | 0,00946088 | 0,57 |
| RDH5 | 682109 | 0,00962973 | 0,63 |
| NCB5OR | 743367 | 0,00984276 | 0,42 |
| CDKN1C | 2381770 | 0,00996888 | 0,48 |
| LIPT1 | 22085 | 0,01008465 | 0,66 |
| FLJ12903 | 730123 | 0,0108591 | 0,71 |
| MORF4L1 | 135875 | 0,01141387 | 0,65 |
| UBE1L | 250883 | 0,01157076 | 0,42 |
| ADCY3 | 667061 | 0,01187515 | 0,57 |
| LOC284371 | 724126 | 0,01210721 | 0,70 |
| JAG2 | 1521706 | 0,01220002 | 0,75 |
| LPXN | 687679 | 0,01248551 | 0,62 |
| ABCC3 | 208097 | 0,01252558 | 0,49 |
| ANKRD20B | 745106 | 0,01264616 | 0,47 |
| PIAS1 | 38789 | 0,01268674 | 0,62 |
| TBC1D22A | 682479 | 0,01361669 | 0,65 |
| ZFOC1 | 744606 | 0,01461132 | 0,50 |
| IMAGE:668684 | 668684 | 0,01465001 | 0,76 |

| Table 21 | OTC | Under | |
|---|---|---|---|
| name | cloid | p-value | fold |
| Transcribed locus, weakly similar to XP_209041.2 PREDICTED: similar to KIAA1503 protein [Homo sapiens] | | | |
| SSH3 | 726272 | 0,01468164 | 0,57 |
| KIAA0759 | 743451 | 0,01491078 | 0,72 |
| GPR108 | 25949 | 0,01515322 | 0,66 |
| RBM5 | 744001 | 0,01521305 | 0,61 |
| APOE | 1870594 | 0,01533491 | 0,39 |
| TJP1 | 179334 | 0,01571594 | 0,59 |
| FMNL2 | 364568 | 0,01590097 | 0,84 |
| CCNE1 | 357807 | 0,0161434 | 0,56 |
| MGP | 590264 | 0,01616941 | 0,33 |
| PEF | 137353 | 0,01649136 | 0,55 |
| SULT1E1 | 207926 | 0,01665512 | 0,78 |
| IMAGE:731298 CDNA FLJ42482 fis, clone BRACE2032134 | 731298 | 0,01703394 | 0,84 |
| BTBD14B | 173288 | 0,01718026 | 0,75 |
| HMGN1 | 565754 | 0,01756741 | 0,79 |
| BMP4 | 69166 | 0,01801726 | 0,58 |
| NYREN18 | 668584 | 0,01840274 | 0,46 |
| LOC92154 | 364926 | 0,01851848 | 0,85 |
| FCGBP | 154172 | 0,01854199 | 0,38 |
| IMAGE:725726 Hypothetical gene supported by AK093801 | 725726 | 0,01874028 | 0,62 |
| TETRAN | 725340 | 0,01946568 | 0,67 |
| IMAGE:23765 | 23765 | 0,01959004 | 0,59 |
| NEXN | 687625 | 0,01961234 | 0,68 |
| PHC3 | 743391 | 0,01961756 | 0,75 |
| DAXX | 292042 | 0,02012924 | 0,62 |
| LILRA1 | 2032639 | 0,02041049 | 0,50 |
| OAZ2 | 128694 | 0,02088621 | 0,67 |
| NR6A1 | 258666 | 0,0209244 | 0,71 |
| NUP214 | 382612 | 0,02101788 | 0,61 |
| CLPX | 725394 | 0,02137618 | 0,68 |
| ANKRD28 | 687381 | 0,02170849 | 0,65 |
| KDR | 469345 | 0,02183637 | 0,52 |

| Table 21 | OTC | Under | |
|---|---|---|---|
| name | cloid | p-value | fold |
| MXD4 | 730036 | 0,0218509 | 0,56 |
| DCTN1 | 180640 | 0,02191199 | 0,73 |
| POU2F2 | 188393 | 0,02191557 | 0,58 |
| TOE1 | 52897 | 0,02192014 | 0,76 |
| RAD51L1 | 295412 | 0,02193486 | 0,59 |
| ESRRBL1 | 51232 | 0,02212668 | 0,59 |
| HMGB2 | 884365 | 0,02272167 | 0,86 |
| MGC4707 | 744945 | 0,02301085 | 0,66 |
| OTUB1 | 174683 | 0,02338881 | 0,55 |
| GC | 195340 | 0,02456764 | 0,71 |
| CASP7 | 279470 | 0,02489014 | 0,73 |
| NFYA | 731648 | 0,02499428 | 0,82 |
| IMAGE:665392 Hypothetical LOC388790 | 665392 | 0,02506006 | 0,73 |
| CTH | 730606 | 0,02560935 | 0,65 |
| C19orf33 | 379540 | 0,02578478 | 0,73 |
| PTK2B | 43541 | 0,0261765 | 0,68 |
| PVRL2 | 725364 | 0,02620575 | 0,71 |
| LOC340073 | 731404 | 0,0263198 | 0,51 |
| CPXM2 | 729924 | 0,0263992 | 0,75 |
| FLJ22794 | 137454 | 0,02646663 | 0,53 |
| DELGEF | 665437 | 0,02657829 | 0,78 |
| NR2F1 | 253386 | 0,02669705 | 0,57 |
| COL18A1 | 359202 | 0,02671076 | 0,51 |
| IMAGE:731685 Transcribed locus | 731685 | 0,02686807 | 0,68 |
| PP1201 | 364974 | 0,02732106 | 0,66 |
| PDLIM1 | 135689 | 0,0279473 | 0,36 |
| MAP3K4 | 727229 | 0,02928203 | 0,72 |
| ZBTB16 | 2467442 | 0,03012785 | 0,50 |
| ABCC8 | 1417901 | 0,03060287 | 0,55 |
| RASGRP1 | 725707 | 0,03127653 | 0,46 |
| FXYD1 | 204686 | 0,03137609 | 0,70 |
| RGL2 | 741891 | 0,03178792 | 0,54 |
| IMAGE:53081 | 53081 | 0,03196289 | 0,57 |
| SMAD4 | 134785 | 0,03265655 | 0,74 |
| IGF1R | 682555 | 0,03280651 | 0,65 |
| IMAGE:22384 CDNA FLJ34183 fis, clone FCBBF3016987 | 22384 | 0,03316977 | 0,67 |
| LOC286170 | 134858 | 0,03322882 | 0,62 |
| IMAGE:379279 | 379279 | 0,03411148 | 0,70 |
| FLJ31438 | 135539 | 0,0343791 | 0,68 |
| BCL2L11 | 300194 | 0,03534285 | 0,74 |

| Table 21 | OTC | Under | |
|---|---|---|---|
| name | cloid | p-value | fold |
| PTHLH | 2214396 | 0,03609798 | 0,74 |
| MEF2C | 687270 | 0,03611238 | 0,66 |
| MGAT3 | 731060 | 0,03670502 | 0,61 |
| IMAGE:174861 Transcribed locus | 174861 | 0,0368892 | 0,83 |
| PSAT1 | 366388 | 0,03731675 | 0,65 |
| PDCD1LG2 | 738944 | 0,03763056 | 0,70 |
| MDC1 | 365288 | 0,03779568 | 0,73 |
| CCNL1 | 251216 | 0,03836932 | 0,63 |
| PAX8 | 545475 | 0,03869026 | 0,52 |
| CNN1 | 726779 | 0,03890173 | 0,55 |
| ACTR1B | 136868 | 0,03907238 | 0,61 |
| DKFZp434M202 | 743118 | 0,04068046 | 0,73 |
| LOC339903 | 135352 | 0,04078367 | 0,62 |
| MDGA1 | 180082 | 0,04209186 | 0,76 |
| IMAGE:2113771 | 2113771 | 0,04219577 | 0,71 |
| GALNT9 | 178750 | 0,04337842 | 0,66 |
| FLJ12604 | 731128 | 0,04447831 | 0,69 |
| ITIH5 | 179733 | 0,04468861 | 0,76 |
| CORO1B | 137205 | 0,04654503 | 0,80 |
| IMAGE:740927 | 740927 | 0,04665612 | 0,58 |
| ADAMTS9 | 376153 | 0,04687807 | 0,55 |
| IMAGE:365097 CDNA FLJ25129 fis, clone CBR06594 | 365097 | 0,04829692 | 0,89 |
| LRP1 | 30219 | 0,04992524 | 0,62 |

| Table 22 | OTC | Over | |
|---|---|---|---|
| name | cloid | p-value | fold |
| MGC29784 | 731019 | 6,47E-04 | 1,40 |
| KIAA1280 | 366085 | 7,06E-04 | 1,63 |
| TIMP2 | 258127 | 8,32E-04 | 1,79 |
| MAK | 382002 | 1,57E-03 | 2,09 |
| UQCRC1 | 714414 | 3,48E-03 | 1,32 |
| ALDH1A3 | 486189 | 4,84E-03 | 1,55 |
| MDH1 | 725188 | 5,29E-03 | 1,51 |
| ZNF258 | 1241974 | 1,04E-02 | 1,47 |
| OAS1 | 666703 | 1,05E-02 | 1,35 |
| LRRIQ2 | 487152 | 1,21E-02 | 2,10 |
| SDHA | 40304 | 1,53E-02 | 3,04 |
| BST2 | 811024 | 0,01594 | 1,39 |
| MESP1 | 25865 | 1,62E-02 | 1,61 |
| IMAGE:666279 CDNA FLJ30779 fis, clone FEBRA2000815 | 666279 | 1,75E-02 | 1,51 |
| IMAGE:745133 Transcribed locus | 745133 | 1,90E-02 | 1,54 |
| CXCL13 | 347362 | 2,06E-02 | 1,76 |
| RPS6KA5 | 258966 | 2,44E-02 | 1,94 |
| FBXO46 | 471664 | 2,69E-02 | 2,57 |
| HP | 82687 | 2,86E-02 | 1,37 |
| IFI30 | 740931 | 2,92E-02 | 1,80 |
| AP2S1 | 739109 | 3,03E-02 | 1,58 |
| GPD1 | 628418 | 3,09E-02 | 2,22 |
| C6orf216 | 731742 | 3,25E-02 | 1,76 |
| SAFB | 42280 | 3,28E-02 | 1,84 |
| SCMH1 | 724014 | 3,33E-02 | 1,75 |
| ABR | 1012903 | 3,39E-02 | 1,22 |
| BAK1 | 1288183 | 3,44E-02 | 2,57 |
| IMAGE:382423 Transcribed locus | 382423 | 3,62E-02 | 3,30 |
| COX6A1 | 512910 | 3,74E-02 | 2,21 |
| IMAGE:667527 | 667527 | 3,79E-02 | 1,39 |
| DECR2 | 730942 | 3,82E-02 | 1,10 |
| AURKC | 731021 | 4,01E-02 | 1,90 |
| IL2RA | 3054031 | 4,03E-02 | 1,75 |
| DCXR | 724596 | 4,03E-02 | 1,76 |
| CTXN1 | 179266 | 4,23E-02 | 1,18 |
| IMAGE:742061 | 742061 | 4,59E-02 | 2,62 |
| IMAGE:667683 Full-length cDNA clone CS0DK008YI09 of HeLa cells Cot 25-normalized of Homo sapiens (human) | 667683 | 4,60E-02 | 1,93 |
| IMAGE:743603 | 743603 | 4,68E-02 | 1,45 |
| LILRB5 | 71428 | 4,70E-02 | 1,85 |
| EIF4EBP1 | 713608 | 4,86E-02 | 1,76 |

| Table 23 | PTC | Under | |
|---|---|---|---|
| name | cloid | p-value | fold |
| CDH16 | 726763 | 4,1E-17 | 0,36 |
| CRABP1 | 739193 | 3E-13 | 0,16 |
| MATN2 | 28584 | 4,3E-13 | 0,29 |
| MGC99813 | 739097 | 5,6E-13 | 0,19 |
| ASGR1 | 25883 | 2,4E-11 | 0,38 |
| IMAGE:1716 286 | 1716286 | 4,1E-10 | 0,37 |
| HSPC159 | 365045 | 2,2E-09 | 0,60 |
| PTPN4 | 666367 | 7,9E-09 | 0,54 |
| IRAK1 | 379200 | 1,3E-08 | 0,63 |
| GLT8D2 | 365271 | 2E-08 | 0,49 |
| MGC20781 | 731227 | 2,2E-08 | 0,47 |
| LOC440934 | 726760 | 2,3E-08 | 0,53 |
| FLJ11184 | 384404 | 2,8E-08 | 0,42 |
| KLF1 | 208991 | 2,8E-08 | 0,45 |
| FLJ14351 | 744943 | 3,2E-08 | 0,25 |
| BMP5 | 1846326 | 6,1E-08 | 0,34 |
| ZNF319 | 364729 | 7,3E-08 | 0,23 |
| OBSCN | 730926 | 7,6E-08 | 0,64 |
| FKBP6 | 795736 | 1,2E-07 | 0,45 |
| IMAGE:1366 86 | 136686 | 1,4E-07 | 0,59 |
| FLJ13197 | 667117 | 1,5E-07 | 0,74 |
| IMAGE:7435 17 Transcribed locus | 743517 | 1,6E-07 | 0,52 |
| MTAP | 724151 | 1,7E-07 | 0,55 |
| DIO1 | 296702 | 1,9E-07 | 0,32 |
| IMAGE:7428 37 Transcribed locus | 742837 | 2,3E-07 | 0,65 |
| PPIL4 | 364777 | 2,6E-07 | 0,29 |
| APIN | 364706 | 4,8E-07 | 0,59 |
| IMAGE:7316 16 MRNA; cDNA DKFZp686A 102 (from clone DKFZp686A 102) | 731616 | 5,6E-07 | 0,40 |
| FLJ30707 | 666986 | 6,4E-07 | 0,60 |
| VAV1 | 80384 | 6,6E-07 | 0,38 |
| USP13 | 666007 | 6,9E-07 | 0,56 |
| ABCB1 | 1837488 | 7,1E-07 | 0,48 |
| MAPRE2 | 383868 | 8,4E-07 | 0,48 |
| SDHA | 40304 | 9,2E-07 | 0,64 |
| MATN2 | 366100 | 9,4E-07 | 0,43 |
| IMAGE:7440 55 | 744055 | 1E-06 | 0,48 |

| Table 23 | PTC | Under | |
|---|---|---|---|
| name | cloid | p-value | fold |
| Transcribed locus | | | |
| CCL28 | 136919 | 1,6E-06 | 0,67 |
| VTN | 230126 | 2E-06 | 0,61 |
| BAK1 | 1288183 | 2,4E-06 | 0,45 |
| TOSO | 813174 | 2,6E-06 | 0,54 |
| HBA2 | 469647 | 2,8E-06 | 0,34 |
| SLC4A4 | 787938 | 2,9E-06 | 0,46 |
| DUSP24 | 740158 | 3,3E-06 | 0,40 |
| EIF4EBP1 | 713608 | 3,8E-06 | 0,72 |
| DEPDC6 | 669318 | 4,2E-06 | 0,52 |
| SOD3 | 795309 | 4,3E-06 | 0,39 |
| TM4SF9 | 812967 | 4,9E-06 | 0,42 |
| KRTHA4 | 325155 | 5,4E-06 | 0,50 |
| GNAS | 382791 | 5,5E-06 | 0,46 |
| DCXR | 724596 | 5,5E-06 | 0,63 |
| TBCA | 743703 | 5,6E-06 | 0,63 |
| ME3 | 724238 | 6,5E-06 | 0,52 |
| FCGBP | 154172 | 8,2E-06 | 0,49 |
| GTPBP1 | 366484 | 8,4E-06 | 0,70 |
| KIF27 | 667657 | 8,4E-06 | 0,67 |
| HBB | 469549 | 8,6E-06 | 0,34 |
| SQLE | 124781 | 9,9E-06 | 0,61 |
| FLJ23577 | 668452 | 9,9E-06 | 0,43 |
| AQP11 | 713831 | 1,2E-05 | 0,58 |
| LEF1 | 713913 | 1,3E-05 | 0,65 |
| IMAGE:3837 18 | 383718 | 1,3E-05 | 0,60 |
| DAB1 | 382621 | 1,5E-05 | 0,58 |
| IMAGE:7446 16 DNA damage repair and recombinatio n protein RAD52 pseudogene | 744616 | 1,6E-05 | 0,63 |
| OSR1 | 364686 | 1,7E-05 | 0,65 |
| HAS3 | 667533 | 1,7E-05 | 0,54 |
| IMAGE:7440 74 Transcribed locus | 744074 | 1,8E-05 | 0,39 |
| KIAA1277 | 364822 | 2E-05 | 0,66 |
| ARF4L | 49888 | 2,1E-05 | 0,59 |
| IMAGE:2458 7 | 24587 | 2,1E-05 | 0,59 |
| IMAGE:3639 55 Transcribed locus | 363955 | 2,2E-05 | 0,51 |
| PDK2 | 114008 | 2,4E-05 | 0,68 |
| PLCG2 | 201467 | 2,4E-05 | 0,63 |

| Table 23 | PTC | Under | |
|---|---|---|---|
| name | cloid | p-value | fold |
| RAD51L1 | 295412 | 2,5E-05 | 0,61 |
| WNT4 | 375746 | 2,7E-05 | 0,66 |
| IMAGE:666315 Homo sapiens, clone IMAGE:5312086, mRNA | 666315 | 2,8E-05 | 0,58 |
| IMAGE:744385 Similar to heat shock 10kDa protein 1 (chaperonin 10); heat shock 10kD protein 1 (chaperonin 10) | 744385 | 3E-05 | 0,59 |
| USP4 | 544818 | 3,1E-05 | 0,43 |
| FLJ12998 | 364846 | 3,2E-05 | 0,64 |
| KLRD1 | 145696 | 3,4E-05 | 0,64 |
| IMAGE:738424 | 738424 | 3,6E-05 | 0,73 |
| MATN1 | 1624260 | 3,7E-05 | 0,43 |
| FLJ35630 | 485652 | 3,8E-05 | 0,70 |
| SPI1 | 1285305 | 3,9E-05 | 0,68 |
| IMAGE:2484270 | 2484270 | 5,3E-05 | 0,55 |
| FLJ21918 | 194302 | 6,2E-05 | 0,74 |
| CD34 | 770858 | 6,2E-05 | 0,71 |
| LOH12CR2 | 366074 | 6,9E-05 | 0,70 |
| SIAT7B | 823590 | 7,3E-05 | 0,57 |
| SCMH1 | 724014 | 7,4E-05 | 0,46 |
| IMAGE:379937 | 379937 | 7,4E-05 | 0,63 |
| TSG101 | 194350 | 7,7E-05 | 0,67 |
| GADD45G | 727321 | 7,8E-05 | 0,58 |
| C2orf7 | 366243 | 8,1E-05 | 0,72 |
| CCNE1 | 68950 | 8,2E-05 | 0,68 |
| IMAGE:364741 | 364741 | 8,4E-05 | 0,58 |
| MCC | 731305 | 8,5E-05 | 0,47 |
| PRKCABP | 1174342 | 8,8E-05 | 0,77 |
| ULK4 | 744895 | 8,9E-05 | 0,67 |
| BCL2 | 342181 | 9,2E-05 | 0,63 |
| PIP | 985457 | 0,00011 | 0,68 |
| BIN1 | 2384812 | 0,00011 | 0,67 |
| IMAGE:727289 Transcribed locus | 727289 | 0,00012 | 0,48 |
| HMG20A | 731277 | 0,00012 | 0,56 |
| COX5A | 1085884 | 0,00012 | 0,70 |

| Table 23 | PTC | Under | |
|---|---|---|---|
| name | cloid | p-value | fold |
| QIL1 | 727296 | 0,00013 | 0,81 |
| IMAGE:383966 | 383966 | 0,00014 | 0,65 |
| CPXM2 | 729924 | 0,00015 | 0,58 |
| ANP32E | 382738 | 0,00016 | 0,56 |
| MRPS11 | 471574 | 0,00016 | 0,65 |
| IMAGE:682585 | 682585 | 0,00017 | 0,68 |
| ALDH3A1 | 525221 | 0,00017 | 0,64 |
| KLK2 | 1102600 | 0,00017 | 0,62 |
| C6orf85 | 665379 | 0,00017 | 0,71 |
| GNAI1 | 753215 | 0,00018 | 0,65 |
| COL18A1 | 359202 | 0,00019 | 0,62 |
| VAV2 | 1581686 | 0,00019 | 0,74 |
| SH3RF2 | 744797 | 0,00021 | 0,58 |
| IMAGE:726513 Similar to 40S ribosomal protein S3 | 726513 | 0,00021 | 0,66 |
| NME7 | 743982 | 0,00023 | 0,43 |
| LOC388284 | 731722 | 0,00023 | 0,75 |
| RASAL2 | 486304 | 0,00023 | 0,48 |
| MAPK3 | 323438 | 0,00024 | 0,64 |
| IMAGE:743422 | 743422 | 0,00024 | 0,76 |
| IMAGE:727491 | 727491 | 0,00024 | 0,41 |
| PITPNC1 | 364436 | 0,00027 | 0,69 |
| CABIN1 | 1844968 | 0,00027 | 0,67 |
| DF | 666128 | 0,00027 | 0,52 |
| GCK | 30981 | 0,00028 | 0,73 |
| SIAT8C | 382069 | 0,00028 | 0,63 |
| S100A2 | 3659591 | 0,00028 | 0,69 |
| DKFZp761P0423 | 730829 | 0,00029 | 0,65 |
| F5 | 433155 | 0,00029 | 0,55 |
| ACADSB | 687953 | 0,00029 | 0,61 |
| NEGR1 | 667176 | 0,0003 | 0,76 |
| LHB | 1671903 | 0,0003 | 0,61 |
| PRUNE | 364324 | 0,00032 | 0,64 |
| CNN1 | 726779 | 0,00037 | 0,60 |
| PHC2 | 668146 | 0,00037 | 0,62 |
| IMAGE:743579 | 743579 | 0,00038 | 0,53 |
| IMAGE:26164 | 26164 | 0,00038 | 0,76 |
| PCGF4 | 740457 | 0,0004 | 0,53 |
| RANBP10 | 307025 | 0,00042 | 0,72 |
| IMAGE:667527 | 667527 | 0,00042 | 0,54 |
| SEMA7A | 135941 | 0,00043 | 0,36 |
| LMLN | 744657 | 0,00044 | 0,68 |

| Table 23 | PTC | Under | |
|---|---|---|---|
| name | cloid | p-value | fold |
| C20orf18 | 665433 | 0,00045 | 0,56 |
| PPP2R4 | 731590 | 0,00047 | 0,61 |
| IMAGE:378458 | 378458 | 0,00047 | 0,76 |
| SPON2 | 723923 | 0,0005 | 0,70 |
| PSG3 | 136747 | 0,00052 | 0,65 |
| CREB3L2 | 136399 | 0,00073 | 0,70 |
| IMAGE:744941 LOC440728 | 744941 | 0,00074 | 0,54 |
| IMAGE:365913 Transcribed locus | 365913 | 0,00076 | 0,69 |
| IMAGE:730924 Transcribed locus | 730924 | 0,00078 | 0,70 |
| SLC2A4RG | 376983 | 0,00081 | 0,70 |
| PRKCQ | 669149 | 0,00081 | 0,69 |
| RANBP10 | 682251 | 0,00087 | 0,68 |
| SGCG | 2046361 | 0,0009 | 0,76 |
| SYP | 30471 | 0,0009 | 0,52 |
| IMAGE:665403 Transcribed locus | 665403 | 0,00096 | 0,82 |
| TIE1 | 743043 | 0,00097 | 0,69 |
| RAB8A | 712118 | 0,00098 | 0,74 |
| CES2 | 153667 | 0,00099 | 0,76 |
| CCL15 | 342290 | 0,00099 | 0,57 |
| RNF32 | 731422 | 0,00104 | 0,47 |
| OAS1 | 666703 | 0,00104 | 0,57 |
| PKNOX1 | 667067 | 0,00108 | 0,61 |
| ATP2B4 | 665814 | 0,00116 | 0,63 |
| LOC253981 | 742743 | 0,00118 | 0,62 |
| HP | 82687 | 0,0012 | 0,78 |
| E2F5 | 809828 | 0,00127 | 0,47 |
| SEPW1 | 173628 | 0,00132 | 0,52 |
| CCR7 | 2345206 | 0,00134 | 0,68 |
| LYPLA3 | 471702 | 0,00143 | 0,75 |
| KCNQ2 | 179534 | 0,00144 | 0,62 |
| C21orf84 | 730814 | 0,00145 | 0,59 |
| CREBBP | 172996 | 0,00146 | 0,63 |
| HSPC072 | 668329 | 0,00148 | 0,72 |
| RODH | 471641 | 0,00149 | 0,54 |
| TRIP12 | 665820 | 0,00155 | 0,55 |
| MGC23909 | 731598 | 0,00155 | 0,50 |
| IMAGE:136121 CDNA FLJ10247 fis, clone HEMBB1000705 | 136121 | 0,00157 | 0,71 |
| GATA3 | 148796 | 0,00158 | 0,72 |
| SIGLEC7 | 743331 | 0,00161 | 0,74 |
| PDCD1LG1 | 1942634 | 0,00162 | 0,68 |
| IMAGE:731758 Transcribed locus, weakly similar to NP_081070.1 kiaa-iso protein homolog; Band 47B [Mus musculus] | 731758 | 0,00163 | 0,77 |
| CD38 | 1352408 | 0,00163 | 0,80 |
| ALDH1A1 | 309697 | 0,00163 | 0,55 |
| MUTED | 731202 | 0,00165 | 0,60 |
| IMAGE:472111 Transcribed locus | 472111 | 0,00168 | 0,61 |
| KLHL9 | 501527 | 0,00169 | 0,69 |
| MT1F | 78353 | 0,0017 | 0,40 |
| GPI | 741474 | 0,0017 | 0,69 |
| IMAGE:738945 | 738945 | 0,00176 | 0,79 |
| GALNT9 | 178750 | 0,0018 | 0,72 |
| IMAGE:743603 | 743603 | 0,0018 | 0,74 |
| PARD3 | 724642 | 0,00183 | 0,70 |
| KIAA0789 | 33621 | 0,00193 | 0,66 |
| AXL | 364083 | 0,00194 | 0,58 |
| TM4SF9 | 381032 | 0,00197 | 0,75 |
| ZNF76 | 745003 | 0,002 | 0,75 |
| C20orf19 | 366032 | 0,00204 | 0,71 |
| RAP1GA1 | 971276 | 0,00208 | 0,80 |
| SLC6A1 | 177967 | 0,0021 | 0,63 |
| BAZ2B | 609631 | 0,00212 | 0,78 |
| NEK6 | 725345 | 0,00212 | 0,74 |
| IMAGE:745512 | 745512 | 0,00215 | 0,74 |
| LILRB5 | 71428 | 0,00216 | 0,68 |
| GFPT2 | 485085 | 0,00217 | 0,72 |
| IMAGE:743903 Homo sapiens, clone IMAGE:5266541, mRNA | 743903 | 0,0022 | 0,60 |
| IMAGE:744439 Transcribed | 744439 | 0,00229 | 0,71 |

| Table 23 | PTC | Under | |
|---|---|---|---|
| name | cloid | p-value | fold |
| locus | | | |
| BIC | 743270 | 0,00237 | 0,68 |
| IMAGE:738966 Transcribed locus | 738966 | 0,00238 | 0,66 |
| GJA5 | 196338 | 0,0024 | 0,65 |
| GRIK2 | 731363 | 0,0024 | 0,77 |
| DMD | 743394 | 0,00243 | 0,63 |
| CDR2 | 366067 | 0,00249 | 0,81 |
| IMAGE:727067 Similar to hypothetical protein SB153 isoform 1 | 727067 | 0,00251 | 0,72 |
| ANKRD20B | 745106 | 0,00257 | 0,61 |
| PCSK6 | 31924 | 0,00257 | 0,63 |
| DC12 | 724895 | 0,00272 | 0,74 |
| LOC388886 | 34007 | 0,00285 | 0,70 |
| IMAGE:744841 LOC441054 | 744841 | 0,00286 | 0,74 |
| LOC51760 | 52226 | 0,00287 | 0,77 |
| AURKC | 731021 | 0,00289 | 0,64 |
| IGSF10 | 682276 | 0,0029 | 0,82 |
| LPXN | 687679 | 0,00294 | 0,79 |
| LOC339903 | 135352 | 0,00295 | 0,72 |
| SERPINB5 | 1662274 | 0,00313 | 0,72 |
| SFRP4 | 285693 | 0,00324 | 0,57 |
| GCAT | 307094 | 0,00333 | 0,76 |
| SAFB | 42280 | 0,00336 | 0,61 |
| CTRL | 1308954 | 0,00345 | 0,81 |
| FLT4 | 668815 | 0,00347 | 0,69 |
| TTC17 | 665668 | 0,00359 | 0,75 |
| BCL2 | 232714 | 0,0036 | 0,69 |
| BMP7 | 1594414 | 0,00364 | 0,68 |
| HOXD11 | 682119 | 0,00365 | 0,59 |
| SIN3A | 26455 | 0,00377 | 0,82 |
| SIAT8F | 667110 | 0,00379 | 0,63 |
| SMAD4 | 134785 | 0,00389 | 0,79 |
| BDKRB2 | 665674 | 0,00394 | 0,65 |
| ARHGAP10 | 731380 | 0,00394 | 0,63 |
| DCTN1 | 180640 | 0,00395 | 0,73 |
| APOD | 838611 | 0,00399 | 0,43 |
| LOC440434 | 742046 | 0,00399 | 0,80 |
| LRP1 | 30219 | 0,00411 | 0,76 |
| SLC17A2 | 207920 | 0,0043 | 0,57 |
| KCNK4 | 743016 | 0,00434 | 0,70 |
| MLPH | 667259 | 0,00447 | 0,43 |
| GPD1 | 628418 | 0,00451 | 0,70 |
| FLJ25067 | 667252 | 0,00453 | 0,57 |

| Table 23 | PTC | Under | |
|---|---|---|---|
| name | cloid | p-value | fold |
| PTGES | 504646 | 0,00472 | 0,72 |
| LOC286272 | 667361 | 0,00476 | 0,80 |
| IMAGE:742061 | 742061 | 0,00479 | 0,62 |
| FLJ44005 | 726675 | 0,00482 | 0,76 |
| UNC13D | 231903 | 0,00505 | 0,58 |
| DCI | 667892 | 0,00511 | 0,64 |
| SYCP2 | 136863 | 0,00522 | 0,67 |
| MGC5178 | 725533 | 0,00522 | 0,66 |
| CD33 | 1917430 | 0,00523 | 0,60 |
| PEF | 137353 | 0,00539 | 0,73 |
| KIAA1838 | 744106 | 0,00542 | 0,83 |
| SRGAP2 | 714493 | 0,00547 | 0,84 |
| FLJ22386 | 32917 | 0,00552 | 0,74 |
| MXD3 | 714013 | 0,0058 | 0,81 |
| BCL2 | 342181 | 0,00587 | 0,61 |
| TIMP3 | 501476 | 0,00587 | 0,68 |
| EIF2C4 | 730834 | 0,00636 | 0,75 |
| MAP7 | 79729 | 0,00642 | 0,76 |
| IMAGE:666794 Hypothetical LOC388170 | 666794 | 0,00646 | 0,71 |
| ZBTB16 | 2467442 | 0,00703 | 0,63 |
| NF2 | 1698236 | 0,00722 | 0,76 |
| APOL1 | 665632 | 0,00739 | 0,54 |
| DDX54 | 743268 | 0,0074 | 0,77 |
| ZNF250 | 741066 | 0,00747 | 0,48 |
| HTATIP2 | 726618 | 0,00801 | 0,83 |
| IMAGE:743290 Transcribed locus | 743290 | 0,00804 | 0,63 |
| C21orf7 | 665495 | 0,00825 | 0,78 |
| GSTM1 | 73778 | 0,00829 | 0,72 |
| IMAGE:744410 Transcribed locus | 744410 | 0,00835 | 0,78 |
| PRKCQ | 669149 | 0,00838 | 0,70 |
| KIAA1463 | 381118 | 0,00846 | 0,73 |
| ETV4 | 809959 | 0,00848 | 0,77 |
| IGHM | 276658 | 0,00848 | 0,79 |
| COX6B1 | 632026 | 0,00854 | 0,72 |
| RUNX3 | 122874 | 0,00873 | 0,74 |
| PSCD3 | 744050 | 0,00895 | 0,75 |
| C6orf118 | 731745 | 0,00902 | 0,74 |
| PLCG1 | 1174287 | 0,00935 | 0,79 |
| IMAGE:745123 Transcribed locus | 745123 | 0,00936 | 0,51 |
| MRC2 | 342581 | 0,00949 | 0,58 |

| Table 23 | PTC | Under | |
|---|---|---|---|
| name | cloid | p-value | fold |
| C4orf16 | 681890 | 0,00979 | 0,78 |
| ANAPC2 | 136462 | 0,00996 | 0,79 |
| CDC42EP2 | 26651 | 0,00999 | 0,84 |
| DEXI | 668186 | 0,0102 | 0,79 |
| ATP5G3 | 740090 | 0,0105 | 0,86 |
| AKR1C1 | 196992 | 0,01063 | 0,31 |
| CBL | 1578721 | 0,0107 | 0,71 |
| IMAGE:745072 Transcribed locus | 745072 | 0,01074 | 0,61 |
| SGSH | 669263 | 0,0108 | 0,78 |
| PINK1 | 729929 | 0,01102 | 0,76 |
| MFAP3L | 726821 | 0,01112 | 0,75 |
| C10orf116 | 740941 | 0,01117 | 0,73 |
| LILRB3 | 1422194 | 0,01144 | 0,79 |
| UQCRC1 | 714414 | 0,01184 | 0,80 |
| CaMKIINalpha | 173820 | 0,01261 | 0,81 |
| TMED4 | 251250 | 0,01265 | 0,74 |
| KIAA1754L | 365056 | 0,01278 | 0,71 |
| PLXNC1 | 724609 | 0,01313 | 0,80 |
| TFF1 | 1075949 | 0,01318 | 0,59 |
| COX6A1 | 512910 | 0,01348 | 0,74 |
| AMD1 | 132752 | 0,01358 | 0,72 |
| IMAGE:740105 | 740105 | 0,01359 | 0,83 |
| NTRK2 | 2048801 | 0,01375 | 0,59 |
| RPL27A | 178255 | 0,01408 | 0,64 |
| MRPL45 | 364717 | 0,01449 | 0,71 |
| ANKRD28 | 687379 | 0,0149 | 0,58 |
| DCAMKL1 | 232388 | 0,01532 | 0,73 |
| BCL2 | 342181 | 0,0154 | 0,72 |
| FBXL16 | 178908 | 0,01584 | 0,86 |
| IMAGE:744604 | 744604 | 0,01636 | 0,76 |
| TRIM8 | 714498 | 0,01646 | 0,75 |
| IMAGE:665342 Transcribed locus, strongly similar to NP_065847.1 semaphorin 6A1; semaphorin 6A-1 [Homo sapiens] | 665342 | 0,01671 | 0,83 |
| MPST | 731241 | 0,01715 | 0,69 |
| LRRIQ2 | 487152 | 0,01726 | 0,77 |
| NK4 | 504158 | 0,01782 | 0,76 |
| FLJ37587 | 668476 | 0,01845 | 0,86 |
| IMAGE:743619 | 743619 | 0,01869 | 0,78 |
| THAP3 | 687800 | 0,01904 | 0,59 |
| FSHPRH1 | 667355 | 0,01935 | 0,78 |
| ADAM12 | 724812 | 0,01958 | 0,73 |
| SIAT8B | 33133 | 0,01996 | 0,77 |
| H17 | 739432 | 0,01996 | 0,80 |
| PH-4 | 730938 | 0,02017 | 0,78 |
| MGP | 590264 | 0,02027 | 0,65 |
| BBS4 | 726634 | 0,02117 | 0,86 |
| FBXW8 | 687532 | 0,02149 | 0,82 |
| FRZB | 146049 | 0,02151 | 0,71 |
| IMAGE:213529 Transcribed locus | 213529 | 0,02296 | 0,83 |
| IMAGE:687896 PREDICTED: Homo sapiens olfactory receptor, family 7, subfamily E, member 31 pseudogene (OR7E31P), mRNA | 687896 | 0,02327 | 0,83 |
| SLAMF1 | 1626951 | 0,0241 | 0,82 |
| ABCA8 | 284828 | 0,0243 | 0,70 |
| CLGN | 1049033 | 0,02437 | 0,81 |
| CASP5 | 341763 | 0,02446 | 0,63 |
| SYN3 | 727056 | 0,02495 | 0,57 |
| IL2RA | 3054031 | 0,02508 | 0,75 |
| NME1 | 726600 | 0,02562 | 0,81 |
| ICAM3 | 156183 | 0,02566 | 0,83 |
| SLC36A3 | 731115 | 0,02573 | 0,83 |
| KLHL9 | 471696 | 0,0258 | 0,61 |
| CKLFSF4 | 143759 | 0,02637 | 0,81 |
| PLAC9 | 366115 | 0,02652 | 0,74 |
| IMAGE:383528 Transcribed locus, weakly similar to XP_375099.1 PREDICTED: similar to hypothetical protein FLJ25224 | 383528 | 0,02704 | 0,36 |

| Table 23 | PTC | Under | |
|---|---|---|---|
| name | cloid | p-value | fold |
| [Homo sapiens] | | | |
| C6orf216 | 731742 | 0,03001 | 0,82 |
| SASH1 | 31120 | 0,0303 | 0,77 |
| IMAGE:174861 Transcribed locus | 174861 | 0,03087 | 0,85 |
| NYREN18 | 668584 | 0,0313 | 0,77 |
| KIAA0759 | 743451 | 0,03159 | 0,55 |
| SLC9A5 | 112819 | 0,0328 | 0,83 |
| JAK1 | 2030501 | 0,03295 | 0,77 |
| IMAGE:724556 Transcribed locus | 724556 | 0,03315 | 0,77 |
| IL11RA | 1101773 | 0,03445 | 0,75 |
| IL6ST | 137010 | 0,03464 | 0,82 |
| CCR7 | 2345206 | 0,03474 | 0,79 |
| FOXN4 | 731076 | 0,03623 | 0,80 |
| SPTLC2 | 365000 | 0,03624 | 0,83 |
| CCL19 | 1707527 | 0,03653 | 0,38 |
| DGKQ | 366233 | 0,03722 | 0,83 |
| SENP6 | 366436 | 0,0378 | 0,85 |
| ID1 | 1087348 | 0,04088 | 0,71 |
| RBM25 | 668360 | 0,04092 | 0,83 |
| CASP10 | 241481 | 0,04117 | 0,72 |
| LOC340073 | 731404 | 0,04151 | 0,81 |
| PTDSS2 | 21716 | 0,04197 | 0,84 |
| KIAA1196 | 738938 | 0,04253 | 0,74 |
| KCNC3 | 53333 | 0,04428 | 0,82 |
| PSMD11 | 383945 | 0,04455 | 0,70 |
| LRP2 | 2055272 | 0,04533 | 0,82 |
| EBAG9 | 682507 | 0,04536 | 0,78 |
| BAP1 | 1012990 | 0,04607 | 0,70 |
| GIMAP5 | 180259 | 0,04611 | 0,80 |
| MMRN1 | 759865 | 0,04613 | 0,81 |
| SAP30L | 723950 | 0,0485 | 0,85 |

| Table 24 | PTC | Over | |
|---|---|---|---|
| name | cloid | p-value | fold |
| HIVEP1 | 758037 | 1,36E-04 | 1,49 |
| IL19 | 1932168 | 1,39E-04 | 1,87 |
| CTTN | 489945 | 1,62E-04 | 1,64 |
| DJ462O23.2 | 738970 | 1,69E-04 | 1,55 |
| TRAF4 | 667756 | 1,72E-04 | 1,47 |
| CITED1 | 265558 | 2,13E-04 | 4,50 |
| IMAGE:2113771 | 2113771 | 2,84E-04 | 1,61 |
| IMAGE:365562 Transcribed locus, moderately similar to XP_521389.1 PREDICTED: similar to UTY [Pan troglodytes] | 365562 | 3,36E-04 | 1,60 |
| ZNF217 | 1712992 | 3,47E-04 | 1,58 |
| SENP6 | 739237 | 3,79E-04 | 1,71 |
| FLJ31438 | 135539 | 4,65E-04 | 1,52 |
| IMAGE:159809 | 159809 | 4,96E-04 | 1,28 |
| CTNNA1 | 268972 | 5,75E-04 | 1,31 |
| HT007 | 135303 | 6,22E-04 | 1,52 |
| STAT6 | 1916307 | 6,41E-04 | 1,36 |
| CD5 | 356841 | 6,57E-04 | 3,22 |
| LOC126669 | 682088 | 6,73E-04 | 1,83 |
| DELGEF | 665437 | 6,79E-04 | 1,30 |
| MGC4707 | 744945 | 7,09E-04 | 1,49 |
| KLRD1 | 2728204 | 7,15E-04 | 2,45 |
| CCND1 | 324079 | 7,23E-04 | 2,88 |
| IMAGE:727540 | 727540 | 7,97E-04 | 1,92 |
| CTTN | 470408 | 8,04E-04 | 1,65 |
| PREB | 740347 | 8,06E-04 | 5,33 |
| FMO5 | 364526 | 8,18E-04 | 2,22 |
| DPP4 | 343987 | 8,52E-04 | 5,49 |
| SPATA12 | 730300 | 8,67E-04 | 1,54 |
| CLDN1 | 594279 | 9,22E-04 | 10,32 |
| TIMP1 | 162246 | 9,38E-04 | 4,99 |
| APLP2 | 549054 | 1,03E-03 | 1,86 |
| IMP-2 | 743774 | 1,25E-03 | 2,09 |
| GRB7 | 510318 | 1,32E-03 | 1,47 |
| IMAGE:136976 Transcribed locus, moderately similar to | 136976 | 1,34E-03 | 2,61 |
| NP_777603.1 hypothetical protein FLJ25976 [Homo sapiens] | | | |
| PCTK3 | 725677 | 1,35E-03 | 1,41 |
| PARP9 | 667440 | 1,37E-03 | 1,54 |
| CAPN3 | 757248 | 1,49E-03 | 1,74 |
| GSS | 140405 | 1,51E-03 | 2,29 |
| PTHLH | 2214396 | 1,73E-03 | 1,86 |
| MPO | 436554 | 1,87E-03 | 1,30 |
| CCNE1 | 357807 | 1,89E-03 | 1,69 |
| STAT2 | 2306096 | 1,92E-03 | 1,58 |
| SCEL | 668239 | 1,95E-03 | 2,05 |
| STAU | 365919 | 2,10E-03 | 1,37 |
| ATIC | 665693 | 0,00217 | 1,63 |
| CD63 | 125552 | 2,40E-03 | 1,58 |
| PITPNA | 725585 | 2,54E-03 | 3,56 |
| CASP3 | 823680 | 2,82E-03 | 2,49 |
| CDH3 | 359051 | 2,87E-03 | 5,54 |
| BMP5 | 1846326 | 2,94E-03 | 1,85 |
| MDK | 309009 | 3,00E-03 | 3,16 |
| LOC120224 | 252291 | 3,05E-03 | 2,20 |
| IMAGE:1877668 | 1877668 | 0,00311 | 1,70 |
| IL13 | 1505308 | 3,26E-03 | 1,57 |
| TARP | 145105 | 3,35E-03 | 3,00 |
| MAPK14 | 1741589 | 3,45E-03 | 1,49 |
| CCND3 | 327182 | 3,54E-03 | 1,69 |
| POU2F2 | 188393 | 3,59E-03 | 2,76 |
| PET112L | 743125 | 3,61E-03 | 2,23 |
| IMAGE:132933 | 132933 | 3,64E-03 | 1,59 |
| TOE1 | 52897 | 3,70E-03 | 1,27 |
| IMAGE:731751 Transcribed locus | 731751 | 3,81E-03 | 1,79 |
| NOTCH1 | 359461 | 3,88E-03 | 2,14 |
| CACNB2 | 173841 | 3,90E-03 | 2,97 |
| ABCC5 | 212366 | 0,00419 | 1,83 |
| DNMT1 | 768241 | 4,26E-03 | 1,60 |
| YWHAH | 324066 | 5,16E-03 | 1,31 |
| SMARCA5 | 730037 | 5,18E-03 | 1,46 |
| MRC2 | 235882 | 5,27E-03 | 4,72 |
| IMAGE:668300 Transcribed locus, strongly similar to NP_683708.1 Toll-interleukin 1 | 668300 | 5,32E-03 | 1,38 |

| | | | |
|---|---|---|---|
| receptor domain-containing adaptor protein isoform b; adapter protein wyatt; MyD88 adapter-like protein; TIR domain-containing adapter protein; Toll-interleukin 1 receptor (TIR) domain-containing adaptor protein [Homo sapiens] | | | |
| IMAGE:2266583 | 2266583 | 5,45E-03 | 1,63 |
| HOXB6 | 738358 | 5,48E-03 | 1,52 |
| SPINT1 | 723914 | 5,81E-03 | 1,60 |
| PMS2 | 116906 | 5,86E-03 | 1,67 |
| RFX2 | 731738 | 6,21E-03 | 2,41 |
| GABRE | 209137 | 6,28E-03 | 1,45 |
| TSC | 745490 | 6,58E-03 | 5,91 |
| ABCC3 | 208097 | 6,60E-03 | 4,33 |
| VCL | 44193 | 6,73E-03 | 1,39 |
| CD8B1 | 1743279 | 6,94E-03 | 1,28 |
| PPP3CC | 110481 | 7,14E-03 | 1,79 |
| ETV1 | 24541 | 7,26E-03 | 1,37 |
| CD9 | 727251 | 7,34E-03 | 1,65 |
| USP18 | 745083 | 7,42E-03 | 2,07 |
| LAMB3 | 1103402 | 7,43E-03 | 1,93 |
| ETHE1 | 681957 | 7,45E-03 | 1,54 |
| PDLIM1 | 135689 | 7,58E-03 | 1,59 |
| CD59 | 132935 | 7,88E-03 | 1,35 |
| FLJ12604 | 731128 | 8,59E-03 | 2,31 |
| ADAMTS9 | 376153 | 9,32E-03 | 2,27 |
| CA11 | 282587 | 9,49E-03 | 1,61 |
| PGR1 | 666361 | 9,68E-03 | 1,22 |
| MORF4L1 | 135875 | 9,72E-03 | 1,46 |
| IBRDC3 | 726636 | 1,00E-02 | 1,29 |
| NOTCH2 | 1641901 | 1,01E-02 | 2,81 |
| ETV4 | 1690788 | 1,03E-02 | 1,60 |
| MAPK14 | 2011082 | 1,06E-02 | 1,51 |
| RNF123 | 366159 | 1,07E-02 | 1,39 |
| COX15 | 32784 | 1,09E-02 | 1,56 |
| RPS6KA2 | 22711 | 1,10E-02 | 1,50 |
| FLJ34433 | 713671 | 1,14E-02 | 1,42 |
| TU3A | 44881 | 1,17E-02 | 2,03 |
| IL13RA1 | 1492440 | 1,19E-02 | 1,31 |
| IKBKE | 364448 | 1,21E-02 | 1,41 |
| CD47 | 365526 | 1,22E-02 | 1,66 |
| RASGRP1 | 725707 | 0,01241 | 4,66 |
| CKS2 | 725454 | 1,25E-02 | 1,55 |
| FUS | 365348 | 1,28E-02 | 1,35 |
| HDAC1 | 1896337 | 1,29E-02 | 1,87 |
| IMAGE:251427 | 251427 | 1,30E-02 | 2,25 |
| KRT18 | 725096 | 1,32E-02 | 1,55 |
| FLJ22794 | 137454 | 1,38E-02 | 1,53 |
| RYK | 727092 | 1,41E-02 | 1,26 |
| CD47 | 357442 | 1,53E-02 | 1,81 |
| GTPBP5 | 725502 | 1,53E-02 | 3,08 |
| CAPNS1 | 152655 | 1,59E-02 | 1,40 |
| WHSC2 | 668283 | 1,60E-02 | 1,47 |
| IMAGE:136801 | 136801 | 1,66E-02 | 1,25 |
| PTK2B | 43541 | 1,70E-02 | 1,81 |
| KPNA2 | 667727 | 1,82E-02 | 1,31 |
| BCAP29 | 725970 | 1,88E-02 | 3,23 |
| MLANA | 266361 | 1,88E-02 | 1,41 |
| BZRP | 135991 | 1,90E-02 | 1,31 |
| SAMD10 | 365801 | 0,02007 | 1,25 |
| NR2F6 | 1238492 | 2,03E-02 | 1,29 |
| ABCC8 | 1417901 | 2,11E-02 | 1,36 |
| LEMD1 | 731047 | 2,12E-02 | 2,25 |
| WDR20 | 665156 | 2,14E-02 | 1,61 |
| S100A1 | 175772 | 2,22E-02 | 1,84 |
| CD4 | 141216 | 2,36E-02 | 1,41 |
| EMILIN2 | 365521 | 2,39E-02 | 1,20 |
| IMAGE:742919 Transcribed locus | 742919 | 2,43E-02 | 1,37 |
| NCB5OR | 743367 | 2,53E-02 | 2,21 |
| IMAGE:731299 | 731299 | 2,65E-02 | 1,43 |
| INHBB | 730012 | 2,67E-02 | 3,67 |
| CKLFSF3 | 489249 | 2,69E-02 | 1,31 |
| MLL4 | 744980 | 2,78E-02 | 1,38 |
| ECM1 | 301122 | 2,79E-02 | 16,96 |
| TM4SF8 | 713647 | 0,02811 | 1,52 |
| NFIB | 416959 | 2,88E-02 | 1,52 |
| TIAM1 | 23612 | 2,96E-02 | 3,87 |
| BID | 128065 | 2,99E-02 | 1,76 |
| SPINL | 236399 | 3,00E-02 | 3,76 |
| CLDN7 | 726335 | 3,31E-02 | 1,41 |
| PSAT1 | 366388 | 3,42E-02 | 1,38 |
| IMAGE:731298 CDNA FLJ42482 fis, clone BRACE2032134 | 731298 | 3,47E-02 | 1,37 |

| | | | |
|---|---|---|---|
| IMAGE:384087 | 384087 | 3,53E-02 | 1,88 |
| CLDN7 | 300268 | 3,57E-02 | 1,40 |
| KCNK5 | 134978 | 3,62E-02 | 2,15 |
| CASP7 | 279470 | 0,03652 | 1,50 |
| LOC389906 | 740718 | 3,72E-02 | 1,20 |
| HCLS1 | 665452 | 3,93E-02 | 1,56 |
| PRDX2 | 208439 | 4,00E-02 | 1,31 |
| IMAGE:738332 Transcribed locus | 738332 | 4,07E-02 | 2,68 |
| SSH3 | 726272 | 4,08E-02 | 1,26 |
| IMAGE:136014 | 136014 | 4,20E-02 | 1,72 |
| EGFR | 135980 | 4,21E-02 | 1,72 |
| MGAT3 | 731060 | 4,24E-02 | 1,61 |
| LGALS3BP | 742100 | 4,31E-02 | 1,57 |
| NSUN4 | 364570 | 4,32E-02 | 1,50 |
| NOG | 487474 | 4,37E-02 | 1,75 |
| S100A4 | 868577 | 4,54E-02 | 3,81 |
| GADD45GIP1 | 1084386 | 4,67E-02 | 1,25 |
| DHRS6 | 364412 | 4,88E-02 | 1,30 |
| PTPN4 | 249311 | 0,04967 | 1,49 |

## Claims

1. An integrated *in vitro* method for the prediction or for the diagnosis of a specific thyroid disease in a patient comprising the steps of :

a) providing a thyroid tissue sample previously collected from the said patient;
b) quantifying, in the said thyroid tissue sample, the expression level of two or more disease-specific marker genes that are indicative of the risk of occurrence of, or of the occurrence of, a specific thyroid disease, wherein the said two or more disease-specific marker genes are selected from two or more of the groups of marker genes consisting of :

(i) marker genes specific for autoimmune thyroiditis (AT) that are contained as inserted DNA in the recombinant vectors selected from the group of vectors having the following IMAGE clone ID N° :

(i-1) 669510, 725096, 366100, 325155, 154172, 787938, 744055, 592276, 740941, 73778, 667174, 739193, 295412, 23827, 136686, 744385, 471641, 724276, 28584, 268972, 739097, 744943, 724609, 2074228, 365913, 687782, 23872, 668452, 681959, 687379, 23271, 731745, 738332, 726760, 666911, 724150, 682555, 587186, 813260, 136646, 309645, 809828, 487152, 564994, 667348, 730149, 731115, 738945, 985457, 179288, 742862, 78353, 503189, 1087348, 753215, 471574, 471696, 549054, 744657, 731357, 713422, 486708, 666794, 726830, 730829, 300268, 725364, 665376, 375746, 324079, 365271, 172982, 724562, 839101, 265558, 52226, 713922, 136919, 682119, 745106, 1877668, 1594414, 471829, 739956, 23945, 173841, 31120, 342181, 742580, 135941, 32339, 687912, 666154, 743774, 738970, 22711, 501971, 364846, 175864, 666792, 724416, 174683, 667892, 744087, 68950, 2467442, 136807, 744439, 1716286, 121551, 744797, 742061, 666778, 342181, 740158, 724596, 143759, 666315, 50202, 667514, 364959, 258666, 148796, 364324, 72744, 669263, 667533, 365177, 2055272, 731298, 512910, 470408, 137720, 2237263, 712295, 284828, 81911, 251452, 135705, 153667, 666007, 2266583, 136863, 724119, 136223, 727251, 485872, 137238, 713653, 364412, 471702, 668684, 727056, 665674, 251250, 364706, 136260, 136560, 738938, 382423, 430263, 2139164, 309697, 773192, 726272, 682088, 666367, 21716, 135689, 383945, 727289, 724554, 364729, 665649, 668152, 811582, 666551, 472111, 489945, 486304, 416744, 258865, 744050, 743016, 1846326, 509606, 666110, 51232, 3659591, 667355, 364974, 666860, 179266, 727092, 24176, 725076, 137353, 307094, 23765, 666168, 725836, 172996, 364926, 525221, 472160, 667110, 730012, 135980, 136014, 2046361, 726763, 731726, 135887, 365288, 44881, 724508, 146976, 744616, 366159, 724895, 724892, 668912, 743987, 23817, 665538, 687625, 136606, 713879, 26164, 667587, 731410, 743422, 687532, 726335, 669318, 214611, 669181, 713647, 178792, 1417901, 135766, 364469, 565317, 382738, 669188, 666361, 667117, 591907, 173288, 257555, 214008, 730938, 868169, 731404, 667112, 135773, 34007, 1521706, 687679, 730534, 33621, 664233, 135125, 155345, 208991, 177827, 667353, 745003, 731685, 79729, 25664, 139242, 666140, 270549, 173200, 364777, 740381, 795309, 731423, 49888, 741891, 232714, 743290, 133531, 665379, 669319, 258127, 1238492, 682555, 726821, 1420676, 364510, 668186, 44193, 365045, 32917, 1174287, 725371, 119772, 730834, 252382, 179214, 1288183, 628418, 382791, 2384812, 364822, 726513, 282587, 731047, 25883, 134858, 207920, 668476, 251147, 667259, 38403, 365311, 178908, 1911930, 135379, 681875, 149809, 971276, 687667, 738558, 136952, 687287, 744606, 743118, 364352, 667375, 726647, 134978, 726675, 743394, 738424, 729929, 782688, 726782, 135303, 742695, 363955, 731348, 366889, 194350, 436554, 668536, 725340, 174861, 1859532, 261500, 714414, 667361, 742739, 364741, 366032, 729510, 745490, 731021, 342551, 740457, 726596, 2032639, 136121, 745072, 665086, 738966, 731073, 135407, 471664, 301122, 738506, 136747, 714437, 485104, 179733, 713913, 742542, 743760, 26651, 743519, 196992, 665392, 364717, 731277, 667067, 136868, 564878, 743245, 668089, 136399, 383718, 741954, 742590, 544683, 134976, 365589, 730300, 381854, 731305, 2348237, 669564, 714049, 366067, 487148, 668239, 1837488, 359202, 266361, 172785, 25895, 545475, 501527, 726981, 359051, 230126, 724642, 668815, 687852, 26455, 286138, 682507, 135539, 469549, 687953, 366085, 744395, 726353, 324666, 135352, 25865, 175772, 134671, 730699, 22908, 343987, 366842, 323438, 26519, 366042, 727067, 365121 and 359461,
(i-2) 43541, 687990, 365990, 562904, 725548, 365877, 742763, 180259, 2449149, 1089025, 713623, 382760, 258747, 743961, 174396, 724306, 1505308, 726454, 665452, 665833, 1352408, 731060, 73703, 207926, 489326, 1743279, 139073, 1626951, 1578721, 188393, 156720, 781738, 1645668, 714493, 1896337, 195340, 203166, 310519, 503874, 743722, 324066, 738944, 201467, 1870594, 122874, 140405, 204686, 2497617, 2306096, 1707527, 609631, 356960, 2345206, 740931, 342256, 725473, 682207, 2242054, 868577, 727154, 279470, 813174, 252489, 2341829, 729924, 263251, 250883, 738896, 1326169, 448163, 731648, 2113771, 365349, 731023, 744980, 668584, 727496, 132933, 592598, 46991, 128065, 730037, 356841, 2238108, 823680, 292042, 504539, 485803, 340657, 590264, 1084386, 742853, 210587, 884365, 134615, 740927, 1088781, 2091591, 667239, 727430, 486401, 1286238, 742577, 1916307, 3054031, 366289, 366233, 80688, 665632, 379200, 162246, 743246, 486935, 287843, 365755, 811024, 489249, 840753, 731689, 50491, 1422194, 1251197, 1942634, 725268, 666410, 743415, 364448, 743367, 1287638, 668442, 28511, 1422791, 743224, 124701, 725612, 471568, 156183, 840687, 731751, 1705397, 26061, 666061, 725345, 740347, 346610, 131839, 123950, 2336358, 135801, 666425, 809959, 1550616, 2148946, 2728204, 366585, 667727, 116906, 288807, 345034, 768241, 667440, 1131054, 1256485, 245426, 427786,742696 and 744911,

(ii) marker genes specific for Grave's disease (GD that are contained as inserted DNA in the recombinant vectors selected from the group of vectors having the following IMAGE clone ID N° :

(ii-1) 25664, 868169, 731380, 1049033, 730942, 731616, 726578, 366388, 485872, 1662274, 2074228, 730926, 745106, 731021, 207920, 258865, 503189, 745490, 1103402, 1911930, 731305, 173296, 669181, 727491, 713879, 359461, 265558, 687667, 668239, 232366, 730814, 484535, 743774, 667587, 665086, 366889, 723950, 256177, 1859532, 665433, 985457, 564878, 376356, 383868, 471664, 724642, 23817, 724596, 487152, 669549, 155345, 347362, 666425, 24541, 290265, 667252, 725231, 343987, 705147, 1238492, 745072, 544683, 3054031, 744604, 731073, 2237263, 136919, 486447, 740620, 1420676, 151247, 738558, 731645, 179288, 667683, 135125, 723914, 666110, 381854, 365056, 738970, 724554, 301122, 382521, 341763, 136747, 364717, 484577, 668510, 145696, 594279, 725340, 712118, 82687, 252291, 666551, 713859, 359051, 786662, 741497, 731298, 729957, 743903, 682555, 30981, 666778, 135407, 742919, 378458, 743579, 258127, 365613, 357807, 383528, 486189, 738938, 279172, 665649, 172828, 738332, 549054, 742695, 213529, 667048, 307094, 436554, 324079, 365526, 471574, 742580, 666277, 2348237, 485652, 744606, 52226, 236119, 730924, 173200, 342551, 744087, 345034, 731590, 743118, 261500, 136223, 668536, 666928 and 730149, 687990, 743961, 486493, 379279 and 667313,

(iii) marker genes specific for macrofollicular adenoma (FTA-a) that are contained as inserted DNA in the recombinant vectors selected from the group of vectors having the following IMAGE clone ID N° :

(iii-1) 2348237, 135379, 743851, 1712992, 376699, 1251197, 1174287, 173841, 486221, 265345, 666564, 252382, 173371, 731073, 253386, 1285305, 342700, 179214, 3054031, 359797, 486189, 258865, 731742, 666061, 135705, 745072, 731044, 731616, 2148946, 258966, 504539, 725188, 213529, 742919, 682585, 666554, 825224, 1049033, 365045, 742837, 127507, 725198, 116906, 135875, 135773, 486282, 343987, 232366, 80162, 566862, 724544, 731115, 725454, 813174, 340657, 838611, 730534, 742763, 178255, 687336, 742542, 667683, 725473, 139073, 731202, 594279, 727289, 382423, 724556, 740457, 682276, 666361, 713879, 1016390, 667110, 359461, 25664, 743519, 486540, 1645668, 744899, 562904, 1174342, 258127, 1916307, 665693, 714109, 665649, 687270, 1942634, 1352408, 364686, 357442, 729957, 667598, 739247, 382738, 145696, 1578721, 544683, 745512, 249311, 705147, 727482, 365526, 724884, 1575056, 739432, 743224, 730779, 137238, 284828, 667048, 739237, 731047, 1326169, 364570, 174396, 665086, 235938, 666860, 743394, 758037, 740090, 594655, 760065, 731076, 1188588, 137478, ,868169, 1012990, 382069, 666425, 135203, 341763, 669549, 726894, 731277, 485104, 668239, 682207, 741406, 201467, 1088781, 382002, 236155, 731758, 364934, 235882, 178792, 365056, 668152, 665452, 136646, 666110, 668146, 347503, 744616, 177857, 258747, 665156, 1308954, 471664, 485872, 587186, 136919, 504596, 265558, 135407, 156183, 484577, 725707, 173200, 137454, 743078, 155345, 666794, 667375, 135303, 134976, 301122, 741497, 725585, 743579, 263251, 744074, 42280, 742695, 731598, 666426, 743603, 665403, 188232, 1859532, 290265, 884365, 512910, 687953, 731357, 723950, 136747, 31924, 279470, 741790, 781738, 204686, 666334, 119939, 1085884, 489326, 356841, 743982, 1707527, 35628, 730814, 738358, 667252, 740927, 342581, 137131, 2150502, 2032639, 2306096, 767779, 743182, 309697, 744374, 2238108, 136223, 666279, 726821, 383945, 726618, 23817, 188393, 741474, 25895, 135980, 136014, 741066, 382521, 731275, 136598, 2345206, 382791, 724238, 485803, 725970, 682119, 276727, 668476, 713647, 742061, 1075949, 241481, 347362, 725548, 1492440, 1117183, 726600, 489249, 2345206, 327182, 133531, 2214396, 71428, 665342, 742743, 666235, 743259, 2030501, 666703, 179534, 744983, 356960, 1561035, 726647, 1741589, 137719, 2048801, 487148, 33664, 43541, 744895, 687667, 132752 and 122874, 2032639, (iii-2) 745003, 1844968, 364324, 357201, 24392, 1420676, 687532, 724562, 729510, 725371, 24083, 738938, 730938, 148796, 137205, 174683, 21808, 3659591, 325155, 666792, 154172, 295412, 176543, 24532, 359202, 68950, 2046361, 725364, 741178, 668360, 300194, 23872, 364352, 364469, 726353, 742100, 23271, 667112, 25949, 22711, 744055, 430263, 738506, 738945, 743245, 812967, 744087, 250883, 741954, 667892, 364974, 744505, 187482, 52226, 23945, 179288, 738507, 1526058, 782688, 174861, 669295, 713859, 136868, 24300, 731726, 682251, 21716, 38789, 25883, 172693, 725340, 669141, 712295, 23641, 738944, 1716286, 723867, 726272, 23827, 357807, 136801, 135887, 2254555, 53333, 472111, 214008, 744106, 136462, 725533, 366042, 173820, 136114, 364575, 68972, 687679, 366436, 2237263, 32339, 26486, 1698236, 279150, 1101773, 666986, 669470, 257259, 667313, 128694, 178750, 2341829, 472160 and 727123,

(iv) marker genes specific for atypical follicular adenoma (FTA-aty) that are contained as inserted DNA in the recombinant vectors selected from the group of vectors having the following IMAGE clone ID N° :

(iv-1) 503874, 666128, 838611, 208991, 739097, 1089025, 725548, 739193, 155839, 28584, 202897, 743331, 743619, 725345, 345034, 235938, 729924, 681957, 30219, 773330, 665632, 2048801, 760065, 119939, 50491, 195340, 740941, 201467, 1581686, 743603, 207920, 731616, 71428, 30471, 504646, 487848, 342581, 1942634, 2238108, 740158, 42280, 196338, 742739, 743615, 1074708, 366484, 30981, 743246, 592598, 485104, 174396, 364777, 379540, 3054031, 123950, 1536968, 366100, 489326, 24587, 665086, 342256, 448163, 35628, 544818, 179534, 665156, 309645, 726454, 738896, 31924, 665452, 564878, 1286238, 379517, 177857, 744410, 730926, 809828, 288807, 740105, 366842, 146049, 713831, 140347, 666279, 341763, 665784, 24532, 33133, 365045, 1288183, 133531, 744926, 742763, 472111, 1012990, 743259, 1707527, 365121, 743270, 666315, 2484270, 471696, 364706, 364741, 80384, 590264, 285693, 173296, 1561035, 469549, 1422194, 731689, 667527, 1860115, 1087348, 204686, 1075949, 378813, 28511, 731422, 813174, 740931, 25883, 310519, 340657, 727289, 743722, 743367, 1662274, 364083, 136399, 731363, 743579, 1088781, 131839, 839101, 2449149, 1846326, 741406, 823590, 724014, 178750, 713623, 767779, 366067, 743078, 383966, 295412, 323438, 364729, 122874, 724642, 795309, 178255, 382760, 135941, 1102600, 69166, 231903, 682276, 1716286, 667533, 40304, 32917, 668452, 731060, 665820, 1624260, 1352408, 471664, 486401, 731722, 135689, 740604, 2139164, 739109, 135671, 666703, 433155, 687800, 502244, 73703, 484577, 1251197, 731202, 381032, 811024, 347503, 238840, 128065, 382791, 1870594, 258114, 731742, 727056, 781738, 487152, 731305, 731726, 342181, 745512, 356960, 365271, 78353, 730924, 742853, 2091591, 731023, 173200, 504596, 666792, 383718, 382002, 741474, 366032, 135766, 383945, 840687, 364436, 729957, 501527 and 364686, (iv-2) 50202, 738970, 128694, 741891, 666671, 366388, 146976, 687667, 587186, 667727, 669136, 744001, 666361, 668300, 713647, 2074228, 282587, 179288, 687270, 726830, 666860, 687896, 364510, 324666, 300194, 268972, 135096, 565754, 136361, 300268, 713422, 758037, 713653, 366042, 723914, 172693, 208097, 549054, 730300, 175864, 178908, 687468, 134978, 729956, 503189 and 681875,

(v) marker genes specific for microfollicular adenoma (FTA-b) that are contained as inserted DNA in the recombinant vectors selected from the group of vectors having the following IMAGE clone ID N° :

(v-1) 740931 ; 731751 ; 840687; 666128; 379540; 667259; 725585; 503874 ; 23612 ; : 3054031 ; 208097 ; 265558 ; 136976, 838611, 725707, 594279, 343987, 487474, 731128, 279172, 773330, 1089025, 725502, 2091591, 141216, 235882, 665452, 1251197, 236399, 50491, 376699, 727496, 725877, 682207, 687667, 729924, 179534, 724366, 30471, 743246, 1088781, 345034, 195340, 868577, 2238108, 2148946, 258114, 136919, 1870594, 1241974, 172828, 731348, 740158, 592598, 1326169, 809959, 741790, 725268, 119939, 135980, , 136014, 740604, 730037, 448163, 668239, 512420, 258865, 365526, 667365, 742577, 116906, 258747, 738896, 342256, 486221, 288807, 743259, 136223, 725970, 342700, 327182, 264166, 1049033, 365801, 156720, 725473, 1074708, 290265, 687379, 71428, 1624260, 486401, 669564, 133531, 232388, 726454, 1705397, 487848, 809719, 251427, 287843, 1870594, 744926, 682119, 1641901, 742901, 1707527, 742853, 33133, 664233, 504596, 364204, 285693, 25664, 174396, 743722, 209137, 310519, 42280, 249311, 1505308, 727178, 667067, 731726, 46991, 137454, 740347, 666334, 666671, 359461, 487302, 1352408, 504646, 743118, 365919, 687800, 26061, 301122, 122874, 129438, 666235, 471664, 741497, 1117183, 364526, 175772, 179214, 471568, 744606, 1103402, 1942634, 727263, 484577, 745490, 31924, 682555, 665693, 823680, 1626951, 562904, 682311, 132752, 359797, 485803, 725548, 705147, 730012, 504539, 491004, 667440, 1917430, 743774, 666110, 309697, 724416, 135303, 731019, 1287638, 742743, 740718, 1645668, 378813, 1016390, 124701, 162246, 731738, 681890, 309645, 665376, 238840, 489326, 665632, 201467, 188232, 486189, 485085, 203166, 744374, 813174, 1743279, 742763, 156183, 669549, 376356, 665086, 2449149, 137719, 139073, 357442, 745083, 724544, 132933, 665433, 2497617, 22085, 364570, 713879, 731023, 713922, 252291, 1911930, 436554, 743415, 145696, 178348, 667239, 236155, 196992, 2484742, 781738 and 210587,

(v-2) 724609, 365913, 666154, 267358, 665379, 667892, 251250, 713653, 365589, 1087348, 366042, 743146, 174654, 744657, 214008, 666007, 137005, 364436, 738558, 146976, 682175, 725533, 365121, 667112, 770858, 3659591, 469345, 154172, 731404, 730036, 135671, 739097, 739193, 743205, 811582, 364352, 725364, 668684, 725076, 666946, 136260, 971276, 2139164, 365271, 307025, 366254, 125552, 669263, 744439, 26519, 564994, 668815, 725335, 592276, 176543, 296702, 668152, 295412, 149809, 24176, 153667, 726675, 486510, 1526058, 366243, 148796, 194350, 143759, 33621, 668089 and 669319,

(vi) marker genes specific for follicular carcinoma (FTC) that are contained as inserted DNA in the recombinant vectors selected from the group of vectors having the following IMAGE clone ID N° :

(vi-1) 501476, 309009, 739193, 251452, 681957, 739097, 364448, 469549, 202897, 195340, 731380, 2345206, 23765, 471641, 502244, 713653, 486189, 740604, 682207, 740941, 469647, 743519, 743367, 966894, 727056, 2336358, 52897, 1326169, 773330, 139073, 725548, 137238, 742853, 838611, 22908, 207920, 375746, 738896, 152802, 343987, 743246, 1089025, 342256, 729924, 727496, 667514, 50491, 726454, 486401, 2238108, 730123, 342551, 1186334, 365589, 1837488, 1942634, 276727, 666986, 324066, 724014, 364083, 2449149, 744943, 258747, 276658, 265558, 731745, 726763, 28511, 235882, 1707527, 1896337, 448163, 301122, 724306, 687800, 669318, 2214396, 175268, 740347, 365271, 366074, 594279, 1942634, 366289, 665086, 1641901, 724366, 509606, 26061, 162246, 489249, 24541, 723923, 731751, 744410, 1870594, 743259, 731277, 381854, 25949, 489326, 491004, 2728204, 666703, 251147, 1870594, 484535, 258114, 26519, 487148, 726684, 149809, 731616, 252291, 71428, 364324, 156183, 25865, 1705397, 251250, 79729, 365562, 811024, 730926, 1085884, 366159, 682276, 364469, 668442, 741905, 356841, 730814, 177827, 42280, 724151, 743270, 136260,485085, 742837, 666128, 666279, 727092, 364777, 179334, 757248, 2484270, 287843, 469345, 667061, 485803, 727491, 213113, 364846, 743619, 743987, 180640, 364706, 365177, 592598, 971276, 1581686, 713671, 129438, 1117183, 345034, 741066, 1286238, 178792, 740931, 744657, 740381, 2237263, 666154, 665538, 135671, 172996, 1698236, 768241 and 188393,

(vi-2) 782688, 136014, 135980, 725335, 724150, 307094, 723752, 510318, 726578, 324079, 2139164, 143759, 739237, 152655, 136646, 309697, 384087, 365408, 376699, 134671, 609631, 238840, 1074708, 366254, 742919, 257259, 840753, 668924, 136801, 743245, 731428, 731410, 738507, 430263, 744374, 1492440, 135887, 726782, 379517 and 726849,

(vii) marker genes specific for multinodular goitres (MNG) that are contained as inserted DNA in the recombinant vectors selected from the group of vectors having the following IMAGE clone ID N° :

(vii-1) 284828, 173841, 667527, 202897, 469549, 207920, 469647, 363955, 323438, 276658, 263251, 731277, 35628, 741474, 743394, 212366, 258865, 724014, 742837, 1117183, 276727, 811582, 1743279, 666279, 666703, 725585, 714498, 525221, 739109, 731380, 382738, 1012990, 188232, 1012903, 726675, 356960, 235882, 208439, 178348, 727056, 124781, 300268, 868577, 327182, 249311, 266361, 241481, 376153, 745490, 31924, 235938, 687667, 44881, 725548, 383868, 342551, 382521, 667727, 365562, 359051, 136863, 773286, 236155, 744899, 366042, 383945, 723923, 340657, 668442, 382791, 666794, 121551, 346610, 232366, 110481, 128065, 379937, 731076, 682207, 50202, 725198, 208991, 173371, 724895, 742763, 666426, 743182, 381854, 347503, 743903, 838611, 155345, 2030501, 811024, 489326, 731645, 365521, 135671, 136747, 665433, 1705397, 731689, 724609, 740105, 731389, 1288183, 80162, 127507, 382002, 665086, 213113, 141216, 713913, 813174, 665495, 667110, 744395, 668300, 594279, 668510, 177857, 745512, 382069, 110101, 2242054, 310519, 38403, 3054031, 1188588, 665814, 667756, 173818, 731758, 2345206, 471568, 301122, 730814, 743270, 1859532, 382423, 725677, 744410, 723914, 1846326, 129438, 667514, 823680, 162246, 731115, 125552, 666367, 484535, 2113771, 667252, 744050, 562904, 666554, 667355, 137131, 760065, 727289, 731616, 230126, 725231, 135801, 512420, 594510, 40304, 1641901, 180259, 296702, 485872, 136919, 364741, 2148946, 364436, 136976, 1917430, 1049033, 264166, 741406, 196338, 1088980, 768241, 742061, 69166, 739237, 512910, 42280, 134416, 724238, 357442, 731598, 740347, 730834, 2384812, 724554, 201467, 1308954, 731305, 256177, 157847, 687336, 134615, 73778, 739851, 258966, 136801, 383718, 666334, 486221, 177775, 740457, 713608, 485085, 119772, 31120, 544683, 145696, 383528, 743619, 341763, 471664, 342581, 726647, 743982, 743290, 356841, 178255, 724812, 156183, 152847, 324066, 731742, 724884, 265558, 124701, 376983, 725188, 743016, 140405, 253386, 713879, 238840, 30471, 485803, 179266, 364885, 592276, 486189, 725473, 723950, 724537, 1645668, 137719, 1174287, 743517, 257555, 188393, 745123, 71428, 741497, 544818, 251427, 366085, 25865, 236119, 485104, 1570420, 174654, 745072, 731202, 179534, 116906 and 1942634,

(vii-2) 687625, 726782, 364324, 148796, 744505, 26164, 665668, 713837, 172828, 731726, 151247, 486356, 342181, 471641, 72744, 135689, 740941, 366032, 154172, 743987, 713422, 279172, 131839, 666551, 665392, 2055272, 729957, 258666, 743391, 53333, 109142, 743615, 133531, 309645, 68972, 682311, 666946, 194302, 26455, 744439, 739451, 137010, 669295, 325155, 731685, 172982, 738506, 51232, 78353, 742695, 1690788, 52226, 136114, 153667 and 134858, 667587,

(viii) marker genes specific for oncocytic adenoma (OTA) that are contained as inserted DNA in the recombinant vectors selected from the group of vectors having the following IMAGE clone ID N° :

(viii-1) 80688, 1870594, 731738, 1870594, 365348, 681875, 731751, 136114, 365161, 491004, 724366, 744983, 740718, 884365, 687381, 236399, 731726, 809719, 757248, 172982, 743774, 666410, 743961, 667239, 725502, 265345, 366436, 668584, 725076, 365349, 148796, 23612, 725726, 667361, 53333, 279470, 729510, 725877, 729956, 345034, 665784, 730300, 382612, 137454, 135689, 667310, 1860115, 731714, 365097, 729971, 731128, 682311, 549054, 726092, 609631, 366042, 714437, 68972, 741954, 725791, 730012, 669564, 666455, 238840, 173820, 743485, 726782, 235882, 743760, 364324, 823680, 179334, 729542, 135930, 738506, 155839, 416959, 682109, 175268, 26486, 725707, 725970, 667348, 669263, 28705, 725612, 162246, 666140, 738332, 365877, 740941, 727056, 838611, 731275, 310519, 384087, 174879, 744087, 727496, 379279, 25226, 731428, 365990, 364568, 2266583, 180082, 590264, 725836, 731648, 253386, 151247, 724562, 713837, 379517, 726454, 135887, 682088, 725340, 730606, 840753, 1422791, 723752, 669149, 741891, 669295, 382760, 208097, 665632, 731665, 486708, 285693, 1932168, 726578, 1896337, 250883, 713671, 730037, 740620, 682149, 116906, 669470, 1526058, 365090, 740245, 24541, 1326169, 486935, 365000, 727123, 1877668, 666066, 687306, 503874, 687541, 738944, 726849, 365609, 782688, 38789, 365271, 739901, 145112, 2381770, 742853, 484535, 356841, 743391, 365613, 738970, 364204, 740347, 742806, 152655, 2341829, 112819, 592598, 668089, 739956, 714049, 668684, 293916, 666128, 139242, 51232, 743118, 2728204, 565754, 727229, 2254555, 742543, 364448, 135096, 486401, 364575, 713831, 687990, 668924, 738896, 131839, 207926, 342551, 210587, 324079, 366388, 251216, 137005, 741894, 22085, 44193, 109142, 666361, 148212, 140405, 726272, 448163, 135875, 24392, 725401, 739247, 366289, 868577, 134858, 140347, 261656, 665376, 743246, 1420676, 726596, 357201, 668283, 172828, 487474, 665833, 309645, 376597, 731685, 2484742, 366089, 286138, 687912, 1536968, 666007, 667313, 1641901, 136361, 282587, 665156, 365311, 730123, 173119, 366842, 178750, 1707527, 364510, 364412, 366159, 668707, 279150, 739451, 365408, 745490, 667365, 510318, 2238108, 68950, 743043, 2306096, 740130, 669141, 743722, 22384, 682555, 682251, 669181, 743259, 208991, 342256, 744106, 666425, 738358, 726684, 365562, 745106, 665392, 731060, 687625, 23945, 1239535, 682528, 725268, 486493, 292042, 2467442, 134671, 486356, 665437, 713623, 666029, 379540, 1942634, 252489, 295412, 365121, 594279, 23827, 666517, 682479, 726779, 730829, 21808, 135450, 742100, 34007, 288807, 1016390, 114008, 366254, 713422, 730149, 469549, 135539, 665452, 725394, 665254, 759865, 667174, 744945, 726634, 172785, 2011082, 714013, 33621, 359461, 137131, 134978, 301122, 23271, 366115, 366074, 687270, 687667, 503189, 357807, 743415, 128694, 731398, 180259, 666235, 667353, 682555, 136868, 135991, 687287, 1741589, 52897, 687852, 781738, 26164, 152802, 1089025, 154172, 43541, 24300, 25949, 725677, 214611, 744439, 23872, 2032639, 730938, 743125, 2214396, 727251, 723923, 738938, 134785, 173288, 725364, 26455, 213113, 730036, 687896, 667587, 22908, 731404, 325155, 731423, 46991, 727457, 300194, 669136, 268972, 726513, 667598, 133531, 713653, 30219, 188232, 727263, 53081, 203166, 2237263, 740604, 743987, 738945, 669510, 123950, 26651, 665342, 724892, 687782, 194302, 307025, 727154, 1911930, 469647, 471641, 366032, 29541, 740224, 724150, 136598, 153667, 212366, 666778, 364526, 738966, 3659591, 22711, 1088781, 744505, 744606, 135203, 667061, 365177, 758037, 25883, 713647, 740580, 726353, 178908, 156720, 1844968, 137010, 136801, 724276, 24176, 740381, 137720, 725096, 179288 and 665668,

(viii-2) 1662274, 145696, 730926, 731115, 155345, 376356, 666794, 739109, 179534, 628418, 741406, 485872, 232388, 667110, 724895, 33133, 730834, 743270, 731380, 713913, 2048801, 485104, 731422, 124781, 731616, 724014, 668186, 731598, 1012990, 382738, 714498, 745072, 1942634, 342581, 347503, 731742, 724537, 365045, 738424, 724556, 730814, 666703, 341763, 739851, 744841, 744895, 726894, 512910, 384404, 382621, 744074, 258127, 364741, 136747, 1174342, 177967, 760065, 177857, 687468, 723950, 2384812, 504646, 740105, 365056, 137478, 378458, 753215, 724642, 731021, 3054031, 178255, 433155, 731076, 744410, 743603, 731722, 742046, 727289, 714493, 178792, 1581686, 1705397, 1308954, 366243, 667252, 731590, 724238, 730924, 504596, 544818, 35628, 486221, 381032, 501527, 258865, 741474, 724884, 276658, 1186334, 731227, 665649, 1859532, 564878, 71428, 666279, 668146, 731241, 257555, 381118, 665814, 726821, 364686, 208439, 82687, 1085884, 727296, 740457, 667527, 1671903, 201467, 135379, 795736, 1075949, 739432, 363955, 213529, 743331, 137238, 743619, 1241974, 632026, 730699, 743451, 665820, 725188, 731202, 379937, 487148, 1288183, 172996, 382069, 731277, 230126, 743982, 743579, 40304, 376983, 179266, 40134, 235938, 731689, 136863, 665433, 727067, 743078, 809959, 381854, 813174, 1846326, 382002, 2030501, 666334, 767779, 731305, 471829, 383868, 566862, 731758, 1624260, 1917430, 364777, 743519, 667355, 364729, 591907, 25865, 241481, 525221, 127507, 307094, 135352, 236119, 743811, 382791, 742061, 323438, 251147, 364706, 487152, 364717, 136686, 364885, 178348, 731363, 666085, 1174287, 667657, 347362, 743268, 196338, 173200, 486189, 665403, 487848, 745512, 731044, 472160, 713624, 177775, 173628, 594655, 744616, 364932, 157847, 744899, 681890, 745133, 713608, 740090, 727178, 743394, 471574, 471664, 486304, 731745, 366585, 1012903, 24587, 742837, 236155, 726600, 152847, 486540, 731019, 985457, 383718, 731645, 724554, 714414, 1578721, 786662, 383966, 261500, 119772, 743182, 366233, 364822, 132752, 364356, 31924, 366085, 38403, 668510, 1743279, 743290, 263251, 30471, ,486447, 729929, 2242054, 382521, 296702, 173371, 383945, 121551, 179133, 136919, 666367, 740158, 682585, 1285305, 730942, 284828, 668329, 42280, 136121, 25664, 1561035, 379200, 669549, 668452, 726618, 256177, 724596, 743016, 135671, 177827, 364469, 124701, 485652, ,667259, 712118, 346610,743220 1049033 and 430471,

(ix) marker genes specific for atypical oncocytic adenoma (OTA-aty) that are contained as inserted DNA in the recombinant vectors selected from the group of vectors having the following IMAGE clone ID N° :

(ix-1) 745003, 740130, 471641, 682088, 731404, 2254555, 2381770, 214008, 300268, 2467442, 125552, 666517, 366159, 740927, 665784, 24392, 727457, 148796, 590264, 665632, 345034, 840753, 309645, 743043, 731275, 51232, 667048, 838611, 773330, 884365, 667514, 364412, 366115, 757248, 146049, 140347, 342181, 727123, 30219, 155839, 382612, 723923, 682276, 682479, 743118, 740941, 356841, 53081, 745490, 687381, 324079, 173818, 731060, 743367, 365000, 669149, 285693, 731398, 110481, 726578, 687990, 23612, 723752, 713831, 1536968, 725076, 471702, 119939, 448163, 365271, 738506, 665674, 666128, 29541, 204686, 731665, 682251, 724126, 22908, 152802, 356960, 173119, 366289, 669141, 1420676, 682109, 301122, 357442, 174879, 868577, 25883, 288807, 366089, 471852, 162246, 742100, 743722, 666455, 265558, 744943, 379279, 668924, 203166, 194302, 729971, 724366, 682311, 667348, 365877, 731423, 713859, 730037, 23641, 713837, 376597, 135125, 669263, 123950, 172982, 1911930, 152655, 122874, 1084386, 839101, 251427, 666168, 112819, 565317, 1188588, 366889, 743485, 471568, 744505, 669510, 667361, 135689, 666564, 738332, 23765, 668584, 669319, 744087, 188393, 667587, 795309, 292042, 812967, 135450, 327182, 666946, 366032, 1707527, 667310, 52897, 137131, 366042, 738938, 725726, 667313, 665392, 1846326, 730149, 744606, 489249, 739237, 731047, 687541, 365161, 687800, 68950, 136260, 109142, 69166, 357807, 207926, 2091591, 1550616, 740381, 731714, 364575, 1286238, 364526 and 151247,

(ix-2) 724556, 668329, 173200, 174654, 731227, 1624260, 743078, 739576, 3054031 and 119772,

(x) marker genes specific for oncocytic varcinoma (OTC) that are contained as inserted DNA in the recombinant vectors selected from the group of vectors having the following IMAGE clone ID N° :

(x-1) 727123, 724366, 366289, 471641, 501476, 744943, 2237263, 155839, 140347, 743774, 1716286, 365271, 236399, 731423, 252291, 812967, 739097, 471702, 1420676, 739193, 740347, 669564, 609631, 731751, 666671, 745490, 285693, 235882, 725791, 23612, 345034, 503874, 838611, 81911, 469647, 667514, 730012, 730300, 740941, 738358, 486493, 809719, 726684, 687667, 162246, 740620, 366436, 726454, 469549, 146976, 665784, 731738, 365349, 238840, 491004, 122874, 46991, 726763, 78353, 738896, 178908, 416959, 486401, 665632, 742590, 180259, 666455, 22711, 665674, 148796, 489326, 25883, 724306, 745003, 594279, 342256, 26455, 143759, 687852, 592598, 713422, 173820, 136560, 209137, 136361, 743205, 366042, 665538, 727056, 1837488, 782688, 744087, 725877, 2238108, 257259, 725836, 666154, 743246, 971276, 448163, 666410, 137720, 1844968, 73703, 770858, 2055272, 487474, 731023, 667348, 50491, 740130, 342551, 713623, 2032639, 723923, 2728204, 176543, 781738, 839101, 32339, 731357, 738970, 364204, 1089025, 364575, 34007, 135096, 364412, 743259, 1870594, 1101773, 366233, 342290, 366067, 565317, 742853, 29541, 682528, 80688, 731398, 713831, 309697, 744983, 187482, 665452, 665376, 359051, 139242, 1707527, 731726, 145112, 744055, 282587, 136114, 1526058, 366115, 123950, 743043, 416744, 1536968, 309645, 738332, 471696, 365090, 667310, 1088781, 149809, 173119, 724416, 743146, 109142, 1239535, 668815, 364510, 738938, 741954, 668089, 151247, 729542, 665833, 549054, 116906, 666946, 135941, 472111, 809828, 135450, 26651, 2115808, 713671, 743615, 731714, 666128, 366842, 724892, 730938, 22908, 324079, 365613, 231903, 743422, 687541, 668360, 287843, 489249, 342181, 207920, 364324, 682109, 743367, 2381770, 22085, 730123, 135875, 250883, 667061, 724126, 1521706, 687679, 208097, 745106, 38789, 682479, 744606, 668684, 726272, 743451, 25949, 744001, 1870594, 179334, 364568, 357807, 590264, 137353, 207926, 731298, 173288, 565754, 69166, 668584, 364926, 154172, 725726, 725340, 23765, 687625, 743391, 292042, 2032639, 128694, 258666, 382612, 725394, 687381, 469345, 730036, 180640, 188393, 52897, 295412, 51232, 884365, 744945, 174683, 195340, 279470, 731648, 665392, 730606, 379540, 43541, 725364, 731404, 729924, 137454, 665437, 253386, 359202, 731685, 364974, 135689, 727229, 2467442, 1417901, 725707, 204686, 741891, 53081, 134785, 682555, 22384, 134858, 379279, 135539, 300194, 2214396, 687270, 731060, 174861, 366388, 738944, 365288, 251216, 545475, 726779, 136868, 743118, 135352, 180082, 2113771, 178750, 731128, 179733, 137205, 740927, 376153, 365097 and 30219,

(x-2) 731019, 366085, 258127, 382002, 714414, 486189, 725188, 1241974, 666703, 487152, 40304, 811024, 25865, 666279, 745133, 347362, 258966, 471664, 82687, 740931, 739109, 628418, 731742, 42280, 724014, 1012903, 1288183, 382423, 512910, 667527, 730942, 731021, 3054031, 724596, 179266, 742061, 667683, 743603, 71428 and 713608,

(xi) marker genes specific for papillary carcinoma (PTC) that are contained as inserted DNA in the recombinant vectors selected from the group of vectors having the following IMAGE clone ID N° :

(xi-1) 726763, 739193, 28584, 739097, 25883, 1716286, 365045, 666367, 379200, 365271, 731227, 726760, 384404, 208991, 744943, 1846326, 364729, 730926, 795736, 136686, 667117, 743517, 724151, 296702, 742837, 364777, 364706, 731616, 666986, 80384, 666007, 1837488, 383868, 40304, 366100, 744055, 136919, 230126, 1288183, 813174, 469647, 787938, 740158, 713608, 669318, 795309, 812967, 325155, 382791, 724596, 743703, 724238, 154172, 366484, 667657, 469549, 124781, 668452, 713831, 713913, 383718, 382621, 744616, 364686, 667533, 744074, 364822, 49888, 24587, 363955, 114008, 201467, 295412, 375746, 666315, 744385, 544818, 364846, 145696, 738424, 1624260, 485652, 1285305, 2484270, 194302, 770858, 366074, 823590, 724014, 379937, 194350, 727321, 366243, 68950, 364741, 731305, 1174342, 744895, 342181, 985457, 2384812, 727289, 731277, 1085884, 727296, 383966, 729924, 382738, 471574, 682585, 525221, 1102600, 665379, 753215, 359202, 1581686, 744797, 726513, 743982, 731722, 486304, 323438, 743422, 727491, 364436, 1844968, 666128, 30981, 382069, 3659591, 730829, 433155, 687953, 667176, 1671903, 364324, 726779, 668146, 743579, 26164, 740457, 307025, 667527, 135941, 744657, 665433, 731590, 378458, 723923, 136747, 136399, 744941, 365913, 730924, 376983, 669149, 682251, 2046361, 30471, 665403, 743043, 712118, 153667, 342290, 731422, 666703, 667067, 665814, 742743, 82687, 809828, 173628, 2345206, 471702, 179534, 730814, 172996, 668329, 471641, 665820, 731598, 136121, 148796, 743331, 1942634, 731758, 1352408, 309697, 731202, 472111, 501527, 78353, 741474, 738945, 178750, 743603, 724642, 33621, 364083, 381032, 745003, 366032, 971276, 177967, 609631, 725345, 745512, 71428, 485085, 743903, 744439, 743270, 738966, 196338, 731363, 743394, 366067, 727067, 745106, 31924, 724895, 34007, 744841, 52226, 731021, 682276, 687679, 135352, 1662274, 285693, 307094, 42280, 1308954, 668815, 665668, 232714, 1594414, 682119, 26455, 667110, 134785, 665674, 731380, 180640, 838611, 742046, 30219, 207920, 743016, 667259, 628418, 667252, 504646, 667361, 742061, 726675, 231903, 667892, 136863, 725533, 1917430, 137353, 744106, 714493, 32917, 714013, 342181, 501476, 730834, 79729, 666794, 2467442, 1698236, 665632, 743268, 741066, 726618, 743290, 665495, 73778, 744410, 669149, 381118, 809959, 276658, 632026, 122874, 744050, 731745, 1174287, 745123, 342581, 681890, 136462, 26651, 668186, 740090, 196992, 1578721, 745072, 669263, 729929, 726821, 740941, 1422194, 714414, 173820, 251250, 365056, 724609, 1075949, 512910, 132752, 740105, 2048801, 178255, 364717, 687379, 232388, 342181, 178908, 744604, 714498, 665342, 731241, 487152, 504158, 668476, 743619, 687800, 667355, 724812, 33133, 739432, 730938, 590264, 726634, 687532, 146049, 213529, 687896, 1626951, 284828, 1049033, 341763, 727056, 3054031, 726600, 156183, 731115, 471696, 143759, 366115, 383528, 731742, 31120, 174861, 668584, 743451, 112819, 2030501, 724556, 1101773, 137010, 2345206, 731076, 365000, 1707527, 366233, 366436, 1087348, 668360, 241481, 731404, 21716, 738938, 53333, 383945, 2055272, 682507, 1012990, 180259, 759865 and 723950,

(xi-2) 758037, 1932168, 489945, 738970, 667756, 265558, 2113771, 365562, 1712992, 739237, 135539, 159809, 268972, 135303, 1916307, 356841, 682088, 665437, 744945, 2728204, 324079, 727540, 470408, 740347, 364526, 343987, 730300, 594279, 162246, 549054, 743774, 510318, 136976, 725677, 667440, 757248, 140405, 2214396, 436554, 357807, 2306096, 668239, 365919, 665693, 125552, 725585, 823680, 359051, 1846326, 309009, 252291, 1877668, 1505308, 145105, 1741589, 327182, 188393, 743125, 132933, 52897, 731751, 359461, 173841, 212366, 768241, 324066, 730037, 235882, 668300, 2266583, 738358, 723914, 116906, 731738, 209137, 745490, 208097, 44193, 1743279, 110481, 24541, 727251, 745083, 1103402, 681957, 135689, 132935, 731128, 376153, 282587, 666361, 135875, 726636, 1641901, 1690788, 2011082, 366159, 32784, 22711, 713671, 44881, 1492440, 364448, 365526, 725707, 725454, 365348, 1896337, 251427, 725096, 137454, 727092, 357442, 725502, 152655, 668283, 136801, 43541, 667727, 725970, 266361, 135991, 365801, 1238492, 1417901, 731047, 665156, 175772, 141216, 365521, 742919, 743367, 731299, 730012, 489249, 744980, 301122, 713647, 416959, 23612, 128065, 236399, 726335, 366388, 731298, 384087, 300268, 134978, 279470, 740718, 665452, 208439, 738332, 726272, 136014, 135980, 731060, 742100, 364570, 487474, 868577, 1084386, 364412 and 249311,

whereby an expression value is provided for each quantified marker gene expression level;

c) comparing (i) the expression value obtained at step b) for each disease-specific marker gene with (ii) a control expression value for the said marker gene, whereby a deregulated expression level of each disease-specific marker gene may be determined;

d) predicting or diagnosing the occurrence of a thyroid disease in the said patient if one or more disease-specific marker genes comprised in a group of marker genes, among the groups (i) to (xi) defined at step b), has a deregulated expression level,; and

e) identifying, among the groups of disease-specific marker genes (i) to (xi) defined at step b), the group comprising the said one or more marker genes having a deregulated expression level, whereby the kind of specific thyroid disease that is predicted or diagnosed in the said patient is determined.

**2.** The integrated *in vitro* method according to claim 1, wherein, at step b), two or more markers comprised in each group (i) to (xi) are quantified.

**3.** The integrated *in vitro* method according to claim 1, wherein, at step b), twelve or more markers comprised in each group (i) to (xi) are quantified.

**4.** The integrated *in vitro* method according to claim 1, wherein, at step b), the disease-specific marker genes are selected from two or more groups of marker genes consisting of :

(i) marker genes specific for autoimmune thyroiditis (AT) that are contained as inserted DNA in the recombinant vectors selected from the group of vectors having the following IMAGE clone ID N° :

(i-1) 669510, 725096, 366100, 325155, 154172, 787938, 744055, 592276, 740941, 73778, 667174, 739193, 295412, 23827, 136686, 744385, 471641, 724276, 28584 and 268972,

(i-2) 43541, 687990, 365990, 562904, 725548, 365877, 742763, 180259, 2449149, 1089025, 713623, 382760, 258747, 743961, 174396, 724306, 1505308, 726454, 665452 and 665833,

(ii) marker genes specific for Grave's disease (GD that are contained as inserted DNA in the recombinant vectors selected from the group of vectors having the following IMAGE clone ID N° :

(ii-1) 25664, 868169, 731380, 1049033, 730942, 731616, 726578, 366388, 485872, 1662274, 2074228, 730926, 745106, 731021, 207920, 258865, 503189, 745490, 1103402 and 1911930,

(ii-2) 687990, 743961, 486493, 379279 and 667313,

(iii) marker genes specific for macrofollicular adenoma (FTA-a) that are contained as inserted DNA in the recombinant vectors selected from the group of vectors having the following IMAGE clone ID N° :

(iii-1) 2348237, 135379, 743851, 1712992, 376699, 1251197, 1174287, 173841, 486221, 265345, 666564, 252382, 173371, 731073, 253386, 1285305, 342700, 179214, 3054031 and 359797, 745003, 1844968, 364324, 357201, 24392, 1420676, 687532, 724562,
(iii-2) 729510, 725371, 24083, 738938, 730938, 148796, 137205, 174683, 21808, 3659591, 325155 and 666792,

(iv) marker genes specific for atypical follicular adenoma (FTA-aty) that are contained as inserted DNA in the recombinant vectors selected from the group of vectors having the following IMAGE clone ID N° :

(iv-1) 503874, 666128, 838611, 208991, 739097, 1089025, 725548, 739193, 155839, 28584, 202897, 743331, 743619, 725345, 345034, 235938, 729924, 681957, 30219 and 773330,
(iv-2) 50202, 738970, 128694, 741891, 666671, 366388, 146976, 687667, 587186, 667727, 669136, 744001, 666361, 668300, 713647, 2074228, 282587, 179288, 687270 and 726830,

(v) marker genes specific for microfollicular adenoma (FTA-b) that are contained as inserted DNA in the recombinant vectors selected from the group of vectors having the following IMAGE clone ID N° :

(v-1) 740931 ; 731751 ; 840687; 666128; 379540; 667259; 725585; 503874 ; 23612 ; : 3054031 ; 208097 ; 265558 ; 136976, 838611, 725707, 594279,343987,487474,731128,279172,773330,
(v-2) 724609, 365913, 666154, 267358, 665379, 667892, 251250, 713653, 365589, 1087348, 366042, 743146, 174654, 744657, 214008, 666007, 137005, 364436, 738558 and 146976,

(vi) marker genes specific for follicular carcinoma (FTC) that are contained as inserted DNA in the recombinant vectors selected from the group of vectors having the following IMAGE clone ID N° :

(vi-1) 501476, 309009, 739193, 251452, 681957, 739097, 364448, 469549, 202897, 195340, 731380, 2345206, 23765, 471641, 502244, 713653, 486189, 740604, 682207 and 740941,
(vi-2) 782688, 136014, 135980, 725335, 724150, 307094, 723752, 510318, 726578, 324079, 2139164, 143759, 739237, 152655, 136646, 309697, 384087, 365408, 376699 and 134671

(vii) marker genes specific for multinodular goitres (MNG) that are contained as inserted DNA in the recombinant vectors selected from the group of vectors having the following IMAGE clone ID N° :

(vii-1) 284828, 173841, 667527, 202897, 469549, 207920, 469647, 363955, 323438, 276658, 263251; 731277, 35628, 741474, 743394, 212366, 258865, 724014, 742837 and 1117183,
(vii-2) 687625, 726782, 364324, 148796, 744505, 26164, 665668, 713837, 172828, 731726, 151247, 486356, 342181, 471641, 72744, 135689, 740941, 366032 and 154172,

(viii) marker genes specific for oncocytic adenoma (OTA) that are contained as inserted DNA in the recombinant vectors selected from the group of vectors having the following IMAGE clone ID N° :

(viii-1) 80688, 1870594, 731738, 1870594, 365348, 681875, 731751, 136114, 365161, 491004, 724366, 744983, 740718, 884365, 687381, 236399, 731726, 809719, 757248 and 172982,
(viii-2) 1662274, 145696, 730926, 731115, 155345, 376356, 666794, 739109, 179534, 628418, 741406, 485872, 232388, 667110, 724895, 33133, 730834, 743270, 731380 and 713913,

(ix) marker genes specific for atypical oncocytic adenoma (OTA-aty) that are contained as inserted DNA in the recombinant vectors selected from the group of vectors having the following IMAGE clone ID N° :

(ix-1) 745003, 740130, 471641, 682088, 731404, 2254555, 2381770, 214008, 300268, 2467442, 125552, 666517, 366159, 740927, 665784, 24392, 727457, 148796, 590264 and 665632,
(ix-2) 724556, 668329, 173200, 174654, 731227, 1624260, 743078, 739576, 3054031 and 119772,

(x) marker genes specific for oncocytic varcinoma (OTC) that are contained as inserted DNA in the recombinant vectors selected from the group of vectors having the following IMAGE clone ID N° :

(x-1) 727123, 724366, 366289, 471641, 501476, 744943, 2237263, 155839, 140347, 743774, 1716286, 365271, 236399, 731423, 252291, 812967, 739097, 471702, 1420676 and 739193,
(x-2) 731019, 366085, 258127, 382002, 714414, 486189, 725188, 1241974, 666703, 487152, 40304, 811024, 25865, 666279, 745133, 347362, 258966, 471664, 82687 and 740931

(xi) marker genes specific for papillary carcinoma (PTC) that are contained as inserted DNA in the recombinant vectors selected from the group of vectors having the following IMAGE clone ID N° :

(xi-1) 726763, 739193, 28584, 739097, 25883, 1716286, 365045, 666367, 379200, 365271, 731227, 726760, 384404, 208991, 744943, 1846326, 364729, 730926, 795736 and 136686,
(xi-2) 758037, 1932168, 489945, 738970, 667756, 265558, 2113771, 365562, 1712992, 739237, 135539, 159809, 268972, 135303, 1916307, 356841, 682088, 665437, 744945 and 2728204.

**5.** The integrated *in vitro* method according to claim 1, wherein, at step b), the disease-specific marker genes are selected from two or more groups of marker genes consisting of :

(i) marker genes specific for autoimmune thyroiditis (AT) that are contained as inserted DNA in the recombinant vectors selected from the group of vectors having the following IMAGE clone ID N° :

(i-1) 1174287, 119772, 133531, 135689, 136919, 139242, 1420676, 148796, 154172, 155345, 1716286, 172982, 173841, 1846326, 1877668, 208991, 214008, 2266583, 22711, 2384812, 258127, 25883, 258865, 265558, 28584, 295412, 301122, 309645, 324079, 325155, 343987, 359051, 359461, 364324, 364412, 365045, 365121, 365271, 365589, 365913, 366032, 366042, 366100, 38403, 471641, 471664, 487152, 512910, 545475, 549054, 587186, 592276, 628418, 665086, 665376, 666154, 666361, 667174, 667348, 667892, 668684, 681875, 687667, 714437, 724562, 724892, 725076, 726596, 727056, 727251, 727289, 72744, 729510, 730012, 730300, 731060, 731115, 731726, 738332, 738970, 739097, 739193, 740457, 740941, 743774, 744087, 744439, 745003, 745072, 745490, 782688, 78353 and 787938,
(i-2) 1088781, 1089513, 1131054, 116906, 122874, 123950, 1251197, 1256485, 1286238, 1326169, 1352408, 140405, 1578721, 162246, 1705397, 1707527, 174396, 180259, 1870594, 188393, 1942634, 201467, 204686, 210587, 2238108, 245426, 250883, 2728204, 279470, 287843, 3054031, 310519, 342256, 345034, 356841, 356960, 366289, 379200, 382760, 427786, 448163, 46991, 485803, 486401, 489326,,, 590264, 592598, 609631, 665452, 666410, 667239, 668584, 682207, 687990, 713623, 725473, 725548, 726454, 727496, 730037, 731751, 738944, 740347, 740931, 742696, 742853, 743246, 743367, 744911, 80688, 811024, 813174, 823680 and 884365,

(ii) marker genes specific for Grave's disease (GD that are contained as inserted DNA in the recombinant vectors selected from the group of vectors having the following IMAGE clone ID N° :

(ii-1) 145696, 151247, 1662274, 173296, 341763, 486189, 594279, 730926, 731616 and 740620,

(iii) marker genes specific for macrofollicular adenoma (FTA-a) that are contained as inserted DNA in the recombinant vectors selected from the group of vectors having the following IMAGE clone ID N° :

(iii-2) 136114, 364575 and 366436,
(iii-1) 1188588, 137131, 137454, 173371, 235882, 235938, 253386, 265345, 276727, 42280, 486221, 666703, 682276, 725707, 726600, 741406, 743259, 744983 and 838611,

(iv) marker genes specific for atypical follicular adenoma (FTA-aty) that are contained as inserted DNA in the recombinant vectors selected from the group of vectors having the following IMAGE clone ID N° :

(iv-1) 1089025, 1536698, 155839, 238840, 30219, 33133, 378813, 379517, 379540, 40304, 665784, 666128, 667527, 724014, 739109, 740105, 740604 and 743270,
(iv-2) 208097, 669136 and 687468,

(v) marker genes specific for microfollicular adenoma (FTA-b) that are contained as inserted DNA in the recombinant vectors selected from the group of vectors having the following IMAGE clone ID N° :

(v-1) 1641901, 23612, 236399, 491004, 667365, 724366, 725877, 731128, 731738 and 809719,

(vi) marker genes specific for follicular carcinoma (FTC) that are contained as inserted DNA in the recombinant vectors selected from the group of vectors having the following IMAGE clone ID N° :

(vi-1) 179334, 713671, 726684 and 757248,

(vii) marker genes specific for multinodular goitres (MNG) that are contained as inserted DNA in the recombinant vectors selected from the group of vectors having the following IMAGE clone ID N° :

(vii-1) 1012990, 208439 and 36521,
(vii-2) 194302,

(viii) marker genes specific for oncocytic adenoma (OTA) that are contained as inserted DNA in the recombinant vectors selected from the group of vectors having the following IMAGE clone ID N° :

(viii-1) 112819, 180082, 365348, 366115, 667310, 713831, 726092, 730606 and 743125,
(viii-2) 430471 (SEQ ID N° 404),

**6.** The integrated *in vitro* method according to claim 1, wherein step b) comprises the steps of :

b1) providing two or more sets of nucleic acids, each nucleic acid contained in a set hybridizing specifically with a nucleic acid expression product of a disease-specific marker gene comprised in one of groups (i) to (xi);
b2) reacting the sets of nucleic acids provided at step b1) with nucleic acid expression products that are previously extracted from the thyroid tissue sample provided at step a);
b3) detecting and quantifying the nucleic acid complexes formed between (i) the sets of nucleic acids provided at step b1) and (ii) the nucleic acid expression products that are extracted from the thyroid tissue sample provided at step a);

**7.** A kit for the *in vitro* prediction or diagnosis of the occurrence of a thyroid disease in a patient, which kit comprises means for quantifying the expression level of two or more disease-specific marker genes that are indicative of the risk of occurrence of, or of the occurrence of, a specific thyroid disease, wherein the said two or more disease-specific marker genes are selected from two or more of the groups of marker genes consisting of :

(i) marker genes specific for autoimmune thyroiditis (AT) that are contained as inserted DNA in the recombinant vectors selected from the group of vectors having the following IMAGE clone ID N° already specified in claim 1;
(ii) marker genes specific for Grave's disease (GD that are contained as inserted DNA in the recombinant vectors selected from the group of vectors having the following IMAGE clone ID N° already specified in claim 1;
(iii) marker genes specific for macrofollicular adenoma (FTA-a) that are contained as inserted DNA in the recombinant vectors selected from the group of vectors having the following IMAGE clone ID N° already specified in claim1;
(iv) marker genes specific for atypical follicular adenoma (FTA-aty) that are contained as inserted DNA in the recombinant vectors selected from the group of vectors having the following IMAGE clone ID N° already specified in claim 1;
(v) marker genes specific for microfollicular adenoma (FTA-b) that are contained as inserted DNA in the recombinant vectors selected from the group of vectors having the following IMAGE clone ID N° already specified in claim 1;
(vi) marker genes specific for follicular carcinoma (FTC) that are contained as inserted DNA in the recombinant vectors selected from the group of vectors having the following IMAGE clone ID N° already specified in claim1;
(vii) marker genes specific for multinodular goitres (MNG) that are contained as inserted DNA in the recombinant vectors selected from the group of vectors having the following IMAGE clone ID N° already specified in claim1;
(viii) marker genes specific for oncocytic adenoma (OTA) that are contained as inserted DNA in the recombinant vectors selected from the group of vectors having the following IMAGE clone ID N° already specified in claim 1;
(ix) marker genes specific for atypical oncocytic adenoma (OTA-aty) that are contained as inserted DNA in the recombinant vectors selected from the group of vectors having the following IMAGE clone ID N° already specified in claim 1;

(x) marker genes specific for oncocytic varcinoma (OTC) that are contained as inserted DNA in the recombinant vectors selected from the group of vectors having the following IMAGE clone ID N° already specified in claim1; and
(xi) marker genes specific for papillary carcinoma (PTC) that are contained as inserted DNA in the recombinant vectors selected from the group of vectors having the following IMAGE clone ID N° already specified in claim 1.

**8.** A method for adapting a pharmaceutical treatment in a patient affected with a thyroid disease, wherein said method comprises the steps of :

a) performing, on at least one thyroid tissue sample collected from the said patient, the *in vitro* prediction or diagnosis method according to claim 1;
b) adapting the pharmaceutical treatment of the said patient by (i) increasing or decreasing the amounts of the pharmaceutical agent, (ii) combining the said pharmaceutical agent with one or more distinct pharmaceutical agent(s) or (iii) replacing the said pharmaceutical agent by one or more distinct pharmaceutical agent(s).

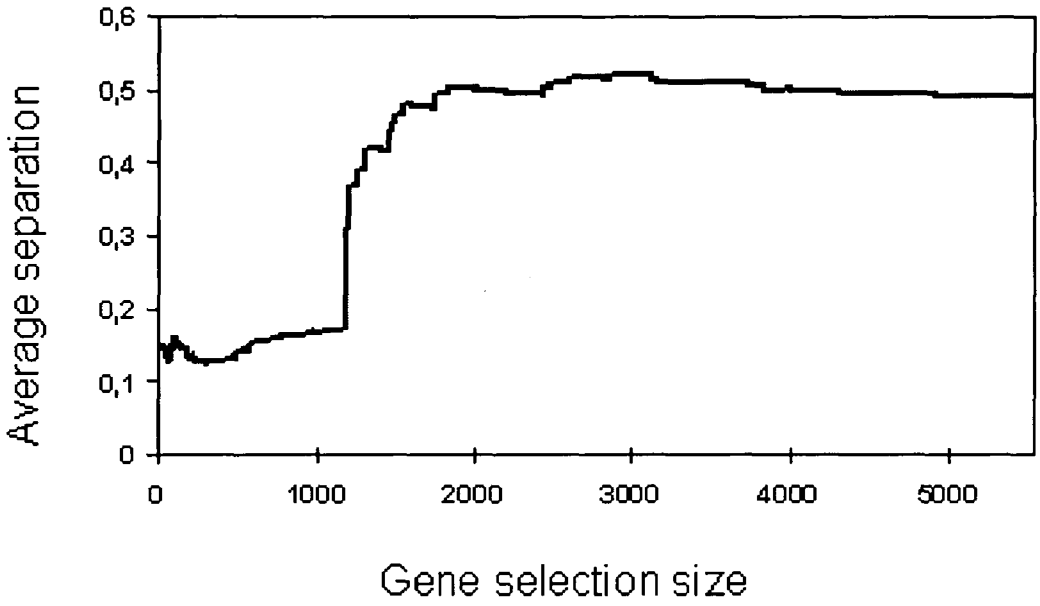
Average separation
0,6
0,5
0,4
0,3
0,2
0,1
0
0
1000
2000
3000
4000
5000
Gene selection size

Figure 1

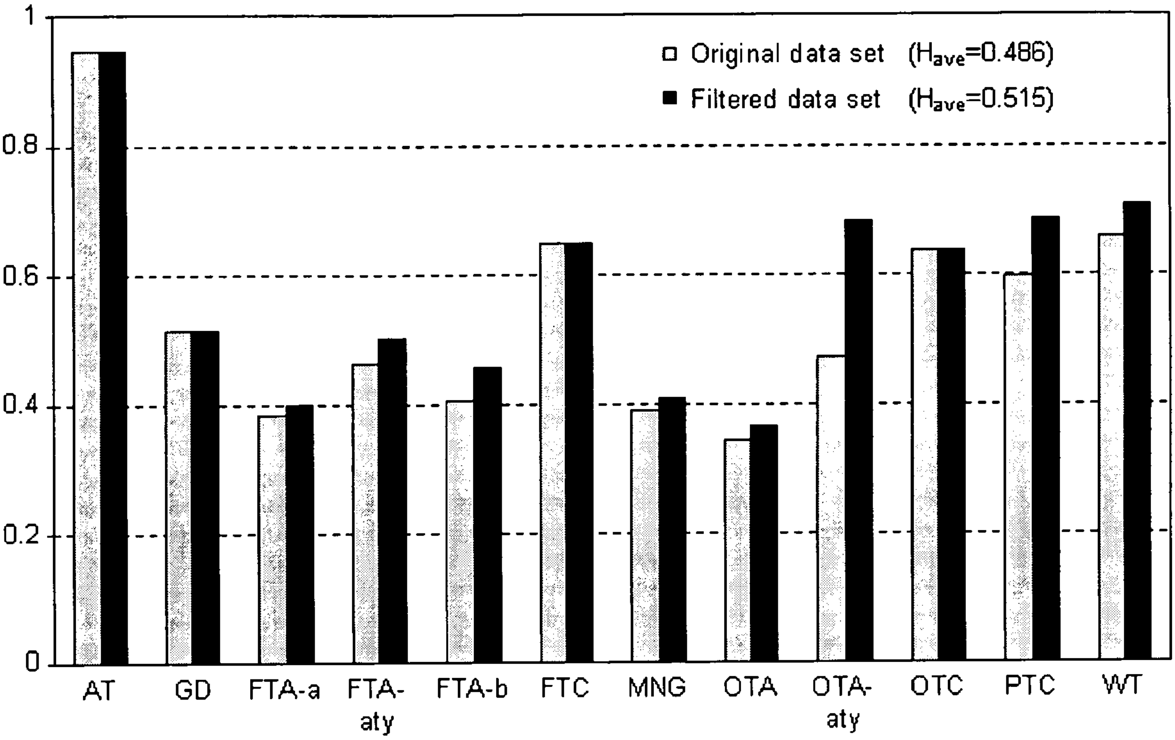
1
0.8
0.6
0.4
0.2
0
Original data set (H_ave=0.486)
Filtered data set (H_ave=0.515)
AT
GD
FTA-a
FTA-aty
FTA-b
FTC
MNG
OTA
OTA-aty
OTC
PTC
WT

**Figure 2**

European Patent Office

# PARTIAL EUROPEAN SEARCH REPORT

which under Rule 45 of the European Patent Convention shall be considered, for the purposes of subsequent proceedings, as the European search report

Application Number
EP 07 29 0373

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ESZLINGER MARKUS ET AL: "Perspectives and limitations of microarray-based gene expression profiling of thyroid tumors." ENDOCRINE REVIEWS MAY 2007, vol. 28, no. 3, March 2007 (2007-03), pages 322-338, XP009087357 ISSN: 0163-769X published online on 12.03.2007 * the whole document * | 1-8 | INV. C12Q1/68 |
| X | WO 2005/100608 A (US GOVERNMENT [US]; UNIV JOHNS HOPKINS [US]; LIBUTTI STEVEN K [US]; MA) 27 October 2005 (2005-10-27) * abstract * * page 3, line 1 - page 7, line 9; figures 1-7 * | 1-8 | |

-/--

TECHNICAL FIELDS SEARCHED (IPC)

C12Q

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| Munich | 31 July 2007 | TILKORN, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 07 29 0373

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X,D | FINLEY DAVID J ET AL: "Advancing the molecular diagnosis of thyroid nodules: defining benign lesions by molecular profiling"<br>THYROID, MARY ANN LIEBERT, NEW YORK, NY, US,<br>vol. 15, no. 6, June 2005 (2005-06), pages 562-568, XP009087256<br>ISSN: 1050-7256<br>* abstract *<br>* page 563, column 1, line 1 - page 564, column 2, paragraph 1; table 2 *<br>----- | 1-8 | |
| D,X | FINLEY DAVID J ET AL: "Discrimination of benign and malignant thyroid nodules by molecular profiling."<br>ANNALS OF SURGERY SEP 2004,<br>vol. 240, no. 3, September 2004 (2004-09), pages 425-436 ; dis, XP002444871<br>ISSN: 0003-4932<br>* abstract *<br>* page 426, column 1, paragraph 2 - page 427, column 1, paragraph 1; tables 3,4 *<br>----- | 1-8 | TECHNICAL FIELDS SEARCHED (IPC) |
| X,D | CHEVILLARD SYLVIE ET AL: "Gene expression profiling of differentiated thyroid neoplasms: diagnostic and clinical implications."<br>CLINICAL CANCER RESEARCH : AN OFFICIAL JOURNAL OF THE AMERICAN ASSOCIATION FOR CANCER RESEARCH 1 OCT 2004,<br>vol. 10, no. 19,<br>1 October 2004 (2004-10-01), pages 6586-6597, XP002444872<br>ISSN: 1078-0432<br>* abstract *<br>* page 6586, column 2, paragraph 5- - page 6589, column 2, paragraph 1; figures 2,3; tables 2-4 *<br>----- | 1-8 | |

1

EPO FORM 1503 03.82 (P04C10)

European Patent Office

INCOMPLETE SEARCH
SHEET C

Application Number
EP 07 29 0373

Claim(s) searched incompletely:
1-8

Reason for the limitation of the search:

The present claim 1 pertains to a method for the prediction or diagnosis of a specific thyroid disease in a patient which involves the quantitation of the expression level of two or more disease specific marker genes selected from 11 groups (i-xi). Each of the groups contains marker genes for a thyroid disease defined by histopathology.
The claim lacks disclosure and technical support (Art 83/84 EPC) to an extent that a meaningful search cannot be carried out over the entire scope:
The claim encompasses the expression analysis of a very large number of possible combinations of marker genes (step b)), but the application only provides two defined marker gene sets namely the sets defined in claims 1 and 4.
Some other sets are mentioned in the description (page 78 para 1, page 83 line 17-19: 258 genes; page 75 para 3: three clusters of thyroid lesions grouping the benign, malign and oncocytic classes by within group similarity and inter-group dissimilarity) but they are not defined by specific marker genes and they can thus not be searched.
In conclusion, a meaningful seach can only be carried out for methods based on the defined sets in claims 1 and 4 (Rule 45 EPC and Guidelines B-VIII, 3).
Claims 1-8 have been searched only on the basis of the above mentioned two sets of marker genes.

## ANNEX TO THE EUROPEAN SEARCH REPORT ON EUROPEAN PATENT APPLICATION NO. EP 07 29 0373

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2007

| Patent document cited in search report | Publication date | Patent family member(s) | Publication date |
|---|---|---|---|
| WO 2005100608 A | 27-10-2005 | EP 1756303 A2 | 28-02-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6892141 B
- US 5143854 A
- US 5288644 A
- US 5324633 A
- US 5432049 A
- US 5470710 A
- US 5492806 A
- US 5503980 A
- US 5510270 A
- US 5525464 A
- US 5547839 A
- US 5580732 A
- US 5661028 A
- US 5800992 A
- WO 9521265 A
- WO 9631622 A
- WO 9710365 A
- WO 9727317 A
- EP 373203 A
- EP 785280 A
- US 4843155 A, Chomczynski
- US 4683202 A, Mullis
- US 5854033 A, Lizardi
- WO 9932660 A
- US 5837832 A, Chee
- WO 9309668 A
- US 20050089862 A, Therianos


### Non-patent literature cited in the description


- **HIROKAWA et al.** *Am J Surg Pathol,* 2002, vol. 26, 1508-1514
- **FRANC et al.** *Hum Pathol,* 2003, vol. 34, 1092-1100
- **LLOYD et al.** *Am J Surg Pathol,* 2004, vol. 28, 1336-13340
- **DELELLIS et al.** *Worl Health Organization Classification of Tumours : Pathology and Genetics of Tumours of Endocrine Organs,* vol. 320
- **HERMANN M.** *Arch Pathol Lab Med,* 2002, vol. 126 (6), 710-716
- **NASCIMENTO M.** *Endocr Pathol,* 2001, vol. 12 (3), 275-279
- **OESTREICHER-KEDEM Y.** *Head neck,* 2004, vol. 26 (11), 960-966
- **HUANG et al.** *Proc. Natl. Acad Sci,* 2001, vol. 98 (26), 15044-15049
- **YANO et al.** *Clinical Cancer Research,* 2004, vol. 10, 2035-2043
- **BARIS et al.** *Oncogene,* 2005, vol. 24, 4155-4161
- **JARZAB et al.** *Cancer Research,* 2005, vol. 65 (4), 1587-1597
- **FINLAY et al.** *The Journal of Clinical Endocrinology & Metabolism,* 2004, vol. 89 (7), 3214-3223
- **GIORDANO et al.** *Clinical Cancer Research,* 2006, vol. 12, 1983-1993
- **WEBER et al.** *J Clin Endocrinol Metab,* 2005, vol. 90, 2512-2521
- **SJOLANDER, S. ; URBANICZKY, C.** *Anal. Chem.,* 1991, vol. 63, 2338-2345
- **SZABO et al.** *Curr. Opin. Struct. Biol.,* 1995, vol. 5, 699-705
- **RIVAS, G. ; MINTON, A. P.** *Trends Biochem Sci.,* 1993, vol. 18 (8), 284-7
- **HEEGAARD, N. H.** *J. Mol. Recognit. Winter,* 1998, vol. 11 (1-6), 141-8
- **HAGE, D. S. ; TWEED, S. A.** *J Chromatogr B Biomed Sci Appl,* 10 October 1997, vol. 699 (1-2), 499-525
- Current Protocols in Molecular Biology. John Wiley & Sons, 1987
- **BARANY.** *Proc. Natl. Acad. Sci. USA,* 1991, vol. 88, 189-193
- **GUATELLI et al.** *Proc. Natl. Acad. Sci. USA,* 1990, vol. 87, 1874-1878
- **KWOH et al.** *Proc. Natl. Acad. Sci. USA,* 1989, vol. 86, 1173-1177
- **LIZARDI et al.** *Bio/Technology,* 1988, vol. 6, 1197
- **LOCKHART et al.** *Nat. Biotechnol.,* 1996, vol. 14, 1675-1680
- **MCGALL et al.** *Proc. Nat. Acad. Sci. USA,* 1996, vol. 93, 13555-13460
- **HIGUCHI et al.** Kinetics PCR Analysis: Real-time Monitoring of DNA Amplification Reactions. *Bio/Technology,* 1993, vol. 11, 1026-1030
- **ISHIGURO et al.** Homogeneous quantitative Assay of Hepatitis C Virus RNA by Polymerase Chain Reaction in the Presence of a Fluorescent Intercalater. *Anal. Biochemistry,* 1995, vol. 229, 20-213

- **WITTWER et al.** Continuous Fluorescence Monitoring of Rapid cycle DNA Amplification. *Biotechniques,* 1997, vol. 22, 130-138
- **SOUTHERN, E. M.** *J. Mol. Biol.,* 1975, vol. 98, 503-517
- **SHARP, P. A. et al.** *Methods Enzymol.,* 1980, vol. 65, 750-768
- **THOMAS, P. S.** *Proc. Nat. Acad. Sci.,* 1980, vol. 77, 5201-5205
- **KELLOGG, D. E. et al.** *Anal. Biochem.,* 1990, vol. 189, 202-208
- **PANG, S. et al.** *Nature,* 1990, vol. 343, 85-89
- **BECKER-ANDRE, M.** *Meth. Mol. Cell Biol.,* 1991, vol. 2, 189-201
- **PIATAK, M. J. et al.** *BioTechniques,* 1993, vol. 14, 70-81
- **PIATAK, M. J. et al.** *Science,* 1993, vol. 259, 1749-1754
- **BERTUCCI F ; VAN HULST S ; BERNARD K ; LORIOD B ; GRANJEAUD S ; TAGETT R ; STARKEY M ; NGUYEN C ; JORDAN B ; BIRNBAUM D.** Expression scanning of an array of growth control genes in human tumor cell lines. *Oncogene,* 1999, vol. 18, 3905-3912
- **YANG YH ; DUDOIT S ; LUU P ; LIN DM ; PENG V ; NGAI J ; SPEED TP.** Normalization for cDNA microarray data: a robust composite method addressing single and multiple slide systematic variation. *Nucleic Acids Res,* 2002, vol. 30, e15
- **BENJAMINI Y ; YEKUTIELI, D.** The control of the false discovery rate in multiple testing under dependency. *ANN STAT,* 2001, vol. 29, 1165-1188
- **EISEN MB ; SPELLMAN PT ; BROWN PO ; BOTSTEIN D.** Cluster analysis and display of genome-wide expression patterns. *Proc Natl Acad Sci USA,* 1998, vol. 95, 14863-14868
- **ZEEBERG BR ; FENG W ; WANG G ; WANG MD ; FOJO AT ; SUNSHINE M ; NARASIMHAN S ; KANE DW ; REINHOLD WC ; LABABIDI S et al.** GoMiner: a resource for biological interpretation of genomic and proteomic data. *Genome Biol,* 2003, vol. 4, R28
- **SHARAN R ; SHAMIR R.** CLICK: a clustering algorithm with applications to gene expression analysis. *Proc Int Conf Intell Syst Mol Biol,* 2000, vol. 8, 307-316
- **SHARAN R ; SHAMIR R.** CLICK: a clustering algorithm with applications to gene expression analysis. *Proc Int Conf Intell Syst Mol Biol,* 2000, vol. 8, 307-316
- **PRASAD ML ; PELLEGATA NS ; KLOOS RT ; BARBACIORU C ; HUANG Y ; DE LA CHAPELLE A.** CITED1 protein expression suggests Papillary Thyroid Carcinoma in high throughput tissue microarray-based study. *Thyroid,* 2004, vol. 14, 169-175
- **YUKINAWA N ; OBA S ; KATO K ; TANIGUCHI K ; LWAO-KOIZUMI K ; TAMAKI Y ; NOGUCHI S ; ISHII S.** A multi-class predictor based on a probabilistic model: application to gene expression profiling-based diagnosis of thyroid tumors. *BMC Genomics,* 2006, vol. 7, 190
- **KUBAT M.** Machine Learning for the Detection of Oil Spills in Satellite Radar Images. *Machine Learning,* 1998, vol. 30, 195-215
- **HIROKAWA M ; CARNEY JA ; GOELLNER JR ; DELELLIS RA ; HEFFESS CS ; KATOH R ; TSUJIMOTO M ; KAKUDO K.** Observer variation of encapsulated follicular lesions of the thyroid gland. *Am J Surg Pathol,* 2002, vol. 26, 1508-1514
- **FRANC B ; DE LA SALMONIERE P ; LANGE F ; HOANG C ; LOUVEL A ; DE ROQUANCOURT A ; VILDE F ; HEJBLUM G ; CHEVRET S ; CHASTANG C.** Interobserver and intraobserver reproducibility in the histopathology of follicular thyroid carcinoma. *Hum Pathol,* 2003, vol. 34, 1092-1100
- **LLOYD RV ; ERICKSON LA ; CASEY MB ; LAM KY ; LOHSE CM ; CHAN JK ; DELELLIS RA ; HARACH HR ; KAKUDO K et al.** Observer variation in the diagnosis of follicular variant of papillary thyroid carcinoma. *Am J Surg Pathol,* 2004, vol. 28, 1336-1340
- **TANAY A ; SHARAN R ; SHAMIR R.** Discovering statistically significant biclusters in gene expression data. *Bioinformatics,* 2002, vol. 18 (1), 136-144
- **FINLEY DJ ; ARORA N ; ZHU B ; GALLAGHER L ; FAHEY TJ, 3RD.** Molecular profiling distinguishes papillary carcinoma from benign thyroid nodules. *J Clin Endocrinol Metab,* 2004, vol. 89, 3214-3223
- **GIORDANO TJ ; AU AY ; KUICK R ; THOMAS DG ; RHODES DR ; WILHELM KG, JR. ; VINCO M ; MISEK DE ; SANDERS D ; ZHU Z et al.** Delineation, functional validation, and bioinformatic evaluation of gene expression in thyroid follicular carcinomas with the PAX8-PPARG translocation. *Clin Cancer Res,* 2006, vol. 12, 1983-1993
- **BARDEN CB ; SHISTER KW ; ZHU B ; GUITER G ; GREENBLATT DY ; ZEIGER MA ; FAHEY TJ, 3RD.** Classification of follicular thyroid tumors by molecular signature: results of gene profiling. *Clin Cancer Res,* 2003, vol. 9, 1792-1800
- **ALDRED MA ; HUANG Y ; LIYANARACHCHI S ; PELLEGATA NS ; GIMM O ; JHIANG S ; DAVULURI RV ; DE LA CHAPELLE A ; ENG C.** Papillary and follicular thyroid carcinomas show distinctly different microarray expression profiles and can be distinguished by a minimum of five genes. *J Clin Oncol,* 2004, vol. 22, 3531-3539

- **FINLEY DJ ; LUBITZ CC ; WEI C ; ZHU B ; FAHEY TJ, 3RD.** Advancing the molecular diagnosis of thyroid nodules: defining benign lesions by molecular profiling. *Thyroid,* 2005, vol. 15, 562-568
- **GIORDANO TJ ; KUICK R ; THOMAS DG ; MISEK DE ; VINCO M ; SANDERS D ; ZHU Z ; CIAMPI R ; ROH M ; SHEDDEN K et al.** Molecular classification of papillary thyroid carcinoma: distinct BRAF, RAS, and RET/PTC mutation-specific gene expression profiles discovered by DNA microarray analysis. *Oncogene,* 2005, vol. 24, 6646-6656
- **NIKIFOROVA MN ; LYNCH RA ; BIDDINGER PW ; ALEXANDER EK ; DORN GW, 2ND ; TALLINI G ; KROLL TG ; NIKIFOROV YE.** RAS point mutations and PAX8-PPAR gamma rearrangement in thyroid tumors: evidence for distinct molecular pathways in thyroid follicular carcinoma. *J Clin Endocrinol Metab,* 2003, vol. 88, 2318-2326
- **CERUTTI JM ; LATINI FR ; NAKABASHI C ; DELCELO R ; ANDRADE VP ; AMADEI MJ ; MACIEL RM ; HOJAIJ FC ; HOLLIS D ; SHOEMAKER J.** Diagnosis of suspicious thyroid nodules using four protein biomarkers. *Clin Cancer Res,* 2006, vol. 12, 3311-3318

SEQUENCE LISTING

<110> INSERM (Institut National de la Sante et de la Recherche Medicale)

<120> Method for the prognosis or for the diagnosis of a thyroid disease

<130> U741EP

<160> 420

<170> PatentIn version 3.1

<210> 1

<211> 25

<212> DNA

<213> artificial sequence

<220>

<223> Primer

<400> 1
aattaattaa ccctcactaa agggt 25

<210> 2

<211> 25

<212> DNA

<213> artificial sequence

<220>

<223> primer

<400> 2
agctgtaata cgactcacta taggg 25

<210> 3

<211> 361

<212> DNA

<213> Homo sapiens

<400> 3
ttagcacaaa cacatttatt tattaaccaa agggatgatc ctaattaatc caacacactt 60

tgaaatagct gcatgtaaaa tgtttatgat aaagataatt gaacacagta gtgccgaaac 120

agcagaaaga gacagtatgg agaattgctc attggactga gcttggtcat tctcttaatt 180

aactcctgtc caaagtgatg atggaatttt tattctactt tttcatagat ccgagtacag 240

gtgacattgt tcatgacaca ctccaccact aatttcccat ctttcaattt tcttgttatt 300

gtgctttcct tcccatccca ctcctgatgc tgaaccaatg caccatctgt aaagttgcag 360

a 361

<210> 4

<211> 358

<212> DNA

<213> Homo sapiens

<220>

<221> misc_feature

<222> (62)..(62)

<223> a,t,g,c

<220>

<221> misc_feature

<222> (357)..(357)

<223> a,t,g,c

<400> 4
gaggtattga tgactttatt attctgcagg tacatgtcca ggggcccagc ctctgggcaa 60

gntaactcag ctactctttg tggctttctt catggctttt tttgtgggct gccacgccca 120

tctttatcac cagaatgagg aactcctgga agttaactgc accatcagtg ttgatatcca 180
actctttgaa ccagacgtct gcacccttttt tcctgatata ctgaggacac tcggtctcta 240
gcaatttctt caggtcatcc ctgtagacgg catggaaatt cccctttatc aagggagtac 300
ttgtggtaaa cgtcgatgat agagttcaag gctttctaca gctcggtcaa catgatng 358

<210> 5
<211> 409
<212> DNA
<213> Homo sapiens

<400> 5
tttttaccaa aaacgcaggg gatttatttg aggtttgggt gaaaaataat cttgtgggtg 60
gtggtaggcc gacagatggg gacaggaagc tgtggacgaa agccccaggt cccgtgggag 120
aggtgacagc agcaggggca cgcagccacg tgggtcccca agggaatgtg aaggcggagg 180
gctccaggcg aactggggat taaacaaata tttacaggca gcagggaagt gcccagcgca 240
cgtgacgggg gcggggcggg actttgggga gggcggggcc taacggtatc gagcgagccg 300
gttgtagacg tggtccaggt ttctgcacag gaatatcgag agcgtcatga acccgagctc 360
gcggtaaatg tggctctctg cagggacagc gcatatggtc tgcactgtg 409

<210> 6
<211> 257
<212> DNA
<213> Homo sapiens

<220>
<221> misc_feature
<222> (82)..(82)
<223> a,t,g,c

<220>
<221> misc_feature
<222> (182)..(182)

<223> a,t,g,c

<220>

<221> misc_feature

<222> (251)..(251)

<223> a,t,g,c

<220>

<221> misc_feature

<222> (255)..(255)

<223> a,t,g,c

<400> 6
taagtagcag gtttcatttt aaaacaaaaa aggttagtga agactctgtc tttcaaaaca 60

taaaaatctg cgataaaacc anttattcca tacagtgact acggtcagtt ctgagaaatg 120

acacccaggt tggcgatgtg tctcatggtt ggccttccat ggggacagtt ccaggggtgg 180

tncatctccc ccatgtgggt gatcagtttc ttcatctcgc ttgtgttgag agcagtccca 240

atcatcaccg ncttncg 257

<210> 7

<211> 344

<212> DNA

<213> Homo sapiens

<220>

<221> misc_feature

<222> (336)..(336)

<223> a,t,g,c

<220>

<221> misc_feature

<222> (343)..(343)

<223> a,t,g,c

<400> 7
gaaatggaaa tcattggtca gtttaacctg gggatttata ataaccaaac tgaatgagga 60

tatcttcata gtggaccagc atgccacgga cgagaagtat aacttcgaga tgctgcagca 120

gcacaccgtc ttccaggggc agaggctcat agcacctcag actctcaact taactgctgt 180

taatgaagct gttctgatag aaaatctgga aatatttaga aagaatggct ttgattttgt 240

tatcgatgaa aatgtcatgg atttttctca aaattgtatt cttttagctc cagtcactga 300

aagggctaaa ctgatttccc ttgccaactg gtaaancctg ggnc 344

<210> 8

<211> 361

<212> DNA

<213> Homo sapiens

<220>

<221> misc_feature

<222> (320)..(320)

<223> a,t,g,c

<220>

<221> misc_feature

<222> (332)..(332)

<223> a,t,g,c

<220>

<221> misc_feature

<222> (341)..(341)

<223> a,t,g,c

<220>

<221> misc_feature

<222> (349)..(349)

<223> a,t,g,c

<220>

<221> misc_feature

<222> (354)..(354)

<223> a,t,g,c

<400> 8
agttaaatag tacagaagac agtttactgt acaagcaagt tgtgcgttaa aaacaaacac 60

caagcaaacg ataggcaaag agttttccac ccagctccat cctctcgcca gctctgggat 120

ggttttacat cagatgagtg cagcaggtgt cacacctcag catgacaata tgtcacaaaa 180

gattggtacc cactactgac aggctcacag taacactata tcaaaacgtc ttcctttcct 240

cgtgcttcct acatcagtgt gtttgcctag gtacaacttt aacggcggcc ttgttaaatt 300

aggggcccta ctttttaccn ggccgggggt tntcttggtt nccactttng gggnctttac 360

a 361

<210> 9

<211> 418

<212> DNA

<213> Homo sapiens

<220>

<221> misc_feature

<222> (1)..(1)

<223> a,t,g,c

<220>

<221> misc_feature

<222> (304)..(304)

<223> a,t,g,c

<220>

<221> misc_feature

<222> (319)..(319)

<223> a,t,g,c

<220>

<221> misc_feature

<222> (334)..(334)

<223> a,t,g,c

<220>

<221> misc_feature

<222> (373)..(373)

<223> a,t,g,c

<220>

<221> misc_feature

<222> (416)..(416)

<223> a,t,g,c

<400> 9
ntttcggcac aggaaatctg ccctacaaaa tgctcttcat tggttgtctc tgtgagagca 60

ctgtccccac ccaacctgtc acaacggcca gaaccataca ccagagacac actggcaggt 120

taggcagtcc ttctggtgat cctattccat tccctcctgc tgcggtttct cttggcctgt 180

cctcactgga aaaacagtct ccatctcctc aaaatagttg ctgactccct ggcacccaag 240

gggccttttc catggccttc ttagggaagg caggtatgga atccatttgt tccttgttag 300

tttntttccc tcctgtttnt ctgggttatt aggntgggtc ccagggttca ggcgtggggg 360

aggggcacct ttnggggttt cccagttggc ccaggatttt tgttaggtct tcattnta 418

<210> 10

<211> 413

<212> DNA

<213> Homo sapiens

<220>

<221> misc_feature

<222> (343)..(343)

<223> a,t,g,c

<220>

<221> misc_feature

<222> (356)..(356)

<223> a,t,g,c

<220>

<221> misc_feature

<222> (384)..(384)

<223> a,t,g,c

<400> 10
ttgctcttta ttttctacat aataaaactt aatcactggg gcacagggat gcacgcttgt 60

aatcccagct actggggaag cttaggcagg acgactgttt gagctcagga gtttcagact 120

gtagtgcatt atgactgcac ctgtgaatag ccactgcatt ccagccctta gcgagacctt 180

gcctcaagac tttggggagg ctatttctta agcatatata accaatccat ttcagtgtca 240

ctatgggtgc ttgtcaaggc ccaatgctat ccaaaaacac aggtaattat tccctcccta 300

ttttgggcag gattctgctg gattctgatt ctgggccagg ggnggaaggg aggggngcca 360

ttttaagggg gagactttat tacntgcttt gctccatcac tgtgctccta aat 413

<210> 11

<211> 344

<212> DNA

<213> Homo sapiens

<400> 11
agtaaaccag aagatttgaa ggtggttaag aactgtgcaa ataccacaag atcattttgt 60

gacctcacag atgagtggag aagcacacac gaggcctatg tcaccgtcct agaaggattc 120

agcgggaaca caacgttgtt cagttgctca cacaatttct ggctggccat agacatgtct 180

tttgaaccac cagagtttga gattgttggt tttaccaacc acatttaatg tgatgggtga 240

atttccatct attgtttgag ggaaggaatt acagttttga ttttttctct cgtcattgga 300

aggaacagtt cagaggggat ttgtttaagg aaggcattaa accc 344

<210> 12

<211> 521

<212> DNA

<213> Homo sapiens

<400> 12
ttttttttt tattaagaaa gcagtttatt tcttaataac tgaatgtata ttattttaca 60

tataattaat gatcagagag aaataacagt tgctgagaac gtaaatttat gttgtacttt 120

agaattgaac agatacaacc acagattcat accaaatgca ttacttttag attattaaca 180

tattctttta cataatttca tttcacatat atggagtcca accaagatac atctggcata 240

gtaagttttc atcagtagct tcttgtataa ggtaatgcac atgtccttca atagataacg 300

gcagtcctgt cactctattt cgagtcttga ttacaccttg tagtcgctgc tcaatgtcaa 360

gaacatgggt cttggccttt tcattgacaa cttctccagt ttcattcagt ggcgctttgg 420

aatgcccttt cactgggtta ctccattcca caagaggata catgtagaaa agttcttaag 480

accctcatta aaggctctcg ctgatcacgc atcagcctca t 521

<210> 13

<211> 450

<212> DNA

<213> Homo sapiens

<400> 13

ccttgtgttg caaagccttt aattagaatg tttgtatttt ttacatcatg cataacttca 60

catttgtgat taattagtaa ttatttcaat acttgtaagc tcatctgcct cagatttaat 120

cataatacat gaattaaatt aatcaaatta aggaacagca atttagaaag aaacacactt 180

taagaaatca aaattctcaa ttcaggcagt ctgtttctat catttggtat tctactcctt 240

taaaaatttc atattgccca acaaaaagtg gttattttta ctgtttttgg agatgactga 300

acagatgaag gcatcagatg ccttcatcag ctggtatttt gcctaagatc tatttaagat 360

aacctttttct tatattttttt acttaatatt gggcataatc gtatcattca agattaagtc 420

tgggttattt ctttgataag aactgaatgt 450

<210> 14

<211> 459

<212> DNA

<213> Homo sapiens

<400> 14
tttttttttt ttttttttta tttttttttt tttttttttt tttttttttt gacaaattca 60

ctttaaaatg ttttccacat caactactta aaagaagtta cagcaatatg atcttccact 120

gctcaggaag acagagtgaa cttggatatg atgaatatta aaaacaaaca tacagagcac 180

ctgggtctgg acttgtgggc taaagaaatg cttcctgaat ttgtccgagg tgatgtgttg 240

agcaaccgtg ccacagttca ccagcagtga cactctagtt cttcagtata ttcctgttgt 300

gaacctattt ctttgaattt aatcatctgg gatgattgct gcagtgtgag tctttagcat 360

tttgaaaatc ccacagaaga gattaagaag gttcctcttc ccagaagaca ctcatataaa 420

gcatgcttct atagggttaa ctgttcagaa attgtaagg 459

<210> 15

<211> 273

<212> DNA

<213> Homo sapiens

<400> 15
ggaaaaaaat taagtgattt ttatttccaa cagttgtaaa ttaaaattgt acaacatgtt 60

ttctaatcta tgtctcatgt gtcttgctgt atgactgaag acaatgaatg tatttttaaa 120

aattatggca gacacaatat acttggtatt tacaatggcc tgtgacaaat ataatacctg 180

gaaataattt ctaattttcc acttattatt gctatttagt cacagaaaag attaacactc 240

tccaagaaat tttagcatat gtcatatata gat 273

<210> 16

<211> 515

<212> DNA

<213> Homo sapiens

<400> 16
ttttttttt tgacccatga ccatgaagtt tatagtttga ctatctgtag gaagggtaca 60

aaaatacatc atgcaacaag gtcattcatt tggaaatgta atgctttctg aaaaacttga 120

gcattcactt ttcttcactt gcctgttcta ataagagagg tttggtgtgg tgatttttca 180

atagaaagga tgttaccaat atataaataa cataaaagga tcacatgaga attaaagtga 240

agtaacttaa catacaattt tcattctaat cttatacaat aatttttctt gactattgat 300

agatcacaat aaaggtatgc tgatgatata agttatttta agactttggg gaaaaaggct 360

tccgtctctg gcattgacct tattgtggcg gaccctccaa aatcttcagc tctgcaccag 420

caggtatgct gagtcctgta tgatgacata aaccacagga gtccaggaag caaaaccacc 480

acagcgactg gctcagatct cagatgtgcc agatg 515

<210> 17

<211> 453

<212> DNA

<213> Homo sapiens

<220>

<221> misc_feature

<222> (196)..(196)

<223> a,t,g,c

<220>

<221> misc_feature

<222> (322)..(322)

<223> a,t,g,c

<220>

<221> misc_feature

<222> (349)..(349)

<223> a,t,g,c

<220>

<221> misc_feature

<222> (378)..(378)

<223> a,t,g,c

<220>

<221> misc_feature

<222> (389)..(389)

<223> a,t,g,c

<220>

<221> misc_feature

<222> (414)..(414)

<223> a,t,g,c

<220>

<221> misc_feature

<222> (423)..(423)

<223> a,t,g,c

<220>

<221> misc_feature

<222> (449)..(449)

<223> a,t,g,c

<400> 17
aatcagcaat gttttttta tttaaggcag aattagggaa aggctatgcc ctccactccc 60

cctcctccta ccactcagtc ctatcccaag tcaggcactg gaacctgctg aaaaggctga 120

tgaggggctg acaggagtgg ggcagggttc atggaccttt acctcagagc agcttaccct 180

gagctatacc cacatntcaa ggatttgggg gcgtctagat ctcatctaag ggagacacaa 240

cttttctggt cctcagatgg aaagctgggg gaaggtacca gtatttaccc ttccataaaa 300

actttggagg tctttaggag gntacccctc aggagggcta ggagaggant gacaaataca 360

gagggataga aggtcccntg gcctggttnt gccctcacac agggtattgg gttnttccag 420

ggnttgccac tcccgttgcc acaccaccnt ttg 453

<210> 18

<211> 453

<212> DNA

<213> Homo sapiens

<220>

<221> misc_feature

<222> (193)..(193)

<223> a,t,g,c

<220>

<221> misc_feature

<222> (205)..(205)

<223> a,t,g,c

<220>

<221> misc_feature

<222> (377)..(377)

<223> a,t,g,c

<220>
<221> misc_feature
<222> (400)..(400)
<223> a,t,g,c

<220>
<221> misc_feature
<222> (406)..(406)
<223> a,t,g,c

<220>
<221> misc_feature
<222> (419)..(419)
<223> a,t,g,c

<220>
<221> misc_feature
<222> (444)..(444)
<223> a,t,g,c

<400> 18
gcggcacgag gtcgaacctg agccttttga gaattgcctg ctacggcctg gcagccctgc 60
ccgagtggtc cagtgcattt cagagctggg catctttggg gtctatgtca ggcaggaaaa 120
gacactcgtg atgaacaagc acgtggggca tctacttcga accaaagcca tcgagcatgc 180
agatggtggt gtngcagcgg gagtnggcag tcctgggaca acccataccc tgtgtgaggg 240
cacaaccagg ccacgggacc ttctatcctc tgtatttgtc attcctctcc ttagccctcc 300
tgaggggtat cctcctaaag acctccaaag tttttatggg aagggtaaat actggtaact 360
ttcccccagc ttttccntct tgagggacca gaaaagttgn tgtttncctt tagatgagnt 420
tttggaaggc cccaaatcc tttnagattt ggg 453

<210> 19

<211> 415

<212> DNA

<213> Homo sapiens

<400> 19

```
catttcacca ctttgaaatc ttggaaccac tgatgtgata tcccacagag agggcaagac      60
ttcactgtga agtcaccaaa ggaactcctg cccttctagg tgccacttga gtaataactc     120
ccaactcact ggtcctctaa acctgcagca gggagatggt gtgctaggta gctacatggg     180
caggagaaga aatggaaaca aattcccgga gaatgttttt ggccatctcg atgttgttct     240
gggcaatgac caaagctttc tggatgtcct ggtaggagta cccctgactc atgaggttct     300
cgatctcact ggagaactga ggtgaggcgg tggcagcaga ggcggcagga ccactacctt     360
gctgaaagct gccagctttc cgttcagagt tgattctccg cgggaatggt tttgg          415
```

<210> 20

<211> 437

<212> DNA

<213> Homo sapiens

<220>

<221> misc_feature

<222> (82)..(82)

<223> a,t,g,c

<220>

<221> misc_feature

<222> (141)..(141)

<223> a,t,g,c

<220>

<221> misc_feature

<222> (159)..(159)

<223> a,t,g,c

<220>

<221> misc_feature

<222> (245)..(245)

<223> a,t,g,c

<220>

<221> misc_feature

<222> (257)..(257)

<223> a,t,g,c

<220>

<221> misc_feature

<222> (309)..(309)

<223> a,t,g,c

<220>

<221> misc_feature

<222> (354)..(354)

<223> a,t,g,c

<220>

<221> misc_feature

<222> (369)..(369)

<223> a,t,g,c

<220>

<221> misc_feature

<222> (386)..(386)

<223> a,t,g,c

<220>

<221> misc_feature

<222> (396)..(396)

<223> a,t,g,c

<400> 20
ggaacagggt ggacactgtg caggcttcag cttccactcc gggcaggatt caggctatct 60

gggaccgcag gacttgccag gngcacagcc ctggctcccg aggcaggcag gcaaggtgac 120

gggactggaa gcccttttca nagccttgga ggagctggnc cgtccacaag caatgagtgc 180

cactctgcag tttgcagggg atggataaac agggaaacac tgtgcattcc tcacagccaa 240

cagtntaggt cttggtnaag ccccggcgct gagctaagct caggcttttc caggggagcc 300

acgaaactnc aggtagtgat gtgcaagagt ccatcctgca gttttccagc aatnagaaac 360

tcctcgttng cggtttttgg ggaccnttgg aagttntccg cagacatttt tccatgggcc 420

gggttttaag acgaacc 437

<210> 21

<211> 466

<212> DNA

<213> Homo sapiens

<220>

<221> misc_feature

<222> (4)..(4)

<223> a,t,g,c

<220>

<221> misc_feature

<222> (55)..(55)

<223> a,t,g,c

<220>

<221> misc_feature

<222> (330)..(330)

<223> a,t,g,c

<220>

<221> misc_feature

<222> (348)..(348)

<223> a,t,g,c

<220>

<221> misc_feature

<222> (367)..(367)

<223> a,t,g,c

<220>

<221> misc_feature

<222> (402)..(402)

<223> a,t,g,c

<220>

<221> misc_feature

<222> (419)..(419)

<223> a,t,g,c

<220>

<221> misc_feature

<222> (453)..(453)

<223> a,t,g,c

<220>

<221> misc_feature

<222> (460)..(460)

<223> a,t,g,c

<400> 21
gccncagatc cagcgcccag agagacacca gagaacccac catggccccc tttgnagccc 60

ctggcttctg gcatcctgtt gttgctgtgg ctgatagccc ccagcagggc ctgcacctgt 120

gtcccacccc acccacagac ggccttctgc aattccgacc tcgtcatcag ggccaagttc 180

gtggggacac cagaagtcaa ccagaccacc ttataccagc gttatgagat caagatgacc 240

aagatgtata aagggttcca agccttaggg gatgccgctg acatccggtt cgtctacacc 300

cccgccatgg agagtgtctg cggatacttn cacaggtccc acaaccgnag cgaggagttt 360

ctcattngct ggaaaactgt aggatggact tcttgcacat tnactacctt gcagttttng 420

tgggttccct gggaacagtc tgaggtttag ttnagcggtn ggggtt 466

<210> 22

<211> 453

<212> DNA

<213> Homo sapiens

<400> 22
tttttttttg aacttttcgt gagtgtgttt attaactctt actccccacg ggcaaggctg 60

ggttttggtg agggaaagaa aagaccctcg ttgaggacat tgctgggtaa aaagccttca 120

gagagctacc tactctttgt gggtgtggta atgggcagcc ttcagcgcaa tggctaccag 180

ggatatgaat tcttgaaagt cgacctgttc atcttgatta gcatccaggc cttggaatat 240

ttcatcaatg acagctttat ctttgatatt cttgatggtg tttgcaagct cctttgtaag 300

cagctgcttc agctcaccct tagagagggt gtcaaaatgc cccttccgaa ctgagtattg 360

gtggaagata ttgacaattc cctccagatg ctcttcaagt tttgtcatct tcccagccta 420

atgttaaccc ctcaatgcac aggaatgtgg cct 453

<210> 23

<211> 544

<212> DNA

<213> Homo sapiens

<400> 23
agttgtaaac accaggcggc tttattggta gcattgcaat ctgggggtgg agcagcttta 60

ctctgaccac actcaccctc tcgctcacac tcacactcac acacacccca ggccctgtcc 120

tggctggtca ggtctggtgc agaggagctg gaagcctggt ccttccttct ggtcctcggt 180

tccccaggtt aggtaataat tcacaatgct tgactcggac tccgggctcc ctctgcacgg 240

tcataggtta actgctgcgg cgcttcatct tggctgaggt cctctgcagt ctctggatga 300

tgcgttctac ccagggctgg tctgggggtg cacagagctg gcggcccctc agtgtggtga 360

acactacagc aggcaccctg cagccatcct tgatgagaag gtagtggaag ttcctcacga 420

tgtacccagg gatgggtttc tgggtcacag acaggcagca gtcttcagca tcattggtgc 480

cactcagagt tggggctggg gaagtccaga gaaccagcag gctgagggcc agtagcaggg 540

ccat 544

<210> 24

<211> 472

<212> DNA

<213> Homo sapiens

<220>

<221> misc_feature

<222> (18)..(18)

<223> a,t,g,c

<220>

<221> misc_feature

<222> (21)..(21)

<223> a,t,g,c

<220>

<221> misc_feature

<222> (23)..(23)

<223> a,t,g,c

<220>

<221> misc_feature

<222> (388)..(388)

<223> a,t,g,c

<220>

<221> misc_feature

<222> (393)..(393)

<223> a,t,g,c

<220>

<221> misc_feature

<222> (416)..(416)

<223> a,t,g,c

<220>

<221> misc_feature

<222> (431)..(431)

<223> a,t,g,c

<220>

<221> misc_feature

<222> (442)..(442)

<223> a,t,g,c

<400> 24

gtgtttttttg tagagatnct ntnttgccat gttgccggca ctggtcttca attctgggct 60

cagacagtcc acccacctca ccctcccaga gtgctgtgat tacaggcatg agccaccatg 120

tccagcctaa ggtcttcttt ttaaaacttt gactatatta tgatttccag agatttacaa 180

aaatgcatta aagatagggt atttggtaaa tagagataga aatagatttt aattaccctt 240

tgtcagtcaa atacagatcc ctattcttaa gcatattctt atacaatatc tcattatttt 300

aaaggaccct aagtgtgacc agaccatatt taaaatgaga tgaaatatat aagaggtact 360

gatgttgtga tttgtaaaac atcacaantt aantatgagg cttttttttt agtgtncaag 420

tttttaaggt ngggaaatcc tntccagttt ataaggtagc caggttaaaa tt 472

<210> 25

<211> 254

<212> DNA

<213> Homo sapiens

<220>

<221> misc_feature

<222> (7)..(7)

<223> a,t,g,c

<220>

<221> misc_feature

<222> (132)..(132)

<223> a,t,g,c

<220>

<221> misc_feature

<222> (150)..(150)

<223> a,t,g,c

<220>

<221> misc_feature

<222> (209)..(209)

<223> a,t,g,c

<400> 25
ttgtttnttt tcttgaatat ttttgacctg cagttggttg aatctgggga tatataatct 60

atggatacag agtgctgact acatatccat attttatcta gctatcttat aaactgaaaa 120

gatttccaac tnaaaaacat gacactaaan aaaagctcat attaattgtg atgttttaca 180

aatcacaaca tcagtacctc ttatatatnt catctcattt taaatatggt ctggtcacac 240

ttagggtcct ttaa 254

<210> 26

<211> 435

<212> DNA

<213> Homo sapiens

<220>

<221> misc_feature

<222> (1)..(1)

<223> a,t,g,c

<220>

<221> misc_feature

<222> (2)..(2)

<223> a,t,g,c

<220>

<221> misc_feature

<222> (10)..(10)

<223> a,t,g,c

<220>

<221> misc_feature

<222> (383)..(383)

<223> a,t,g,c

<220>

<221> misc_feature

<222> (410)..(410)

<223> a,t,g,c

<220>

<221> misc_feature

<222> (433)..(433)

<223> a,t,g,c

<400> 26

```
nnaataatan gtggagaaat ttttattact ttgaatgttt tagaatgcag gtagaagaga      60
cccggagctc aaatagctta aataagtagg aaatctattg gctaatacaa ctggaaatgc     120
ggagataggg caggctgcag ggctggtggc tcagggctca gggggcccca actctctgtg     180
ctgctctgag gcactgcctt aaatctcagg ttggcagcac aaagctgctg agagtcccag     240
tgtcacgccc agaccccaca atgccagggg aggaagacag gttctatcta aggagagatc     300
ttccatcccc acctctgcgg acttttactc acttctcagg ggacctgcat ggggatctca     360
gggcccattc ctgaaggcag ttntttgggc agacagagtg ggaataagtn ttttacagca     420
gggcagggca gcntt                                                      435
```

<210> 27

<211> 498

<212> DNA

<213> Homo sapiens

<220>

<221> misc_feature

<222> (26)..(26)

<223> a,t,g,c

<220>

<221> misc_feature

<222> (346)..(346)

<223> a,t,g,c

<220>

<221> misc_feature

<222> (458)..(458)

<223> a,t,g,c

<220>

<221> misc_feature

<222> (464)..(464)

<223> a,t,g,c

<400> 27
gaccctggag cacttctaat gtatcnaccc atggagtcat tgttctaata atcaccaatt 60

cagactcaga tcctcgtggt ctatggagca tgctgcttgc tgtctgtgca gctcccattt 120

ccccttcttc ctgatagact tggagctgtg tgcctccact ccaaggctgc ctgcctgctg 180

taaacactat tccactctgt ctgccaacaa ctgcttcagg aatgggcctg agatcccatg 240

caggtccctg agaagtgagt aaaagtccgc agaggtgggg atggaagatc tctccttaga 300

tagaacctgt cttcctgcct ggcattgtgg ggtctgggcg tgacantggg actctcagca 360

gctttgtgct gccaaacctg agatttaaag gcagtgctca gagcagcaca gagagttggg 420

gccccctgga gcctgaggca acagcctgca gcctgccnga tctncggatt tccagttgta 480

tttaggcaat agattttc 498

<210> 28

<211> 278

<212> DNA

<213> Homo sapiens

<400> 28
gcgtgaaact tggtgaatct ttattaaact agggtccacc ccaggaggac ggctggggcg 60

gggacagggt ctcccgctgc aggctgcgcg gaggcaggag gcacggggtg gcgtggggtc 120

gcatggctgc aggcttcggc gttcagtgat tgtcgctggg cacaggggcg gcgctggtgc 180

ccacggcagc ctgcaccttc tccaccagcc cggcccactg gcgctgcatg tcttccacca 240

ggggctcgaa ccagctcttg aggcgggcct ggaaggcc 278

<210> 29

<211> 389

<212> DNA

<213> Homo sapiens

<220>

<221> misc_feature

<222> (10)..(10)

<223> a,t,g,c

<220>

<221> misc_feature

<222> (11)..(11)

<223> a,t,g,c

<220>

<221> misc_feature

<222> (17)..(17)

<223> a,t,g,c

<220>

<221> misc_feature

<222> (228)..(228)

<223> a,t,g,c

<400> 29
cagggaattn nttcatnatg gaaaaagaca actgaatgcc ctcaactgaa tgtcttcatc 60

ccctcttgcc tgaaatttcc accttcccat aggctgggga gggagtcagt tccagagcag 120

aggagggtga cagggttgag gagggacttg tgagagctag aacttggcaa aatggcctag 180

cccacccttc aaaggggaaa agagggagga acaggggatg aaaagttntc cgcagccttc 240

ccttgaactc tcccctgctg gggagggag gaggttaaag caagaccccc tgcccaggtg 300

gggagagctg ggggccaggg gagaagggga caaatggtag ggacacattc tgtttgagca 360

caatgctaaa aattctgtac atcctttgg 389

<210> 30

<211> 157

<212> DNA

<213> Homo sapiens

<220>

<221> misc_feature

<222> (1)..(1)

<223> a,t,g,c

<220>

<221> misc_feature

<222> (13)..(13)

<223> a,t,g,c

<220>

<221> misc_feature

<222> (94)..(94)

<223> a,t,g,c

<220>

<221> misc_feature

<222> (97)..(97)

<223> a,t,g,c

<220>

<221> misc_feature

<222> (127)..(127)

<223> a,t,g,c

<400> 30
nattcggcaa cgnggaaagg aaagaaacta accaacaaaa gagaaaacca aaaataatca 60

caacagaaac cagctgcccc aaaggaggcc agtngtnggg acgcagaggg tcctcagagc 120

aggagtnaca agggaggaaa gaccaaaaaa acaacca 157

<210> 31

<211> 329

<212> DNA

<213> Homo sapiens

<400> 31
tttttttttt ttttcacctt acaaatactc catgttttac tagatgtgag caaatcatta 60

agcagcaagt ttagtttggc gacaaaattg taacatctac tacaatatat cttcaaaaga 120

aatcattcac aaccacactc acatgacaag aagacctcac agactcaaaa taaataggaa 180

aaactcatac ataaatactg tcccgttcca acactgagac tctcagtcat gcagaaaaca 240

aattgaggca ttgagtggag gcaaagggca ctttacatta tcttcttgac cctctgtttt 300

ctctactgga aaatgaggat aaattctag 329

<210> 32

<211> 466

<212> DNA

<213> Homo sapiens

<220>

<221> misc_feature

<222> (2)..(2)

<223> a,t,g,c

<400> 32

anattgatca aggttttcat tgcttttatt caatgaggtt tggaacatga gaggccaaaa 60

atgaggagca cattttgggg aatcctctat aaagaatata agttaagaaa gttgtccaag 120

atgaaaataa taggaaatta ccccaactca tgccagttgg gtcttaagaa atgtccttga 180

tggcaggctt gaagaaaaga tgtcacagat tagcgtcttc ccagagtgtg aatagggcac 240

gttctcctac atgcaaacac acatgcgcac acacacaata cccttacaca cataccccag 300

cttctatgag gtaaaacttg ctgttgctga ctctcttctc tcttaacctc ttgttgcatt 360

tctccgggga cagctgggag ctgggttctc attaacactg aactctttaa ccggggcatc 420

ccggttgggc ttgcgcaagt tctgggggtg tgtcatacag gaaggg 466

<210> 33

<211> 469

<212> DNA

<213> Homo sapiens

<220>

<221> misc_feature

<222> (2)..(2)

<223> a,t,g,c

<220>

<221> misc_feature

<222> (6)..(6)

<223> a,t,g,c

<220>

<221> misc_feature

<222> (415)..(415)

<223> a,t,g,c

<400> 33
tnaganccag tccatcgcca tcgagctctc tgacctggtt gtctactgca aaccaaccag 60

caaaaccaag gacaacttag aaaatcctga cttccgagaa atccgctcct ttgtggagac 120

gaaggctgac agcatcatca gacagaagcc cgtcgacctc ctgaagtaca atcaaaaggg 180

cctgacccgc gtctacccaa agggacaaag agttgactct tcaaactacg accccttccg 240

cctctggctg tgcggttctc agatggtggc actcaatttc cagacggcag ataagtacat 300

gcagatgaat cacggcattg ttttctcttc aatggggcgc acgggctacg ttcttgcagc 360

cttgaggagc atggagggac agaggaaata tggaccccga tggccacccg agttnccaga 420

gggaaggttc cttgatggac ggttgacagt tcaaggtttt tcggtgctt 469

<210> 34

<211> 272

<212> DNA

<213> Homo sapiens

<220>

<221> misc_feature

<222> (99)..(99)

<223> a,t,g,c

<220>

<221> misc_feature

<222> (105)..(105)

<223> a,t,g,c

<220>

<221> misc_feature

<222> (108)..(108)

<223> a,t,g,c

<220>

<221> misc_feature

<222> (124)..(124)

<223> a,t,g,c

<220>

<221> misc_feature

<222> (133)..(133)

<223> a,t,g,c

<220>

<221> misc_feature

<222> (135)..(135)

<223> a,t,g,c

<220>

<221> misc_feature

<222> (144)..(144)

<223> a,t,g,c

<220>

<221> misc_feature

<222> (161)..(161)

<223> a,t,g,c

<220>

<221> misc_feature

<222> (169)..(169)

<223> a,t,g,c

<220>

<221> misc_feature

<222> (176)..(176)

<223> a,t,g,c

<220>

<221> misc_feature

<222> (199)..(199)

<223> a,t,g,c

<220>

<221> misc_feature

<222> (218)..(218)

<223> a,t,g,c

<220>

<221> misc_feature

<222> (259)..(259)

<223> a,t,g,c

<220>

<221> misc_feature

<222> (265)..(265)

<223> a,t,g,c

<400> 34

```
aacgcacgtt ttattggaag tcttggcggc agggggagtc tgcgggggca gggctgggga      60
aggggcggcg gagggggcgg tgggcgcgca ggtggagcnt ggganatntc aggtgccagg     120
ggantcctgg ccngnttcca tcgntccagg tttttctacc ncctgcggnt ggacanacgg     180
cggatggagc tgcggaaant tccctcctct tcatcggntt ccccagtcct ctgctgctgg     240
ttgaacttgc accggcatnt tctgntcagc ac                                   272
```

<210> 35

<211> 448

<212> DNA

<213> Homo sapiens

<220>

<221> misc_feature

<222> (28)..(28)

<223> a,t,g,c

<220>

<221> misc_feature

<222> (30)..(30)

<223> a,t,g,c

<220>

<221> misc_feature

<222> (56)..(56)

<223> a,t,g,c

<220>

<221> misc_feature

<222> (376)..(376)

<223> a,t,g,c

<220>

<221> misc_feature

<222> (437)..(437)

<223> a,t,g,c

<220>

<221> misc_feature

<222> (445)..(445)

<223> a,t,g,c

<400> 35
cctttcccac aggggcagcc tgtacgantn gtggcagctg ggcctcaccc cggcangctt 60

gtgcgtgacc ccctgagtgg gggaaggcag gctgttgcat ggtggcctga gcgagcagaa 120

ttcctccagg gacaatggcg tctcttggcc acatcttggt tttctgtgtg ggtctcctca 180

ccatggccaa ggcagaaagt ccaaaggaac acgacccgtt cacttacgac taccagtccc 240

tgcagatcgg aggcctcgtc atcgccggga tcctcttcat cctggggcat cctcatcgtg 300

ctgagcagaa gatgccggtg caagttcaac cagcagcaga ggactgggga acccgatgga 360

agaggaggga actttncgca gcttccattc cgccgtctgt tccacccgca ggcggttaga 420

aacaactggg agcgatngga tttcngcc 448

<210> 36

<211> 423

<212> DNA

<213> Homo sapiens

<220>

<221> misc_feature

<222> (186)..(186)

<223> a,t,g,c

<220>

<221> misc_feature

<222> (337)..(337)

<223> a,t,g,c

<220>

<221> misc_feature

<222> (354)..(354)

<223> a,t,g,c

<220>

<221> misc_feature

<222> (360)..(360)

<223> a,t,g,c

<220>

<221> misc_feature

<222> (367)..(367)

<223> a,t,g,c

<220>

<221> misc_feature

<222> (383)..(383)

<223> a,t,g,c

<400> 36
cacaaacatc cagagtttat tgctcttcac catggaaaaa aagtctcttc ccgatgggta 60

tttacaaagc ggggagatgg agtgtgaaat taaccagtgt tgaaatcgta ccacccattc 120

acatcttcag gacagtggca ggaacagcgc cgtatgcttg agggtgctgt ggtcacagct 180

cgcggncccc ggccaccgag gcccagttcc cttccagtag tgggcagctc agtgaggctg 240

gatagaaagt ctgggcaggc agtgggtaag caattgaagc aagaagagaa aggagatttt 300

cagagaacca aaggcgccct tttgaaggtt ccatctntct cacagggagg tttntgaccn 360

acagctntgg ggcttccaga ttntacccgc actcccattt tcagctttgg ggccagtctg 420

ggt 423

<210> 37

<211> 359

<212> DNA

<213> Homo sapiens

<220>

<221> misc_feature

<222> (317)..(317)

<223> a,t,g,c

<220>

<221> misc_feature

<222> (323)..(323)

<223> a,t,g,c

<220>

<221> misc_feature

<222> (335)..(335)

<223> a,t,g,c

<400> 37
agaagcagtt gtgttaatat ttagaagaag atgtatatct tccagatttt gttatatatt 60

tggcataaaa tacggcttac gttgcttaag attctcaggg ataaacttcc ttttgctaaa 120

tgcattcttt ctgcttttag aaatgtagac ataaacactc cccggagccc actcaccttt 180

tttctttttc tttttttttt ttttaacttt attccttgag ggaagcattg tttttggaga 240

gattttcttt ctgtacttcg ttttactttt cttttttttt aacttttact ctctcgaaga 300

agaggacctt cccacancca cgnaggtggg ttttnaggca aggggaaggt agccgggga 359

<210> 38

<211> 633

<212> DNA

<213> Homo sapiens

<220>

<221> misc_feature

<222> (606)..(606)

<223> a,t,g,c

<400> 38
ccttctccat catttctata tattttgggg atttttgttt tgttttgttt tttaaaaaaa 60

gatacaaaca atcttcaaag tttacctttt tggacttaag gcataacatg aaattgcgca 120

ttattctaaa gcttaatttt gaaatgaact agtttaagtg ctatcaaacc ctgtttgcgt 180

ttacattatt taaattatat agtattttat ttctgttctt gctgtaaatt ctaatgctgt 240

tcatggattg tgcaattcct atgcaatcgg tctttgcctg ctgagagtta ttaacagtgc 300

agtgtggaat ccagagtgag ctttcatttt ctcagttatc ttttcagttc aatgcatgct 360

gtttaattgt gtggaagatc caatccattt ttgttgtcca gccaccatga tgtgcatcat 420

tcatttgttt catgaaatac tccaaagcct cttgctcagt tttatctaag gctagggtct 480

ttcgaatgta tgcaatgtca tcaaaagatt gtagttctgg cattccagag ccaagcatca 540

ttgagaaaag atttatgaag agatttgcat gctgtcgaat agctagatta gccttgtaca 600

catctnctga aacctttcaa attctcttgg ctt 633

<210> 39

<211> 414

<212> DNA

<213> Homo sapiens

<400> 39
aaaatcatga actttttatt gaaagttttt ttgttctgaa aacagcagct ttgtcatatt 60

atgacagatg tgttttttat tgctgcaaaa tagttaatgt agttaaatat aagcacttag 120

aggagcaatg cctggcacac agtgaatgtt acatattagc tgagctgtta ctgttattcc 180

ttaataatta agttctgata attattcagc ctgaaaatta aaaaaaaatt agcacaaggc 240

tttgtaggta agaccattat agatctttct aaatatttaa ggtgtgtttt gtgtcaccat 300

taggtgtaga tggtcagcct tttgaacaaa ctgacactac agaagaggca ggtttcagct 360

atctaaaaat gggcaactgt taaaaagcag ttgggattgc tacgttaggg tggt 414

<210> 40

<211> 442

<212> DNA

<213> Homo sapiens

<220>

<221> misc_feature

<222> (8)..(8)

<223> a,t,g,c

<220>

<221> misc_feature

<222> (169)..(169)

<223> a,t,g,c

<220>

<221> misc_feature

<222> (221)..(221)

<223> a,t,g,c

<220>

<221> misc_feature

<222> (296)..(296)

<223> a,t,g,c

<220>

<221> misc_feature

<222> (345)..(345)

<223> a,t,g,c

<220>

<221> misc_feature

<222> (408)..(408)

<223> a,t,g,c

<220>

<221> misc_feature

<222> (418)..(418)

<223> a,t,g,c

<400> 40
atcttcanca atggcaaaac tttcttccaa taatgaacac ttctccctat gtattctcgt 60

tgatcataaa aagaattaag accgctgccc taaaaaagaa agttaaaagc acacaacttt 120

aaggaacacg catgattaag atctaaagtt catttacaag ttggtaagna cctgggctaa 180

aatttcaagt caaaaccaaa tgggtacctc aatagtcatt ngttcaactt ccaaatactg 240

taataactaa accaccttaa taccagtgaa ctcactgcaa cctctgccgt gcaggnttcg 300

ggcgattctc ctgcgctaag cttcccaagt tggctgggat tgcanggcac ctggcaccat 360

gtctgggcta attttggtag ttttaagtag agtttttttt ggaatttngg gtaaaagncg 420

gggtttcacc gggttgggcc ag 442

<210> 41

<211> 432

<212> DNA

<213> Homo sapiens

<400> 41
cctttaacaa gcatttattg agtgcctact gtgggcttac aatgcagggc ttgggatccg 60

gggctccatt gagctaggtg acagggtggc tgccttgtca cagctcatag tgcagaggtg 120

ggaaggcagt gtcctcgtcg tcaccctcac agctcagctc tagcaccaac acccgctgcc 180

caggagcagg tgcctggcct gtcagctgct gaaccagttc tgtcaccctg aggggcaggt 240

gctgggcctg cttttcaggt gaccatccgg ccgcatagag cagggctgag ccgtgcagca 300

ggatcctcac cctcaacccg tgctgctcct gaagatgaag ccagcaagcg acttccaagg 360

gttcatctca aggctggccc aagctgggta cctttcagac gggtcccaag aaggtccact 420

tcaggtgatg ga 432

<210> 42

<211> 417

<212> DNA

<213> Homo sapiens

<220>

<221> misc_feature

<222> (61)..(61)

<223> a,t,g,c

<220>

<221> misc_feature

<222> (278)..(278)

<223> a,t,g,c

<220>

<221> misc_feature

<222> (286)..(286)

<223> a,t,g,c

<220>

<221> misc_feature

<222> (340)..(340)

<223> a,t,g,c

<220>

<221> misc_feature

<222> (352)..(352)

<223> a,t,g,c

<220>

<221> misc_feature

<222> (387)..(387)

<223> a,t,g,c

<220>

<221> misc_feature

<222> (393)..(393)

<223> a,t,g,c

<220>

<221> misc_feature

<222> (413)..(413)

<223> a,t,g,c

<400> 42

```
ctcccgcttt cattatggca tcaaacagct gctgaggcac ttcccaccta ataaagtgct     60
ngaggatgga actcccttct ggtcaggtcc caaacagtgt ccccagccct tggagtttga    120
caccaaccaa gacacacacc tcctctacgt actggcagct gcaagcctgt atgcccagat    180
gcatgggctg cctggctcac aggactggac tgcactcagg gagctgctga agctgctgcc    240
acagcctgac ccccaacaga tggcccccat ctttgctnag ttaatnctag agctggcttc    300
ggcttctgct ggagtttggc cctgtagcag cagaaggaan tgaacaaagc cntggaagtc    360
tggagtgtgg ggccttcccc tggaagnctt tgnatgtttg agaaggatga tgncttg       417
```

<210> 43

<211> 450

<212> DNA

<213> Homo sapiens

<400> 43
tttttttttt ttttttaac ttgtttagat agtatatttt tctagatcaa agaaaatgta 60

gaaaatcaga ctcagcacat gccagttcat ctgtgttttg aagctgaagc aggctggatt 120

tctgagaagc acagagatgc cgactttcgt tccaagtttt ctgttcactg gaaatgaagt 180

aacagttgca ccggtaccca acccattttt cttggcaaga acagcagtca gagtctttct 240

ggagttctat gttgggtcct ggagtaaatg ctggctcaat actcagttta gtaaaagctg 300

ccatgagaaa ttatgttcca agagcgaagt agagaaggca cgatgtgtac tttttcagaa 360

agaacagcgt tgaattgttg aagctgagct ggagattaaa gaatctgaaa gttgagtatg 420

aagaaattta gcaaaaaaaa cctcgtgccg 450

<210> 44

<211> 476

<212> DNA

<213> Homo sapiens

<400> 44
aggaattaag caaccacatt tttaatcaat aaatatgaac atttgttttt taaccagtta 60

ctattaggca gaaaaaaaca gtcatttatc aagaattcaa aagataacac catcttgaaa 120

acaaagtgcc aaaaatcttt gccataaact cttcctcact tatagaacta aaagtaacaa 180

atatttactc aaaataaggc ttggaataat ctaaaaagtt ccagatatat tttaaagcta 240

taacagattt tgcacataaa gccaaaacag attgttttac acatacatag actatctgac 300

aacccaatga ataaatgatt gaatggataa attgttgagc attagtgatt acttatgttc 360

acattatggg ccaggcttac atccatttct tgttcttctc ctggcctcac tggtcccttg 420

ctcttccaag gtccttgggt gaggtggctt ccagataggc ctgggcagta aggtgc 476

<210> 45

<211> 258

<212> DNA

<213> Homo sapiens

<220>

<221> misc_feature

<222> (173)..(173)

<223> a,t,g,c

<400> 45
ttttagtaga ctgaccatgt cagaccctgt caccaatgct cagctctgta aacaatggtt 60

atttccatgt tctgggggaa ttgttaataa taggggcact agactcagcc tagaaagttc 120

tagcacatca ctatatacag tcctcaaaaa taatttatcg tactgtttct aanaaaaaag 180

gtactagggg ttgttctttt gctctgtttc catattctgg agctgcacgt gagcactttt 240

ggcttacaag tccaaaaa 258

<210> 46

<211> 612

<212> DNA

<213> Homo sapiens

<400> 46
tttttttttt tttttttcca taccattcat gctttaatga acacatatac atgaaaataa 60

gatggagagt tctagtggtt ttgcccttcc tcttcaacgg cgaaattgct atttagaagt 120

gggtagcgct cgctctctga tgggactgag ctggcataga gacaaggttg ccactgcccc 180

gtgtcctgtg atgtgacttc agagcttcca aaacgcaggc aagcacaacg gatgtctcct 240

gggcgaccat ttagcacctt tgatttcact tgggcttcat gacttctgtt gtctgttccc 300

ggcttcttac caagaaattc ttgttctttt ggttttctag attgttcttc tactcttcct 360

ctgtctccgc tgccaggtga gcccactcag gaggaggacg ctgatcagca ggaaaacaca 420

gccggccact gctacctggt actctgttgt aaatatggac gtctccatgg ttgcagccat 480

ttctgtctgt atttgaaaat ctgttgttgt gacgaggcag gaagtctcac tctcaagacg 540

gccttcgggg cttgcctgag gcttctcttc acctggaaac tgactggtct ccatttcacc 600

tgtgcatatg ag 612

<210> 47

<211> 499

<212> DNA

<213> Homo sapiens

<220>

<221> misc_feature

<222> (353)..(353)

<223> a,t,g,c

<400> 47

```
cacgagggcc agcccaatac ttaaagagag caactcctga ctccgataga gactggatgg      60
acccacaagg gtgacagccc aggcggaccg atcttcccat cccacatcct ccggcgcgat     120
gccaaaaaga ggctgacggc aactgggcct tctgcagaga aagacctccg cttcactgcc     180
ccggctggtc ccaagggtca ggaagatgga ttcatacctg ctgatgtggg gactgcccac     240
gttcatcatg gtgcctggct gccaggcaga gctctgtgac gatgacccgc cagagatccc     300
acacgccaca ttcatcatgg tgcctggctg ccaggcagag ctctgtgacg atnacccgcc     360
agagatccca cacgccacat tcaaagccat ggcctacaag gaaggaacca tgttgaactg     420
tgaatgcaag agaggtttcc gcagaataaa aagcgggtca ctctatatgc tctgtacagg     480
aaactctagc cactcgtcc                                                  499
```

<210> 48

<211> 434

<212> DNA

<213> Homo sapiens

<220>

<221> misc_feature

<222> (68)..(68)

<223> a,t,g,c

<220>

<221> misc_feature

<222> (158)..(158)

<223> a,t,g,c

<220>

<221> misc_feature

<222> (222)..(222)

<223> a,t,g,c

<220>

<221> misc_feature

<222> (224)..(224)

<223> a,t,g,c

<220>

<221> misc_feature

<222> (305)..(305)

<223> a,t,g,c

<220>

<221> misc_feature

<222> (319)..(319)

<223> a,t,g,c

<220>

<221> misc_feature

<222> (429)..(429)

<223> a,t,g,c

<400> 48
tttttgagtg ggatctcact ttaatggaga ggacgttatg acctccgggg catggctctt 60

ggcttggnaa gcccctggtt ttcatggacc tgtcgatcca cttgaggaag gcggtgacct 120

tggtgtagat cccgtacttc cccttacggg cacagctnct ccccagctga cgatgcctgt 180

cacgaatagg tgtccttgaa gcgggtgacg tgcgggcccc cntngtcccc ctggcaggca 240

tcctcctgct tggtgtcgta gccggcacag aacatgttct gggtgatgat gaagctgctg 300

gacanttgca gtgtttgcng tccacgtagg gcacctccag catcttgaag cctggtggac 360

tgccggccct tctcgtgggt gcgcccgaag ccgctcacaa tccccgtctt ctgcgtcatc 420

agcgtggant cggg 434

<210> 49

<211> 612

<212> DNA

<213> Homo sapiens

<220>

<221> misc_feature

<222> (5)..(5)

<223> a,t,g,c

<220>

<221> misc_feature

<222> (11)..(11)

<223> a,t,g,c

<220>

<221> misc_feature

<222> (164)..(164)

<223> a,t,g,c

<220>

<221> misc_feature

<222> (374)..(374)

<223> a,t,g,c

<220>

<221> misc_feature

<222> (422)..(422)

<223> a,t,g,c

<220>

<221> misc_feature

<222> (457)..(457)

<223> a,t,g,c

<220>

<221> misc_feature

<222> (479)..(479)

<223> a,t,g,c

<220>

<221> misc_feature

<222> (520)..(520)

<223> a,t,g,c

<220>

<221> misc_feature

<222> (539)..(539)

<223> a,t,g,c

<220>

<221> misc_feature

<222> (560)..(560)

<223> a,t,g,c

<220>

<221> misc_feature

<222> (576)..(576)

<223> a,t,g,c

<220>

<221> misc_feature

<222> (587)..(587)

<223> a,t,g,c

<400> 49
agcangttgt nggcggtgag gcggtgcacg aggtggaggt ggtcatcaag cacaaccggt 60

tcacaaagga gacctatgac ttcgacatcg ccgtgctccg gctcaagacc cccatcacct 120

tccgcatgaa cgtggcgcct gcctgccctc cccgagcgtg actngggccg aagtccacgc 180

tgatgacgca gaagacgggg attgtgagcg gcttcggggc gaacccacga gaaggggccg 240

gcaagtccac caggctcaag atgctggaag gtgccctacg tggaccgcaa caagctgcaa 300

gttttcccaa gcagctttaa tcattaaccc agaacaatgt tcttgttgcc gggttacgac 360

aaccaagcaa gganggatgc ctgccaaggg ggaaaagccg gggggcccga acgtcaaccc 420

gnttcaagga cacctaattt cgtgaaaggg aatcgtnagc tggggaaaag ggctttccnt 480

aaaaggggaa agtacgggat tttaaaccaa ggttaccgcn ttccttaaag tggattgana 540

ggtccttgaa aaccaggggn tttccaaagg gccaanagcc attcccnggg ggtcataaaa 600

gtcttttccc tt 612

<210> 50

<211> 225

<212> DNA

<213> Homo sapiens

<220>

<221> misc_feature

<222> (134)..(134)

<223> a,t,g,c

<400> 50
tgaaatgcaa ccaggcccaa cacacaattt tttattggtt tagcctattt cgtacttggg 60

ggttgggagc tcaatcttca gggaaaaaaa aaaaaaagag aagtattcat gtaagtagga 120

gatgagaagc agantgagaa tcacttttag gaacacagat tgggagcagg tacaggagaa 180

tcctgggtga ggatgaagaa tgacctggga tggttttgga gctag 225

<210> 51

<211> 420

<212> DNA

<213> Homo sapiens

<220>

<221> misc_feature

<222> (1)..(1)

<223> a,t,g,c

<220>

<221> misc_feature

<222> (176)..(176)

<223> a,t,g,c

<220>

<221> misc_feature

<222> (414)..(414)

<223> a,t,g,c

<400> 51
ngtggtgtga ggccatcacg gaagatgctg ctgcttctgc tgcttctggg gccaggctcc 60

gggcttggtg ctgtcgtctc tcaacatccg agcagggtta tctgtaagag tggaacctct 120

gtgaagatcg agtgccgttc cctggacttt caggccacaa ctatgttttg gtatcngtca 180

gttcccgaaa cagagtctca tgctgatggc aacttccaat gagggctcca aggccacata 240

cgagcaaggc gtcgagaagg acaagtttct catcaaccat gcaagcctga ccttgtccac 300

tctgacagtg accagtgccc atcctgaaga cagcagcttc tacatctgca gtgctaagga 360

caggggcaga tacgcagtat tttgggccca ggcacccggc tgacagtgct cgangacctg 420

<210> 52

<211> 335

<212> DNA

<213> Homo sapiens

<220>

<221> misc_feature

<222> (4)..(4)

<223> a,t,g,c

<220>

<221> misc_feature

<222> (34)..(34)

<223> a,t,g,c

<220>

<221> misc_feature

<222> (84)..(84)

<223> a,t,g,c

<220>

<221> misc_feature

<222> (87)..(87)

<223> a,t,g,c

<220>

<221> misc_feature

<222> (226)..(226)

<223> a,t,g,c

<400> 52
gcgnccgcgc tgctcctgct gctgctcggc gctngtacac cgcgcgtgtc ggacgggtcc 60

aaatgcaagt gctcccggaa gacnccnaag atccgctaca gcacgtgaag aagctggaaa 120

tgaagccaaa gtacccgcac tgcgaggaga agatggttat catcaccacc aagagcgtgt 180

ccaggtaccg aggtcaggag cactgcctgc accccaagct gcaganacca agcgcttcat 240

caagtggtac aacgcctgga acgagaagcg cagggtctac gaagaatacg ggtgaaaaac 300

ctcagaaggg aaaactccaa accagttggg agact 335

<210> 53

<211> 306

<212> DNA

<213> Homo sapiens

<220>

<221> misc_feature

<222> (87)..(87)

<223> a,t,g,c

<220>

<221> misc_feature

<222> (164)..(164)

<223> a,t,g,c

<220>

<221> misc_feature

<222> (273)..(273)

<223> a,t,g,c

<220>

<221> misc_feature

<222> (283)..(283)

<223> a,t,g,c

<400> 53
tttttttaat ctgcaaagtc ctttgcacaa gtctcccaac tggtttggag ttttcccttc 60

tgaggttttt caccctattc ttcgtanacc ctgcgcttct cgttccaggc gttgtaccac 120

ttgatgaagc gcttggtgct ctgcagcttg gggtgcaggc agtnctcctg acctcggtac 180

ctggacacgc tcttggtggt gatgataacc atcttctcct cgcagtgcgg gttactttgg 240

cttcatttcc aagcttcttc acgtcgctgt agngaatctt ggntcccttc cgggaagcac 300

ttgcaa 306

<210> 54

<211> 430

<212> DNA

<213> Homo sapiens

<220>

<221> misc_feature

<222> (73)..(73)

<223> a,t,g,c

<220>

<221> misc_feature

<222> (74)..(74)

<223> a,t,g,c

<220>

<221> misc_feature

<222> (353)..(353)

<223> a,t,g,c

<400> 54

```
cctcaactaa actctttatt tttggtttgg tttttaagta ggccatggtt gctttgaggc      60
cactgtatgg tgnnaaacta tagatgaaag agcttttatt aaaaaaggca ctgatgggag     120
acactggctg cccccgaacc tttgccctcc ctaagagcag cctctcagtt cccttttgag     180
gagccccctc acctccacca gatatgggaa gacaaatggg gtccctgttt ctggattccc     240
agcatggtcg gggaagatc tgtgacctgg gcttggcacg ggggctctca gaaggcaagg      300
cctggttgga gggtgttggt ggggggcaga aagctgagca ggattctggg gangcagccc     360
accccatatc ctagtaactc ccttttggct cttccagact tattatgcgg cttggaacca     420
tttgatgcct                                                            430
```

<210> 55

<211> 410

<212> DNA

<213> Homo sapiens

<220>

<221> misc_feature

<222> (309)..(309)

<223> a,t,g,c

<220>

<221> misc_feature

<222> (319)..(319)

<223> a,t,g,c

<220>

<221> misc_feature

<222> (354)..(354)

<223> a,t,g,c

<220>
<221> misc_feature
<222> (368)..(368)
<223> a,t,g,c

<220>
<221> misc_feature
<222> (374)..(374)
<223> a,t,g,c

<220>
<221> misc_feature
<222> (400)..(400)
<223> a,t,g,c

<220>
<221> misc_feature
<222> (408)..(408)
<223> a,t,g,c

<400> 55
cgaaaacaga ctcctctagt acttggagat cttggacgta cacctaatcc catggggcct 60
cggcttcctt aactgcaagt gagaagagga ggtctaccca ggagcctcgg gtctgatcaa 120
gggagaggcc aggcgcagtc gacctgcggc ggctccctaa gaaggtgaag caacatggga 180
acacatccta agacaggtcc tttctccacg ccatttgatg ctgtatctcc tgggagcaca 240
ggcatcaatg gtccaagccg cataataagt ctggaagagc aaaagggagt tactaggata 300
tggggtggnc tgctcccana atctgctcag ctttctgccc ccaccaacac cctnccaacc 360
aggccttncc ttcntggaga gcccccgtgg ccaagcccan gtcacagntc 410

<210> 56

<211> 547

<212> DNA

<213> Homo sapiens

<220>

<221> misc_feature

<222> (409)..(409)

<223> a,t,g,c

<220>

<221> misc_feature

<222> (467)..(467)

<223> a,t,g,c

<220>

<221> misc_feature

<222> (482)..(482)

<223> a,t,g,c

<220>

<221> misc_feature

<222> (537)..(537)

<223> a,t,g,c

<220>

<221> misc_feature

<222> (545)..(545)

<223> a,t,g,c

<400> 56
gtcttcactt ttattgaaat acaaaatgtt aaatatgcaa gctgtactaa tgaaggtgct 60

ccttgaagtt gattaaggag ggctgggctg cttgtggctt ccattttcaa ttgccaggaa 120

agaggtagaa atatcttgtc atggacagtc gttctatggt gggcatttga gctttggccc 180

ttggagtttc aaatgattgc tgtaccttcc gaaatacttc ctctaggtgg cagcaccaag 240

aatatttctg gaagcatgtg atgagttgtg tgatgaagat agagcccatt gtgctgtctc 300

tccaggacac gttgtgtggc gttgaagagc agaaagcaat gaagtccttc tccacgtggg 360

tcttgtaaac agcatcttcc tccaggttct cagatgactg tgaagaggnc acttccaagg 420

atgccggaga gtctctgacc ccacagtttc cccacgggtt tggaccnctg caggcctgga 480

cnatgatgac cctgggggtt gtccctcaaa ctgaggcagt tccggttgtt gaaaacncgg 540

aaaangt 547

<210> 57

<211> 406

<212> DNA

<213> Homo sapiens

<400> 57
accaaatatc ccccaataaa aaagctcatc cgaatatgga ggctggacca cctgagtcag 60

gagaatctac agatgccctc aagctttgtc ctcatgaaga attcctgaga ctatgtaaag 120

aaagagctga agagatctat ccaataaagg agagaaacaa ccgcacacgc ctggctctca 180

tcatatgcaa tacagagttt gaccatctgc ctccgaggaa tggagctgac tttgacatca 240

cagggatgaa ggagctactt gacgggtctg gactatagtg tagatgtaga agagaatctg 300

acagccaggg atatggagtc agcgctgagg gcatttgcta ccagaccaga gcacaagtcc 360

tctgacagca cattcttggg tactcatgtc tcatggcatc ctggga 406

<210> 58

<211> 180

<212> DNA

<213> Homo sapiens

<220>

<221> misc_feature

<222> (177)..(177)

<223> a,t,g,c

<400> 58
gtgtgcagga accagcaatg agaaggccag gaaaagaaag agctgaaaat gcagaaagcc 60

gaagagttag aacttttgga tacagcagaa gaaacagcgg ctccactacc gacctgcccc 120

cggttcgatg tccttccaag aatgaagtct ttccctggtg atggtcccct ggcctgnctt 180

<210> 59

<211> 421

<212> DNA

<213> Homo sapiens

<400> 59
caacatacgt ttttattact caaggacaac ctggacgtca ccaatgccca gcttcacggg 60

ggcatgtagt gtgactcacg gctgaacaca aaatcactgt gaagcctgtg ctacagccct 120

gggctacttt tggacacgca agaggacact cggttacatg aaacctgact tgcttctgaa 180

gacatgtgat acatgtcttt tccctgaaaa tgtttccctt ccctgtctgc catgctccca 240

acttttctgc cttccagggg cttctcactg tctgggtaac taggtcttgg gcctagctag 300

accttgggta gtggcccctc tgccacaatc agtgcctggg cctgaggctg agcttgtggc 360

ctccgaagcc tgaactggct cggagctcgt ctgtggcgcc cagggatggc ctggcttgca 420

g 421

<210> 60

<211> 458

<212> DNA

<213> Homo sapiens

<220>

<221> misc_feature

<222> (414)..(414)

<223> a,t,g,c

<400> 60

tttttttttt aaaatgccaa gataagaaac gatttattat agagagaaga aaaatttctc 60

atccaaaata tagaaatctg tacaactttg ccacaatcaa tatacatgaa ctgtacaaat 120

ttacaccagt tcataattta ccaaataaaa gatgactaac aaagttcaca aaatagatgg 180

tggtttgtgg aaaagacttt tacccaatta agtacaagga aagttacaaa ccagacctcc 240

actttctaaa aataagaagt ttactcagtc ttagaaaact acaagctagc aaatgtacag 300

agagctggct ggtgctaaca ccacagttga gacagtgtct ttttaagggt ctttttttaaa 360

gcctgttgcc atggcagatt ctggtcactt gctactttca aggccaaaaa cacnatacca 420

gggtctgacc atttccccag gtcatgctta ctagtttt 458

<210> 61

<211> 263

<212> DNA

<213> Homo sapiens

<220>

<221> misc_feature

<222> (10)..(10)

<223> a,t,g,c

<220>

<221> misc_feature

<222> (13)..(13)

<223> a,t,g,c

<220>

<221> misc_feature

<222> (246)..(246)

<223> a,t,g,c

<220>

<221> misc_feature

<222> (254)..(254)

<223> a,t,g,c

<220>

<221> misc_feature

<222> (260)..(260)

<223> a,t,g,c

<400> 61
cctcgcctan ctnttatttg ctagggaggg aagtcctagg gtggtttcag tttctcccag 60

acatacctaa atttttacat caatcctttt aaagaaaatc tgtatttcaa agaatctttc 120

tctgcagtaa atctcgcagg ggaatttgca ctattacact tgaaagttgt tattgttaac 180

cttttcggca gcttttaata ggaaagttaa acgttttaaa catggtagta ctggaaattt 240

taccanactt ttanctagcn ctt 263

<210> 62

<211> 420

<212> DNA

<213> Homo sapiens

<400> 62
gtatttttac tagggacagc gtttaccatg ttggccaggc tggtctcgaa ctcctgacct 60

gaagtgatct gccctccttg gcctcccaaa gtgctgggat tacaggtgtg agccactacg 120

cccagccttt tggtttctta tgtgtaggag aggataagtt tctttctcag agtgtgctgc 180

agccagacat aatatttctc cttggcagaa actctgctgt tccaatcata catgtagcca 240

ttgagttcaa tgtgaagatc caggagtggg cgttttcttt cttgtctcag tttctgggag 300

aggcactgca ggtacaggga tggtgagctg tgagactgct ccagcagggg acatgtccgc 360

agtacacagg ctccagaaga agtcagcttc tcggtgaact gtgcacagcc ctcgcttctg 420

<210> 63

<211> 615

<212> DNA

<213> Homo sapiens

<220>
<221> misc_feature
<222> (120)..(120)
<223> a,t,g,c

<220>
<221> misc_feature
<222> (415)..(415)
<223> a,t,g,c

<220>
<221> misc_feature
<222> (416)..(416)
<223> a,t,g,c

<220>
<221> misc_feature
<222> (459)..(459)
<223> a,t,g,c

<220>
<221> misc_feature
<222> (528)..(528)
<223> a,t,g,c

<220>
<221> misc_feature
<222> (601)..(601)
<223> a,t,g,c

<220>
<221> misc_feature
<222> (606)..(606)
<223> a,t,g,c

<400> 63
acaagttaca ggaatgttct ccaagcagca atccaaaaga gtctcaagga tccttcaaat 60
aacttcaggc tccataatgg gagaagtaaa gaacaaagac ttaaggaaca gcttggcgcn 120
tcaacaagaa ccagtgaaga aatccattca ggaatcagaa gctttttgc ctcagagcat 180
acctgaagag agatacaaga tgaagagcaa gcccctagga atctgcctga taatcgattg 240
cattggcaat gagacagagc ttcttcgaga caccttcact tccctgggct actgaagtcc 300
agaaattctt gcatctcagt atgcatggta tatcccagat tcttgggcca atttgcctgt 360
atgcccgagc accgagacta cgacagcttt gtgtgtgtcc tggtgagccg aggannccc 420
agagtgtgta tggtgtggat cagactcact cagggctcnc ctgcatcaca tcaggaggat 480
gttcatggga gattcatgcc cttatctagc agggaagcca aagatgtntt ttattcagaa 540
ctatgtggtg tcagagggcc agctggagga cagcagcctc tttggaggtg gatggggcca 600
ncgatnaaga atgtg 615

<210> 64
<211> 660
<212> DNA
<213> Homo sapiens

<220>
<221> misc_feature
<222> (434)..(434)
<223> a,t,g,c

<400> 64
gacgttcacc aacattgttc tcctgtcacc aggacaggac gcggcattca caacggaaga 60
aaacggagca tcgtgtggcg ggaagggatc atgcgggtgg gaacatcggg aggcctaggc 120

tcaggatccc ggcacaccac agcggtgctg gaggagaaga ggcctcgagc gagggcaggg 180
gacggcaccg agagttgcca ggaggtggct caccgcccag tgccccgggg gccctccagg 240
tcgggccggg ctgtggagaa ggggtgtaag cggtcacgac cccgctcctt gcagcctctg 300
gcctccttat gtctggaggg ctcccatttg attcagccca accctgcccg ctagcgtctg 360
gggataaggg aagactcggc ccgcctcgac agaaaatggg gtctcagcac tcgctgggtg 420
acccgcggga gagngcaaag gagggctccc aagtccgttc tgcagcactg gggcagggaa 480
cagacccagg atcctgggaa tcctcttctg cctagctttg cctgcctgcc agagcagggc 540
ctgcgatttg ggtgctgtga ccgtccgggg gcgcgggaag ggcaagggag gcggatctct 600
gaagttccgc ccaacttcgc tcctgatacc caaggtcaga gagggccagc tgggggcggg 660

<210> 65
<211> 510
<212> DNA
<213> Homo sapiens

<220>
<221> misc_feature
<222> (224)..(224)
<223> a,t,g,c

<220>
<221> misc_feature
<222> (251)..(251)
<223> a,t,g,c

<220>
<221> misc_feature
<222> (374)..(374)
<223> a,t,g,c

<220>

<221> misc_feature

<222> (383)..(383)

<223> a,t,g,c

<220>

<221> misc_feature

<222> (442)..(442)

<223> a,t,g,c

<400> 65
```
tttttttttc tcaggaaact ttttaatgaa agtgtcggcc acggtggtgt gtaggtggct      60

gagctcagat tgcagctgct aagacaccag ccacttacca agagaaagcc aggctgcttc     120

aaacccaggg cccaaggcaa aaaagcatca cttccggccg gggagtctgg aagccacgcc     180

ttgtgggagg tcacactggc atctaggcct tcgcctgcat tgcngaagga gagccaggtc     240

cccctcctgg nagaacgctg cgttccccag ccccacaccg gctttgccac cacacaggct     300

gttgaggcag gaggtgggta agacgtagct gtagacccaa aggcaaccac cagccctggg     360

gaccctgcgg gagnagggag canttttttag gaacatggga aaagtgtggt tcattccctt    420

ctttaggaca gcacacttcc tnacttaatt aaaaattctt ttggggggaa ggaggtttgg     480

gggagggatt aaaaaatttg ggcacagttt                                      510
```

<210> 66

<211> 463

<212> DNA

<213> Homo sapiens

<220>

<221> misc_feature

<222> (91)..(91)

<223> a,t,g,c

<220>

<221> misc_feature

<222> (350)..(350)

<223> a,t,g,c

<400> 66
cttgctgatg acttgtttta aaactttcat cctaaataac cttttgatac ttgaatattt 60

gtaagtttta tacatagttt ctaatttttt nccgaacaga tccagatacc taataagatg 120

ctggaatgta atccctggac aatccgtgtc ctggcagcat ttggtcttcc tctaagcgcc 180

tggctccgct gttctcagga gtgggttctg aagtctctgg agaacaggat acgtggaggg 240

ttaggaaggg gccaggccta gagacgggag actccctccc ggagcaggtg gaggcacagg 300

accgttcgct accccatctg ccggcacctg cgggggagcc cagggcattn tttgttaaac 360

cctcctggac cacctgggtt caaaggaaaa cagaagcatg ggagggccgc caattttttt 420

caaggaaatt aacccccttga atatttcctt catttttta gga 463

<210> 67

<211> 439

<212> DNA

<213> Homo sapiens

<220>

<221> misc_feature

<222> (235)..(235)

<223> a,t,g,c

<220>

<221> misc_feature

<222> (439)..(439)

<223> a,t,g,c

<400> 67
tgctttaaaa atatcttcaa taataagaca tcctattttt gggggttag ggatagagat 60

aaatatgaaa actttttttt gataaaacac ctagtaagga catgtttcca ggtttaaagt 120

tcatgaacat ataaaacaga aattgggtcc tgccaccttg agtgcaggat tctgaggtcc 180

taaatctggt tctgaggttc tgtgtttgat tctggggtac agacagctca agtanggagg 240

tctccttatg tcacaaaact acacgatgcg gaggttctgt tccccaccaa aggcttgtct 300

ggggcccatt ggtgcctggg gaggaggccc cagcgaggct catgggtcca gcatggctcc 360

tggggggacc ccttactctc accacgcaag agcccccagc caggagcact gggccagcca 420

gctgcgggcc gatgagggn 439

<210> 68

<211> 189

<212> DNA

<213> Homo sapiens

<220>

<221> misc_feature

<222> (32)..(32)

<223> a,t,g,c

<220>

<221> misc_feature

<222> (33)..(33)

<223> a,t,g,c

<400> 68
ggttgtcggc agggctgtca gggcacatgc tnngccggct ggggtcccca cccgcggaga 60

cggtcctaga cccagtgggg agtgctgtgt ctcctgggtc tgcccacccg tggccaagca 120

ggaactccag gtgcagggcg ggcctccagg tgcagggcgg gccagaagcc ccctcatcgg 180

cccgcagct 189

<210> 69

<211> 467

<212> DNA

<213> Homo sapiens

<220>

<221> misc_feature

<222> (399)..(399)

<223> a,t,g,c

<220>

<221> misc_feature

<222> (451)..(451)

<223> a,t,g,c

<400> 69
ttacactaaa aagagtaaat tttattgttt ataaattatg cttcaataaa cctgccttta 60

aaaaattttt tatcagtaat tacaattttc aaaagtggag atacttggta ggcccctgct 120

ttcttggccc ctaaggtccc tctctctgtt aaactgtaga gctccttaga accaaaacca 180

aaccaccttt cattttaatg aaaatgaaat gcaaaaactg gggctccaag caaggaagaa 240

accgtctacc tgcagttcat ctgggggttg gtgggagagg tggtggagag agtaaggcta 300

agcgccccca gcaggcccaa gttctctgta ccacacttgg aggcaggtat atgggactcg 360

aaagtggatg ttctcagcca caagcctggg cctggatang aaacaaagga taagactgga 420

aggatgtcag gtaaggggag acccagagca ncagctgggc tcacttt 467

<210> 70

<211> 226

<212> DNA

<213> Homo sapiens

<220>

<221> misc_feature

<222> (210)..(210)

<223> a,t,g,c

<220>

<221> misc_feature

<222> (218)..(218)

<223> a,t,g,c

<400> 70
cattctggga aatcaggctg tctgtgtaaa ggttactgct gtgctcagga gttgaaccgt 60

gtggtgctgt atccggatac tcatgacgaa tggatggagg gcgtgaaaag tgagcccagc 120

tggtgctctg ggtctaccct acctgacatc cttccagtct tatcctttgt ttcctatcca 180

ggcccaggct tgtggcttga gaacatccan tttcattncc atatac 226

<210> 71

<211> 519

<212> DNA

<213> Homo sapiens

<220>

<221> misc_feature

<222> (372)..(372)

<223> a,t,g,c

<220>

<221> misc_feature

<222> (457)..(457)

<223> a,t,g,c

<220>

<221> misc_feature

<222> (475)..(475)

<223> a,t,g,c

<220>

<221> misc_feature

<222> (494)..(494)

<223> a,t,g,c

<400> 71
tttttacaat gtgtttattg gacacacaaa aaaactttgc aaccatcata atacatcaat 60

atttaaccta gataattctg aaataatttg gattctttca tttttcagga tttgagctca 120

tcaattatgt taaatttcct atattctgtt acaaatataa tacagatttc ataagtctgc 180

cttgattcac tgctccctaa tccagagcag ttaaatttca aaatcacata gttatatagc 240

ctacctgcca aaaaagaata acccatccct gtcttcttaa agtggtaggg ttttaaaatt 300

catgatacaa gtaacaaagt aagttgataa caaatactat tttgaactgg ggcaaaatct 360

agcccaatat gnaattgtgt tccccttgta tctgagaggc ctgtgttgga caaccatagt 420

actctgcttt cactgtgctc aaataacctg aaatatntag ttgtttctga gtagntctga 480

gtaggaaaga accnaatggt aaatttcctt agagaccat 519

<210> 72

<211> 254

<212> DNA

<213> Homo sapiens

<220>

<221> misc_feature

<222> (8)..(8)

<223> a,t,g,c

<220>

<221> misc_feature

<222> (184)..(184)

<223> a,t,g,c

<220>

<221> misc_feature

<222> (246)..(246)

<223> a,t,g,c

<400> 72
ctgtggcnga gttttgaaca tctgttatag ggagtgatca aattagaagg caatgtggaa 60

aaacaattct gggaaagatt tctttatatg aagtccctgc cactagccag ccatcctaat 120

tgatgaaagt tatctgttca caggcctgca gtgatggtga ggaatgttct gaaatttgcg 180

aagnatttga gtagtgaaat gtaagcacaa aacctcctga acccagagtg tgtatacaca 240

ggaatnaacc ttat 254

<210> 73

<211> 693

<212> DNA

<213> Homo sapiens

<220>

<221> misc_feature

<222> (38)..(38)

<223> a,t,g,c

<220>

<221> misc_feature

<222> (46)..(46)

<223> a,t,g,c

<220>

<221> misc_feature

<222> (105)..(105)

<223> a,t,g,c

<220>

<221> misc_feature

<222> (443)..(443)

<223> a,t,g,c

<220>

<221> misc_feature

<222> (477)..(477)

<223> a,t,g,c

<220>

<221> misc_feature

<222> (524)..(524)

<223> a,t,g,c

<220>

<221> misc_feature

<222> (627)..(627)

<223> a,t,g,c

<220>

<221> misc_feature

<222> (635)..(635)

<223> a,t,g,c

<220>

<221> misc_feature

<222> (645)..(645)

<223> a,t,g,c

<220>

<221> misc_feature

<222> (677)..(677)

<223> a,t,g,c

<400> 73
ggtgttgaag gaatttattc atgatagact gagggtcnat cagggngact gtccaatggt 60

gacaagctcc agaagcccgc gtcgcaacag ccaggagggc caggncaccc caggcaggag 120

gcagtgggct ggcagccacc ctgggcacag aagagcagac gcagacagtg ctgggcaacg 180

aggggctttc ttcatgggcc cgcctgccct gtccctcccc ccaggtcccc accttctagg 240

gttaaagtgc agctgggagg gaggaggcag gcagaattgg ggagctagag agagcccaag 300

tgaaccctga ctgtccacgc aagtcccatg tcctcctcgt cctggagttc ctcgaggttc 360

agcgagccca tcccgcctag ggcctctgga accttggggc gagggaggta acccctcact 420

cccacaccca tccgatattt acnaagagca tccctgggag aggggagaag gcggccngct 480

ctgacagctt tctgggggc cagaacttgg ggcaagtgcg gggntaacat tctaacagga 540

gagaagggga tgctctcttc aggatggtct tggggggttgt ccctccttct ggccttgaaa 600

ctcttccttc ctttttcctc tcccttncca acagnaagca agaancagaa agccgaaact 660

gtccatccgg cacattnagt tccagtgatc aaa 693

<210> 74

<211> 175

<212> DNA

<213> Homo sapiens

<400> 74
aaggcctctc accccacttc ccatctccag ggaagcgtcg ccccagtggc actgaagtgg 60

ccctccctca gcggaggggt ttgggagtca ggcctgggca ggaccctgct gactcgtggc 120

gcgggactgg gagccaggct ctccgggcct ttctctggct tccttggctt gcctg 175

<210> 75

<211> 485

<212> DNA

<213> Homo sapiens

<220>

<221> misc_feature

<222> (467)..(467)

<223> a,t,g,c

<220>

<221> misc_feature

<222> (480)..(480)

<223> a,t,g,c

<400> 75
tgaaaggcta ttgcactaca aagtaaaccc tgaattcaat atgaatcccc ctgtaatgga 60

ccagttcaat atgaacttag agcaacatct cgcaccccag gaacatatgc agaatttgcc 120

ctttgaacct cgcacagctg tgaagaattt catggcctct gagttggatg ataatgcagg 180

actatcaaga agtgaaactg gatcaacgat atcaatgagt tctttagaga gaagaaaatc 240

acgatattca gaccttgact ttgagaaggt catgcataca aggaagaggc atatggaact 300

atttcaagaa ctaaatcaga aatttcaaac tttgggacag atttcgggat ataccaaata 360

caaggcagta tgggaaaacc ccggcaccaa acaaggattc catggggaca tttttcaaaa 420

accccgtgga ttacccgctt tacaccacat tcatgttttt ggacacnggg gcaagggtgn 480

ttttt 485

<210> 76

<211> 581

<212> DNA

<213> Homo sapiens

<220>

<221> misc_feature

<222> (528)..(528)

<223> a,t,g,c

<220>

<221> misc_feature

<222> (538)..(538)

<223> a,t,g,c

<220>

<221> misc_feature

<222> (560)..(560)

<223> a,t,g,c

<400> 76
tttttttttt tttttttttt tttttttttt ttttaaagta tcacaccaga agtttattat 60

ggaacaatca catatgttga ctctcctttg accctcactg cagtgcactt tcattactta 120

tcaatctggg ggcgggaaaa aggggtgcag ccagacaaga gaatatacag gaaagaagca 180

ttgtatataa gcctatgtat ttcagtaatg ctgctacagg gggatacaaa atcaggtgcc 240

agcctccaga aaaaaagaga tttttttct tccctcagtc tcatttggcc cctcggcgct 300

tcctgaagta gcgattatag gcagcattgt atccataaac catggcgtag tttcgcaaag 360

tctgtagtca tcacaggctt ccctattgag ctcgtggaca ggcttagagc gttctcggat 420

cctctcttgg actttagctc tccatctctg ctgaggggat atgaaggtat ttgcattctc 480

ctgttaatga agggattaag ttcataagat tccatgcttt catgtgantc ataacacnaa 540

gttctaccgt aaggggccan gatggaagaa ggtcagctct c 581

<210> 77

<211> 541

<212> DNA

<213> Homo sapiens

<220>

<221> misc_feature

<222> (14)..(14)

<223> a,t,g,c

<220>

<221> misc_feature

<222> (529)..(529)

<223> a,t,g,c

<220>

<221> misc_feature

<222> (533)..(533)

<223> a,t,g,c

<400> 77
cctactgctg ctanacaaga ccctgagact gacctgcagg acgaaaccat gaagagcctg 60

atccttcttg ccatcctggc cgccttagcg taggtaactt tgtgttatga atcacatgaa 120

agcatggaat cttatgaact taatcccttc attaacagga gaaatgcaaa taccttcata 180

tcccctcagc agagatggag agctaaagtc caagagagga tccgagaacg ctctaagcct 240

gtccacgagc tcaataggga agcctgtgat gactacagac tttgcgaacg tacgccatgg 300

tttatggata caatgctgcc tataatcgct acttcaggaa gcgcccgaggg gccaaatgag 360

actgagggaa gaaaaaaaat ctcttttttt ctggaggctg gcacctgatt ttgtatcccc 420

ctgtagcagc attactgaaa tacataggct tatatacaat gcttctttcc tgtatattcc 480

ttgtctggct gcaccctttt tccgccccag attgataagt aatgaagtna ctnagtgagg 540

g 541

<210> 78

<211> 454

<212> DNA

<213> Homo sapiens

<220>

<221> misc_feature

<222> (165)..(165)

<223> a,t,g,c

<400> 78
gcaggtaaaa attatacttt tatttgagtc accaggagaa agattcactt gtggttcaag 60

tcaaatgttc agaatcataa caggccagaa aggtttgatc ccgagcacaa gcccacgagg 120

gaggggacca aaacagacca aaatgagaca acaaccccat ataanaagat gaactggcgg 180

cttcacacac tcacacacat acacatacac acggatgaaa tgtttggaca gaggcaaatt 240

tcacgtggtc atttctgttt ctttttaaat acaggtttgt agggtggtat tttgtttttt 300

ccagctataa aaaaaggccc aaaagtgcat gtgtgagggg ggaaaggcag aaattaagca 360

ataaagtcat tttcccctgg agggacatga gaggggagaa aacagggagg cagtgcttgg 420

gagaacgcac tttcctcacc actggggctt cttg 454

<210> 79

<211> 383

<212> DNA

<213> Homo sapiens

<400> 79
aacaatcagt catcatgggt gtttttgtca actgcttgtt aattgatttg gggatgtttg 60

ccccgaatga gaggttgagg aaaagactgt gggtggggag gccctgcctg acccatccct 120

tttcctttct ggccccagct aggtggaggc aagtggaata tcttatattg ggcgatttgg 180

gggctcgggg aggcagagaa tctcttggga gtcttgggtg gcgctggtgc attctgtttc 240

ctcttgatct caaagcacaa tgtggatttg gggaccaaag gcaaggggac acatcccctt 300

aagaggacct gagtttggga agagtggtga gtggaaggga ggagcagcaa gaagcagcct 360

gttttcactc agcttaattc tcc 383

<210> 80

<211> 545

<212> DNA

<213> Homo sapiens

<220>

<221> misc_feature

<222> (497)..(497)

<223> a,t,g,c

<400> 80
actgttgacc tgtcactgtt tattatttca gcactaaaac tgaggagcct caactgctgg 60

ctcttcttcc ctttgtattt gtgtaaggag cactgcactc ccataaaagg ttttaaaata 120

caaaatgtac aagaacacac aattccaagt gctgtaaaca taactgagaa ccagttcctt 180

tactaaacat ccattttata aaatacaagg tttcaatttg agcccatctg agccttaaag 240

atccattctg aataccaaaa acagggcttc acagccaggc ccagaagagg tctggtgata 300

atggctggcc ctgggtgggg atagtttaca cccgggcagc agcaccacac atgaacccaa 360

agacatgttc tttttaaagc tgttttcagc catgtttctc tggtgcatct ccagtaagca 420

gaaggctacc cattccattc ctcaacccca agagctagca cagttagagt aggagggggg 480

tgcgtactag cacgtgncca gttgctcagt gcggcaggta gaaatgattt gcataggtcc 540

atggg 545

<210> 81

<211> 506

<212> DNA

<213> Homo sapiens

<220>

<221> misc_feature

<222> (11)..(11)

<223> a,t,g,c

<220>

<221> misc_feature

<222> (393)..(393)

<223> a,t,g,c

<220>

<221> misc_feature

<222> (499)..(499)

<223> a,t,g,c

<400> 81
gaacaatata nacaggcatt cccatcacag gttaaagaaa caagagtcat cgaagagcct 60

gaagaaggtt attgcagctt tgtcaaatcc aaaagcaacc tctagttcac cagcacatcc 120

aaaacaaaca ttagaaaaca accacccaaa tccattcttg acaaatgcac ttttaggtaa 180

tcaccaacca aatggagtta ttcaaagtgt cattcaagaa gctcctctag cacttactac 240

caaaactaaa atgcagagaa gattaatgaa aacattgctg ctgcaagtag caccccttttt 300

tcctcacctg taaatctgag tacaagtggg agaagaaccc ctggcaatca gacacctgta 360

atgccctctg cctctcccat cctgcatagt cangggaagg aaaaagcagt tagcaataat 420

gttaacccag taaaaacaca gcatcactcc catcctgcaa atctttagtg ggacaattca 480

gagggacaga ttcagacant tcccag 506

<210> 82

<211> 476

<212> DNA

<213> Homo sapiens

<400> 82
acaggtgaac catagtctca ctttatttcg aattttagga ctgaacagaa agaaggttca 60

cagatatttc ttcagggaag gaaagtgggc aaatagcaca atgaggcaaa ccacacaatc 120

tagacgtact ttcccgggta aacaaactgg gaagcccaga gctcacagag gaggagagca 180

gagcttgggg tggcagagag gtgttcattg ggaaggagtc aggaacacaa gagaaatgtt 240

catgagctca tccaggatag agaggactct tggggaaggg gacctccttc cccatgctgt 300

ctctgcccca agcccttaat gaagcaggag agggaagtct gtccagaaac tcatctggtt 360

agacatttgc atcaggaata gggagggggt ggaggcagag cactttggac atgggaaatc 420

acagttgcag tagacagtga gctctagtca ctccagaagc ttgacataat ttgcag 476

<210> 83

<211> 442

<212> DNA

<213> Homo sapiens

<400> 83
atgagctttc ctttgatccg gacgacgtaa tcactgacat tgagatggtg gacgagggct 60

ggtggcgggg acgttgccat ggccactttg gactcttccc tgcaaattat gtcaagcttc 120

tggagtgact agagctcact gtctactgca actgtgattt cccatgtcca aagtggctct 180

gctccacccc ctccctattc ctgatgcaaa tgtctaacca gatgagtttc tggacagact 240

tccctctcct gcttcattaa gggcttgggg cagagacagc atggggaagg aggtcccctt 300

ccccaagagt cctctctatc ctggatgagc tcatgaacat ttctcttgtg ttcctgactc 360

cttcccaatg aacacctctc tgccacccca agctctgctc tcctcctctg tgagctctgg 420

gcttcccagt ttgtttaccc gg 442

<210> 84

<211> 321

<212> DNA

<213> Homo sapiens

<400> 84
ctggaaagag aatagaatta aagagaagaa tcatctatgt atgaagcctg gtctaactga 60

taacgcagtc agattgccat ttttaagatg gaaaaagggg aacatgcttg ccttgagcag 120

aaaagttttt ggtggggctg gagctcctaa atattatttt ggtcatttgc ccaaaaactg 180

aagtacacca ccaatgtccc aattatgaag ttgaatacag aattataaaa aaaaattgtt 240

gaaagtcaat aagaataaat cttagaattt ggactcctgc cacatggcag aaaaaaagca 300

tgatgtttta agacaacctg g 321

<210> 85

<211> 425

<212> DNA

<213> Homo sapiens

<400> 85
gcatttattt gaatatcagg ttttagctaa ctgcctaatg taccaaaaaa aaattagcaa 60

tgtcttatgt gggatgaggg gttaagttaa agttgctgat caagtaatgg ccgtgtgcac 120

aagatgataa ctaagaattt ctggttctaa ggtagtaatg ccttttggga tttatatttg 180

tttaatgttg agattctggg gaccataggt gggcctgtca actcttaaca gactatagta 240

aaggagaggt ggtggcacat gtgagataaa acttgtttat atttacattc ttattagtgg 300

atagatatca cctcagtaca acactgaaat taaactgcct tgttcatgct ttcatgtctc 360

aaatcaaggc ctaaatgagt aaaaagatga ttacagatta cctaaaaccc ttaaccagtt 420

ttggt 425

<210> 86

<211> 367

<212> DNA

<213> Homo sapiens

<400> 86
ccttatttgg gccagttcct ttaggaaaac agcaaagtcc catcccaagt tagggactga 60

gatttcacag cctctcagtc tgcgctctgc aggattctcc gcatttccag gcaaagccaa 120

aaaaccaaag caaagcacag gggccctct ttctacctct cctcacccag ccaaatggca 180

gaggtgccac agaggcagag gggtggtgga agtgctgctt ctgaggcagg cagtggaagt 240

gagatgcccg tggggcagga aggaggtgcc tcgccagccc cgccatccac catccggcat 300

caggatgggc tgctcgccaa cccagcactc ccactgcaga ggtcgcgggt gtgaggtgca 360

tggattc 367

<210> 87

<211> 298

<212> DNA

<213> Homo sapiens

<400> 87
aactttttaa aagtttttat ttacctaaaa acataaattt atcaaacata tcaatttgct 60

ttgcagacaa tttacataaa aattcatgat gtataccaac gacagcatag cattatctac 120

ctcagtttgt gaagcatcct ttcctacaga tctcagcaaa acgcagagcc agtaggggaa 180

attcaagctt tcatttgggg gcctctgggg tcctacctgc tggcagttct ttcagcagaa 240

agtggggtac gggggatgct gaagaggggg aagtggaccc cattgcctgt taagtccc 298

<210> 88

<211> 293

<212> DNA

<213> Homo sapiens

<400> 88
gtaacaattc agatccagtg taaacttccg ttcattgctc tccagtcaca tgcccccact 60

tccccacagg tgaaagtttt tctgaaagtg ttgggattgg ttaaggtctt tatttgtatt 120

acgtatctcc ccaagtcctc tgtggccagc tgcatctgtc tgaatggtgc gtgaaggctc 180

tcagacctta cacaccattt tgtaagttat gttttacatg cccggttttt gagactgatc 240

tcgatgcagg tggatctcct tgagatcctg atagcctgtt acaggaatga agg 293

<210> 89

<211> 419

<212> DNA

<213> Homo sapiens

<400> 89
tataaagcac tgtttatttt tatgtattga aaatgtccat tttaaaagtc ttcaaaataa 60

aaacaagtta gaaaatttga gcttgtgtta agaaggggaa ggacagaaaa ggaactgaaa 120

ggcctctacg ggcagcagtt taattacagg gcaacaggaa cccatttata gagtattgta 180

aacaacacaa ctatggcgac actgctctca gattaacatg gcttcatttc ctttatatta 240

tagtttagtg tgtaagcatg gttaccactc tgcacaagct tcgttatcca gagtaccgag 300

ggcccctcat cagaagggcc aaatccccga aacatgcata tgtactcatc actgtaaaat 360

gaagaccttt caatattttc agcaatcaac taaagtattc cgtaatcaca aaaatgttt 419

<210> 90

<211> 426

<212> DNA

<213> Homo sapiens

<400> 90
ctttcattca ctttattact ctctcatata tgctgtgaga agttgtaata ttaattggcc 60

tctttgggtg ggactctaag cagtatttgc agaaatatgc ttctggtcca acttgtacat 120

ccagaacaaa agggcccctc tagtagctgt gtgctggatt attcaggact gattaactca 180

agttgctcat tgaatcacat catccacttt atcccagaaa ctagaactgt gagaaaccag 240

gtttagaaga cagtcacaga acagcacaca tccaaattca gcgccattga aagttggcct 300

aaggctcagt gccactcacc cctccctccc caaaagacaa gagaaatttg gccagatggg 360

gaaggtcact ggaaattgag gccaaagggc tgatcagaat tgcctttata atatatttga 420

tggatg 426

<210> 91

<211> 313

<212> DNA

<213> Homo sapiens

<400> 91
accagaaaca cgtgcacttt attgaatgcc attgtagaaa agcgtgtgag gataaagggc 60

tgatacagga ctcggctccg ggggcagggc gaggaatgga agttggagta cgtgggatac 120

aggtcatggg cagactcctg gcctcagtga tgcctcctga tctatccata ggcctggaag 180

atcagcactg ggatgacgat gagcagaatg gtcatgagga tgcccagaat cagggcccag 240

atgttcaggc acttggcggt ggaggcatag gcctgggccc cggtcacgtc gccaaccatc 300

ttcctgtccc tag 313

<210> 92

<211> 331

<212> DNA

<213> Homo sapiens

<400> 92
ctgggcttca tagcattcgc ctactccgtg aagtctaggg acaggaagat ggttggcgac 60

gtgaccgggg cccaggccta tgcctccacc gccaagtgcc tgaacatctg ggccctgatt 120

ctgggcatcc tcatgaccat tctgctcatc gtcatcccag tgctgatctt ccaggcctat 180

ggatagatca ggaggcatca catgaggcca ggagctctgc ccatgacctg tatcccacgt 240

actccaactt ccattcctcg ccctgccccc ggagccgagt cctgtatcag ccctttatcc 300

tcacacgctt ttctacaatg gcattcaata a 331

<210> 93

<211> 505

<212> DNA

<213> Homo sapiens

<400> 93
ttgttttttt ttttgcatga gactcaagat tctatttatt cagaggcaaa attccttctt 60

aactgtgaac ctgtgaaacc aaacaagtta tgtgcttcca aaatacaata gtgggacaaa 120

cataggatgg atattactat tccaaaagga agaaagagag gaagagaaac agtcatggga 180

ctcaagcaag tacaaatcat agcaaaggaa acgtcctaag atcttaaggc tggagaataa 240

tgcctttaag ataatcatgg cccactgggg cagcaccacc tccctgaccc cgttgggtgg 300

aggacccatt aaaagtggca gcatgacccc cacaggcagg gaaggcactg cccatgctgt 360

gtctctctgc tgtggccctg cccatggagg caatttcctg tggagggtgg ggttgcactc 420

taactgatct gctggccttc tcttccttca acgattccct gggtgttggg tccaccttta 480

gaaatttaaa cagaacccct ccccc 505

<210> 94

<211> 590

<212> DNA

<213> Homo sapiens

<400> 94
ggatcttggc agtgggaaga tggctccatt ctctcaccca acctactaac aataattgaa 60

atgcagaagg gagactgtgc actctatgcc tcgagcttta aaggctatat agaaaactgt 120

tcaactccaa atacatacat ctgcatgcaa aggactgtgt aaagatgatc aaccatctca 180

ataaaagcca ggaacagaga agagattaca ccagcggtaa cactgccaac cgagactaaa 240

ggaaacaaac aaaaacagga caaaatgacc aaagactgtc agatttctta gactccacag 300

gaccaaacca tagaacaatt tcactgcaaa catgcatgat tctccaagac aaaagaagag 360

agatcctaaa ggcaattcag atatccccaa ggctgcctct cccaccacaa gcccagagtg 420

gatgggctgg gggaggggtg ctgttttaat ttctaaaggt aggaccaaca cccaggggat 480

cagtgaagga agagaaggcc agcagatcag tgagagtgca acccaccatc acaggaaatg 540

cctcatggca gggcacagca gagagcacag catgggcagt gcttccctgc 590

<210> 95

<211> 272

<212> DNA

<213> Homo sapiens

<400> 95
catgttgggt tttgattatt tgtggtgcta agaagaaggg aacatggttg gaggcccagt 60

gagagaaaca gtgctttgaa tcaaagagca gaatgataga aactgacttc agagcaactt 120

cttggcagca gtatccaatt tggaagttga aggtctgtcc tggagccaga tgctaacgaa 180

acacagcaaa tgcttttcct aaggcacaat agtcttttca gtgagctcag gaaccctgtt 240

tatgcagatc ctcgttgaac tttcttgctc ct 272

<210> 96

<211> 287

<212> DNA

<213> Homo sapiens

<400> 96
atcatctgtg gagtgggcat cttcatgcac aggaggagca agaaagttca acgaggatct 60

gcataaacag ggttcctgag ctcactgaaa agactattgt gccttaggaa aagcatttgc 120

tgtgtttcgt tagcatctgg ctccaggaca gaccttcaac ttccaaattg gatactgctg 180

ccaagaagtt gctctgaagt cagtttctat cattctgctc tttgattcaa agcactgttt 240

ctctcactgg gcctccaacc atgttccctt cttcttagca ccacaaa 287

<210> 97

<211> 133

<212> DNA

<213> Homo sapiens

<400> 97
tttttttttc aactttttgc aaagcagcat agcaacaatc gtgattgtag cacttgcctg 60

aggttgtggt cacaaccaac gtagtaaaca tcatttgcat atcagtaaga aaaagaaaac 120

aggaggagat gag 133

<210> 98

<211> 425

<212> DNA

<213> Homo sapiens

<400> 98
gctcaatggc ggtctggccg tgaagaccaa tgagatctct gtgaatccag agtggggtag 60

gttcctaccc tcccggtttc cttgggggcc acctccgttg tgaataacgc cactgtctcc 120

aagatggatg gctcccagtc gggtatcagt gcagatgtgg aaaaaccaag tgctactgac 180

ggcgttccca aacaccagtt tcctcacttc ctggaagaaa acaagattgc ggtcactaag 240

ggagaacttg cgtggaagga gcaatgcaga cacagtgaaa tctctagaat ctgctttgtt 300

ttgtaagaac tcatctcctc ctgttttctt tttcttactg atatgcaaat gatgtttact 360

acgttggttg tgaccacaac ctcaggcaag tgctacaatc acgattgttg ctatgctgct 420

ttgca 425

<210> 99

<211> 468

<212> DNA

<213> Homo sapiens

<400> 99
tttttttttt tttttgctgc taaatttgcg gtagtttatt actgaagcaa tggaaagcca 60

atacagacag aatatacaga gagaaagaga atgagttctt tcctgataat ttgtaaatat 120

ttgggtcttc actggacaag cttcacagag gattcactgg ttccctagca aaccagcatg 180

tccagtcctg cagcctccct ttcttaggcc cagcatatgt cagctgtgtg catagaaaaa 240

tcaaagcagg accctgagta gttggaaaga aaagatggtt ggaaatgggt tgcacttcaa 300

gtgaggaaac aagaggtagg agaccggcat ctctttctca tatgtcccag gctgactctt 360

gtgagttgtt ttcccttgga ggctatcgat gacagtcaca gtaacctgat ggaactaact 420

ccaagatgcc tggtctcaac ttgacaaaaa taccccaagt tgggaaat 468

<210> 100

<211> 232

<212> DNA

<213> Homo sapiens

<400> 100
tccgtcttta ttaacgaaag tgtttactgc ctaagccaga cactgagagc caccatgtat 60

tcctctctct ctctcaccta ttcccatgtt taatccatta tttggattgg tccattttat 120

ctccattttc tcaaatacag gaaaagacag atcatcatca accagcaatt gtgactatct 180

acatattcac ataaggattt ccaacttggg gtatttttgc aagttgaacc ag 232

<210> 101

<211> 302

<212> DNA

<213> Homo sapiens

<400> 101
acggattata aaagttatat ttattcacga tgctacattt attgcattcc cttagaaaaa 60

tggagaactg tttatgtacc caatctgcac atataaaatt ttatacaaat tatgtgtagc 120

acataaaggc ctctggtaca gctaaaatcc tgacactata atttgggtat tcctgcttta 180

gggtctccag tttatcaggt ctgtccatag aaaacagaaa ctggaattat agtcagtctt 240

gctaacactt agaaactact ttaaaataca ataaaatttt catttaccct aaaagtccaa 300

at 302

<210> 102

<211> 412

<212> DNA

<213> Homo sapiens

<400> 102
ctagaagaaa aagagaaggc agagaaaaag aaacgaggac caaagccttc aacacagaaa 60

cgtaaaatgg atggcgcacc tgatggtcga ggaagaaaaa agaagctgaa actatgaata 120

tgtttttgtt tcataatcac taactttaaa ccagtagttc tttaatttac gggtcttcat 180

aagatgtact gtacaatgct caattgttat gtcatttaaa gacatcaggt tcatctgttt 240

actgagctag aaacatagta tgtagtttca ctttttaaa tgcaacagct gtgctgaaat 300

tttttatca ttaacacttg aagtaataaa ataggcttca tttattacta agtgtttcat 360

ttgatttatt tttctattgt agttccattt gtgaagattg tgactttttg tt 412

<210> 103

<211> 388

<212> DNA

<213> Homo sapiens

<400> 103
tttttttttt tttttttttt tttttttttt aatgaactaa catcttttct ttattcctat 60

aacaatttgt tggaaattgt aattactgaa gatacagttg tcagttcata ggtagtaaaa 120

gtacgtaacc ccagaatttg gggaaaaaca aatatttttc gtctgaaatt aactactaaa 180

actcaactca taatgatggg gactagtaat gcccagagta accttctggg actgaaagtt 240

ctagtatttc ctgaatcttg gtcaagactc cacatgaagt aattgcaaag gtatggcatc 300

tagtaaaatg gagaatcaaa gaaacatagt tttttttct gcacagtcaa gagaaaagca 360

tgctttcttc tttctttcac tggaaata 388

<210> 104

<211> 270

<212> DNA

<213> Homo sapiens

<400> 104
gcagtttgca gtgatgtggc taagataccc ttgtgctatt aaaggcacat ttacgtatct 60

ttcaacgcag tttgtacctt tgtttagggt tctattgaac tttctctgtg ctctttagaa 120

aaccgtcgaa taaagaactc aaatgatgct gtttttggac agggaggtca tggaccaagt 180

acaaagcggc aaaaggatgg aattaaggta cctgacattg ttgaacccat gcctgatgcc 240

atattagctc aggactacct acatggacat 270

<210> 105

<211> 389

<212> DNA

<213> Homo sapiens

<220>

<221> misc_feature

<222> (173)..(173)

<223> a,t,g,c

<220>

<221> misc_feature

<222> (361)..(361)

<223> a,t,g,c

<220>

<221> misc_feature

<222> (370)..(370)

<223> a,t,g,c

<400> 105
taaaacaaac aggtttattg ataagcagga atccggtcca gcatacagtg caaactttca 60

ttgtcttggg atcaacacac acacacaagg gaaccaaggc cccacagagt ctccaaattc 120

atcgcttccc ggctctgcct gtgatgccaa ggaagcctga ggttctccca ggnctgtcca 180

ccagggggca ggtgcgctcc atcatgtcct catgtgggtc caatgtgggc agaaacctgg 240

gccagtccca gcaggtctga cttttttttt cttttaagag acagggtctc actctgttgc 300

tcaggctggg agtgtagtgg cgcgatcata gctcgctttg cagcctcaaa ctcctggggg 360

ntccagtgan tccttccctt caagccttc 389

<210> 106

<211> 381

<212> DNA

<213> Homo sapiens

<220>

<221> misc_feature

<222> (97)..(97)

<223> a,t,g,c

<220>

<221> misc_feature

<222> (100)..(100)

<223> a,t,g,c

<220>

<221> misc_feature

<222> (339)..(339)

<223> a,t,g,c

<400> 106
cttttcaggg aatttcagga actagagatg actgagtcct cgtagccatc tctctactcc 60

tacctcagcc tagaccctcc tcctccccca gaggtgnggn ttcctcttcc ccactcccca 120

ccttcaattc ctgggcccca aacgggctgc cctgccactt tggtacatgg ccagtgtgat 180

cccaagtgcc agtcttgtgt ctgcgtctgt gttgcgtgtc gtggggtgtg tgtagccaag 240

gtcggtaagt tgaatggcct gcctttgaag ccacttgaag ctggggattc ctccccatta 300

ggagtcagcc tttcccccttt cccaggggcc aggggctgnc aagaggggga aaaccagtgt 360

taggcctttg cccggatttc t 381

<210> 107

<211> 240

<212> DNA

<213> Homo sapiens

<400> 107
atcttttccg cccagctttt tcttattaca atacaactac aatgaacatg gcagattatt 60

tacttaggac aaacttatag gggtagaaat gttggtgaaa aggcaaatac atttttaggc 120

ttttgatacc tactgtgaaa ctacactaaa gaaagattat aggagtgtat aggttaccag 180

cagtgtagga gtggtcactg gcacacctgc agaaacacta aacatttcat atacatatat 240

<210> 108

<211> 469

<212> DNA

<213> Homo sapiens

<400> 108
gttttcatga gtatcccgac ttccttctaa gaacttccac ctgagaactg accacagcgt 60

cacgattcca catgggtgtg tttcctttcc cctttcccat ttcagtggtt tccaatttct 120

ttttcttttg gcactataaa cctttcgcaa aggaaatatt agacagaact cctacatgtc 180

aagcaaatta aaatagtggt gaaattagag tggaggacat aatcacccta tcatataggc 240

tatttgtcca tatcatattt gtccctacaa aggcttctaa ggcaggggtc cccaacctct 300

gggccgcaga ccggtaccag tggcctgtta ggaactgagc cacacagcag gaggtgagcg 360

ggagacaagc gggcactact acctgagctc tacctcctgt gagatcagca gcagcattag 420

attctcatag aagcgtgaat cctattgtga actgcacata tgagggatc 469

<210> 109

<211> 312

<212> DNA

<213> Homo sapiens

<400> 109
tcactctttc cttgtttatt aaatatcaac ttttcctgcc taatgggctg aggttcattt 60

tcccattcct caaggtaagg gtagactacc taggaactta ttgcatcttt aggccagctg 120

gcttagtgct acccatctga acccccagat tactacccaa gtcttccttt tgccccttcc 180

tgccctaaca gcaagtacca ggccagtccc ttccccagca aatgccaggg gcttcatgtg 240

aagaggaact ggccacaagg atgaggggag gaggagaaac tgtttctgca ggaaggacag 300

cagtgcctcc ag 312

<210> 110

<211> 289

<212> DNA

<213> Homo sapiens

<400> 110
cctgctcctg ctgtgtgtcg ggcttattat tgtgaccatc ctgctgctcc agagtgcctt 60

tccaggtttc ctttagcttc cctgcttcct gggaatcagg agcctggaca ctgccatctc 120

tagagcagag tggaggcctg gactcccttt gctcactcca ttcgggtcca cagcgtgagg 180

ttgcctctga caagatgaat gggcactgcc tgcccttcta gtgaaaaggc ttggctatgg 240

ccctgtgtga ctccaggtcc caggaacctt gccttcgtca tctgtggat 289

<210> 111

<211> 314

<212> DNA

<213> Homo sapiens

<400> 111
taagggctta ttaaactagt tttactttag caccattctt agtggagcag gattcttgat 60

catggggtgg aattttgtgt atctgggctt catgggatgc ataaaatttt ccagttggta 120

agtagcaggt gccgagggtc tggatcagaa aaaaaaggca ggcagccggg ttcccactcg 180

ccttccatga gctctccgca gctcaccggc catcacttga agcaaacact cctgagggag 240

ctggttgagg aaggcgtgca tgagctgcat gccgcggtcc cccattgcac actccatgat 300

agtgtctggc gaca 314

<210> 112

<211> 529

<212> DNA

<213> Homo sapiens

<400> 112
caggcgtccc ctctgcaagc gttagacttc tttgggaatg ggccaccagt taactacaag 60

acaggcaatc tatacctgcg ggggcccctg aagaagtcca atgcaccgct tgtcaatgtg 120

accctctact atgaagcact gtgcggtggc tgcctagcct tcctgaccat tgtctgcatg 180

gaagagtttg aggacatgga gagaagtctg ccactatgcc tgcagctcta cgcccccaggg 240

ctgtcgccag acactatcat ggagtgtgca atgggggacc gcggcatgca gctcatgcac 300

gccttcctca accagctccc tcaggagtgt ttgcttcaag tgatggccgg tgagctgcgg 360

agagcttcat ggaaggcgag tgggaacccg gctgcctgcc tttttttct gatccagaac 420

ctcgggacct gctacttacc aactggaaaa ttttatgcat cccatgaagc ccagatcaca 480

aaattcaccc atgatcaaga tccttgtcac taagatggtg ccaagtaaa 529

<210> 113

<211> 377

<212> DNA

<213> Homo sapiens

<400> 113
tttcttatgc ataagaagta cagtttattg ggagtgccag ataggtaaac aattaattac 60

aatattgtgt acgttcaata acagaggaac atgctgcctg agtcttcctg aggacagaag 120

gctgcaaaat gagagcacaa catctgtgac atcaaatgca ctgtgatttg gcagttaaac 180

aaaatgtaac ctgggctagt agaaaagtca tgaattctga agtcgaagga atttgtcttt 240

tagatcttgt tctgctattt ctttcttgtc cttggctcaa tgcataaatt tgttgggcct 300

taagtttctt cctcccagtg gcacctggaa ccccagtgag atggaaccgt tcactcccct 360

ggaaaggggg ctgaagc 377

<210> 114

<211> 397

<212> DNA

<213> Homo sapiens

<400> 114
gcctcggtaa tgccgggcga ccctccacct accaagctct atcatttcag gtcaacttca 60

gactgctgtg ctggcagtga gaatttcaag ccaatggttc ttaggtttct gggatgtgtg 120

ggagtgggac ccactaagcg agaccacttg gctccctggc ttcagcccct ttccagggga 180

gtgaacggtt ccatctcact ggggttccag gtgccactgg gaggaagaaa cttaaggccc 240

aacaaattta tgcattgagc caaggacaag aaagaaatag cagaacaaga tctaaaagac 300

aaattccttc gacttcagaa ttcatgactt ttctactagc ccaggttaca ttttgtttaa 360

ctgccaaatc acagtgcatt tgatgtcaca gatgttg 397

<210> 115

<211> 450

<212> DNA

<213> Homo sapiens

<400> 115
ttttttttg gaaaggtagg aaagctttaa taaaaactga ataaaaggta taaaaataaa 60

taaattctag cttatcttct cttgaggtct tctgtacttc tttgcagatg tcacaaggtt 120

tggggatata gaacatgtga tgatgacaga gctgggtcca tatgaacttt tcaaatagag 180

ggaatatgaa gctaatgaga tgtgcagaat cctggaagga cttgggtatt aaaggccttg 240

ggtatctgaa gacatgggtg tgaggaaggc ctgcacagag agcttccaat cagcatttta 300

gtgcctcatc attaatttgg acttataagc aaggatcacc agacacctga gaacatcctt 360

aaaacaaaac ggaatcacaa agaatccaga gaatcaagag gtaatccaca gaatggaggg 420

aaacatcaaa atacctctta ttaatatact 450

<210> 116

<211> 428

<212> DNA

<213> Homo sapiens

<400> 116
cagcctcctc tggatctttt ctggttcctt cgctcctaga actgtgtctg atggcttatg 60

aaggcaaatg acaaatgttt gccatatgca catgtatgtc atgaatgaat acatccaggt 120

tctgacttcc tcggggcaac ctctactttc tcaatgcccc tcttaagatg ggggtggggg 180

agggtcctcc tagaactgaa catgatattc caggtgtgat ctaatacatt taaatgtcca 240

aaagctcttt cttccctaaa cgttcctttt taaaagctac tgttctcaat taatgggtgt 300

ctcatcttct taagtatatt aataagaggt attttgatgt ttccctccat tctgtggatt 360

acctcttgat tctctggatt ctttgtgatt ccgttttgtt ttaaggatgt tctcaggtgt 420

ctggtgat 428

<210> 117

<211> 462

<212> DNA

<213> Homo sapiens

<400> 117
ttttttgac atttcatcag ctttaatgct gtctcctcaa gtaatatgga catgatatta 60

ggagagtttc ccagaattta tgtgaccatt cccaactcag cttctcccct gcttttaaga 120

caagctatag ttaggttttc ctatcaccac cacagtcctg actttggtaa atttttctct 180

ctcacactta gagattccat ctcagacctt ggcccttcat ctttagcagg tgatactgta 240

gtcaccggac caaccccag ccttgatctc tctgttggaa tcacagtgac gttttcatca 300

caggcaggcg atctggaagt taagtagttg agaagagaga tgatcccagc aaacatgtaa 360

aagatgtcac tgcaccagtt aagataatag gtccatagga gatctgactc catggcatcc 420

ctagtatgcc agtgaacctg tgccacaaac aggttgaggc ag 462

<210> 118

<211> 559

<212> DNA

<213> Homo sapiens

<400> 118
attcggcaca ggtcgaagac cgagcgaagg gaacatttaa ccttgacttt ccacagtcct 60

gaggttccca aaatacaggg gaacggaaat accaaaggat tatctccaat attccagggc 120

cttctttctc atctctgtct ttaccatact tactggcctt ggtggctctt cagctcttgg 180

atccttaatc gaggaagcat gaccaccaac ttggatctga aggtatccat gctcagcttc 240

atctcagcta cctgcttgct cctctgcctc aacctgtttg tggcacaggt tcactggcat 300

actagggatg ccatggagtc agatctccta tggacctatt atcttaactg gtgcagtgac 360

atcttttaca tgtttgctgg gatcatctct cttctcaact acttaacttc cagatcgcct 420

gcctgtgatg aaaacgtcac tgtgattcca acagagagat caaggctggg ggttggtccg 480

gtgactacag tatcacctgc taaagatgaa gggccaaggt ctgagatgga atctctaagt 540

gtgagagaga aaaatttac 559

<210> 119

<211> 342

<212> DNA

<213> Homo sapiens

<400> 119
ttttaacagt gcaaggtaaa atttaatctg tattgctaat aagacatttt acagcataga 60

aacatgatcg aagtgatcat ttactttatc atgtcataaa acacaagtgc ttattgtaga 120

acagtgatat aatttaaatg gcaaggaaat ttgtgattca taacaaagaa tcctgtatgt 180

atttgttgcc atgattaact ttccaggtca aatatcagta gtaaaataag ttgtggtaca 240

aaagaaagcc aagaagttac ctgtactgaa ggaacatttg atttcattta agaaactgaa 300

ggctggattc tagttaacct tttgttatgt acaaaaatgt tc 342

<210> 120

<211> 486

<212> DNA

<213> Homo sapiens

<400> 120
gaagccttat ggattggatt cgactgacca aaagtggaaa ggatctaacg ggattaaaag 60

gcaggttaat tgaagtaact gaagaagaac ttaagaaaca caacaaaaaa gatgattgtt 120

ggatatgcat aagaggtttc gtttataatg tcagccctta tatggagtat catcctggtg 180

gagaagatga actaatgaga gcagcaggat cagatggtac tgaacttttt gatcaggttc 240

atcgttgggt caattatgaa tccatgctga aagaatgcct ggttggcaga atggccatta 300

aacctgctgt tctgaaagac tatcgtgagg aggaaaagaa agtcttaaat ggcatgcttc 360

ccaagagcca agtgacagat acacttgcca aagaaggtcc tagttatcca agctatgatt 420

ggttccaaac agactcttta gtcaccattg ccatatatac taaacagaag gatatcaatt 480

tagact 486

<210> 121

<211> 179

<212> DNA

<213> Homo sapiens

<400> 121
tttttgtgat aatgtcattt aaggttttat tgtaaaagtc tatttttttct aattaaaaca 60

tttttcccac tataaaatat ttaaacagtg cagatatatg tatatataat acagaattca 120

ttagaaatat ccactctgtt ctatatcctt ctgtttacat atatttttat attaaatac 179

<210> 122

<211> 320

<212> DNA

<213> Homo sapiens

<220>

<221> misc_feature

<222> (3)..(3)

<223> a,t,g,c

<220>

<221> misc_feature

<222> (10)..(10)

<223> a,t,g,c

<220>

<221> misc_feature

<222> (11)..(11)

<223> a,t,g,c

<220>

<221> misc_feature

<222> (12)..(12)

<223> a,t,g,c

<220>

<221> misc_feature

<222> (13)..(13)

<223> a,t,g,c

<220>

<221> misc_feature

<222> (16)..(16)

<223> a,t,g,c

<220>

<221> misc_feature

<222> (19)..(19)

<223> a,t,g,c

<220>

<221> misc_feature

<222> (21)..(21)

<223> a,t,g,c

<220>

<221> misc_feature

<222> (22)..(22)

<223> a,t,g,c

<220>

<221> misc_feature

<222> (24)..(24)

<223> a,t,g,c

<220>

<221> misc_feature

<222> (30)..(30)

<223> a,t,g,c

<220>

<221> misc_feature

<222> (32)..(32)

<223> a,t,g,c

<220>

<221> misc_feature

<222> (34)..(34)

<223> a,t,g,c

<220>

<221> misc_feature

<222> (35)..(35)

<223> a,t,g,c

<220>

<221> misc_feature

<222> (46)..(46)

<223> a,t,g,c

<220>

<221> misc_feature

<222> (54)..(54)

<223> a,t,g,c

<220>

<221> misc_feature

<222> (56)..(56)

<223> a,t,g,c

<220>

<221> misc_feature

<222> (57)..(57)

<223> a,t,g,c

<220>

<221> misc_feature

<222> (61)..(61)

<223> a,t,g,c

<220>

<221> misc_feature

<222> (62)..(62)

<223> a,t,g,c

<220>

<221> misc_feature

<222> (63)..(63)

<223> a,t,g,c

<220>

<221> misc_feature

<222> (192)..(192)

<223> a,t,g,c

<220>

<221> misc_feature

<222> (229)..(229)

<223> a,t,g,c

<220>

<221> misc_feature

<222> (250)..(250)

<223> a,t,g,c

<220>

<221> misc_feature

<222> (302)..(302)

<223> a,t,g,c

<400> 122
gtntctcccn nnnccnccnc nntntctctn gnannccccc cccttncccc cccntnnccc 60

nnntccccca aatcaaggga gctacatggt gctcactgca ttggccgggg gtttgacgga 120

ggcgagcagg gtggcgggct gggtcccggc aaaggagtct ggttgaaaac gtatagctgc 180

cagggtttcc cncttaattc ttgttattgg tgtaaaaaca gaggggttnt gcgagaggtg 240

gtgggtggcn aaggagggag ccaagagacc aagttccggc caaagggtca gcggcatgca 300

gntattctgg ttacacagtt 320

<210> 123

<211> 271

<212> DNA

<213> Homo sapiens

<400> 123
tgctctccct caaaggaagt gtttattttt tggagctcaa agaccccaga aaaaagcaac 60

cccccccgca aaaaaaaccc ataacaacag gcagcacatg ccccccacac cgcaccccat 120

gcccaatctc agggtgggag acaagagggg ggactcattg tccggagaga ggctctggag 180

ggagacagcc ctgtgtcaac ccgactgtgt ccccacaccc aggacttccc catggcccct 240

ccagacctgc tccagaggcc cttctccggc t 271

<210> 124

<211> 476

<212> DNA

<213> Homo sapiens

<400> 124
tcactacatt aaaccataag cttcaggacg cgtctgcaga ggtggagcga ctgagaagag 60

aaaaccaggt cttaagcgtg agaatcgcgg acaagaagta ctaccccagc tcccaggact 120

ccagctccgc tgcggcgccc cagctgctga ttgtgctgct gggcctcagc gctctgctgc 180

agtgagatcc caggaagctg gcacatcttg gaaggtccgt cctgctcggc ttttcgcttg 240

aacattccct tgatctcatc agttctgagc gggtcatggg gcaacacggt tagcggggag 300

agcacggggt agccggagaa gggcctctgg agcaggtctg gaggggccat ggggaagtcc 360

tgggtgtggg gacacagtcg ggttgaccca gggctgtctc cctccagagc ctccctccgg 420

acaatgagtc ccccctcttg tctcccaccc tgagattggg catggggtgc ggtgtg 476

<210> 125

<211> 476

<212> DNA

<213> Homo sapiens

<400> 125
gaggggagat gctgaggtca gggcacttat gtgatcaagt gatggagaca gagtaaagag 60

aatttatgga tatatatgca tatcttgcta ataagtttgg tttatcatca catcaagata 120

aattgccttt agcatagaac ttgagataag tagacgttca ctagcaagtg ctaacatttg 180

gatcagggca aaggcgtttc caggagtgtt tttataccag acccctgctt agtccatggg 240

gctgaggcat gtcacataac tcagagcaag agccttctat ttgtgtaaga cccaaccctg 300

ggaaggatgc ccgagacaat agctatgcct ctgccccagc ctgatgccat gtgatctaga 360

gcctgggcag caacaaccct gtggtcaaag acatggaaga aacactctgc aaatcccaca 420

gtctgttcga cggcttggaa aggaagcaag tggcattggg acaattgctc tacatg 476

<210> 126

<211> 404

<212> DNA

<213> Homo sapiens

<400> 126
gaagccctct ccaggcgggc ccgccgactg gccgtgagga tgcgcgccct ggagagctcc 60

cagaggcccc gcgggtcgcc ggaccgcgct cccaaaacaa catctacagc gcctgcccgc 120

ggcgcgctcg tggagcggac gctgcaggca caggggaggc cccgttccc ggccccggag 180

cgccgttgcc ccccgccccg ctgcaggtgt ctgaatctcc ctggctccat gccccatctc 240

tgaagaccag ctgtgaatac gtgagcctct accaccagcc tgccgccatg atggaggaca 300

gtgattcaga tgactacatc aatgttcctg cctgacaact ccccagctat cccccaaccc 360

caggctcgga ctgtggtgcc aaggagtctc atctatctgc tgat 404

<210> 127

<211> 452

<212> DNA

<213> Homo sapiens

<400> 127
taattttaag tttgaatgta tattttgaaa taaaaatgga aaataacaaa tttgtacaaa 60

attgtcacat agaaacacac tttaagatac cattacatgc tgtgtgtatt tacaaaagtt 120

acaggtctag taagaaaaaa gaaactgtca ttgacaaatg cgagctcatt ttttgagcaa 180

caagaaatct cccgtgaaat gtcatactga cagccagtga gacttggtgc agtgacggct 240

ccgcacctgc tgaggcctgg agcctgcctc ccgggctgag tgctcagctc tggcctccgc 300

ctggatgccg tctagagtcc tatgtgctca taggtctttt tctttcagta aacagtataa 360

aacattacta ttttcctttg attgtaaact tcaacagaaa ataacatact taagaatttt 420

gctacaaaga gtactatgat ttttattgcc tc 452

<210> 128

<211> 201

<212> DNA

<213> Homo sapiens

<400> 128
gagaaagaaa ctaatatttt atgtgagaga aagtgtgagc aaactaactt gacttttaag 60

gctaaaactt aacattcata gaggggtgga gttttaactg taaggtgcta caatgcccct 120

ggatctacca gcataaatat cttctgattt gtccctatgc atatcagttg agcttcatat 180

accagcaata tatctgaaga g 201

<210> 129

<211> 417

<212> DNA

<213> Homo sapiens

<400> 129
aatatacata attttattac aaaatttttt ttaaaaaaac gaaatgcaac atcctaaaaa 60

acccaaaatt tactattgat actaattcct acaagtttgc tgtgctacca tacacaagaa 120

attaaaaaaa ccattaaata tttaggaaca ttcaacatca gaagctgtaa aatctaactg 180

tatgagtagc ccatcaaaaa gctacaacct gcattttttta aaagtatttt ctctacagag 240

aatcttatca gctatacaaa aatctgtaca gtttttatac tgaagctagt attgagctgc 300

acttgaattc acattcttag caaaataatt gcctgagcac acacacacat tccacacgca 360

tcattaaagg atagccattt attcttcatc ttcatcctct tcctcctcat cttcatc 417

<210> 130

<211> 468

<212> DNA

<213> Homo sapiens

<400> 130
aacgaactga atgcaacatt tttgaaagga gaggtttctg tggaactgga aaggggctct 60

ggttgtttcc agctcaccac caaaactggc tggccacaaa gaacccaggc aggatgtact 120

ctgctcagac acaccctcag agggtgggca ggagagagtg gacccacaac tcctgttagc 180

ccagaggagg ggttacagct cttgctgctt aacctgagaa atttccagaa atttcctacc 240

tgcttctact gcagaataat catctggcga ctgtttctag tgagcatgtc ccatgcaaca 300

ggctttcctg agcccccgaa agagcataca agtcaaagga aaaaggacat gaggatgatt 360

tatttggcag tcagatctta agagggcagc agaactagca aatggccaac cctgagccca 420

aatgtgggca gtaggtttgt gtgtgctgaa tgctggctat gctatgag 468

<210> 131

<211> 228

<212> DNA

<213> Homo sapiens

<220>

<221> misc_feature

<222> (169)..(169)

<223> a,t,g,c

<220>

<221> misc_feature

<222> (203)..(203)

<223> a,t,g,c

<220>

<221> misc_feature

<222> (218)..(218)

<223> a,t,g,c

<400> 131
ggaaacacac ttgtaccctg ctcccagcat cacaaggcac ttgtctacaa gtgtgtccca 60

acacagtcct gggccacttt ccccaccctg ggagcacata aagaagcttg ccaagggggg 120

cgtccttgct ccccagttgt cctgtttctg taacttatga tgtcttttnc ctgagatggg 180

ggctcagagg gggaaggcct gtnctgcatt gcttcccnat ggcccacg 228

<210> 132

<211> 323

<212> DNA

<213> Homo sapiens

<220>

<221> misc_feature

<222> (79)..(79)

<223> a,t,g,c

<220>

<221> misc_feature

<222> (297)..(297)

<223> a,t,g,c

<400> 132
tcatgtctgc ctggctgagt cacttcaggg atcccgtgct ggcaccagag agctgtaaga 60

gccacctgca atgggcagng tgtgtgcctg ggacctttca cttccctgct tgtcttctgg 120

ccgcgtttct gagaggcacg ccccccacac acatccatgc acacacgctg taattggcca 180

acttcctcct tctgttcttg gaatagcaac cgagacaaaa agcagttagc acgagtggct 240

tgggcttgcc aacactacaa tttcaggaca aaacccttgt aatttttcca aatggtnggg 300

gtttttaaat taggttggga ttt 323

<210> 133

<211> 232

<212> DNA

<213> Homo sapiens

<220>

<221> misc_feature

<222> (1)..(1)

<223> a,t,g,c

<220>

<221> misc_feature

<222> (94)..(94)

<223> a,t,g,c

<220>

<221> misc_feature

<222> (152)..(152)

<223> a,t,g,c

<220>

<221> misc_feature

<222> (188)..(188)

<223> a,t,g,c

<220>

<221> misc_feature

<222> (209)..(209)

<223> a,t,g,c

<400> 133
naaccgttaa acatttttat tgtgcaggct tgtaaccttt acaggacatg catgataaaa 60

aattgtaaaa tatatcagta agcatataaa attncagcaa tcttttggac cagtaattga 120

tttgcatgag ggactaagca tctatgtagt ancagacatg tcttggaata cacagtttac 180

agattgantc acaaagccat ttcctaagnt gattttcttc atataaggct aa 232

<210> 134

<211> 381

<212> DNA

<213> Homo sapiens

<220>

<221> misc_feature

<222> (1)..(1)

<223> a,t,g,c

<220>

<221> misc_feature

<222> (18)..(18)

<223> a,t,g,c

<220>

<221> misc_feature

<222> (20)..(20)

<223> a,t,g,c

<220>

<221> misc_feature

<222> (340)..(340)

<223> a,t,g,c

<220>

<221> misc_feature

<222> (366)..(366)

<223> a,t,g,c

<220>

<221> misc_feature

<222> (372)..(372)

<223> a,t,g,c

<400> 134
nattcggcac taggccgngn gttggtttag tggctcaaca ttgtgttccc atttcagctg 60

atcagtgggc ctccaaggag gggctgtaaa atggaggcca ttgtgtgagc ctatcagagt 120

tgctgcaaac ctgacccctg ctcagtaaag cacttgcaac catctgttat gctgtgacac 180

atggcccctc cccctgccag ggagctttgg gacctaatcc aaggcatccc tttgcccaga 240

aagaagatgg gggaggaggc agtaattaaa aagattgaag tattttgctg ggaataagtt 300

tcaaattctt cttgaacttc aaacttgagg ggattttcan cctgttaaac ctgagtccgt 360

accagnaagc tngcctgggg g 381

<210> 135

<211> 468

<212> DNA

<213> Homo sapiens

<220>

<221> misc_feature

<222> (127)..(127)

<223> a,t,g,c

<220>

<221> misc_feature

<222> (349)..(349)

<223> a,t,g,c

<220>

<221> misc_feature

<222> (366)..(366)

<223> a,t,g,c

<220>

<221> misc_feature

<222> (461)..(461)

<223> a,t,g,c

<400> 135
ctttgtagtc tatacattta ttgagtaaaa acaaaatcag tgtcagacac gttatatttg 60

attgggttca aatttggctg atgtccaaat gcagcagaga agaacggcgg ggcgaaggga 120

cacagtnttg ctgacaaggt gacactgaac acaacagttt tcctttaatt gtaaaagcgg 180

gcatcgacag ctggtgtgag tcaattaacc aaggcaggga ggggactcaa tgttttacaa 240

gcagagggaa aaccaaagta ggcagagaac tttcaagaga ggagagggcc agaacactga 300

ggaggaaaaa gctgcagcag aggccttgct gggagaacag tgggtcagnt cttctggctt 360

cacttngggg gaacacagtt gacccctttc attaaccctc agggtgggtg ttgacttcgc 420

ttcccggggc atgctttctt cacagtaggt tttgatccct nccaccaa 468

<210> 136

<211> 387

<212> DNA

<213> Homo sapiens

<400> 136
cagaagttgc ctatgtgtga caaatgtggc actgggattg ttggtgtgtt tgtgaagctg 60

cgggaccgtc accgccaccc tgagtgttat gtgtgcactg actgtggcac caacctgaaa 120

cagaagggcc atttctttgt ggaggatcaa atctactgtg agaagcatgc ccgggagcga 180

gtcacaccac ctgagggtta tgaagtggtc actgtgttcc ccaagtgagc cagcagatct 240

gaccactgtt ctccagcagg cctctgctgg cagctttttc tctcagtgtt ctgggccctc 300

tcctcttctt gaaagttctc tggcctactt tggtttttcc ctctggcttg taaaaacatt 360

gagtcccctt ccctgccttg ggttaat 387

<210> 137

<211> 290

<212> DNA

<213> Homo sapiens

<220>

<221> misc_feature

<222> (20)..(20)

<223> a,t,g,c

<220>

<221> misc_feature

<222> (189)..(189)

<223> a,t,g,c

<220>

<221> misc_feature

<222> (234)..(234)

<223> a,t,g,c

<220>

<221> misc_feature

<222> (256)..(256)

<223> a,t,g,c

<220>

<221> misc_feature

<222> (261)..(261)

<223> a,t,g,c

<220>

<221> misc_feature

<222> (267)..(267)

<223> a,t,g,c

<220>

<221> misc_feature

<222> (273)..(273)

<223> a,t,g,c

<400> 137
gagtttaatg attacatggn gctgagtcag gaggtaggag gagattctta aatctctgaa 60

gagttctggg ctggggttct gggaggcaag gggctggaaa atttgggcca ctgattggtc 120

agggtaaggg agattgaatc attaggatat ggaaattgca ttctttgatg atttagcttc 180

tggtagggnc cttcagacca ggctgacatc agtagtttca tcagtatgca gggncaacca 240

atcatggcca agtccncttt naggganttt gtncccgtag gatttatccg 290

<210> 138

<211> 417

<212> DNA

<213> Homo sapiens

<220>

<221> misc_feature

<222> (298)..(298)

<223> a,t,g,c

<220>

<221> misc_feature

<222> (345)..(345)

<223> a,t,g,c

<220>

<221> misc_feature

<222> (359)..(359)

<223> a,t,g,c

<220>

<221> misc_feature

<222> (386)..(386)

<223> a,t,g,c

<220>

<221> misc_feature

<222> (399)..(399)

<223> a,t,g,c

<400> 138
gctgatcgaa cagcctcact tgtgttgctg tcagtgccag tagggaggca ggaatgcagc 60

agagaggact cgccatcgtg gccttggctg tctgtgcggc cctacatgcc tcagaagcca 120

tacttcccat tgcctccagc tgttgcacgg aggtttcaca tcatatttcc agaaggctcc 180

tgggaaagag tgaatatgtg tcgcatccag agagctgatg gggattgtga cttgggctgc 240

tgtcatcctt catgttcaag cgcaggaagg aatctgtgtt cagcccgcac aaccatantg 300

tttaaggcag tgggatggaa agtggcaagc ttgcccaagg aaaangggtt aaagggaant 360

tttttgccac agggaaggaa acaccntggg caagagggna ccatttacca gggggca 417

<210> 139

<211> 442

<212> DNA

<213> Homo sapiens

<220>

<221> misc_feature

<222> (334)..(334)

<223> a,t,g,c

<220>
<221> misc_feature
<222> (342)..(342)
<223> a,t,g,c

<220>
<221> misc_feature
<222> (360)..(360)
<223> a,t,g,c

<220>
<221> misc_feature
<222> (384)..(384)
<223> a,t,g,c

<220>
<221> misc_feature
<222> (407)..(407)
<223> a,t,g,c

<220>
<221> misc_feature
<222> (437)..(437)
<223> a,t,g,c

<400> 139
acacttatcc cactttttat tttttacaaa aacgtgctca tttaatagaa atgattgatt 60
atataaaatt gtttacacat gtataaaaac taggaacacg tatgtaggta agcaatcaaa 120
atcatgcttt ctgttagaaa agttaacact tctgtgaata gactggatga cttcctttta 180
gtttggatgt ctgggtcgga ctcacgagac gctggggttg gggtgtgggt gcttgacatc 240

agcctctggc aaagcactaa taaaagggaa ccccaaagct gactgtgtac acaaatgggg 300
ctttccataa ggttcattac atttcctttt ccangtcagg gnaaacttca acagtgggtn 360
gctactgtgg ggtctgtccc ttgnaggttt ctggaggccg tgccaantgt taatatcccc 420
gcatccatcg tctccgnagt ct 442

<210> 140
<211> 380
<212> DNA
<213> Homo sapiens

<220>
<221> misc_feature
<222> (60)..(60)
<223> a,t,g,c

<220>
<221> misc_feature
<222> (329)..(329)
<223> a,t,g,c

<220>
<221> misc_feature
<222> (334)..(334)
<223> a,t,g,c

<220>
<221> misc_feature
<222> (349)..(349)
<223> a,t,g,c

<220>

<221> misc_feature

<222> (363)..(363)

<223> a,t,g,c

<220>

<221> misc_feature

<222> (368)..(368)

<223> a,t,g,c

<400> 140
caaccatctt ggcttcttca aacaaagtga aaagtgggga cgacatcagt gtgctctgcn 60

tgtcctgggg gagcccgatg tggaggtgga gttcacctgg atcttcccag ggcagaagga 120

tgaaaggcct gtgacgatcc aagacacttg gaggttgatc cacagaggac tgggacacac 180

cacgagaatc tcccagagtg tcattacagt ggaagacttc gagacgattg atgcaggata 240

ttacatttgc actgcttcag aatcttcaag gacagaccac agtagcttac cactgttgag 300

ttttcctgat tgggaaaagg gaatgttant gaanttatgg gaaagcccnt ttgtgttaca 360

cantcagntt tggggtttcc 380

<210> 141

<211> 500

<212> DNA

<213> Homo sapiens

<400> 141
ggagtgaaaa agacgctgta tttgatttac aatgaacaag atttacaaaa aggggtgggg 60

tggtcttgga actgctccca gtccccccgg actgggtggg gctctagggc agcctgtctg 120

acagaccagg accccaggat gtctgggccc cgacgtagga cttgacctac gtctcacttg 180

acctttgacg tggggcccag cagccgtgag tccacccaga gtgccggcac ccttggtgag 240

gccggtgagg tcaggaaggc atcgtaccgc tttttctcct cctcccatct cgtggtggac 300

agacagacat aggatctggg aacttgccct ggggcgcaca ggctcagtat cccccagggg 360

cccaacctag ggcatggagg cggctgctgg tgcgtgggcg gaggcggagg cagtctgccc 420

ccagcgtggc agcgtaaggc acattttcaa atcactcgag actcgacagt gaacacccga 480

tgctggttct gcggccggag 500

<210> 142

<211> 256

<212> DNA

<213> Homo sapiens

<220>

<221> misc_feature

<222> (53)..(53)

<223> a,t,g,c

<400> 142
gtgctgtgaa aaaaaaaat gctgaacatt gcatataact tatattgtaa ganatactgt 60

acaatgactt tattgcatct gggtagctgt aaggcatgaa ggatgccaag aagtttaagg 120

aatatgggag aaatagtgtg gaaattaaga agaaactagg tctgatattc aaatggacaa 180

actgccagtt ttgtttcctt tcactggcca cagttgtttg atgcattaaa agaaaataaa 240

aaaaagaaaa aagaga 256

<210> 143

<211> 434

<212> DNA

<213> Homo sapiens

<220>

<221> misc_feature

<222> (10)..(10)

<223> a,t,g,c

<220>

<221> misc_feature

<222> (120)..(120)

<223> a,t,g,c

<220>

<221> misc_feature

<222> (147)..(147)

<223> a,t,g,c

<220>

<221> misc_feature

<222> (197)..(197)

<223> a,t,g,c

<220>

<221> misc_feature

<222> (241)..(241)

<223> a,t,g,c

<220>

<221> misc_feature

<222> (249)..(249)

<223> a,t,g,c

<220>

<221> misc_feature

<222> (405)..(405)

<223> a,t,g,c

<220>

<221> misc_feature

<222> (421)..(421)

<223> a,t,g,c

<400> 143
tagcttaagn tatttattca atgctctcac atttggcctc tgtgacccag aaaagcggta 60

gagcagggta ggagcacatg atgcatcctt cagccactgc aacctccaga gggaccaggn 120

cttcttcagg aaatcttcgt tcctggngga tgactgatca gccataactg caagacacag 180

acatttagca cctccgnttg cagactaaac acccaccctg actgccctca tcccatgagg 240

ntccagggng cctttgcccc aagaagcctg ggtttccata ctacatccct caccatgggg 300

agaagtcctg cgttctccat ttctccatcg ttgggcttct cctggggagt catgccaatc 360

attgggtctg ggtccccgtt caaagttttc cacagggccc cacanagttt tcccagctgg 420

ngcatttgtt gaca 434

<210> 144

<211> 542

<212> DNA

<213> Homo sapiens

<220>

<221> misc_feature

<222> (23)..(23)

<223> a,t,g,c

<220>

<221> misc_feature

<222> (165)..(165)

<223> a,t,g,c

<220>

<221> misc_feature

<222> (321)..(321)

<223> a,t,g,c

<220>

<221> misc_feature

<222> (369)..(369)

<223> a,t,g,c

<220>

<221> misc_feature

<222> (376)..(376)

<223> a,t,g,c

<220>

<221> misc_feature

<222> (461)..(461)

<223> a,t,g,c

<220>

<221> misc_feature

<222> (472)..(472)

<223> a,t,g,c

<220>

<221> misc_feature

<222> (499)..(499)

<223> a,t,g,c

<220>

<221> misc_feature

<222> (506)..(506)

<223> a,t,g,c

<220>

<221> misc_feature

<222> (511)..(511)

<223> a,t,g,c

<220>

<221> misc_feature

<222> (526)..(526)

<223> a,t,g,c

<220>

<221> misc_feature

<222> (537)..(537)

<223> a,t,g,c

<400> 144
gcaaagattc acaatattta ttnattctcc tccaacatta gcataattaa agccaaggag 60

gaggaggggg gtgaggtgaa agatgagctg gaggaccgca ataggggtag gtcccctgtg 120

gaaaaagggt cagaggccaa aggatgggag ggggtcaggc tgganctgag gagcaggtgg 180

gggcacttct ccctctaaca ctctcccctg ttgaagctct ttgtgacggg cgagctcagg 240

ccctgatggg tgacttcgca ggcgtagact ttgtgtttct cgtagtctgc tttgctcagc 300

gtcagggtgc tgctgaggct ntagggtgct gtccttgctg tcctgctctg tgacactctc 360

ctgggggant tacccnattt gggagggcgt tatccacctt ccactgtact ttggcctctc 420

tggggataga agtttttca gcaggcacac aacagaggca nttccagatt tncaactgct 480

catcagatgg ccgggaagnt gaaggncagt nggtgcagcc acattncttt tgatccncca 540

ct 542

<210> 145

<211> 514

<212> DNA

<213> Homo sapiens

<220>
<221> misc_feature
<222> (390)..(390)
<223> a,t,g,c

<220>
<221> misc_feature
<222> (418)..(418)
<223> a,t,g,c

<220>
<221> misc_feature
<222> (436)..(436)
<223> a,t,g,c

<220>
<221> misc_feature
<222> (459)..(459)
<223> a,t,g,c

<220>
<221> misc_feature
<222> (470)..(470)
<223> a,t,g,c

<220>
<221> misc_feature
<222> (496)..(496)
<223> a,t,g,c

<220>

<221> misc_feature

<222> (502)..(502)

<223> a,t,g,c

<400> 145
cagcctcccg cgcctcgctc agctccaaca tggcaaaaat ctccagccct acagagactg 60

agcggtgcat cgagtccctg attgctgtct tccagaagta tgctggaaag gatggttata 120

actacactct ctccaagaca gagttcctaa gcttcatgaa tacagaacta gctgccttca 180

caaagaacca gaaggaccct ggtgtccttg accgcatgat gaagaaactg gacaccaaca 240

gtgatggtca gctagatttc tcagaatttc ttaatctgat tggtggccta gctatggctt 300

gccatggact ccttcctcaa ggctgtccct tcccagaagc gggacctgga gggacccctt 360

gggccctggg cctttcaaac ccacccccctn ttcctttcca gcctttctgt tcatcatntt 420

ccacagccca cccttncctg gaggcacatt aaccacctna tggtagggtn ccaactggtc 480

attagttatt aaaggnaatg tnaatttttt ttaa 514

<210> 146

<211> 514

<212> DNA

<213> Homo sapiens

<220>

<221> misc_feature

<222> (383)..(383)

<223> a,t,g,c

<400> 146
tttttttttt tttttttttt agtataaaca tgattaatta gcttttaata ctactctttg 60

atgtattact catattacca aggaataact ggcgggcaca gggtcaggtg ctgaagggac 120

attgtgagaa gtgacctaaa aggcaagagg tgagccctct gtcacgctgg cataagggcc 180

gcttgagggc tctttggtca agcagtaacg ccagtgtctg ggaaggcacc tgttactcag 240

cagaccatga aagggcgtct ccctttcctt ggaggagtca gggaacactc tgctccacca 300

gcttcttgtg ggaggctgga tattatccag gcctgcccgc agtcatccgg aggcctaacc 360

cctccctgtg gtgcttcagt ggncacactc cttgtccact ttcatgctcc tcttggcctc 420

ctggttccct tttgaagctt tgagtacata gccagagaag aaatagcgaa agtccttaag 480

tcttgatctt tcttttaggg ccgagaaaaa atgc 514

<210> 147

<211> 192

<212> DNA

<213> Homo sapiens

<220>

<221> misc_feature

<222> (192)..(192)

<223> a,t,g,c

<400> 147
catgtgggaa gtgatgagtt atggagaacg gccttactgg gacatgagtg agcaggaggt 60

actaaatgca atagagcagg agttccggct gcccccgcct ccaggctgtc ctcctggatt 120

acatctactt atgttggaca cttggcagaa ggaccgtgcc cggcggcctc attttgacca 180

gctggtgggc tn 192

<210> 148

<211> 370

<212> DNA

<213> Homo sapiens

<220>

<221> misc_feature

<222> (138)..(138)

<223> a,t,g,c

<220>

<221> misc_feature

<222> (255)..(255)

<223> a,t,g,c

<220>

<221> misc_feature

<222> (265)..(265)

<223> a,t,g,c

<220>

<221> misc_feature

<222> (282)..(282)

<223> a,t,g,c

<400> 148
acttgtttga ggtgatttag gattaccaac tctgggaaaa aatattcaca gccacattat 60

aaaaaacatt atcggcagga ttcatctcct gctcctttag aataagaact tttcttttgt 120

ttgtggaact ttggaatnta caagcaagac gttcattcat gctttaaaat gctgtttaac 180

aattgcttag cactttacca gggagaatgt ttctcctgag tgtttaccac cctattctgt 240

aatacagttg agggnccaca tcaangctat ctattccttc cngggcccag aggaacacac 300

acatttaatc tgaggtgcat tgaggtacca caggctgagt gggaggaaag ggattagggg 360

agggcaatta 370

<210> 149

<211> 351

<212> DNA

<213> Homo sapiens

<220>

<221> misc_feature

<222> (253)..(253)

<223> a,t,g,c

<400> 149
```
ggcatatgca tagtgaaagg agtgagggct gcaagttcat ggatgcatta ttttctcgtt      60

ttcgtttgta gggaaataca tgctctgtca ttgacttgca aggattttct ttttattctt     120

aattgcctcc ctaatccttt ctccactcag ctgtggtact caatgcactc agattaaatg     180

tgtgtgttct tctgggccag gaaggaatag atagcattga tgtggaccct caactgtatt     240

acagaatagg gtngtaaaca ctcaggagaa acattctccc tgggtaaagt gctaagcaat     300

tgttaaacag cattttaaag catgaatgaa cgtcttgctt gtacattcca a              351
```

<210> 150

<211> 414

<212> DNA

<213> Homo sapiens

<400> 150
```
tgtctgaaag tatgcttttt attgcagcca taaaccttaa tacagatctt tttgttatta      60

tcaatattat ttaatattag tggcagccac atgagcgtac taccatattt ctatattttt     120

tcaaaatgac attggagctg tcatcaaagt acagaacaga gatggcattt aatttggttg     180

gagcacaaca aggctttggt acgtggtcag gaaacatcag atgaaccaga gtctgaacta     240

tagcgtggtt ggtggcattc atatgggcgt taagtggaaa agaacattct ccatcacaat     300

aaaatgcagc gtatccttct ggtgctataa tccagtcctg ccatcccaga tcccggaagc     360

tcacatagag ttcgtgcttc ttacaggctt gttttgctca cttgtgtata atct           414
```

<210> 151

<211> 439

<212> DNA

<213> Homo sapiens

<400> 151
```
tgtaactcca gttttacttt aagaggataa tacagatttt tgtagctggg gaaggtgagt      60
```

gggaaggtag aggttgtgga aaggctggga aacatcagaa tatatttatg acacaagaca 120

tttgaccaac ccaagcaatg ctttgtgcct gtgcctccag acacacacac acacacacac 180

acgcacacac acacacacac catgtaaggc accactggat tatagcatgg aggggacaaa 240

tgccccctgc cctaaacctg gccacagcca gcccatcccc ctgtgggccc tctgtccaag 300

tcctgtgggg agcgtggctt tgctactcaa tggcaactgg atttcaagag tttcaggaag 360

ggtgggggag caagatatca aaggctcaag ctcactcccc ttcgtccaga cagacttttc 420

atttttgtt tgatgaaga 439

<210> 152

<211> 389

<212> DNA

<213> Homo sapiens

<220>

<221> misc_feature

<222> (3)..(3)

<223> a,t,g,c

<400> 152
aanaaaaaaa tctgggaggg gcatttttat aggacccata accattgaca gttaatatca 60

gccacaataa gggaccttca ggagccgctt tctagaccca gggtctctgg gaagcctcat 120

cccacaccct ctcctgctgt ccagggccat ctgggagctg gtctgagtgt ccactgagtc 180

cgtttatttg gcggtctgtc tcactgggtt tgcacgacag tttggacatc tctgtgtggc 240

tccctgtggc tgaaggcttg tcggattttc cgtaagaggc tcccccaggg ctgtctatgg 300

gtccgtgttt gatatttggg tggatctttg gggaacgcga gtccagggag agggtccatt 360

cgtggggaaa accacccagc attgtgtca 389

<210> 153

<211> 474

<212> DNA

<213> Homo sapiens

<220>

<221> misc_feature

<222> (71)..(71)

<223> a,t,g,c

<220>

<221> misc_feature

<222> (319)..(319)

<223> a,t,g,c

<220>

<221> misc_feature

<222> (427)..(427)

<223> a,t,g,c

<220>

<221> misc_feature

<222> (442)..(442)

<223> a,t,g,c

<220>

<221> misc_feature

<222> (456)..(456)

<223> a,t,g,c

<220>

<221> misc_feature

<222> (472)..(472)

<223> a,t,g,c

<400> 153

ggctgcggct ggagattcgc gcgctcggac gactgacccg ccactaccgg aaacacacgg 60

ggcagcgctt nccgctgcca gctctgccca cgtgcttttt cgcgctctga ccacctggcc 120

ttgcacatga agcgccacct ttgagccctg ccctggcact tggactctcc tagtgactgg 180

ggatgggaca agaagcctgt ttggtggtct cttcacacgg acgcgcgtga cacaatgctg 240

ggtggttttc ccacgaatgg accctctcct gggactcgcg ttcccaaaga tccacccaaa 300

tatcaaacac ggacccatna gacagccctg ggggagcctc ttacggaaaa tccgacaagc 360

ttttagccac agggagccac acagagatgt tccaaatttt cgttgcaaac ccagtgagac 420

agaccgncaa ttaaacgggt tnattggaca tttagnccag tttccagatg gnct 474

<210> 154

<211> 423

<212> DNA

<213> Homo sapiens

<220>

<221> misc_feature

<222> (14)..(14)

<223> a,t,g,c

<220>

<221> misc_feature

<222> (346)..(346)

<223> a,t,g,c

<220>

<221> misc_feature

<222> (398)..(398)

<223> a,t,g,c

<400> 154
ggcacgagca aganaggagt tctctgatgc agaaattatt gggctctttt agggtaagaa 60

gtttgtgtct ttgtctggcc acatcttgac taggtattgt ctactctgaa gacctttaat 120

ggcttccctc tttcatctcc tgagtatgta acttgcaatg ggcagctatc cagtgacttg 180

ttctgagtaa gtgtgttcat taatgtttat ttagctctga agcaagagtg atatactcca 240

gggacttaga atagtgccta aagtgctgca gccaaagaca gagcggaact atgaaaagtg 300

ggcttggaga tggcaggaga gcttgtcatt gagcctgggc aatttnagca aactgatgtc 360

tgaggatgat tcgaggtggg tcttacctca tctactgnaa aattctggta aggaatggga 420

ggg 423

<210> 155

<211> 477

<212> DNA

<213> Homo sapiens

<400> 155
tttttttca ttgccataat tttttattat gtatcaaatt gtcttcaata taagttacaa 60

cttgattaaa gttgatagac atttgtatct atttaaagac aaaaaaattc ttttatgtac 120

aatatcttgt ctagagtcta gcaaatatag tacctttcat tgcaggattt ctgcttaata 180

taacaagcaa aaacaaacaa ctgaaaaaat ataaaccaaa gcaaaccaaa ccccccgctc 240

aactacaaat gtcaatattg aatgaagcat taaaagacaa acataaagta acttcagctt 300

ttatctagca atgcagaatg aatactaaaa ttagtggcaa aaaaacaaac aacaaacaac 360

aaacaaaaca aaacaaacaa acaaaaaatc ccaccaatct tcatgggtaa actttcctgc 420

tcagggatgt aagctgactc tagaccatct cgcggttcct gcggatagca cagcaca 477

<210> 156

<211> 477

<212> DNA

<213> Homo sapiens

<220>

<221> misc_feature

<222> (277)..(277)

<223> a,t,g,c

<220>

<221> misc_feature

<222> (386)..(386)

<223> a,t,g,c

<220>

<221> misc_feature

<222> (428)..(428)

<223> a,t,g,c

<220>

<221> misc_feature

<222> (433)..(433)

<223> a,t,g,c

<220>

<221> misc_feature

<222> (449)..(449)

<223> a,t,g,c

<220>

<221> misc_feature

<222> (461)..(461)

<223> a,t,g,c

<220>

<221> misc_feature

<222> (468)..(468)

<223> a,t,g,c

<400> 156
gtagcgggtg ggccctggcc cagcgtcccc tgccagcatc ctcgtgggct cacagacccc 60

ggcctcggag cccgtctggc acccagagtg accacaagtc cagcagggag gcggcgccgc 120

catcgccgtg tccgtgtttt ctttttcagc cccggagagg gtcctgacct gggggcttct 180

ccaagcctca ctgcgccagc tccccgcccg ctctcttttc tcccaagcaa aaccaaatgc 240

gccccttcac ctcgcgtgcc cgtgcgaggc cgggggnttc tttcagagcc cgcgggtcct 300

ctcatacatg gcttctgttt ctgccgagag atctgttttc caattatgaa gccggtcggt 360

ttggtcagac tcccgacaac cacgtnccag gtaaccggtg ggaaaagtgg gcagttccga 420

gggcgcanat ttngggtggt tggcagggnc cccagaggtt ncgggttnaa ctgggga 477

<210> 157

<211> 608

<212> DNA

<213> Homo sapiens

<220>

<221> misc_feature

<222> (590)..(590)

<223> a,t,g,c

<400> 157
atcatttttt ttttgtttca ttttgttttg aacactaaga tttattttca aacagcacac 60

agaccgtctg cggggcagag ccaggctagg ctggtgtctg ggccccaccc acagcagctg 120

ccaggaaaag aggacccttg cccgggtggc gcggccgaag cttcaggcaa gcatggtggc 180

tcggcagccc ccagccccgc cctgcggcca ggcacacatg cgggcacagg caggggcgcc 240

agaaactcaa ctagaggaca cagcagcttc aggaacactg gtgaattccg ccggacttgc 300

cgggacgcgg ctctttggaa aacgacctaa tctttgggag aacgcccctc tgcctggggg 360

tctcctcttg atttcccttt gctcttcaaa agatgaaaaa cgaaaacaaa acaaaaaaaa 420

gaaccacaca tttttcggga ggaggtgttc ttcacacgcc cggaggctgc ctgggccccg 480

ccgtcatggg accctctcag tgaagttctt cgggaagacg ccacggcact tctccagctc 540

cttgtgctgg tccagtcgct cttcttcacg cccatgagcc agccttcatn ctgctcttta 600

ggggtctg 608

<210> 158

<211> 553

<212> DNA

<213> Homo sapiens

<220>

<221> misc_feature

<222> (391)..(391)

<223> a,t,g,c

<220>

<221> misc_feature

<222> (502)..(502)

<223> a,t,g,c

<220>

<221> misc_feature

<222> (525)..(525)

<223> a,t,g,c

<220>

<221> misc_feature

<222> (535)..(535)

<223> a,t,g,c

<400> 158
ttagactgca cactagaatt tgattcagaa tcaaggtttg taacaagctg aaagtgactc 60

gtcagttcag ttttgcagat agatgagcag tcccttgttt ccttggaaac tcggacctga 120

gcatatgcag cttggaatgt gcaccttcat gttttcaatc gagcagatac aggatgagtt 180

tctcccaaga gattcaatat gttgcctttc ttttccttga tgtagcttct agtggtgacc 240

aggtggtttc agtccattca agtctcaagt aaaactccaa gtaacgaggt gactgttgca 300

tcttttaaaa atgagtattg tacacatact tcttccaagg caaatactta gtcaaaacta 360

aacaaatttg tagagggcca aattgtcctg ntgtttctca gtttatttat ttcataataa 420

cagccaccaa tacaggtggg tttgaatgtt ttccaaaaat aaacaactac tacccaaaat 480

aaaacaaaca gggttaggat cnggtttcct ggtccatact tctcnctgtc cttcncactg 540

ggggtcccta cct 553

<210> 159

<211> 370

<212> DNA

<213> Homo sapiens

<220>

<221> misc_feature

<222> (277)..(277)

<223> a,t,g,c

<400> 159
tttttttttt tatggtatat ttctttttac tcttaaaaaa aaaaaagttc acaatgataa 60

cctgcaaact tgcagaaagc gccacgggtt tcaagctcct caccttcgga acatcaccct 120

atccttcccc ttcccttaaa atcctagatg aaaattcccg agaaagcaga agaggccccc 180

agatgggcgg attcccaatc ccggacccct gcggcaggtc gaggcattga agaaataaat 240

taaaggagag gtggctcctg ggctggcctt gtccagntct gtctcgcaga cccagcggta 300

gggcctctgg gaggacgtcg tcgttccagc ggccgtcgtc gggtgaagtg gggcacagtc 360

cttcgccttc 370

<210> 160

<211> 413

<212> DNA

<213> Homo sapiens

<220>

<221> misc_feature

<222> (97)..(97)

<223> a,t,g,c

<220>

<221> misc_feature

<222> (215)..(215)

<223> a,t,g,c

<400> 160
accgcccgca tttcttcatg tacatggttc ctcctagact actagggccg ccttagcttg 60

ctaccctttt aggaccctgg agctgtgcca gggtccnctc tgtccccgcg ctcctgacac 120

cccctcctct tgcagggcca cctcctcccc agcccctcct gcagcgtctc tgctccggac 180

ctcgcctcct cctgctctcc ctgggcctca gcctncctgc tgcttgtggt tgtctgtgtg 240

atcggatccc aaaactccca gctgcaggag gagctgcggg gcctgagaga gacgttcagc 300

aactttcaca gcgagcacgg aggcccaggg tcaagggttt gagcacccag gggaggcatt 360

tggggaagaa agatgaattc gttagagttc ccagtttgga gaaaacagca gaa 413

<210> 161

<211> 442

<212> DNA

<213> Homo sapiens

<220>

<221> misc_feature

<222> (50)..(50)

<223> a,t,g,c

<220>

<221> misc_feature

<222> (377)..(377)

<223> a,t,g,c

<400> 161
tacttaacag attatatgtg tcctgtgata gcagggttat gttttaaccn tctttgcctt 60

tcccagtatc ttgcaaagag taaataattt ctaaacatat attatcattg ttgccatttt 120

tcttgataat tatgatgaca aggagaagaa ttttctactt tacttccacc atatttaaaa 180

attgaaatac aaactcacaa aaatgggtag tgtcctgatt attgtagtca tgcatacatg 240

gagtatgttt gaaaggattt gttttaaggc gaaacacagt tttttaaatt agttaaaaat 300

aaaagcatat actatgcact ttatgtgctt aaggcatgct atgattataa ttatagagat 360

attaaaaatg aattttnggc caagtatggt ggcttacacc tgtaatccca acactgggag 420

atcgaggtag gcagatcgct tt 442

<210> 162

<211> 466

<212> DNA

<213> Homo sapiens

<220>

<221> misc_feature

<222> (434)..(434)

<223> a,t,g,c

<220>

<221> misc_feature

<222> (452)..(452)

<223> a,t,g,c

<400> 162
tctctttgta agtaacttta tttttattta caacagaatt ggtggcttta ttcctccatc 60

tttagggaca cttggcatta gcagctagat ggaaagtccg cagtgaagtc aaactcattc 120

tgccccagcc acagctccgg aagctcattg gctcggtcca accccagttc caccaccagc 180

gacatcagca cttcctcatc cactgggtcc gaatcgatga tagcagggct ctgggcacca 240

gcagaaggag agagtgattc tgcccctccc gcctgggccc caaagtccca gttttgcagg 300

ggtcctgcct ccccgggttg gcctggagtg gcaagcagca tcccctgata ctgggctatt 360

aagtttctgc agctgcatac tagccagcaa gtgaggggcg gggtgcaggt tgaaggatgg 420

gggtttagtg ggangggtgg ttgtaggaga gnctattgga gattcc 466

<210> 163

<211> 515

<212> DNA

<213> Homo sapiens

<220>

<221> misc_feature

<222> (456)..(456)

<223> a,t,g,c

<220>

<221> misc_feature

<222> (507)..(507)

<223> a,t,g,c

<400> 163
cggacttgga gtcagagaga cctggctgcg aatcccggct ccgcactttt ctagcgctgt 60

gaccttggtt ttcaggtggc gaaactgact ggtctggtta gagcgcgtcc aagccaagcc 120

gcagtgctgt ttggattttt aaatttctcc ttctgagtga gaaaatagca atcgagatgg 180

aaccatccgc acaacagctc cagctggcag catcacttcc cgccaattta tccaacttct 240

gccaaggctc tgaaatgcca acaacgtcga ggcctgcact tgatgtcaag ggtggcacct 300

cacctgcgaa ggaggatgcc aaccaagaga tgagctccgt ggcctactcc aaccttgcgg 360

tgaaagatcg caaagcagtg gccattctgc actaccctgg ggtagcctca aatggaacca 420

aaggccagtg gggctcccca ctaagttcct cgggantctc caataggctc tcctacaacc 480

acccccttcca ctaaaccccc atccttnaaa ccttt 515

<210> 164

<211> 333

<212> DNA

<213> Homo sapiens

<220>

<221> misc_feature

<222> (177)..(177)

<223> a,t,g,c

<220>

<221> misc_feature

<222> (264)..(264)

<223> a,t,g,c

<400> 164
ttttttttt ttctgcaagt ggtggaattt taatattgta taaaaaatcc aacttgttcc 60

acaagtacat atgtcctatg attttatgca tacatccata tacatatatc aaggtaaagt 120

ccaatacaaa aaaacagcat ttcctatggc cagtgttcta cagaagtaag actgtgncaa 180

actttatcgt atagtcaaat gagtattgac acactaagga caggatgagg cagaagcaag 240

ttgtgtccac agtatattac aaantacctt gcatagctta ttcattctca cctgggtaaa 300

ttcatcttag gaattctgaa gggttttttt cct 333

<210> 165

<211> 404

<212> DNA

<213> Homo sapiens

<220>

<221> misc_feature

<222> (54)..(54)

<223> a,t,g,c

<220>

<221> misc_feature

<222> (149)..(149)

<223> a,t,g,c

<220>

<221> misc_feature

<222> (151)..(151)

<223> a,t,g,c

<400> 165
cagagttcac tctgagaaac ttcaactcag ccaaagacat gaaaaaagcc gtgnccacat 60

gaaatacatg ggaaagggct ctatgactgg gctggcctga aacacatgtt tgagagaagt 120

tttacccaag gagaagggc caggccttnt nccacaaggg tgcccagagc agccattgtg 180

ttcaccgacg gacgggctca ggatgacgtc tccgagtggg ccagtaaagc caaggccaat 240

ggtattcact atgtatgctg ttgggggtag ggaaaagcca ttgagggagg gaactacaag 300

agatttgcct cttgagccca caaacaaggc atctctttct attgcccgaa ggattttcag 360

cacattggat tgaggttaag ttgaaaaaac ttccaaggaa gggc 404

<210> 166

<211> 466

<212> DNA

<213> Homo sapiens

<400> 166
ttgacactat atgattccat ttatatgaaa tatccagaat aggtaaagcc atagcaacaa 60

agtagattgg tgggtttcca ggagctggga ggagggggaa ataggaacac ttgcataatg 120

gttatgggtc ttgcttttgg ggtgatgaaa atatttggaa ctagacagag atgctggttg 180

cacatcatga tatctagttg ccacggaatt gtttacttta aaatggttaa tttcatctta 240

tataaatttc acctccatca aaatgaaata ttttagaaag agaaagttaa aaaaaaaaaa 300

cctatgtgtt ccaccgaaca caacccaacc cttgctctca ttcccatttc ctggttccct 360

gtgtgcccat tgtcagtgcc atgggtatca aatccagtgc ccattgtcag tgccatgggt 420

atcaaatcag cttaatgatg cagattactg ttggggcctg ttcctg 466

<210> 167

<211> 453

<212> DNA

<213> Homo sapiens

<220>

<221> misc_feature

<222> (440)..(440)

<223> a,t,g,c

<400> 167
caccatcaag gaggaaggcc tggttcttca agcctatgga aattcctaga gacagataaa 60

tgtgcaaacc tgttcatctt gccaagaaaa atccgctttt ctgccacaga aacaaaatat 120

tgggaaagag tcttgtggtg aaacacccat cgttctctgc taaaacattt ggttgctact 180

cgtgtagact cagcttaagt catggaattc tagaggatgt atctcacaag taggatcaag 240

aacaagccca acagtaatct gcatcataag ctgatttgat accatggcac tgacaatggg 300

cactgatttg ataccatggc actgacaatg ggcacacagg ggaacaggga aattgggaat 360

tgagagccaa gggtttgggg tttgtgttcc gtgggaacac catagggttt tttttttttt 420

ttaaactttt cccctttctn aaaaatattt tca 453

<210> 168

<211> 186

<212> DNA

<213> Homo sapiens

<220>

<221> misc_feature

<222> (131)..(131)

<223> a,t,g,c

<220>

<221> misc_feature

<222> (158)..(158)

<223> a,t,g,c

<400> 168
aagaggtgtt tagtgtaatg agtgttcaga tgggtggggc agggtggggt tcccccatct 60

gaccctctag ggctctgggg tgactcattc ttccttgggc tcagaggtgg agctgatatt 120

tgttcctcca nttgagccct gctccccctg gcccttgngt ttctcagtgt ctccagacac 180

tagagc 186

<210> 169

<211> 510

<212> DNA

<213> Homo sapiens

<220>

<221> misc_feature

<222> (357)..(357)

<223> a,t,g,c

<220>

<221> misc_feature

<222> (411)..(411)

<223> a,t,g,c

<220>

<221> misc_feature

<222> (434)..(434)

<223> a,t,g,c

<220>
<221> misc_feature
<222> (454)..(454)
<223> a,t,g,c

<220>
<221> misc_feature
<222> (466)..(466)
<223> a,t,g,c

<220>
<221> misc_feature
<222> (503)..(503)
<223> a,t,g,c

<220>
<221> misc_feature
<222> (432)..(432)
<223> a,t,g,c

<400> 169
tttttttaa ccgttaaaca tttttattgt gcaggcttgt aacctttaca ggacatgcat 60
gataaaaaat tgtaaaatat atcagtaagc atataaaatt tcagcaatct tttggaccag 120
taattgattt gcatgaggga ctaagcatct atgtagaaca gacatgtctt ggaatacaca 180
gtttacagat tgaatcacaa agccatttcc taagtgattt tcttcatata aagataaaaa 240
tatctttaca tttaaaaagt atgcagtata gcacattttc agttatgtac acagtttaaa 300
agtaaaacta tatccatgcc agtgtctgtt ttaaatgcaa aaagtcaaag taggtancag 360
gttggtaatt aaagtgtcag gaagctggaa gaggcaaaaa caaccagagt ncaataagtg 420
tatgaaaaaa aaanatactg aggtttaaga aagncccaag cagaanccat tgacagagga 480
ataaaacttt ggatatccag canttctgtc 510

<210> 170

<211> 448

<212> DNA

<213> Homo sapiens

<220>

<221> misc_feature

<222> (432)..(432)

<223> a, t, g, c

<400> 170
ccaaactaat ccatcaccgg ggtggtttag tggctcaaca ttgtgttccc atttcagctg 60

atcagtgggc ctccaaggag gggctgtaaa atggaggcca ttgtgtgagc ctatcagagt 120

tgctgcaaac ctgaccccctg ctcagtaaag cacttgcaac cgtctgttat gctgtgacac 180

atggcccctc cccctgccag gaagctttgg acctaatccc aaagcaatct ctttgcccag 240

aaagaagatg ggggaaggag gcagtaataa aaagattgaa gtattttgct ggaataagtt 300

caaattcttc tgaactcaaa ctgaggaaat ttcacctgta aaccctgagt cggtacagaa 360

agctgcctgg tatatccaaa agctttttaa ttcctccctg gctcatattg tggattccgg 420

ccttttgggg ancttttcct taaacctt 448

<210> 171

<211> 377

<212> DNA

<213> Homo sapiens

<220>

<221> misc_feature

<222> (97)..(97)

<223> a,t,g,c

<220>

<221> misc_feature

<222> (203)..(203)

<223> a,t,g,c

<220>

<221> misc_feature

<222> (327)..(327)

<223> a,t,g,c

<220>

<221> misc_feature

<222> (332)..(332)

<223> a,t,g,c

<220>

<221> misc_feature

<222> (409)..(409)

<223> a,t,g,c

<400> 171
gggttttac cagttttatt tctagacttt catgtttgtc tttttgtctt ctgctggaaa 60

catgccggtt acatgttggt gctgggaagc gccgcgntgc aaccagaaat gcacagaccc 120

agccgcccgc cgcccagacc ctcagacttg cgcgtcacag gacagactcc gctgtgcccc 180

gtgcacttgc caccagcctt tgnctctcga tacacacaac atccaggact tgtgcccttg 240

ccccatcacg acagacaaag cgtccctcaa ggcccccgcg tggttcagac agacgccgca 300

gccaggatgg ttcgaggtaa gcgtganccg tngttctgga ggggtggggc ggggtgggcc 360

tgtgccgaag atggcct 377

<210> 172

<211> 434

<212> DNA

<213> Homo sapiens

<400> 172
agctatggaa gttgcataat tattattatt attattataa caagtgtgtc ttacgtgcca 60

ccacggcgtt gtacctgtag gactctcatt cgggatgatt ggaatagctt ctggaatttg 120

ttcaagtttt gggtatgttt aatctgttat gtactagtgt tctgtttgtt attgttttgt 180

taattacacc ataatgctaa tttaaagaga ctccaaatct caatgaagcc agctcacagt 240

gctgtgtgcc ccggtcacct aagcaagctg cccgaaccaa aagaatttgc accccgctgc 300

gggcccaccg tggttggggc ccctgccaat ggcagggtca atcctgtgct cggaggccat 360

ctcgggcaca ggccaccccc gccccacccc tccagaacac ggctcacgct tacctcaacc 420

atcctgggct gcgg 434

<210> 173

<211> 413

<212> DNA

<213> Homo sapiens

<220>

<221> misc_feature

<222> (409)..(409)

<223> a, t, g, c

<400> 173
attatgccag agaaatgaag tttattggga agtaaatgaa gtctcctcct tgcattccag 60

agaatgccaa agaaaaaagg aaaacaggaa atgcagtagt acgttcagga aacaccttaa 120

gtagtaactg ggagctcaga ttacagggag ctgaacatct ttagaaacaa acaggctcga 180

ccctcaacag gaaggagttg tccagacatt ggaagttgat gatgtgtgtc tttgcttggg 240

tcaggaaagc ttttcagcat tctagaaata acatagaggc aagatgaggt ttcttgatgt 300

cagctgagct ccagaaaagt caggacttga ggatccttcc tgtggttgat ctaaatggct 360

ttgtagatgt cttcaaagag gatacaagct tttcaattac agcaacccnt ttg 413

<210> 174

<211> 464

<212> DNA

<213> Homo sapiens

<400> 174
ctggagagtg aggactgcaa gctcccctgc aacccatgcg ccaccaccaa tgctagtggc 60

aactcctgtg gaccctgtgg cacctctcaa aagggttgct gtaattgaaa agcttgtatc 120

ctctttgaag acatctacaa agccatttag atcaaccaca ggaaggatcc tcaagtcctc 180

gacttttctg gagctcagct gacatcaaga aacctcatct tgcctctatg ttatttctag 240

aatgctgaaa agctttcctg acccaagcaa agacacacat catcaacttc caatgtctgg 300

acaactcctt cctgttgagg gtcgagcctg tttgtttcta aagatgttca gctccctgta 360

atctgagctc cagttactac ttaaggtgtt tcctgaaccg tactactgca tttcctgttt 420

tccttttttc tttggcattc tctggaatgc aaggaggaga cttc 464

<210> 175

<211> 368

<212> DNA

<213> Homo sapiens

<220>

<221> misc_feature

<222> (11)..(11)

<223> a,t,g,c

<220>

<221> misc_feature

<222> (48)..(48)

<223> a,t,g,c

<220>

<221> misc_feature

<222> (205)..(205)

<223> a,t,g,c

<220>

<221> misc_feature

<222> (347)..(347)

<223> a,t,g,c

<220>

<221> misc_feature

<222> (349)..(349)

<223> a,t,g,c

<220>

<221> misc_feature

<222> (351)..(351)

<223> a,t,g,c

<400> 175
ctgaaaaaat ngaaggcaca tttattaaat gactgggaga aattccanag tatgtagaat 60

gggaataata atacataaca ttgtatttta tgttccattt tttaaaatga gtccaaggaa 120

gttaaaatat tcttttaatt aagacactca aagaaatgga aataagaaaa attgatgcaa 180

ggactccttc aagttaagat ttgtnataca aatattttca tcttttaaca gggcaagctg 240

atgtgttcac atctcagttt caagctgcct ctttcactag gaacatcagt attttttttt 300

aaaagcacat tttacaatgc tttcccatca cccttggctg tggtttntng ntagcaccta 360

ttagccat 368

<210> 176

<211> 426

<212> DNA

<213> Homo sapiens

<220>

<221> misc_feature

<222> (378)..(378)

<223> a,t,g,c

<400> 176
ctgggaaaag tttctttaat aaaaaagttc tagtacatat acacgattgt cctcaccctt 60

catctatagc aacgcaacag ggaaaataaa aaataagggg caacctaggc acactcagta 120

taaaaacgca gagatccatc cgaatgggag gcattcgggg tctggaaacc agaaatgcag 180

gacggccagt gggccagcag ctctgggctg cacttttgaa gaactttctc taacgttttg 240

aagatagcat taaaaaaaaa aattaagttg caccaggagg cctaagaagg ttccattcca 300

gagagaaagt ccactcgtaa ggtcagtcac agcaggccca ggcccaggac acagggttcg 360

agcattttac ggcaggtnaa gaggcttctg gaaacttggc ctctgaggca accctggccc 420

aggtga 426

<210> 177

<211> 437

<212> DNA

<213> Homo sapiens

<220>

<221> misc_feature

<222> (26)..(26)

<223> a,t,g,c

<220>

<221> misc_feature

<222> (27)..(27)

<223> a,t,g,c

<220>

<221> misc_feature

<222> (392)..(392)

<223> a,t,g,c

<400> 177
gcgacgacta ggcggcctgc tgcagnnggg cggaccaaac gtcaggccac agagcatctc 60

caaggggtct cagttccccc ttcagctgag gacttcggag cttgtcagga agtggccgta 120

gcaacttggc ggagaaggct atgagtctga cgttagagtg gttgcttcct tagcctttca 180

ggatggagga atgtgggcag tttgacttca gcactgaaaa cctctccacc tgggccaggg 240

ttgcctcaga ggccaagttt ccagaagcct cttacctgcc gtaaaatgct caaccctgtg 300

tcctgggcct gggcctgctg tgactgacct acagttggac tttctctctg gaatggaacc 360

ttcttaggcc tcctggtgca acttaatttt tntttttaat gctatcttca aaacgttaga 420

gaaagttctt caaaagg 437

<210> 178

<211> 483

<212> DNA

<213> Homo sapiens

<400> 178
tttttttttt tttttttttt tttttttttt tcaccatcag tatcattttt attgcaaatc 60

agttaacaaa aaagatgaaa aaaatacatc atctacagtt cctcatgtag atcactttta 120

aagtcttttt ctgtaaacta cactctattt tataaaacac agtaaaaatc aacatcttgg 180

gacgcatctg gtcatgcccc ctggggatgg caccacgcgg cccccgtaga gccgggggag 240

gctgccctga ggagtgcagg cggcacggca gcggagttca cccagtgcat gggcggctaa 300

ggctccccga gctgagccga gtctgacgtc cctcactggc atgacacaca acagactcat 360

tcattaagat tttttaaaca aaatccacct ttaaaaacat atttacagac atttttttctg 420

ccataggcta atacttggta ttggcaaaaa tctgctcctc ccaaactagg gaggggtggc 480

cca 483

<210> 179

<211> 268

<212> DNA

<213> Homo sapiens

<220>

<221> misc_feature

<222> (23)..(23)

<223> a,t,g,c

<220>

<221> misc_feature

<222> (40)..(40)

<223> a,t,g,c

<220>

<221> misc_feature

<222> (267)..(267)

<223> a,t,g,c

<400> 179
ctgaagtggg agcggccctt gcncaccctc ctagtttggn aggagcagat ttttgcaata 60

ccaagtatag cctatgcaga aaaaatgtct gtaaatatgt ttttaaaggt ggattttgtt 120

taaaaaatct taatgaatga gtctgttgtg tgtcatgcca gtgagggacg tcagactccg 180

gctcagctcg gggagcctta gccgcccatg cactggggac gctccgctgc cgtgccgcct 240

gcactcctca gggcagcctc ccccggnt 268

<210> 180

<211> 480

<212> DNA

<213> Homo sapiens

<220>
<221> misc_feature
<222> (273)..(273)
<223> a,t,g,c

<220>
<221> misc_feature
<222> (307)..(307)
<223> a,t,g,c

<220>
<221> misc_feature
<222> (343)..(343)
<223> a,t,g,c

<220>
<221> misc_feature
<222> (480)..(480)
<223> a,t,g,c

<400> 180
cattaaaagg aagcatggag ttctaatgct cccataaact atgtattttg gcaagacact 60
tcactactcc aggtctcact ttccccatct gtaaaacagg gtttggacta ggtgttccct 120
ggtattctgt gatctgcctc ttgctgccat tctttctctc ctctgcttct ctgtattttt 180
cttctgttat ccctgggggt gctcaggttc acttgattgt ctgtatttct gtgtggttgt 240
agcaaggact cagcctcatg taagcacgaa tanggggtgt ggttcatggc gtgttgaccc 300
agcagancac tccctcccac taactttgtt ctgcatgtgt aanagtctcc ccattttttt 360
taacgcaacc ctttcccctt tttcctaccc cacagtctgt tccatgtaag ttgccaacag 420
tttcactgaa cagtggggta tgtgatggtt ttggcatgac atcttcagta tgaaggggan 480

<210> 181

<211> 274

<212> DNA

<213> Homo sapiens

<220>

<221> misc_feature

<222> (229)..(229)

<223> a,t,g,c

<220>

<221> misc_feature

<222> (236)..(236)

<223> a,t,g,c

<220>

<221> misc_feature

<222> (252)..(252)

<223> a,t,g,c

<220>

<221> misc_feature

<222> (262)..(262)

<223> a,t,g,c

<220>

<221> misc_feature

<222> (270)..(270)

<223> a,t,g,c

<400> 181
taacaggaaa aaattattta atagtataac aaaatgcaaa ataaagtacc caagttacaa 60

aacataaatt cctttggttc atgatcacac cactattttt accttccaca tagctacaga 120

catcacaccc tcaaagtgaa gtcaaactgt ccccctcata ctgaagatgt catgccaaaa 180

ccatcacata ccccactgtt cagtgaaact gttggcaact tacatgggna cagagntgtt 240

gggggtgggg anaaaggggg angggggggn gtgg 274

<210> 182

<211> 503

<212> DNA

<213> Homo sapiens

<220>

<221> misc_feature

<222> (302)..(302)

<223> a,t,g,c

<220>

<221> misc_feature

<222> (439)..(439)

<223> a,t,g,c

<400> 182
tatgagttca atatttttat ttctttacaa tgatttcaga agagattaca aagagattaa 60

tatacttaaa gaatcagact cttgcaaaca gtgacatcat taaaaagagc ttattttcat 120

taacatgtga ttaacaggaa ggagatgatt ggtgagtttt cttcgtaacc aggttcactg 180

tggataggaa gggcctgcct tccttcccac catggagatc ctaaaatcac aagctccagc 240

ctccatcaat gatgacaggg ttaccagtta cataagcaga ttcatcagaa gccaaataca 300

cngcagagca tggctatttc ttctggcagt tgcgaatctt cccgtctttt ggtctcttca 360

ggaaatcatt cccgtgcctc ttcaggattt cctctggctt ggtattcctt tcctgtagag 420

atgggcggat caactggtnc cgggcacaca cagttggcac ctggatgccc tggctgggat 480

ggaaatctgg cagccacagg att 503

<210> 183

<211> 482

<212> DNA

<213> Homo sapiens

<220>

<221> misc_feature

<222> (64)..(64)

<223> a,t,g,c

<220>

<221> misc_feature

<222> (268)..(268)

<223> a,t,g,c

<220>

<221> misc_feature

<222> (334)..(334)

<223> a,t,g,c

<220>

<221> misc_feature

<222> (345)..(345)

<223> a,t,g,c

<220>

<221> misc_feature

<222> (358)..(358)

<223> a,t,g,c

<220>

<221> misc_feature

<222> (474)..(474)

<223> a,t,g,c

<400> 183
atttccctag atccagaaac tgactctatt gaaggaaaaa aatctgatat caatcttttt 60

aaangggggag gaatgtggga gaaagcatga aaatggctac tgggaaaact tatttgtgtt 120

acctttctga aggaaaatac atttttttatt ccttcaattg ttgaaccttt cctccacccct 180

caggagttgt gaacagatgt gtgtacagca caaccaaggc agccgtgatt ggcctcacaa 240

aatctgtggc tgcaagattt catccagnca gggcatcagg gttgcaactt gtgttgttgc 300

ccagggaaca ggttgaatac gccatctcct acanagaaag aattncaagc cagagggnaa 360

tcctgaagag ggcaccggaa tggatttccc tggaagggga ccaaaagacc ggggaagatt 420

cgccaactgc caggaaggaa attagccatg gctctggcgg ggtattttgg gctncctgag 480

tg 482

<210> 184

<211> 438

<212> DNA

<213> Homo sapiens

<220>

<221> misc_feature

<222> (438)..(438)

<223> a,t,g,c

<400> 184
ttttagtttg gtctgaataa aatccagcat aaatgtatta ttttattctg ggtataatgg 60

ctcggtgtct ttgtcccaac agctttcttc ttaagggcaa accaccctta agtgaaactt 120

gccatttgtt tttgttttct ttttaagtct ccagatcttg ctaggacact tcttccagac 180

caacagtcag atagttgtgg cacgtgatcc tatttgaaat agaggtggtt taaatcagcc 240

aagcttggtg atctggaggt ctccatttat ctttatggtg tcaattgcag ataacgtttg 300

aatgcgatgg taaaaatcaa aaagttggtg tccatccaca aacactcgga aacgtgggtg 360

ctcacaaaga atttcccacc tggaagaaac caaaataata atcccccatt tcaatggacg 420

ctactggggg aaacaaan 438

<210> 185

<211> 465

<212> DNA

<213> Homo sapiens

<400> 185
cggaaaaagc ttttacactt acattctaca caggaatcag aaattttgga ggtagagccc 60

aacaatctgt gttctaacaa gccctctgca ggtctcaatg cattctgatg tttgtgaacc 120

actgaagata ctgcctttct ttctctaaaa tgaagtattg tttgttttcc ccagtagccg 180

ttcattgaaa ttgtggatta ttattttgtt tcttccaggt ggaaattctt tgtgagcacc 240

cacgtttccg agtgtttgtg gatggacacc aactttttga tttttaccat cgcattcaaa 300

ccgttatctg caattgacac cataaagata aatggagacc tccagatcac caagcttggc 360

tgatttaaac cacctctatt tcaaatagga tcacgtgcca caactatctg gactgttggt 420

ctggaagaag tgtcctagca agatctggag acttaaaaag aaaac 465

<210> 186

<211> 468

<212> DNA

<213> Homo sapiens

<220>

<221> misc_feature

<222> (446)..(446)

<223> a,t,g,c

<220>

<221> misc_feature

<222> (458)..(458)

<223> a,t,g,c

<400> 186
gagggcatat tgataaatct ttattgacaa aatattgaca ttgacatact tcttggaagt 60
atatagtgtg ttagaattct aacaaattaa cacaaaacac aaaaatattt acattctggt 120
atagaagaca ttaaggaagc atttgtcact ctctttagta agtctatgat cttggaatag 180
aaactcagtg cttgaaaact tgccgccgtg cctttggcca cacttaacat catccccgct 240
aactacagtc cttcaggttt tgcaatagat agatttaaag tttggaatag gcattgcagt 300
gaatggttga actcggccaa tttctccaac cactgaaagg agaagtttgc atcagggttt 360
taagcctcag gatgttagga aagggaattg tccaagaaat ataattaatt taggggtttt 420
ttttcccagt accaagtcct gattcntttt tgtgggcncc cttccccc 468

<210> 187
<211> 401
<212> DNA
<213> Homo sapiens

<220>
<221> misc_feature
<222> (22)..(22)
<223> a,t,g,c

<400> 187
agctgaacgt gcactctgac anggggagct gtcaatcaaa caccaaaccg gggagacaag 60
atgattggca ggtattccgt ttatcacagt ccacttaaaa aatgatgatg atgataaaaa 120
ccacgaccca accaggcaca ggactttttt gtttttgca cttcgctgtg tttccccccc 180
atctttaaaa ataattagta ataaaaaaca aaaattccat atctagcccc atcccacacc 240
tgtttcaaat ccttgaaatg catgtagcag ttgttgggcg aatggtgttt aaagaccgaa 300
aatgaattgt aattttcttt tccttttaaa gacaggttct gtgtgctttt tattttgatt 360
ttttttccca agaaatgtgc agtctgtaaa cactttttga t 401

<210> 188
<211> 469

<212> DNA

<213> Homo sapiens

<220>

<221> misc_feature

<222> (356)..(356)

<223> a,t,g,c

<220>

<221> misc_feature

<222> (440)..(440)

<223> a,t,g,c

<220>

<221> misc_feature

<222> (467)..(467)

<223> a,t,g,c

<400> 188
tttttaaaa atgatgaata ttttattttt cagacgtcca tattttaaat gtaatagttt 60

tataaaagaa aaggttggca acctgttaag gagatcttca tgtgaaaaat acatgtagaa 120

gttttaaaaa tttgtggata taattgtcat tcagaattaa gcaggttgat tgctgttatc 180

tagatgggtc tcttccttta tgtttttcag tcacataatc ttgatttcca tagttatcac 240

atgtacttaa agaagttaat caatgcctat atggtccaag gtataatttg cacacagtag 300

tttttggttt ttatattgtg ggatcatatg tatcaaaggt aatatttcat aaagancaat 360

ggttatagtt ggctacaaag gggaccaatt ccacatttcc tatacaggga atattttaag 420

gggtagagtt atatccggcn atgggtgatt ggggtttaaa tatcccngc 469

<210> 189

<211> 364

<212> DNA

<213> Homo sapiens

<220>

<221> misc_feature

<222> (16)..(16)

<223> a,t,g,c

<220>

<221> misc_feature

<222> (258)..(258)

<223> a,t,g,c

<400> 189

```
ggctctttca atcctnggtg tgattgttgc caacatgaca gaatggaagc accagcgcat     60
caccaagcaa ttggagaaat ggatgcaaaa gaatgtggag gaaaacctct atagcagctc    120
cctgggagga ggggtggcca cctccccagt gctgattgtg tttcatggga aatattccac    180
aattaacccc ctgtggcaca taaggcacct gggctggaat ccagatgcca gatattcggg    240
agcattttct gcaggaangc taaattactc cactggaatg gaagacataa acctttggga    300
cttcccctag tgttcacaac gacttaatgg gaaagctggt ttgttcctga ccctggcagg    360
gata                                                                 364
```

<210> 190

<211> 451

<212> DNA

<213> Homo sapiens

<220>

<221> misc_feature

<222> (6)..(6)

<223> a,t,g,c

<220>

<221> misc_feature

<222> (325)..(325)

<223> a,t,g,c

<220>

<221> misc_feature

<222> (352)..(352)

<223> a,t,g,c

<220>

<221> misc_feature

<222> (363)..(363)

<223> a,t,g,c

<220>

<221> misc_feature

<222> (413)..(413)

<223> a,t,g,c

<220>

<221> misc_feature

<222> (440)..(440)

<223> a,t,g,c

<220>

<221> misc_feature

<222> (448)..(448)

<223> a,t,g,c

<400> 190

```
tttttnttct gcaggtttcc ttttaatcaa ggctctactg aaggtgtttt gtggggctaa      60
aagccataaa catgaaatgg acatgtaaca ccacctggat cccccatagc aggccagacc     120
actctggcga gcactgctgg tctgcccaaa tctgggtaat cagactgggt attcattggc     180
tgcatttcaa agcacagcac tgctttcagc caggatgaag tgggagtgaa cccagctgct     240
agcagagtgc cactccaggc tgaagagcca agtaccagcc actgccagtg aagactggcc     300
cctttaactg aaggaagttg ttcanagttc cagccaccgg gccctggggg anggaagaga     360
aantcagggt aattctgctc cggggatggg tcagggttcc gcaagtttca atnggccagg     420
catcctttgg aaaagcccgn cttctggntg a                                    451

<210>  191
<211>  506
<212>  DNA
<213>  Homo sapiens


<220>
<221>  misc_feature
<222>  (38)..(38)
<223>  a,t,g,c


<220>
<221>  misc_feature
<222>  (51)..(51)
<223>  a,t,g,c


<220>
<221>  misc_feature
<222>  (53)..(53)
<223>  a,t,g,c


<220>
<221>  misc_feature
```

<222> (54)..(54)

<223> a,t,g,c

<220>

<221> misc_feature

<222> (107)..(107)

<223> a,t,g,c

<220>

<221> misc_feature

<222> (179)..(179)

<223> a,t,g,c

<220>

<221> misc_feature

<222> (207)..(207)

<223> a,t,g,c

<220>

<221> misc_feature

<222> (220)..(220)

<223> a,t,g,c

<220>

<221> misc_feature

<222> (222)..(222)

<223> a,t,g,c

<220>

<221> misc_feature

<222> (225)..(225)

<223> a,t,g,c

<220>

<221> misc_feature

<222> (284)..(284)

<223> a,t,g,c

<220>

<221> misc_feature

<222> (285)..(285)

<223> a,t,g,c

<220>

<221> misc_feature

<222> (287)..(287)

<223> a,t,g,c

<220>

<221> misc_feature

<222> (289)..(289)

<223> a,t,g,c

<220>

<221> misc_feature

<222> (299)..(299)

<223> a,t,g,c

<220>

<221> misc_feature

<222> (332)..(332)

<223> a,t,g,c

<220>

<221> misc_feature

<222> (380)..(380)

<223> a,t,g,c

<400> 191
ccacgacccc caggaggatt gaaggagacc gggagctngc cggcgtggac ngnnggaatg 60

gaggggctgg ggctcggcgg gaggcacccc cacagccgcc ccgggangag cgcgcccagc 120

agctgctgac gggttggagc agcggcacgc tagctcctgg acaccatcgc agcctgcang 180

gagatgttac ggcagctggg ccgccgngcc cggagccgtn gngtnggcgg gaacgtctca 240

gccaaacctg gagcgccccc ccagccggct gtctccgcca gagnntntnt ccaaaggant 300

gctggcgatg gaagctgcgg acccgtgacc antccccgag cagaataccc tgacttctct 360

ccctccccag ggcccggtgn ttggactctg aacaactccc ttcagtaaag gggccagtct 420

tcactggcag tggctggtac ttggctctca gctggagtgg cagctctgct agcagctggg 480

ttcactccca ctttcatcct ggctga 506

<210> 192

<211> 350

<212> DNA

<213> Homo sapiens

<220>

<221> misc_feature

<222> (254)..(254)

<223> a,t,g,c

<220>

<221> misc_feature

<222> (263)..(263)

<223> a,t,g,c

<220>

<221> misc_feature

<222> (350)..(350)

<223> a,t,g,c

<400> 192
gttgtaatat gcattttatc gtgtcactta agtactaaga ggtgctttta aagtttttat 60

taatgctgct caaacttgac cccgacggtc caggatacag gccaaggtgt cagtgcggac 120

acaatcgcaa ggcggctgtt aggcttcagg gctgggcagg aaacttgcca ggtgctacta 180

ggaacgtatc agccctttct acaaagcagg tgtctttccc atcataaaat gcaaagcaat 240

cagccgttcc cttnctcccc agngccctcg tccggagacc gagaggatat ttaccctggg 300

ctcttgcagt tctcttataa ctgaacccag cggcggttcc ttctccgagn 350

<210> 193

<211> 508

<212> DNA

<213> Homo sapiens

<220>

<221> misc_feature

<222> (356)..(356)

<223> a,t,g,c

<220>

<221> misc_feature

<222> (454)..(454)

<223> a,t,g,c

<220>

<221> misc_feature

<222> (461)..(461)

<223> a,t,g,c

<220>

<221> misc_feature

<222> (508)..(508)

<223> a,t,g,c

<400> 193
tagtttgttt aaagacttta tttgaaacca atgtcacagc acgttagtca taaaaatcat 60

cagaatcatc ggtgttatgg tttctggggc gtaaagcagc ctcaatttct gagttactgt 120

catcagactc atcccagagg cattagattt taaattgatc acgggtttct ttttgttttc 180

atgccttgac aaaggacttc cttcactaga tgaaaaacta gcttccgaag caattttgtt 240

agctgtcttt gtctcgaatg caccgctgcc aatattcaaa cctgacaagt gtgacacaga 300

agcatctgtc ttttgaaacc tgttttcagt tttgatttgt attatcctga agggtnggcc 360

ttcttcagca actgataggc ttctgtttct gtgattggga tatctgcagc cacattttct 420

ttcttggctc ttgtctccac agccacttga aacntcttgt ncccttgtag gaacactgtt 480

gagccataca gcaggtttgg cttcctgn 508

<210> 194

<211> 456

<212> DNA

<213> Homo sapiens

<220>

<221> misc_feature

<222> (398)..(398)

<223> a,t,g,c

<220>

<221> misc_feature

<222> (437)..(437)

<223> a,t,g,c

<400> 194
agtgacctta ccatttcaat aagtgaagat gatctgattt tagagagccc agaaccacag 60

ccaaatccag gtggcaagat ggagggagaa gatggaatag aggccttaaa attaatccat 120

gctgagcaag aaagagttgc cctatccact gaaaaaaatt gtattttgca aaccctaagc 180

tctcctgatt cagaaaagga atcctccact aacgcaccaa caagagaacc tggacaaaca 240

ccagactcag acgtaccgag ggcacaggtg ggtcagcatg ttgccacctt gaaagaacat 300

gataattctg tcaaagaaga ggcaacagca ttattgagaa aagcccttac agaagagtgt 360

ggcgtagtca gctattcaca gtagtgatca tcttgcantt gcatctattc tgaatgacaa 420

tagtggaata aaggaancaa cctgctgtat ggctca 456

<210> 195

<211> 610

<212> DNA

<213> Homo sapiens

<220>

<221> misc_feature

<222> (521)..(521)

<223> a,t,g,c

<220>

<221> misc_feature

<222> (594)..(594)

<223> a,t,g,c

<220>

<221> misc_feature

<222> (602)..(602)

<223> a,t,g,c

<400> 195
tttttttttt tttcttgcgt tgcttttatt taatcacttc cccagccctt ccatcgcctt 60

ttttgaggat aagaaaccca aagttcccag agattaactt gcccaagatc acttggcctg 120

gatttgagcc tgaggtcaga ctccaaaacc cacactcaat ttgctacatc tcccaggggt 180

ggggccaagg cagctgtgag caaaaggaga agtatcagct tctcaagggc ctagggtttg 240

ttggaagggc aaggcaaggg caaaggggga tacagaacaa gggggcaagt accagtgcct 300

gggatggacc catccattca ggcagggggt gtggggtgtc ccctgtgctt agaaaccacc 360

tagcatcata gctgcaacag cactttattg ggatcttagt ctacagttca catagggagg 420

tgaagccgtg ggagaagcag gggtaaaaaa aaaaaggggg gggacttcac cccctaggga 480

cagctgcttc caaacctaac aaaaccccag ggtaagtccc ngtgctgggc ctcgagcagc 540

aactctagtc aaatcccaag gcaccggtca accatgtggg tcaaagggcc cctnttggga 600

cntcactcaa 610

<210> 196

<211> 534

<212> DNA

<213> Homo sapiens

<220>

<221> misc_feature

<222> (319)..(319)

<223> a,t,g,c

<220>

<221> misc_feature

<222> (399)..(399)

<223> a,t,g,c

<220>
<221> misc_feature
<222> (446)..(446)
<223> a,t,g,c

<220>
<221> misc_feature
<222> (451)..(451)
<223> a,t,g,c

<220>
<221> misc_feature
<222> (460)..(460)
<223> a,t,g,c

<220>
<221> misc_feature
<222> (507)..(507)
<223> a,t,g,c

<220>
<221> misc_feature
<222> (515)..(515)
<223> a,t,g,c

<400> 196
aggcaatctg attctgaagc taaagagctt tcatcctctt gagtgtatgt ccccatagtg 60

ggcccccttga cccacatgct gaccggtgcc ttgggatttg actagagttg ctggctcgag 120

gcccagcacg aggacttacc ctggggtttt gttaggtttg gaagcagctg tccctagggg 180

gtgaagtccc cccccttttt ttttttaccc ctgcttctcc cacggcttca cctccctatg 240

tgaactgtag actaagatcc caataaagtg ctgttgcagc tatgatgcta ggtggtttct 300

aagcacaggg gacacccana ccccctgcct gaatggatgg gtcaatccag gcactggtac 360

ttggcccctt gttctgaatc ccctttggcc ttgccttgnc ttccaacaaa cctaaggcct 420

tgagaagctg atactctcct tttgcnaaag ntgccttggn ccaacccctg ggagatgtag 480

caaatgaggg ggggtttgga gtttagncta aggtnaaatc aggccagtaa tttg 534

<210> 197

<211> 455

<212> DNA

<213> Homo sapiens

<220>

<221> misc_feature

<222> (131)..(131)

<223> a,t,g,c

<220>

<221> misc_feature

<222> (425)..(425)

<223> a,t,g,c

<400> 197
ctctgaagtg gtgaatttta atattgtata aaaaatccaa cttgttccac aagttacata 60

tgtcctatga ttttatgcat acatccatat acatatatca aggtaaagtc cagtacaaaa 120

aaacagcatt ncctatggcc agtgttctac agaagtaaga ctgtgcaaac tttatcgtat 180

agtcaaatga gattgcacac taaggcagga tgaggcagaa gcaagttgtg tccacagtat 240

attacaaaat accttgcata gcttattcat tctcacctgg taaattcatc ttagaattct 300

gaaggatttt tttcctagat aaattttata caagttagtg tatacttctt gtctttggtt 360

ctgtggcaaa ccaggtttct cagtactgat tgttttacct tcacaacatt attggattta 420

accantagcc cgagctttgg gggctctgca ctgcg 455

<210> 198

<211> 374

<212> DNA

<213> Homo sapiens

<220>

<221> misc_feature

<222> (141)..(141)

<223> a,t,g,c

<220>

<221> misc_feature

<222> (281)..(281)

<223> a,t,g,c

<220>

<221> misc_feature

<222> (282)..(282)

<223> a,t,g,c

<220>

<221> misc_feature

<222> (303)..(303)

<223> a,t,g,c

<220>

<221> misc_feature

<222> (308)..(308)

<223> a,t,g,c

<220>

<221> misc_feature

<222> (335)..(335)

<223> a,t,g,c

<400> 198
ctaattttgc agtgcaacac agatatctgt ttgaagaaga caatctttta cggtctacac 60

aaaagctttc ccattcaaca aaaccttcag gaagcccttt ggaagaaaaa cacgatcaat 120

gcaaatgtga aaaccttata ntgttccaga accttgcaaa cgaagaagta agaaaattaa 180

cacagcgctt agaagaaatg acacagagaa tggaagccct ggaaaatcgc ctgagataca 240

gatgaagatt agaaatcgcg acacatttgt agtcattgta nncggattac aatgaacgca 300

gtnaagancc caaagctcag gctattgtta aatcnataat gttgtgaagt aaacaatcag 360

tctgagaacc tggt 374

<210> 199

<211> 303

<212> DNA

<213> Homo sapiens

<220>

<221> misc_feature

<222> (62)..(62)

<223> a,t,g,c

<220>

<221> misc_feature

<222> (67)..(67)

<223> a,t,g,c

<220>

<221> misc_feature

<222> (165)..(165)

<223> a,t,g,c

<220>

<221> misc_feature

<222> (196)..(196)

<223> a,t,g,c

<220>

<221> misc_feature

<222> (199)..(199)

<223> a,t,g,c

<220>

<221> misc_feature

<222> (212)..(212)

<223> a,t,g,c

<400> 199
ctaaagggag gcaaagttgc actctccttc cccagaggct tcaccaagag ggcacacccc 60

cngggtntct ggtgggcaac gggggtgagc atgtccctgc cctggctccc tccatctgtg 120

accaggaggc atggctgggt gtatgttcag gtgaggctca gagtngcatt gtgtccctgt 180

cccctgccca gggcantgna ggggagccct tnatgctgat tagaaggcta gaactggggt 240

agaggtgcct ggcatgtctc atgccatggg gactcaatct agcaactgtg agtcctgggg 300

gtc 303

<210> 200

<211> 452

<212> DNA

<213> Homo sapiens

<220>

<221> misc_feature

<222> (319)..(319)

<223> a,t,g,c

<220>

<221> misc_feature

<222> (438)..(438)

<223> a,t,g,c

<400> 200

```
tgaagagaaa gaaaaatcaa atatttatta aaagtaccat aatacagacc catttcaagt      60
aaggacaaac acaccaacat atttcttagt agtttcctca caatagatta ttaaagcata     120
gaacaattat tcatattcat aaagaaatga cttcaaaata ggttaaattg ttttccatct     180
actctgttta ataaggcaag aacaaatgat tcactttaga caaatagtct catcaaaaaa     240
gggctaaaat agtaaagatt catcacctaa agtggtaagc tttggatatc tgaaatataa     300
acatgttagt actctgatng atgccagata aatgaattta ggcaagaaaa cacattgtta     360
caaaaagcct ggggttctaa atcagggatt actgagacac taacaatttc agatttttgc     420
ctcattccag aagcaccnac ccagttttct tt                                   452
```

<210> 201

<211> 257

<212> DNA

<213> Homo sapiens

<220>

<221> misc_feature

<222> (45)..(45)

<223> a,t,g,c

<220>

<221> misc_feature

<222> (159)..(159)

<223> a,t,g,c

<220>

<221> misc_feature

<222> (253)..(253)

<223> a,t,g,c

<400> 201
gtcaatttcc tataagaata ttattctgaa gacctggagg aggangtagc agcagcgctg 60

aaaaatggga atgacttcta atagtttaca atatcatgaa ggaagggctg ttgaattgta 120

gcacaaacct gaacctggtc actgactgca tggaacatng tctgacatcg gtgcatccgc 180

gaactcgata ttcagctggc tgggatgcta aatttttctt catccctcta tcttagttac 240

ctacatcact ggnagac 257

<210> 202

<211> 318

<212> DNA

<213> Homo sapiens

<220>

<221> misc_feature

<222> (161)..(161)

<223> a,t,g,c

<220>

<221> misc_feature

<222> (199)..(199)

<223> a,t,g,c

<400> 202
tctagaaatg gactgtcata aaaaaatgcc agatcccacc tgtgacctga cagcccccaa 60

ttcctagaga ggtgagagct gtcgtgtggc aggttaggag aggtgccaac cttgggcgat 120

gcggcttctt gggaaataac agctccagcg agaaagaatt nggggtgagg gagaggatgg 180

gggccagaat ggggggacnc ttaagtggta ccaaaattag ctgccgtctg ctccctgctg 240

gtgggtcctt ggggtggaaa gcatggcagg gagggggttg gggctgaatg gagaagaaag 300

tgagatgctg ataaaaaa 318

<210> 203

<211> 268

<212> DNA

<213> Homo sapiens

<400> 203
tatcagcatc tcactttctt ctccattcag ccccaacccc ctccctgcca tgctttccac 60

cccaaggacc caccagcagg gagcagacgg cagctaattt tggtaccact taagggtccc 120

cccattctgg cccccatcct ctccctcacc ccaattcttt ctcgctggag ctgttatttc 180

ccaagaagcc gcatcgccca aggttggcac ctctcctaac ctgccacacg acagctctca 240

cctctctagg aattgggggc tgtcaggt 268

<210> 204

<211> 403

<212> DNA

<213> Homo sapiens

<220>

<221> misc_feature

<222> (403)..(403)

<223> a,t,g,c

<400> 204
caaatgtata aaccttttat ttaaaattag acagactcaa atacaaatta aataagtaca 60

atataaaata gtaagccttc ataaaacaca tcataaccac tttaaatcat ttactatttc 120

tacagcaaaa ttaaagagaa atctaaatac agtccaaaat caaattattt ttaaactctt 180

attgtcaaca ttaaaattat tacaaaacat tcaaagatat ttaattagaa gatgaacaag 240

gccaggtgta gtggcccgag agttgtcgat atgctcgcag gaagtgtttc tgtgttaaaa 300

agttgagaag atggaaactg aatcctcttt gtattcagaa ggctgtttcg gaaggtcact 360

gttggcaggc ttctccctac agggattcag cagtgaggga gcn 403

<210> 205

<211> 479

<212> DNA

<213> Homo sapiens

<220>

<221> misc_feature

<222> (308)..(308)

<223> a,t,g,c

<220>

<221> misc_feature

<222> (472)..(472)

<223> a,t,g,c

<400> 205
caagacttca aaacccattt tcttgaccca aagcttatga aagaagaaca aatgtcacag 60

gcccagctct tcaccagaag ctttgatgat ggcctgggct ttgaatacgt gatgttctac 120

aatgacattg agaaaaggat ggtttgctta tttcaaggag gcccttacct ggaaggacca 180

cctggattca ttcatggagg tgccattgca accatgattg atgctactgt tggtatgtgt 240

gcaatgatgg ctgggggaat cgtcatgact gccaatctca acatcaatta taaaagacct 300

atccctcntt tgttctgttg ttatgataaa tagccaactt gataaaagtt tgaaaggaag 360

gaaatttttt ggtttcctgt aatgttccag aagtgttgga tgaagaagac cctatactcc 420

agaggcgaca agcttaattt ataaagccga atcccggcta aaaagtctga cnataaagg 479

<210> 206

<211> 450

<212> DNA

<213> Homo sapiens

<400> 206
aagtttaagt tatttattta agccatctcc tgccacaagc aaagggatgg gagaccagta 60

taccagtcat catttgatac aagtaaagaa ggtgagctta aggtcccata tactttttct 120

ggtccatgtg cagagtaacg gtccaaacca gtgctgtggt ccctggtgcc cgggtagtgg 180

tccagattct ctttattcat cttcgtagcc agttggaagt ggattcacat gagggttatg 240

gaatagagta tggttaccat ctccccaggg aaacggcttg gtcctgatgc ggagatgggg 300

gtaggcgatg aactcgggtc tctcgtgctc tccgtggtgc gacttcaggt acacattcag 360

catgctgact gccaccccgg ggagcgcgac gaagaaggtg agagtcttcc acatggcgag 420

ctgagccctc ttcgccatgg gcccaactcg 450

<210> 207

<211> 349

<212> DNA

<213> Homo sapiens

<220>

<221> misc_feature

<222> (82)..(82)

<223> a,t,g,c

<220>

<221> misc_feature

<222> (85)..(85)

<223> a,t,g,c

<220>

<221> misc_feature

<222> (272)..(272)

<223> a,t,g,c

<220>

<221> misc_feature

<222> (280)..(280)

<223> a,t,g,c

<400> 207
ggtggcagtg tcctcggttt ctcggctgct gggtcggtcc cgcccacagc tggggcggcc 60

tatgtcgagt ggcgcccatg gnaangaggg ctcagctcgc atgtggaaga ctctcacctt 120

cttcgtcgcg ctccccgggg tggcagtcag catgctgaat gtgtacctga agtcgcacca 180

cggagagcac gagagacccc gagttcatcg cctaccccca tctccgcatc aaggaccaaa 240

gccgtttccc ttggggaaga atgggtaacc antactctan ttccataacc ctcatgttga 300

atccactttc caacttggct aacgaagatt gaataaaaga gaatcttgg 349

<210> 208

<211> 341

<212> DNA

<213> Homo sapiens

<220>

<221> misc_feature

<222> (162)..(162)

<223> a,t,g,c

<220>

<221> misc_feature

<222> (339)..(339)

<223> a,t,g,c

<400> 208
tttttgaccc aaacaaagtt tcatttacag tgtatacagt caggccttgg gggcaggagc 60

tgctggggag cagagaggga ccctctatcg ctggcttcag gggtgcctt gggtcccttc 120

agtagctggt gggcgtgact ggcaggaagc acatttggct tnggcaggag ctgtcaggaa 180

ggggaagcgg gtttcctgcg attctgcctt gttctgccct gggtgaagcc gcacaccaca 240

cctcacctcc caggggccca aggcctgttg gaaatgtctg ttttaaagct ccctgggggc 300

tgggcctggc ttgacggcag cttgcaaagg ggaatgccnc a 341

<210> 209

<211> 287

<212> DNA

<213> Homo sapiens

<220>

<221> misc_feature

<222> (190)..(190)

<223> a,t,g,c

<220>

<221> misc_feature

<222> (229)..(229)

<223> a,t,g,c

<220>

<221> misc_feature

<222> (232)..(232)

<223> a,t,g,c

<220>

<221> misc_feature

<222> (273)..(273)

<223> a,t,g,c

<400> 209
ctgcccgggc ccaaggggtg cacagggcac tccatggctc cattattaac acaactctag 60

caattatgga ccataagcac ttccctccag cccacaagtc acagcctggt gccgaggctc 120

tcctcaccag ccacccaggg agtcacctcc ctcagcctcc cgcctggccc acacggaggc 180

tctggctgtn ctctttctcc acttcatttg ctttggctct ttctacacnt cnctcttggg 240

catgggctga gggctggagc gagtccctca agnaatttca ccaggct 287

<210> 210

<211> 488

<212> DNA

<213> Homo sapiens

<220>

<221> misc_feature

<222> (394)..(394)

<223> a,t,g,c

<220>

<221> misc_feature

<222> (462)..(462)

<223> a,t,g,c

<220>

<221> misc_feature

<222> (472)..(472)

<223> a,t,g,c

<220>

<221> misc_feature

<222> (474)..(474)

<223> a,t,g,c

<220>

<221> misc_feature

<222> (480)..(480)

<223> a,t,g,c

<400> 210
tggaagttaa agtatttatt gatgtgttta aactgtgtac attctccaca gatcatatta 60

aggagtttgt aggtgaagtt taatctgtgc atagtgggta gagacatgaa tagggtcaaa 120

ggggaggaaa aaggaaaaaa acaaaacaaa aacagtcaca ggaaaataaa aatacaccac 180

aggttaccag aaccttcaga tttaaaataa aaagaaagaa aaagcagaag cagtgagcat 240

cgacatcaac tgtacaagca ttacaaagac tcctgtgacg aaaacacaat tgttcaaagc 300

tttccacagt gtccacagac tcagtttcag ggacagcctg gactactttc ctttcacaca 360

aacaaacctc cccgtgttct ctcctgggcc agcnggccct cccttggtgt ggtcttctct 420

gagtgaatgt cacaaggccg gtgacaggag ggggtggagg tnaggggcaa antngagccn 480

agggtcag 488

<210> 211

<211> 478

<212> DNA

<213> Homo sapiens

<220>

<221> misc_feature

<222> (7)..(7)

<223> a,t,g,c

<220>

<221> misc_feature

<222> (14)..(14)

<223> a,t,g,c

<220>

<221> misc_feature

<222> (29)..(29)

<223> a,t,g,c

<220>

<221> misc_feature

<222> (38)..(38)

<223> a,t,g,c

<220>

<221> misc_feature

<222> (44)..(44)

<223> a,t,g,c

<220>

<221> misc_feature

<222> (48)..(48)

<223> a,t,g,c

<220>

<221> misc_feature

<222> (107)..(107)

<223> a,t,g,c

<220>

<221> misc_feature

<222> (162)..(162)

<223> a,t,g,c

<220>

<221> misc_feature

<222> (214)..(214)

<223> a,t,g,c

<220>

<221> misc_feature

<222> (217)..(217)

<223> a,t,g,c

<220>

<221> misc_feature

<222> (226)..(226)

<223> a,t,g,c

<220>

<221> misc_feature

<222> (276)..(276)

<223> a,t,g,c

<220>

<221> misc_feature

<222> (329)..(329)

<223> a,t,g,c

<220>

<221> misc_feature

<222> (343)..(343)

<223> a,t,g,c

<220>

<221> misc_feature

<222> (349)..(349)

<223> a,t,g,c

<220>

<221> misc_feature

<222> (354)..(354)

<223> a,t,g,c

<220>

<221> misc_feature

<222> (361)..(361)

<223> a,t,g,c

<220>

<221> misc_feature

<222> (363)..(363)

<223> a,t,g,c

<220>

<221> misc_feature

<222> (364)..(364)

<223> a,t,g,c

<220>

<221> misc_feature

<222> (371)..(371)

<223> a,t,g,c

<220>

<221> misc_feature

<222> (375)..(375)

<223> a,t,g,c

<220>

<221> misc_feature

<222> (376)..(376)

<223> a,t,g,c

<220>

<221> misc_feature

<222> (383)..(383)

<223> a,t,g,c

<220>

<221> misc_feature

<222> (391)..(391)

<223> a,t,g,c

<220>

<221> misc_feature

<222> (409)..(409)

<223> a,t,g,c

<220>

<221> misc_feature

<222> (419)..(419)

<223> a,t,g,c

<220>

<221> misc_feature

<222> (423)..(423)

<223> a,t,g,c

<220>

<221> misc_feature

<222> (437)..(437)

<223> a,t,g,c

<220>

<221> misc_feature

<222> (450)..(450)

<223> a,t,g,c

<220>

<221> misc_feature

<222> (473)..(473)

<223> a,t,g,c

<400> 211
tgacccntcg gccntctact ttgtccccnt cacctccncc cccntccntg tcaccggcct 60

tgtgacattc acgtcagaga agaccacacc aaggaggcgg ccgctgncca ggagagaaca 120

cggggaggtt tgtttgtgtg aaaggaaagt agtccaggct gncctgaaac gtgagtctgt 180

ggacactgtg gaaagctttg aacaattgtg tttncgncac aggagncttt gtaatgcttg 240

tacagttgat gtcgatgctc actgcttctg cttttncttt ctttttattt taaatctgaa 300

ggttctggta acctgtggtg tatttttant ttcctgtgac tgnttttgnt ttgntttttt 360

ncnnttttcg ncccnnttga ccntaatcaa ngtctctacc cactattcnc aggttaacnt 420

canctacaaa ctccttnata tgtctgtggn gaatgtacac aggtttaaca cancataa 478

<210> 212

<211> 214

<212> DNA

<213> Homo sapiens

<220>

<221> misc_feature

<222> (138)..(138)

<223> a,t,g,c

<220>

<221> misc_feature

<222> (160)..(160)

<223> a,t,g,c

<220>

<221> misc_feature

<222> (166)..(166)

<223> a,t,g,c

<220>

<221> misc_feature

<222> (213)..(213)

<223> a,t,g,c

<400> 212
ggtttaaata ctgcactcta ttttttaagg actctgtgca tggtatttcc ggtggcagat 60

gtgtaagatt ttttatgtaa atcgtgttgt tttagcagag gactatcaga cctgacagca 120

gctggacatc gaggtccncc acttgggggt ggtgacttgn cacgcntgaa tgctgaagtg 180

gaagccaagc ccccttcagt cagagctggg gcng 214

<210> 213

<211> 391

<212> DNA

<213> Homo sapiens

<220>

<221> misc_feature

<222> (8)..(8)

<223> a,t,g,c

<220>

<221> misc_feature

<222> (23)..(23)

<223> a,t,g,c

<220>

<221> misc_feature

<222> (59)..(59)

<223> a,t,g,c

<220>

<221> misc_feature

<222> (61)..(61)

<223> a,t,g,c

<220>

<221> misc_feature

<222> (153)..(153)

<223> a,t,g,c

<220>

<221> misc_feature

<222> (294)..(294)

<223> a,t,g,c

<220>

<221> misc_feature

<222> (311)..(311)

<223> a,t,g,c

<220>

<221> misc_feature

<222> (321)..(321)

<223> a,t,g,c

<220>

<221> misc_feature

<222> (322)..(322)

<223> a,t,g,c

<220>

<221> misc_feature

<222> (326)..(326)

<223> a,t,g,c

<220>

<221> misc_feature

<222> (331)..(331)

<223> a,t,g,c

<220>

<221> misc_feature

<222> (360)..(360)

<223> a,t,g,c

<220>

<221> misc_feature

<222> (378)..(378)

<223> a,t,g,c

<220>

<221> misc_feature

<222> (385)..(385)

<223> a,t,g,c

<400> 213
cgtttctncg cgggctcccg gcntgctact gctgctgctc ttcctcgggc cctggcttng 60

ncgagccacg gcgtcaagta ctcgcgggag aagaaccagc ccaagccgtc cccgaaacgc 120

gagtccggcg aagaactgga caagctctgg cgngagttcc tgcatcacaa agagaaagtt 180

cacgagtaca acgtcctgct ggagaccctg agcaggaccg aagaaatcca cgagaacgtc 240

attagcccct cggacctgag cgacatcaag ggcagcgtcc ttgcacagca gcancacgga 300

gcttgaagga naagctgcga nntcanacca ngggcctggg accgccttgc gcagggtcan 360

gccaccaggg gctacagnac ttganggctg a 391

<210> 214

<211> 398

<212> DNA

<213> Homo sapiens

<220>

<221> misc_feature

<222> (12)..(12)

<223> a,t,g,c

<220>

<221> misc_feature

<222> (175)..(175)

<223> a,t,g,c

<400> 214
gaatgacgtg cntttattgg aagctattct gacatcactt tccagactgt ctcactgtct 60

tgggaccagg catgggaggc aggggtggga atcttcttgt gattgtgggt ggtggctgga 120

ggagtggagt ggtgggaggc ggctcagtcg cgggcactcc tgcgactggc ggatncggtc 180

cggatggaat aagccttcag gagcccagga cccgcactgc tggagaagct cagggcattg 240

atgccatggt tcccccgagg gtcagcccaa tgccaccgcc actgctactg ccaccagtgg 300

aattcatcac agagatattc acggctccca ctccatctcc agccaaccgg ttctcctcgc 360

cctccagcag cttgcggtag gtggcgatct cgaatgtc 398

<210> 215

<211> 237

<212> DNA

<213> Homo sapiens

<220>

<221> misc_feature

<222> (27)..(27)

<223> a,t,g,c

<400> 215
cctacatgag caaggtggag ctggagncaa ggtggatgcc ctgaatgatg agatcaactt 60

cctcaggacc ctcaatgaga cggagttgac agagctgcag tcccagatct ccgacacatc 120

tgtggtgctg tccatggaca acagtcgctc cctggacctg gacggcatca tcgctgaggt 180

caaggcgcag tatgaggaga tggccaaatg cagccgggct gaggctgaag ctggtac 237

<210> 216

<211> 519

<212> DNA

<213> Homo sapiens

<220>

<221> misc_feature

<222> (319)..(319)

<223> a,t,g,c

<220>

<221> misc_feature

<222> (421)..(421)

<223> a,t,g,c

<400> 216
ttgtttgaga tctttgtatt ttgttaacaa acactgagat ccatcgaagc tgtgtgggtc 60

ctggcagagg tcagcaaagg ggtccttggg gcctgcatca gctcagaagc ccagacccct 120

ccctgacaat ccttgcctca tcgctcctgt ggctaagata cttcttcagc cctttaacag 180

gattactggt gtatgtgaga cagccactag cagcccctgc cctaccggcc tctcccccaa 240

cccttctcca catacatcac ccccgccacc aagcaggcag cactggggct tgattccacc 300

cctcccagag gatatggang aaagccccac atctggagag ggacagggca actgcatttc 360

tggcaaatgt ggtggcatga ggctgccagt ggtttgacag ttcaggatct ccaggtgcgt 420

ntggggcccc atagcctcct gtcctccagt gaaaagatga gaagactctg ctcctgggag 480

tcctggagat ctggtgacta ggtacccagg cttctgctg 519

<210> 217

<211> 398

<212> DNA

<213> Homo sapiens

<400> 217
ttcaggcaga gacgcggcac catggctagc aagaaagtct gcattgtagg ctccgggaac 60

tggggctcag ccatcgccaa gatcgtgggt ggaaatgcag cccagctggc acagtttgac 120

ccacgggtga ccatgtgggt atttgaggaa gacattggag gcaaaaagct gacttgagat 180

catcaacacg cagcatgaga atgtcaaata cctgccaggg cacaagttgc cccaaatgt 240

ggtggctgtc ccagatgtgg tccaggctgc agaggatgct tgacatcctg atctttgtgg 300

tgccccatca gttcatcggc aagatcttgt gaccagctca agggccatct gaaaggcaaa 360

cgccacttgg catatctctt aattaagggg ggtagaca 398

<210> 218

<211> 355

<212> DNA

<213> Homo sapiens

<220>

<221> misc_feature

<222> (48)..(48)

<223> a,t,g,c

<220>

<221> misc_feature

<222> (67)..(67)

<223> a,t,g,c

<220>

<221> misc_feature

<222> (75)..(75)

<223> a,t,g,c

<220>

<221> misc_feature

<222> (139)..(139)

<223> a,t,g,c

<400> 218
tttttttttt tttttttttt ttttttatac ttcaaaagca attttccntt attaaaagaa 60

tgcttanaaa aagtncattt cacttctgcg tcatttcttc tggtcactcc cagctcactt 120

gcagcacttg gccttcccnc tgtaacaggt gccttgaatt ttggtaaaga tcgggcaggc 180

agaatagaga cattgccctc cactgctgac gcaattgtaa tgatcagatc tgcggccaag 240

gcctgtgaga aagttaccac ctgaggccat ctcagacaaa agtaagcaga gagtaaacag 300

cagaaggtag gaagttctca tggcgactgg caggcaacac tcaggatttc aggaa 355

<210> 219

<211> 371

<212> DNA

<213> Homo sapiens

<220>

<221> misc_feature

<222> (37)..(37)

<223> a,t,g,c

<220>

<221> misc_feature

<222> (38)..(38)

<223> a,t,g,c

<220>

<221> misc_feature

<222> (39)..(39)

<223> a,t,g,c

<220>

<221> misc_feature

<222> (40)..(40)

<223> a,t,g,c

<220>

<221> misc_feature

<222> (41)..(41)

<223> a,t,g,c

<220>

<221> misc_feature

<222> (42)..(42)

<223> a,t,g,c

<220>

<221> misc_feature

<222> (46)..(46)

<223> a,t,g,c

<220>

<221> misc_feature

<222> (52)..(52)

<223> a,t,g,c

<220>

<221> misc_feature

<222> (56)..(56)

<223> a,t,g,c

<220>

<221> misc_feature

<222> (61)..(61)

<223> a,t,g,c

<220>

<221> misc_feature

<222> (63)..(63)

<223> a,t,g,c

<220>

<221> misc_feature

<222> (65)..(65)

<223> a,t,g,c

<220>

<221> misc_feature

<222> (156)..(156)

<223> a,t,g,c

<220>

<221> misc_feature

<222> (158)..(158)

<223> a,t,g,c

<220>

<221> misc_feature

<222> (186)..(186)

<223> a,t,g,c

<400> 219
gctcagctcc aaaggagcca gcctctcccc agttccnnnn nncccncgtg anagtncccc 60

ncncnccatg agaacttcct accttctgct gtttactctc tgcttacttt tgtctgagat 120

ggcctcaggt ggtaactttc tcacaggcct tgcgcnanga tctgatcatt acaattgcgt 180

caagtnggag ggcaatgtct ctattctgcc tgcccgatct ttaccaaaat tcaaggcacc 240

tgttacagag ggaaggccaa gtgctgcaag tgagctggga gtgaccagaa gaaatgacgc 300

agaaagtgaa atgaactttt tataaagcat tcttttaaat aaaaggaaaa tttgcttttg 360

aaagtataaa a 371

<210> 220

<211> 669

<212> DNA

<213> Homo sapiens

<220>

<221> misc_feature

<222> (614)..(614)

<223> a,t,g,c

<220>

<221> misc_feature

<222> (620)..(620)

<223> a,t,g,c

<220>

<221> misc_feature

<222> (661)..(661)

<223> a,t,g,c

<400> 220
tttttttttt tttttttct gcttcacatg ttttattttg gtgtacattc ttctagaacc 60

aggttcattt ttccagtttt gtagaaaaat agatgttcca gccacctttt acttaactgt 120

ctagtctttt aagaccaatc agtatgttcc ctggaaagat gaataagtct catgactaat 180

tttttaaaaa ttctttaaga caaagaaata actttctttt tttactccca aagcacagta 240

tctcaacagc agcagccaac atggggtttt agcagcttaa ctttaccccc taaataaagc 300

tttgtataaa ccagtgattt tactacaaaa aacactgtcc ttgaaagaaa ggagtggcag 360

tcagacatca atgcaaaact tggaatgatt agataataaa catggcactt acaaaaggta 420

gcttattaga atattccact taagaagagg ttacttttct gtccctcctt gccccctcga 480

aaaacaaaaa aaaaagaaaa aacatttcct taaaaattcc ccttaaatgt aggtttataa 540

aatgtcgaaa tgcctgtacc caaaacaagt gcttaaaaaa aaaaaaaaaa aaacttgtgg 600

ctaacaactc ttanaattcn gatatatatt tttataacat ctaagacggc agagttctta 660

nattccgta 669

<210> 221

<211> 597

<212> DNA

<213> Homo sapiens

<220>

<221> misc_feature

<222> (370)..(370)

<223> a,t,g,c

<220>

<221> misc_feature

<222> (375)..(375)

<223> a,t,g,c

<220>

<221> misc_feature

<222> (470)..(470)

<223> a,t,g,c

<220>

<221> misc_feature

<222> (490)..(490)

<223> a,t,g,c

<220>

<221> misc_feature

<222> (523)..(523)

<223> a,t,g,c

<220>

<221> misc_feature

<222> (545)..(545)

<223> a,t,g,c

<400> 221
gagagaattt acaatgaaat tccggacatt aatctggatg tcccacattc atactctgtg 60

ctggagcggt ttgtagaaga atgttttcag gctggaataa tttccaaaca actcagagat 120

ctttgtcctt caaggggcag aaagcgtttt gtaagcgaag gagatggagg tcgtcttaaa 180

ccagagagct actgaatata agaactcttg cagtcttaga tgttataaaa atatatatct 240

gaattgtaag agttgttagc acaagttttt tttttttttt ttttaagcac ttgttttggg 300

tacaaggcat ttctgacatt ttataaacct acatttaagg ggaattttta aaggaaatgt 360

tttttccctn ttgtnttgtt tttcgagggg gcaaggaggg acagaaaagt aacctcttct 420

taagtggaat attccaataa gctacctttt gtaagtgcca tggttattan ccaatcattc 480

ccagtttttn ccttgatgtc tgactggcca ctcccttctt tcnagggcag tggtttttgg 540

tagtnaaaat ccccgggttt tatacaaagc tttatttagg ggggtaaagt ttaaggc 597

<210> 222

<211> 203

<212> DNA

<213> Homo sapiens

<400> 222
gatattaata atcaagttca aattttaatt tttcatctgc tactcaattt taaaagagaa 60

gtcactctca gtagtgttcc caagtgagcg attgcacctt ctatgatgtg aaaaagttgc 120

taaagatcct attgccatta gccgttctta agtgctgaac gtaactgaga gaattgtttg 180

caatagacat gctctgtcac tgg 203

<210> 223

<211> 418

<212> DNA

<213> Homo sapiens

<400> 223
attattgaca acaactttaa atactggaaa ggactggacg aaatgaagct gcggaacctc 60

cgaccacctc ctgaatagtg ggagacacca cccagagccc tgaagctttg ttccttcggt 120

catttggaat tcctgagggc agccagagct ccttggtcct ttcagtacta ggcagaacag 180

cccccgatct gcatagcctg tgaaagccca cggggacatc agtaaccttc tgcagccacc 240

atccaatgcc attactgtca agtgagactt ggccactgta gcctgggcct gctgcaggag 300

ctcttcagaa aggcacatga ggaccacggt ttgcctcagt ttctggtaaa acacaaggtc 360

tggagtgccc ctgcaaaggg tattgatgga cttcctgcca gtgacagagc atgtctat 418

<210> 224

<211> 392

<212> DNA

<213> Homo sapiens

<400> 224
caagatccaa agaaaaaatt ttatttacaa tagagaattt tatttgaaac atgcatttct 60

tgttttttta aaaacaaatc agcaaatgca gatcaagttt acactcctta aggcaagagt 120

ccctatgcac gctgtacatg ttcatattaa atccaaaagc tgagttaggt tgtccaaaca 180

aactcaccag acaggttaca taagatagtg taccacaaca aatttaaatt ttgaacaaaa 240

cacatctctc ctcctttggt ctcacacaga aattaaagtc tccaaataca gacaatacta 300

tccattctcc agctcttctc cagattcacg agtcccagcc aggtcggccc atccacactc 360

cccgttgaag tctggtcctt tcttcagtag tt 392

<210> 225

<211> 467

<212> DNA

<213> Homo sapiens

<220>

<221> misc_feature

<222> (454)..(454)

<223> a,t,g,c

<400> 225
ctcttgtgtg acctgatagc tccctggaac tttgggtctg tgtgtgacac atgagactca 60

cagttggagt tctccagctc tggaggtgct gaaggagctg cattaattct ggaagacgac 120

tccatgcagc aactactgaa gaaaggacca gacttcaacg gggagtgtgg atgggccgac 180

ctggctggga ctcgtgaatc tggagaagag ctggagaatg gatagtattg tctgtatttg 240

gagactttaa tttctgtgtg agaccaaagg aggagagatg tgttttgttc aaaatttaaa 300

tttgttgtgg tacactatct tatgtaacct gtctggtgag tttgtttgga caacctaact 360

cagcttttgg atttaatatg aacatgtaca gcgtgcatag ggactcttgc cttaaggagt 420

gtaaacttga tctgcatttg ctgatttgtt tttnaaaaac aagaaat 467

<210> 226

<211> 368

<212> DNA

<213> Homo sapiens

<400> 226
gtgtgtaaag ggtttttatt ggagagcctg tgcggctgaa gtgccaccaa ggatttggaa 60

ggtcactttc agcagccgcc atttctgcca ggaccagtgg caagcacctg gcagatggag 120

cccgggtgtt tctgcgtaag gcagaggaat ccagcttttc catgagattc agctgcagtt 180

gtcgaaaacc ctgtgtgagc cagcagttcc agttcaaagg ttgagggggc gaacagctgc 240

gaggtggcca ggctcccgtg agtcaccact caggcctgag tacaccgtgg agaggagaga 300

taaagcagcc acggctgttc ttgttgccag tcccacccct ctggcaaaca gatgggggaa 360

aacagaga 368

<210> 227

<211> 426

<212> DNA

<213> Homo sapiens

<400> 227
taaagatgag attcattcct tttttgaaag aataaagact gagaattata ggtaagaggg 60

gatagaaaag gaaaatggat aagaattatt aaaagttatg caaatctcta atgtgaaaac 120

ttgggttcaa ggaaaattct gtttgagcca gtggtttaat ttaagaaaaa ttaaaatctg 180

cagagaaaag ctttcctaat catcctgata taaattactc aacagcacta agctgttcat 240

aatgtaccag gctgagaaat ctgattgcag agaaggaaaa aaaaaaaaaa agacttctaa 300

aataacgtca cttcttcagc tttaacattt ggctgtgcac ccttttttaaa aataatacca 360

gtttttttcc agcagtttac acctacagta tgcaaacaaa tatcagatgt ttgtctcagt 420

cagaaa 426

<210> 228

<211> 445

<212> DNA

<213> Homo sapiens

<400> 228
ctaggagtag ctggtaaagg cacatagctg tccctcatgt cctgagctct cagaggactt 60

ggtatggaat gctgcctatg cctgtgaact ttgtaattag aaacaggttc aagttttggg 120

gttttctttt tgccagatac tagctgtgtg attttgagca agttaacctc cctgtgcctc 180

agtttcttct gctgtaaaat gggggtgaca atggtcccta taccttagga cagtgagctg 240

tcacatgaag cccttaacac agtggctgga atgtgataag ggatattaac ttgtagatac 300

cttgtggagt gaaacattga ggtgattttg tagacaacaa gaggagaagg aaggtatttt 360

aaaatccagg cgctaccctc atgccgacta aggtgtgaga ggctcacagc tcacatgaga 420

gggtgatctt gcctgaagca catga 445

<210> 229

<211> 391

<212> DNA

<213> Homo sapiens

<400> 229
gagaatgaca agagcttccg cggtgtcatt ctgacctcgg accgcccggg tgtcttctcg 60

gccggcctgg acctgacgga tatgtgtggg aggagccccg cccactacgc tgggtactgg 120

aaggccgttc aggagctgtg gctgcggttg taccagtcca acctggtgct ggtctccgcc 180

atcaacggag cctgccccgc tggaggctgc ctggtggccc tgacctgtga ctaccgcatc 240

ctggcggaca accccaggta ctgcatagga ctcaatgaga cccagctggg catcatcgcc 300

cctttctggt tgaaagacac cctggagaac accatcgggc accgggcggc ggacgtgccc 360

tgcagctggg gctgtcttcc cgccggcgag g 391

<210> 230

<211> 459

<212> DNA

<213> Homo sapiens

<400> 230
ccttaagcct tttctgttcc cttcaggacc taggcttttg aaacccaaaa gccaggaaaa 60

catgcctttg ttatctgctt tctgcaatca cgtctcttcc atggggcact gagcagagaa 120

tggtgtggcc aagtgagtag tgagaagcag tgaggaggtg tgagctaggt gtctgttccc 180

attttagaaa atactgttcc tacatcagaa ataccacatt aagacgtata gagccaggtc 240

actgggatgc ttgaaccaaa tagctgggat tctggacaga gtcagcagag tacagaaggc 300

tctgaagtgg gagacggagc tggggtgcat ccctcccagt gaggaggggt catgaggggc 360

gtctgggaag agggacattt gaactaggat tagctgagtt gccatgatgc taagataatg 420

ggagagtgtt ctttgtggtc accagtgtcc acatggcat 459

<210> 231

<211> 413

<212> DNA

<213> Homo sapiens

<400> 231

cttttaatat gacattcttt tctaatattg taatcagaaa atacagtata gccattttca 60

tttgacacaa aaaatattca gggtgtaaaa ataacaaaag actcattctt ttgtttacat 120

tattttgtgc cacattttaa aatgtcttct gttactgcaa actattttac agctttcaaa 180

attgactagt aattcttgac attttaaata caaaattgtc ctaaacaaaa ccagcacgta 240

ttcaatatga tctataagtt aatctttgga aagacatttc gttttctaat attgacaatt 300

ttacacaggt gatttgtaac ttttgaagtt ctggctgaac tcagcatctt ttttcaggtg 360

tctaagacct tcttcagaga agactcctag aggatatatt taagacccaa ctc 413

<210> 232

<211> 514

<212> DNA

<213> Homo sapiens

<400> 232
gaaaaaactt ctttttcttg gcaattgctt gggatcctgt acattgccct tccacttgtg 60

agggaaaaaa ataatgacac ggacatcagg tgtgttgcag tgattcacag tcatccacag 120

accctcacca ccatctccac tggaaaatat tgaacactcg ccactgctag ggtagtatgt 180

agtacatgaa tggcagacac cggcgagatc tcaagtgtta cgacggatga aaattccacc 240

tgttaagtct cctttccatc caagctctag cagttggttg ttccatgggt gtggtgaggc 300

tgatctgatt acatgcttat gtaaaatcca aataaagccc cttgaatgga aaacaggtta 360

atttgggaag tcactagtaa agcaggttaa tttggtaagt cactagtaaa gcactaggct 420

catcaggagt tgggtcttaa atatatcctc taggagtctt ctctgaagaa ggtcttagac 480

acctgaaaaa agatgctgag ttcagccaga actt 514

<210> 233

<211> 327

<212> DNA

<213> Homo sapiens

<400> 233
cttgtatgaa tcatgacaac gtctaaatct ttactattct tctggcaaaa gcatcagtaa 60

gaaagaaggc gaaaaagaga agtatagcct ttatgtcaga aaaacattct ttttagctgc 120

ttactttctc atgaaaagta aagatgttta cagtgtatgc caagttttca gtttctgtat 180

aacaacaggt agaggttcta atcatattga aaattgtgtt ataatggtct gagccatgtt 240

gctaggaaac aataggttcc aattttgtat tcctgctctc ctgtgctgaa aagtgactgg 300

atactgtaca ggttcatgtt ctctggc 327

<210> 234

<211> 345

<212> DNA

<213> Homo sapiens

<400> 234
aacagtaatt cttcagactt tattaaaaaa tgacataaag tgcatcttat taaaaaatgt 60

ataaaaacca cataaattca gggcccctgt gctgggcagt gttgatatcc cttagagtgg 120

aggaaggtga gggatggagg gtgaactggg gactggggag aggaccaggg tgcagttagt 180

tcctcgtgtt tgagttcaaa gatggagcga gggtggatat ggtgggaagg ggcacacggg 240

ttctcacgca acaacggagg aaggcaggcg acagtctctt ccctgaattc tgagggaaag 300

gcgtacattg tcacgaaatc tctcctgagc tcgcgctgtc ctctc 345

<210> 235

<211> 340

<212> DNA

<213> Homo sapiens

<400> 235
gagggaagag atagcatggc atgcagcaca cacggctgct ccagttcatg gcctcccagg 60

ggtgctgggg atgcatccaa agtggttgtc tgagacagag ttggaaaccc tcaccaactg 120

gcctcttcac cttccacatt atcccgctgc caccggctgc cctgtctcac tgcagattca 180

ggaccagctt gggctgcgtg cgttctgcct tgccagtcag ccgaggatgt agttgttgct 240

gccgtcgtcc caccacctca gggaccagag ggctaggttg gcactgcggc cctcaccagg 300

tcctgggctc ggacccaact cctggacctt tccagcctgt 340

<210> 236

<211> 329

<212> DNA

<213> Homo sapiens

<400> 236
aaatatttaa ttcccattta tttaaagttt cgtcagtctg tgtacactat ttatattaaa 60

aacacaggaa gctggggctc ctagcaaaaa tatacatttc aattttggag attgttcaga 120

cactgagaag agctgtatcc tcagcaccag acccgggtgg gggcagggac gcggcatgtg 180

gcgcgggagg gggaggtggg tcccagcagc tgtcccttca tagccttggc ctgaaaggaa 240

gcaccccaac ccccatcagc tgggtaggtg gggagcggga agaatcctgc cagcagagag 300

tgatcctggg gcatggaagg gaggctgca 329

<210> 237

<211> 292

<212> DNA

<213> Homo sapiens

<400> 237
ttacttggta acaccttttt aataggtcaa agtaactgta cagttcatta tattcttcct 60

gagaataatt tatatccccc aacaaactaa agggagggta tatttttca aaattaacag 120

aatggatggt gtaactggac atcaaaagaa aaccaaattc ctgttttgct tcattctctc 180

ctgggaagac agatacaagg aagggttagt taactccagt ggctgtaaaa tagtcaacat 240

ggctttaatc tttctttata aattatataa aaatggaaaa aggggatggt tc 292

<210> 238

<211> 532

<212> DNA

<213> Homo sapiens

<400> 238
atctctctaa aaaagaaaaa aaaaatcact gaaaacagtt ctcctgacct atctcttgtc 60

tttcagattg agatggcaca gaagctattg aactctgacc tgggtgagct catcaacaag 120

atgaaactgg cccagcagta tgtcatgacc agcctccagc aagagtacaa aaagcaaatg 180

ctgactgctg ctcacgccct ggctgtggat gccaaaaact tactcgatgt cattgaccaa 240

gcaagactga aaatgcttgg gcagacgaga ccacactgag cctcccctag gagcacgtct 300

tgctaccctc ttttgaagat gttctctagc cttccaccag cagcgaggaa ttaaccctgt 360

gtcctcagtc gccagcactt acagctccaa ctttttgaa tgaccatctg gttgaaaaat 420

ctttctccat atagttttac cacaccttga attggggttc aatatttggt gtggtttttt 480

caatcatgat gttcggaaaa atccgggttc aaaagtggcg gtttctagaa tg 532

<210> 239

<211> 516

<212> DNA

<213> Homo sapiens

<400> 239
tagaaatatt acactttatt gaattctgga gcagctaatc taccctcccc tattgaccaa 60

tgacaagaat ggaagaaaat atacaatggt taaagaaaga gtctataacc actgtttgtt 120

taaaatgttg aaacaacttt cactgtactg gtgaaacagt tttaataccc taacatacac 180

agtaatgatt cattcttgtt taaaacaaga agtgatttcg acttgggatt gggattttta 240

ttttttgaga tggagttttg ttcttgttgc ccaggctgga gtgcaatggc acaatcctgg 300

ctcactgcaa cctccgcctc ctgggttcaa gcaattctcc tgcctcagcc tcctgagtag 360

atgatgggat tacaggcatg caccaccacg cccaggtaat tttgtatttt tagtagagat 420

ggggtttctc catgttggta aggctggtct cgaactcccg acctcaggtg atccgcctgt 480

ctcggcctcc caaagtgctg ggattacagg cgcgac 516

<210> 240

<211> 572

<212> DNA

<213> Homo sapiens

<400> 240
caagttttct tttataagcc tttaattcat cctcagtgac tctggcaagg ctgcttctct 60

atcactggct ttgcacagaa gtatgctcta cttgcgttgc tttagggcag gattctattt 120

tgagggaaaa gacagtatcc ttattacctt ttgtttgttt aatagcacaa atgcttattt 180

gttatccaaa aacaacctcc ttcttatctg tgataaatct atagaaagaa tttagctgca 240

agtggacaaa ggaacaagcc cccagaaaag aaagggaaga actgccttct tatactacag 300

aacatgcatt agtgtgggct atatagctgt ggctcatgct acccaattcc agatttcttt 360

gtcctctaag agttgattgc tgtatattaa aattgaacat cagaggatgg gaagagggct 420

ctgtaagcca gaaccttact aaagtagagg gcacaatcag tgtgaataaa ttcacttcag 480

aatctcaagt caaggccagg cacggcagct cgcgcctgta atcccagcac tttgggaggc 540

cgagacaggc ggatcacctg aggtcgggag tt 572

<210> 241

<211> 364

<212> DNA

<213> Homo sapiens

<400> 241
ttttctgaga gtcatttatt tcttttgtca ttgccaaaca ttccccaggt cttactcacc 60

agaaattaac atagcttctt catttctcct ccctgacaac cctcatctac tgaaagggct 120

ttaagggcac actgccaatg aaaatgcaga gtgcatatac catgtcctta acttgtttca 180

aaactaccta ttctggcagt atccaattca ggtttcagtg ctcccttgtt tgaaagtggt 240

cttcacaaaa gcccacattt taaagctatt tggaggagca caaagagtta aagtggtaat 300

agcctttcag aatttgaaaa ggtagtactt gtctatatca ggttcatttt ttatgtggca 360

tatg 364

<210> 242

<211> 579

<212> DNA

<213> Homo sapiens

<220>

<221> misc_feature

<222> (10)..(10)

<223> a,t,g,c

<400> 242
attcggcacn gagggtttgt cctctcaata tggttttaag attgaaagta agaaaacgga 60

tttaggatga aaactctaga actaccctat gctgtttata ctgggaaatg ctttgtacca 120

agtagcagtg actagaccca cagacatgaa aagcaacctt aggagtaaag tgacccaaac 180

attaaaatga caggaaagag aaagtagaag cagcaataaa tactgcccca actttcctgg 240

agcccgaggc ctcatccata gctatgatca cttgccctct gaagcttatt tttgcttctt 300

tggttttaag aattgagaaa tatcacattg cccctgatgt tttgacagtc tcctagtgct 360

cctggtagtg ccactaaggg aaaaccaagg tgcgcattcc ttctccctgg actttacctt 420

acttgttagt ctacgcccca ctgtttccac ccatcccctt agccaacctc tgtctttttg 480

aattttctga gaatattgtc ctatcctctt ttatatatgg agttctctcc tctttatatc 540

ctgagacttt gacaccagat gtagatattt atctggagc 579

<210> 243

<211> 382

<212> DNA

<213> Homo sapiens

<400> 243
tttaaattag tagagacagg gaatcttact atgtgaccca gactggtctt caattcctgg 60

gctcaagcga tcctctcgcc tcagcctccc aaggtggggt tatatgcgtg acgcgctgtg 120

cccggctcca aagaacattt cttaagattg gtggtgcaag gatcacacct tgagaaacac 180

tgatttaggc cttcccacag tacaaagaaa tgttgcctgc cccatcctta cagcacacct 240

gatgacttac aagaggtgct gctgaattcc tcccagggaa gcaaccttaa ttcttctcag 300

caagacaagg aggcagcctt caggaaggac ccaggagctt ggtattagag gatgatccaa 360

gtctgatggc aaatttagag tg 382

<210> 244

<211> 210

<212> DNA

<213> Homo sapiens

<400> 244
gggtgagcac ttagccagta aaaatctgtg ctttgtataa aaagaacttt ggactgaggt 60

tatatgagtc ttcctgcagg ctggagacct ctgttacctt tacttctcta cttttgttgt 120

cttgagacca gctttttat ttttggtgag gagaacagca ggcagttcaa atgtcaggga 180

aagacaagtt cagcttgctt tttccttatt 210

<210> 245

<211> 393

<212> DNA

<213> Homo sapiens

<400> 245
tttgacattg tcataatttt ttattatgta tcaaattgtc ttcaatataa gttacaactt 60

gattaaagtt gatagacatt tgtatctatt taaagacaaa aaaattcttt tatgtacaat 120

atcttgtcta gagtctagca aatatagtac ctttcattgc aggatttctg cttaatataa 180

caagcaaaaa caaacacctg aaaaaatata aaccaaagca aaccaaaccc cccgttcaac 240

tacaaatgtc aatatggaat gaagcattaa aagccaacct taaagtaact tcagctttaa 300

tctagcaatg cagaatgatt actaaaatta gtggcaaaaa accaaccacc aaccaccaac 360

caaaccaaac caaccaacca aaaaatccca cca 393

<210> 246

<211> 315

<212> DNA

<213> Homo sapiens

<400> 246
tttaagaggt actcaaatat ttatttaata catgaataaa tggtgagtca gtgaaaaaaa 60

atctgaagtt gtacaaaagt ggaagctcag tgttccaaat tatttaatag cctgtcatta 120

tcagatgatc cgagcaattt atttttgcat tgttacaggc tgtgttctcc agaagtagac 180

agtagaccct cagatgtagt ctggtgttca agatgtttgt taaagatcag tatctgtaaa 240

aggaaaagag ttagaagcag tatcaactag aggaaaaagc taacatgcag tgcaagccca 300

atgaagcctc tgtag 315

<210> 247

<211> 429

<212> DNA

<213> Homo sapiens

<400> 247
cggttattgg atatgtgaaa tatcatacta tagcagatat ggaggtttgc cactcagatc 60

acttcaagaa aggacttccc gcccagcagt ggggagtgca gtcaccaggc agcctcctac 120

tctcagcttc ttcatagtct tagctgcaga aagccacctc actcaagttc acattcttcc 180

cagagcagcc tgcatcagat gactaagcag agtgtcaggg gataggagat agtagggata 240

tggaaactcg gtcatttcaa cccaatgtaa gaccagtcta acaggcaatg cttattctaa 300

agctacctac agaggcttca ttgggcttgc actgcatgtt agctttttcc tctagttgat 360

actgcttcta actcttttcc ttttacagat actgatcttt aacaaacatc ttgaacacca 420

gactacatc 429

<210> 248

<211> 184

<212> DNA

<213> Homo sapiens

<220>

<221> misc_feature

<222> (133)..(133)

<223> a,t,g,c

<220>

<221> misc_feature

<222> (142)..(142)

<223> a,t,g,c

<220>

<221> misc_feature

<222> (152)..(152)

<223> a,t,g,c

<220>

<221> misc_feature

<222> (156)..(156)

<223> a,t,g,c

<220>

<221> misc_feature

<222> (163)..(163)

<223> a,t,g,c

<220>

<221> misc_feature

<222> (176)..(176)

<223> a,t,g,c

<400> 248
aatgcattct agtaaaagtt catttgacag tctttttttt tttttttgaa attcacagtc 60

cattaaattc ttcccctctc ttcttttagc aaacttgtaa catcctttta tatcctttct 120

taacagaagg aanttaagca ancaaaccag tntttngcct ttncttcccc ggttanactc 180

cccc 184

<210> 249

<211> 300

<212> DNA

<213> Homo sapiens

<220>

<221> misc_feature

<222> (185)..(185)

<223> a,t,g,c

<220>

<221> misc_feature

<222> (187)..(187)

<223> a,t,g,c

<400> 249
cgaactggcc gcgagattgc tcttcagcgc cgtcgagtgg gcccggaaca tccccttctt 60

ccccgacctg cagatcacgg accaggtggc cctgcttcgc ctcacctgga gcgagctgtt 120

tgtgttgaat gcggcgagtg ctccatgccc ctccacgtcg ccccgctcct gggccgccgc 180

cgctngncat tgcttcgccc atgtccgccg accgggtggt gcctttatgg gaccacatac 240

ggatttttcc aagagcaagt gggagaagct tcaaggcgct tgcacgtttg gatttcagcc 300

<210> 250

<211> 338

<212> DNA

<213> Homo sapiens

<400> 250
ttttagagtt gaaatatatt ctttattttc aggatggaaa taggataggg aaggaggaaa 60

gatacctttg ttagttgcca ctgcagtacc atcgaaagaa catccacgcc aacactgtcg 120

cactcgctga tgcgagggcc ccgaggactg caagcaggaa cagattgctg ggcgtcagag 180

cctcgggcct ggccagcagc tcgcccaggc tgggcctcca gggctcgatc agctcggcat 240

cccgcggcgc ccagccgagc acttcccgga acgaggcctg caactctgtc tccgcccgcg 300

gggcgcccag tcacgttaag aaagaagtag agccgggc 338

<210> 251

<211> 349

<212> DNA

<213> Homo sapiens

<400> 251
ctggcgcgga tcggccggct cactcacgca ccgcgggacg ttctcctgcg tgatcaagca 60

agaccagcgc cccctggccc ggctctactt ctttcttaac gtgacgggcc cgcccccgcg 120

gggaggacag agttgcaggc ctcgttccgg gaagtgctgc gctggcgcgc gggatgccga 180

gctgatcgag ccctggaggc ccagcctggg cgagctgctg gccaggcccg aggctctgac 240

gcccagcaat ctgttcctgc ttgcagtcct cggggccctc gcatcagcag tgcgacagtg 300

ttggcgtgga tgttctttcg atggtactgc agtggcaact aaacaaagg 349

<210> 252

<211> 493

<212> DNA

<213> Homo sapiens

<400> 252
tttttttttt tttttcccgt ggcactcaat cttttatttt cttaaactgt acacaaacgt 60

acaatacttt aacagcaagt ctatgggaaa attgtttggt tttaaattat tatgaaacag 120

aatctatggg gagctttatt aaataaacat aaagatgagg atttaaggat acacacctgc 180

attctatcat agtcattttt actctacctt ctgggtgtgt aaggatggaa aagacacatc 240

aaaccgaata aacaaaatcc attcataccc tgaaacgttg gcaggccact caagggactg 300

ctcagaacgt ccacctcatc tcagatggcc tcaccgtcta ataaaattaa aactgatctg 360

ttggcctctt tggttccaaa attatgtata atacatttaa ctgtattctc tttttttttt 420

ttttgctgct ataaaataac tttttttcaa tggcagttct gactaatctg cacttaatca 480

gtgcaacata aaa 493

<210> 253

<211> 227

<212> DNA

<213> Homo sapiens

<400> 253
ctcgctcgcc ctctagatgt ccaagtgcca cgtgaactat gcaatttaaa gggttgaccc 60

acactagacg aaactggact cgtacgactc tttttatatt ttttatactt gaaatgaaat 120

cctttgcttc ttttttaagc gaatgattgc ttttaatgtt tgcactgatt tagttgcatg 180

attagtcaga aactgccatt tgaaaaaaag ttatttttat agcagca 227

<210> 254

<211> 419

<212> DNA

<213> Homo sapiens

<400> 254
cccatgtcca gttctgctct gacttgtggg tccaccttag aaaagtcagg agacacctgg 60

gaaatgaagg cactagactc ttccagactc gttccatggc cacccagagg ccttgggtca 120

tccacccaac atcccaacaa accccactgt gcactggcat catgccaggg tccaggtgtc 180

ctgccaggag cagcctctgc cctcccagag ctgacatttc agggggatgt gtgccaaagt 240

gagacctgtc agagatattt acaagcagcc atctctcttg acatcgctgt atcccaaata 300

aatcttctgg gaagaccctc ttcacctcca gctctcctga tacagcaagg cagttgtgag 360

caagttattc ataactctac acctcaattt cttggtatgg aagatgggga taatgagag 419

<210> 255

<211> 331

<212> DNA

<213> Homo sapiens

<400> 255
tttttttttt cagactttgt agctgttttt attattaata ttatcttctc ctttaagcat 60

ccctttaagg gtgaaatgaa atccaaccat ttacagagaa aatgatttca gaatgtacat 120

attgattttc ccttacaaaa aaaataatac tattattgat gtccttacat tatgcatttc 180

ccagggtcct gccttctgct tggacaagtc ccgtctcccc acccagactc tccctgttgc 240

ccccaaagat ctcccatgct gcccctggga accgacgtga cccatgggtg gataaggcca 300

gccatgcttc ccggatgctt ggcatggaca g 331

<210> 256

<211> 388

<212> DNA

<213> Homo sapiens

<400> 256

cactgctgcc atgtcccatg tccacctttc tccccgggaa taactggccc tgagacccct 60

agacccaagg aggcctgtcc atgccaagca tccgggaagc atggctggcc ttatccaccc 120

atgggtcacg tcggttccca ggggcagcat gggagatctt tgggggcaac agggagagtc 180

tgggtgggga gacgggactt gtccaagcag aaggcaggac cctgggaaat gcataatgta 240

aggacatcaa taatagtatt atttttttg taagggaaaa tcaatatgta cattctgaaa 300

tcattttctc tgtaaatggt tggatttcat ttcaccctta aagggatgct taaaggagaa 360

gataatatta ataataaaaa cagctaca 388

<210> 257

<211> 385

<212> DNA

<213> Homo sapiens

<400> 257
tttttcaaag aaactccaca tttattgtct catctagtca ttatcataac acagcaaggt 60

gggtggggat ggttacattt ttctcattct acagctctgg atttgtggga aataaatgca 120

ccatcatcct actcaccttg gaaaaatgaa ggctgtgggg atgcataaca atgataatag 180

ctaacgctca tgaatgctta ccacgtgcta agcacttttc taagcattat ctatgtatta 240

atccaattaa tccccacggt cactgcatca ggtagcacag cagttatttg tgctgttcac 300

caaatacttc caggcacatg gcagaattgc acttccctgc ccccttggag tgaggtgcgg 360

ccatgtggct tgctttgact gatgc 385

<210> 258

<211> 420

<212> DNA

<213> Homo sapiens

<400> 258
ttactgggga cactgggcta catgaagttt gggtcagaca cccaggccag catcaccctc 60

aacttgccca attgctggta tgtcctgccc acctcaggtg agatagggag agacactgga 120

actgttctgg ttgtcatagc agagagcaca gcaaagctga gccatgaagc tggtaatcca 180

tcactggaag tgacatatgt ctctcctgct cacactgcat cagtcaaagc aagccacatg 240

gccgcacctc actccaaggg ggcagggaag tgcaattctg ccatgtgcct ggaagtattt 300

ggtgaacagc acaaataact gctgtgctac ctgatgcagt gaccgtgggg attaattgga 360

ttaatacata gataatgctt agaaaagtgc ttagcacgtg gtaagcattc atgagcgtta 420

<210> 259

<211> 278

<212> DNA

<213> Homo sapiens

<400> 259
tttttcaga ggcaaataaa aaaacacttg gatttattca tgaggttaga gtcatctggt 60

cagttctact gttctcagct gatcttggtg gggcttactc aagagtggtc aacagtattt 120

gggccaggca gctctgcaga tcagaggagg gtgcttacat gtctggaggc ttgactggga 180

tgaccaggct gattcagcgc tgttccactt gccgccttct tcagcagtct gtcttaggca 240

tgttctcatg gggaaggctt ttctccattc tgaagagg 278

<210> 260

<211> 340

<212> DNA

<213> Homo sapiens

<400> 260
caggtccctg gactttggga ctcattctgc tcgacttgat ccccgctctg cccattgatc 60

acatacacac gtttacacct ggcagaagag gaagaggagg catgaaggaa tggcctcttc 120

agaatggaga aaagccttcc ccatgagaac atgcctaaga cagactgctg aagaaggcgg 180

caagtggaac agcgctgaat cagcctggtc atcccagtca agcctccaga catgtaagca 240

ccctcctctg atctgcagag ctgcctggcc caaatactgt tgaccactct tgagtaagcc 300

ccaccaagat cagctgagaa cagtagaact gaccagatga 340

<210> 261

<211> 446

<212> DNA

<213> Homo sapiens

<400> 261
tttttcgag tactgtaccg tatttattga ttctgtaagt ttacaagatg aacagtcggt 60

ctaagatggc agcaactgca gctgcagacc tcaatcaatg gtccatatca agcaattcaa 120

ctttttctta tatatcacgt catgaatttt ccctgcttct tggggaaact tccagcatga 180

ctagtggcgc tgcctttgta taagtcccat gtcacttggt attgcacagc tgtgtgtgat 240

cccaggcaca cccacttcac ttcacccaag catgcccatc tagtaaggaa gagatctgca 300

tttgaatcca ggtctgtctt tctccaaagc tgtctttact ctttctctag gtaggaaatt 360

agtgagctgg gcttcgattt tccacagtag ctgagagggc tttatgaact gactcccctc 420

ctctggggct tcgtgttctc atcagc 446

<210> 262

<211> 477

<212> DNA

<213> Homo sapiens

<400> 262
ctgtgtagtc aagagtaacc accagatggg aagagaatac agatcagctt gtgcaataaa 60

gaaaggaaaa ctccaaagga gattatttcc atagactcat caacttctaa gcaaggaatc 120

tcaaaaagac aatctaccag agaagtgact tggaccaaag agtaaacgaa caaaattaat 180

gatgtggaca gccctaggaa tcagcttaga ggttcagcct ggcccctggt cggcacattc 240

tacgagctcg tggaaaaggg caagcacaga ctatttacag ccgcagagaa atggcctcat 300

aaaaatgcat aaggaatgat ggaggatatt tgccatctca tttttgaagc ctgattcacc 360

gtggcccttg gggctttggt ccttcctaat gacatctggt ctactgccct ttgtgctgat 420

gagaacacga agccccagag gaggggagtc agttcataag ccctctcagc tactgtg 477

<210> 263

<211> 361

<212> DNA

<213> Homo sapiens

<400> 263
aaataagaca tgccataagt cgtgaagtta acaaaatata agcatccgca cagaatatat 60

tctaaggtga cttcatttac accgcttctc agagaaacac acaagtaacc ttttgtctgc 120

ctatcagcca gtgttgaaac agctttggaa ttcacatgga aggctgccgg gctggttccc 180

caacactagc ctgatggagt cctgtatccg caccgtgccg tcaaactggc tggtttccac 240

tagaaaagca atggagagtc agctctccct tctttaccca gcgttcaact ccacactgca 300

aaaactgcat aacctgaacc ctgctgtgat ggcaactgaa gtttaaaccc aagtttggtg 360

a 361

<210> 264

<211> 511

<212> DNA

<213> Homo sapiens

<220>

<221> misc_feature

<222> (152)..(152)

<223> a,t,g,c

<400> 264
gggccgagct gtccgacgtg tcactgcagg gacccgcccg gggtgggtct cgggctctcg 60

ctaccggaga gggaggagaa gggggaggtt aaaggggaag gaccccggaa gtgccccctc 120

ctcagtgcgg gagagggaga cgccgggggc angtccatgc ctcccgcggc gtggttggtg 180

cgtcccaggt gacgtcagaa gcagcccgcc cctgcctgga tggtgcgccc tgagtgacgt 240

caggagcaga ggccggagct gtccatcagc accaaaggcc gcgggcgggc tcagggcatg 300

gggccgcggt tctggggcgg ccgagccccg gctcctgcgc cttccccttc ctcaggccca 360

gcccgagttc ccggacgccg cgggactgga gtgccagccg gtgttggacg tggagcggcg 420

ccgccaccgc gccgacacca ttctctccgg cccagcagcc cccttcctcg cacgacggac 480

tttccctgga ccccagtcag ttggagcctc t 511

<210> 265

<211> 519

<212> DNA

<213> Homo sapiens

<400> 265
ttttttttt tttttttca gtttggcaca tttataaaag atgtaaaaca gagtggggtg 60

ggggagacag aagcatgagg tgtctaggtt ggcatgacag acaccagaca tgcaccgtgc 120

atttaaaccc ctccccaact cccttcccaa cccagcccag agcagcaccc ccccaccccc 180

atctcctgcc actgccctca ggctggtggc tttacagagg ccaggggacc agggtgaggg 240

atgctacagt ttgccccata gacaaggaca agaaccctta gatgagtctg gctgcgaggc 300

agggcgatgg gaaggaaggt agcccctccg cggagacatg caggaggtgg cccatgggac 360

cttggcaggg agtgaagagg tggggctgag cctggagaaa ccctaccagt cagaaatcta 420

aggcaagggt cccagcgtcc cctccaagcc tgcccacccc cacacacata cacacagaca 480

cacgggcaac acactcacaa ccctgagcac accccaatg 519

<210> 266

<211> 218

<212> DNA

<213> Homo sapiens

<400> 266
ttaaaggttg agacacgtct aaccagttta atgacttcga aaccgtgcaa atgccaaact 60

atggagcact agggatacaa gaggcaccaa ggcctggggg gtgggggtgg gggacactac 120

aacattgtca tggggaaaac gggatcacct aatattggta ggggaaaagg gcggtccact 180

ggcagctcag aactatgaca tattcctcag gggagcct 218

<210> 267

<211> 237

<212> DNA

<213> Homo sapiens

<400> 267
agctggcttg ctcctacttt caggaaggga tgcaggctcc cctgaggaat atgtcatagt 60

tctgagctgc cagtggaccg cccttttccc ctaccaatat taggtgatcc cgttttcccc 120

atgacaatgt tgtagtgtcc cccaccccca ccccccaggc cttggtgcct cttgtatccc 180

tagtgctcca tagtttggca tttgcacggt ttcgaagtca ttaaactggt tagacgt 237

<210> 268

<211> 551

<212> DNA

<213> Homo sapiens

<400> 268
gcggccgcgg ctgctcgggg ccgcgctggt tgcccattga cagcggcgtc tgcagctcgc 60

ttcaagatgg ccgcttggct cgcattcatt ttctgctgaa cgacttttaa ctttcattgt 120

cttttccgcc cgcttcgatc gcctcgcgcc ggctgctctt tccgggattt tttatcaagc 180

agaaatgcat cgaacaacga gaatcaagat cactgagcta aatccccacc tgatgtgtgt 240

gctttgtgga gggtacttca ttgatgccac aaccataata gaatgtctac attccttctg 300

taaaacgtgt attgttcgtt acctggagac cagcaagtat tgtcctattt gtgatgtcca 360

agttcacaag accagaccac tactgaatat aaggtcagat aaaactctcc aagatattgt 420

atacaaatta gttccagggc ttttcaaaaa tgaaatgaag agaagaaggg atttttatgc 480

agctcatcct tctgctgatg ctgccatggc tctaatggag atagagagag gttgcagatg 540

aagataagag a 551

<210> 269

<211> 518

<212> DNA

<213> Homo sapiens

<400> 269
gttaccaatc tgaagtggga gcggccgggg tttttttttt tttttttcc ttaacagtct 60

caggtatcaa ccagaagaag ttgctgatga cccatttact gatgattttc gaggtctatt 120

ggcaaaagaa gattggtggt taccgctggg gctgttgctg gttccattca tagtactgga 180

aatgtgagga aactgtggat gaggagactg cactggagta ctggggctag gcaaacaaga 240

agaggtggag ggaatacctc ctgctgggct gttggccttg tcactcccag agtcactttc 300

cagttctcca gcatttgtca gtccatctct ctggtgactg atcttcattc ttttacaagt 360

aggtcgaact ctgtatttca atggaagtgg accattcctt ctccaggtat aaatgtaggc 420

aatatcaatt agtgtataat aatcctttaa aggtcccccc atacatgaca caatctggaa 480

agtttaagga tgtcatttac ttccggaaac tttctaag 518

<210> 270

<211> 379

<212> DNA

<213> Homo sapiens

<400> 270
gggaacgtga attttaatga gggggcagac cgaggaggtg gtggctgccc ggagatcagg 60

gccaggctgt gctagatggc gcctggaagg ggggtcaccc aagtctccct gctgtcattt 120

caggaggccg acccaagtct ccctgctgtc atttcaggag gccgaatttt ttcccaatcc 180

cagagaaggt gtcagaggcc tggttagcag tcttgtcgat ggtttcctgg gtggtcttgg 240

ccagctggtc catggctttc tgccccgcct ctgtggcctg gtccaccact tgctgagctg 300

ccgctccggc cgctgacacg gcttcctggg cggtcccctc cacctgttgc ttcaggtcct 360

gcaagcactt gcttgccat 379

<210> 271

<211> 465

<212> DNA

<213> Homo sapiens

<400> 271
tttaaataaa cattttcaag gtttgtccaa aagaaggcca tataggttct tggctagcgg 60

aagacaattc agaacagctg ttgcacactt ggactgtcac cttctccagg ctggcagttg 120

atatcttatt ttttttccaa ctcattttta ttaaaaaaat aaaaaaatgc tccaactatc 180

agctttacaa aatctctaag ggaaacacaa gagcaaggtg ctgaggtaaa aacacctgag 240

gtagcttctt ctgtgtgttt ttctcgttaa aaaaatctgt gaatttaacg ccctgggcca 300

acaaccttgg taaatttcta ctttcctcca catttttttt ttttaaagaa aggaatcatt 360

ttgctgaata ttgatggctt atacaccaaa atgcaaaaag acaaaataca ttctttcatt 420

gtggaatttt ttctttgttt ggttgattgg ttggtttggt gggtt 465

<210> 272

<211> 343

<212> DNA

<213> Homo sapiens

<400> 272
tttttttaac ttgaatccaa atgatttatt tcagttttgg ctcttaagcc acttccattg 60

ggaagaagaa actcccagga gtgacctttg agagctgagg gttctcgggg tggggcagtg 120

tgaggggctg ggccagtgtg aggggcaaag tccaacgggc atcccactcc tgcggcctgg 180

gctttcccag ctgatggggt gggggacacc catttcttta gtcccagccc ccagggaacg 240

gagcagaggg cagagggagt gaggctgcac ccaccaggaa gcctgggagt gcccttgctg 300

ggggctgggg cagaggcagc ccggcaggcc ctacagggag gct 343

<210> 273

<211> 477

<212> DNA

<213> Homo sapiens

<400> 273
tttgatcgcc caagcagaga ctgtatttcc cagattttct ggcagccaga gttggtcaca 60

gggctaattc tggtccatgg aatgtggctg gaaatgatgt gtccaacttc cgagtcacgc 120

cctgcaaagg aggttgtttc ccctccattc tgcttttcca tcctcctgct ggcttggaag 180

tggacggtcc tggatcagct gccctgtgac acactggcga tgaaagcagt ctcctcaggt 240

gaacacattt tatgttgcag tcacagtatg agcttcagat tgtcttttaa aaatactttt 300

ccttcttcca tcatggaccc aggaacacgc cagcagagag gtgtctgaag gcagcgctca 360

ggtcgcagaa gcatgaggat ctggctgtca ccagccccgt agccagggct gtgaggacca 420

gggcagccgc tgtcccctgc ctagcctggc agcctgggat gaaggaggac accaggc 477

<210> 274

<211> 258

<212> DNA

<213> Homo sapiens

<400> 274
tttgaaattt ggaactttta ttaaatactc attcatccat gtgccaccac cacccagagc 60

aggaaaaaaa ttatcaaaat ggaattaaaa aaaacaaaac aaatcaaacc caatccccag 120

cagtctatgt acagggccac tccctgcctc tctgccatag agaggttggg gggcagctga 180

ggagtggtgg gggctgggca ccttttcttc agccacaggc ccctgaggaa ttaattgact 240

gtggtgtcag gaagtccc 258

<210> 275

<211> 179

<212> DNA

<213> Homo sapiens

<400> 275
ttatttagaa cttgaaaact ttatttcagt aaatggaaaa aaaataatgc atgtagcttt 60

aacagttcac aagaagaaaa tagaggacac aaaagaacac cttgagaggc atctgtgtaa 120

gaagcagctg aaatcattcc cagggtaacc ccccatccca taacaaacgg atagggaga 179

<210> 276

<211> 395

<212> DNA

<213> Homo sapiens

<400> 276
taacaaacct tttttttttt tttattggag ataaaaacag cgaagtccca cataccatac 60

cctacaagac acaaggtgcg cagacgagcc ttggctatgt accggcgctg caggaagagg 120

ctgtccgccg ggcctgggct gctccagcta cgcgaggagg cggccccatt gcaaagtgca 180

gtttctccgc ggaggtggcg gtgggtcagt ggcagagggc catggtttcc atgttaagga 240

agcggacgtg catcttggtc tcaatgtcga tcccctgcca gatcatctgc cccatgcaca 300

cgctggccgc ctccatcatg gccccgtcgg cgatggagcg agcggactcc ttctcgatgt 360

gagggtttcc cgacagcagc tcctcgacca cttta 395

<210> 277

<211> 406

<212> DNA

<213> Homo sapiens

<400> 277
attacaaagg gcttatgatg attttattgg ctgccagagg aacacagtaa ataactccca 60

ggtgtcttgt tggaaattaa tcatgtggaa attatcactt ctttggtgca ataatgcctt 120

ccagttgggc cttcagctgc tgatttgttc gcttcaggaa ctgaatctcc tcattgtgct 180

tcttctcctc cacctgccgc ctctcgctct cccgcttctc agtggcttca cattttgcct 240

tctgctcgtt cacttgcctc tccaggtctc tcttttccgt ctccaattct gcgattttcc 300

tctccatgtc tgacttcccc tgctcagcct gcagtgcctt cctcatgcca aacgccacgc 360

tgctctcgta cagggtctgg taggcagcga tggtcatgcg gatctc 406

<210> 278

<211> 495

<212> DNA

<213> Homo sapiens

<400> 278
ccgcccgcag actctttgct caagtacgac accccagtgc tggtgagccg gaacacggag 60

aaacggagcc ccaaggtcgg ctactgaaag tcagcccccca gcagcctgga ccttcaggtt 120

cagccccaca gccacccaag accaagctcc cctcaactcc ctgtgtccca gatcctacaa 180

agcaggcaga agaaatcttg aatgccatac taccccccaag ggagtgggtg gaagacacgc 240

agctatggat ccagcaggtg tccagcaccc ctagcaccag gatggacgtg gtgcacctcc 300

aggagcagtt agacttaaag ctgcagcagc ggcaggccag ggaaacaggc atctgccctg 360

tccgcaggga actctactca cagtgttttg atgagttgat ccgggaggtc accatcaact 420

gtgcggagag gggctgctg ctgctgcgag tccgggacga gatccgcatg accatcgctg 480

cctaccagac cctgt 495

<210> 279

<211> 470

<212> DNA

<213> Homo sapiens

<220>

<221> misc_feature

<222> (6)..(6)

<223> a,t,g,c

<220>

<221> misc_feature

<222> (10)..(10)

<223> a,t,g,c

<220>

<221> misc_feature

<222> (13)..(13)

<223> a,t,g,c

<220>

<221> misc_feature

<222> (49)..(49)

<223> a,t,g,c

<220>

<221> misc_feature

<222> (51)..(51)

<223> a,t,g,c

<220>

<221> misc_feature

<222> (73)..(73)

<223> a,t,g,c

<220>

<221> misc_feature

<222> (94)..(94)

<223> a,t,g,c

<220>

<221> misc_feature

<222> (130)..(130)

<223> a,t,g,c

<220>

<221> misc_feature

<222> (232)..(232)

<223> a,t,g,c

<220>

<221> misc_feature

<222> (235)..(235)

<223> a,t,g,c

<220>

<221> misc_feature

<222> (256)..(256)

<223> a,t,g,c

<220>

<221> misc_feature

<222> (328)..(328)

<223> a,t,g,c

<220>

<221> misc_feature

<222> (412)..(412)

<223> a,t,g,c

<220>

<221> misc_feature

<222> (432)..(432)

<223> a,t,g,c

<220>

<221> misc_feature

<222> (454)..(454)

<223> a,t,g,c

<400> 279
caggtntatn ttntttaatt atcactcaca tatttcacag gaaaaggant ntagcaaatg 60

ggtcaaggtg gtntaaaaaa aaaatccagg tttntacatg tctctctgtt tacatctggg 120

agaaaggttn tcctggcatc agtcgcagca gctgcacttc tctgacgccc ctttgcaaac 180

acagccctgg gcacacttgc tacagcccac ggggaggcag gagcagcagc tnttnttgca 240

ggagggtgca tttgcnctct ttgcacttgc agggaaccag cgcagggtgc agggagacac 300

cagcgggcgc agggagcagt tgggggggncc cattgcaagc ccgagggaga gactgggact 360

tttcccaagg agagaagcga aggaagccag tgggggggcag ctcgtgcccg anttccttca 420

gccccggggg gntcccccta gttctaggag cggnccccac cgggtgggat 470

<210> 280

<211> 321

<212> DNA

<213> Homo sapiens

<220>

<221> misc_feature

<222> (138)..(138)

<223> a,t,g,c

<220>

<221> misc_feature

<222> (164)..(164)

<223> a,t,g,c

<220>

<221> misc_feature

<222> (236)..(236)

<223> a,t,g,c

<220>

<221> misc_feature

<222> (242)..(242)

<223> a,t,g,c

<220>

<221> misc_feature

<222> (291)..(291)

<223> a,t,g,c

<220>

<221> misc_feature

<222> (302)..(302)

<223> a,t,g,c

<220>

<221> misc_feature

<222> (367)..(367)

<223> a,t,g,c

<400> 280
gtgccccaca tgcttctttc gcattctctc ttggaaagtc cagtctctcc tcggcttgca 60

atggacccca actgctcctg cgccgctggt gtctcctgca cctgcgctgg ttcctgcaag 120

tgcaaagagt gcaaatgnaa ctcctgcaag aagagctgct gctnctgctg ccccgtgggt 180

tgttagcaag tgtgcccagg ggttgtgttt gcaaaggggc gtcagagaag tgcagntgct 240

tngacttgat gccaggggaa actttttttc cagatgttaa aacagagaga natgttacaa 300

antggggttt ttttttatt a 321

<210> 281

<211> 321

<212> DNA

<213> Homo sapiens

<400> 281
tttttgacag tcataaatgc ataaagttac tttattttta agtatacata taattttgaa 60

aaatataaat gctaaaatat ctcatcttct tcatctctta attccataaa agtcacaata 120

gaaagcttca taaaactgta aaatactatg ttataggaat tttaggattt atattggaaa 180

aagttattag taatatattg aaatgttttc tgttctttat cttttttgt tccaccatgt 240

tgatctactt catttaaaat aaaaagatgc atcatttctc tctccaaagt ttaataaaga 300

cttaagagaa taaaaataag a 321

<210> 282

<211> 388

<212> DNA

<213> Homo sapiens

<220>

<221> misc_feature

<222> (367)..(367)

<223> a, t, g, c

<400> 282
ggattcttta atctccagct cagcttcaac aattcaacgc tgttctttct gaaaaagtac 60

acatcgtgcc ttctctactt cgctcttgga acataatttc tcatggcagc ttttactaaa 120

ctgagtattg agccagcatt tactccagga cccaacatag aactccagaa agactctgac 180

tgctgttctt gccaagaaaa atgggttggg taccggtgca actgttactt catttccatg 240

aacagaaaac ttggaacgaa agtcggcatc tctgtgcttc tcagaaatcc agcctgcttc 300

agcttcaaaa cacagatgaa ctgggatttt atgagctcca gtccaacaat tttactggga 360

ttgggantct cttacagtga ggggcaca 388

<210> 283

<211> 155

<212> DNA

<213> Homo sapiens

<220>

<221> misc_feature

<222> (5)..(5)

<223> a, t, g, c

<220>

<221> misc_feature

<222> (20)..(20)

<223> a, t, g, c

<220>

<221> misc_feature

<222> (23)..(23)

<223> a, t, g, c

<220>

<221> misc_feature

<222> (35)..(35)

<223> a, t, g, c

<220>

<221> misc_feature

<222> (39)..(39)

<223> a, t, g, c

<220>

<221> misc_feature

<222> (57)..(57)

<223> a, t, g, c

<220>

<221> misc_feature

<222> (65)..(65)

<223> a, t, g, c

<220>

<221> misc_feature

<222> (80)..(80)

<223> a, t, g, c

<220>

<221> misc_feature

<222> (90)..(90)

<223> a, t, g, c

<220>

<221> misc_feature

<222> (97)..(97)

<223> a, t, g, c

<220>

<221> misc_feature

<222> (100)..(100)

<223> a, t, g, c

<220>

<221> misc_feature

<222> (133)..(133)

<223> a, t, g, c

<220>

<221> misc_feature

<222> (152)..(152)

<223> a, t, g, c

<400> 283
accgngaaaa ctaaaagggn gtnggggaaa gacancttng gactgcttct ctcagtnccc 60

gctgncagac aatctgaagn acttttaagn gtaaccngcn cactaaccct cagcctgaca 120

cccagctatg tgncgttgtc ttgacactgt gnggg 155

<210> 284

<211> 451

<212> DNA

<213> Homo sapiens

<220>

<221> misc_feature

<222> (1)..(1)

<223> a, t, g, c

<220>

<221> misc_feature

<222> (193)..(193)

<223> a, t, g, c

<400> 284
naaggagttg ctgacttctg actctcctcc ccataatgac attatgacac cagttgatcc 60

tggggtagcc cagcccaaca gccgggttct gggccagaac gtcatgatgg gccctaattc 120

ggtcatgtca acctatggca gccaggcatc tcataacaaa atgatgaatc ccagctccca 180

tacccaccct ggnacatgct cagcagacat ctgcagttaa cgggcgtccc ctgccccaca 240

cggtaagcac catgccccac acctcgggta tgaaccgcct gacccaagtg aagacacctg 300

tacaagtgcc tcttgcccca ccccatggca gatgagtgcc tggggggggtt aacttccttc 360

cgtgaggcag ctggcaatgg gttatgggca ggatggggcc tttttccacc aggagaagct 420

tcccaagttg attttggatg ggcttttttt t 451

<210> 285

<211> 471

<212> DNA

<213> Homo sapiens

<400> 285
gtagttcaat aatattttat tggcaatagc ataggagaaa ttcaatattg aatctcagaa 60

caagaagaag ctatttacaa tgcatgtcaa ggaagagatg ggagaaggaa tgtcacaaaa 120

ttttttggta aatacatatt ttttatagag aagtaatcca tgaacctgca acatggatag 180

cttatccaac caactttaca aattactatt aatataagtt acatgcttgc catctaaagt 240

aactaaaccc atagactgaa aaactatgtg tcaaggtaac gtgagcactt taatcacttt 300

acttatattt tctaaaggca gtagtttcct ctccttttcc cgctatcata ttagatgaag 360

agacaagttc cttcaacaca aattctgata tcggctattg tgagaatccc tgtgcgagtc 420

agctagaagc ccctgccacc agtctagtgc caaccaaatg accagatggg a 471

<210> 286

<211> 472

<212> DNA

<213> Homo sapiens

<220>

<221> misc_feature

<222> (7)..(7)

<223> a, t, g, c

<220>

<221> misc_feature

<222> (38)..(38)

<223> a, t, g, c

<220>

<221> misc_feature

<222> (114)..(114)

<223> a, t, g, c

<220>

<221> misc_feature

<222> (432)..(432)

<223> a, t, g, c

<220>

<221> misc_feature

<222> (464)..(464)

<223> a, t, g, c

<400> 286
ctgcgantga ccaaaataaa ggcagatccc gacggttncg aggctcaggc ggaggcgtgt 60

tccggggagc gcacctacca ggagctgctg gtcaaccaga accccatcgc gcanccctgg 120

cttctcgccg cctcacgcga agctctacaa atgcatcaag aaagcggtga agcagaagca 180

gattggcgcg gggtgaaaga ggttcagaaa tttgtcaaca aaggagaaaa agggatcatg 240

gttttggcag gagacacact gcccattgag gtatactgcc atctcccagt catgtgtgag 300

gaccgaaatt tgccctatgt ctatatcccc tctaagacgg acctgggtgc agccgcattc 360

caaagcgccc cacctgtgtg ataattggtc aagccccatg aggagtacca ggaggcttac 420

gatcgagttg cngggaggag gttgcattcc ttgcccttac cctnttgagg gg 472

<210> 287

<211> 430

<212> DNA

<213> Homo sapiens

<220>

<221> misc_feature

<222> (311)..(311)

<223> a, t, g, c

<220>

<221> misc_feature

<222> (335)..(335)

<223> a, t, g, c

<220>

<221> misc_feature

<222> (354)..(354)

<223> a, t, g, c

<220>

<221> misc_feature

<222> (373)..(373)

<223> a, t, g, c

<220>

<221> misc_feature

<222> (378)..(378)

<223> a, t, g, c

<220>

<221> misc_feature

<222> (387)..(387)

<223> a, t, g, c

<220>

<221> misc_feature

<222> (395)..(395)

<223> a, t, g, c

<220>

<221> misc_feature

<222> (416)..(416)

<223> a, t, g, c

<400> 287
tagtcttagc atttactttc cccaccccac attcttggaa cagcctttag ttctacagga 60

aatggcactg atggacagaa gactagcatt accttcatga aagggctgtt agagctgcct 120

gggaagaagg cgtgccttgg ggaactggga agatgccgtc agtgtgggtg ggcaggagga 180

cagccagtcg tcctgctgcc agcccaatag cttccagcgg tcaggtgccc aggtgctacc 240

ggagcccctc ataggggtaa ggggcaggga ctgcacctgc ctccagggca ctcattcgta 300

agcctcctgg nactcctcat ggggcttgac cattnatgca cacaggtgtg gggngctttg 360

gagccttgcg ctnggcancc cagggtnccg ttttnaggag gggatatgag gacatnaggg 420

gcaaattttc 430

<210> 288

<211> 561

<212> DNA

<213> Homo sapiens

<220>

<221> misc_feature

<222> (554)..(554)

<223> a, t, g, c

<220>

<221> misc_feature

<222> (495)..(495)

<223> a, t, g, c

<220>

<221> misc_feature

<222> (536)..(536)

<223> a, t, g, c

<400> 288
ttttaaaaag atacagtctt tacttttatt gaaataccaa atattgacta tgcaagctat 60

actggtaaat gtgctctttg atgttgacag aggagggctg ggctgcctgt ggtttccttt 120

cagattgtct gtgagtctaa tttctcatgg tcgtgtgaca aggaccccat ctttagctga 180

aattttcaat tgccaggaaa gaggtagaaa tctcttgtca aggttgctcg ttctatggtg 240

ggcatctggg ctttagctcg tggaacttca aatgatttct gtaccttccg aaatatttcc 300

attaggtggc agcagcaaga atatttctgg aagcatgtga tgagttccgt aatgaagatg 360

gagccccttg tgcggtctct ccaggacacg ttatgtggtg ttgaagaaca gaaagcaatg 420

aagtccttct cctccgtgga tcttgcaaac agaatctgcc tccaaggttc tcagaggact 480

tgtgaagaga tgacngccaa aggatgctgg agagtcttgg acccagagtt ccccanggtt 540

ttcaacctct gcangccccg g 561

<210> 289

<211> 578

<212> DNA

<213> Homo sapiens

<220>

<221> misc_feature

<222> (545)..(545)

<223> a, t, g, c

<400> 289
gaaaagaaaa aatattatga taccaaaatt gaagacaagg ccctgatctt ggtagactct 60

ttgcgaaaga atcgcgtggc tcatcaaatg tttacccaaa cacttctcaa tatggaccaa 120

aagatcacca gtgtaaaacc tcttctgcaa atcgaggctg gaccacctga gtcagcagaa 180

tctacaaata tactcaaact ttgtcctcgt gaagaattcc tgagactgtg taaaaaaaat 240

catgatgaga tctatccaat aaaaaagaga gaggaccgca gacgcctggc tctcatcata 300

tgcaatacaa agtttgatca cctgcctgca aggaatgggg gctcactatg acatcgtggg 360

gatgaaaagg ctgcttcaag gcctgggcta cactgtggtt gacgaaaaga atctcacagc 420

cagggatatg gagtcagtgc tgaagggcat ttgctggcca gaccagagca caagtcctct 480

gacagcacgt ccttgggtac tcatgtctca tgggcatcct agaggggaat ttgcggaact 540

gggcnttaaa agggaaaaac cgggattttt ctgctttt 578

<210> 290

<211> 469

<212> DNA

<213> Homo sapiens

<220>

<221> misc_feature

<222> (454)..(454)

<223> a, t, g, c

<220>

<221> misc_feature

<222> (456)..(456)

<223> a, t, g, c

<400> 290

```
ctcaaatatc caattttatt ttatcattct cgcattgtgg gatgcgatct gcagctagga     60
tcggaattcc caggcctata gatttttaaa ccacaccaca ggggtaaacc ttaaaagaag    120
tgaaacctaa cactatatat atttccattt ctaaatacag tatattacag aagtttaaat    180
ataccacctc tgtgtactta caactataaa aagatacaat aactctacca attataaata    240
atgtagcatt tcatattaaa gacattatcg tacaatggaa gaataggaac cctctaacgt    300
atcactatca aggttagtgt ctatatctac ttgagataaa atactgaaaa ttcagtgtat    360
gaagccaaat cctgatttaa caagttattg ggtagtataa gtggataagt gttagccgga    420
tgaagggaag gccaatggtg ggtaatttat atcncnggac aaggggtga                469
```

<210> 291

<211> 456

<212> DNA

<213> Homo sapiens

<220>

<221> misc_feature

<222> (178)..(178)

<223> a, t, g, c

<220>

<221> misc_feature

<222> (456)..(456)

<223> a, t, g, c

<400> 291
gggattctat ggaggacctc ggtcttcatc caagtggcct gagtatttca ctggcaggtt 60

gtgaattttt cttttcctct ttggggatcc aaatgatgat gtgcaatttc atgttttaac 120

ttgggaaact gaaagtgttc ccatatagct tcaaaaacaa aaacaaatgt gttatccnga 180

cggatacttt tatggttact aactagtact ttcctaattg ggaaagtagt gcttaagttt 240

gcaaattaag ttggggaggg caataataaa atgagggccc gtaacagaac cagtgtgtgt 300

ataacgaaaa ccatgtataa aatgggccta tcacccttgt cagagatata aattaccaca 360

tttgccttcc cttcatcagc taacacttat cacttatact accaataact tgttaaatca 420

ggatttggct tcatacactg aattttcagt attttn 456

<210> 292

<211> 447

<212> DNA

<213> Homo sapiens

<220>

<221> misc_feature

<222> (351)..(351)

<223> a, t, g, c

<400> 292
taaattaaaa aacaatttat tgaaaaagag taatgcttta tacaaattcc tattataaaa 60

ccccaaaatg tctattggtc tgtttccagg tgtggtagaa gaatataaaa agatcaaaat 120

tggataaatt ctattgtaac aatttcgttg gtcattttgg gccataaaat ttttttgtaa 180

tgtttggtaa ctgatatcca catggaatta cactcacaca tcatgaagat ctatgtatgt 240

ggcaaaagcc atttaaattt taacttccaa aagcatatat tctcaggttt ggaaggcaca 300

ctaaatttta ttaggtccaa ttcctcataa gacacagtgg ctgactttcc ntgtgtagtt 360

tattatgaag taccatttcc aaactaacta tcctagcagc gtccgcaagg ctaacagcaa 420

gagctctgag ggcatcactg aacagat 447

<210> 293

<211> 330

<212> DNA

<213> Homo sapiens

<400> 293
tttaaaatct catctgaatg catctaagtc aagcatgagg gaggtgaagt atgagtcatt 60

ccaaggcaaa attcttcttc agttgtgaag catggaacca gacaagtttt gtgcttccaa 120

aatgcagtgg tggtgcaggc ataggataga cgttccattc tggaggctag atggttgaga 180

tccaggtgtc agtacggttg gctccttctg aggcctctct cctttgcttg tagacagcca 240

ttttctctgt gtcttcactg gtccttctct gtttgtgtcc taatctcctt ttcttataag 300

gacagcattt atattggatt agataccacc 330

<210> 294

<211> 417

<212> DNA

<213> Homo sapiens

<400> 294
gaccagacag ggcttccatc cagcagtgcc catgcctaga tatacctggc gcaccgcaca 60

acatcccagc ccacacaagg ccctgctacc tccatccaac atactgctag actgacccta 120

ctgaatccac attcccacat attaccctga ctcatatcac ctagatcatg acagacacca 180

gatgtggcct tgaaccacac ctctgaacct ccaatctacc attctctctt gcctcctgac 240

tccaggcagg gggctcacaa gttagcagag cagggcctaa cccaattaca tagccataga 300

ctgctatggg ttgtgtttct acagaagtca aatgttgaag tcctaacttc cactatttca 360

gaatatgact gtttttgaga tagggccttt aaagaggtga ttaatgctaa atgggat 417

<210> 295

<211> 427

<212> DNA

<213> Homo sapiens

<400> 295
ttttgccaag gatattcaga gcggctttat ttataattca tatacgtata tgtgtattta 60

gctttagtaa atattgccaa gtaattttgc aaagtgctcg taacactgct ttaggttttg 120

tcacggaaga ggaagttgga ggccccagta ggagacagga ataatcagta gcaggggtga 180

taatgggcca ttgcaatcca aaggcatgtt gtctatgacc aggaatggca aattgctgat 240

ccttgcaata tgtaatgagt ggaaaatgct gagactttgc cacctggccc attcttaaac 300

atctccttag agagctctgc ccggagaaga gctacttcac gcgacgacaa ccgccgtttc 360

cgacctcaca ggtccaggcg ttcccgacgc tctcgctccg acaacgccct ccacctggcc 420

agcgaac 427

<210> 296

<211> 368

<212> DNA

<213> Homo sapiens

<400> 296
ccgacccccc ggcgcggccg tgcttctgcc cctacaaggt ttgggccgag gtgggggagg 60

gtcctggttg ccggccccgc cgtcacctcc ccgcctttta ggcaccgcgt ggccgggacg 120

tcccagtcgc ctccgtcctc ctcgcctgcc accggtgcac ccagtccgct cacccagccc 180

agtccgtccg gtcctcaccg cctgccggcc ggcccacccc ccaccgcagc catggacgcc 240

atcaagaaga agatgcagat gctgaagctg gacaaggaga acgccatcga ccgcgccgag 300

caggcgaagc cgacaagaag caagctgagg accgctgcaa cagctggagg aggagcagca 360

ggccctcc 368

<210> 297

<211> 541

<212> DNA

<213> Homo sapiens

<400> 297
ttttgagagg ctagtaacat cagttttatt gggttggggt ggcaacatag cctggctggg 60

ggtggggctg gcctcacagg ttgttgagtt ccagcagggt ctggtccaag gtctggtgaa 120

tctcgacgtt ctcctccttg gcactggcaa ggtctcttct aggtcatcga tggttttctc 180

caactttgcc acagacctct cggcaaactc tgctcgggtc tcagcctcct tcagcttctc 240

ctccaacagt ttgatctcct cttcatattt atcttctttg gtggaatact tgtccgcctg 300

ggcctccagg gatttcaagt tgttggtaac aattttcagc tcctcctcta ggtccccaca 360

tttactcctc ctctgaggcc atcagggact tgagggcctg gtccatggtt cgaagttcct 420

cctccagctg tctggctcgg ctctcggcca cctcagccct ctcctccgag cgctccagct 480

ctccttccag gatcaccagc ttcctggcca cctcttcata tttgcggtct gaatcctcag 540

c 541

<210> 298

<211> 397

<212> DNA

<213> Homo sapiens

<220>

<221> misc_feature

<222> (11)..(11)

<223> a, t, g, c

<220>

<221> misc_feature

<222> (12)..(12)

<223> a, t, g, c

<220>

<221> misc_feature

<222> (13)..(13)

<223> a, t, g, c

<220>

<221> misc_feature

<222> (14)..(14)

<223> a, t, g, c

<220>

<221> misc_feature

<222> (374)..(374)

<223> a, t, g, c

<400> 298
aattaatata nnnntgaaac aatttggtaa tgcagactgg gacaatgtca ctatacccac 60

tgaggtaact gagacatgag agctttccaa cccaaggtca gaaatcactg aaacagctta 120

tgaaaccctc actattttag tgttttaaat tcagaatgct gtatttttct cttgggcaac 180

agataacctt catttttgac gtaaatgata atggtggctt cacatggccc agggcaaact 240

tcaaaacaaa actgtaggga ccacattttt ctaacctgga aaataagtca tataaggggt 300

ccaaataagg gtaaaatttg ggaatttaat tattcctggg gggaaaatat tgtgggggta 360

tccctggtat cctngggccc ggtaatcccg ggtttta 397

<210> 299

<211> 352

<212> DNA

<213> Homo sapiens

<220>

<221> misc_feature

<222> (326)..(326)

<223> a, t, g, c

<220>

<221> misc_feature

<222> (346)..(346)

<223> a, t, g, c

<400> 299

tcaatttaaa aaattacaca tggaacaagc caaataaata tttttaaaat actgttttta 60

tttagagaga gagacagctg tataaactgc tctgtgtatg tgtaaaacat tgataataca 120

ttagacataa tttgcagaaa ctactaattt tcacatcatt tccaagtaga actgcaaagg 180

tacagtactg gtttagcttt atggttaaaa gcattgggct ctaggagcca ggataacaca 240

ggcttcaaac cctagttctg ccgtttactg ggttgtgtta ccttggggta gggttacttg 300

gaccttttg aggcttccag gtttcntaat cttttatttt aatggngttt tt 352

<210> 300

<211> 169

<212> DNA

<213> Homo sapiens

<400> 300
aaatttaaaa agatgtcctc actgcacaag tgactacggg ctacaggcaa ggatgggaga 60

cggaggcttc aacacaactc attgcactta gaaccgttac taaccgaaac accatttgct 120

tgtcaacaat gtacccttga cagcagggag aaacttcttt atagtctct 169

<210> 301

<211> 219

<212> DNA

<213> Homo sapiens

<220>

<221> misc_feature

<222> (203)..(203)

<223> a, t, g, c

<220>

<221> misc_feature

<222> (208)..(208)

<223> a, t, g, c

<400> 301
atcacagaga aagactcaga ccctgcagat ggagaaggcc cagagacatt aagctcagca 60

ctctctaaag gagcaacagt ttacagccct tccagataca gctaccagct cctgcagtgt 120

gatagtcctc ggacagaatc acaaagcctc cttcagcaga gttcctcccc cttcagagga 180

cattcctaca cagtctccca ggntacantt atcggaact 219

<210> 302

<211> 469

<212> DNA

<213> Homo sapiens

<220>

<221> misc_feature

<222> (379)..(379)

<223> a, t, g, c

<400> 302
actgtgataa aacatacaca gtttacaaga atatgaaatg ctttactaat tcgtgtgtca 60

tccatgctaa tcatctctct attgttccaa ttttagtaca ggtgcttttg aagtgggcac 120

tcttaatttt ttgaacattt tctaggtttc tgataccata ctcattctgt gtcttaccta 180

tcacaacccc agaatcagcc atttctccaa attcctttta gtggagagtg gtatttagaa 240

accaggatct ggacaccatt tctcttttttg ttattgttgt ttgccttgct ttaatgatag 300

ctctttttat taatttttcc attattataa aagatggcca aatacataca tttctatgga 360

aaatgaatca agtcttatnt attttacagt taaaatttca ttattcctat tttaactgat 420

aaaccagttt aattttcaag atgtattaaa gtctcccaca attgtattc 469

<210> 303

<211> 299

<212> DNA

<213> Homo sapiens

<220>

<221> misc_feature

<222> (296)..(296)

<223> a, t, g, c

<400> 303
aatagaatac aattgtggga gactttaata catcttgaaa attaaactgg tttatcagtt 60

aaataggaat aatgaatttt aactgtaaaa taaataagac ttgattcatt ttcatagaaa 120

tgtatgtatt tggccatctt ttataataat ggaaaaatta ataaaaagag ctatcattaa 180

agcaaggcaa acaacaataa caaaaagaga aatggtgtcc agatcctggt ttctaaatac 240

cactctccac taaaaggaat ttggagaaat ggctgattct ggggttgtga taggtnagc 299

<210> 304

<211> 606

<212> DNA

<213> Homo sapiens

<400> 304
cagttctcta gtatctttat tatccattcc acgggggcac tcatggctgc acaactcagg 60

ggtgcagatg tctccctgca ccatgccagg tgtggtgcag aatgggaggc agggggatgg 120

agcagagaga ggcctggact gcctgtggct cttgtcgagt cactgccctt ctgtaggctc 180

tgctctggaa atgctggggt cagaggcagg tgttggggag cagccctgtc tccctctgtc 240

ctccagggga gcagctgggc ctgaggagga agcacccctg ccccagggtc ccgaagcccc 300

agaggaccca tatccccctg gggcagttgg ttgcccctcc ctgaccttgg gctctggaag 360

gtcagagcag cagagcagac tgcccgtttt cccccatctc agggcctggc tgaggcaggg 420

caggcaggag agaggcccct gaggccgggt ttcaggccct gggccaggca ctcagatggg 480

gtgcacgaac tggtagacga ggcgctggga aatgtctggc ttccggatga tgcccttctt 540

gtaatactgg ccgatggagc cgctcagctt gtcgtagttc atggcgggac gttcttgcgg 600

atcccc 606

<210> 305

<211> 514

<212> DNA

<213> Homo sapiens

<400> 305
ggagggcatc ttcaaaattg aggactcagc ccaggtggcc cggctgtggg gcatccgcaa 60
gaaccgtccc gccatgaact acgacaagct gagccgctcc atccgccagt attacaagaa 120
gggcatcatc cggaagccag acatctccca gcgcctcgtc taccagttcg tgcaccccat 180
ctgagtgcct ggaccagggc atgaaacccg ccctcagggg cctctctcct gcctgccctg 240
cctcagccag gccctgagat gggggaaaac gggcagtctg ctctgctgct ctgaccttcc 300
agagcccaag gtcagggagg ggcaaccaac tgccccaggg ggatatgggt cctctggggc 360
cttcgggacc atggggcagg ggtgcttcct cctcaggccc agctgctccc ctggaggaca 420
gagggagaca gggctgctcc ccaacacctg cctctgaccc cagcatttcc agagcagagc 480
ctacagaagg gcagtgactc gacaaaggcc acag 514

<210> 306
<211> 375
<212> DNA
<213> Homo sapiens

<220>
<221> misc_feature
<222> (298)..(298)
<223> a, t, g, c

<220>
<221> misc_feature
<222> (304)..(304)
<223> a, t, g, c

<220>
<221> misc_feature
<222> (309)..(309)
<223> a, t, g, c

<220>

<221> misc_feature

<222> (341)..(341)

<223> a, t, g, c

<220>

<221> misc_feature

<222> (349)..(349)

<223> a, t, g, c

<220>

<221> misc_feature

<222> (358)..(358)

<223> a, t, g, c

<400> 306

```
tatttgagca atttattttt tttcaaagaa atccatgcaa agagagaacg atagaaaggg      60
agggagtgag agagaaagac atggtaacca tgttctagca gccaaacata ggagaataat     120
ttggcacaac ttgatggttt tttttctttc tttgcaaaag gacaatctat atgctaccac     180
taaaatgtat cttcctccaa aagataccat ttgattttcg aaaacataac acatgggtta     240
ggtcctctac ttcatgggct gggagggagg gtatacaggg ataacaaaaa aaaaaaanta     300
gggnaaaant aaggaaagtg ggttcccctt tatcgggggg nggaccccng gggtgctnta     360
gggggcaggc acatg                                                      375
```

<210> 307

<211> 433

<212> DNA

<213> Homo sapiens

<220>

<221> misc_feature

<222> (243)..(243)

<223> a, t, g, c

<220>

<221> misc_feature

<222> (368)..(368)

<223> a, t, g, c

<220>

<221> misc_feature

<222> (379)..(379)

<223> a, t, g, c

<220>

<221> misc_feature

<222> (432)..(432)

<223> a, t, g, c

<400> 307
tggaatgata aaagtgaaga atcagctccg cttccttgca gaactggcct atgatctgga 60

tgtggatgat gcgcctggaa acagtcagca ggcaactccg aagacaacga gataagcacc 120

tttcacaacc tcgggaacgt tcattccccg ctgaagcttc tcaccagcat ggccatctcg 180

gtggtgtgct tcttcttcct gggtgcactg actgcctggg tgcccagcac atgtgctgcc 240

ctnacagcac cctgtgggtc ttccttcgat aaaaggggaa ccactttctt atttttttct 300

attttttttt ttttgttatc ctgtataacc tcctccagcc atgaagtagg agggcttaac 360

cagtgttnat gtttttcgna aatccaaatg ggtatctttt tgggagggaa ggtacatttt 420

agtgggtagg cna 433

<210> 308

<211> 491

<212> DNA

<213> Homo sapiens

<220>

<221> misc_feature

<222> (393)..(393)

<223> a, t, g, c

<220>

<221> misc_feature

<222> (402)..(402)

<223> a, t, g, c

<220>

<221> misc_feature

<222> (409)..(409)

<223> a, t, g, c

<220>

<221> misc_feature

<222> (421)..(421)

<223> a, t, g, c

<220>

<221> misc_feature

<222> (438)..(438)

<223> a, t, g, c

<220>

<221> misc_feature

<222> (460)..(460)

<223> a, t, g, c

<220>

<221> misc_feature

<222> (478)..(478)

<223> a, t, g, c

<220>

<221> misc_feature

<222> (491)..(491)

<223> a, t, g, c

<400> 308
ctgaaactgt cggaatatat gggtcttgaa attcagaaga tgatagtcac tcttcccata 60

tttataggct attaaggcaa gggatatctt aaacatcata ttactttatt tagatttcta 120

ctactccaat tattaatgtt atgtatttct cattgtttta cttcttcatg gtattatgaa 180

gactatatag atgattcaac caagcctgca aatctccctc ttgtgggaat tccactggga 240

cccaatctgt tttccatttc cattgcaata ctactaaagc catacaatat caaggcaccc 300

tccctctagg gtccagggga ctatccacag gaggaggcag ggcatgtaag gattttaagg 360

gactgggttt cgagggggtc cgagtgtagg ggnaaacagc cntgtttgnc atttgttagg 420

ngtggatgtc catcttgnag gagccgctgg gcctggatgn cctgctgttt tgactccngg 480

catacccaga n 491

<210> 309

<211> 471

<212> DNA

<213> Homo sapiens

<220>

<221> misc_feature

<222> (6)..(6)

<223> a, t, g, c

<220>

<221> misc_feature

<222> (92)..(92)

<223> a, t, g, c

<220>

<221> misc_feature

<222> (106)..(106)

<223> a, t, g, c

<220>

<221> misc_feature

<222> (118)..(118)

<223> a, t, g, c

<220>

<221> misc_feature

<222> (142)..(142)

<223> a, t, g, c

<220>

<221> misc_feature

<222> (183)..(183)

<223> a, t, g, c

<220>

<221> misc_feature

<222> (189)..(189)

<223> a, t, g, c

<220>

<221> misc_feature

<222> (268)..(268)

<223> a, t, g, c

<220>

<221> misc_feature

<222> (297)..(297)

<223> a, t, g, c

<220>

<221> misc_feature

<222> (337)..(337)

<223> a, t, g, c

<220>

<221> misc_feature

<222> (430)..(430)

<223> a, t, g, c

<220>

<221> misc_feature

<222> (463)..(463)

<223> a, t, g, c

<400> 309
ggtttngaga taaaccaatt ttatgtctat catgttatac aaaaatctag aaataataga 60

tttgtacaga aaaaaatgat aataaatgag ancacaaaac atatanttta aatttggnat 120

ttttcccccc atgatattag gntgataatc atttcaaagc acatgtctag cttcagagta 180

ggntttgtnc actggccaaa gcctgccatg aaactatggc tttcagcatc tgtctgctct 240

actggctctt gacaaaactc ttgaggtntt caagaaaagt aatgtactcc tggggcnccg 300

ggctgtgctg agctccacca gctcatctgc aaaagtnttg tccacccctc ggtcggcaag 360

gaaatccatt aggtggtcat ataaggccca gtccaaggaa tccgtgttga gtgtataatt 420

agtatccccn cgttcccgaa tttttggcct cgagggcaaa ttnccccata g 471

<210> 310

<211> 438

<212> DNA

<213> Homo sapiens

<220>

<221> misc_feature

<222> (1)..(1)

<223> a, t, g, c

<220>

<221> misc_feature

<222> (372)..(372)

<223> a, t, g, c

<220>

<221> misc_feature

<222> (384)..(384)

<223> a, t, g, c

<220>

<221> misc_feature

<222> (397)..(397)

<223> a, t, g, c

<220>

<221> misc_feature

<222> (409)..(409)

<223> a, t, g, c

<220>

<221> misc_feature

<222> (432)..(432)

<223> a, t, g, c

<400> 310
ngggatacta attatacact caacacagat tccttggact gggccttata tgaccaccta 60

atggatttcc ttgccgaccg aggggtggac aacacttttg cagatgagct ggtggagctc 120

agcacagccc tggagcacca ggagtacatt acttttcttg aagacctcaa gagttttgtc 180

aagagccagt agagcagaca gatgctgaaa gccatagttt catggcaggc tttggccagt 240

gaacaaatcc tactctgaag ctagacatgt gctttgaaat gattatcatc ctaatatcat 300

gggggaaaaa ataccaaatt taaattatat gttttgtgtt ctcatttatt atcatttttt 360

tctgtacaaa tnctattatt tctnagattt ttgtatnaac atggatagnc ataaatttgg 420

gtttatcctc cnccaaaa 438

<210> 311

<211> 255

<212> DNA

<213> Homo sapiens

<400> 311
ttaaaaaatc aactacagag aaagggccat gtcttcgagt ccatgttcac attgtcccac 60

gctgtcgcac caggatgggc cgcatacttt gtcctggctg ggactgtggc ctcttggaag 120

ggtactgggc cactccaggg ctcgggtgct gctcctcctg ggatccacta gagggtgaac 180

tctgggccct cccagactcc tgctcctctg gggcccccag ggactctggg caacccttcc 240

ccttgctcca aacca 255

<210> 312

<211> 305

<212> DNA

<213> Homo sapiens

<220>

<221> misc_feature

<222> (224)..(224)

<223> a, t, g, c

<400> 312

```
gataagttcc ctaacatctc cagctcctgg ctctggtttg gagcaagggg aagggttgcc      60
agagtcctgg gggccccaga ggagcaggag tctgggaggg cccagagttc accctctagt     120
ggatccagga ggagcagcac ccgagccctg gagtggccca gtacccttcc aagaggccac     180
agtcccagcc aggacaaagt atgcggccca tcctggtgcg acangtgggg acaatgtgaa     240
catggactcg aagacatggc cctttctctg tagttgattt tttaaatgtg ccattattgt     300
tttta                                                                 305
```

<210> 313

<211> 449

<212> DNA

<213> Homo sapiens

<220>

<221> misc_feature

<222> (2)..(2)

<223> a, t, g, c

<220>

<221> misc_feature

<222> (3)..(3)

<223> a, t, g, c

<220>

<221> misc_feature

<222> (14)..(14)

<223> a, t, g, c

<220>

<221> misc_feature

<222> (43)..(43)

<223> a, t, g, c

<220>

<221> misc_feature

<222> (419)..(419)

<223> a, t, g, c

<220>

<221> misc_feature

<222> (424)..(424)

<223> a, t, g, c

<220>

<221> misc_feature

<222> (434)..(434)

<223> a, t, g, c

<220>

<221> misc_feature

<222> (447)..(447)

<223> a, t, g, c

<400> 313
annagtggat attncacagt ttatttccaa acactcagag ganagggggt ggcctatggg 60

tccatctgcc tcccctccct gcatttggct gaatcaagaa cttctccccc ctgacccccca 120

agaccctagg ctgtgccaat agagaaggca ctgtcacccc ccaccctgag gagacctaga 180

caggtgagga gcatggaggg tgggggaaca gagttcaagt attcacagtt cccccatcta 240

caccccggga ttacactgtg ccagagccat gagggaggat cccacctctg ggattcctag 300

tgggtgttga tagtccttct cccacttagg aaccctccag atgaactccc tactcctttt 360

ccctggaaat aacaaaccaa ggtcctaagg ccctgtagtt tggggcaagg gagggagtnt 420

aggnagggcc attnacccaa gggcccntt 449

<210> 314

<211> 409

<212> DNA

<213> Homo sapiens

<220>

<221> misc_feature

<222> (227)..(227)

<223> a, t, g, c

<220>

<221> misc_feature

<222> (232)..(232)

<223> a, t, g, c

<220>

<221> misc_feature

<222> (283)..(283)

<223> a, t, g, c

<220>

<221> misc_feature

<222> (349)..(349)

<223> a, t, g, c

<220>

<221> misc_feature

<222> (360)..(360)

<223> a, t, g, c

<220>

<221> misc_feature

<222> (386)..(386)

<223> a, t, g, c

<220>

<221> misc_feature

<222> (391)..(391)

<223> a, t, g, c

<400> 314

```
ctatgttccc caggattcag ctattctgga agatcagcac cctaagagat gggactagga      60
cctgagcctg gtcctggccg tccctaagca tgtgtcccag gagcaggacc tactaggaga     120
ggggggccaa ggtcctgctc aactctaccc ctgctcccat tcctccctcc ggccatactg     180
cctttgcagt tggactctca gggattctgg gcttggggtg tggggtnggg tngagtcgca     240
gacagagctg tctgaactca cgtgtcagaa gcctccaagc ctncctccca aggtcctctc     300
agttctctcc cttcctctct ccttatagac acttgctccc aacccattnc actacaggtn     360
aaggcttttc acccccatcc tggggncctt nggttgagtt gcctggtta                 409
```

<210> 315

<211> 308

<212> DNA

<213> Homo sapiens

<220>

<221> misc_feature

<222> (16)..(16)

<223> a, t, g, c

<220>

<221> misc_feature

<222> (194)..(194)

<223> a, t, g, c

<220>

<221> misc_feature

<222> (199)..(199)

<223> a, t, g, c

<220>

<221> misc_feature

<222> (245)..(245)

<223> a, t, g, c

<220>

<221> misc_feature

<222> (274)..(274)

<223> a, t, g, c

<400> 315
cacttcaatt ttaatncttg aagaacagcc tcgacaacat atatcgcact cattataaga 60

agcaaagggt tatatcaaaa attattagta tagaatcaca ggaaatctgg tttggaacca 120

gaaaaaaata caaaacagat atagatacat agacacagga caatttttta taattatttg 180

gaaaatgtta tttnccccna attcttgttt ccttttttctt tatgcgcata cgatgtttaa 240

atttnacaaa aaatgaaaat aaagactagt attntacaaa atgaataaca atgttcagtg 300

tgtgtgtg 308

<210> 316

<211> 388

<212> DNA

<213> Homo sapiens

<220>

<221> misc_feature

<222> (357)..(357)

<223> a, t, g, c

<220>

<221> misc_feature

<222> (366)..(366)

<223> a, t, g, c

<220>

<221> misc_feature

<222> (375)..(375)

<223> a, t, g, c

<220>

<221> misc_feature

<222> (383)..(383)

<223> a, t, g, c

<400> 316
aagcatggga aaaagagact cttttaggat cagatctgtg agcacgttgg cgaggaaaaa 60

caaaacaaac aaaaaaaaga accttgtgtc tgtctggtga aaaaaagaaa aacaaattgg 120

aagagaggac catgagaatt ttaataaaac agaaggaaac taatggacct tccaggattt 180

attgtggacg gatgtggata tattctgtac aggaacaaca catatggaag tggactgaag 240

cctatgtaga aacacacaca cactgaacat tgttattcct tttgtaaaat actagtcttt 300

attttccatt tttttgtaaa attttaaaca tcgtatggcg ccttaaagaa aaagggnaac 360

cagganttag ggggnaaatt acnttttt 388

<210> 317

<211> 514

<212> DNA

<213> Homo sapiens

<220>

<221> misc_feature

<222> (374)..(374)

<223> a, t, g, c

<220>

<221> misc_feature

<222> (409)..(409)

<223> a, t, g, c

<220>

<221> misc_feature

<222> (425)..(425)

<223> a, t, g, c

<220>

<221> misc_feature

<222> (454)..(454)

<223> a, t, g, c

<220>

<221> misc_feature

<222> (486)..(486)

<223> a, t, g, c

<220>

<221> misc_feature

<222> (501)..(501)

<223> a, t, g, c

<400> 317

```
gaatagctat ggaagttctc tttattgatt acttaatgtg taacaataat tggcatcttt     60
ttcacacatt acaaaaaatt atacttggct cagtatgcaa ccttttaagc atagccatat    120
tatttaacaa aagaggggaa aacctattct acccaacaca gcatttacaa atgcacaaaa    180
catgccactt tggcttgtat attgtctaga ttaaaaaaaa tcttttaaca taaataagtt    240
agtataaatt tttcagtgtt tttacagagt tatgtacaca ggtacacttc aaatggtttt    300
tccatacaca ggcaatgaaa tactggttta aagatgtagt atccatttca cttatcctac    360
aagtgtgctt ttcnctacat gaaccttccc tgaaaaataa cactttccng tgggataaat    420
ggacngagta agccttaaat taaaaaatat ttcnattttt aatgggaagt tacctacaga    480
tccttnaaaa tatcgggggg nttaggaatt aatt                                514
```

<210> 318

<211> 505

<212> DNA

<213> Homo sapiens

<220>

<221> misc_feature

<222> (4)..(4)

<223> a, t, g, c

<220>

<221> misc_feature

<222> (5)..(5)

<223> a, t, g, c

<400> 318
tccnnttcag agacagctga tacttcattt aaaaaaatca caaaaatttg aacactggct 60

aaagataatt gctatttatt tttacaagaa gtttattctc atttgggaga tctggtgatc 120

tcccaagcta tctaaagttt gttagatagc tgcatgtggc tttttaaaa aagcaacaga 180

aacctatcct cactgccctc cccagtctct cttaaagttg gaatttacca gttaattact 240

cagcagaatg gtgatcactc caggtagttt ggggcaaaaa tccgaggtgc ttgggagttt 300

tgaatgttaa gaattgacca tctgctttta ttaaatttgt tgacaaaatt ttctcatttt 360

cttttcactt cgggctgtgt aaacacagtc aaaaataaat tctaaatccc tcgatatttt 420

taaaagatct gtaagtaact tcacattaaa aaaatgaaat attttttaa tttaaagcct 480

tactctggcc catttatccc acagg 505

<210> 319

<211> 249

<212> DNA

<213> Homo sapiens

<220>

<221> misc_feature

<222> (244)..(244)

<223> a, t, g, c

<400> 319
ataataagga aacgtgttca cttattgact attatagaat ggaactcatg gaaatctgtg 60

tcagtggatg ctgctctgtg gtccgaagtc ttccatagag actttgtgaa aaaaaatttt 120

atagtgtctt gggaattttc ttccaaacag aactatggaa aaaaaggaag aaattccagg 180

aaaatctgca ctgtgggctt ttattgccat gagctagaag catcacaggg tgaccaataa 240

cccngacgc 249

<210> 320

<211> 376

<212> DNA

<213> Homo sapiens

<220>

<221> misc_feature

<222> (172)..(172)

<223> a, t, g, c

<220>

<221> misc_feature

<222> (195)..(195)

<223> a, t, g, c

<220>

<221> misc_feature

<222> (229)..(229)

<223> a, t, g, c

<220>

<221> misc_feature

<222> (288)..(288)

<223> a, t, g, c

<220>

<221> misc_feature

<222> (292)..(292)

<223> a, t, g, c

<220>

<221> misc_feature

<222> (365)..(365)

<223> a, t, g, c

<400> 320
ttttttttt ttttttttta aagggtatgt gaacggaaac acatttatta caaaaaaaaa 60

aaaccccaaa acgaaaaaca aattcacatt gtattgagct acaatatggc agcagattaa 120

aaaaaaatat ttttacacag tttaaggtaa ctcctaacag aacatagcct tnttgccacg 180

agacaggaca caggnttcca agtactcagt agacggcgag tgaagcggnc atcgctgggg 240

cctgctcccc cggctctggc ctccaaaccc gccctgcatg ccaccgtntg gnatggggtg 300

gccccgggag gccccgcctg gggaaaaagt tgccgcgccc tgacattcct cctcggttca 360

tcatntgggc cagggc 376

<210> 321

<211> 466

<212> DNA

<213> Homo sapiens

<220>

<221> misc_feature

<222> (321)..(321)

<223> a, t, g, c

<220>

<221> misc_feature

<222> (429)..(429)

<223> a, t, g, c

<400> 321
atgaagggct ccatttgata cttccttcac tgtggaagga agaaatttgt aaaaattatt 60

tccagaatat gcacacatta aaggtttata aacctgacct ttctttagta gaagacagta 120

agagaatact gttggctctg ggaactaacc atttgagctg tgggacccag ggaatctctc 180

ttgtaagcat catctttagt gggtgactcc tggtggccct cccagctctg gtggtgctgt 240

ggggacctgc cttggagatg ccttggtact agtacctttt ttgtaccagg ccatgagcct 300

agggcacagg gcttctgctg ncagatttca ggaaggcagc tgtgttgtgt gaaccgatac 360

agcctgccag cacttctgtc ccgagttgac tgtcgttgtt catccttagc atgtttgcct 420

gaaaaagcnt ttggggtctt gggcgttgac attgaagttt tttttt 466

<210> 322

<211> 341

<212> DNA

<213> Homo sapiens

<400> 322

ccattttggg gtggagaagg tggatgtgat gaagccaata attcaggact tattccttct 60

tgtgttgtgt tttttttttg cccttgcacc agagtatgaa atagcttcca ggagctccag 120

ctataagctt ggaagtgtct gtgtgattgt aatcacatgg tgacaacact cagaatctaa 180

attggacttc tgttgtattc tcaccactca atttgttttt tagcagttta atgggtacat 240

tttagagtct tccattttgt tggaattaga tcctcccctt caaatgctgt aattaacaac 300

acttaaaaaa cttgaataaa atattgaaac ccctccgtgc c 341

<210> 323

<211> 350

<212> DNA

<213> Homo sapiens

<400> 323

ggtttcaata ttttattcaa gtttttttaag tgttgttaat tacagcattt gaaggggagg 60

atctaattcc aacaaaatgg aagactctaa aatgtaccca ttaaactgct aaaaaacaaa 120

ttgagtggtg agaatacaac agaagtccaa tttagattct gagtgttgtc accatgtgat 180

tacaatcaca cagacacttc caagcttata gctggagctc ctggaagcta tttcatactc 240

tggtgcaagg gcaaaaaaaa aacacaacac aagaaggaat aagtcctgaa ttattggctt 300

catcacatcc accttctcca ccccaaaatg gcacaaaaaa aacaaaaaag 350

<210> 324

<211> 479

<212> DNA

<213> Homo sapiens

<400> 324
gtgttagaga ttatttatta ttactcttag gggaatgaat tctggacaag ggatgtgaaa 60

attccagaaa ccctcgattg tgaccaaaat gactgtaaaa tatggagcac agcctgtcac 120

tatatgaata taaatggcct ttgcagagag gtaagttctt tggaaagaat ggtctccagc 180

tatatgtctc aagcttcatg gagaggggca gggatgaatg gcagggagga agcaggaggt 240

ctcaccagca gaatccagga gctcactggg gacccatccc aattcttaaa gcatgggtaa 300

ttcagccagg cctcagcctc ttgtgccagc tgcctccaac cctttgggtc tccaccaccc 360

aagtttcctg tagggtccgc cgggtccagg atcacaggcc tgggtttcgt gagctgcctt 420

ctcaggtact tttcaataat ggggttttta aagtcataat actttgtcca gtagatgca 479

<210> 325

<211> 554

<212> DNA

<213> Homo sapiens

<400> 325
gagcgaggga gcatgaaaac acatttcaac acagactgtg gatttcggac ggtcttggca 60

ttagtcataa actaccagca actctgcatc tactggacaa agtattatga ctttaaaaac 120

cccattattg aaaagtacct gagaaggcag ctcacgaaac ccaggcctgt gatcctggac 180

ccggcggacc ctacaggaaa cttgggtggt ggagacccaa aggttcggag gcagctggca 240

caagaggctg aggcctggct gaattaccca tgctttaaga attgggatgg gtcccagtga 300

gctcctggat tctgctggtg agacctcctg cttcctccct gccattcatc cctgcccctc 360

tccatgaagc ttgagacata tagctggaga ccattctttc caaagaactt acccttccc 420

aaggccattt aaatccctaa agtcacaggc tgcgctcaaa atttacagtc attttggcca 480

caatcgaggg tctcgggaat tttcacatcc cttgtccaga tttcattccc ctaagagtat 540

aataataatc tcta 554

<210> 326

<211> 331

<212> DNA

<213> Homo sapiens

<400> 326
atttacaagt ccttttattt tgcatgttca ttgtaaattt aatactgtaa atgtattcaa 60

attcatttac atgcctatgg ttgcctttga ttaaacttct tccaaaaaat aaattctgcc 120

cagatgttgt tgactttatt atttcatgtc agattacttg aatatacgtt gctgcataat 180

cactaccaag tgggttcttt gctgtgcatt tgtatatccc agaatcggag gtttggggat 240

tctgaatgac tagggtacct tgtaagtgaa gctgctcact tccatgtgtc ctctctttac 300

ttgccgttga gagaagggag tggtcaggca t 331

<210> 327

<211> 446

<212> DNA

<213> Homo sapiens

<400> 327
tccagatttc tgtgatccca ctagccccat gttgctcaac acaaatacta ttaacttgta 60

tactatacct ctttggccct tgctggactg taaaaacctg tgactaaact atttcctctt 120

atccatttca gtgataaatt ctgcagtact tgatactgtt tagccattgc taatttgact 180

aattaagtgg actgtgacac cttttgaatc cctatagatt atttatataa aaactaactg 240

gggatcttta cttcgaatgg agaggaggaa gaatgaaaaa aattgtgttc cattactgtt 300

ttaaaaaatt agagtacacc atctttggtc tatatcaatc aaaggaaaac aagaatagtg 360

aagaatatga atttttagag tttgtcctaa ctcatgcttg tcctattaaa aagaaagtaa 420

caaagtgtta ctataagttt atatgc 446

<210> 328

<211> 457

<212> DNA

<213> Homo sapiens

<400> 328
aattcggaac gaggctattt agaaataagc taattggatc agtggcttaa ataatagctg 60
actgtgtgta catatgtata taatatgtat atacaatatc aggcatgcat gtggcttgga 120
attttgtttc ctccataaaa tgtggaagtg aattaaacaa gttttagtca tttatacaaa 180
gtcacaaata taaagttcag tttgtcacaa gattaaattg ctcacaaggt aaaattgtat 240
tgtttggcaa aatcacaagt aacaatcctg tgagttttct attatgaagg ttaataataa 300
atgggctcat ttagttgcct gggcacctat tcacaaattc atttgtcagc ctctttttag 360
ttctcttaaa aaaaaaaaaa tcatatgatc attttccttt ttgggggtac ttagcttcca 420
tgcctataaa gtctggtacc agactgactt gaaattc 457

<210> 329
<211> 494
<212> DNA
<213> Homo sapiens

<400> 329
tttcctatgc tgggaggaag cattttaatc agatggtcgg ggatggtaac actgtacagg 60
gagaactcac agctccaaga gcttccctcc aaattatgat gaagttcctg taaataacta 120
gatttcctac agcctggtcc tctgcccatg ggaaggaggg gaaatggatg tgaaaagcaa 180
tgtggtctgc cctcctgtca ttcatagatc cagttctgag cacagctcgt gtaatctacc 240
agttcctcag ggtgaaacac aagccgatct ccttctctgc actctccaca gaatcactgc 300
catgtataat gttcctgcca acttgtatgc agaagtctcc acggatggtc ccaggcttgg 360
agtctgcagg gttggtctcc ccgagcatga ctcggcccgt cttcaccaca ttcagcgcct 420
ccagaccatg gcaataccgg gcctgagtgc atgtatttca caggccggca agaatggacg 480
gtccttcagt caac 494

<210> 330
<211> 507
<212> DNA
<213> Homo sapiens

<400> 330

aattcggcac gcagggttga cctgaaggac cgtccattct ttgccggcct ggtgaaatac 60

atgcactcag ggccggtagt tgccatggtc tgggaggggc tgaatgtggt gaagacgggc 120

cgagtcatgc tcggggagac caaccctgca gactccaagc ctgggaccat ccgtggagac 180

ttctgcatac aagttggcag gaacattata catggcagtg attctgtgga gagtgcagag 240

aaggagatcg gcttgtggtt tcaccctgag gaactggtag attacacgag ctgtgctcag 300

aactggatct atgaatgaca ggagggcaga ccacattgct tttcacatcc atttcccctc 360

cttcccatgg gcagaggacc aggctgtagg aaatctagtt atttacagga acttcatcat 420

aatttggagg gaagctcttg gagctgtgag ttctccctgt acagtgttac catccccgac 480

catctgatta aaatgcttcc tcccagc 507

<210> 331

<211> 483

<212> DNA

<213> Homo sapiens

<400> 331
cacagtctgg gggccccgaa gcccctcaat ctttattcca gttcctcaga tgtggcagtt 60

cccccagctt ccctggaaac aagtatcaga caaaggtggt ggcagacaca ggacagggcc 120

ctgggtgggg cttgctggcg ctaaaaggat ttctcagcag gctcagggtc tcccgccccg 180

aatgaccctt ctggccactt caagctactc ctctgctgcc cggcttccct ctgcctctgg 240

ttcccctggc tgagcagggc cttcctgggc ttgaccctgg ccccccttcag cagttgtctc 300

agactgttcc cgaatgtccc tggggctggc tttgacccca gcatccccat ctccctggca 360

cccttgctcc tcccctctgc ttcttgcctc tgactgccca ctgccctcct cctctgtcac 420

agccccctgg gtctcaggcc actctgcccg gcggtagacg aggtagccag tggtgggcag 480

ctg 483

<210> 332

<211> 492

<212> DNA

<213> Homo sapiens

<400> 332
gcccacaccc tcccactgag ccccgtgcag gagctacggc gctccctcag cctctgggag 60

cagcgccgcc tgcctgccac ccactgcttc cagcacctcc tccgagtagc ctatcaggat 120

cccagcagtg gctgcacctc caagaccctg gccgtgcccc agaggcctcg attgccaccc 180

tgaaccagct ctgtgccacc aagttccgag tgacccagcc caacactttt ggcctcttcc 240

tgtacaagga gcagggctac caccgcctgc cccctggcgc ctggccacag gctgcccacc 300

actggctacc tcgtctaccg ccgggcagag tggcctgaga cccagggggc tgtgacagag 360

gaggagggca gtgggcagtc agaggcaaga agcagagggg aggagcaagg gtgccaggga 420

gatggggatg ctggggtcaa agccagcccc agggacattc gggaacagtc tgagacaact 480

gctgaagggg gc 492

<210> 333

<211> 440

<212> DNA

<213> Homo sapiens

<400> 333
tttaagttta tttttaacaa caaaaaaatc tgtatttttc tcctaaaagg taccaaaatt 60

tgggcaacta taaatgtttc attctatact tagaaataaa ataaatggtt tcttttttat 120

cattatctga aagaatggat tgtctggctt cttgaaatcc atggcctctt actgggacag 180

gaaaggcatt ggagttatgt ccctagacta gaaagagtat ggttcatgag gtccctggcc 240

ctctccataa ctctgcattt ggtcactaaa tattggtaga aaaagtattt gtccattact 300

ttacttgttc caccacctag agccaaagga gttagaaggt aattaatagt gatacggata 360

gaatgcaagc cacagtcacc tccatccttc tcaccatctc tcaagcccat gtccttgacc 420

tttggtattt acatccatgt 440

<210> 334

<211> 321

<212> DNA

<213> Homo sapiens

<400> 334
gatgaataac tttgacaggc tggaaaaagg tgacatttca ggtagagcgt gtcacatgga 60

tgtaaatacc aaaggtcaag gacatgggct tgagagatgg tgagaaggat ggaggtgact 120

gtggcttgca ttctatccgt atcactatta attaccttct aatgcctttg gtctaggtgg 180

tggaacaagt aaagtaatgg acaaatactt tttctaccaa tatttagtga ccaaatgcag 240

agttatggag agggccaggg acctcatgaa ccatactctt tctagtctag ggacataact 300

ccaatgcctt tcctgtccca g 321

<210> 335

<211> 241

<212> DNA

<213> Homo sapiens

<400> 335
tttaagaaaa gtaaattcat cttgctcaca gtcctttctg gaagagttta gaaagcaaag 60

aattcaccga ctcagcagga agcagaacga gctgttcctt cttttgacac gcacaagcta 120

atcccctaga gagtggggat gtgggaaacg gagggtaatt aattctttgg tcactggttc 180

aatgctgaat agccttggtc agttttggct ctctcctatt ttagggggaa aaatattttt 240

g 241

<210> 336

<211> 564

<212> DNA

<213> Homo sapiens

<400> 336
gcaggtcagc aacaagttta ttttgcagct agcaaggtaa cttggtaggg catggttaca 60

tgttcaggtc aacttccttt gtcgtggttg attggtttgt ctttattggt tgggggtagg 120

ggaaagcgac gagaagtaac atggagtggg tgctgcctcc ctgtagaacc tggttacgag 180

agcttggggc agttcacctg gtctgtgacc gtcattttct tgacatcaat gttattagaa 240

gtcaggatat tttttagaga gtccactgtt tcttgaggga gattagggtt tcttgccaag 300

atccaagcaa aatccacgtg aaaaagttgg atgatgcagg tacaggaata cacgatggca 360

tagttctcat agtcggtggc caggttccag tacggtgccg atggcataaa ccaggaaaac 420

ttaacttcca gcttggcagg ctctgtgagg ttaactgggg tggcttcacc ttcgatttga 480

ttcacagttc catcagctct caactcctgg tttaacactt tgatctttcc gttttccata 540

gtgagtagtt ggcccgtatg cagc 564

<210> 337

<211> 490

<212> DNA

<213> Homo sapiens

<400> 337
ctgattctgc atctggaaac tgccttcatc ttgaaagaaa agctccaggt cccttctcca 60

gccacccagc cccaagatgg tgatgctgct gctgctgctt tccgcactgg ctggcctctt 120

cggtgcggca gagggacaag catttcatct tgggaagtgc cccaatcctc cggtgcagga 180

gaattttgac gtgaataagt atctcggaag atggtacgaa attgagaaga tcccaacaac 240

ctttgagaat ggacgctgca tccaggccaa ctactcacta atggaaaacg gaaagatcca 300

agtgttaaac caggagttga gagctgatgg aactgtgaat caaatcgaag gtgaagccac 360

cccagttaac ctcacagagc ctgccaagct ggaagttaag ttttcctggt ttatgccatc 420

ggcaccgtac tggatcctgg ccaccgacta tgagaactat gccctcgtgt attcctgtaa 480

ctgcatcatc 490

<210> 338

<211> 394

<212> DNA

<213> Homo sapiens

<220>

<221> misc_feature

<222> (1)..(1)

<223> a, t, g, c

<220>

<221> misc_feature

<222> (4)..(4)

<223> a, t, g, c

<220>

<221> misc_feature

<222> (7)..(7)

<223> a, t, g, c

<220>

<221> misc_feature

<222> (130)..(130)

<223> a, t, g, c

<400> 338
naanaanatg ttgaccatgt agttttattt catcagtact cccattttta atggattcag 60

gcagcacccc agagtacagg actgagttcc taggggtggc ctgacccagc agctgtcttc 120

tgttccaggn ggaaaaagct ttttattaaa aaacagaaac gaaactttag aaaatatgaa 180

aaatcagagg gaggacagag gtcatttcat aaaatcgtta tttgaaaaca gtgcagtgtg 240

caaataacac tcagttgggg aatcgggaga ccgggggact agtctaactg ggctcaaact 300

aggctgtgac cttagggcgt gtcattcacc acttggggga tcctttcctc atctaaagca 360

ttcagcacag ttaccctgtt agggcacttt tacg 394

<210> 339

<211> 413

<212> DNA

<213> Homo sapiens

<220>

<221> misc_feature

<222> (33)..(33)

<223> a, t, g, c

<220>

<221> misc_feature

<222> (251)..(251)

<223> a, t, g, c

<220>

<221> misc_feature

<222> (313)..(313)

<223> a, t, g, c

<220>

<221> misc_feature

<222> (346)..(346)

<223> a, t, g, c

<220>

<221> misc_feature

<222> (400)..(400)

<223> a, t, g, c

<400> 339
atgacccttt gcctgttccg catcctggag gcngcaaagg gtgaaatccg cattgatggc 60

ctcaatgtgg cagacatcgg cctccatgac ctgcgctctc tctgaccatc atcccgcaga 120

ccccatcctg ttctcgggga ccctgcgcat gaacctggac cccttcggca gctactcaga 180

ggaggacatt tggtgggctt tggagctgtc ccacctgcac acgtttgtga gctcccagcc 240

ggcagcttgg nacttccagt gctcagaggg cggggagaat ctcagcgtgg gccagaggag 300

cttcgtgtgc ctngcccgag ccctgctccg caagagccgc atcctnggtt ttagacgagg 360

ccacagctgc cattcgacct gggagattga caacctcatn cagggttacc atc 413

<210> 340

<211> 396

<212> DNA

<213> Homo sapiens

<400> 340
tataattatt ttaacatttt tattgaatga tttcaacacc ttggtttatg ggttgttttg 60

attttgtttt tttgagacag ggtctcactc tgtcttgagg ggggaaaaaa accatgcaac 120

aatggaatac atatatttaa ataactgcac ataatacacc ctccattttt gttaccaaga 180

gaaagcttcc atcaggtagc tttaccaata catttccatt cccatgaaat gcaataccac 240

agtactaata ttctattcag aaaaagacta caattccctt gtaactcttt ttctttttcc 300

cttgtaactc tgagttcttt cttttaatcg ttcaggccac ataggtaagg ccattttaga 360

aaaggactgc tttcaagtat ctaaatatac ttccgg 396

<210> 341

<211> 299

<212> DNA

<213> Homo sapiens

<400> 341
tgagttttca tctgattgtg gggaccaagt cctgagtaga gggccaagag ctagggacag 60

ggggaagaga ctggcccagg tggtagggag gaaagaactc ccagagtttc ctttagccag 120

gaaacctgct ctactgaccc cgtgacttgg acagtcaagc atcaccctga gagtgacaag 180

tgtaaaatga ctcccttcct cccccgccct ccggaagtat atttagatac ttgaaagcag 240

tccttttcta aaatggcctt acctatgtgg cctgaacgat taaaagaaag aactcagag 299

<210> 342

<211> 430

<212> DNA

<213> Homo sapiens

<400> 342
cctgtgttat cgatttattg cagctccaat atgagtccac tcctactcaa accctgattc 60

tcaggggcct ggggaaggca ccccactagg gccctgtccc caccagagcc tggacatggg 120

actctctccc aagcaggggt gttgtccttc acaggggctt ccacgtctct cccgggcctg 180

tccgcactct gggcagggct ggcacctggc tattccctca atctgggccc tgggcactgg 240

caatgtggaa tggtccaggt gttcatgtca gtggggcttg gggacatggc catccacgta 300

gaagttcctg gtgatcttgg gcagggtgaa ttccgctcct tccagctccg gtgtcagcac 360

aatctggcag cccagccgcg agttcctcct ggaggaaggg gggcatgttt agcaagtcgt 420

cttcctcttc 430

<210> 343

<211> 447

<212> DNA

<213> Homo sapiens

<400> 343
gagcggcctg tgaagcctcc ctggcctgct ccacctgcca tgtgtatgtg agtgaagacc 60

acctggatct cctgcctcct cccgaggaga gggaagacga catgctagac atggcccccc 120

tcctccagga gaactcgcgg ctgggctgcc agattgtgct gacaccggag ctggaaggag 180

cggaattcac cctgcccaag atcaccagga acttctacgt ggatggccat gtccccaagc 240

cccactgaca tgaacacctg gaccattcca cattgccatg gcccaggcca gattgaggga 300

atagccaggt gccagccctg cccagagtgc ggacaggccc gggagagacg tggaagcccc 360

tgtgaaggac aacacccctg cttgggagag agtccatgtc caggctctgg tggggacagg 420

gcccctagtg gggtggcctt ccccagg 447

<210> 344

<211> 284

<212> DNA

<213> Homo sapiens

<400> 344
tttttttttc atgctttgtt tcactttatt ggatacaccg ggtgtgggat ttacaaatag 60

gaccaacaat gtgtgcgggg ccagacggct gcctgtgggt ctgtgtgtaa caggggctgt 120

aaaatgaatg gacctcatgt gcacgtggta cagagagaag caaaccaaga ccaactggat 180

cccaacgtgg gtaacataaa cagttaaatt actaaagtca tcttaagacc aaaatacttc 240

aatgaaaatg gcatattaaa aacatataaa ttacatttaa gctc 284

<210> 345

<211> 319

<212> DNA

<213> Homo sapiens

<400> 345
tttttttgca gccaaataca gtttattttt ttctctctca gctgaggagc agacacattt 60

acggagatgc aaatgaccat ccagtggcct acagtggagg gggggtggag ggagtagggg 120

attagggggt tggtagggag tgttggggga gggaggagca cagggctggc tcagggccag 180

gatctgtgaa cggggcaggg ctctgtcaac atttacccag tccatcccca gagagggctg 240

caaaagggga cggcgataga aaagtagatg gaatactttg ggatggggga caacttacat 300

gcaagaaaag gagggaact 319

<210> 346

<211> 355

<212> DNA

<213> Homo sapiens

<220>

<221> misc_feature

<222> (1)..(1)

<223> a, t, g, c

<220>

<221> misc_feature

<222> (270)..(270)

<223> a, t, g, c

<220>

<221> misc_feature

<222> (329)..(329)

<223> a, t, g, c

<400> 346
ntctttattc atacttgttg acatttccca tggggaatgg ttgaagggag gggcagggag 60

ggctgagcgg gaagccacgg cttggttttc ctccatccag tggagaagag actcggtagg 120

gacatgctaa gtagagtgaa caggggaacc atcactatgt gggctgagat gcactgtgca 180

gtgacttcag acaacctgag tccttctgtc aggaaatgca atttctaagt ggaaaaaaaa 240

aacaagaccc aaagaaaaaa acaaagtgcn tacaaagcaa actgctaatg taagaaaggt 300

tctaagggca gccagcagtt gaaaagccng gggtctggg agtgggccac ctggg 355

<210> 347

<211> 485

<212> DNA

<213> Homo sapiens

<400> 347
ggctgactgt actcctccca ggcgcccctt ccccctccaa tcccaccaac cctcagagcc 60

acccctaaag agatcctttg atattttcaa cgcagccctg ctttgggctg ccctggtgct 120

gccacacttc aggctcttct cctttcacaa ccttctgtgg ctcacagaac ccttggagcc 180

aatggagact gtctcaagag ggcactggtg gcccgacagc ctggcacagg gcagtgggac 240

agggcatggc caggtggcac ttccagaccc ctgggctttt cactgctggc tgccttagaa 300

cctttcttac attagcagtt tgctttgtat gcactttgtt tttttctttg ggtcttgtt 360

ttttttttcc acttagaaat ttgcatttcc tgacagaagg attcaggttg ttctgaagtc 420

aattgcaaca gtgcattttc aagcccacat tagtgatggg ttcccctgtt tcaattttaa 480

ttttt 485

<210> 348

<211> 315

<212> DNA

<213> Homo sapiens

<220>

<221> misc_feature

<222> (4)..(4)

<223> a, t, g, c

<220>

<221> misc_feature

<222> (268)..(268)

<223> a, t, g, c

<220>

<221> misc_feature

<222> (284)..(284)

<223> a, t, g, c

<220>

<221> misc_feature

<222> (297)..(297)

<223> a, t, g, c

<400> 348
ttanataaac agctttattt gccttgtaca gcatcaattt tcttacattc tcagttaatt 60

ggccattaaa gtgctggaaa ttttcttaat catgataaca tttgttaaaa agaaatcaga 120

actaatatca ggaacatggc ggcatgaagg aaacagttcc cttacaaaac acagaaaatg 180

gaagcccctc atgttgaggg gggtggggtt ggacaatttg caaacagatt ctaatttcct 240

ctcaccgtca gcaccaaact ggctgggncc accacccctg gggngaaagg gaaccancac 300

taagggaccc ctaaa 315

<210> 349

<211> 372

<212> DNA

<213> Homo sapiens

<220>

<221> misc_feature

<222> (283)..(283)

<223> a, t, g, c

<220>

<221> misc_feature

<222> (293)..(293)

<223> a, t, g, c

<400> 349
agcaaacgcc cacagatggc ccagaggtgg tggtagtcag ggtgtgtggg tgtttttagg 60

gttctttagt gttgtttctt tcacccaggg gtggtggtcc cagccagttt ggtgctgacg 120

gtgagaggaa attagaatct gtttgcaaat tgtccaaccc accccctcaa catgaggggc 180

ttccattttc tgtgttttgt aagggaactg tttccttcat gccgccatgt tcctgatatt 240

agttctgatt tctttttaac aatgttatca tgattaagga aantttccag cantttaatg 300

ggccaattaa ctgaggatgt aagaaaattg gatgctgtac aaggcaatta aagctgttta 360

tttaaccttt aa 372

<210> 350

<211> 237

<212> DNA

<213> Homo sapiens

<220>

<221> misc_feature

<222> (156)..(156)

<223> a, t, g, c

<220>

<221> misc_feature

<222> (163)..(163)

<223> a, t, g, c

<220>

<221> misc_feature

<222> (189)..(189)

<223> a, t, g, c

<220>

<221> misc_feature

<222> (207)..(207)

<223> a, t, g, c

<400> 350
ttttttttta caattttatt tataacaata tctgtgttat ttagttgtaa aggaattcag 60

caaaaattat taaaacgttc acgtccccaa aacggggctg cccgctttcc cttcctgggt 120

gtggcccacc tccttggcgt ccccagggga ggggangctg gangaatgtt taccagagga 180

agtctccanc cctggggaac ctcggtnggg gccctgggag acagggaggg cctttct 237

<210> 351

<211> 370

<212> DNA

<213> Homo sapiens

<220>

<221> misc_feature

<222> (95)..(95)

<223> a, t, g, c

<220>

<221> misc_feature

<222> (335)..(335)

<223> a, t, g, c

<400> 351
ctccctgcct gctccttggc acccccatgc tgccttcagg gagacaggca gggagggctt 60

ggggctgcac ctcctaccct cccaccagaa cgtancccac tgggagagct ggtggtgcag 120

ccttcccctc cctgtataag acactttgcc aaggctctcc cctctcgccc catccctgct 180

tgcccgctcc cacagcttcc tgagggctaa ttctgggaag ggagagttct ttgctgcccc 240

tgtctggaag acgtgggctc tgggtgaggt agggcgggaa aggatgggag tgttttagtt 300

cttgggggga ggccacccca aaccccagcc ccaantccag gggggcacct atggagatgg 360

gccatggttg 370

<210> 352

<211> 385

<212> DNA

<213> Homo sapiens

<220>

<221> misc_feature

<222> (368)..(368)

<223> a, t, g, c

<400> 352
tttttttttt agattttaga cactctcctg ccttattcta gggtcaagtg ggcccttaac 60

aggaggtctt gatttttcaa aaacatgatt cggaacatta ctcctcttca ttgtcatctg 120

cttgcttcac ccaaagttct tggtggctct tgctaagcca gaacatgaac catccattgt 180

gtctcctcca gccttctctc catagccgtg ggaggcctgt ctggaaacat gtgagtcatt 240

cgtgctgtcc ccttagggcag ttcaaactct acggttcgtg ggaacgaggg ccctctgtgg 300

ctgatggggg cctctgctgc ttgggtttcc ttgtggctcc tttgcccggt tgaaggtctt 360

ccaacctncc tcttcctccg tggaa 385

<210> 353

<211> 488

<212> DNA

<213> Homo sapiens

<220>

<221> misc_feature

<222> (391)..(391)

<223> a, t, g, c

<220>

<221> misc_feature

<222> (476)..(476)

<223> a, t, g, c

<220>

<221> misc_feature

<222> (481)..(481)

<223> a, t, g, c

<400> 353
atcgagacag caccatgaat gtgtcccaga acctctacga gctcctcccc aggacttcgc 60

cactgaagaa taagcacttt gggacttgtg ccatcgtggg caactcgggg gtcttgctga 120

acagcggctg tgggcaggag attgacgccc acagcttcgt catcaggtgc aacctggtcc 180

ccagtacagg agtatgcccg ggatgtgggg ctcaagacag acctggtaac catgaacccc 240

tcggtcatcc agcgggcctt tgaggacttg gtcaatgcca cgtggcgggg agaagcttgc 300

ttgcaacggc ttgcacagct tcaatgggca gcatcctgtg ggatccctgc ctttcatggg 360

cccggggcgg gcaagggagc gtgtttgagt ngggttcaac gagtttattc cttgaaaggc 420

accacgttca aacttgcggc atttgcatta acccctttcg ttggcggctt gtttgnaacg 480

ncttttcg 488

<210> 354

<211> 528

<212> DNA

<213> Homo sapiens

<400> 354
aggctcgttt atttattcat tgatcaactg gcacttcttg aaagcctgct gtgtgccaag 60

cctttcccca aaggaggata tcagtggtgg agccaagtct cagggtggaa aggacctgga 120

ccacacagag caggactcca gagcctcctc catatggcag gaatcaagct ttcacagggg 180

aaacgcagga tttcccacac atgcccatgc aacacttcaa gtcacgcttg cactggccat 240

ccatctcaca gaaattgggg gggttaagca tcaaacattg gccataagtc actgggcact 300

tcccaggctt cctccttgtt gggtttgggg tgtcaacagg atccaggcat ttgatgccac 360

aagtgtcagg acaacatctc ttcttccctg gacactgcca gtcactctgg cactcaggtt 420

tcttgtatct aaggcactgg gcagatttct taggaggaca gactccagct ttgaaggact 480

ttccagagcc ttccacagcc caaggtgcca gagttcccag ggcaagca 528

<210> 355

<211> 440

<212> DNA

<213> Homo sapiens

<400> 355
ttcagtttta aatatttatt taaaatccag aggggaaaag gagaaacgga acccattggg 60

gttttaatac actgacatgt ggacagagac gtaaacgaag acagcaggaa aacccaagaa 120

tgagacagag gccagtggat tctggcagca ggagggatcc gagcgctgag atgaggcccg 180

agctgctaca aacacgcact tccacgcaga gcgtccaggc tggggcggca gggcgaggat 240

acagaagtgt tgggaggggg gacgggccaa agtgaggtat taaataataa aaatcaaatc 300

caattcccaa agagacacaa ctttaggaga gaatacacaa atagagactt tcacatacat 360

tttccccttc tatagagata gttccatggt taaaataact caaaatccaa ttcaagcgcc 420

gacttgttcg ctgatgtagc 440

<210> 356

<211> 415

<212> DNA

<213> Homo sapiens

<400> 356
gaaggcagag aggaaggttt aatgagccct gtccaggcgc cttcagtggg gagcctcctt 60

cttcttgccc ttctccttct tgcccttctc cttcttcttc actttgggct tcttggcctt 120

gcccgggatg ctctcgtgct gcttggagcc agcagcgtgg gacttcacat ccgtgtccga 180

atcgctggag ctgctgctgg agtcggaaga gctgtggtgt ccttgctgga tggaggtgcg 240

gcagtgaggc ggcgtccctt acccagcccc ctgaagttgg aggcctttgg acccggacct 300

gcccctgcct ccgaccggcc ctgaactttg tggggactga gcttgggatc tccccgtggc 360

acgccccaca ccgggcttct gggaggtggg ctcagggctg ctccaggtcg aactc 415

<210> 357

<211> 394

<212> DNA

<213> Homo sapiens

<220>

<221> misc_feature

<222> (22)..(22)

<223> a, t, g, c

<220>

<221> misc_feature

<222> (335)..(335)

<223> a, t, g, c

<220>

<221> misc_feature

<222> (365)..(365)

<223> a, t, g, c

<220>

<221> misc_feature

<222> (377)..(377)

<223> a, t, g, c

<220>

<221> misc_feature

<222> (383)..(383)

<223> a, t, g, c

<220>

<221> misc_feature

<222> (391)..(391)

<223> a, t, g, c

<400> 357
tttttgttc atttatattt tntttaagag ctgtgcccag ttttatcatc tcacaagaat 60

gaagcaaggg acaaaggtaa gtgccacgct ccctggccac tgggttcctg gcaagctccc 120

agccactagg tgccaatctc ccttcaatgt actccttctt ccccagagtg cagaagcgta 180

tgaagacagt tatgacatgg acacatgcat gagctattat acataattac aaaagctgat 240

tctgtcatca ccacatcttg tctcatcagt aggagcgaat ggctggcggg acggtggcac 300

agtcagcctt gttcaaagtt ttgtcgatca cgggncctat attccagagt gacctttccc 360

agtgnccaac gttccanata ggncagggtc ntgc 394

<210> 358

<211> 293

<212> DNA

<213> Homo sapiens

<220>

<221> misc_feature

<222> (55)..(55)

<223> a, t, g, c

<220>

<221> misc_feature

<222> (80)..(80)

<223> a, t, g, c

<220>

<221> misc_feature

<222> (109)..(109)

<223> a, t, g, c

<220>

<221> misc_feature

<222> (122)..(122)

<223> a, t, g, c

<220>

<221> misc_feature

<222> (169)..(169)

<223> a, t, g, c

<220>

<221> misc_feature

<222> (183)..(183)

<223> a, t, g, c

<220>

<221> misc_feature

<222> (186)..(186)

<223> a, t, g, c

<220>

<221> misc_feature

<222> (201)..(201)

<223> a, t, g, c

<220>

<221> misc_feature

<222> (210)..(210)

<223> a, t, g, c

<220>

<221> misc_feature

<222> (219)..(219)

<223> a, t, g, c

<220>

<221> misc_feature

<222> (222)..(222)

<223> a, t, g, c

<220>

<221> misc_feature

<222> (230)..(230)

<223> a, t, g, c

<220>

<221> misc_feature

<222> (239)..(239)

<223> a, t, g, c

<220>

<221> misc_feature

<222> (247)..(247)

<223> a, t, g, c

<220>

<221> misc_feature

<222> (262)..(262)

<223> a, t, g, c

<220>

<221> misc_feature

<222> (265)..(265)

<223> a, t, g, c

<220>

<221> misc_feature

<222> (269)..(269)

<223> a, t, g, c

<220>

<221> misc_feature

<222> (272)..(272)

<223> a, t, g, c

<220>

<221> misc_feature

<222> (274)..(274)

<223> a, t, g, c

<220>

<221> misc_feature

<222> (275)..(275)

<223> a, t, g, c

<220>

<221> misc_feature

<222> (277)..(277)

<223> a, t, g, c

<220>

<221> misc_feature

<222> (282)..(282)

<223> a, t, g, c

<400> 358
ttttccagga tttaatatgg tgcttttaag aagagagcca ccggtctcag cttanaatta 60

cattttcaca aattaatccn aaattttacg tatgaataaa aaggagtana acaatacata 120

gnaaatgaaa ttggagaact gatttaatac taaagttctg aataaaggng tgcactttat 180

gantgnttct atctttttgc ncaagttggn tactccagnt tnccatcccn acatgttgnt 240

cgccgangtg tgagaacgtg gntgnaagnc gntnntnccc gnttgaacgc aaa 293

<210> 359

<211> 500

<212> DNA

<213> Homo sapiens

<220>

<221> misc_feature

<222> (1)..(1)

<223> a, t, g, c

<220>

<221> misc_feature

<222> (299)..(299)

<223> a, t, g, c

<220>

<221> misc_feature

<222> (381)..(381)

<223> a, t, g, c

<220>

<221> misc_feature

<222> (390)..(390)

<223> a, t, g, c

<220>

<221> misc_feature

<222> (446)..(446)

<223> a, t, g, c

<220>

<221> misc_feature

<222> (480)..(480)

<223> a, t, g, c

<400> 359

nttcagttga gtaccaatgc cagaacttgt atcaacttga gggtaacaag cgaataacat 60

gtagaaatgg acaatggtca gaaccaccaa aatgcttaca tccgtgtgta atatcccgag 120

aaattatgga aaattataac atagcattaa ggtggacagc caaacagaag ctttattcga 180

gaacaggtga atcagttgaa tttgtgtgta aacggggata tcgtctttca tcacgttctc 240

acaccattgc gaacaacatg ttgggatggg aaactggagt atccaacttg tgcaaaaang 300

atagaatcaa tcataaagtg cacaccttta ttcagaactt taagtattaa atcagttctc 360

aatttcattt tttaatgtaa ntgttttacn cccttttat tccatacgta aaattttgga 420

ttaattttgg gaaaaatgta attatnagct gagaccggtg ggtcctcttc ttaaaaggcn 480

ccatattaaa atcccgggaa 500

<210> 360

<211> 284

<212> DNA

<213> Homo sapiens

<400> 360
gcggccgcct gcgcgcacag ccccagtggt cggatcctgg gcggcagaga ggccgaggcg 60

cacgcgcggc cttacagtgg cgtcggtgca gctgaacggc gcgcacctgt gcgcggcgtc 120

actggtggcg gacgagtggg tgctgagcgc ggcgcactgc ctggaggacg cggccgacgg 180

gaaggtgcag gttctcctgg gcgcgcactc cctgtcgcag ccggagccct ccaagcgcct 240

gtacgacgtg ctccgcgcag tgccccaccc ggacagccag cccg 284

<210> 361

<211> 410

<212> DNA

<213> Homo sapiens

<400> 361
cagctaattt tttatttatt tgtagagatg gagtcgcgct acgtggccca tgctgatctc 60

gaactcctga gatccaatga tcctcccacc tcagcctccc cttctgcata tagtaggtgc 120

tcaataaaga ccaaccagat gcaggagtgg atgacttcat tgctcgggac tttgttgctt 180

gggtgaccct gaccttcagg ccccggcacc ctaggccagg acgctgtcga tccaggccgc 240

atagctcgcc acgcgggtgt agatcccggg cttcttgcgg ttgccgaaac gcgcgacccg 300

aggtgaccac gccctcgagc acgcccccgc acaccagcgg tcccccggag tcacccttgc 360

agctgtcccg gcgattgctc tccgcgcaca tcaagcgctc ggtgatggcg 410

<210> 362

<211> 393

<212> DNA

<213> Homo sapiens

<400> 362
aatttttccc aacattttat tattaaaaaa agttcaagaa aacagaaaag ttcaaagaat 60

tgtacagttt acattcatct gcccaaaatc tagaccgtat aattaatttt ttattgtatt 120

tgctttatca catcacgtat caatcattct tatttattga tggatttcag agtaagttgc 180

aaaaatgcat acatttcacc cctaaacatt tcagcatgca tatcctcaac ttagagtcta 240

atatttgttt atagctcctt ttttgaggta aaatgtacat acaatgaaat gtatataact 300

taagtgtcca atttgatgag tttggcaaat gcatacactt gtgtaaccca aattcctatt 360

gtaatataga acatgaccat taccttagaa act 393

<210> 363

<211> 296

<212> DNA

<213> Homo sapiens

<400> 363
aaacccaaat aaataatttt attaaagaag ggtggattaa gtaaaacagt cagtaggaat 60

aaatgaacaa accagaccaa aaaaaccccc agaactcaat tatatttgtt tataagaggg 120

ccctgagata caaagacact caaaatataa aaacacagga agagaaaagc aaacagatga 180

aaataatgta ctctataaac actaaccgaa agaaaactaa taacatgaat aaccaattaa 240

ccaaccaacc taactgatac atatacctaa taaatataga atacgcagag acaatt 296

<210> 364

<211> 464

<212> DNA

<213> Homo sapiens

<400> 364
attcggaacg aggagagaga ctcatatatt atgtaaataa tggaacaaac aaacagattt 60

tttaaaaatc ataattaaga tgttgaaatg tagaaaagat gaacaaagtg taagaaaaaa 120

tagtacccac agaaatatct atataaaaaa ttaaatggac atcttatacc taaagaatac 180

agtattggga ataactcatt gcatgggttt gagggcagac aagagccaac agaaaggagt 240

aataaacttg atgaaaaaat ggaaaatatc cagaataaag tacagtgaga aaatacaaaa 300

aaaatttaga gcattagaga taagagatag agcctgtaat ccaggctgaa gtgcagtggt 360

gtgatatagc tcactgcaac ctcgacctgg gctcaagcga tcctcttgag tagctagggc 420

tacaggcaca caccatgctc aactaatttg tttttgtaga gatg 464

<210> 365

<211> 475

<212> DNA

<213> Homo sapiens

<400> 365
gccaggcagg accacaggtt tattggggac tccacgcaca gacgcttatg gcatcacacg 60

acaacggcac ggttactcgg gacacacacg gtggcctctg cccacagcca gggcccagag 120

gcagtggggt gcagtctcct cccttgtggc ccagacccag ctgggtccct tcctcctagg 180

cagctgaggg aaggactgct gggttggcca cgggcctggg aaggggaagc gagcaggcga 240

gtccaggagg ggccggggcg ggtgtggggc tcgcctgccc tcactccagg gactgtagca 300

tcagcagctg tttcagcacc ttcgtctggc tggtctctcg gatttcgcca gccaggaaca 360

tctcgtccac gaccgtgtaa accttgtaga agttgaacac caagtccagt tcacagacat 420

tgtggaaata ttcgcttaag acctccacga aattttgaaa tggctccacg taacc 475

<210> 366

<211> 435

<212> DNA

<213> Homo sapiens

<400> 366

gtccttggtc atgtcaggag ggacaggacg ttgtatcaat atgtctggtt cctcattaaa 60

tttgcaacta ctcttttagc ctttattgtt tattttctga ctctgttttt ttttttcttt 120

agccaactca cgccatgact ctacttttgc ttctatgttt attagttggc attctgtact 180

aagacagctt tacccttctt gcctacttat ttatgtcagt atgagctcag aagttcttat 240

tcaggggctt ataatacagt actcttatta ttttatggtt acacagtccc aactttgtcc 300

tgtgggaacc ctttcaggct ggctcttatg ttctggtgac ataatctcat ccttctttaa 360

gtactttctt tctggtatta acaatatgct ccagactcat tcttcccgag ccccagcccc 420

agaatcagct ttttc 435

<210> 367

<211> 321

<212> DNA

<213> Homo sapiens

<220>

<221> misc_feature

<222> (156)..(156)

<223> a, t, g, c

<400> 367
ggcgtagaga aaatatagag ccactttact attaaaaatg tattttccaa aagcaaggta 60

gttgctgtcc caaaaagccg aaagcctcta gtccctgcgg aacgggctgg atggggcttc 120

agtctgacac agccaggcgg ggcagccctc ggcggngctc ggattctctg ggagatttga 180

tagagctcca tcgttgccct cgcatcttcc accgagctgt gtccaagcag gctgttctgg 240

atgctcttgt gcaggaggcg ctcactcagc acccgcagga gacacgcctg cagtggtcca 300

gcttggcctc acgccacaac a 321

<210> 368

<211> 337

<212> DNA

<213> Homo sapiens

<400> 368
ggctacacaa tctacgacac gtccactgac aggctgttgt ggcgtgaggc caagctggac 60

cactgcaggc gtgtctccct gcgggtgctg agtgagcgcc tcctgcacaa gagcatccag 120

aacagcctgc ttggacacag ctcggtggaa gatgcgaggg caacgatgga gctctatcaa 180

atctcccaga gaatccgagc ccgccgaggg tgccccgcct ggctgtgtca gactgaagcc 240

ccatccagcc cgttccgcag ggactagagg ctttcggctt tttgggacag caactacctt 300

gcttttggaa aatacatttt taatagtaaa gtggctc 337

<210> 369

<211> 410

<212> DNA

<213> Homo sapiens

<400> 369
ttttttagga taaacttgcc aggctttaga tctatttgtt cctttccatc ccccaaaatc 60

aaatggctgc tccaggaaaa tgttcccctt gtggcagggt ccgggagaaa agagagaagc 120

gacaaaacca aaaattaaaa cgaccgaagt cccatatgct ccaggaatat gtcctggaga 180

tgggagtgga gggcaggggg agaatgttgt tgaggtcaaa atttttgaag ttttaagtcc 240

tatatcttga catcccgagt ataaatgcgg gtaccagaca cagtacaaac gttctcaaag 300

cccagttacg tattccaaac caaacgcggg ctcttgaagg gtgatgaggt agggatgaaa 360

tccaggatcg cctgaagacc atttcttcct ctcttaggga cctgctggtc 410

<210> 370

<211> 434

<212> DNA

<213> Homo sapiens

<400> 370
attcggcacc ggggaggttt ttcctcctgg accgaatcag ctggagacca caggtcctaa 60

gagaggaaga aatggtcttc aggcgatcct ggatttcatc cctacctcat cacccttcaa 120

gagcccgcgt ttggtttgga atacgtaact gggctttgag aacgtttgta ctgtgtctgg 180

tacccgcatt tatactcggg atgtcaagat ataggactta aaacttcaaa aattttgacc 240

tcaacaacat tctcccccctg ccctccactc ccatctccag gacatattcc tggagcatat 300

gggacttcgg tcgttttaat ttttggtttt gtcgcttctc tcttttctcc cggaccctgc 360

cacaagggga acattttcct ggagcagcca tttgattttg ggggatggaa aggaacaaat 420

agatctaaag cctg 434

<210> 371

<211> 519

<212> DNA

<213> Homo sapiens

<400> 371
ttttaaatgt aaattgttta ttttagaggc cattagttgc ttttctgtga agcaacaaga 60

aatcttactg accaggctaa ttttttaatg ttatgctaat gttgtatccc ttccatattc 120

tgtgtttcat ggaatatcaa gatgccaaac aaccagataa aactgacaac ataaaccaca 180

acatcagagc tagcaagaga tgggagaata aaatggcttg aattactagg atgtagaact 240

aaatgcttgg aatgaagcta gtcttcctat ctttaatttt ataaaatccc ctgaacacta 300

agaatggatt tataacttaa gacaatgccc tggatggaaa atggataagg atgatttcat 360

accccgagtg cctggtgggc aagagcactt gaaatggttc acaaggtcaa tacaggtgcc 420

tccattctgg cagggctgat tctggcactc atccacttca tactcacagt tgacaccctg 480

atagcctggg acacactcgc atctgtaatc accaatgaa 519

<210> 372

<211> 205

<212> DNA

<213> Homo sapiens

<220>

<221> misc_feature

<222> (64)..(64)

<223> a, t, g, c

<220>

<221> misc_feature

<222> (171)..(171)

<223> a, t, g, c

<400> 372
tttttttttt tcacttttcg ttattttat tgtttgacaa gcatttacaa cgttccattc 60

atanacctaa aaacataaaa atagaccttc tttcagctta taaaagaaat atataagaac 120

ttttgacata gacacgtcat gaccttatgt acaagagaca atggcaccct ntccaagcac 180

cagacttccg agtgttttca gatgg 205

<210> 373

<211> 488

<212> DNA

<213> Homo sapiens

<220>

<221> misc_feature

<222> (412)..(412)

<223> a, t, g, c

<220>

<221> misc_feature

<222> (417)..(417)

<223> a, t, g, c

<220>

<221> misc_feature

<222> (449)..(449)

<223> a, t, g, c

<220>

<221> misc_feature

<222> (459)..(459)

<223> a, t, g, c

<220>

<221> misc_feature

<222> (466)..(466)

<223> a, t, g, c

<400> 373
aatgaaatgt gtcagcttcc ggtgttctga ctgtaccctt ccctcttcca tgtctgagaa 60

tctccgtgta ttttaagaat gtgtgaggag agggtggcga ttcatgtttc aatgagcctc 120

tttttttttt ttccttcctg ttttggtcta tggctggtct tactctgtgt ccatgttcgg 180

aagctctagt tttgcataga attatagaga tgccaaactc tttgaaaaga gatccaaatt 240

tatcgcttga gagaaagaaa agaaacacta ttttttgtat tttacctgag atacaggggc 300

acaaatagga tgaggaattt tacagtgtta gtgtatgtat ccctgagcct aaaaaatgag 360

ggatataacc ttttacagag gaggagtgag ggcgtgggtg ggtttttata tnttatnata 420

tggaaagggc ccagccaagc ttcatgcgna agggatatna cttttncttc ccaaaaagcg 480

gggttttt 488

<210> 374

<211> 320

<212> DNA

<213> Homo sapiens

<220>

<221> misc_feature

<222> (28)..(28)

<223> a, t, g, c

<220>

<221> misc_feature

<222> (31)..(31)

<223> a, t, g, c

<220>

<221> misc_feature

<222> (188)..(188)

<223> a, t, g, c

<220>

<221> misc_feature

<222> (212)..(212)

<223> a, t, g, c

<220>

<221> misc_feature

<222> (223)..(223)

<223> a, t, g, c

<220>

<221> misc_feature

<222> (237)..(237)

<223> a, t, g, c

<220>

<221> misc_feature

<222> (248)..(248)

<223> a, t, g, c

<220>

<221> misc_feature

<222> (274)..(274)

<223> a, t, g, c

<220>

<221> misc_feature

<222> (304)..(304)

<223> a, t, g, c

<400> 374
ccgttacaaa tagcaccgag cccttggntg ncccctgtgg agagggatcc ccctcctgga 60

cccccacctc ctcccctgag ctacccatgt agtgtctggg agctggcaca cggcccaggg 120

tccctctgga agctgggcca ggccaagggg ttaggcccag gcccttcgtg gggtgggccc 180

agggcagnct gtggcagatg tgcaggtagg tnagcggcag ctntcagatg agcacantgg 240

ccacgggnac gcgggtggag cggccccttg gganccccaca atccggtcgt ttgtgggacc 300

ctgnttcgtg cagcaggccc 320

<210> 375

<211> 377

<212> DNA

<213> Homo sapiens

<220>

<221> misc_feature

<222> (41)..(41)

<223> a, t, g, c

<220>

<221> misc_feature

<222> (259)..(259)

<223> a, t, g, c

<220>

<221> misc_feature

<222> (263)..(263)

<223> a, t, g, c

<220>

<221> misc_feature

<222> (296)..(296)

<223> a, t, g, c

<220>

<221> misc_feature

<222> (374)..(374)

<223> a, t, g, c

<400> 375

```
ggagagaccc tcctgtccat gaactgggcc atcgtggcga ncattctgct gtacgtggtg      60
atccctaccc gacgctccac cgccgaggcc ttccagatcg tgctgtccca cctgctgggt     120
gatgctggga gcccctacct cattggcctg atctctgacc gcctgcgccg gaactggccc     180
ccctccttct tgtccgagtt ccgggctctg cagttctcgc tcatgctctg cgcgtttgtt     240
ggggcactgg gcggcgcant ttnctgggca ccgcattctt cattgaggcc gaccgncggc     300
ggcacagctg gcacgtgcag ggcctgctgc agtaagcagg gttccacaga cgtaccggat     360
tgtggtgccc cagnggg                                                    377
```

<210> 376

<211> 306

<212> DNA

<213> Homo sapiens

<220>

<221> misc_feature

<222> (200)..(200)

<223> a, t, g, c

<220>

<221> misc_feature

<222> (261)..(261)

<223> a, t, g, c

<220>

<221> misc_feature

<222> (269)..(269)

<223> a, t, g, c

<220>

<221> misc_feature

<222> (287)..(287)

<223> a, t, g, c

<220>

<221> misc_feature

<222> (289)..(289)

<223> a, t, g, c

<220>

<221> misc_feature

<222> (292)..(292)

<223> a, t, g, c

<220>

<221> misc_feature

<222> (295)..(295)

<223> a, t, g, c

<220>

<221> misc_feature

<222> (302)..(302)

<223> a, t, g, c

<400> 376

```
gctgccaaaa gcctttaata tgccctggtc ccaggctgtg ttcatgaaag cggacacagc      60
agtgcttcca gcttcatggt tcccaggttc aggttcctcc cagcggaggt gggagggcag     120
ccctcacacc tggcacccct gagtgcatac tcctggagga agtcgttgag ctgggcacag     180
gctgcccgct ggcggttgcn tccggcacag gcgttcagag ggcatctcct cgatccagct     240
attcgagtcc agcaagtact nggggggggnc cctcccaggg gcataantng gnccntccag     300
anccat                                                                306
```

<210> 377

<211> 449

<212> DNA

<213> Homo sapiens

<220>

<221> misc_feature

<222> (2)..(2)

<223> a, t, g, c

<220>

<221> misc_feature

<222> (297)..(297)

<223> a, t, g, c

<220>

<221> misc_feature

<222> (432)..(432)

<223> a, t, g, c

<220>

<221> misc_feature

<222> (446)..(446)

<223> a, t, g, c

<400> 377
antagaagtg acagtcaaag gccacgtcga gtacacgatg gaagcaaacg aggactatga 60

ggactatgag tacgatgagc ttccagccaa ggatgaccca gatgcccctc tgcagcccgt 120

gacacccctg cagctgtttg agggtcggag gaaccgccgc agagggaggc gcccaaggtg 180

gtggaggagc aggagtccag ggtgcactac accgtgtgca tctggcggaa cggcaaggtg 240

gggctgtctg gcatggcatc gcggacgtca ccctcctgag tggattccac gccctgntgc 300

ttgaccttgg agaagctgac ctccctctct gaccgttacg tgagtcactt ttgagacccg 360

aaggggcccc aacgttcttg cttgtaattt tgaacttcgg tccccaactt ccccgggaag 420

ttgccttggg cnttttaagg cttgtncaa 449

<210> 378

<211> 420

<212> DNA

<213> Homo sapiens

<400> 378
acaaaaataa atttttatta aatcagtaga gtctgagcag gaagacagta caaagaatgt 60

aaacaatgta aacatcacta cattcagctc agcctgattg aatgctgaaa gacaactcta 120

aatgctctac gtgcccttac tagcaagata cattaattct attttacaca gacaacaaac 180

acactctaat tttgattagg caaaagtatt ttatcgaagt cgatcccagc gccaaatact 240

ggcatcatca caaacagcta taagaatgct gctatccctg ctaaaactgg tttgtcgaat 300

agcagcacca catttatgat gagtcagtgt tgtacatttg gctttatgag gatcttctac 360

ttctaaatcc caaacataaa gtttgccaac ttggatgccc aatgcaagca tcttttgcca 420

<210> 379

<211> 430

<212> DNA

<213> Homo sapiens

<400> 379
actattcttg ggcgatttga ttacagccag tgtgacattt ggtacatgag cgttttctat 60

ggatttctgg caaaagatgc ttgcattggg caatcaagtt ggcaaacttt atgtttggga 120

tttagaagta gaagatcctc ataaagccaa atgtacaaca ctgactcatc ataaatgtgg 180

tgctgctatt cgacaaacca gttttagcag ggatagcagc attcttatag ctgtttgtga 240

tgatgccagt atttggcgct gggatcgact tcgataaaat acttttgcct aatcaaaatt 300

agagtgtgtt tgttgtctgt gtaaaataga attaatgtat cttgctagta agggcacgta 360

gagcatttag agttgtcttt cagcattcaa tcaggctgag ctgaatgtag tgatgtttac 420

attgtttaca 430

<210> 380

<211> 371

<212> DNA

<213> Homo sapiens

<400> 380
gccaacactg acactttgct gccaaaagcc tttaatatgc cctggtccca ggctgtgttc 60

atgaaagcgg acacagcagt gcttccagct tcatggttcc caggttcagg ttcctcccag 120

cggaggtgga agggcagccc tcacacctgg cacccctgag tgccatactc ctggaggaag 180

tcgttgagct gggcacaggc tgcccgctgg cgttgcttcc ggcacaggcc tttcaaaggg 240

catctcctcg atccagttat tggattccag cagttactgg ggttttccct cgagtccaaa 300

agtgggccca atccggcccc atgttaaaat ttctttccca ggtttcaaag caaagggcac 360

aggggtctcg a 371

<210> 381

<211> 474

<212> DNA

<213> Homo sapiens

<400> 381
gcagatgttc tgtgttttac ggggcaccaa gtaagagcag actcttggcc accttgtgtt 60

ctgctgaagt ctgccagtgt gctgagggga agtgccctcg ccagcgtcgc gcccgtggag 120

cggggtctgc aggacgagga tggctacagg atgaagtttg cctgctacta cccccgtgtg 180

gagtacggct tccaggttaa ggttctccga gaagacagca gactgctttc cgcctctttg 240

agaccaagat cacccaagtc ctgcacttca ccaaggatgt caaggccgct gctaatcaga 300

tgcgcaactt cctggttcga gcctcctgcc gccttcgctt ggaacctggg aaagaatatt 360

tgatcatggg tctggatggg gccacctatg acctcgaggg acaccccagt acctgctgga 420

ctcgaatagc tggatcgagg agatgcctct gaacgcctgt gccggagcac ccgc 474

<210> 382

<211> 329

<212> DNA

<213> Homo sapiens

<220>

<221> misc_feature

<222> (311)..(311)

<223> a, t, g, c

<400> 382
gtgatatttt tggcgtttta ttaaaaatgg aaaaagttgt tttagtgagc actcatggtg 60

ctttctcccc tcctccccca ggcagacccc aaaagtgtca gggaaaggca ggccccctgg 120

aggggtgcgg tggggctgaa gcaggggctg ctatggctac cagaggtgag ctggtgatgt 180

gagctgagtt gtcacatgac actctccacc acgacgacct tgctgctctc ggacactggg 240

gtcagggtgg tcaggcccct gacatctgcg tcctgagctc ggtactccaa gtggtggagg 300

ccccagatgg ngctgggtca ggtgctgcc 329

<210> 383

<211> 248

<212> DNA

<213> Homo sapiens

<400> 383
tgagatgtgg atgttgcttt tgcacctacg ggggcatctg agtccagctc cccccaagat 60

gagctgcagc cccccagaga gagctctgca cgtcaccaag taaccaggcc ccagcctcca 120

ggccccccaac tccgcccagc ctctccccgc tctggatcct gcactctaac actcgactct 180

gctgctcatg ggaagaacag aattgctcct gcatgcaact aattcaataa aactgtcttg 240

tgagctga 248

<210> 384

<211> 385

<212> DNA

<213> Homo sapiens

<400> 384
tttgactatt aaacatggct ttatttaagt ttgggcaaaa agatttctga gttaatctgg 60

gagatagaga aaataagaat ctgtaggatt ttggtttttg ttttatatat acaactcttc 120

tctgctgaat gtaatataga tggaaaaatt gtgatgctgc tatcaagctg ttgaaaacag 180

tctgctcacc cagatgggca ctcatttgaa agacaggcac accctgtcat ttcccagtag 240

ccagccctgc agttcttaca cctggcttta tgcaagcaca gaaggtgagg cataagccaa 300

ttaataactt gtgtgattcc aggccgtctc tacccccatg gccaaattct ggactgaaga 360

ttgcaaaccc acaggagact ctgct 385

<210> 385

<211> 465

<212> DNA

<213> Homo sapiens

<220>

<221> misc_feature

<222> (356)..(356)

<223> a, t, g, c

<400> 385
tagacacctg acagaactcc tggccttgat gtctcagctg gaacaccaga acagtaccat 60
ctactacggc ttttgcatgt agcagcaaag aaaaaacaac tcgagcttgc aaataggaag 120
gactcaatca gtatttgtga atgagtgaat taatggataa atataaagag aagggagtga 180
acagatctgt gggattcttg ggcattggaa tcaacaggac atgatgattg cttggataac 240
agctgctggt agttcagaag tgtattcctt tcctaattgg gcatttgaag gattcaaccc 300
ataatgacat catcctaaac atcctcatag agatagcagt ctatgagcca gtggtnttga 360
acagttttct tccaatgctg aaagagattg gtgagagatt cccctacctc actggacaga 420
tggcaaggat ttatggagct gttgggcatg tggatgaaca aactc 465

<210> 386

<211> 472

<212> DNA

<213> Homo sapiens

<400> 386
ttaagaactg aaggtttacc atcttgatgt aggccttgcc gtcctgaaga gcactcgggc 60
tcatgtgttt cattcactca catgttaaat atgaagccct atgtacaaca tagcctgtgg 120
gaactgtggc aggggtagct gtctccatcc tccagaacct tgtcacaatc aagaatagga 180
aatgagacgc acaaatcact accacctgtc gaagtggcct tgcagtgcta aatctaagtg 240
ctgaggcaat ttgcctgcag tccttgccac ttcttgttga ttagtccagc agcccccagc 300
tgggaggcaa ttctgttcac cctcactccc acctcaggac atctggcaat gtctggaaac 360
agttggttgt caccacttgg ggaaagggga gtgtgctttt ggcaggtaga ggcctgggag 420
gctgctccac atcctacagt gcacagaaca gtctccccaa ccaagaataa tc 472

<210> 387

<211> 422

<212> DNA

<213> Homo sapiens

<400> 387
tagccaaagc ggacaaatct gtcccctccc ctttgttgta accacacatt caaggcttcc 60
ttcagtccag ccccatctcc cttttcgttg cctccctttc actactccaa atgagcttgc 120
catactttaa acgggtcttg cactttctta tcccttctct ggaatgtcat ttctactgaa 180
gaaaatccta ctcttccctc aagacccttc tttaatcccc tcttgccatg gaaacttgag 240
attttcattg aggagctaag ttggaaaata taaactagag ctgagtctct accctggcac 300
tattgacatt tggggccaga ttattcttgg ttggggagac tgttctgtgc actgtaggat 360
gtggagcagc ctcccaggcc tctacctgcc aaaagcacac tcccctttcc ccaagtggtg 420
ac 422

<210> 388
<211> 418
<212> DNA
<213> Homo sapiens

<400> 388
aggtgaaagt ttgctattta tttagtctta gaaaaacact gaaagaaaaa ggcaggaaat 60
gtagtacgca tgtggaagaa tggagctggc cacatgtagt tttagcagct gcagaggaaa 120
ctggctgagt ctaaggttac atatttctgc tcagtaaggg tatccatggt gataactctc 180
atgatgacgg tacatccctg gtaaccatcc tggtatcctt ggtggtaact atggtaacca 240
tagcctgcat actcatcttc tgggcagcca tccccagtga ctgctgaatc gccatttcct 300
gtctccgaag ttgcatcgaa tcaattaaat cgtacacaat caccatggac cacattgggg 360
aaggatacca ggatttgaca tttccgtctt ttccgactag aagcatggag aagtactc 418

<210> 389
<211> 295
<212> DNA
<213> Homo sapiens

<400> 389
gcagctctct gcactcagtg gtcaggcgtg caattttggt ctgcgccaca taaccattct 60
gaagctttta ggcgttggag aggaagttgg gggagtgtta gaactgttcc caattaatgg 120

gagctctgtt gttgagcgag aagacgtacc agcccatttg gtgaaagaca ttcgtaacta 180

ttttcaagtg agcccggagt acttctccat gcttctagtc ggaaaagacg gaaatgtcaa 240

atcctggtat ccttccccaa tgtggtccat ggtgattgtg tacgatttaa ttgat 295

<210> 390

<211> 443

<212> DNA

<213> Homo sapiens

<220>

<221> misc_feature

<222> (352)..(352)

<223> a, t, g, c

<220>

<221> misc_feature

<222> (407)..(407)

<223> a, t, g, c

<220>

<221> misc_feature

<222> (412)..(412)

<223> a, t, g, c

<400> 390
acagtgacgc ttcacagggc tcctggattt ggatttggaa ttgcaatatc tggtggacga 60

gataatcctc attttcagag tggggaaacg tcaatagtga tttcagatgt gctgaaagga 120

ggaccagctg aaggacagct acaggaaaat gaccgagttg caatggttaa cggagtttca 180

atggataatg ttgaacatgc ttttgctgtt cagcaactaa ggaaaagtgg gaaaaatgca 240

aaaattacaa ttagaaggaa gaagaaagtt caaataccag taagtcgtcc tgatcctgaa 300

ccagtatctg ataatgaaga agatagttat gatgagggaa atacatgatc cnagaagtgg 360

gccccgagtg ggtgtgggtt aacagaaggg agttgaggaa ggttttnggc cnaggggtta 420

ggaagttgca agtaggagag agg 443

<210> 391

<211> 355

<212> DNA

<213> Homo sapiens

<220>

<221> misc_feature

<222> (118)..(118)

<223> a, t, g, c

<220>

<221> misc_feature

<222> (133)..(133)

<223> a, t, g, c

<220>

<221> misc_feature

<222> (144)..(144)

<223> a, t, g, c

<220>

<221> misc_feature

<222> (147)..(147)

<223> a, t, g, c

<220>

<221> misc_feature

<222> (151)..(151)

<223> a, t, g, c

<220>

<221> misc_feature

<222> (152)..(152)

<223> a, t, g, c

<220>

<221> misc_feature

<222> (157)..(157)

<223> a, t, g, c

<220>

<221> misc_feature

<222> (164)..(164)

<223> a, t, g, c

<220>

<221> misc_feature

<222> (175)..(175)

<223> a, t, g, c

<220>

<221> misc_feature

<222> (180)..(180)

<223> a, t, g, c

<220>

<221> misc_feature

<222> (185)..(185)

<223> a, t, g, c

<220>

<221> misc_feature

<222> (186)..(186)

<223> a, t, g, c

<220>

<221> misc_feature

<222> (188)..(188)

<223> a, t, g, c

<220>

<221> misc_feature

<222> (190)..(190)

<223> a, t, g, c

<220>

<221> misc_feature

<222> (195)..(195)

<223> a, t, g, c

<220>

<221> misc_feature

<222> (204)..(204)

<223> a, t, g, c

<220>

<221> misc_feature

<222> (206)..(206)

<223> a, t, g, c

<220>

<221> misc_feature

<222> (233)..(233)

<223> a, t, g, c

<220>

<221> misc_feature

<222> (326)..(326)

<223> a, t, g, c

<400> 391
gggccatcat cttcttgaat attgccatct cttgctgcca aactatcttg tgaaatttcc 60

ttaacaaata tatggcttgc aatcgaagac catattcttc atttttccgg gatttcanca 120

gtgtgacttt agnagggtta gganggntgg nnggagncac tganccgcct gtttnaccgn 180

ggggnnangn tcctntctct actngnactt ctatccctcg gccaaatctt ctnactcctt 240

ctgttaacca caccactccg gccacttctt gggatcatgt atttcctcat cataactatc 300

ttcttcatta tcaggatact gggttnaggg atcagggagg atttaatggg tattt 355

<210> 392

<211> 512

<212> DNA

<213> Homo sapiens

<400> 392
cagagatgga cagagaactt tattttggat tgtggatgtg gactttttg tacataaata 60

agaaaaacca aaatactcca aagatgactt cccctgcctc ctactccagt atgacagagg 120

aggatgtaag gccttagcca tgatctgcag gggtctggga gtcaggcccg gcctattgct 180

tgggtctctc tctatttata tatctaagtt cacagtgttt cttattcccc cctaagcttc 240

tagaggctca tggccctgta gttaggcctg gctcattctg cacctttcca gggaggtgga 300

aggaccctgt gccctccttc ccaatcttct ttttcaggct cgccaaggcc taggacctat 360

gttgtaattt tactttttat ttctaaagtt gtagtgaagc tctcacccat aataaaggtt 420

gtgaatgttc tgtgagtgtc atggagatgg gctagggagg ggattttaca cttcactttc 480

cagacccctg gtttggggga agagggtcca tg 512

<210> 393

<211> 425

<212> DNA

<213> Homo sapiens

<400> 393
gcagtggctg ccgcagagga tgggacagag acacccggac ttctccccca ggacctacca 60

ccctctgtcc ttgcagtgga actagaggag gatggaagag cccgacctct ggtcctgcac 120

atgtccaccc tcctgcggat gatcccaacc atggcagagg acaaagagag agtcagcact 180

ggaaccaagt ttgccctcaa gctctggctg caacatctgg gcagaacacc cccaccgtgt 240

aaaagtgcag cttacctgga cccagggcca gcaaaggaag aatggaacat ggaccctctt 300

cccccaaacc aggggtctgg aaagtgaagt gtaaaatccc ctccctagcc catctccatg 360

acactcacag aacattcaca acctttatta tgggtgagac ttcactacaa ctttagaaat 420

aaaag 425

<210> 394

<211> 291

<212> DNA

<213> Homo sapiens

<400> 394
caaaagacaa catattttat atcaaacaag tttgaagagc cctgaattgc agcattctgt 60

aacataaaca aacaaaaagc tggtatagga tttattgtca aaggcagaat ttcttcaggc 120

aggtaagtaa ggaggtggtg gttctttttc aggcattttc acggccattt cataggttgg 180

caaaacgtac tgaggaggtg cttcaaaggc agggtacaca gcaatctccg gcacgtttcg 240

gttgttgatg tatttatagc agttccaaac acagttaatt agataagccc t 291

<210> 395

<211> 588

<212> DNA

<213>  Homo sapiens

<400>  395
aagacgcgtc gttgggtttt ggaggccgtg aaacagccgt ttgagtttgg ctgcgggtgg    60

agaacgtttg tcaggggccc ggccaagaag gaggcccgcc tgttacgatg gtgtccatga    120

gtttcaagcg gaaccgagtg accggttcta cagcacccgg tgtgcggctg ttgccatgtc    180

cgcaccggga cgatcatcct ggggacctgg tacatggtag taaacctatt gatggcaatt    240

ttgctgactg tggaagtgac tcatccaaac tccatgccag ctgtcaacat tcagtatgaa    300

gtcatcggta attactattc gtctgagaga atggctgata atgcctgtgt tctttttgcc    360

gtctctgttc ttatgtttat aatcagttca atgctggttt atggagcaat ttcttatcaa    420

gtgggttggc tgattccatt cttctgttac cgactttttg acttcgtcct cagttgcctg    480

gttgctatta gttctctcac ctatttgcca agaatcaaag aatatctgga tcaactacct    540

gattttccct acaaagatga cctcctggcc ttggactcca gctgcctc    588

<210>  396

<211>  328

<212>  DNA

<213>  Homo sapiens

<400>  396
tttttagcca tacacatgcc tttatttaga tcagcttttt tcaaaatgca gccaaactta    60

tgagttggac agcccaaagt aaccagccct attccactga gttagtttac cccacagcag    120

tagaacccag tgctggtttg gttcctggcc catggtggga cagcgtgaag gtgatggagg    180

gctctagcac aaggaggtgc tgagtgccac cggaggtcat atctgcagac agcctcattg    240

ctttactaaa gggtcaggct aggcatgacc gatcaaggat gggtaggggg taaaatggag    300

gaggaacatg actgatgagg tgcctgga    328

<210>  397

<211>  457

<212>  DNA

<213>  Homo sapiens

<220>

<221> misc_feature

<222> (3)..(3)

<223> a, t, g, c

<220>

<221> misc_feature

<222> (4)..(4)

<223> a, t, g, c

<220>

<221> misc_feature

<222> (5)..(5)

<223> a, t, g, c

<220>

<221> misc_feature

<222> (7)..(7)

<223> a, t, g, c

<220>

<221> misc_feature

<222> (51)..(51)

<223> a, t, g, c

<220>

<221> misc_feature

<222> (93)..(93)

<223> a, t, g, c

<220>

<221> misc_feature

<222> (235)..(235)

<223> a, t, g, c

<220>

<221> misc_feature

<222> (245)..(245)

<223> a, t, g, c

<220>

<221> misc_feature

<222> (284)..(284)

<223> a, t, g, c

<220>

<221> misc_feature

<222> (300)..(300)

<223> a, t, g, c

<220>

<221> misc_feature

<222> (335)..(335)

<223> a, t, g, c

<220>

<221> misc_feature

<222> (341)..(341)

<223> a, t, g, c

<220>

<221> misc_feature

<222> (359)..(359)

<223> a, t, g, c

<220>

<221> misc_feature

<222> (367)..(367)

<223> a, t, g, c

<220>

<221> misc_feature

<222> (393)..(393)

<223> a, t, g, c

<220>

<221> misc_feature

<222> (427)..(427)

<223> a, t, g, c

<220>

<221> misc_feature

<222> (453)..(453)

<223> a, t, g, c

<400> 397
aannnantgt tgacctgtca ctgtttatta tttcagcact aaaactgagg ngcctaactg 60

ctggctcttc ttccctttgt atttgtgtaa ggngcactgc actcccataa aaggttttaa 120

aatacaaaat gtacaagaac acacaattcc aagtgctgta aacataactg agaaccagtt 180

cctttactaa acatccattt tataaaacac aaggtttcaa tttgagccca tctgngcctt 240

aaagntccat tctgaatacc aaaaacaggg cttcacagcc aggnccagaa gaggtctggn 300

gataatggct ggccctgggt ggggatagtt tacanccggg nagcagcacc acacatggna 360

cccaaangac atgttctttt taaagctgtt ttnagccatg ttttctctgt ggcatctcca 420

gtaagcngga gggtaccctt tcctttcctc aanccaa 457

<210> 398

<211> 394

<212> DNA

<213> Homo sapiens

<220>

<221> misc_feature

<222> (374)..(374)

<223> a, t, g, c

<400> 398
gtctattctt cagcagatca ctgtggcccc tcttggagct gggtgttttc aaattttctt 60

gaaaagtgag ttgtgtcaca gaatgtggat agtgagattt gcttctgggc ctgtggttgg 120

acgcccacag cttcctttgg aggggcaaga agttggcctc tcggttgaca ttccagagaa 180

atggccagac ttgcccctac ccttttgccc ctggtgtagc tgcctcacat gaggttattg 240

caagagttcc ccttgcaata ccctttgtgc cctcaagaat taaaagcctc tatgatccag 300

agttgctatg caccaaattt tgatgccttc taaatacctt gatgcctgta gcactagaaa 360

tgctttccta tttnaaaatg aaacatttaa tttt 394

<210> 399

<211> 541

<212> DNA

<213> Homo sapiens

<400> 399
ttttcaggag attcttagga gagttttatt cattcattga tccagtattt acaggggcta 60

gaggggtcaa gctgtgctca gcccagaggc agctgccaca cttgccagca ccccccactc 120

agtcactatg tacagataaa ggggcctgct tggatcacct ttttcaaagc catctggcag 180

aggccatggg gctgtgttgg ggcctgggct ccagaggcac tgctgggccc attacccctt 240

```
ggcatcaggt cctctggaac acaggggctc caacgggttg tcttgatcct gctgtccccc    300
accctgagtg ccttgcagag gctgaggaaa ctggagtagc aggaagagct gagtggtgcc    360
agcttcctat aagcaaccct gtctctgcta cccctgagag ggagacatgg taatactgag    420
gggctggaca gaggctcttc tgagctcaag cgccagggac agagacctag agcccaattt    480
aggcccatag ctagggccac aacactgaag gcgaagagcc tagaaccttg ctatggagag    540
c                                                                    541
```

<210> 400

<211> 468

<212> DNA

<213> Homo sapiens

<220>

<221> misc_feature

<222> (2)..(2)

<223> a, t, g, c

<220>

<221> misc_feature

<222> (3)..(3)

<223> a, t, g, c

<220>

<221> misc_feature

<222> (14)..(14)

<223> a, t, g, c

<220>

<221> misc_feature

<222> (33)..(33)

<223> a, t, g, c

<220>
<221> misc_feature
<222> (36)..(36)
<223> a, t, g, c

<220>
<221> misc_feature
<222> (40)..(40)
<223> a, t, g, c

<220>
<221> misc_feature
<222> (210)..(210)
<223> a, t, g, c

<220>
<221> misc_feature
<222> (257)..(257)
<223> a, t, g, c

<220>
<221> misc_feature
<222> (263)..(263)
<223> a, t, g, c

<220>
<221> misc_feature
<222> (281)..(281)
<223> a, t, g, c

<220>
<221> misc_feature
<222> (359)..(359)
<223> a, t, g, c

<220>
<221> misc_feature
<222> (412)..(412)
<223> a, t, g, c

<220>
<221> misc_feature
<222> (424)..(424)
<223> a, t, g, c

<220>
<221> misc_feature
<222> (435)..(435)
<223> a, t, g, c

<220>
<221> misc_feature
<222> (460)..(460)
<223> a, t, g, c

<400> 400
tnnttttttt tttncacctt tccctaatac ttnatnggtn acctctaggc ctgtgtgcgg 60
ctgggtgggc ttgggggagg gcgtcactat tcagcttcta ggtggaggca tgagaaggcc 120
ttggctaggc cctccagggt cccatactgt ggagtttgga ggggcaggtc tggcctttcc 180
tgggtcagca tagggcaccc aggtgggggn acaggtggac acccagcaca ggcacctagg 240

caggggcaca agctcantat ccnttagcca gcctaattgt ntttggagaa atattccttg 300

ctgtcatcca cgttgggttt aatcgtgtca ctgccaggtt tccagccagc gggacaaant 360

ttccccatgt tcgtttgtgt attgggaagg cctgggacca gccgcagagt tnatcccacg 420

gagngtccca aaggnaaatc attaaacagt gattttggcn aaggaaaa 468

<210> 401

<211> 394

<212> DNA

<213> Homo sapiens

<400> 401
acttcaaggc cacagcggtg gttgatggcg ccttcaaaga ggtgaagctg tcggactaca 60

aagggaagta cgtggtcctc tttttctacc ctctggactt cacttttgtg tgccccaccg 120

agatcatcgc gttcagcaac cgtgcagagg acttccgcaa gctgggctgt gaagtgctgg 180

gcgtctcggg tggactctca gttcacccac ctggcttgga tcaacacccc ccggaaagag 240

ggaggcttgg gccccctgaa catccccctg cttgctgacg tgaccagacg cttgtctgag 300

gattacggcg tgctgaaaac agatgagggc attgctaaca ggggcctctt tatcatcgat 360

gggcaagggt gttcctttcg ccagatcaat gtta 394

<210> 402

<211> 446

<212> DNA

<213> Homo sapiens

<220>

<221> misc_feature

<222> (17)..(17)

<223> a, t, g, c

<220>

<221> misc_feature

<222> (28)..(28)

<223> a, t, g, c

<220>

<221> misc_feature

<222> (30)..(30)

<223> a, t, g, c

<220>

<221> misc_feature

<222> (45)..(45)

<223> a, t, g, c

<220>

<221> misc_feature

<222> (51)..(51)

<223> a, t, g, c

<220>

<221> misc_feature

<222> (62)..(62)

<223> a, t, g, c

<220>

<221> misc_feature

<222> (67)..(67)

<223> a, t, g, c

<220>

<221> misc_feature

<222> (70)..(70)

<223> a, t, g, c

<220>

<221> misc_feature

<222> (73)..(73)

<223> a, t, g, c

<220>

<221> misc_feature

<222> (84)..(84)

<223> a, t, g, c

<220>

<221> misc_feature

<222> (89)..(89)

<223> a, t, g, c

<220>

<221> misc_feature

<222> (92)..(92)

<223> a, t, g, c

<220>

<221> misc_feature

<222> (97)..(97)

<223> a, t, g, c

<220>

<221> misc_feature

<222> (106)..(106)

<223> a, t, g, c

<220>

<221> misc_feature

<222> (111)..(111)

<223> a, t, g, c

<220>

<221> misc_feature

<222> (117)..(117)

<223> a, t, g, c

<220>

<221> misc_feature

<222> (119)..(119)

<223> a, t, g, c

<220>

<221> misc_feature

<222> (133)..(133)

<223> a, t, g, c

<220>

<221> misc_feature

<222> (136)..(136)

<223> a, t, g, c

<220>

<221> misc_feature

<222> (139)..(139)

<223> a, t, g, c

<400> 402
gtttgttgtt ttttttntta agtggaantn ggctttccag ctttnttgac nctccctgtc 60

tntgcanttn aantgtacat gccntttang gngcccnaca agcttnggga ncagtcncna 120

acgcatccct atntgnagnt gggcgaaggc gcactggagc aggttaagtc ccaggcggca 180

atggtgagcc aagcgtggga acagcccaaa ctaaagtcgg ggtctctgtg cggcggccgg 240

gccacttcgg cacttcagtg tttaaacttc tgcatgcagt gtaaagacag acagctggcc 300

acagtgctgc cctgcagtaa agaccagcca ccgtgaccgg ctctcccctg cctccctcct 360

cccagcccac agtcagacgg agcctctgtg gcaagctcag gcccagtcca gggccctcag 420

agtgtacctc gtgccgaatt cttggc 446

<210> 403

<211> 406

<212> DNA

<213> Homo sapiens

<220>

<221> misc_feature

<222> (25)..(25)

<223> a, t, g, c

<220>

<221> misc_feature

<222> (63)..(63)

<223> a, t, g, c

<220>

<221> misc_feature

<222> (66)..(66)

<223> a, t, g, c

<220>

<221> misc_feature

<222> (67)..(67)

<223> a, t, g, c

<220>

<221> misc_feature

<222> (70)..(70)

<223> a, t, g, c

<220>

<221> misc_feature

<222> (86)..(86)

<223> a, t, g, c

<220>

<221> misc_feature

<222> (108)..(108)

<223> a, t, g, c

<220>

<221> misc_feature

<222> (149)..(149)

<223> a, t, g, c

<220>

<221> misc_feature

<222> (155)..(155)

<223> a, t, g, c

<220>

<221> misc_feature

<222> (167)..(167)

<223> a, t, g, c

<220>

<221> misc_feature

<222> (180)..(180)

<223> a, t, g, c

<220>

<221> misc_feature

<222> (181)..(181)

<223> a, t, g, c

<220>

<221> misc_feature

<222> (183)..(183)

<223> a, t, g, c

<220>

<221> misc_feature

<222> (219)..(219)

<223> a, t, g, c

<220>

<221> misc_feature

<222> (287)..(287)

<223> a, t, g, c

<400> 403

tacactctga gggccctgga ctggncttga gcttgccaca gaggctccgt ctgactgtgg 60

gcnggnnggn gggaggcagg ggaganccgg tcacgtggct ggtctttnct gcagggcagc 120

actgtggcca gctgtctgtc tttacacang cctgntaagt ttaaacnctg gcttgccgan 180

ntngccgtgc cgccgcacag agaccccgac tttagtttng gctgttccac gcttggctca 240

ccattgccgc ctgggactta acctgctcag gcgggccttc gccagcntgc aaatagggat 300

gcgttagatg actgttccca aagcttgttg ggctccttaa atggcattgt acaatttaag 360

tgcaaagaca gggagtgtca ataaagatgg aaagccattt ccagtt 406

<210> 404

<211> 396

<212> DNA

<213> Homo sapiens

<400> 404
atgactgaca gattatctta ttttattatt taacgtatct catgttttct tttaaggtct 60

ctattgagat aaagagttcc agggaaagaa aggtcacagt gctcggtaaa caacccagca 120

aacggcggct ctgctgctgc gtgggccaag cgggtggtct ggggctgtga cggcctctca 180

gtgtgtgatg aagtttcagc gccctccaca gtggcagtgc atgcttggaa tcccgcttcc 240

ggttgtgttg ttcattctgg ctagctgagt ttcagttagt tcatgatctg tttaaaccag 300

agtgagtacc tggagacctt ggtgacaagg accatgtgag cctgccctcc tactggctgc 360

gagcccagga cccccgtgag aaaccaggag cctctg 396

<210> 405

<211> 359

<212> DNA

<213> Homo sapiens

<220>

<221> misc_feature

<222> (5)..(5)

<223> a, t, g, c

<220>

<221> misc_feature

<222> (13)..(13)

<223> a, t, g, c

<220>

<221> misc_feature

<222> (117)..(117)

<223> a, t, g, c

<220>

<221> misc_feature

<222> (312)..(312)

<223> a, t, g, c

<400> 405
ttctngctgg gtnctgttgt atttttttgtt ttgtttttca atccattgtt aattctggaa 60

aagctgggct ggcaggtcct gtggtgaata tgttggggct gtggcctggg gttcttntgg 120

gaggccaagg catccatcct catagccagg cggggctgtc attgccagta caaagccagc 180

tgcagtcgcc acttcatctc agaggatgcg caggagcgca ggacaaggag gtcttccagc 240

agaacatgaa gcggcggctg gagtccttta agtccaccaa gcacaacatc tgcttcacca 300

agagcaagcc angaccccgc aagactggcc gcaggaaggg catgtcttcc ctcagggac 359

<210> 406

<211> 454

<212> DNA

<213> Homo sapiens

<220>

<221> misc_feature

<222> (9)..(9)

<223> a, t, g, c

<220>

<221> misc_feature

<222> (10)..(10)

<223> a, t, g, c

<220>

<221> misc_feature

<222> (14)..(14)

<223> a, t, g, c

<220>

<221> misc_feature

<222> (16)..(16)

<223> a, t, g, c

<220>

<221> misc_feature

<222> (18)..(18)

<223> a, t, g, c

<220>

<221> misc_feature

<222> (41)..(41)

<223> a, t, g, c

<220>

<221> misc_feature

<222> (69)..(69)

<223> a, t, g, c

<220>

<221> misc_feature

<222> (90)..(90)

<223> a, t, g, c

<220>

<221> misc_feature

<222> (197)..(197)

<223> a, t, g, c

<220>

<221> misc_feature

<222> (226)..(226)

<223> a, t, g, c

<220>

<221> misc_feature

<222> (263)..(263)

<223> a, t, g, c

<220>

<221> misc_feature

<222> (282)..(282)

<223> a, t, g, c

<220>

<221> misc_feature

<222> (307)..(307)

<223> a, t, g, c

<220>

<221> misc_feature

<222> (365)..(365)

<223> a, t, g, c

<220>

<221> misc_feature

<222> (379)..(379)

<223> a, t, g, c

<400> 406

```
ttttttttnn ctcngntnta gaacactgca gctttattga nagaacaacc gcgtccgcaa      60
ctagaggtna catttggggg cttggaggtn aaagagaggc aaatggggaa cagaggctgg     120
ggtggagaca gacagcaagg caaaggggtg aggatggggc tgtgtgacta aaaaaacaac     180
cccaatcatt tcacccntta ataaggggt ctcctgttgt gctttnacct tggtggcggg     240
gtgggctggg gtggtcagac atntgggcct gggcatgagg anttttggct tttctcccaa     300
gtctttnagg tctcagggct ggtgcagccc cttccctccc tgacccttct cctggggctg     360
ggganaaagg ccttgggtna gctggctact ctaggggcag gaagaatgca tgggagacct     420
agagggccac cagagttggg ctgagatggt gatt                                 454
```

<210> 407

<211> 511

<212> DNA

<213> Homo sapiens

<220>

<221> misc_feature

<222> (338)..(338)

<223> a, t, g, c

<220>

<221> misc_feature

<222> (474)..(474)

<223> a, t, g, c

<220>

<221> misc_feature

<222> (479)..(479)

<223> a, t, g, c

<220>

<221> misc_feature

<222> (492)..(492)

<223> a, t, g, c

<400> 407
cggaacaggg aggcattttg tggccttaaa acggggtgtt cagggtcagc cctcagggtg 60

ctggggccct tccatccctt gtccccttca ggttggtgaa aaggactccg gggccaggt 120

gctgtatagc agcttatgga agtctcagct gggctatcct gcccttggga gcacagacag 180

gctcctaggg tgctgagtga agcctgaaga ccaagccccc tcctcctgga atgctccttc 240

cacccctacc tctcagagat gggcctgaca cctctttctc attcattctc cttttccctg 300

tgctctggga aagcccctgg ctcaggctgt tcagagtnag gatggacctc aaaagtttga 360

tcccctcgtg cagatgaaga agctgaagcc cagagtgcgg aaggggagtt ggcccaaggt 420

tcacacagct aagttttgag ttagagccag tctttgggag agccaataat taanagccnt 480

gggggtgttc anaacgtttg ttaatgcccg t 511

<210> 408

<211> 343

<212> DNA

<213> Homo sapiens

<220>

<221> misc_feature

<222> (155)..(155)

<223> a, t, g, c

<220>

<221> misc_feature

<222> (245)..(245)

<223> a, t, g, c

<220>

<221> misc_feature

<222> (259)..(259)

<223> a, t, g, c

<220>

<221> misc_feature

<222> (294)..(294)

<223> a, t, g, c

<220>

<221> misc_feature

<222> (303)..(303)

<223> a, t, g, c

<220>

<221> misc_feature

<222> (311)..(311)

<223> a, t, g, c

<220>

<221> misc_feature

<222> (323)..(323)

<223> a, t, g, c

<220>

<221> misc_feature

<222> (330)..(330)

<223> a, t, g, c

<220>

<221> misc_feature

<222> (332)..(332)

<223> a, t, g, c

<400> 408
tttaactaaa atggtcactt ttaatgggaa ccagaggtat agttacaatt acatagtccg 60

acggggggaa acccttgggt gatcaggaat ggggaaggtt acaaaataac gagggtaaca 120

cttgggtaca ggacaaaaag ctgttccaga acctngggag ccaggctaat taatacggcc 180

tctccctgcg atcctatctg tgctcacccc tggaatccat cttgccaggg ccaaagccac 240

ctctntcccc accaccccng ccccctcgga agcctccacg gtccccgcct gcanccccgg 300

tangccgcct ncgatcataa gcntcctctn gncaccactg acg 343

<210> 409

<211> 377

<212> DNA

<213> Homo sapiens

<220>

<221> misc_feature

<222> (54)..(54)

<223> a, t, g, c

<220>

<221> misc_feature

<222> (212)..(212)

<223> a, t, g, c

<220>

<221> misc_feature

<222> (213)..(213)

<223> a, t, g, c

<220>

<221> misc_feature

<222> (216)..(216)

<223> a, t, g, c

<220>

<221> misc_feature

<222> (241)..(241)

<223> a, t, g, c

<220>

<221> misc_feature

<222> (251)..(251)

<223> a, t, g, c

<220>

<221> misc_feature

<222> (263)..(263)

<223> a, t, g, c

<220>

<221> misc_feature

<222> (274)..(274)

<223> a, t, g, c

<220>

<221> misc_feature

<222> (360)..(360)

<223> a, t, g, c

<220>

<221> misc_feature

<222> (456)..(456)

<223> a, t, g, c

<220>

<221> misc_feature

<222> (486)..(486)

<223> a, t, g, c

<220>

<221> misc_feature

<222> (507)..(507)

<223> a, t, g, c

<220>

<221> misc_feature

<222> (554)..(554)

<223> a, t, g, c

<400> 409
ggcggtggag gacagcagcg agctggtgac tggaagtgtc ctaatcccac ctcnagagaa 60

tatgaacttc tcttggagga atgaatgcaa ccagtgtaag gcccctaaac cagatggccc 120

aggaggggga ccaggtggct ctcacatggg gggtaactac ggggatgatc gtcgtggtgg 180

cagaggaggc tatgatcgag gcggctaccg gnnccnggcg gggaccgtgg aggcttccga 240

nggggccggg ntggtgggga canaggtggc tttngcctgg caagatggat tccaaggggt 300

gagcacagac aggatcgcaa gggataaggc cgtattaaat tagcctggct ccccaggttc 360

ttggaacatg ccttttt 377

<210> 410

<211> 521

<212> DNA

<213> Homo sapiens

<400> 410
aaaaacaaca acaaaccact ttaatggaca cataaaatcc ctggggggtct cattgcaatg 60

cagatttgga ttaaacagtc tgggtgggtc ctgagaccct gatccccttg cttttcacct 120

atggccacat tttgagaagc aaggccactc aaagggcccc catctgtgtg aatcaacccc 180

tgctccatct gcccctataa atgacaggag tgggggtgtc cccaggtctg agcctggagg 240

gggttgcagg cttcctccag gcatggaagt ggagtgagag gaggctgtag ttaaacgggt 300

tactctgtca gcagacacag aggagaccag aatagctttt atttcacatg aagctaagga 360

aggaagtcga gaggacaggg ctggttctgt gggcgctgct ggcaggtgca ccacctccaa 420

ctgctgggct ccagtccagg gctcagaagc catctccgag aaggtcggga gcggggctga 480

agggtcccgg gggcaggttc caggccagct cctccagcaa g 521

<210> 411

<211> 567

<212> DNA

<213> Homo sapiens

<220>

<221> misc_feature

<222> (360)..(360)

<223> a, t, g, c

<220>

<221> misc_feature

<222> (456)..(456)

<223> a, t, g, c

<220>

<221> misc_feature

<222> (486)..(486)

<223> a, t, g, c

<220>

<221> misc_feature

<222> (507)..(507)

<223> a, t, g, c

<220>

<221> misc_feature

<222> (554)..(554)

<223> a, t, g, c

<400> 411
agagaagacc gtggatcacc tggggacaga ggtgaaaggc ctgctgggct gctggaggag 60

ctggcctgga acctgccccc gggacccttc agccccgctc ccgaccttct cggagatggc 120

ttctgagccc tggagctgga gcccagcagt tggaggtggt gcacctgcca ggcagcgcca 180

cagaaccagc cctgtcctct cgacttcctt ccttagcttc atgtgaaata aaagctattc 240

tggtctcctc tgtgtctgct gacagagtaa cccgtttaac tacagcctcc tctcactcca 300

cttccatgcc tggaggaagc ctgcaacccc ctccaggctc agacctgggg acacccccan 360

tcctgtcatt tataggggaa gatggagcag gggttgattc acacagatgg ggggccctct 420

gaattggcct gcttctcaga atgttggcca taggtnaaaa gcaaggggat cggggttcag 480

gaccancaga atgtttagtg aatctgnatg aatgagaccc caggatttat gtgtccatta 540

agtggttgtt gtgntttaaa aaaaaaa 567

<210> 412

<211> 332

<212> DNA

<213> Homo sapiens

<400> 412
tttaaatgga gtcggggtcc cgctgtgttg cccaggctgg tctggacctc ctgggctaaa 60

gtgatcctcc tgccttggct gattttgtta atttttaaat atcttcattt tgatctatca 120

ttaatggaga aatattccgt ctaccatgta tataaggact ttcagcagat gaaatgctta 180

aatagtttaa gggtggaaat gaatacagca attagaaata aggggttgat aaaatatgca 240

tttagttttg ctgttacagt taaagaagca ttttttaaga aagactttct catttatcta 300

ctttgtgcaa taaaacttaa tttttgttgt tg 332

<210> 413

<211> 431

<212> DNA

<213> Homo sapiens

<400> 413
ctacaccagg cacttctgaa gcggtccaaa gagaaacgtt ccctgcccgt cgtagctagg 60

cacgataact ccgtttaatt tctcgagcac accgatgcct atctcgttta tcattcagca 120

gttaagtaat tcatcacttc atatttttac caaagtatat ttacaagtct gtttatctgg 180

atctggcaaa ctggaaaatg tttgaaggga tactcaggct ttgatccatg ccaacatgaa 240

aggactccaa gcacatttaa gtaggctggt cttttcatca tgcatgattt ttttttagt 300

gcaaatttag actttagttt gggaaagaga acagtataca gtatcctttg taagataaat 360

cacaacaaca aaaattaagt tttattgcac aaagtagata aatgagaaag tctttcttaa 420

aaaatgcttc t 431

<210> 414

<211> 474

<212> DNA

<213> Homo sapiens

<400> 414
tatgctatgg tttagctctt tatttctaca attaaaagca gtaaaaactt aaaaaagaat 60

ttttacatca atagaacatt aactacagtt ggatttttca atacctatgt tcctcttccc 120

tctcttgatt aacctgaagt tgatattctt tctgtctact tttaatacat atttatgatt 180

ccatagataa tactgtatat attagccatt ccctatttta tcagtgacaa aaaatttgag 240

agaagagaca cagataatac ttggagttat agatccagtc atagatataa ggaaagttat 300

tagaagtatc tataaaatat caacagatca aaaacactgc ccttttttc ttgaaatgag 360

ggtactaatt gatgggggag agggagagta taaagggaac actagagcag tgtttctctc 420

tgtattaaaa tcacctgggg agctctttaa acgtccttgt tctgggccac agat 474

<210> 415

<211> 375

<212> DNA

<213> Homo sapiens

<400> 415
gaagatcata ctgcattata aatttctgta actaatagta atcaacaatt gtctattttg 60

ttctacccat gcaaacagac tctagcactg tccaatcctg tacccactag ctacagcaca 120

atttgacaac tgaccacttg aaatgtggct actctgaatc taatttcagt taatttaagt 180

gtaaacagcc acatgcagct agtggctact gaattgcaca gtgcaggtct agactcctaa 240

agcagtgctt ctttaccttg gtttcataat gggatcacct gtgaagcttt acaatacacc 300

gctgcctagg ccccttgcca gagcaattgg attttaatct gtgggccccg aacaaggacg 360

tttaaagagc tcccc 375

<210> 416

<211> 374

<212> DNA

<213> Homo sapiens

<400> 416
tttttttttt tttttttaat atctcaaaaa ggaagccact tgcttgatat caaaagtgct 60

gtggaaagaa aggaggggaa aaaaacccac aaataaatgt agaatctagt ttattttacc 120

aaactgtata tttttccact taacatttct acattagcaa tgatgtaact ctccaacatt 180

tccttataaa aaaggcaaac aagaagtgac aactcatgct ccaaatatta aaaaatatat 240

ttaaacatat gatcaagaag atttgggccg tgagtggtag ctcacatctg taaccccagg 300

attttgggag gctgaggtga gtggatcacg aggtcaggag atcgagacca tcctggctaa 360

catggtgaaa cccc 374

<210> 417

<211> 425

<212> DNA

<213> Homo sapiens

<400> 417
cccaggttca agcgattctc ccacctcagc ctcccgagta gctgggatta cagggtgtgt 60

accaccacgc ctggcaattt ttttgtattt ttagcagagt tggggtttcc catgttagcc 120

aggatggtct cgatctcctg acctcgtgat ccactcacct cagcctccca aaatcctggg 180

gttacagatg tgagctacca ctcacggccc aaatcttctt gatcatatgt ttaaatatat 240

tttttaatat ttggagcatg agttgtcact tcttgtttgc ctttttttata aggaaatgtt 300

ggagagttac atcattgcta atgtagaaat gttaagtgga aaaatataca gtttggtaaa 360

atagactaga ttctacattt atttgtgggt ttttttccct cctttctttc cacagcactt 420

ttgat 425

<210> 418

<211> 249

<212> DNA

<213> Homo sapiens

<400> 418
ttttgttggg gtggatttta ttagcgggtc tgcagtctca cgatcacagt ggggcgggac 60

cagcgcggcc cattgagcgc aggcccgacg cgtctcccgc tccttattgg ctgacagcag 120

aggcgttgct gtaaacgtca ccgcgaccgc cccacccagt gcacaaagcg gagtgaagct 180

ggagttcctg ggtccaagtg gtgttagtcc ctgcgggcga aattcctgag tacccgtcct 240

gaggaggct 249

<210> 419

<211> 384

<212> DNA

<213> Homo sapiens

<400> 419
tcaggtccct accggcacat cacacactcc tggaagcatg atcttcactc ccttccttcc 60

acctgctgac ctgtctgtct ttcagaatgt gaaagggctg caaaacgacc ctgaggaatg 120

ggtggctgtg tctgacgcca cggaggaccc atccggtggc acaggcttgc tcagggaacc 180

tgctcttctg cgagggtctt ggaggagccg gttccagaga gccctggcat gtttcaccaa 240

gtgtttcagg ggaggatacc gggcactcgg aatctgagac caccggagcc acagacacag 300

agccatgaac ggtgaccaag ccccttctgc tgctccatgc ccctgctcgc cgcagtgtcc 360

cagtcaccag gcctgaaata aatg 384

<210> 420

<211> 305

<212> DNA

<213> Homo sapiens

<400> 420
aatttttttt tttttttttt ttttaacagt atctccaaca tacattttat ttagtttaaa 60

attccttaat acaagaaggt gttactcctt aaccactgct gcagcctcac cggaccctcc 120

tgatgtggat gaagcaggcg gggtggctgc tggtcggctg tggaggcacc tccaggcaca 180

gggcctagag ggtgagaaca ctggtgacat taatgccata gctgtggctt gggacaggga 240

ttgagaggca gctctcaggg cacatgggca tcagctttca caggatcctg ttcccagtca 300

ggctg 305